# EUROPEAN PATENT APPLICATION

(11) **EP 3 632 923 A1**
(43) Date of publication of application: **08.04.2020**
(21) Application number: 19209982.8
(22) Date of filing: 15.01.2016
(51) Int. Cl.: C07K 14/005, A61K 31/7088

(54) **CENTRAL NERVOUS SYSTEM TARGETING POLYNUCLEOTIDES**

(30) Priority: 16.01.2015 US 201562104101 P; 24.04.2015 US 201562152185 P; 24.04.2015 US 201562152283 P; 11.05.2015 US 201562159888 P; 11.05.2015 US 201562159882 P
(62) Divisional of application: 16737993.2
(71) Applicant: Voyager Therapeutics, Inc., Cambridge, Massachusetts 02139 (US)
(72) Inventor: STEWART, Gregory R., Plymouth, MN 55446 (US); SAH, Dinah Wen-Yee, Hopkinton, MA 01748 (US); HOU, Jinzhao, Belmont, MA 02478 (US); KELLS, Adrian Philip, Arlington, MA 02476 (US); GOULET, Martin, Weston, MA 02493 (US); RAMAMOORTHI, Kartik, Pennington, NJ 08534 (US); ZHOU, Pengcheng, Lexington, MA 02421 (US); SHU, Yanqun, Winchester, MA 01890 (US)
(74) Representative: Hoffmann Eitle

(57) **Abstract**

The invention relates to compositions and methods for the preparation, manufacture and therapeutic use of polynucleotides encoding payloads for the treatment of CNS disorders.

SEQUENCE LISTING

<110> VOYAGER THERAPEUTICS, INC.

<120> CENTRAL NERVOUS SYSTEM TARGETING POLYNUCLEOTIDES

<130> 219 187

<140> TBD
<141> 2016-01-15

<150> 62/159,888
<151> 2015-05-11

<150> 62/159,882
<151> 2015-05-11

<150> 62/152,283
<151> 2015-04-24

<150> 62/152,185
<151> 2015-04-24

<150> 62/104,101
<151> 2015-01-16

<160> 94

<170> PatentIn version 3.5

<210> 1
<211> 6119
<212> DNA
<213> Artificial Sequence

<220>
<223> Description of Artificial Sequence: Synthetic
      polynucleotide

<400> 1
gacggatcgg gagatctccc gatcccctat ggtgcactct cagtacaatc tgctctgatg      60

ccgcatagtt aagccagtat ctgctccctg cttgtgtgtt ggaggtcgct gagtagtgcg     120

cgagcaaaat ttaagctaca acaaggcaag gcttgaccga caattgcatg aagaatctgc     180

ttagggttag gcgttttgcg ctgcttcgcg atgtacgggc cagatatacg cgttgacatt     240

gattattgac tagttattaa tagtaatcaa ttacggggtc attagttcat agcccatata     300

tggagttccg cgttacataa cttacggtaa atggcccgcc tggctgaccg cccaacgacc     360

cccgcccatt gacgtcaata atgacgtatg ttcccatagt aacgccaata gggactttcc     420

attgacgtca atgggtggag tatttacggt aaactgccca cttggcagta catcaagtgt     480

atcatatgcc aagtacgccc cctattgacg tcaatgacgg taaatggccc gcctggcatt     540

atgcccagta catgacctta tgggactttc ctacttggca gtacatctac gtattagtca     600

tcgctattac catggtgatg cggttttggc agtacatcaa tgggcgtgga tagcggtttg     660

actcacgggg atttccaagt ctccacccca ttgacgtcaa tgggagtttg ttttggcacc     720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagc 900

gtttaaactt aagcttggta ccgagctcgg atccatgtgg actctcgggc gccgcgcagt 960

agccggcctc ctggcgtcac ccagcccagc ccaggcccag accctcaccc gggtcccgcg 1020

gccggcagag ttggccccac tctgcggccg ccgtggcctg cgcaccgaca tcgatgcgac 1080

ctgcacgccc cgccgcgcaa gttcgaacca acgtggcctc aaccagattt ggaatgtcaa 1140

aaagcagagt gtctatttga tgaatttgag gaaatctgga actttgggcc acccaggctc 1200

tctagatgag accacctatg aaagactagc agaggaaacg ctggactctt tagcagagtt 1260

ttttgaagac cttgcagaca agccatacac gtttgaggac tatgatgtct cctttgggag 1320

tggtgtctta actgtcaaac tgggtggaga tctaggaacc tatgtgatca acaagcagac 1380

gccaaacaag caaatctggc tatcttctcc atccagtgga cctaagcgtt atgactggac 1440

tgggaaaaac tgggtgtact cccacgacgg cgtgtccctc catgagctgc tggccgcaga 1500

gctcactaaa gccttaaaaa ccaaactgga cttgtcttcc ttggcctatt ccggaaaaga 1560

tgctggatcc gactacaaag accatgacgg tgattataaa gatcatgaca tcgactacaa 1620

ggatgacgat gacaagggat ccgaacaaaa actcatctca gaagaggatc tgtagctcga 1680

gtctagaggg cccgtttaaa cccgctgatc agcctcgact gtgccttcta gttgccagcc 1740

atctgttgtt tgcccctccc ccgtgccttc cttgaccctg gaaggtgcca ctcccactgt 1800

cctttcctaa taaaatgagg aaattgcatc gcattgtctg agtaggtgtc attctattct 1860

ggggggtggg gtggggcagg acagcaaggg ggaggattgg gaagacaata gcaggcatgc 1920

tggggatgcg gtgggctcta tggcttctga ggcggaaaga accagctggg gctctagggg 1980

gtatccccac gcgccctgta gcggcgcatt aagcgcggcg ggtgtggtgg ttacgcgcag 2040

cgtgaccgct acacttgcca gcgccctagc gcccgctcct ttcgctttct tcccttcctt 2100

tctcgccacg ttcgccggct ttccccgtca agctctaaat cgggggctcc ctttagggtt 2160

ccgatttagt gctttacggc acctcgaccc caaaaaactt gattagggtg atggttcacg 2220

tagtgggcca tcgccctgat agacggtttt tcgccctttg acgttggagt ccacgttctt 2280

taatagtgga ctcttgttcc aaactggaac aacactcaac cctatctcgg tctattcttt 2340

tgatttataa gggattttgc cgatttcggc ctattggtta aaaaatgagc tgatttaaca 2400

aaaatttaac gcgaattaat tctgtggaat gtgtgtcagt tagggtgtgg aaagtcccca 2460

ggctccccag caggcagaag tatgcaaagc atgcatctca attagtcagc aaccaggtgt 2520

ggaaagtccc caggctcccc agcaggcaga agtatgcaaa gcatgcatct caattagtca 2580

gcaaccatag tcccgcccct aactccgccc atcccgcccc taactccgcc cagttccgcc 2640

cattctccgc cccatggctg actaattttt tttatttatg cagaggccga ggccgcctct 2700

gcctctgagc tattccagaa gtagtgagga ggcttttttg gaggcctagg cttttgcaaa 2760

aagctcccgg gagcttgtat atccattttc ggatctgatc aagagacagg atgaggatcg 2820

tttcgcatga ttgaacaaga tggattgcac gcaggttctc cggccgcttg ggtggagagg 2880

ctattcggct atgactgggc acaacagaca atcggctgct ctgatgccgc cgtgttccgg 2940

ctgtcagcgc aggggcgccc ggttcttttt gtcaagaccg acctgtccgg tgccctgaat 3000

gaactgcagg acgaggcagc gcggctatcg tggctggcca cgacgggcgt tccttgcgca 3060

gctgtgctcg acgttgtcac tgaagcggga agggactggc tgctattggg cgaagtgccg 3120

gggcaggatc tcctgtcatc tcaccttgct cctgccgaga aagtatccat catggctgat 3180

gcaatgcggc ggctgcatac gcttgatccg gctacctgcc cattcgacca ccaagcgaaa 3240

catcgcatcg agcgagcacg tactcggatg gaagccggtc ttgtcgatca ggatgatctg 3300

gacgaagagc atcaggggct cgcgccagcc gaactgttcg ccaggctcaa ggcgcgcatg 3360

cccgacggcg aggatctcgt cgtgacccat ggcgatgcct gcttgccgaa tatcatggtg 3420

gaaaatggcc gcttttctgg attcatcgac tgtggccggc tgggtgtggc ggaccgctat 3480

caggacatag cgttggctac ccgtgatatt gctgaagagc ttggcggcga atgggctgac 3540

cgcttcctcg tgctttacgg tatcgccgct cccgattcgc agcgcatcgc cttctatcgc 3600

cttcttgacg agttcttctg agcgggactc tggggttcga aatgaccgac caagcgacgc 3660

ccaacctgcc atcacgagat ttcgattcca ccgccgcctt ctatgaaagg ttgggcttcg 3720

gaatcgtttt ccgggacgcc ggctggatga tcctccagcg cggggatctc atgctggagt 3780

tcttcgccca ccccaacttg tttattgcag cttataatgg ttacaaataa agcaatagca 3840

tcacaaattt cacaaataaa gcattttttt cactgcattc tagttgtggt ttgtccaaac 3900

tcatcaatgt atcttatcat gtctgtatac cgtcgacctc tagctagagc ttggcgtaat 3960

catggtcata gctgtttcct gtgtgaaatt gttatccgct cacaattcca cacaacatac 4020

gagccggaag cataaagtgt aaagcctggg gtgcctaatg agtgagctaa ctcacattaa 4080

ttgcgttgcg ctcactgccc gctttccagt cgggaaacct gtcgtgccag ctgcattaat 4140

gaatcggcca acgcgcgggg agaggcggtt tgcgtattgg gcgctcttcc gcttcctcgc 4200

tcactgactc gctgcgctcg gtcgttcggc tgcggcgagc ggtatcagct cactcaaagg 4260

cggtaatacg gttatccaca gaatcagggg ataacgcagg aaagaacatg tgagcaaaag 4320

gccagcaaaa ggccaggaac cgtaaaaagg ccgcgttgct ggcgtttttc cataggctcc 4380

gcccccctga cgagcatcac aaaaatcgac gctcaagtca gaggtggcga aacccgacag 4440

gactataaag ataccaggcg tttccccctg gaagctccct cgtgcgctct cctgttccga 4500

ccctgccgct taccggatac ctgtccgcct ttctcccttc gggaagcgtg gcgctttctc 4560

atagctcacg ctgtaggtat ctcagttcgg tgtaggtcgt tcgctccaag ctgggctgtg 4620

tgcacgaacc ccccgttcag cccgaccgct gcgccttatc cggtaactat cgtcttgagt 4680

ccaacccggt aagacacgac ttatcgccac tggcagcagc cactggtaac aggattagca 4740

gagcgaggta tgtaggcggt gctacagagt tcttgaagtg gtggcctaac tacggctaca 4800

ctagaagaac agtatttggt atctgcgctc tgctgaagcc agttaccttc ggaaaaagag 4860

ttggtagctc ttgatccggc aaacaaacca ccgctggtag cggttttttt gtttgcaagc 4920

agcagattac gcgcagaaaa aaaggatctc aagaagatcc tttgatcttt tctacggggt 4980

ctgacgctca gtggaacgaa aactcacgtt aagggatttt ggtcatgaga ttatcaaaaa 5040

ggatcttcac ctagatcctt ttaaattaaa aatgaagttt taaatcaatc taaagtatat 5100

atgagtaaac ttggtctgac agttaccaat gcttaatcag tgaggcacct atctcagcga 5160

tctgtctatt tcgttcatcc atagttgcct gactccccgt cgtgtagata actacgatac 5220

gggagggctt accatctggc cccagtgctg caatgatacc gcgagaccca cgctcaccgg 5280

ctccagattt atcagcaata aaccagccag ccggaagggc cgagcgcaga agtggtcctg 5340

caactttatc cgcctccatc cagtctatta attgttgccg ggaagctaga gtaagtagtt 5400

cgccagttaa tagtttgcgc aacgttgttg ccattgctac aggcatcgtg gtgtcacgct 5460

cgtcgtttgg tatggcttca ttcagctccg gttcccaacg atcaaggcga gttacatgat 5520

cccccatgtt gtgcaaaaaa gcggttagct ccttcggtcc tccgatcgtt gtcagaagta 5580

agttggccgc agtgttatca ctcatggtta tggcagcact gcataattct cttactgtca 5640

tgccatccgt aagatgcttt tctgtgactg gtgagtactc aaccaagtca ttctgagaat 5700

agtgtatgcg gcgaccgagt tgctcttgcc cggcgtcaat acgggataat accgcgccac 5760

atagcagaac tttaaaagtg ctcatcattg gaaaacgttc ttcggggcga aaactctcaa 5820

ggatcttacc gctgttgaga tccagttcga tgtaacccac tcgtgcaccc aactgatctt 5880

cagcatcttt tactttcacc agcgtttctg ggtgagcaaa aacaggaagg caaaatgccg 5940

caaaaaaggg aataagggcg acacggaaat gttgaatact catactcttc ctttttcaat 6000

attattgaag catttatcag ggttattgtc tcatgagcgg atacatattt gaatgtattt 6060

agaaaaataa acaaataggg gttccgcgca catttccccg aaaagtgcca cctgacgtc 6119

<210> 2
<211> 6011
<212> DNA
<213> Artificial Sequence

<220>
<223> Description of Artificial Sequence: Synthetic

polynucleotide

<400> 2
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagc 900

gtttaaactt aagcttggta ccgagctcgg atccatgtgg actctcgggc gccgcgcagt 960

agccggcctc ctggcgtcac ccagcccagc ccaggcccag accctcaccc gggtcccgcg 1020

gccggcagag ttggccccac tctgcggccg ccgtggcctg cgcaccgaca tcgatgcgac 1080

ctgcacgccc cgccgcgcaa gttcgaacca acgtggcctc aaccagattt ggaatgtcaa 1140

aaagcagagt gtctatttga tgaatttgag gaaatctgga actttgggcc acccaggctc 1200

tctagatgag accacctatg aaagactagc agaggaaacg ctggactctt tagcagagtt 1260

ttttgaagac cttgcagaca agccatacac gtttgaggac tatgatgtct cctttgggag 1320

tggtgtctta actgtcaaac tgggtggaga tctaggaacc tatgtgatca acaagcagac 1380

gccaaacaag caaatctggc tatcttctcc atccagtgga cctaagcgtt atgactggac 1440

tgggaaaaac tgggtgtact cccacgacgg cgtgtccctc catgagctgc tggccgcaga 1500

gctcactaaa gccttaaaaa ccaaactgga cttgtcttcc ttggcctatt ccggaaaaga 1560

tgcttgactc gagtctagag ggcccgttta aacccgctga tcagcctcga ctgtgccttc 1620

tagttgccag ccatctgttg tttgcccctc ccccgtgcct tccttgaccc tggaaggtgc 1680

cactcccact gtcctttcct aataaaatga ggaaattgca tcgcattgtc tgagtaggtg 1740

tcattctatt ctggggggtg gggtggggca ggacagcaag ggggaggatt gggaagacaa 1800

tagcaggcat gctggggatg cggtgggctc tatggcttct gaggcggaaa gaaccagctg 1860

gggctctagg gggtatcccc acgcgccctg tagcggcgca ttaagcgcgg cgggtgtggt 1920

ggttacgcgc agcgtgaccg ctacacttgc cagcgcccta gcgcccgctc ctttcgcttt 1980

cttcccttcc tttctcgcca cgttcgccgg ctttccccgt caagctctaa atcgggggct 2040

ccctttaggg ttccgattta gtgctttacg gcacctcgac cccaaaaaac ttgattaggg 2100

tgatggttca cgtagtgggc catcgccctg atagacggtt tttcgccctt tgacgttgga 2160

gtccacgttc tttaatagtg gactcttgtt ccaaactgga acaacactca accctatctc 2220

ggtctattct tttgatttat aagggatttt gccgatttcg gcctattggt taaaaaatga 2280

gctgatttaa caaaaattta acgcgaatta attctgtgga atgtgtgtca gttagggtgt 2340

ggaaagtccc caggctcccc agcaggcaga agtatgcaaa gcatgcatct caattagtca 2400

gcaaccaggt gtggaaagtc cccaggctcc ccagcaggca gaagtatgca aagcatgcat 2460

ctcaattagt cagcaaccat agtcccgccc ctaactccgc ccatcccgcc cctaactccg 2520

cccagttccg cccattctcc gccccatggc tgactaattt tttttattta tgcagaggcc 2580

gaggccgcct ctgcctctga gctattccag aagtagtgag gaggcttttt tggaggccta 2640

ggcttttgca aaaagctccc gggagcttgt atatccattt tcggatctga tcaagagaca 2700

ggatgaggat cgtttcgcat gattgaacaa gatggattgc acgcaggttc tccggccgct 2760

tgggtggaga ggctattcgg ctatgactgg gcacaacaga caatcggctg ctctgatgcc 2820

gccgtgttcc ggctgtcagc gcaggggcgc ccggttcttt ttgtcaagac cgacctgtcc 2880

ggtgccctga atgaactgca ggacgaggca gcgcggctat cgtggctggc cacgacgggc 2940

gttccttgcg cagctgtgct cgacgttgtc actgaagcgg gaagggactg gctgctattg 3000

ggcgaagtgc cggggcagga tctcctgtca tctcaccttg ctcctgccga gaaagtatcc 3060

atcatggctg atgcaatgcg gcggctgcat acgcttgatc cggctacctg cccattcgac 3120

caccaagcga aacatcgcat cgagcgagca cgtactcgga tggaagccgg tcttgtcgat 3180

caggatgatc tggacgaaga gcatcagggg ctcgcgccag ccgaactgtt cgccaggctc 3240

aaggcgcgca tgcccgacgg cgaggatctc gtcgtgaccc atggcgatgc ctgcttgccg 3300

aatatcatgg tggaaaatgg ccgcttttct ggattcatcg actgtggccg gctgggtgtg 3360

gcggaccgct atcaggacat agcgttggct acccgtgata ttgctgaaga gcttggcggc 3420

gaatgggctg accgcttcct cgtgctttac ggtatcgccg ctcccgattc gcagcgcatc 3480

gccttctatc gccttcttga cgagttcttc tgagcgggac tctggggttc gaaatgaccg 3540

accaagcgac gcccaacctg ccatcacgag atttcgattc caccgccgcc ttctatgaaa 3600

ggttgggctt cggaatcgtt ttccgggacg ccggctggat gatcctccag cgcggggatc 3660

tcatgctgga gttcttcgcc caccccaact tgtttattgc agcttataat ggttacaaat 3720

aaagcaatag catcacaaat ttcacaaata aagcattttt ttcactgcat tctagttgtg 3780

gtttgtccaa actcatcaat gtatcttatc atgtctgtat accgtcgacc tctagctaga 3840

gcttggcgta atcatggtca tagctgtttc ctgtgtgaaa ttgttatccg ctcacaattc 3900

cacacaacat acgagccgga agcataaagt gtaaagcctg gggtgcctaa tgagtgagct 3960

aactcacatt aattgcgttg cgctcactgc ccgctttcca gtcgggaaac ctgtcgtgcc 4020

agctgcatta atgaatcggc caacgcgcgg ggagaggcgg tttgcgtatt gggcgctctt 4080

ccgcttcctc gctcactgac tcgctgcgct cggtcgttcg gctgcggcga gcggtatcag 4140

ctcactcaaa ggcggtaata cggttatcca cagaatcagg ggataacgca ggaaagaaca 4200

tgtgagcaaa aggccagcaa aaggccagga accgtaaaaa ggccgcgttg ctggcgtttt 4260

tccataggct ccgcccccct gacgagcatc acaaaaatcg acgctcaagt cagaggtggc 4320

gaaacccgac aggactataa agataccagg cgtttccccc tggaagctcc ctcgtgcgct 4380

ctcctgttcc gaccctgccg cttaccggat acctgtccgc ctttctccct tcgggaagcg 4440

tggcgctttc tcatagctca cgctgtaggt atctcagttc ggtgtaggtc gttcgctcca 4500

agctgggctg tgtgcacgaa ccccccgttc agcccgaccg ctgcgcctta tccggtaact 4560

atcgtcttga gtccaacccg gtaagacacg acttatcgcc actggcagca gccactggta 4620

acaggattag cagagcgagg tatgtaggcg gtgctacaga gttcttgaag tggtggccta 4680

actacggcta cactagaaga acagtatttg gtatctgcgc tctgctgaag ccagttacct 4740

tcggaaaaag agttggtagc tcttgatccg gcaaacaaac caccgctggt agcggttttt 4800

ttgtttgcaa gcagcagatt acgcgcagaa aaaaaggatc tcaagaagat cctttgatct 4860

tttctacggg gtctgacgct cagtggaacg aaaactcacg ttaagggatt ttggtcatga 4920

gattatcaaa aaggatcttc acctagatcc ttttaaatta aaaatgaagt tttaaatcaa 4980

tctaaagtat atatgagtaa acttggtctg acagttacca atgcttaatc agtgaggcac 5040

ctatctcagc gatctgtcta tttcgttcat ccatagttgc ctgactcccc gtcgtgtaga 5100

taactacgat acgggagggc ttaccatctg gccccagtgc tgcaatgata ccgcgagacc 5160

cacgctcacc ggctccagat ttatcagcaa taaaccagcc agccggaagg gccgagcgca 5220

gaagtggtcc tgcaacttta tccgcctcca tccagtctat taattgttgc cgggaagcta 5280

gagtaagtag ttcgccagtt aatagtttgc gcaacgttgt tgccattgct acaggcatcg 5340

tggtgtcacg ctcgtcgttt ggtatggctt cattcagctc cggttcccaa cgatcaaggc 5400

gagttacatg atcccccatg ttgtgcaaaa aagcggttag ctccttcggt cctccgatcg 5460

ttgtcagaag taagttggcc gcagtgttat cactcatggt tatggcagca ctgcataatt 5520

ctcttactgt catgccatcc gtaagatgct tttctgtgac tggtgagtac tcaaccaagt 5580

cattctgaga atagtgtatg cggcgaccga gttgctcttg cccggcgtca atacgggata 5640

ataccgcgcc acatagcaga actttaaaag tgctcatcat tggaaaacgt tcttcggggc 5700

gaaaactctc aaggatctta ccgctgttga gatccagttc gatgtaaccc actcgtgcac 5760

ccaactgatc ttcagcatct tttactttca ccagcgtttc tgggtgagca aaaacaggaa 5820

ggcaaaatgc cgcaaaaaag ggaataaggg cgacacggaa atgttgaata ctcatactct 5880

tcctttttca atattattga agcatttatc agggttattg tctcatgagc ggatacatat 5940

ttgaatgtat ttagaaaaat aaacaaatag gggttccgcg cacatttccc cgaaaagtgc 6000

cacctgacgt c 6011

<210> 3
<211> 6083
<212> DNA
<213> Artificial Sequence

<220>
<223> Description of Artificial Sequence: Synthetic
      polynucleotide

<400> 3
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagc 900

gtttaaactt aagcttggta ccgagctcgg atccatgtgg actctcgggc gccgcgcagt 960

agccggcctc ctggcgtcac ccagcccagc ccaggcccag accctcaccc gggtcccgcg 1020

gccggcagag ttggccccac tctgcggccg ccgtggcctg cgcaccgaca tcgatgcgac 1080

ctgcacgccc cgccgcgcaa gttcgaacca acgtggcctc aaccagattt ggaatgtcaa 1140

aaagcagagt gtctatttga tgaatttgag gaaatctgga actttgggcc acccaggctc 1200

tctagatgag accacctatg aaagactagc agaggaaacg ctggactctt tagcagagtt 1260

ttttgaagac cttgcagaca agccatacac gtttgaggac tatgatgtct cctttgggag 1320

tggtgtctta actgtcaaac tgggtggaga tctaggaacc tatgtgatca acaagcagac 1380

gccaaacaag caaatctggc tatcttctcc atccagtgga cctaagcgtt atgactggac 1440

tgggaaaaac tgggtgtact cccacgacgg cgtgtccctc catgagctgc tggccgcaga 1500

gctcactaaa gccttaaaaa ccaaactgga cttgtcttcc ttggcctatt ccggaaaaga 1560

tgctggatcc gactacaaag accatgacgg tgattataaa gatcatgaca tcgactacaa 1620

ggatgacgat gacaagtagc tcgagtctag agggcccgtt taaacccgct gatcagcctc 1680

gactgtgcct tctagttgcc agccatctgt tgtttgcccc tcccccgtgc cttccttgac 1740

cctggaaggt gccactccca ctgtcctttc ctaataaaat gaggaaattg catcgcattg 1800

tctgagtagg tgtcattcta ttctgggggg tggggtgggg caggacagca agggggagga 1860

ttgggaagac aatagcaggc atgctgggga tgcggtgggc tctatggctt ctgaggcgga 1920

aagaaccagc tggggctcta gggggtatcc ccacgcgccc tgtagcggcg cattaagcgc 1980

ggcgggtgtg gtggttacgc gcagcgtgac cgctacactt gccagcgccc tagcgcccgc 2040

tcctttcgct ttcttccctt cctttctcgc cacgttcgcc ggctttcccc gtcaagctct 2100

aaatcggggg ctccctttag ggttccgatt tagtgcttta cggcacctcg accccaaaaa 2160

acttgattag ggtgatggtt cacgtagtgg gccatcgccc tgatagacgg tttttcgccc 2220

tttgacgttg gagtccacgt tctttaatag tggactcttg ttccaaactg gaacaacact 2280

caaccctatc tcggtctatt cttttgattt ataagggatt ttgccgattt cggcctattg 2340

gttaaaaaat gagctgattt aacaaaaatt taacgcgaat taattctgtg gaatgtgtgt 2400

cagttagggt gtggaaagtc cccaggctcc ccagcaggca gaagtatgca aagcatgcat 2460

ctcaattagt cagcaaccag gtgtggaaag tccccaggct ccccagcagg cagaagtatg 2520

caaagcatgc atctcaatta gtcagcaacc atagtcccgc ccctaactcc gcccatcccg 2580

cccctaactc cgcccagttc cgcccattct ccgccccatg gctgactaat tttttttatt 2640

tatgcagagg ccgaggccgc ctctgcctct gagctattcc agaagtagtg aggaggcttt 2700

tttggaggcc taggcttttg caaaaagctc ccgggagctt gtatatccat tttcggatct 2760

gatcaagaga caggatgagg atcgtttcgc atgattgaac aagatggatt gcacgcaggt 2820

tctccggccg cttgggtgga gaggctattc ggctatgact gggcacaaca gacaatcggc 2880

tgctctgatg ccgccgtgtt ccggctgtca gcgcaggggc gcccggttct ttttgtcaag 2940

accgacctgt ccggtgccct gaatgaactg caggacgagg cagcgcggct atcgtggctg 3000

gccacgacgg gcgttccttg cgcagctgtg ctcgacgttg tcactgaagc gggaagggac 3060

tggctgctat tgggcgaagt gccggggcag gatctcctgt catctcacct tgctcctgcc 3120

gagaaagtat ccatcatggc tgatgcaatg cggcggctgc atacgcttga tccggctacc 3180

tgcccattcg accaccaagc gaaacatcgc atcgagcgag cacgtactcg gatggaagcc 3240

ggtcttgtcg atcaggatga tctggacgaa gagcatcagg ggctcgcgcc agccgaactg 3300

ttcgccaggc tcaaggcgcg catgcccgac ggcgaggatc tcgtcgtgac ccatggcgat 3360

gcctgcttgc cgaatatcat ggtggaaaat ggccgctttt ctggattcat cgactgtggc 3420

cggctgggtg tggcggaccg ctatcaggac atagcgttgg ctacccgtga tattgctgaa 3480

gagcttggcg gcgaatgggc tgaccgcttc ctcgtgcttt acggtatcgc cgctcccgat 3540

tcgcagcgca tcgccttcta tcgccttctt gacgagttct tctgagcggg actctggggt 3600

tcgaaatgac cgaccaagcg acgcccaacc tgccatcacg agatttcgat tccaccgccg 3660

ccttctatga aaggttgggc ttcggaatcg ttttccggga cgccggctgg atgatcctcc 3720

agcgcgggga tctcatgctg gagttcttcg cccaccccaa cttgtttatt gcagcttata 3780

atggttacaa ataaagcaat agcatcacaa atttcacaaa taaagcattt ttttcactgc 3840

attctagttg tggtttgtcc aaactcatca atgtatctta tcatgtctgt ataccgtcga 3900

cctctagcta gagcttggcg taatcatggt catagctgtt tcctgtgtga aattgttatc 3960

cgctcacaat tccacacaac atacgagccg gaagcataaa gtgtaaagcc tggggtgcct 4020

aatgagtgag ctaactcaca ttaattgcgt tgcgctcact gcccgctttc cagtcgggaa 4080

acctgtcgtg ccagctgcat taatgaatcg gccaacgcgc ggggagaggc ggtttgcgta 4140

ttgggcgctc ttccgcttcc tcgctcactg actcgctgcg ctcggtcgtt cggctgcggc 4200

gagcggtatc agctcactca aaggcggtaa tacggttatc cacagaatca ggggataacg 4260

caggaaagaa catgtgagca aaaggccagc aaaaggccag gaaccgtaaa aaggccgcgt 4320

tgctggcgtt tttccatagg ctccgccccc ctgacgagca tcacaaaaat cgacgctcaa 4380

gtcagaggtg gcgaaacccg acaggactat aaagatacca ggcgtttccc cctggaagct 4440

ccctcgtgcg ctctcctgtt ccgaccctgc cgcttaccgg atacctgtcc gcctttctcc 4500

cttcgggaag cgtggcgctt tctcatagct cacgctgtag gtatctcagt tcggtgtagg 4560

tcgttcgctc caagctgggc tgtgtgcacg aaccccccgt tcagcccgac cgctgcgcct 4620

tatccggtaa ctatcgtctt gagtccaacc cggtaagaca cgacttatcg ccactggcag 4680

cagccactgg taacaggatt agcagagcga ggtatgtagg cggtgctaca gagttcttga 4740

agtggtggcc taactacggc tacactagaa gaacagtatt tggtatctgc gctctgctga 4800

agccagttac cttcggaaaa agagttggta gctcttgatc cggcaaacaa accaccgctg 4860

gtagcggttt ttttgtttgc aagcagcaga ttacgcgcag aaaaaaagga tctcaagaag 4920

atcctttgat cttttctacg gggtctgacg ctcagtggaa cgaaaactca cgttaaggga 4980

ttttggtcat gagattatca aaaaggatct tcacctagat ccttttaaat taaaaatgaa 5040

gttttaaatc aatctaaagt atatatgagt aaacttggtc tgacagttac caatgcttaa 5100

tcagtgaggc acctatctca gcgatctgtc tatttcgttc atccatagtt gcctgactcc 5160

ccgtcgtgta gataactacg atacgggagg gcttaccatc tggccccagt gctgcaatga 5220

taccgcgaga cccacgctca ccggctccag atttatcagc aataaaccag ccagccggaa 5280

gggccgagcg cagaagtggt cctgcaactt tatccgcctc catccagtct attaattgtt 5340

gccgggaagc tagagtaagt agttcgccag ttaatagttt gcgcaacgtt gttgccattg 5400

ctacaggcat cgtggtgtca cgctcgtcgt ttggtatggc ttcattcagc tccggttccc 5460

aacgatcaag gcgagttaca tgatccccca tgttgtgcaa aaaagcggtt agctccttcg 5520

gtcctccgat cgttgtcaga agtaagttgg ccgcagtgtt atcactcatg gttatggcag 5580

cactgcataa ttctcttact gtcatgccat ccgtaagatg cttttctgtg actggtgagt 5640

actcaaccaa gtcattctga gaatagtgta tgcggcgacc gagttgctct tgcccggcgt 5700

caatacggga taataccgcg ccacatagca gaactttaaa agtgctcatc attggaaaac 5760

gttcttcggg gcgaaaactc tcaaggatct taccgctgtt gagatccagt tcgatgtaac 5820

ccactcgtgc acccaactga tcttcagcat cttttacttt caccagcgtt tctgggtgag 5880

caaaaacagg aaggcaaaat gccgcaaaaa agggaataag ggcgacacgg aaatgttgaa 5940

tactcatact cttccttttt caatattatt gaagcattta tcagggttat tgtctcatga 6000

gcggatacat atttgaatgt atttagaaaa ataaacaaat aggggttccg cgcacatttc 6060

cccgaaaagt gccacctgac gtc 6083

<210> 4
<211> 6119
<212> DNA
<213> Artificial Sequence

<220>
<223> Description of Artificial Sequence: Synthetic
      polynucleotide

<400> 4
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagc 900

gtttaaactt aagcttggta ccgagctcgg atccatgtgg actctcgggc gccgcgcagt 960

agccggcctc ctggcgtcac ccagcccagc ccaggcccag accctcaccc gggtcccgcg 1020

gccggcagag ttggccccac tctgcggccg ccgtggcctg cgcaccgaca tcgatgcgac 1080

ctgcacgccc cgccgcgcaa gttcgaacca acgtggcctc aaccagattt ggaatgtcaa 1140

aaagcagagt gtctatttga tgaatttgag gaaatctgga actttgggcc acccaggctc 1200

tctagatgag accacctatg aaagactagc agaggaaacg ctggactctt tagcagagtt 1260

ttttgaagac cttgcagaca agccatacac gtttgaggac tatgatgtct cctttgggag 1320

tggtgtctta actgtcaaac tgggtggaga tctaggaacc tatgtgatca acaagcagac 1380

gccaaacaag caaatctggc tatcttctcc atccagtgga cctaagcgtt atgactggac 1440

tgggaaaaac tgggtgtact cccacgacgg cgtgtccctc catgagctgc tggccgcaga 1500

gctcactaaa gccttaaaaa ccaaactgga cttgtcttcc ttggcctatt ccggaaaaga 1560

tgctggatcc gaacaaaaac tcatctcaga agaggatctg ggatccgact acaaagacca 1620

tgacggtgat tataaagatc atgacatcga ctacaaggat gacgatgaca agtagctcga 1680

gtctagaggg cccgtttaaa cccgctgatc agcctcgact gtgccttcta gttgccagcc 1740

atctgttgtt tgcccctccc ccgtgccttc cttgaccctg gaaggtgcca ctcccactgt 1800

cctttcctaa taaaatgagg aaattgcatc gcattgtctg agtaggtgtc attctattct 1860

ggggggtggg gtggggcagg acagcaaggg ggaggattgg gaagacaata gcaggcatgc 1920

tggggatgcg gtgggctcta tggcttctga ggcggaaaga accagctggg gctctagggg 1980

gtatccccac gcgccctgta gcggcgcatt aagcgcggcg ggtgtggtgg ttacgcgcag 2040

cgtgaccgct acacttgcca gcgccctagc gcccgctcct ttcgctttct tcccttcctt 2100

tctcgccacg ttcgccggct ttccccgtca agctctaaat cgggggctcc ctttagggtt 2160

ccgatttagt gctttacggc acctcgaccc caaaaaactt gattagggtg atggttcacg 2220

tagtgggcca tcgccctgat agacggtttt tcgccctttg acgttggagt ccacgttctt 2280

taatagtgga ctcttgttcc aaactggaac aacactcaac cctatctcgg tctattcttt 2340

tgatttataa gggattttgc cgatttcggc ctattggtta aaaaatgagc tgatttaaca 2400

aaaatttaac gcgaattaat tctgtggaat gtgtgtcagt tagggtgtgg aaagtcccca 2460

ggctccccag caggcagaag tatgcaaagc atgcatctca attagtcagc aaccaggtgt 2520

ggaaagtccc caggctcccc agcaggcaga agtatgcaaa gcatgcatct caattagtca 2580

gcaaccatag tcccgcccct aactccgccc atcccgcccc taactccgcc cagttccgcc 2640

cattctccgc cccatggctg actaattttt tttatttatg cagaggccga ggccgcctct 2700

gcctctgagc tattccagaa gtagtgagga ggcttttttg gaggcctagg cttttgcaaa 2760

aagctcccgg gagcttgtat atccattttc ggatctgatc aagagacagg atgaggatcg 2820

tttcgcatga ttgaacaaga tggattgcac gcaggttctc cggccgcttg ggtggagagg 2880

ctattcggct atgactgggc acaacagaca atcggctgct ctgatgccgc cgtgttccgg 2940

ctgtcagcgc aggggcgccc ggttcttttt gtcaagaccg acctgtccgg tgccctgaat 3000

gaactgcagg acgaggcagc gcggctatcg tggctggcca cgacgggcgt tccttgcgca 3060

gctgtgctcg acgttgtcac tgaagcggga agggactggc tgctattggg cgaagtgccg 3120

gggcaggatc tcctgtcatc tcaccttgct cctgccgaga aagtatccat catggctgat 3180

gcaatgcggc ggctgcatac gcttgatccg gctacctgcc cattcgacca ccaagcgaaa 3240

catcgcatcg agcgagcacg tactcggatg gaagccggtc ttgtcgatca ggatgatctg 3300

gacgaagagc atcaggggct cgcgccagcc gaactgttcg ccaggctcaa ggcgcgcatg 3360

cccgacggcg aggatctcgt cgtgacccat ggcgatgcct gcttgccgaa tatcatggtg 3420

gaaaatggcc gcttttctgg attcatcgac tgtggccggc tgggtgtggc ggaccgctat 3480

caggacatag cgttggctac ccgtgatatt gctgaagagc ttggcggcga atgggctgac 3540

cgcttcctcg tgctttacgg tatcgccgct cccgattcgc agcgcatcgc cttctatcgc 3600

cttcttgacg agttcttctg agcgggactc tggggttcga aatgaccgac caagcgacgc 3660

ccaacctgcc atcacgagat ttcgattcca ccgccgcctt ctatgaaagg ttgggcttcg 3720

gaatcgtttt ccgggacgcc ggctggatga tcctccagcg cggggatctc atgctggagt 3780

tcttcgccca ccccaacttg tttattgcag cttataatgg ttacaaataa agcaatagca 3840

tcacaaattt cacaaataaa gcattttttt cactgcattc tagttgtggt ttgtccaaac 3900

tcatcaatgt atcttatcat gtctgtatac cgtcgacctc tagctagagc ttggcgtaat 3960

catggtcata gctgtttcct gtgtgaaatt gttatccgct cacaattcca cacaacatac 4020

gagccggaag cataaagtgt aaagcctggg gtgcctaatg agtgagctaa ctcacattaa 4080

ttgcgttgcg ctcactgccc gctttccagt cgggaaacct gtcgtgccag ctgcattaat 4140

gaatcggcca acgcgcgggg agaggcggtt tgcgtattgg gcgctcttcc gcttcctcgc 4200

tcactgactc gctgcgctcg gtcgttcggc tgcggcgagc ggtatcagct cactcaaagg 4260

cggtaatacg gttatccaca gaatcagggg ataacgcagg aaagaacatg tgagcaaaag 4320

gccagcaaaa ggccaggaac cgtaaaaagg ccgcgttgct ggcgtttttc cataggctcc 4380

gcccccctga cgagcatcac aaaaatcgac gctcaagtca gaggtggcga aacccgacag 4440

gactataaag ataccaggcg tttccccctg gaagctccct cgtgcgctct cctgttccga 4500

ccctgccgct taccggatac ctgtccgcct ttctcccttc gggaagcgtg gcgctttctc 4560

atagctcacg ctgtaggtat ctcagttcgg tgtaggtcgt tcgctccaag ctgggctgtg 4620

tgcacgaacc ccccgttcag cccgaccgct gcgccttatc cggtaactat cgtcttgagt 4680

ccaacccggt aagacacgac ttatcgccac tggcagcagc cactggtaac aggattagca 4740

gagcgaggta tgtaggcggt gctacagagt tcttgaagtg gtggcctaac tacggctaca 4800

ctagaagaac agtatttggt atctgcgctc tgctgaagcc agttaccttc ggaaaaagag 4860

ttggtagctc ttgatccggc aaacaaacca ccgctggtag cggttttttt gtttgcaagc 4920

agcagattac gcgcagaaaa aaaggatctc aagaagatcc tttgatcttt tctacggggt 4980

ctgacgctca gtggaacgaa aactcacgtt aagggatttt ggtcatgaga ttatcaaaaa 5040

ggatcttcac ctagatcctt ttaaattaaa aatgaagttt taaatcaatc taaagtatat 5100

atgagtaaac ttggtctgac agttaccaat gcttaatcag tgaggcacct atctcagcga 5160

tctgtctatt tcgttcatcc atagttgcct gactccccgt cgtgtagata actacgatac 5220

gggagggctt accatctggc cccagtgctg caatgatacc gcgagaccca cgctcaccgg 5280

ctccagattt atcagcaata aaccagccag ccggaagggc cgagcgcaga agtggtcctg 5340

caactttatc cgcctccatc cagtctatta attgttgccg ggaagctaga gtaagtagtt 5400

cgccagttaa tagtttgcgc aacgttgttg ccattgctac aggcatcgtg gtgtcacgct 5460

cgtcgtttgg tatggcttca ttcagctccg gttcccaacg atcaaggcga gttacatgat 5520

cccccatgtt gtgcaaaaaa gcggttagct ccttcggtcc tccgatcgtt gtcagaagta 5580

agttggccgc agtgttatca ctcatggtta tggcagcact gcataattct cttactgtca 5640

tgccatccgt aagatgcttt tctgtgactg gtgagtactc aaccaagtca ttctgagaat 5700

agtgtatgcg gcgaccgagt tgctcttgcc cggcgtcaat acgggataat accgcgccac 5760

atagcagaac tttaaaagtg ctcatcattg gaaaacgttc ttcggggcga aaactctcaa 5820

ggatcttacc gctgttgaga tccagttcga tgtaacccac tcgtgcaccc aactgatctt 5880

cagcatcttt tactttcacc agcgtttctg ggtgagcaaa aacaggaagg caaaatgccg 5940

caaaaaaggg aataagggcg acacggaaat gttgaatact catactcttc ctttttcaat 6000

attattgaag catttatcag ggttattgtc tcatgagcgg atacatattt gaatgtattt 6060

agaaaaataa acaaataggg gttccgcgca catttccccg aaaagtgcca cctgacgtc 6119

<210> 5
<211> 6041
<212> DNA
<213> Artificial Sequence

<220>
<223> Description of Artificial Sequence: Synthetic
      polynucleotide

<400> 5
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagc 900

gtttaaactt aagcttggta ccgagctcgg atccatgtgg actctcgggc gccgcgcagt 960

agccggcctc ctggcgtcac ccagcccagc ccaggcccag accctcaccc gggtcccgcg 1020

gccggcagag ttggccccac tctgcggccg ccgtggcctg cgcaccgaca tcgatgcgac 1080

ctgcacgccc cgccgcgcaa gttcgaacca acgtggcctc aaccagattt ggaatgtcaa 1140

aaagcagagt gtctatttga tgaatttgag gaaatctgga actttgggcc acccaggctc 1200

tctagatgag accacctatg aaagactagc agaggaaacg ctggactctt tagcagagtt 1260

ttttgaagac cttgcagaca agccatacac gtttgaggac tatgatgtct cctttgggag 1320

tggtgtctta actgtcaaac tgggtggaga tctaggaacc tatgtgatca acaagcagac 1380

gccaaacaag caaatctggc tatcttctcc atccagtgga cctaagcgtt atgactggac 1440

tgggaaaaac tgggtgtact cccacgacgg cgtgtccctc catgagctgc tggccgcaga 1500

gctcactaaa gccttaaaaa ccaaactgga cttgtcttcc ttggcctatt ccggaaaaga 1560

tgctgaacaa aaactcatct cagaagagga tctgtagctc gagtctagag ggcccgttta 1620

aacccgctga tcagcctcga ctgtgccttc tagttgccag ccatctgttg tttgcccctc 1680

ccccgtgcct tccttgaccc tggaaggtgc cactcccact gtcctttcct aataaaatga 1740

ggaaattgca tcgcattgtc tgagtaggtg tcattctatt ctggggggtg gggtggggca 1800

ggacagcaag ggggaggatt gggaagacaa tagcaggcat gctggggatg cggtgggctc 1860

tatggcttct gaggcggaaa gaaccagctg gggctctagg gggtatcccc acgcgccctg 1920

tagcggcgca ttaagcgcgg cgggtgtggt ggttacgcgc agcgtgaccg ctacacttgc 1980

cagcgcccta gcgcccgctc ctttcgcttt cttcccttcc tttctcgcca cgttcgccgg 2040

ctttccccgt caagctctaa atcgggggct ccctttaggg ttccgattta gtgctttacg 2100

gcacctcgac cccaaaaaac ttgattaggg tgatggttca cgtagtgggc catcgccctg 2160

atagacggtt tttcgccctt tgacgttgga gtccacgttc tttaatagtg gactcttgtt 2220

ccaaactgga acaacactca accctatctc ggtctattct tttgatttat aagggatttt 2280

gccgatttcg gcctattggt taaaaaatga gctgatttaa caaaaattta acgcgaatta 2340

attctgtgga atgtgtgtca gttagggtgt ggaaagtccc caggctcccc agcaggcaga 2400

agtatgcaaa gcatgcatct caattagtca gcaaccaggt gtggaaagtc cccaggctcc 2460

ccagcaggca gaagtatgca aagcatgcat ctcaattagt cagcaaccat agtcccgccc 2520

ctaactccgc ccatcccgcc cctaactccg cccagttccg cccattctcc gccccatggc 2580

tgactaattt tttttattta tgcagaggcc gaggccgcct ctgcctctga gctattccag 2640

aagtagtgag gaggcttttt tggaggccta ggcttttgca aaaagctccc gggagcttgt 2700

atatccattt tcggatctga tcaagagaca ggatgaggat cgtttcgcat gattgaacaa 2760

gatggattgc acgcaggttc tccggccgct tgggtggaga ggctattcgg ctatgactgg 2820

gcacaacaga caatcggctg ctctgatgcc gccgtgttcc ggctgtcagc gcaggggcgc 2880

ccggttcttt ttgtcaagac cgacctgtcc ggtgccctga atgaactgca ggacgaggca 2940

gcgcggctat cgtggctggc cacgacgggc gttccttgcg cagctgtgct cgacgttgtc 3000

actgaagcgg gaagggactg gctgctattg ggcgaagtgc cggggcagga tctcctgtca 3060

tctcaccttg ctcctgccga gaaagtatcc atcatggctg atgcaatgcg gcggctgcat 3120

acgcttgatc cggctacctg cccattcgac caccaagcga aacatcgcat cgagcgagca 3180

cgtactcgga tggaagccgg tcttgtcgat caggatgatc tggacgaaga gcatcagggg 3240

ctcgcgccag ccgaactgtt cgccaggctc aaggcgcgca tgcccgacgg cgaggatctc 3300

gtcgtgaccc atggcgatgc ctgcttgccg aatatcatgg tggaaaatgg ccgcttttct 3360

ggattcatcg actgtggccg gctgggtgtg gcggaccgct atcaggacat agcgttggct 3420

acccgtgata ttgctgaaga gcttggcggc gaatgggctg accgcttcct cgtgctttac 3480

ggtatcgccg ctcccgattc gcagcgcatc gccttctatc gccttcttga cgagttcttc 3540

tgagcgggac tctggggttc gaaatgaccg accaagcgac gcccaacctg ccatcacgag 3600

atttcgattc caccgccgcc ttctatgaaa ggttgggctt cggaatcgtt ttccgggacg 3660

ccggctggat gatcctccag cgcggggatc tcatgctgga gttcttcgcc caccccaact 3720

tgtttattgc agcttataat ggttacaaat aaagcaatag catcacaaat ttcacaaata 3780

aagcattttt ttcactgcat tctagttgtg gtttgtccaa actcatcaat gtatcttatc 3840

atgtctgtat accgtcgacc tctagctaga gcttggcgta atcatggtca tagctgtttc 3900

ctgtgtgaaa ttgttatccg ctcacaattc cacacaacat acgagccgga agcataaagt 3960

gtaaagcctg gggtgcctaa tgagtgagct aactcacatt aattgcgttg cgctcactgc 4020

ccgctttcca gtcgggaaac ctgtcgtgcc agctgcatta atgaatcggc caacgcgcgg 4080

ggagaggcgg tttgcgtatt gggcgctctt ccgcttcctc gctcactgac tcgctgcgct 4140

cggtcgttcg gctgcggcga gcggtatcag ctcactcaaa ggcggtaata cggttatcca 4200

cagaatcagg ggataacgca ggaaagaaca tgtgagcaaa aggccagcaa aaggccagga 4260

accgtaaaaa ggccgcgttg ctggcgtttt tccataggct ccgcccccct gacgagcatc 4320

acaaaaatcg acgctcaagt cagaggtggc gaaacccgac aggactataa agataccagg 4380

cgtttccccc tggaagctcc ctcgtgcgct ctcctgttcc gaccctgccg cttaccggat 4440

acctgtccgc ctttctccct tcgggaagcg tggcgctttc tcatagctca cgctgtaggt 4500

atctcagttc ggtgtaggtc gttcgctcca agctgggctg tgtgcacgaa ccccccgttc 4560

agcccgaccg ctgcgcctta tccggtaact atcgtcttga gtccaacccg gtaagacacg 4620

acttatcgcc actggcagca gccactggta acaggattag cagagcgagg tatgtaggcg 4680

gtgctacaga gttcttgaag tggtggccta actacggcta cactagaaga acagtatttg 4740

gtatctgcgc tctgctgaag ccagttacct tcggaaaaag agttggtagc tcttgatccg 4800

gcaaacaaac caccgctggt agcggttttt ttgtttgcaa gcagcagatt acgcgcagaa 4860

aaaaaggatc tcaagaagat cctttgatct tttctacggg gtctgacgct cagtggaacg 4920

aaaactcacg ttaagggatt ttggtcatga gattatcaaa aaggatcttc acctagatcc 4980

ttttaaatta aaaatgaagt tttaaatcaa tctaaagtat atatgagtaa acttggtctg 5040

acagttacca atgcttaatc agtgaggcac ctatctcagc gatctgtcta tttcgttcat 5100

ccatagttgc ctgactcccc gtcgtgtaga taactacgat acgggagggc ttaccatctg 5160

gccccagtgc tgcaatgata ccgcgagacc cacgctcacc ggctccagat ttatcagcaa 5220

taaaccagcc agccggaagg gccgagcgca gaagtggtcc tgcaacttta tccgcctcca 5280

tccagtctat taattgttgc cgggaagcta gagtaagtag ttcgccagtt aatagtttgc 5340

gcaacgttgt tgccattgct acaggcatcg tggtgtcacg ctcgtcgttt ggtatggctt 5400

cattcagctc cggttcccaa cgatcaaggc gagttacatg atcccccatg ttgtgcaaaa 5460

aagcggttag ctccttcggt cctccgatcg ttgtcagaag taagttggcc gcagtgttat 5520

cactcatggt tatggcagca ctgcataatt ctcttactgt catgccatcc gtaagatgct 5580

tttctgtgac tggtgagtac tcaaccaagt cattctgaga atagtgtatg cggcgaccga 5640

gttgctcttg cccggcgtca atacgggata ataccgcgcc acatagcaga actttaaaag 5700

tgctcatcat tggaaaacgt tcttcggggc gaaaactctc aaggatctta ccgctgttga 5760

gatccagttc gatgtaaccc actcgtgcac ccaactgatc ttcagcatct tttactttca 5820

ccagcgtttc tgggtgagca aaaacaggaa ggcaaaatgc cgcaaaaaag ggaataaggg 5880

cgacacggaa atgttgaata ctcatactct tcctttttca atattattga agcatttatc 5940

agggttattg tctcatgagc ggatacatat ttgaatgtat ttagaaaaat aaacaaatag 6000

gggttccgcg cacatttccc cgaaaagtgc cacctgacgt c 6041

<210> 6
<211> 6053
<212> DNA
<213> Artificial Sequence

<220>
<223> Description of Artificial Sequence: Synthetic
      polynucleotide

<400> 6
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagc 900

gtttaaactt aagcttggta ccgagctcgg atccatgtgg actctcgggc gccgcgcagt 960

agccggcctc ctggcgtcac ccagcccagc ccaggcccag accctcaccc gggtcccgcg 1020

gccggcagag ttggccccac tctgcggccg ccgtggcctg cgcaccgaca tcgatgcgac 1080

ctgcacgccc cgccgcgcaa gttcgaacca acgtggcctc aaccagattt ggaatgtcaa 1140

aaagcagagt gtctatttga tgaatttgag gaaatctgga actttgggcc acccaggctc 1200

tctagatgag accacctatg aaagactagc agaggaaacg ctggactctt tagcagagtt 1260

ttttgaagac cttgcagaca agccatacac gtttgaggac tatgatgtct cctttgggag 1320

tggtgtctta actgtcaaac tgggtggaga tctaggaacc tatgtgatca acaagcagac 1380

gccaaacaag caaatctggc tatcttctcc atccagtgga cctaagcgtt atgactggac 1440

tgggaaaaac tgggtgtact cccacgacgg cgtgtccctc catgagctgc tggccgcaga 1500

gctcactaaa gccttaaaaa ccaaactgga cttgtcttcc ttggcctatt ccggaaaaga 1560

tgcttatcct tatgacgtgc ctgactatgc cagcctggga ggaccttgac tcgagtctag 1620

agggcccgtt taaacccgct gatcagcctc gactgtgcct tctagttgcc agccatctgt 1680

tgtttgcccc tcccccgtgc cttccttgac cctggaaggt gccactccca ctgtcctttc 1740

ctaataaaat gaggaaattg catcgcattg tctgagtagg tgtcattcta ttctgggggg 1800

tggggtgggg caggacagca agggggagga ttgggaagac aatagcaggc atgctgggga 1860

tgcggtgggc tctatggctt ctgaggcgga aagaaccagc tggggctcta gggggtatcc 1920

ccacgcgccc tgtagcggcg cattaagcgc ggcgggtgtg gtggttacgc gcagcgtgac 1980

cgctacactt gccagcgccc tagcgcccgc tcctttcgct ttcttccctt cctttctcgc 2040

cacgttcgcc ggctttcccc gtcaagctct aaatcggggg ctccctttag ggttccgatt 2100

tagtgcttta cggcacctcg accccaaaaa acttgattag ggtgatggtt cacgtagtgg 2160

gccatcgccc tgatagacgg tttttcgccc tttgacgttg gagtccacgt tctttaatag 2220

tggactcttg ttccaaactg gaacaacact caaccctatc tcggtctatt cttttgattt 2280

ataagggatt ttgccgattt cggcctattg gttaaaaaat gagctgattt aacaaaaatt 2340

taacgcgaat taattctgtg gaatgtgtgt cagttagggt gtggaaagtc cccaggctcc 2400

ccagcaggca gaagtatgca aagcatgcat ctcaattagt cagcaaccag gtgtggaaag 2460

tccccaggct ccccagcagg cagaagtatg caaagcatgc atctcaatta gtcagcaacc 2520

atagtcccgc ccctaactcc gcccatcccg cccctaactc cgcccagttc cgcccattct 2580

ccgccccatg gctgactaat tttttttatt tatgcagagg ccgaggccgc ctctgcctct 2640
gagctattcc agaagtagtg aggaggcttt tttggaggcc taggcttttg caaaaagctc 2700
ccgggagctt gtatatccat tttcggatct gatcaagaga caggatgagg atcgtttcgc 2760
atgattgaac aagatggatt gcacgcaggt tctccggccg cttgggtgga gaggctattc 2820
ggctatgact gggcacaaca gacaatcggc tgctctgatg ccgccgtgtt ccggctgtca 2880
gcgcaggggc gcccggttct ttttgtcaag accgacctgt ccggtgccct gaatgaactg 2940
caggacgagg cagcgcggct atcgtggctg gccacgacgg gcgttccttg cgcagctgtg 3000
ctcgacgttg tcactgaagc gggaagggac tggctgctat tgggcgaagt gccggggcag 3060
gatctcctgt catctcacct tgctcctgcc gagaaagtat ccatcatggc tgatgcaatg 3120
cggcggctgc atacgcttga tccggctacc tgcccattcg accaccaagc gaaacatcgc 3180
atcgagcgag cacgtactcg gatggaagcc ggtcttgtcg atcaggatga tctggacgaa 3240
gagcatcagg ggctcgcgcc agccgaactg ttcgccaggc tcaaggcgcg catgcccgac 3300
ggcgaggatc tcgtcgtgac ccatggcgat gcctgcttgc cgaatatcat ggtggaaaat 3360
ggccgctttt ctggattcat cgactgtggc cggctgggtg tggcggaccg ctatcaggac 3420
atagcgttgg ctacccgtga tattgctgaa gagcttggcg gcgaatgggc tgaccgcttc 3480
ctcgtgcttt acggtatcgc cgctcccgat tcgcagcgca tcgccttcta tcgccttctt 3540
gacgagttct tctgagcggg actctggggt tcgaaatgac cgaccaagcg acgcccaacc 3600
tgccatcacg agatttcgat tccaccgccg ccttctatga aaggttgggc ttcggaatcg 3660
ttttccggga cgccggctgg atgatcctcc agcgcgggga tctcatgctg gagttcttcg 3720
cccaccccaa cttgtttatt gcagcttata atggttacaa ataaagcaat agcatcacaa 3780
atttcacaaa taaagcattt ttttcactgc attctagttg tggtttgtcc aaactcatca 3840
atgtatctta tcatgtctgt ataccgtcga cctctagcta gagcttggcg taatcatggt 3900
catagctgtt tcctgtgtga aattgttatc cgctcacaat tccacacaac atacgagccg 3960
gaagcataaa gtgtaaagcc tggggtgcct aatgagtgag ctaactcaca ttaattgcgt 4020
tgcgctcact gcccgctttc cagtcgggaa acctgtcgtg ccagctgcat taatgaatcg 4080
gccaacgcgc ggggagaggc ggtttgcgta ttgggcgctc ttccgcttcc tcgctcactg 4140
actcgctgcg ctcggtcgtt cggctgcggc gagcggtatc agctcactca aaggcggtaa 4200
tacggttatc cacagaatca ggggataacg caggaaagaa catgtgagca aaaggccagc 4260
aaaaggccag gaaccgtaaa aaggccgcgt tgctggcgtt tttccatagg ctccgccccc 4320
ctgacgagca tcacaaaaat cgacgctcaa gtcagaggtg gcgaaacccg acaggactat 4380
aaagatacca ggcgtttccc cctggaagct ccctcgtgcg ctctcctgtt ccgaccctgc 4440
cgcttaccgg atacctgtcc gcctttctcc cttcgggaag cgtggcgctt tctcatagct 4500

cacgctgtag gtatctcagt tcggtgtagg tcgttcgctc caagctgggc tgtgtgcacg 4560

aaccccccgt tcagcccgac cgctgcgcct tatccggtaa ctatcgtctt gagtccaacc 4620

cggtaagaca cgacttatcg ccactggcag cagccactgg taacaggatt agcagagcga 4680

ggtatgtagg cggtgctaca gagttcttga agtggtggcc taactacggc tacactagaa 4740

gaacagtatt tggtatctgc gctctgctga agccagttac cttcggaaaa agagttggta 4800

gctcttgatc cggcaaacaa accaccgctg gtagcggttt ttttgtttgc aagcagcaga 4860

ttacgcgcag aaaaaaagga tctcaagaag atcctttgat cttttctacg gggtctgacg 4920

ctcagtggaa cgaaaactca cgttaaggga ttttggtcat gagattatca aaaaggatct 4980

tcacctagat ccttttaaat taaaaatgaa gttttaaatc aatctaaagt atatatgagt 5040

aaacttggtc tgacagttac caatgcttaa tcagtgaggc acctatctca gcgatctgtc 5100

tatttcgttc atccatagtt gcctgactcc ccgtcgtgta gataactacg atacgggagg 5160

gcttaccatc tggccccagt gctgcaatga taccgcgaga cccacgctca ccggctccag 5220

atttatcagc aataaaccag ccagccggaa gggccgagcg cagaagtggt cctgcaactt 5280

tatccgcctc catccagtct attaattgtt gccgggaagc tagagtaagt agttcgccag 5340

ttaatagttt gcgcaacgtt gttgccattg ctacaggcat cgtggtgtca cgctcgtcgt 5400

ttggtatggc ttcattcagc tccggttccc aacgatcaag gcgagttaca tgatccccca 5460

tgttgtgcaa aaaagcggtt agctccttcg gtcctccgat cgttgtcaga agtaagttgg 5520

ccgcagtgtt atcactcatg gttatggcag cactgcataa ttctcttact gtcatgccat 5580

ccgtaagatg cttttctgtg actggtgagt actcaaccaa gtcattctga gaatagtgta 5640

tgcggcgacc gagttgctct tgcccggcgt caatacggga taataccgcg ccacatagca 5700

gaactttaaa agtgctcatc attggaaaac gttcttcggg gcgaaaactc tcaaggatct 5760

taccgctgtt gagatccagt tcgatgtaac ccactcgtgc acccaactga tcttcagcat 5820

cttttacttt caccagcgtt tctgggtgag caaaaacagg aaggcaaaat gccgcaaaaa 5880

agggaataag ggcgacacgg aaatgttgaa tactcatact cttccttttt caatattatt 5940

gaagcattta tcagggttat tgtctcatga gcggatacat atttgaatgt atttagaaaa 6000

ataaacaaat aggggttccg cgcacatttc cccgaaaagt gccacctgac gtc 6053

<210> 7
<211> 6038
<212> DNA
<213> Artificial Sequence

<220>
<223> Description of Artificial Sequence: Synthetic
      polynucleotide

<400> 7
gacggatcgg gagatctccc gatcccctat ggtgcactct cagtacaatc tgctctgatg 60
ccgcatagtt aagccagtat ctgctccctg cttgtgtgtt ggaggtcgct gagtagtgcg 120
cgagcaaaat ttaagctaca acaaggcaag gcttgaccga caattgcatg aagaatctgc 180
ttagggttag gcgttttgcg ctgcttcgcg atgtacgggc cagatatacg cgttgacatt 240
gattattgac tagttattaa tagtaatcaa ttacggggtc attagttcat agcccatata 300
tggagttccg cgttacataa cttacggtaa atggcccgcc tggctgaccg cccaacgacc 360
cccgcccatt gacgtcaata atgacgtatg ttcccatagt aacgccaata gggactttcc 420
attgacgtca atgggtggag tatttacggt aaactgccca cttggcagta catcaagtgt 480
atcatatgcc aagtacgccc cctattgacg tcaatgacgg taaatggccc gcctggcatt 540
atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagagctct ctggctaact agagaaccca 840
ctgcttactg gcttatcgaa attaatacga ctcactatag ggagacccaa gctggctagc 900
gtttaaactt aagcttggta ccgagctcgg atccatgtgg actctcgggc gccgcgcagt 960
agccggcctc ctggcgtcac ccagcccagc ccaggcccag accctcaccc gggtcccgcg 1020
gccggcagag ttggccccac tctgcggccg ccgtggcctg cgcaccgaca tcgatgcgac 1080
ctgcacgccc cgccgcgcaa gttcgaacca acgtggcctc aaccagattt ggaatgtcaa 1140
aaagcagagt gtctatttga tgaatttgag gaaatctgga actttgggcc acccaggctc 1200
tctagatgag accacctatg aaagactagc agaggaaacg ctggactctt tagcagagtt 1260
ttttgaagac cttgcagaca agccatacac gtttgaggac tatgatgtct cctttgggag 1320
tggtgtctta actgtcaaac tgggtggaga tctaggaacc tatgtgatca acaagcagac 1380
gccaaacaag caaatctggc tatcttctcc atccagtgga cctaagcgtt atgactggac 1440
tgggaaaaac tgggtgtact cccacgacgg cgtgtccctc catgagctgc tggccgcaga 1500
gctcactaaa gccttaaaaa ccaaactgga cttgtcttcc ttggcctatt ccggaaaaga 1560
tgcttatcct tatgacgtgc ctgactatgc ctgactcgag tctagagggc ccgtttaaac 1620
ccgctgatca gcctcgactg tgccttctag ttgccagcca tctgttgttt gcccctcccc 1680
cgtgccttcc ttgaccctgg aaggtgccac tcccactgtc ctttcctaat aaaatgagga 1740
aattgcatcg cattgtctga gtaggtgtca ttctattctg gggggtgggg tggggcagga 1800
cagcaagggg gaggattggg aagacaatag caggcatgct ggggatgcgg tgggctctat 1860

ggcttctgag gcggaaagaa ccagctgggg ctctaggggg tatccccacg cgccctgtag 1920

cggcgcatta agcgcggcgg gtgtggtggt tacgcgcagc gtgaccgcta cacttgccag 1980

cgccctagcg cccgctcctt tcgctttctt cccttccttt ctcgccacgt tcgccggctt 2040

tccccgtcaa gctctaaatc gggggctccc tttagggttc cgatttagtg ctttacggca 2100

cctcgacccc aaaaaacttg attagggtga tggttcacgt agtgggccat cgccctgata 2160

gacggttttt cgccctttga cgttggagtc cacgttcttt aatagtggac tcttgttcca 2220

aactggaaca acactcaacc ctatctcggt ctattctttt gatttataag ggattttgcc 2280

gatttcggcc tattggttaa aaaatgagct gatttaacaa aaatttaacg cgaattaatt 2340

ctgtggaatg tgtgtcagtt agggtgtgga aagtccccag gctccccagc aggcagaagt 2400

atgcaaagca tgcatctcaa ttagtcagca accaggtgtg gaaagtcccc aggctcccca 2460

gcaggcagaa gtatgcaaag catgcatctc aattagtcag caaccatagt cccgccccta 2520

actccgccca tcccgcccct aactccgccc agttccgccc attctccgcc ccatggctga 2580

ctaatttttt ttatttatgc agaggccgag gccgcctctg cctctgagct attccagaag 2640

tagtgaggag gcttttttgg aggcctaggc ttttgcaaaa agctcccggg agcttgtata 2700

tccattttcg gatctgatca agagacagga tgaggatcgt ttcgcatgat tgaacaagat 2760

ggattgcacg caggttctcc ggccgcttgg gtggagaggc tattcggcta tgactgggca 2820

caacagacaa tcggctgctc tgatgccgcc gtgttccggc tgtcagcgca ggggcgcccg 2880

gttctttttg tcaagaccga cctgtccggt gccctgaatg aactgcagga cgaggcagcg 2940

cggctatcgt ggctggccac gacgggcgtt ccttgcgcag ctgtgctcga cgttgtcact 3000

gaagcgggaa gggactggct gctattgggc gaagtgccgg ggcaggatct cctgtcatct 3060

caccttgctc ctgccgagaa agtatccatc atggctgatg caatgcggcg gctgcatacg 3120

cttgatccgg ctacctgccc attcgaccac caagcgaaac atcgcatcga gcgagcacgt 3180

actcggatgg aagccggtct tgtcgatcag gatgatctgg acgaagagca tcaggggctc 3240

gcgccagccg aactgttcgc caggctcaag gcgcgcatgc ccgacggcga ggatctcgtc 3300

gtgacccatg gcgatgcctg cttgccgaat atcatggtgg aaaatggccg cttttctgga 3360

ttcatcgact gtggccggct gggtgtggcg gaccgctatc aggacatagc gttggctacc 3420

cgtgatattg ctgaagagct tggcggcgaa tgggctgacc gcttcctcgt gctttacggt 3480

atcgccgctc ccgattcgca gcgcatcgcc ttctatcgcc ttcttgacga gttcttctga 3540

gcgggactct ggggttcgaa atgaccgacc aagcgacgcc caacctgcca tcacgagatt 3600

tcgattccac cgccgccttc tatgaaaggt tgggcttcgg aatcgttttc cgggacgccg 3660

gctggatgat cctccagcgc ggggatctca tgctggagtt cttcgcccac cccaacttgt 3720

ttattgcagc ttataatggt tacaaataaa gcaatagcat cacaaatttc acaaataaag 3780

catttttttc actgcattct agttgtggtt tgtccaaact catcaatgta tcttatcatg 3840

tctgtatacc gtcgacctct agctagagct tggcgtaatc atggtcatag ctgtttcctg 3900

tgtgaaattg ttatccgctc acaattccac acaacatacg agccggaagc ataaagtgta 3960

aagcctgggg tgcctaatga gtgagctaac tcacattaat tgcgttgcgc tcactgcccg 4020

ctttccagtc gggaaacctg tcgtgccagc tgcattaatg aatcggccaa cgcgcgggga 4080

gaggcggttt gcgtattggg cgctcttccg cttcctcgct cactgactcg ctgcgctcgg 4140

tcgttcggct gcggcgagcg gtatcagctc actcaaaggc ggtaatacgg ttatccacag 4200

aatcagggga taacgcagga aagaacatgt gagcaaaagg ccagcaaaag gccaggaacc 4260

gtaaaaaggc cgcgttgctg gcgtttttcc ataggctccg cccccctgac gagcatcaca 4320

aaaatcgacg ctcaagtcag aggtggcgaa acccgacagg actataaaga taccaggcgt 4380

ttccccctgg aagctccctc gtgcgctctc ctgttccgac cctgccgctt accggatacc 4440

tgtccgcctt tctcccttcg ggaagcgtgg cgctttctca tagctcacgc tgtaggtatc 4500

tcagttcggt gtaggtcgtt cgctccaagc tgggctgtgt gcacgaaccc cccgttcagc 4560

ccgaccgctg cgccttatcc ggtaactatc gtcttgagtc caacccggta agacacgact 4620

tatcgccact ggcagcagcc actggtaaca ggattagcag agcgaggtat gtaggcggtg 4680

ctacagagtt cttgaagtgg tggcctaact acggctacac tagaagaaca gtatttggta 4740

tctgcgctct gctgaagcca gttaccttcg gaaaaagagt tggtagctct tgatccggca 4800

aacaaaccac cgctggtagc ggtttttttg tttgcaagca gcagattacg cgcagaaaaa 4860

aaggatctca agaagatcct ttgatctttt ctacggggtc tgacgctcag tggaacgaaa 4920

actcacgtta agggattttg gtcatgagat tatcaaaaag gatcttcacc tagatccttt 4980

taaattaaaa atgaagtttt aaatcaatct aaagtatata tgagtaaact tggtctgaca 5040

gttaccaatg cttaatcagt gaggcaccta tctcagcgat ctgtctattt cgttcatcca 5100

tagttgcctg actccccgtc gtgtagataa ctacgatacg ggagggctta ccatctggcc 5160

ccagtgctgc aatgataccg cgagacccac gctcaccggc tccagattta tcagcaataa 5220

accagccagc cggaagggcc gagcgcagaa gtggtcctgc aactttatcc gcctccatcc 5280

agtctattaa ttgttgccgg gaagctagag taagtagttc gccagttaat agtttgcgca 5340

acgttgttgc cattgctaca ggcatcgtgg tgtcacgctc gtcgtttggt atggcttcat 5400

tcagctccgg ttcccaacga tcaaggcgag ttacatgatc ccccatgttg tgcaaaaaag 5460

cggttagctc cttcggtcct ccgatcgttg tcagaagtaa gttggccgca gtgttatcac 5520

tcatggttat ggcagcactg cataattctc ttactgtcat gccatccgta agatgctttt 5580

ctgtgactgg tgagtactca accaagtcat tctgagaata gtgtatgcgg cgaccgagtt 5640

gctcttgccc ggcgtcaata cgggataata ccgcgccaca tagcagaact ttaaaagtgc 5700

tcatcattgg aaaacgttct tcggggcgaa aactctcaag gatcttaccg ctgttgagat 5760

ccagttcgat gtaacccact cgtgcaccca actgatcttc agcatctttt actttcacca 5820

gcgtttctgg gtgagcaaaa acaggaaggc aaaatgccgc aaaaaaggga ataagggcga 5880

cacggaaatg ttgaatactc atactcttcc tttttcaata ttattgaagc atttatcagg 5940

gttattgtct catgagcgga tacatatttg aatgtattta gaaaaataaa caaatagggg 6000

ttccgcgcac atttccccga aaagtgccac ctgacgtc 6038

<210> 8
<211> 5978
<212> DNA
<213> Artificial Sequence

<220>
<223> Description of Artificial Sequence: Synthetic
      polynucleotide

<400> 8
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagc 900

gtttaaactt aagcttggta ccgagctcgg atccgccgcc accatggctt tacttactgc 960

ggccgcccgg ctcttgggaa ccaagaatgc atcttgtctt gttcttgcag cccggcatgc 1020

tagtctgcgc accgacatcg atgcgacctg cacgccccgc cgcgcaagtt cgaaccaacg 1080

tggcctcaac cagatttgga atgtcaaaaa gcagagtgtc tatttgatga atttgaggaa 1140

atctggaact ttgggccacc caggctctct agatgagacc acctatgaaa gactagcaga 1200

ggaaacgctg gactctttag cagagttttt tgaagacctt gcagacaagc catacacgtt 1260

tgaggactat gatgtctcct ttgggagtgg tgtcttaact gtcaaactgg gtggagatct 1320

aggaacctat gtgatcaaca agcagacgcc aaacaagcaa atctggctat cttctccatc 1380

cagtggacct aagcgttatg actggactgg gaaaaactgg gtgtactccc acgacggcgt 1440

gtccctccat gagctgctgg ccgcagagct cactaaagcc ttaaaaacca aactggactt 1500

gtcttccttg gcctattccg gaaaagatgc ttgactcgag tctagagggc ccgtttaaac 1560

ccgctgatca gcctcgactg tgccttctag ttgccagcca tctgttgttt gcccctcccc 1620

cgtgccttcc ttgaccctgg aaggtgccac tcccactgtc ctttcctaat aaaatgagga 1680

aattgcatcg cattgtctga gtaggtgtca ttctattctg gggggtgggg tggggcagga 1740

cagcaagggg gaggattggg aagacaatag caggcatgct ggggatgcgg tgggctctat 1800

ggcttctgag gcggaaagaa ccagctgggg ctctaggggg tatccccacg cgccctgtag 1860

cggcgcatta agcgcggcgg gtgtggtggt tacgcgcagc gtgaccgcta cacttgccag 1920

cgccctagcg cccgctcctt tcgctttctt cccttccttt ctcgccacgt tcgccggctt 1980

tccccgtcaa gctctaaatc gggggctccc tttagggttc cgatttagtg ctttacggca 2040

cctcgacccc aaaaaacttg attagggtga tggttcacgt agtgggccat cgccctgata 2100

gacggttttt cgccctttga cgttggagtc cacgttcttt aatagtggac tcttgttcca 2160

aactggaaca acactcaacc ctatctcggt ctattctttt gatttataag ggattttgcc 2220

gatttcggcc tattggttaa aaaatgagct gatttaacaa aaatttaacg cgaattaatt 2280

ctgtggaatg tgtgtcagtt agggtgtgga aagtccccag gctccccagc aggcagaagt 2340

atgcaaagca tgcatctcaa ttagtcagca accaggtgtg gaaagtcccc aggctcccca 2400

gcaggcagaa gtatgcaaag catgcatctc aattagtcag caaccatagt cccgccccta 2460

actccgccca tcccgcccct aactccgccc agttccgccc attctccgcc ccatggctga 2520

ctaatttttt ttatttatgc agaggccgag gccgcctctg cctctgagct attccagaag 2580

tagtgaggag gcttttttgg aggcctaggc ttttgcaaaa agctcccggg agcttgtata 2640

tccattttcg gatctgatca agagacagga tgaggatcgt ttcgcatgat tgaacaagat 2700

ggattgcacg caggttctcc ggccgcttgg gtggagaggc tattcggcta tgactgggca 2760

caacagacaa tcggctgctc tgatgccgcc gtgttccggc tgtcagcgca ggggcgcccg 2820

gttctttttg tcaagaccga cctgtccggt gccctgaatg aactgcagga cgaggcagcg 2880

cggctatcgt ggctggccac gacgggcgtt ccttgcgcag ctgtgctcga cgttgtcact 2940

gaagcgggaa gggactggct gctattgggc gaagtgccgg ggcaggatct cctgtcatct 3000

caccttgctc ctgccgagaa agtatccatc atggctgatg caatgcggcg gctgcatacg 3060

cttgatccgg ctacctgccc attcgaccac caagcgaaac atcgcatcga gcgagcacgt 3120

actcggatgg aagccggtct tgtcgatcag gatgatctgg acgaagagca tcaggggctc 3180

gcgccagccg aactgttcgc caggctcaag gcgcgcatgc ccgacggcga ggatctcgtc 3240

gtgacccatg gcgatgcctg cttgccgaat atcatggtgg aaaatggccg cttttctgga 3300

ttcatcgact gtggccggct gggtgtggcg gaccgctatc aggacatagc gttggctacc 3360

cgtgatattg ctgaagagct tggcggcgaa tgggctgacc gcttcctcgt gctttacggt 3420

atcgccgctc ccgattcgca gcgcatcgcc ttctatcgcc ttcttgacga gttcttctga 3480

gcgggactct ggggttcgaa atgaccgacc aagcgacgcc caacctgcca tcacgagatt 3540

tcgattccac cgccgccttc tatgaaaggt tgggcttcgg aatcgttttc cgggacgccg 3600

gctggatgat cctccagcgc ggggatctca tgctggagtt cttcgcccac cccaacttgt 3660

ttattgcagc ttataatggt tacaaataaa gcaatagcat cacaaatttc acaaataaag 3720

catttttttc actgcattct agttgtggtt tgtccaaact catcaatgta tcttatcatg 3780

tctgtatacc gtcgacctct agctagagct tggcgtaatc atggtcatag ctgtttcctg 3840

tgtgaaattg ttatccgctc acaattccac acaacatacg agccggaagc ataaagtgta 3900

aagcctgggg tgcctaatga gtgagctaac tcacattaat tgcgttgcgc tcactgcccg 3960

ctttccagtc gggaaacctg tcgtgccagc tgcattaatg aatcggccaa cgcgcgggga 4020

gaggcggttt gcgtattggg cgctcttccg cttcctcgct cactgactcg ctgcgctcgg 4080

tcgttcggct gcggcgagcg gtatcagctc actcaaaggc ggtaatacgg ttatccacag 4140

aatcagggga taacgcagga aagaacatgt gagcaaaagg ccagcaaaag gccaggaacc 4200

gtaaaaaggc cgcgttgctg gcgtttttcc ataggctccg cccccctgac gagcatcaca 4260

aaaatcgacg ctcaagtcag aggtggcgaa acccgacagg actataaaga taccaggcgt 4320

ttccccctgg aagctccctc gtgcgctctc ctgttccgac cctgccgctt accggatacc 4380

tgtccgcctt tctcccttcg ggaagcgtgg cgctttctca tagctcacgc tgtaggtatc 4440

tcagttcggt gtaggtcgtt cgctccaagc tgggctgtgt gcacgaaccc cccgttcagc 4500

ccgaccgctg cgccttatcc ggtaactatc gtcttgagtc caacccggta agacacgact 4560

tatcgccact ggcagcagcc actggtaaca ggattagcag agcgaggtat gtaggcggtg 4620

ctacagagtt cttgaagtgg tggcctaact acggctacac tagaagaaca gtatttggta 4680

tctgcgctct gctgaagcca gttaccttcg gaaaaagagt tggtagctct tgatccggca 4740

aacaaaccac cgctggtagc ggtttttttg tttgcaagca gcagattacg cgcagaaaaa 4800

aaggatctca agaagatcct ttgatctttt ctacggggtc tgacgctcag tggaacgaaa 4860

actcacgtta agggattttg gtcatgagat tatcaaaaag gatcttcacc tagatccttt 4920

taaattaaaa atgaagtttt aaatcaatct aaagtatata tgagtaaact tggtctgaca 4980

gttaccaatg cttaatcagt gaggcaccta tctcagcgat ctgtctattt cgttcatcca 5040

tagttgcctg actccccgtc gtgtagataa ctacgatacg ggagggctta ccatctggcc 5100

ccagtgctgc aatgataccg cgagacccac gctcaccggc tccagattta tcagcaataa 5160

accagccagc cggaagggcc gagcgcagaa gtggtcctgc aactttatcc gcctccatcc 5220

agtctattaa ttgttgccgg gaagctagag taagtagttc gccagttaat agtttgcgca 5280

acgttgttgc cattgctaca ggcatcgtgg tgtcacgctc gtcgtttggt atggcttcat 5340

tcagctccgg ttcccaacga tcaaggcgag ttacatgatc ccccatgttg tgcaaaaaag 5400

cggttagctc cttcggtcct ccgatcgttg tcagaagtaa gttggccgca gtgttatcac 5460

tcatggttat ggcagcactg cataattctc ttactgtcat gccatccgta agatgctttt 5520

ctgtgactgg tgagtactca accaagtcat tctgagaata gtgtatgcgg cgaccgagtt 5580

gctcttgccc ggcgtcaata cgggataata ccgcgccaca tagcagaact ttaaaagtgc 5640

tcatcattgg aaaacgttct tcggggcgaa aactctcaag gatcttaccg ctgttgagat 5700

ccagttcgat gtaacccact cgtgcaccca actgatcttc agcatctttt actttcacca 5760

gcgtttctgg gtgagcaaaa acaggaaggc aaaatgccgc aaaaaaggga ataagggcga 5820

cacggaaatg ttgaatactc atactcttcc tttttcaata ttattgaagc atttatcagg 5880

gttattgtct catgagcgga tacatatttg aatgtattta gaaaaataaa caaatagggg 5940

ttccgcgcac atttccccga aaagtgccac ctgacgtc 5978

<210> 9
<211> 6020
<212> DNA
<213> Artificial Sequence

<220>
<223> Description of Artificial Sequence: Synthetic
      polynucleotide

<400> 9
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatgacgg taaatggccc gcctggcatt 540
atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagagctct ctggctaact agagaaccca 840
ctgcttactg gcttatcgaa attaatacga ctcactatag ggagacccaa gctggctagc 900
gtttaaactt aagcttggta ccgagctcgg atcctccacc ggtatgtgga ctctcgggcg 960
ccgcgcagta gccggcctcc tggcgtcacc cagcccagcc caggcccaga ccctcacccg 1020
ggtcccgcgg ccggcagagt tggccccact ctgcggccgc cgtggcctgc gcaccgacat 1080
cgatgcgacc tgcacgcccc gccgcgcaag ttcgaaccaa cgtggcctca accagatttg 1140
gaatgtcaaa aagcagagtg tctatttgat gaatttgagg aaatctggaa ctttgggcca 1200
cccaggctct ctagatgaga ccacctatga aagactagca gaggaaacgc tggactcttt 1260
agcagagttt tttgaagacc ttgcagacaa gccatacacg tttgaggact atgatgtctc 1320
ctttgggagt ggtgtcttaa ctgtcaaact gggtggagat ctaggaacct atgtgatcaa 1380
caagcagacg ccaaacaagc aaatctggct atcttctcca tccagtggac ctaagcgtta 1440
tgactggact gggaaaaact gggtgtactc ccacgacggc gtgtccctcc atgagctgct 1500
ggccgcagag ctcactaaag ccttaaaaac caaactggac ttgtcttcct tggcctattc 1560
cggaaaagat gcttgactcg agtctagagg gcccgtttaa acccgctgat cagcctcgac 1620
tgtgccttct agttgccagc catctgttgt ttgcccctcc cccgtgcctt ccttgaccct 1680
ggaaggtgcc actcccactg tcctttccta ataaaatgag gaaattgcat cgcattgtct 1740
gagtaggtgt cattctattc tggggggtgg ggtggggcag gacagcaagg gggaggattg 1800
ggaagacaat agcaggcatg ctggggatgc ggtgggctct atggcttctg aggcggaaag 1860
aaccagctgg ggctctaggg ggtatcccca cgcgccctgt agcggcgcat taagcgcggc 1920
gggtgtggtg gttacgcgca gcgtgaccgc tacacttgcc agcgccctag cgcccgctcc 1980
tttcgctttc ttcccttcct ttctcgccac gttcgccggc tttccccgtc aagctctaaa 2040
tcgggggctc cctttagggt tccgatttag tgctttacgg cacctcgacc ccaaaaaact 2100
tgattagggt gatggttcac gtagtgggcc atcgccctga tagacggttt ttcgcccttt 2160
gacgttggag tccacgttct ttaatagtgg actcttgttc caaactggaa caacactcaa 2220
ccctatctcg gtctattctt ttgatttata agggattttg ccgatttcgg cctattggtt 2280
aaaaaatgag ctgatttaac aaaaatttaa cgcgaattaa ttctgtggaa tgtgtgtcag 2340
ttagggtgtg gaaagtcccc aggctcccca gcaggcagaa gtatgcaaag catgcatctc 2400

aattagtcag caaccaggtg tggaaagtcc ccaggctccc cagcaggcag aagtatgcaa 2460

agcatgcatc tcaattagtc agcaaccata gtcccgcccc taactccgcc catcccgccc 2520

ctaactccgc ccagttccgc ccattctccg ccccatggct gactaatttt ttttatttat 2580

gcagaggccg aggccgcctc tgcctctgag ctattccaga agtagtgagg aggctttttt 2640

ggaggcctag gcttttgcaa aaagctcccg ggagcttgta tatccatttt cggatctgat 2700

caagagacag gatgaggatc gtttcgcatg attgaacaag atggattgca cgcaggttct 2760

ccggccgctt gggtggagag gctattcggc tatgactggg cacaacagac aatcggctgc 2820

tctgatgccg ccgtgttccg gctgtcagcg caggggcgcc cggttctttt tgtcaagacc 2880

gacctgtccg gtgccctgaa tgaactgcag gacgaggcag cgcggctatc gtggctggcc 2940

acgacgggcg ttccttgcgc agctgtgctc gacgttgtca ctgaagcggg aagggactgg 3000

ctgctattgg gcgaagtgcc ggggcaggat ctcctgtcat ctcaccttgc tcctgccgag 3060

aaagtatcca tcatggctga tgcaatgcgg cggctgcata cgcttgatcc ggctacctgc 3120

ccattcgacc accaagcgaa acatcgcatc gagcgagcac gtactcggat ggaagccggt 3180

cttgtcgatc aggatgatct ggacgaagag catcaggggc tcgcgccagc cgaactgttc 3240

gccaggctca aggcgcgcat gcccgacggc gaggatctcg tcgtgaccca tggcgatgcc 3300

tgcttgccga atatcatggt ggaaaatggc cgcttttctg gattcatcga ctgtggccgg 3360

ctgggtgtgg cggaccgcta tcaggacata gcgttggcta cccgtgatat tgctgaagag 3420

cttggcggcg aatgggctga ccgcttcctc gtgctttacg gtatcgccgc tcccgattcg 3480

cagcgcatcg ccttctatcg ccttcttgac gagttcttct gagcgggact ctggggttcg 3540

aaatgaccga ccaagcgacg cccaacctgc catcacgaga tttcgattcc accgccgcct 3600

tctatgaaag gttgggcttc ggaatcgttt tccgggacgc cggctggatg atcctccagc 3660

gcggggatct catgctggag ttcttcgccc accccaactt gtttattgca gcttataatg 3720

gttacaaata aagcaatagc atcacaaatt tcacaaataa agcatttttt tcactgcatt 3780

ctagttgtgg tttgtccaaa ctcatcaatg tatcttatca tgtctgtata ccgtcgacct 3840

ctagctagag cttggcgtaa tcatggtcat agctgtttcc tgtgtgaaat tgttatccgc 3900

tcacaattcc acacaacata cgagccggaa gcataaagtg taaagcctgg ggtgcctaat 3960

gagtgagcta actcacatta attgcgttgc gctcactgcc cgctttccag tcgggaaacc 4020

tgtcgtgcca gctgcattaa tgaatcggcc aacgcgcggg gagaggcggt ttgcgtattg 4080

ggcgctcttc cgcttcctcg ctcactgact cgctgcgctc ggtcgttcgg ctgcggcgag 4140

cggtatcagc tcactcaaag gcggtaatac ggttatccac agaatcaggg gataacgcag 4200

gaaagaacat gtgagcaaaa ggccagcaaa aggccaggaa ccgtaaaaag gccgcgttgc 4260

tggcgttttt ccataggctc cgcccccctg acgagcatca caaaaatcga cgctcaagtc 4320

agaggtggcg aaacccgaca ggactataaa gataccaggc gtttccccct ggaagctccc 4380

tcgtgcgctc tcctgttccg accctgccgc ttaccggata cctgtccgcc tttctccctt 4440

cgggaagcgt ggcgctttct catagctcac gctgtaggta tctcagttcg gtgtaggtcg 4500

ttcgctccaa gctgggctgt gtgcacgaac cccccgttca gcccgaccgc tgcgccttat 4560

ccggtaacta tcgtcttgag tccaacccgg taagacacga cttatcgcca ctggcagcag 4620

ccactggtaa caggattagc agagcgaggt atgtaggcgg tgctacagag ttcttgaagt 4680

ggtggcctaa ctacggctac actagaagaa cagtatttgg tatctgcgct ctgctgaagc 4740

cagttacctt cggaaaaaga gttggtagct cttgatccgg caaacaaacc accgctggta 4800

gcggtttttt tgtttgcaag cagcagatta cgcgcagaaa aaaaggatct caagaagatc 4860

ctttgatctt ttctacgggg tctgacgctc agtggaacga aaactcacgt taagggattt 4920

tggtcatgag attatcaaaa aggatcttca cctagatcct tttaaattaa aaatgaagtt 4980

ttaaatcaat ctaaagtata tatgagtaaa cttggtctga cagttaccaa tgcttaatca 5040

gtgaggcacc tatctcagcg atctgtctat ttcgttcatc catagttgcc tgactccccg 5100

tcgtgtagat aactacgata cgggagggct taccatctgg ccccagtgct gcaatgatac 5160

cgcgagaccc acgctcaccg gctccagatt tatcagcaat aaaccagcca gccggaaggg 5220

ccgagcgcag aagtggtcct gcaactttat ccgcctccat ccagtctatt aattgttgcc 5280

gggaagctag agtaagtagt tcgccagtta atagtttgcg caacgttgtt gccattgcta 5340

caggcatcgt ggtgtcacgc tcgtcgtttg gtatggcttc attcagctcc ggttcccaac 5400

gatcaaggcg agttacatga tcccccatgt tgtgcaaaaa agcggttagc tccttcggtc 5460

ctccgatcgt tgtcagaagt aagttggccg cagtgttatc actcatggtt atggcagcac 5520

tgcataattc tcttactgtc atgccatccg taagatgctt ttctgtgact ggtgagtact 5580

caaccaagtc attctgagaa tagtgtatgc ggcgaccgag ttgctcttgc ccggcgtcaa 5640

tacgggataa taccgcgcca catagcagaa ctttaaaagt gctcatcatt ggaaaacgtt 5700

cttcggggcg aaaactctca aggatcttac cgctgttgag atccagttcg atgtaaccca 5760

ctcgtgcacc caactgatct tcagcatctt ttactttcac cagcgtttct gggtgagcaa 5820

aaacaggaag gcaaaatgcc gcaaaaaagg gaataagggc gacacggaaa tgttgaatac 5880

tcatactctt cctttttcaa tattattgaa gcatttatca gggttattgt ctcatgagcg 5940

gatacatatt tgaatgtatt tagaaaaata aacaaatagg ggttccgcgc acatttcccc 6000

gaaaagtgcc acctgacgtc 6020

<210> 10
<211> 4703

<212> DNA
<213> Artificial Sequence

<220>
<223> Description of Artificial Sequence: Synthetic
      polynucleotide

<400> 10
gccttaatta ggctgcgcgc tcgctcgctc actgaggccg cccgggcaaa gcccgggcgt      60

cgggcgacct ttggtcgccc ggcctcagtg agcgagcgag cgcgcagaga gggagtggcc     120

aactccatca ctaggggttc cttgtagtta atgattaacc cgccatgcta cttatctacc     180

agggtaatgg ggatcctcta gaactatagc tagtcgacat tgattattga ctagttatta     240

atagtaatca attacggggt cattagttca tagcccatat atggagttcc gcgttacata     300

acttacggta aatggcccgc ctggctgacc gcccaacgac ccccgcccat tgacgtcaat     360

aatgacgtat gttcccatag taacgccaat agggactttc cattgacgtc aatgggtgga     420

gtatttacgg taaactgccc acttggcagt acatcaagtg tatcatatgc caagtacgcc     480

ccctattgac gtcaatgacg gtaaatggcc cgcctggcat tatgcccagt acatgacctt     540

atgggacttt cctacttggc agtacatcta cgtattagtc atcgctatta ccatgtcgag     600

gccacgttct gcttcactct ccccatctcc cccccctccc cacccccaat tttgtattta     660

tttatttttt aattattttg tgcagcgatg ggggcggggg gggggggcgc gcgccaggcg     720

gggcggggcg gggcgagggg cggggcgggg cgaggcggag aggtgcggcg gcagccaatc     780

agagcggcgc gctccgaaag tttcctttta tggcgaggcg gcggcggcgg cggccctata     840

aaaagcgaag cgcgcggcgg gcgggagcaa gcttgccgcc accatgtgga ctctcgggcg     900

ccgcgcagta gccggcctcc tggcgtcacc cagcccggcc caggcccaga ccctcacccg     960

ggtcccgcgg ccggcagagt tggccccact ctgcggccgc cgtggcctgc gcaccgacat    1020

cgatgcgacc tgcacgcccc gccgcgcaag ttcgaaccaa cgtggcctca accagatttg    1080

gaatgtcaaa aagcagagtg tctatttgat gaatttgagg aaatctggaa ctttgggcca    1140

cccaggctct ctagatgaga ccacctatga aagactagca gaggaaacgc tggactcttt    1200

agcagagttt tttgaagacc ttgcagacaa gccatacacg tttgaggact atgatgtctc    1260

ctttgggagt ggtgtcttaa ctgtcaaact gggtggagat ctaggaacct atgtgatcaa    1320

caagcagacg ccaaacaagc aaatctggct atcttctcca tccagtggac ctaagcgtta    1380

tgactggact gggaaaaact gggtgttctc ccacgacggc gtgtccctcc atgagctgct    1440

ggccgcagag ctcactaaag ccttaaaaac caaactggac ttgtcttggt tggcctattc    1500

cggaaaagat gcttgactcg aggacggggt gaactacgcc tgaggatccg atctttttcc    1560

ctctgccaaa aattatgggg acatcatgaa gccccttgag catctgactt ctggctaata    1620

aaggaaattt attttcattg caatagtgtg ttggaatttt ttgtgtctct cactcggaag    1680

caattcgttg atctgaattt cgaccaccca taatacccat taccctggta gataagtagc 1740

atggcgggtt aatcattaac tacaaggaac ccctagtgat ggagttggcc actccctctc 1800

tgcgcgctcg ctcgctcact gaggccgggc gaccaaaggt cgcccgacgc ccgggctttg 1860

cccgggcggc ctcagtgagc gagcgagcgc gcagccttaa ttaacctaat tcactggccg 1920

tcgttttaca acgtcgtgac tgggaaaacc ctggcgttac ccaacttaat cgccttgcag 1980

cacatccccc tttcgccagc tggcgtaata gcgaagaggc ccgcaccgat cgcccttccc 2040

aacagttgcg cagcctgaat ggcgaatggg acgcgccctg tagcggcgca ttaagcgcgg 2100

cgggtgtggt ggttacgcgc agcgtgaccg ctacacttgc cagcgcccta gcgcccgctc 2160

ctttcgcttt cttcccttcc tttctcgcca cgttcgccgg ctttccccgt caagctctaa 2220

atcgggggct ccctttaggg ttccgattta gtgctttacg gcacctcgac cccaaaaaac 2280

ttgattaggg tgatggttca cgtagtgggc catcgccctg atagacggtt tttcgccctt 2340

tgacgttgga gtccacgttc tttaatagtg gactcttgtt ccaaactgga acaacactca 2400

accctatctc ggtctattct tttgatttat aagggatttt gccgatttcg gcctattggt 2460

taaaaaatga gctgatttaa caaaaattta acgcgaattt taacaaaata ttaacgctta 2520

caatttaggt ggcacttttc ggggaaatgt gcgcggaacc cctatttgtt tatttttcta 2580

aatacattca aatatgtatc cgctcatgag acaataaccc tgataaatgc ttcaataata 2640

ttgaaaaagg aagagtatga gtattcaaca tttccgtgtc gcccttattc ccttttttgc 2700

ggcattttgc cttcctgttt ttgctcaccc agaaacgctg gtgaaagtaa aagatgctga 2760

agatcagttg ggtgcacgag tgggttacat cgaactggat ctcaacagcg gtaagatcct 2820

tgagagtttt cgccccgaag aacgttttcc aatgatgagc acttttaaag ttctgctatg 2880

tggcgcggta ttatcccgta ttgacgccgg gcaagagcaa ctcggtcgcc gcatacacta 2940

ttctcagaat gacttggttg agtactcacc agtcacagaa aagcatctta cggatggcat 3000

gacagtaaga gaattatgca gtgctgccat aaccatgagt gataacactg cggccaactt 3060

acttctgaca acgatcggag gaccgaagga gctaaccgct tttttgcaca acatggggga 3120

tcatgtaact cgccttgatc gttgggaacc ggagctgaat gaagccatac caaacgacga 3180

gcgtgacacc acgatgcctg tagcaatggc aacaacgttg cgcaaactat taactggcga 3240

actacttact ctagcttccc ggcaacaatt aatagactgg atggaggcgg ataaagttgc 3300

aggaccactt ctgcgctcgg cccttccggc tggctggttt attgctgata aatctggagc 3360

cggtgagcgt gggtctcgcg gtatcattgc agcactgggg ccagatggta agccctcccg 3420

tatcgtagtt atctacacga cggggagtca ggcaactatg gatgaacgaa atagacagat 3480

cgctgagata ggtgcctcac tgattaagca ttggtaactg tcagaccaag tttactcata 3540

tatactttag attgatttaa aacttcattt ttaatttaaa aggatctagg tgaagatcct 3600

ttttgataat ctcatgacca aaatccctta acgtgagttt tcgttccact gagcgtcaga 3660

ccccgtagaa aagatcaaag gatcttcttg agatcctttt tttctgcgcg taatctgctg 3720

cttgcaaaca aaaaaaccac cgctaccagc ggtggtttgt ttgccggatc aagagctacc 3780

aactcttttt ccgaaggtaa ctggcttcag cagagcgcag ataccaaata ctgttcttct 3840

agtgtagccg tagttaggcc accacttcaa gaactctgta gcaccgccta catacctcgc 3900

tctgctaatc ctgttaccag tggctgctgc cagtggcgat aagtcgtgtc ttaccgggtt 3960

ggactcaaga cgatagttac cggataaggc gcagcggtcg ggctgaacgg ggggttcgtg 4020

cacacagccc agcttggagc gaacgaccta caccgaactg agatacctac agcgtgagct 4080

atgagaaagc gccacgcttc ccgaagggag aaaggcggac aggtatccgg taagcggcag 4140

ggtcggaaca ggagagcgca cgagggagct tccaggggga aacgcctggt atctttatag 4200

tcctgtcggg tttcgccacc tctgacttga gcgtcgattt ttgtgatgct cgtcaggggg 4260

gcggagccta tggaaaaacg ccagcaacgc ggccttttta cggttcctgg ccttttgctg 4320

gccttttgct cacatgttct ttcctgcgtt atcccctgat tctgtggata accgtattac 4380

cgcctttgag tgagctgata ccgctcgccg cagccgaacg accgagcgca gcgagtcagt 4440

gagcgaggaa gcggaagagc gcccaatacg caaaccgcct ctccccgcgc gttggccgat 4500

tcattaatgc agctggcacg acaggtttcc cgactggaaa gcgggcagtg agcgcaacgc 4560

aattaatgtg agttagctca ctcattaggc accccaggct ttacacttta tgcttccggc 4620

tcgtatgttg tgtggaattg tgagcggata acaatttcac acaggaaaca gctatgacca 4680

tgattacgcc agatttaatt aag 4703

<210> 11
<211> 4945
<212> DNA
<213> Artificial Sequence

<220>
<223> Description of Artificial Sequence: Synthetic
      polynucleotide

<400> 11
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420
gtatttacgg taaactgccc acttggcagt acatcaagtg tatcatatgc caagtacgcc 480
ccctattgac gtcaatgacg gtaaatggcc cgcctggcat tatgcccagt acatgacctt 540
atgggacttt cctacttggc agtacatcta cgtattagtc atcgctatta ccatgtcgag 600
gccacgttct gcttcactct ccccatctcc cccccctccc cacccccaat tttgtattta 660
tttatttttt aattattttg tgcagcgatg ggggcggggg gggggggcgc gcgccaggcg 720
gggcggggcg gggcgagggg cggggcgggg cgaggcggag aggtgcggcg gcagccaatc 780
agagcggcgc gctccgaaag tttcctttta tggcgaggcg gcggcggcgg cggccctata 840
aaaagcgaag cgcgcggcgg gcgggagcaa gcttggagtc gctgcgttgc cttcgccccg 900
tgccccgctc cgcgccgcct cgcgccgccc gccccggctc tgactgaccg cgttactccc 960
acaggtgagc gggcgggacg gcccttctcc tccgggctgt aattagcaag aggtaagggt 1020
ttaagggatg gttggttggt ggggtattaa tgtttaatta cctgttttac aggcctgaaa 1080
tcacttggtt ttaggttggg cggccgatcc accggtgccg ccaccatgtg gactctcggg 1140
cgccgcgcag tagccggcct cctggcgtca cccagcccgg cccaggccca gaccctcacc 1200
cgggtcccgc ggccggcaga gttggcccca ctctgcggcc gccgtggcct gcgcaccgac 1260
atcgatgcga cctgcacgcc ccgccgcgca agttcgaacc aacgtggcct caaccagatt 1320
tggaatgtca aaaagcagag tgtctatttg atgaatttga ggaaatctgg aactttgggc 1380
cacccaggct ctctagatga gaccacctat gaaagactag cagaggaaac gctggactct 1440
ttagcagagt tttttgaaga ccttgcagac aagccataca cgtttgagga ctatgatgtc 1500
tcctttggga gtggtgtctt aactgtcaaa ctgggtggag atctaggaac ctatgtgatc 1560
aacaagcaga cgccaaacaa gcaaatctgg ctatcttctc catccagtgg acctaagcgt 1620
tatgactgga ctgggaaaaa ctgggtgttc tcccacgacg gcgtgtccct ccatgagctg 1680
ctggccgcag agctcactaa agccttaaaa accaaactgg acttgtcttg gttggcctat 1740
tccggaaaag atgcttgact cgaggacggg gtgaactacg cctgaggatc cgatcttttt 1800
ccctctgcca aaaattatgg ggacatcatg aagccccttg agcatctgac ttctggctaa 1860
taaaggaaat ttattttcat tgcaatagtg tgttggaatt ttttgtgtct ctcactcgga 1920
agcaattcgt tgatctgaat ttcgaccacc cataataccc attaccctgg tagataagta 1980
gcatggcggg ttaatcatta actacaagga acccctagtg atggagttgg ccactccctc 2040
tctgcgcgct cgctcgctca ctgaggccgg gcgaccaaag gtcgcccgac gcccgggctt 2100
tgcccgggcg gcctcagtga gcgagcgagc gcgcagcctt aattaaccta attcactggc 2160
cgtcgtttta caacgtcgtg actgggaaaa ccctggcgtt acccaactta atcgccttgc 2220
agcacatccc cctttcgcca gctggcgtaa tagcgaagag gcccgcaccg atcgcccttc 2280

ccaacagttg cgcagcctga atggcgaatg ggacgcgccc tgtagcggcg cattaagcgc 2340

ggcgggtgtg gtggttacgc gcagcgtgac cgctacactt gccagcgccc tagcgcccgc 2400

tcctttcgct ttcttccctt cctttctcgc cacgttcgcc ggctttcccc gtcaagctct 2460

aaatcggggg ctccctttag ggttccgatt tagtgcttta cggcacctcg accccaaaaa 2520

acttgattag ggtgatggtt cacgtagtgg gccatcgccc tgatagacgg tttttcgccc 2580

tttgacgttg gagtccacgt tctttaatag tggactcttg ttccaaactg gaacaacact 2640

caaccctatc tcggtctatt cttttgattt ataagggatt ttgccgattt cggcctattg 2700

gttaaaaaat gagctgattt aacaaaaatt taacgcgaat tttaacaaaa tattaacgct 2760

tacaatttag gtggcacttt tcggggaaat gtgcgcggaa cccctatttg tttatttttc 2820

taaatacatt caaatatgta tccgctcatg agacaataac cctgataaat gcttcaataa 2880

tattgaaaaa ggaagagtat gagtattcaa catttccgtg tcgcccttat tccctttttt 2940

gcggcatttt gccttcctgt ttttgctcac ccagaaacgc tggtgaaagt aaaagatgct 3000

gaagatcagt tgggtgcacg agtgggttac atcgaactgg atctcaacag cggtaagatc 3060

cttgagagtt ttcgccccga agaacgtttt ccaatgatga gcacttttaa agttctgcta 3120

tgtggcgcgg tattatcccg tattgacgcc gggcaagagc aactcggtcg ccgcatacac 3180

tattctcaga atgacttggt tgagtactca ccagtcacag aaaagcatct tacggatggc 3240

atgacagtaa gagaattatg cagtgctgcc ataaccatga gtgataacac tgcggccaac 3300

ttacttctga caacgatcgg aggaccgaag gagctaaccg cttttttgca caacatgggg 3360

gatcatgtaa ctcgccttga tcgttgggaa ccggagctga atgaagccat accaaacgac 3420

gagcgtgaca ccacgatgcc tgtagcaatg gcaacaacgt tgcgcaaact attaactggc 3480

gaactactta ctctagcttc ccggcaacaa ttaatagact ggatggaggc ggataaagtt 3540

gcaggaccac ttctgcgctc ggcccttccg gctggctggt ttattgctga taaatctgga 3600

gccggtgagc gtgggtctcg cggtatcatt gcagcactgg ggccagatgg taagccctcc 3660

cgtatcgtag ttatctacac gacggggagt caggcaacta tggatgaacg aaatagacag 3720

atcgctgaga taggtgcctc actgattaag cattggtaac tgtcagacca agtttactca 3780

tatatacttt agattgattt aaaacttcat ttttaattta aaaggatcta ggtgaagatc 3840

ctttttgata atctcatgac caaaatccct taacgtgagt tttcgttcca ctgagcgtca 3900

gaccccgtag aaaagatcaa aggatcttct tgagatcctt tttttctgcg cgtaatctgc 3960

tgcttgcaaa caaaaaaacc accgctacca gcggtggttt gtttgccgga tcaagagcta 4020

ccaactcttt ttccgaaggt aactggcttc agcagagcgc agataccaaa tactgttctt 4080

ctagtgtagc cgtagttagg ccaccacttc aagaactctg tagcaccgcc tacatacctc 4140

gctctgctaa tcctgttacc agtggctgct gccagtggcg ataagtcgtg tcttaccggg 4200

ttggactcaa gacgatagtt accggataag gcgcagcggt cgggctgaac ggggggttcg 4260

tgcacacagc ccagcttgga gcgaacgacc tacaccgaac tgagatacct acagcgtgag 4320

ctatgagaaa gcgccacgct tcccgaaggg agaaaggcgg acaggtatcc ggtaagcggc 4380

agggtcggaa caggagagcg cacgagggag cttccagggg gaaacgcctg gtatctttat 4440

agtcctgtcg ggtttcgcca cctctgactt gagcgtcgat ttttgtgatg ctcgtcaggg 4500

gggcggagcc tatggaaaaa cgccagcaac gcggcctttt tacggttcct ggccttttgc 4560

tggccttttg ctcacatgtt ctttcctgcg ttatcccctg attctgtgga taaccgtatt 4620

accgcctttg agtgagctga taccgctcgc cgcagccgaa cgaccgagcg cagcgagtca 4680

gtgagcgagg aagcggaaga gcgcccaata cgcaaaccgc ctctccccgc gcgttggccg 4740

attcattaat gcagctggca cgacaggttt cccgactgga aagcgggcag tgagcgcaac 4800

gcaattaatg tgagttagct cactcattag gcaccccagg ctttacactt tatgcttccg 4860

gctcgtatgt tgtgtggaat tgtgagcgga taacaatttc acacaggaaa cagctatgac 4920

catgattacg ccagatttaa ttaag 4945

<210> 12
<211> 4883
<212> DNA
<213> Artificial Sequence

<220>
<223> Description of Artificial Sequence: Synthetic
      polynucleotide

<400> 12
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780
agagcggcgc gctccgaaag tttcctttta tggcgaggcg gcggcggcgg cggccctata 840
aaaagcgaag cgcgcggcgg gcgggagcaa gcttgaactg aaaaaccaga aagttaactg 900
gtaagtttag tctttttgtc ttttatttca ggtcccggat ccggtggtgg tgcaaatcaa 960
agaactgctc ctcagtggat gttgccttta cttctaggcc tgtacggaag tgttacttct 1020
gctctaaaag ctgcggaatt gtacccgcgg ccgatccacc ggtatgtgga ctctcgggcg 1080
ccgcgcagta gccggcctcc tggcgtcacc cagcccggcc caggcccaga ccctcacccg 1140
ggtcccgcgg ccggcagagt tggccccact ctgcggccgc cgtggcctgc gcaccgacat 1200
cgatgcgacc tgcacgcccc gccgcgcaag ttcgaaccaa cgtggcctca accagatttg 1260
gaatgtcaaa aagcagagtg tctatttgat gaatttgagg aaatctggaa ctttgggcca 1320
cccaggctct ctagatgaga ccacctatga aagactagca gaggaaacgc tggactcttt 1380
agcagagttt tttgaagacc ttgcagacaa gccatacacg tttgaggact atgatgtctc 1440
ctttgggagt ggtgtcttaa ctgtcaaact gggtggagat ctaggaacct atgtgatcaa 1500
caagcagacg ccaaacaagc aaatctggct atcttctcca tccagtggac ctaagcgtta 1560
tgactggact gggaaaaact gggtgttctc ccacgacggc gtgtccctcc atgagctgct 1620
ggccgcagag ctcactaaag ccttaaaaac caaactggac ttgtcttggt tggcctattc 1680
cggaaaagat gcttgactcg aggacggggt gaactacgcc tgaggatccg atctttttcc 1740
ctctgccaaa aattatgggg acatcatgaa gccccttgag catctgactt ctggctaata 1800
aaggaaattt attttcattg caatagtgtg ttggaatttt ttgtgtctct cactcggaag 1860
caattcgttg atctgaattt cgaccaccca taatacccat taccctggta gataagtagc 1920
atggcgggtt aatcattaac tacaaggaac ccctagtgat ggagttggcc actccctctc 1980
tgcgcgctcg ctcgctcact gaggccgggc gaccaaaggt cgcccgacgc ccgggctttg 2040
cccgggcggc ctcagtgagc gagcgagcgc gcagccttaa ttaacctaat tcactggccg 2100
tcgttttaca acgtcgtgac tgggaaaacc ctggcgttac ccaacttaat cgccttgcag 2160
cacatccccc tttcgccagc tggcgtaata gcgaagaggc ccgcaccgat cgcccttccc 2220
aacagttgcg cagcctgaat ggcgaatggg acgcgccctg tagcggcgca ttaagcgcgg 2280
cgggtgtggt ggttacgcgc agcgtgaccg ctacacttgc cagcgcccta gcgcccgctc 2340
ctttcgcttt cttcccttcc tttctcgcca cgttcgccgg ctttccccgt caagctctaa 2400
atcgggggct ccctttaggg ttccgattta gtgctttacg gcacctcgac cccaaaaaac 2460
ttgattaggg tgatggttca cgtagtgggc catcgccctg atagacggtt tttcgccctt 2520
tgacgttgga gtccacgttc tttaatagtg gactcttgtt ccaaactgga acaacactca 2580
accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 13
<211> 4883
<212> DNA
<213> Artificial Sequence

<220>
<223> Description of Artificial Sequence: Synthetic
      polynucleotide

<400> 13
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtatgtgga ccctgggccg 1080

gcgggctgtg gccggcctgt tggcctctcc cagccctgct caagcccaga cactgacccg 1140

ggtcccccga cccgcagagc tcgctcctct gtgcggacgg cgggggctga gaacagacat 1200

tgacgcaacc tgcaccccac ggagagcttc atcaaaccaa cgcggactca atcagatctg 1260

gaatgtcaag aagcagtctg tatatttgat gaatctgcgc aaatccggaa ccttgggaca 1320

tcctggaagc cttgatgaga ccacatacga acggctcgct gaagagacat tggacagtct 1380

ggctgagttc tttgaggacc tcgcggataa gccatataca tttgaagact atgatgtttc 1440

ttttggctca ggggtactga cagtgaaact gggaggggat ctggggacct atgtcatcaa 1500

taagcaaaca cccaacaaac agatctggct gtcatcccca agcagcgggc ccaagcgcta 1560

tgattggaca gggaaaaact gggtctattc ccatgacggg gtatccctgc acgaactgct 1620

tgcggcggaa cttactaagg ccctgaagac aaagctggac ctgtcttcac ttgcctacag 1680

tggtaaagat gcgtaactcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 14
<211> 4883
<212> DNA
<213> Artificial Sequence

<220>
<223> Description of Artificial Sequence: Synthetic
      polynucleotide

<400> 14
gccttaatta ggctgcgcgc tcgctcgctc actgaggccg cccgggcaaa gcccgggcgt      60

cgggcgacct ttggtcgccc ggcctcagtg agcgagcgag cgcgcagaga gggagtggcc     120

aactccatca ctaggggttc cttgtagtta atgattaacc cgccatgcta cttatctacc     180

agggtaatgg ggatcctcta gaactatagc tagtcgacat tgattattga ctagttatta     240

atagtaatca attacggggt cattagttca tagcccatat atggagttcc gcgttacata     300

acttacggta aatggcccgc ctggctgacc gcccaacgac ccccgcccat tgacgtcaat     360

aatgacgtat gttcccatag taacgccaat agggactttc cattgacgtc aatgggtgga     420

gtatttacgg taaactgccc acttggcagt acatcaagtg tatcatatgc caagtacgcc     480

ccctattgac gtcaatgacg gtaaatggcc cgcctggcat tatgcccagt acatgacctt     540

atgggacttt cctacttggc agtacatcta cgtattagtc atcgctatta ccatgtcgag     600

gccacgttct gcttcactct ccccatctcc cccccctccc cacccccaat tttgtattta     660

tttatttttt aattattttg tgcagcgatg ggggcggggg gggggggcgc gcgccaggcg     720

gggcggggcg gggcgagggg cggggcgggg cgaggcggag aggtgcggcg gcagccaatc     780

agagcggcgc gctccgaaag tttcctttta tggcgaggcg gcggcggcgg cggccctata     840

aaaagcgaag cgcgcggcgg gcgggagcaa gcttgaactg aaaaaccaga aagttaactg     900

gtaagtttag tctttttgtc ttttatttca ggtcccggat ccggtggtgg tgcaaatcaa     960

agaactgctc ctcagtggat gttgccttta cttctaggcc tgtacggaag tgttacttct    1020

gctctaaaag ctgcggaatt gtacccgcgg ccgatccacc ggtatgtgga ctctgggtcg    1080

cagagcggtg gcaggcttgc tggcatctcc gtccccggct caggcccaga ccctgacacg    1140

ggtgcccagg ccggccgagc tggctcctct gtgcggccgg cgcggcctta gaacagacat    1200

tgacgcaacc tgcaccccca gaagagccag ttccaaccag cgcgggttga atcagatctg    1260

gaacgtcaag aagcagagtg tgtaccttat gaatctccgg aaaagtggca ccctcggaca    1320

ccccggatcc ctcgatgaaa ccacttatga gcgcctggcc gaggaaaccc tggattccct    1380

ggctgagttc ttcgaggacc tggctgacaa accctacacc ttcgaagact acgacgtttc    1440

cttcggaagt ggggtgctga cggtcaaact tggtggcgat cttggaacct atgttataaa    1500

caagcagact cccaacaaac agatctggct cagctctcca agttcaggac ccaagaggta    1560

tgactggacg ggcaagaatt gggtctattc tcacgacgga gtgagccttc atgaactgtt 1620

ggctgccgaa ctgacaaaag cccttaagac gaaactggac ctgtcctcat tggcgtactc 1680

tggtaaggat gcctaactcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 15
<211> 4883
<212> DNA
<213> Artificial Sequence

<220>
<223> Description of Artificial Sequence: Synthetic
      polynucleotide

<400> 15
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtatgtgga cgctggggcg 1080

gcgcgcagtg gctggcctgc tggcctcacc gtcaccggcg caggcacaaa ccctgaccag 1140

agtaccaagg cccgctgagc ttgcacccct ttgtggacgg cgcggcctga gaaccgacat 1200

tgatgctacc tgcaccccgc gcagggctag ctctaaccag cgaggtctga atcagatttg 1260

gaacgtcaaa aaacagtctg tttacctgat gaacctgcga aaaagcggca cccttggcca 1320

tccaggcagt ttggacgaaa ctacttatga gcgactggcc gaagagaccc tggactctct 1380

ggccgagttt ttcgaggatc tcgccgataa gccctatact ttcgaggatt acgacgtaag 1440

cttcggatca ggggtgctga ctgtgaaatt gggtggagat ctcggtacct atgtcatcaa 1500

taaacagacg cctaataaac agatttggct ttctagccct tcctcaggcc ccaagcgcta 1560

tgactggacc ggaaaaaatt gggtgtacag ccacgacggt gttagccttc acgaactgct 1620

ggcagccgag ctgactaaag ccttgaagac taaattggac ttgtccagcc ttgcctacag 1680

tgggaaagac gcttgactcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 16
<211> 4883
<212> DNA
<213> Artificial Sequence

<220>
<223> Description of Artificial Sequence: Synthetic
      polynucleotide

<400> 16
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtatgtgga ctctggggag 1080

gcgcgccgtt gccggactcc tggcatctcc aagcccagcg caggctcaaa ccctcacgcg 1140

cgttcccagg ccagccgagt tggcccccct ttgtggcagg cgcggcctga ggacagatat 1200

cgacgccaca tgcactcctc gccgcgcctc cagcaatcaa aggggtttga accaaatttg 1260

gaacgtcaag aagcaatcag tgtatctcat gaatctgaga aagagcggca cactcggtca 1320

tcccggaagt ctggacgaaa ccacatacga acggctcgcc gaggaaacgc tggatagcct 1380

ggccgagttt ttcgaggacc tggctgataa accgtatact ttcgaagatt atgacgtgtc 1440

attcgggagc ggcgtactga ccgtcaaatt gggcggtgac ctggggacat acgtgattaa 1500

caagcagact cctaataaac agatttggtt gagctctcct tccagcgggc ctaaacgcta 1560

tgattggaca gggaagaatt gggtttacag tcacgacgga gttagccttc acgagctctt 1620

ggcagcggag ctgacaaagg cgctcaagac caaactcgac ctgtcttcac tcgcctattc 1680

aggcaaagac gcctgactcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 17
<211> 4883
<212> DNA
<213> Artificial Sequence

<220>
<223> Description of Artificial Sequence: Synthetic
      polynucleotide

<400> 17
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtatgtgga cactgggccg 1080

gcgggcggtc gccggcttgc tcgcttcccc ctcccccgcc caggcacaga cccttacccg 1140

cgtcccacgc cctgccgaac tggcccctct ttgcggccgc agagggctga gaacggatat 1200

tgacgccaca tgtacacctc ggagggcttc cagtaaccag cgggggctta accagatctg 1260

gaacgtgaaa aaacagagcg tgtatctcat gaatctcagg aaatccggga ctcttggcca 1320

ccctggctct ctggacgaaa caacttacga acggctggcg gaagaaactt tggattcctt 1380

ggccgagttc tttgaagacc tcgctgacaa gccctatacc tttgaggatt atgacgtgag 1440

cttcggcagc ggggtcctga ccgtaaagct ggggggcgac ctgggcactt atgtaattaa 1500

caagcagacc cccaacaaac agatctggct cagtagtccc agttccggcc ctaaacgcta 1560

cgattggacc gggaaaaatt gggtttactc ccacgacggc gtctccttgc acgagctcct 1620

ggcagctgag ctgaccaaag ccctgaagac caaactcgat ttgagttctc ttgcctattc 1680

tggcaaagac gcataactcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 18
<211> 4883
<212> DNA
<213> Artificial Sequence

<220>
<223> Description of Artificial Sequence: Synthetic
      polynucleotide

<400> 18
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtatgtgga cactcggacg 1080

ccgagccgtg gcgggcttgc ttgccagtcc tagtcctgca caggctcaaa ccctgactcg 1140

cgtcccgcga ccagccgaac tggccccgct gtgcgggcgg agagggctca ggacagatat 1200

tgatgctacc tgtaccccga gaagggccag ctcaaatcag agaggtctga atcagatctg 1260

gaacgtaaag aagcaatctg tgtatctgat gaacctccgc aagtcaggga ccctgggtca 1320

ccctggcagc ctcgatgaga ccacatacga gcgcctggcc gaagagacgt tggactcttt 1380

ggcagagttc ttcgaagacc tcgctgataa gccttacaca tttgaggatt acgacgtgtc 1440

ttttggatct ggggtgctca cagtgaagct ggggggtgat ctgggcactt acgtgattaa 1500

caagcagacc cccaataaac aaatctggct cagttctccc tcctccgggc ctaagcggta 1560

tgactggact gggaagaact gggtttacag ccacgatggt gtctctttgc acgaactcct 1620

ggccgcggag ctcactaagg cacttaagac caagttggac ctttcttcct tggcatattc 1680

cggcaaagac gcataactcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 19
<211> 4883
<212> DNA
<213> Artificial Sequence

<220>

<223> Description of Artificial Sequence: Synthetic
      polynucleotide

<400> 19
gccttaatta ggctgcgcgc tcgctcgctc actgaggccg cccgggcaaa gcccgggcgt      60

cgggcgacct ttggtcgccc ggcctcagtg agcgagcgag cgcgcagaga gggagtggcc     120

aactccatca ctaggggttc cttgtagtta atgattaacc cgccatgcta cttatctacc     180

agggtaatgg ggatcctcta gaactatagc tagtcgacat tgattattga ctagttatta     240

atagtaatca attacggggt cattagttca tagcccatat atggagttcc gcgttacata     300

acttacggta aatggcccgc ctggctgacc gcccaacgac ccccgcccat tgacgtcaat     360

aatgacgtat gttcccatag taacgccaat agggactttc cattgacgtc aatgggtgga     420

gtatttacgg taaactgccc acttggcagt acatcaagtg tatcatatgc caagtacgcc     480

ccctattgac gtcaatgacg gtaaatggcc cgcctggcat tatgcccagt acatgacctt     540

atgggacttt cctacttggc agtacatcta cgtattagtc atcgctatta ccatgtcgag     600

gccacgttct gcttcactct ccccatctcc cccccctccc cacccccaat tttgtattta     660

tttatttttt aattattttg tgcagcgatg ggggcggggg gggggggcgc gcgccaggcg     720

gggcggggcg gggcgagggg cggggcgggg cgaggcggag aggtgcggcg gcagccaatc     780

agagcggcgc gctccgaaag tttcctttta tggcgaggcg gcggcggcgg cggccctata     840

aaaagcgaag cgcgcggcgg gcgggagcaa gcttgaactg aaaaaccaga aagttaactg     900

gtaagtttag tctttttgtc ttttatttca ggtcccggat ccggtggtgg tgcaaatcaa     960

agaactgctc ctcagtggat gttgccttta cttctaggcc tgtacggaag tgttacttct    1020

gctctaaaag ctgcggaatt gtacccgcgg ccgatccacc ggtatgtgga cgcttggaag    1080

gcgggccgtg gccggcctgc ttgcatcacc gtctcctgct caagctcaga ctttgactcg    1140

cgtgccaaga ccagctgagc tcgctccact ttgcgggcga cgaggcctga ggactgatat    1200

tgatgctacc tgcaccccaa ggcgcgcctc ctcaaaccag agaggcctga accagatttg    1260

gaatgttaag aagcagtctg tttacctcat gaacctccgg aagtcaggga cactgggaca    1320

cccagggtca cttgacgaga caacctatga gagactggcc gaagagaccc tcgattccct    1380

tgcagaattc ttcgaggatc ttgctgataa gccctatacc tttgaagact atgacgtttc    1440

ctttgggagt ggtgtgctca ccgtcaaact ggggggcgat ctgggaacat acgtaatcaa    1500

caaacagact ccaaataaac aaatttggct gtcaagcccc agttctgggc ctaagcggta    1560

tgactggact ggtaagaact gggtttacag tcatgacggt gtgagcctgc acgaacttct    1620

ggccgccgag cttacaaagg ccctgaagac aaagctggat ctgagcagtt tggcctattc    1680

tggcaaggac gcgtgactcg aggacggggt gaactacgcc tgaggatccg atctttttcc    1740

ctctgccaaa aattatgggg acatcatgaa gccccttgag catctgactt ctggctaata    1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 20
<211> 4883
<212> DNA
<213> Artificial Sequence

<220>
<223> Description of Artificial Sequence: Synthetic
      polynucleotide

<400> 20
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtatgtgga cactcgggcg 1080

acgcgccgtg gctgggctgc ttgcttcccc aagtcccgca caggcgcaaa ccctcacaag 1140

ggtcccacgg ccagctgaat tggccccgct gtgcggtcgg aggggcctgc gaacagatat 1200

agacgcaacg tgcaccccca gaagagcaag cagcaaccag agaggcctca atcagatttg 1260

gaacgtcaag aaacagagtg tctatctgat gaacctccgc aaatcaggga cccttggtca 1320

tccgggatct cttgatgaaa cgacctatga acggctggct gaggagacgc ttgactccct 1380

ggcagagttc ttcgaggatc tggctgataa gccatacacg ttcgaggact atgatgtttc 1440

ttttggttct ggcgtcctga cggtgaaact cggaggggat ctgggcactt acgttatcaa 1500

caaacagact cccaataaac agatttggtt gtctagtcct agcagtggcc caaaacgcta 1560

tgactggacg ggcaagaact gggtgtactc ccacgatgga gttagcctgc acgaacttct 1620

ggccgctgag cttactaaag cgctgaagac taagctggat ctctccagcc tcgcatacag 1680

cggcaaagac gcctagctcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 21
<211> 4883
<212> DNA
<213> Artificial Sequence

<220>
<223> Description of Artificial Sequence: Synthetic
      polynucleotide

<400> 21
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtatgtgga cactgggccg 1080

gcgagcggtg gccggactcc tcgccagccc aagccctgct caggctcaga cactgactcg 1140

cgtgcctcgc ccagccgagc ttgcaccatt gtgtggacgc cggggactca gaactgacat 1200

agacgccacc tgcactcctc gacgggcctc atctaaccag aggggcttga atcaaatatg 1260

gaatgtgaag aagcagtcag tctacctgat gaatttgcgg aagagtggga ccttgggcca 1320

cccaggttcc cttgacgaaa ccacctatga gcgacttgcg gaagagaccc tggacagtct 1380

ggccgagttc ttcgaggatt tggcagataa accatacacg ttcgaagact acgacgtgag 1440

cttcggcagc ggcgtactga cagttaaact cggcggtgac ctggggacgt acgtgattaa 1500

caagcagaca ccaaacaaac aaatttggtt gtccagccct tcttccggac caaaacggta 1560

cgattggaca ggaaaaaatt gggtttactc ccatgacggc gtgagcctgc acgaactcct 1620

cgctgcggaa ctgacaaagg cacttaagac caaactggac ctctcaagcc tggcctacag 1680

cggaaaagac gcatagctcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 22
<211> 4883
<212> DNA
<213> Artificial Sequence

<220>
<223> Description of Artificial Sequence: Synthetic
      polynucleotide

<400> 22
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtatgtgga ctctgggtcg 1080

acgcgccgta gccggtctct tggcctcccc atcccctgcg caggcacaga cccttacacg 1140

agttccacgg cccgctgaac tggcaccact ctgcggacgc cggggcctga gaaccgatat 1200

tgacgctacg tgcactcccc gacgagcctc ttctaatcag agggggctca accagatctg 1260

gaacgtgaag aagcagtcag tctacttgat gaatctgaga aagagcggga cccttgggca 1320

ccccgggtct ctcgacgaga ctacatacga gaggctggcc gaggagactt tggactctct 1380

cgccgaattc ttcgaggatt tggcagacaa gccgtacacc tttgaagact acgatgtctc 1440

attcggatca ggggtgctga cggttaaact ggggggcgat ctcggaacct acgtcataaa 1500

caaacagaca cccaataagc agatctggtt gtctagccca tcatccggtc ctaaacggta 1560

cgactggacc ggcaagaact gggtctactc ccatgacgga gtcagtcttc atgaactgct 1620

tgccgcagag ctgaccaagg ctcttaagac gaaactggat ctctctagcc tggcctactc 1680

cgggaaggat gcatagctcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 23
<211> 4883
<212> DNA
<213> Artificial Sequence

<220>
<223> Description of Artificial Sequence: Synthetic
      polynucleotide

<400> 23
gccttaatta ggctgcgcgc tcgctcgctc actgaggccg cccgggcaaa gcccgggcgt      60

cgggcgacct ttggtcgccc ggcctcagtg agcgagcgag cgcgcagaga gggagtggcc     120

aactccatca ctaggggttc cttgtagtta atgattaacc cgccatgcta cttatctacc     180

agggtaatgg ggatcctcta gaactatagc tagtcgacat tgattattga ctagttatta     240

atagtaatca attacggggt cattagttca tagcccatat atggagttcc gcgttacata     300

acttacggta aatggcccgc ctggctgacc gcccaacgac ccccgcccat tgacgtcaat     360

aatgacgtat gttcccatag taacgccaat agggactttc cattgacgtc aatgggtgga     420

gtatttacgg taaactgccc acttggcagt acatcaagtg tatcatatgc caagtacgcc     480

ccctattgac gtcaatgacg gtaaatggcc cgcctggcat tatgcccagt acatgacctt     540

atgggacttt cctacttggc agtacatcta cgtattagtc atcgctatta ccatgtcgag     600

gccacgttct gcttcactct ccccatctcc cccccctccc cacccccaat tttgtattta     660

tttatttttt aattattttg tgcagcgatg ggggcggggg gggggggcgc gcgccaggcg     720

gggcggggcg gggcgagggg cggggcgggg cgaggcggag aggtgcggcg gcagccaatc     780

agagcggcgc gctccgaaag tttcctttta tggcgaggcg gcggcggcgg cggccctata     840

aaaagcgaag cgcgcggcgg gcgggagcaa gcttgaactg aaaaaccaga aagttaactg     900

gtaagtttag tctttttgtc ttttatttca ggtcccggat ccggtggtgg tgcaaatcaa     960

agaactgctc ctcagtggat gttgccttta cttctaggcc tgtacggaag tgttacttct    1020

gctctaaaag ctgcggaatt gtacccgcgg ccgatccacc ggtatgtgga ctctcgggcg    1080

acgcgccgta gccggtctcc tggcttcccc aagccctgca caggcacaga ccctcacacg    1140

ggtcccacgg ccagctgaat tggccccact ctgcggtcgc cggggcctgc gaaccgatat    1200

agacgcaacg tgcactcccc gacgagcaag ttccaaccag agagggctca accagatttg    1260

gaacgtcaag aagcagagtg tctatttgat gaatctgaga aaatccggga cccttgggca    1320

ccccggatct cttgatgaaa ctacctatga aaggctggcc gaggagacgc tggactctct    1380

ggcagagttc ttcgaggatc tggcagacaa gccatacacg tttgaggact atgatgtctc    1440

ttttggatct ggcgtcctga cggttaaact gggaggggat ctcggaacct acgttatcaa    1500

caaacagact cccaataagc agatctggtt gtctagtcca tccagtggtc ctaaacgcta    1560

tgactggacg ggcaagaact gggtgtactc ccacgacgga gttagccttc atgaactgct 1620

ggccgcagag ctgactaaag ctcttaagac taaactggat ctctctagcc tggcctactc 1680

cgggaaagat gcctagctcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 24
<211> 4883
<212> DNA
<213> Artificial Sequence

<220>
<223> Description of Artificial Sequence: Synthetic
      polynucleotide

<400> 24
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtatgtgga ctctggggcg 1080

acgcgccgtg gccgggctcc tggcatcccc atcccctgcg caggcgcaaa ccctcacacg 1140

ggttccacgg ccagctgaac tggccccact gtgcggccgg cggggcctga gaacagatat 1200

tgacgcaacg tgcactccca gaagagccag cagcaaccag agggggttga atcagatctg 1260

gaacgtcaag aagcagtcag tgtacctgat gaatctccga aaaagcggga cccttggtca 1320

ccccggatct ctcgatgaaa ccacatatga acggctggcc gaggaaacgc tggactccct 1380

ggccgagttc ttcgaggacc tggcagacaa gccgtacacc ttcgaagact atgatgtttc 1440

attcggaagt ggcgtgctga cggtcaaact gggaggcgat ctgggaacct acgttataaa 1500

caaacagact cccaataaac agatctggtt gtctagtcca tccagcggtc ctaaacgcta 1560

tgactggacg ggcaagaact gggtctactc ccacgacgga gttagccttc acgaactgtt 1620

ggccgcagag ctgacaaagg cgcttaagac gaaactggat ctctctagcc tggcctactc 1680

cggcaaagat gcctagctcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 25
<211> 4850
<212> DNA
<213> Artificial Sequence

<220>
<223> Description of Artificial Sequence: Synthetic
      polynucleotide

<400> 25
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtgccgcca ccatggcgcc 1080

ctacagccta ctggtgactc ggctgcagaa agctctgggt gtgcggcagt accatgtggc 1140

ctcagtcctg tgcctgcgca ccgacatcga tgcgacctgc acgccccgcc gcgcaagttc 1200

gaaccaacgt ggcctcaacc agatttggaa tgtcaaaaag cagagtgtct atttgatgaa 1260

tttgaggaaa tctggaactt tgggccaccc aggctctcta gatgagacca cctatgaaag 1320

actagcagag gaaacgctgg actctttagc agagtttttt gaagaccttg cagacaagcc 1380

atacacgttt gaggactatg atgtctcctt tgggagtggt gtcttaactg tcaaactggg 1440

tggagatcta ggaacctatg tgatcaacaa gcagacgcca aacaagcaaa tctggctatc 1500

ttctccatcc agtggaccta agcgttatga ctggactggg aaaaactggg tgttctccca 1560

cgacggcgtg tccctccatg agctgctggc cgcagagctc actaaagcct taaaaaccaa 1620

actggacttg tcttggttgg cctattccgg aaaagatgct tgactcgagg acggggtgaa 1680

ctacgcctga ggatccgatc tttttccctc tgccaaaaat tatggggaca tcatgaagcc 1740

ccttgagcat ctgacttctg gctaataaag gaaatttatt ttcattgcaa tagtgtgttg 1800

gaattttttg tgtctctcac tcggaagcaa ttcgttgatc tgaatttcga ccacccataa 1860

tacccattac cctggtagat aagtagcatg gcgggttaat cattaactac aaggaacccc 1920

tagtgatgga gttggccact ccctctctgc gcgctcgctc gctcactgag gccgggcgac 1980

caaaggtcgc ccgacgcccg ggctttgccc gggcggcctc agtgagcgag cgagcgcgca 2040

gccttaatta acctaattca ctggccgtcg ttttacaacg tcgtgactgg gaaaaccctg 2100

gcgttaccca acttaatcgc cttgcagcac atcccccttt cgccagctgg cgtaatagcg 2160

aagaggcccg caccgatcgc ccttcccaac agttgcgcag cctgaatggc gaatgggacg 2220

cgccctgtag cggcgcatta agcgcggcgg gtgtggtggt tacgcgcagc gtgaccgcta 2280

cacttgccag cgccctagcg cccgctcctt tcgctttctt cccttccttt ctcgccacgt 2340

tcgccggctt tccccgtcaa gctctaaatc gggggctccc tttagggttc cgatttagtg 2400

ctttacggca cctcgacccc aaaaaacttg attagggtga tggttcacgt agtgggccat 2460

cgccctgata gacggttttt cgccctttga cgttggagtc cacgttcttt aatagtggac 2520

tcttgttcca aactggaaca acactcaacc ctatctcggt ctattctttt gatttataag 2580

ggattttgcc gatttcggcc tattggttaa aaaatgagct gatttaacaa aaatttaacg 2640

cgaattttaa caaaatatta acgcttacaa tttaggtggc acttttcggg gaaatgtgcg 2700

cggaacccct atttgtttat ttttctaaat acattcaaat atgtatccgc tcatgagaca 2760

ataaccctga taaatgcttc aataatattg aaaaaggaag agtatgagta ttcaacattt 2820

ccgtgtcgcc cttattccct tttttgcggc attttgcctt cctgtttttg ctcacccaga 2880

aacgctggtg aaagtaaaag atgctgaaga tcagttgggt gcacgagtgg gttacatcga 2940

actggatctc aacagcggta agatccttga gagttttcgc cccgaagaac gttttccaat 3000

gatgagcact tttaaagttc tgctatgtgg cgcggtatta tcccgtattg acgccgggca 3060

agagcaactc ggtcgccgca tacactattc tcagaatgac ttggttgagt actcaccagt 3120

cacagaaaag catcttacgg atggcatgac agtaagagaa ttatgcagtg ctgccataac 3180

catgagtgat aacactgcgg ccaacttact tctgacaacg atcggaggac cgaaggagct 3240

aaccgctttt ttgcacaaca tgggggatca tgtaactcgc cttgatcgtt gggaaccgga 3300

gctgaatgaa gccataccaa acgacgagcg tgacaccacg atgcctgtag caatggcaac 3360

aacgttgcgc aaactattaa ctggcgaact acttactcta gcttcccggc aacaattaat 3420

agactggatg gaggcggata aagttgcagg accacttctg cgctcggccc ttccggctgg 3480

ctggtttatt gctgataaat ctggagccgg tgagcgtggg tctcgcggta tcattgcagc 3540

actggggcca gatggtaagc cctcccgtat cgtagttatc tacacgacgg ggagtcaggc 3600

aactatggat gaacgaaata gacagatcgc tgagataggt gcctcactga ttaagcattg 3660

gtaactgtca gaccaagttt actcatatat actttagatt gatttaaaac ttcattttta 3720

atttaaaagg atctaggtga agatcctttt tgataatctc atgaccaaaa tcccttaacg 3780

tgagttttcg ttccactgag cgtcagaccc cgtagaaaag atcaaaggat cttcttgaga 3840

tccttttttt ctgcgcgtaa tctgctgctt gcaaacaaaa aaaccaccgc taccagcggt 3900

ggtttgtttg ccggatcaag agctaccaac tctttttccg aaggtaactg gcttcagcag 3960

agcgcagata ccaaatactg ttcttctagt gtagccgtag ttaggccacc acttcaagaa 4020

ctctgtagca ccgcctacat acctcgctct gctaatcctg ttaccagtgg ctgctgccag 4080

tggcgataag tcgtgtctta ccgggttgga ctcaagacga tagttaccgg ataaggcgca 4140

gcggtcgggc tgaacggggg gttcgtgcac acagcccagc ttggagcgaa cgacctacac 4200

cgaactgaga tacctacagc gtgagctatg agaaagcgcc acgcttcccg aagggagaaa 4260

ggcggacagg tatccggtaa gcggcagggt cggaacagga gagcgcacga gggagcttcc 4320

agggggaaac gcctggtatc tttatagtcc tgtcgggttt cgccacctct gacttgagcg 4380

tcgatttttg tgatgctcgt caggggggcg gagcctatgg aaaaacgcca gcaacgcggc 4440

ctttttacgg ttcctggcct tttgctggcc ttttgctcac atgttctttc ctgcgttatc 4500

ccctgattct gtggataacc gtattaccgc ctttgagtga gctgataccg ctcgccgcag 4560

ccgaacgacc gagcgcagcg agtcagtgag cgaggaagcg gaagagcgcc caatacgcaa 4620

accgcctctc cccgcgcgtt ggccgattca ttaatgcagc tggcacgaca ggtttcccga 4680

ctggaaagcg ggcagtgagc gcaacgcaat taatgtgagt tagctcactc attaggcacc 4740

ccaggcttta cactttatgc ttccggctcg tatgttgtgt ggaattgtga gcggataaca 4800

atttcacaca ggaaacagct atgaccatga ttacgccaga tttaattaag 4850

<210> 26
<211> 4850
<212> DNA
<213> Artificial Sequence

<220>
<223> Description of Artificial Sequence: Synthetic
      polynucleotide

<400> 26
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtgccgcca ccatggcttt 1080

acttactgcg gccgcccggc tcttgggaac caagaatgca tcttgtcttg ttcttgcagc 1140

ccggcatgct agtctgcgca ccgacatcga tgcgacctgc acgccccgcc gcgcaagttc 1200

gaaccaacgt ggcctcaacc agatttggaa tgtcaaaaag cagagtgtct atttgatgaa 1260

tttgaggaaa tctggaactt tgggccaccc aggctctcta gatgagacca cctatgaaag 1320

actagcagag gaaacgctgg actctttagc agagtttttt gaagaccttg cagacaagcc 1380

atacacgttt gaggactatg atgtctcctt tgggagtggt gtcttaactg tcaaactggg 1440

tggagatcta ggaacctatg tgatcaacaa gcagacgcca aacaagcaaa tctggctatc 1500

ttctccatcc agtggaccta agcgttatga ctggactggg aaaaactggg tgttctccca 1560

cgacggcgtg tccctccatg agctgctggc cgcagagctc actaaagcct taaaaaccaa 1620

actggacttg tcttggttgg cctattccgg aaaagatgct tgactcgagg acggggtgaa 1680

ctacgcctga ggatccgatc tttttccctc tgccaaaaat tatggggaca tcatgaagcc 1740

ccttgagcat ctgacttctg gctaataaag gaaatttatt ttcattgcaa tagtgtgttg 1800

gaattttttg tgtctctcac tcggaagcaa ttcgttgatc tgaatttcga ccacccataa 1860

tacccattac cctggtagat aagtagcatg gcgggttaat cattaactac aaggaacccc 1920

tagtgatgga gttggccact ccctctctgc gcgctcgctc gctcactgag gccgggcgac 1980

caaaggtcgc ccgacgcccg ggctttgccc gggcggcctc agtgagcgag cgagcgcgca 2040

gccttaatta acctaattca ctggccgtcg ttttacaacg tcgtgactgg gaaaaccctg 2100

gcgttaccca acttaatcgc cttgcagcac atcccccttt cgccagctgg cgtaatagcg 2160

aagaggcccg caccgatcgc ccttcccaac agttgcgcag cctgaatggc gaatgggacg 2220

cgccctgtag cggcgcatta agcgcggcgg gtgtggtggt tacgcgcagc gtgaccgcta 2280

cacttgccag cgccctagcg cccgctcctt tcgctttctt cccttccttt ctcgccacgt 2340

tcgccggctt tccccgtcaa gctctaaatc gggggctccc tttagggttc cgatttagtg 2400

ctttacggca cctcgacccc aaaaaacttg attagggtga tggttcacgt agtgggccat 2460

cgccctgata gacggttttt cgccctttga cgttggagtc cacgttcttt aatagtggac 2520

tcttgttcca aactggaaca acactcaacc ctatctcggt ctattctttt gatttataag 2580

ggattttgcc gatttcggcc tattggttaa aaaatgagct gatttaacaa aaatttaacg 2640

cgaattttaa caaaatatta acgcttacaa tttaggtggc acttttcggg gaaatgtgcg 2700

cggaacccct atttgtttat ttttctaaat acattcaaat atgtatccgc tcatgagaca 2760

ataaccctga taaatgcttc aataatattg aaaaaggaag agtatgagta ttcaacattt 2820

ccgtgtcgcc cttattccct tttttgcggc attttgcctt cctgtttttg ctcacccaga 2880

aacgctggtg aaagtaaaag atgctgaaga tcagttgggt gcacgagtgg gttacatcga 2940

actggatctc aacagcggta agatccttga gagttttcgc cccgaagaac gttttccaat 3000

gatgagcact tttaaagttc tgctatgtgg cgcggtatta tcccgtattg acgccgggca 3060

agagcaactc ggtcgccgca tacactattc tcagaatgac ttggttgagt actcaccagt 3120

cacagaaaag catcttacgg atggcatgac agtaagagaa ttatgcagtg ctgccataac 3180

catgagtgat aacactgcgg ccaacttact tctgacaacg atcggaggac cgaaggagct 3240

aaccgctttt ttgcacaaca tgggggatca tgtaactcgc cttgatcgtt gggaaccgga 3300

gctgaatgaa gccataccaa acgacgagcg tgacaccacg atgcctgtag caatggcaac 3360

aacgttgcgc aaactattaa ctggcgaact acttactcta gcttcccggc aacaattaat 3420

agactggatg gaggcggata aagttgcagg accacttctg cgctcggccc ttccggctgg 3480

ctggtttatt gctgataaat ctggagccgg tgagcgtggg tctcgcggta tcattgcagc 3540

actggggcca gatggtaagc cctcccgtat cgtagttatc tacacgacgg ggagtcaggc 3600

aactatggat gaacgaaata gacagatcgc tgagataggt gcctcactga ttaagcattg 3660

gtaactgtca gaccaagttt actcatatat actttagatt gatttaaaac ttcattttta 3720

atttaaaagg atctaggtga agatcctttt tgataatctc atgaccaaaa tcccttaacg 3780

tgagttttcg ttccactgag cgtcagaccc cgtagaaaag atcaaaggat cttcttgaga 3840

tccttttttt ctgcgcgtaa tctgctgctt gcaaacaaaa aaaccaccgc taccagcggt 3900

ggtttgtttg ccggatcaag agctaccaac tctttttccg aaggtaactg gcttcagcag 3960

agcgcagata ccaaatactg ttcttctagt gtagccgtag ttaggccacc acttcaagaa 4020

ctctgtagca ccgcctacat acctcgctct gctaatcctg ttaccagtgg ctgctgccag 4080

tggcgataag tcgtgtctta ccgggttgga ctcaagacga tagttaccgg ataaggcgca 4140

gcggtcgggc tgaacggggg gttcgtgcac acagcccagc ttggagcgaa cgacctacac 4200

cgaactgaga tacctacagc gtgagctatg agaaagcgcc acgcttcccg aagggagaaa 4260

ggcggacagg tatccggtaa gcggcagggt cggaacagga gagcgcacga gggagcttcc 4320

agggggaaac gcctggtatc tttatagtcc tgtcgggttt cgccacctct gacttgagcg 4380

tcgatttttg tgatgctcgt caggggggcg gagcctatgg aaaaacgcca gcaacgcggc 4440

ctttttacgg ttcctggcct tttgctggcc ttttgctcac atgttctttc ctgcgttatc 4500

ccctgattct gtggataacc gtattaccgc ctttgagtga gctgataccg ctcgccgcag 4560

ccgaacgacc gagcgcagcg agtcagtgag cgaggaagcg gaagagcgcc caatacgcaa 4620

accgcctctc cccgcgcgtt ggccgattca ttaatgcagc tggcacgaca ggtttcccga 4680

ctggaaagcg ggcagtgagc gcaacgcaat taatgtgagt tagctcactc attaggcacc 4740

ccaggcttta cactttatgc ttccggctcg tatgttgtgt ggaattgtga gcggataaca 4800

atttcacaca ggaaacagct atgaccatga ttacgccaga tttaattaag 4850

<210> 27
<211> 4892
<212> DNA
<213> Artificial Sequence

<220>
<223> Description of Artificial Sequence: Synthetic
      polynucleotide

<400> 27
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtgccgcca ccatggcttt 1080

acttactgcg gccgcccggc tcttgggaac caagaatgca tcttgtcttg ttcttgcagc 1140

ccggcatgct agtctgcgca ccgacatcga tgcgacctgc acgccccgcc gcgcaagttc 1200

gaaccaacgt ggcctcaacc agatttggaa tgtcaaaaag cagagtgtct atttgatgaa 1260

tttgaggaaa tctggaactt tgggccaccc aggctctcta gatgagacca cctatgaaag 1320

actagcagag gaaacgctgg actctttagc agagtttttt gaagaccttg cagacaagcc 1380

atacacgttt gaggactatg atgtctcctt tgggagtggt gtcttaactg tcaaactggg 1440

tggagatcta ggaacctatg tgatcaacaa gcagacgcca aacaagcaaa tctggctatc 1500

ttctccatcc agtggaccta agcgttatga ctggactggg aaaaactggg tgttctccca 1560

cgacggcgtg tccctccatg agctgctggc cgcagagctc actaaagcct taaaaaccaa 1620

actggacttg tcttggttgg cctattccgg aaaagatgct tatccttatg acgtgcctga 1680

ctatgccagc ctgggaggac cttgactcga ggacggggtg aactacgcct gaggatccga 1740

tctttttccc tctgccaaaa attatgggga catcatgaag ccccttgagc atctgacttc 1800

tggctaataa aggaaattta ttttcattgc aatagtgtgt tggaattttt tgtgtctctc 1860

actcggaagc aattcgttga tctgaatttc gaccacccat aatacccatt accctggtag 1920

ataagtagca tggcgggtta atcattaact acaaggaacc cctagtgatg gagttggcca 1980

ctccctctct gcgcgctcgc tcgctcactg aggccgggcg accaaaggtc gcccgacgcc 2040

cgggctttgc ccgggcggcc tcagtgagcg agcgagcgcg cagccttaat taacctaatt 2100

cactggccgt cgttttacaa cgtcgtgact gggaaaaccc tggcgttacc caacttaatc 2160

gccttgcagc acatccccct ttcgccagct ggcgtaatag cgaagaggcc cgcaccgatc 2220

gcccttccca acagttgcgc agcctgaatg gcgaatggga cgcgccctgt agcggcgcat 2280

taagcgcggc gggtgtggtg gttacgcgca gcgtgaccgc tacacttgcc agcgccctag 2340

cgcccgctcc tttcgctttc ttcccttcct ttctcgccac gttcgccggc tttccccgtc 2400

aagctctaaa tcgggggctc cctttagggt tccgatttag tgctttacgg cacctcgacc 2460

ccaaaaaact tgattagggt gatggttcac gtagtgggcc atcgccctga tagacggttt 2520

ttcgcccttt gacgttggag tccacgttct ttaatagtgg actcttgttc caaactggaa 2580

caacactcaa ccctatctcg gtctattctt ttgatttata agggattttg ccgatttcgg 2640

cctattggtt aaaaaatgag ctgatttaac aaaaatttaa cgcgaatttt aacaaaatat 2700

taacgcttac aatttaggtg gcacttttcg gggaaatgtg cgcggaaccc ctatttgttt 2760

atttttctaa atacattcaa atatgtatcc gctcatgaga caataaccct gataaatgct 2820

tcaataatat tgaaaaagga agagtatgag tattcaacat ttccgtgtcg cccttattcc 2880

cttttttgcg gcattttgcc ttcctgtttt tgctcaccca gaaacgctgg tgaaagtaaa 2940

agatgctgaa gatcagttgg gtgcacgagt gggttacatc gaactggatc tcaacagcgg 3000

taagatcctt gagagttttc gccccgaaga acgttttcca atgatgagca cttttaaagt 3060

tctgctatgt ggcgcggtat tatcccgtat tgacgccggg caagagcaac tcggtcgccg 3120

catacactat tctcagaatg acttggttga gtactcacca gtcacagaaa agcatcttac 3180

ggatggcatg acagtaagag aattatgcag tgctgccata accatgagtg ataacactgc 3240

ggccaactta cttctgacaa cgatcggagg accgaaggag ctaaccgctt ttttgcacaa 3300

catgggggat catgtaactc gccttgatcg ttgggaaccg gagctgaatg aagccatacc 3360

aaacgacgag cgtgacacca cgatgcctgt agcaatggca acaacgttgc gcaaactatt 3420

aactggcgaa ctacttactc tagcttcccg gcaacaatta atagactgga tggaggcgga 3480

taaagttgca ggaccacttc tgcgctcggc ccttccggct ggctggttta ttgctgataa 3540

atctggagcc ggtgagcgtg ggtctcgcgg tatcattgca gcactggggc cagatggtaa 3600

gccctcccgt atcgtagtta tctacacgac ggggagtcag gcaactatgg atgaacgaaa 3660

tagacagatc gctgagatag gtgcctcact gattaagcat tggtaactgt cagaccaagt 3720

ttactcatat atactttaga ttgatttaaa acttcatttt taatttaaaa ggatctaggt 3780

gaagatcctt tttgataatc tcatgaccaa aatcccttaa cgtgagtttt cgttccactg 3840

agcgtcagac cccgtagaaa agatcaaagg atcttcttga gatccttttt ttctgcgcgt 3900

aatctgctgc ttgcaaacaa aaaaaccacc gctaccagcg gtggtttgtt tgccggatca 3960

agagctacca actctttttc cgaaggtaac tggcttcagc agagcgcaga taccaaatac 4020

tgttcttcta gtgtagccgt agttaggcca ccacttcaag aactctgtag caccgcctac 4080

atacctcgct ctgctaatcc tgttaccagt ggctgctgcc agtggcgata agtcgtgtct 4140

taccgggttg gactcaagac gatagttacc ggataaggcg cagcggtcgg gctgaacggg 4200

gggttcgtgc acacagccca gcttggagcg aacgacctac accgaactga gatacctaca 4260

gcgtgagcta tgagaaagcg ccacgcttcc cgaagggaga aaggcggaca ggtatccggt 4320

aagcggcagg gtcggaacag gagagcgcac gagggagctt ccagggggaa acgcctggta 4380

tctttatagt cctgtcgggt ttcgccacct ctgacttgag cgtcgatttt tgtgatgctc 4440

gtcagggggg cggagcctat ggaaaaacgc cagcaacgcg gcctttttac ggttcctggc 4500

cttttgctgg ccttttgctc acatgttctt tcctgcgtta tcccctgatt ctgtggataa 4560

ccgtattacc gcctttgagt gagctgatac cgctcgccgc agccgaacga ccgagcgcag 4620

cgagtcagtg agcgaggaag cggaagagcg cccaatacgc aaaccgcctc tccccgcgcg 4680

ttggccgatt cattaatgca gctggcacga caggtttccc gactggaaag cgggcagtga 4740

gcgcaacgca attaatgtga gttagctcac tcattaggca ccccaggctt tacactttat 4800

gcttccggct cgtatgttgt gtggaattgt gagcggataa caatttcaca caggaaacag 4860

ctatgaccat gattacgcca gatttaatta ag 4892

<210> 28
<211> 4877
<212> DNA
<213> Artificial Sequence

<220>
<223> Description of Artificial Sequence: Synthetic
      polynucleotide

<400> 28

gccttaatta ggctgcgcgc tcgctcgctc actgaggccg cccgggcaaa gcccgggcgt 60
cgggcgacct ttggtcgccc ggcctcagtg agcgagcgag cgcgcagaga gggagtggcc 120
aactccatca ctaggggttc cttgtagtta atgattaacc cgccatgcta cttatctacc 180
agggtaatgg ggatcctcta gaactatagc tagtcgacat tgattattga ctagttatta 240
atagtaatca attacggggt cattagttca tagcccatat atggagttcc gcgttacata 300
acttacggta aatggcccgc ctggctgacc gcccaacgac ccccgcccat tgacgtcaat 360
aatgacgtat gttcccatag taacgccaat agggactttc cattgacgtc aatgggtgga 420
gtatttacgg taaactgccc acttggcagt acatcaagtg tatcatatgc caagtacgcc 480
ccctattgac gtcaatgacg gtaaatggcc cgcctggcat tatgcccagt acatgacctt 540
atgggacttt cctacttggc agtacatcta cgtattagtc atcgctatta ccatgtcgag 600
gccacgttct gcttcactct ccccatctcc cccccctccc cacccccaat tttgtattta 660
tttatttttt aattattttg tgcagcgatg ggggcggggg gggggggcgc gcgccaggcg 720
gggcggggcg gggcgagggg cggggcgggg cgaggcggag aggtgcggcg gcagccaatc 780
agagcggcgc gctccgaaag tttcctttta tggcgaggcg gcggcggcgg cggccctata 840
aaaagcgaag cgcgcggcgg gcgggagcaa gcttgaactg aaaaaccaga aagttaactg 900
gtaagtttag tctttttgtc ttttatttca ggtcccggat ccggtggtgg tgcaaatcaa 960
agaactgctc ctcagtggat gttgccttta cttctaggcc tgtacggaag tgttacttct 1020
gctctaaaag ctgcggaatt gtacccgcgg ccgatccacc ggtgccgcca ccatggcttt 1080
acttactgcg gccgcccggc tcttgggaac caagaatgca tcttgtcttg ttcttgcagc 1140
ccggcatgct agtctgcgca ccgacatcga tgcgacctgc acgccccgcc gcgcaagttc 1200
gaaccaacgt ggcctcaacc agatttggaa tgtcaaaaag cagagtgtct atttgatgaa 1260
tttgaggaaa tctggaactt tgggccaccc aggctctcta gatgagacca cctatgaaag 1320
actagcagag gaaacgctgg actctttagc agagtttttt gaagaccttg cagacaagcc 1380
atacacgttt gaggactatg atgtctcctt tgggagtggt gtcttaactg tcaaactggg 1440
tggagatcta ggaacctatg tgatcaacaa gcagacgcca aacaagcaaa tctggctatc 1500
ttctccatcc agtggaccta agcgttatga ctggactggg aaaaactggg tgttctccca 1560
cgacggcgtg tccctccatg agctgctggc cgcagagctc actaaagcct taaaaaccaa 1620
actggacttg tcttggttgg cctattccgg aaaagatgct tatccttatg acgtgcctga 1680
ctatgcctga ctcgaggacg gggtgaacta cgcctgagga tccgatcttt ttccctctgc 1740
caaaaattat ggggacatca tgaagcccct tgagcatctg acttctggct aataaaggaa 1800
atttattttc attgcaatag tgtgttggaa ttttttgtgt ctctcactcg gaagcaattc 1860
gttgatctga atttcgacca cccataatac ccattaccct ggtagataag tagcatggcg 1920

ggttaatcat taactacaag gaacccctag tgatggagtt ggccactccc tctctgcgcg 1980

ctcgctcgct cactgaggcc gggcgaccaa aggtcgcccg acgcccgggc tttgcccggg 2040

cggcctcagt gagcgagcga gcgcgcagcc ttaattaacc taattcactg gccgtcgttt 2100

tacaacgtcg tgactgggaa aaccctggcg ttacccaact taatcgcctt gcagcacatc 2160

cccctttcgc cagctggcgt aatagcgaag aggcccgcac cgatcgccct tcccaacagt 2220

tgcgcagcct gaatggcgaa tgggacgcgc cctgtagcgg cgcattaagc gcggcgggtg 2280

tggtggttac gcgcagcgtg accgctacac ttgccagcgc cctagcgccc gctcctttcg 2340

ctttcttccc ttcctttctc gccacgttcg ccggctttcc ccgtcaagct ctaaatcggg 2400

ggctcccttt agggttccga tttagtgctt tacggcacct cgaccccaaa aaacttgatt 2460

agggtgatgg ttcacgtagt gggccatcgc cctgatagac ggtttttcgc cctttgacgt 2520

tggagtccac gttctttaat agtggactct tgttccaaac tggaacaaca ctcaacccta 2580

tctcggtcta ttcttttgat ttataaggga ttttgccgat ttcggcctat tggttaaaaa 2640

atgagctgat ttaacaaaaa tttaacgcga attttaacaa aatattaacg cttacaattt 2700

aggtggcact tttcggggaa atgtgcgcgg aacccctatt tgtttatttt tctaaataca 2760

ttcaaatatg tatccgctca tgagacaata accctgataa atgcttcaat aatattgaaa 2820

aaggaagagt atgagtattc aacatttccg tgtcgccctt attccctttt ttgcggcatt 2880

ttgccttcct gtttttgctc acccagaaac gctggtgaaa gtaaaagatg ctgaagatca 2940

gttgggtgca cgagtgggtt acatcgaact ggatctcaac agcggtaaga tccttgagag 3000

ttttcgcccc gaagaacgtt ttccaatgat gagcactttt aaagttctgc tatgtggcgc 3060

ggtattatcc cgtattgacg ccgggcaaga gcaactcggt cgccgcatac actattctca 3120

gaatgacttg gttgagtact caccagtcac agaaaagcat cttacggatg gcatgacagt 3180

aagagaatta tgcagtgctg ccataaccat gagtgataac actgcggcca acttacttct 3240

gacaacgatc ggaggaccga aggagctaac cgcttttttg cacaacatgg gggatcatgt 3300

aactcgcctt gatcgttggg aaccggagct gaatgaagcc ataccaaacg acgagcgtga 3360

caccacgatg cctgtagcaa tggcaacaac gttgcgcaaa ctattaactg gcgaactact 3420

tactctagct tcccggcaac aattaataga ctggatggag gcggataaag ttgcaggacc 3480

acttctgcgc tcggcccttc cggctggctg gtttattgct gataaatctg gagccggtga 3540

gcgtgggtct cgcggtatca ttgcagcact ggggccagat ggtaagccct cccgtatcgt 3600

agttatctac acgacgggga gtcaggcaac tatggatgaa cgaaatagac agatcgctga 3660

gataggtgcc tcactgatta agcattggta actgtcagac caagtttact catatatact 3720

ttagattgat ttaaaacttc atttttaatt taaaaggatc taggtgaaga tcctttttga 3780

taatctcatg accaaaatcc cttaacgtga gttttcgttc cactgagcgt cagaccccgt 3840

agaaaagatc aaaggatctt cttgagatcc tttttttctg cgcgtaatct gctgcttgca 3900

aacaaaaaaa ccaccgctac cagcggtggt ttgtttgccg gatcaagagc taccaactct 3960

ttttccgaag gtaactggct tcagcagagc gcagatacca aatactgttc ttctagtgta 4020

gccgtagtta ggccaccact tcaagaactc tgtagcaccg cctacatacc tcgctctgct 4080

aatcctgtta ccagtggctg ctgccagtgg cgataagtcg tgtcttaccg ggttggactc 4140

aagacgatag ttaccggata aggcgcagcg gtcgggctga acggggggtt cgtgcacaca 4200

gcccagcttg gagcgaacga cctacaccga actgagatac ctacagcgtg agctatgaga 4260

aagcgccacg cttcccgaag ggagaaaggc ggacaggtat ccggtaagcg gcagggtcgg 4320

aacaggagag cgcacgaggg agcttccagg gggaaacgcc tggtatcttt atagtcctgt 4380

cgggtttcgc cacctctgac ttgagcgtcg atttttgtga tgctcgtcag gggggcggag 4440

cctatggaaa aacgccagca acgcggcctt tttacggttc ctggcctttt gctggccttt 4500

tgctcacatg ttctttcctg cgttatcccc tgattctgtg gataaccgta ttaccgcctt 4560

tgagtgagct gataccgctc gccgcagccg aacgaccgag cgcagcgagt cagtgagcga 4620

ggaagcggaa gagcgcccaa tacgcaaacc gcctctcccc gcgcgttggc cgattcatta 4680

atgcagctgg cacgacaggt ttcccgactg gaaagcgggc agtgagcgca acgcaattaa 4740

tgtgagttag ctcactcatt aggcacccca ggctttacac tttatgcttc cggctcgtat 4800

gttgtgtgga attgtgagcg gataacaatt tcacacagga aacagctatg accatgatta 4860

cgccagattt aattaag 4877

<210> 29
<211> 4892
<212> DNA
<213> Artificial Sequence

<220>
<223> Description of Artificial Sequence: Synthetic
      polynucleotide

<400> 29
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtgccgcca ccatgtggac 1080

tctcgggcgc cgcgcagtag ccggcctcct ggcgtcaccc agcccggccc aggcccagac 1140

cctcacccgg gtcccgcggc cggcagagtt ggccccactc tgcggccgcc gtggcctgcg 1200

caccgacatc gatgcgacct gcacgccccg ccgcgcaagt tcgaaccaac gtggcctcaa 1260

ccagatttgg aatgtcaaaa agcagagtgt ctatttgatg aatttgagga aatctggaac 1320

tttgggccac ccaggctctc tagatgagac cacctatgaa agactagcag aggaaacgct 1380

ggactcttta gcagagtttt ttgaagacct tgcagacaag ccatacacgt ttgaggacta 1440

tgatgtctcc tttgggagtg gtgtcttaac tgtcaaactg ggtggagatc taggaaccta 1500

tgtgatcaac aagcagacgc caaacaagca aatctggcta tcttctccat ccagtggacc 1560

taagcgttat gactggactg ggaaaaactg ggtgttctcc cacgacggcg tgtccctcca 1620

tgagctgctg gccgcagagc tcactaaagc cttaaaaacc aaactggact tgtcttggtt 1680

ggcctattcc ggaaaagatg cttgactcga ggacggggtg aactacgcct gaggatccga 1740

tctttttccc tctgccaaaa attatgggga catcatgaag ccccttgagc atctgacttc 1800

tggctaataa aggaaattta ttttcattgc aatagtgtgt tggaattttt tgtgtctctc 1860

actcggaagc aattcgttga tctgaatttc gaccacccat aatacccatt accctggtag 1920

ataagtagca tggcgggtta atcattaact acaaggaacc cctagtgatg gagttggcca 1980

ctccctctct gcgcgctcgc tcgctcactg aggccgggcg accaaaggtc gcccgacgcc 2040

cgggctttgc ccgggcggcc tcagtgagcg agcgagcgcg cagccttaat taacctaatt 2100

cactggccgt cgttttacaa cgtcgtgact gggaaaaccc tggcgttacc caacttaatc 2160

gccttgcagc acatccccct ttcgccagct ggcgtaatag cgaagaggcc cgcaccgatc 2220

gcccttccca acagttgcgc agcctgaatg gcgaatggga cgcgccctgt agcggcgcat 2280

taagcgcggc gggtgtggtg gttacgcgca gcgtgaccgc tacacttgcc agcgccctag 2340

cgcccgctcc tttcgctttc ttcccttcct ttctcgccac gttcgccggc tttccccgtc 2400

aagctctaaa tcgggggctc cctttagggt tccgatttag tgctttacgg cacctcgacc 2460

ccaaaaaact tgattagggt gatggttcac gtagtgggcc atcgccctga tagacggttt 2520

ttcgcccttt gacgttggag tccacgttct ttaatagtgg actcttgttc caaactggaa 2580

caacactcaa ccctatctcg gtctattctt ttgatttata agggattttg ccgatttcgg 2640

cctattggtt aaaaaatgag ctgatttaac aaaaatttaa cgcgaatttt aacaaaatat 2700

taacgcttac aatttaggtg gcacttttcg gggaaatgtg cgcggaaccc ctatttgttt 2760

atttttctaa atacattcaa atatgtatcc gctcatgaga caataaccct gataaatgct 2820

tcaataatat tgaaaaagga agagtatgag tattcaacat ttccgtgtcg cccttattcc 2880

cttttttgcg gcattttgcc ttcctgtttt tgctcaccca gaaacgctgg tgaaagtaaa 2940

agatgctgaa gatcagttgg gtgcacgagt gggttacatc gaactggatc tcaacagcgg 3000

taagatcctt gagagttttc gccccgaaga acgttttcca atgatgagca cttttaaagt 3060

tctgctatgt ggcgcggtat tatcccgtat tgacgccggg caagagcaac tcggtcgccg 3120

catacactat tctcagaatg acttggttga gtactcacca gtcacagaaa agcatcttac 3180

ggatggcatg acagtaagag aattatgcag tgctgccata accatgagtg ataacactgc 3240

ggccaactta cttctgacaa cgatcggagg accgaaggag ctaaccgctt ttttgcacaa 3300

catgggggat catgtaactc gccttgatcg ttgggaaccg gagctgaatg aagccatacc 3360

aaacgacgag cgtgacacca cgatgcctgt agcaatggca acaacgttgc gcaaactatt 3420

aactggcgaa ctacttactc tagcttcccg gcaacaatta atagactgga tggaggcgga 3480

taaagttgca ggaccacttc tgcgctcggc ccttccggct ggctggttta ttgctgataa 3540

atctggagcc ggtgagcgtg ggtctcgcgg tatcattgca gcactggggc cagatggtaa 3600

gccctcccgt atcgtagtta tctacacgac ggggagtcag gcaactatgg atgaacgaaa 3660

tagacagatc gctgagatag gtgcctcact gattaagcat tggtaactgt cagaccaagt 3720

ttactcatat atactttaga ttgatttaaa acttcatttt taatttaaaa ggatctaggt 3780

gaagatcctt tttgataatc tcatgaccaa aatcccttaa cgtgagtttt cgttccactg 3840

agcgtcagac cccgtagaaa agatcaaagg atcttcttga gatccttttt ttctgcgcgt 3900

aatctgctgc ttgcaaacaa aaaaaccacc gctaccagcg gtggtttgtt tgccggatca 3960

agagctacca actctttttc cgaaggtaac tggcttcagc agagcgcaga taccaaatac 4020

tgttcttcta gtgtagccgt agttaggcca ccacttcaag aactctgtag caccgcctac 4080

atacctcgct ctgctaatcc tgttaccagt ggctgctgcc agtggcgata agtcgtgtct 4140

taccgggttg gactcaagac gatagttacc ggataaggcg cagcggtcgg gctgaacggg 4200

gggttcgtgc acacagccca gcttggagcg aacgacctac accgaactga gatacctaca 4260

gcgtgagcta tgagaaagcg ccacgcttcc cgaagggaga aaggcggaca ggtatccggt 4320

aagcggcagg gtcggaacag gagagcgcac gagggagctt ccagggggaa acgcctggta 4380

tctttatagt cctgtcgggt ttcgccacct ctgacttgag cgtcgatttt tgtgatgctc 4440

gtcagggggg cggagcctat ggaaaaacgc cagcaacgcg gcctttttac ggttcctggc 4500

cttttgctgg ccttttgctc acatgttctt tcctgcgtta tcccctgatt ctgtggataa 4560

ccgtattacc gcctttgagt gagctgatac cgctcgccgc agccgaacga ccgagcgcag 4620

cgagtcagtg agcgaggaag cggaagagcg cccaatacgc aaaccgcctc tccccgcgcg 4680

ttggccgatt cattaatgca gctggcacga caggtttccc gactggaaag cgggcagtga 4740

gcgcaacgca attaatgtga gttagctcac tcattaggca ccccaggctt tacactttat 4800

gcttccggct cgtatgttgt gtggaattgt gagcggataa caatttcaca caggaaacag 4860

ctatgaccat gattacgcca gatttaatta ag 4892

<210> 30
<211> 4850
<212> DNA
<213> Artificial Sequence

<220>
<223> Description of Artificial Sequence: Synthetic
      polynucleotide

<400> 30
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtgccgcca ccatggcttt 1080

acttactgcg gccgcccggc tcttgggaac caagaatgca tcttgtcttg ttcttgcagc 1140

ccggcatgct agtctgcgaa cagatataga cgcaacgtgc acccccagaa gagcaagcag 1200

caaccagaga ggcctcaatc agatttggaa cgtcaagaaa cagagtgtct atctgatgaa 1260

cctccgcaaa tcagggaccc ttggtcatcc gggatctctt gatgaaacga cctatgaacg 1320

gctggctgag gagacgcttg actccctggc agagttcttc gaggatctgg ctgataagcc 1380

atacacgttc gaggactatg atgtttcttt tggttctggc gtcctgacgg tgaaactcgg 1440

aggggatctg ggcacttacg ttatcaacaa acagactccc aataaacaga tttggttgtc 1500

tagtcctagc agtggcccaa aacgctatga ctggacgggc aagaactggg tgtactccca 1560

cgatggagtt agcctgcacg aacttctggc cgctgagctt actaaagcgc tgaagactaa 1620

gctggatctc tccagcctcg catacagcgg caaagacgcc tagctcgagg acggggtgaa 1680

ctacgcctga ggatccgatc tttttccctc tgccaaaaat tatggggaca tcatgaagcc 1740

ccttgagcat ctgacttctg gctaataaag gaaatttatt ttcattgcaa tagtgtgttg 1800

gaattttttg tgtctctcac tcggaagcaa ttcgttgatc tgaatttcga ccacccataa 1860

tacccattac cctggtagat aagtagcatg gcgggttaat cattaactac aaggaacccc 1920

tagtgatgga gttggccact ccctctctgc gcgctcgctc gctcactgag gccgggcgac 1980

caaaggtcgc ccgacgcccg ggctttgccc gggcggcctc agtgagcgag cgagcgcgca 2040

gccttaatta acctaattca ctggccgtcg ttttacaacg tcgtgactgg gaaaaccctg 2100

gcgttaccca acttaatcgc cttgcagcac atcccccttt cgccagctgg cgtaatagcg 2160

aagaggcccg caccgatcgc ccttcccaac agttgcgcag cctgaatggc gaatgggacg 2220

cgccctgtag cggcgcatta agcgcggcgg gtgtggtggt tacgcgcagc gtgaccgcta 2280

cacttgccag cgccctagcg cccgctcctt tcgctttctt cccttccttt ctcgccacgt 2340

tcgccggctt tccccgtcaa gctctaaatc gggggctccc tttagggttc cgatttagtg 2400

ctttacggca cctcgacccc aaaaaacttg attagggtga tggttcacgt agtgggccat 2460

cgccctgata gacggttttt cgccctttga cgttggagtc cacgttcttt aatagtggac 2520

tcttgttcca aactggaaca acactcaacc ctatctcggt ctattctttt gatttataag 2580

ggattttgcc gatttcggcc tattggttaa aaaatgagct gatttaacaa aaatttaacg 2640

cgaattttaa caaaatatta acgcttacaa tttaggtggc acttttcggg gaaatgtgcg 2700

cggaacccct atttgtttat ttttctaaat acattcaaat atgtatccgc tcatgagaca 2760

ataaccctga taaatgcttc aataatattg aaaaaggaag agtatgagta ttcaacattt 2820

ccgtgtcgcc cttattccct tttttgcggc attttgcctt cctgtttttg ctcacccaga 2880

aacgctggtg aaagtaaaag atgctgaaga tcagttgggt gcacgagtgg gttacatcga 2940

actggatctc aacagcggta agatccttga gagttttcgc cccgaagaac gttttccaat 3000

gatgagcact tttaaagttc tgctatgtgg cgcggtatta tcccgtattg acgccgggca 3060

agagcaactc ggtcgccgca tacactattc tcagaatgac ttggttgagt actcaccagt 3120

cacagaaaag catcttacgg atggcatgac agtaagagaa ttatgcagtg ctgccataac 3180

catgagtgat aacactgcgg ccaacttact tctgacaacg atcggaggac cgaaggagct 3240

aaccgctttt ttgcacaaca tgggggatca tgtaactcgc cttgatcgtt gggaaccgga 3300

gctgaatgaa gccataccaa acgacgagcg tgacaccacg atgcctgtag caatggcaac 3360

aacgttgcgc aaactattaa ctggcgaact acttactcta gcttcccggc aacaattaat 3420

agactggatg gaggcggata aagttgcagg accacttctg cgctcggccc ttccggctgg 3480

ctggtttatt gctgataaat ctggagccgg tgagcgtggg tctcgcggta tcattgcagc 3540

actggggcca gatggtaagc cctcccgtat cgtagttatc tacacgacgg ggagtcaggc 3600

aactatggat gaacgaaata gacagatcgc tgagataggt gcctcactga ttaagcattg 3660

gtaactgtca gaccaagttt actcatatat actttagatt gatttaaaac ttcattttta 3720

atttaaaagg atctaggtga agatcctttt tgataatctc atgaccaaaa tcccttaacg 3780

tgagttttcg ttccactgag cgtcagaccc cgtagaaaag atcaaaggat cttcttgaga 3840

tccttttttt ctgcgcgtaa tctgctgctt gcaaacaaaa aaaccaccgc taccagcggt 3900

ggtttgtttg ccggatcaag agctaccaac tctttttccg aaggtaactg gcttcagcag 3960

agcgcagata ccaaatactg ttcttctagt gtagccgtag ttaggccacc acttcaagaa 4020

ctctgtagca ccgcctacat acctcgctct gctaatcctg ttaccagtgg ctgctgccag 4080

tggcgataag tcgtgtctta ccgggttgga ctcaagacga tagttaccgg ataaggcgca 4140

gcggtcgggc tgaacggggg gttcgtgcac acagcccagc ttggagcgaa cgacctacac 4200

cgaactgaga tacctacagc gtgagctatg agaaagcgcc acgcttcccg aagggagaaa 4260

ggcggacagg tatccggtaa gcggcagggt cggaacagga gagcgcacga gggagcttcc 4320

agggggaaac gcctggtatc tttatagtcc tgtcgggttt cgccacctct gacttgagcg 4380

tcgatttttg tgatgctcgt caggggggcg gagcctatgg aaaaacgcca gcaacgcggc 4440

ctttttacgg ttcctggcct tttgctggcc ttttgctcac atgttctttc ctgcgttatc 4500

ccctgattct gtggataacc gtattaccgc ctttgagtga gctgataccg ctcgccgcag 4560

ccgaacgacc gagcgcagcg agtcagtgag cgaggaagcg gaagagcgcc caatacgcaa 4620

accgcctctc cccgcgcgtt ggccgattca ttaatgcagc tggcacgaca ggtttcccga 4680

ctggaaagcg ggcagtgagc gcaacgcaat taatgtgagt tagctcactc attaggcacc 4740

ccaggcttta cactttatgc ttccggctcg tatgttgtgt ggaattgtga gcggataaca 4800

atttcacaca ggaaacagct atgaccatga ttacgccaga tttaattaag 4850

<210> 31
<211> 4892
<212> DNA
<213> Artificial Sequence

<220>
<223> Description of Artificial Sequence: Synthetic
      polynucleotide

<400> 31
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtgccgcca ccatgtggac 1080

tctcgggcga cgcgccgtag ccggtctcct ggcttcccca agccctgcac aggcacagac 1140

cctcacacgg gtcccacggc cagctgaatt ggccccactc tgcggtcgcc ggggcctgcg 1200

aaccgatata gacgcaacgt gcactccccg acgagcaagt tccaaccaga gagggctcaa 1260

ccagatttgg aacgtcaaga agcagagtgt ctatttgatg aatctgagaa aatccgggac 1320

ccttgggcac cccggatctc ttgatgaaac tacctatgaa aggctggccg aggagacgct 1380

ggactctctg gcagagttct tcgaggatct ggcagacaag ccatacacgt ttgaggacta 1440

tgatgtctct tttggatctg gcgtcctgac ggttaaactg ggaggggatc tcggaaccta 1500

cgttatcaac aaacagactc ccaataagca gatctggttg tctagtccat ccagtggtcc 1560

taaacgctat gactggacgg gcaagaactg ggtgtactcc cacgacggag ttagccttca 1620

tgaactgctg gccgcagagc tgactaaagc tcttaagact aaactggatc tctctagcct 1680

ggcctactcc gggaaagatg cctagctcga ggacggggtg aactacgcct gaggatccga 1740

tctttttccc tctgccaaaa attatgggga catcatgaag ccccttgagc atctgacttc 1800

tggctaataa aggaaattta ttttcattgc aatagtgtgt tggaattttt tgtgtctctc 1860

actcggaagc aattcgttga tctgaatttc gaccacccat aatacccatt accctggtag 1920

ataagtagca tggcgggtta atcattaact acaaggaacc cctagtgatg gagttggcca 1980

ctccctctct gcgcgctcgc tcgctcactg aggccgggcg accaaaggtc gcccgacgcc 2040

cgggctttgc ccgggcggcc tcagtgagcg agcgagcgcg cagccttaat taacctaatt 2100

cactggccgt cgttttacaa cgtcgtgact gggaaaaccc tggcgttacc caacttaatc 2160

gccttgcagc acatccccct ttcgccagct ggcgtaatag cgaagaggcc cgcaccgatc 2220

gcccttccca acagttgcgc agcctgaatg gcgaatggga cgcgccctgt agcggcgcat 2280

taagcgcggc gggtgtggtg gttacgcgca gcgtgaccgc tacacttgcc agcgccctag 2340

cgcccgctcc tttcgctttc ttcccttcct ttctcgccac gttcgccggc tttccccgtc 2400

aagctctaaa tcgggggctc cctttagggt tccgatttag tgctttacgg cacctcgacc 2460

ccaaaaaact tgattagggt gatggttcac gtagtgggcc atcgccctga tagacggttt 2520

ttcgcccttt gacgttggag tccacgttct ttaatagtgg actcttgttc caaactggaa 2580

caacactcaa ccctatctcg gtctattctt ttgatttata agggattttg ccgatttcgg 2640

cctattggtt aaaaaatgag ctgatttaac aaaaatttaa cgcgaatttt aacaaaatat 2700

taacgcttac aatttaggtg gcacttttcg gggaaatgtg cgcggaaccc ctatttgttt 2760

atttttctaa atacattcaa atatgtatcc gctcatgaga caataaccct gataaatgct 2820

tcaataatat tgaaaaagga agagtatgag tattcaacat ttccgtgtcg cccttattcc 2880

cttttttgcg gcattttgcc ttcctgtttt tgctcaccca gaaacgctgg tgaaagtaaa 2940

agatgctgaa gatcagttgg gtgcacgagt gggttacatc gaactggatc tcaacagcgg 3000

taagatcctt gagagttttc gccccgaaga acgttttcca atgatgagca cttttaaagt 3060

tctgctatgt ggcgcggtat tatcccgtat tgacgccggg caagagcaac tcggtcgccg 3120

catacactat tctcagaatg acttggttga gtactcacca gtcacagaaa agcatcttac 3180

ggatggcatg acagtaagag aattatgcag tgctgccata accatgagtg ataacactgc 3240

ggccaactta cttctgacaa cgatcggagg accgaaggag ctaaccgctt ttttgcacaa 3300

catgggggat catgtaactc gccttgatcg ttgggaaccg gagctgaatg aagccatacc 3360

aaacgacgag cgtgacacca cgatgcctgt agcaatggca acaacgttgc gcaaactatt 3420

aactggcgaa ctacttactc tagcttcccg gcaacaatta atagactgga tggaggcgga 3480

taaagttgca ggaccacttc tgcgctcggc ccttccggct ggctggttta ttgctgataa 3540

atctggagcc ggtgagcgtg ggtctcgcgg tatcattgca gcactggggc cagatggtaa 3600

gccctcccgt atcgtagtta tctacacgac ggggagtcag gcaactatgg atgaacgaaa 3660

tagacagatc gctgagatag gtgcctcact gattaagcat tggtaactgt cagaccaagt 3720

ttactcatat atactttaga ttgatttaaa acttcatttt taatttaaaa ggatctaggt 3780

gaagatcctt tttgataatc tcatgaccaa aatcccttaa cgtgagtttt cgttccactg 3840

agcgtcagac cccgtagaaa agatcaaagg atcttcttga gatccttttt ttctgcgcgt 3900

aatctgctgc ttgcaaacaa aaaaaccacc gctaccagcg gtggtttgtt tgccggatca 3960

agagctacca actctttttc cgaaggtaac tggcttcagc agagcgcaga taccaaatac 4020

tgttcttcta gtgtagccgt agttaggcca ccacttcaag aactctgtag caccgcctac 4080

atacctcgct ctgctaatcc tgttaccagt ggctgctgcc agtggcgata agtcgtgtct 4140

taccgggttg gactcaagac gatagttacc ggataaggcg cagcggtcgg gctgaacggg 4200

gggttcgtgc acacagccca gcttggagcg aacgacctac accgaactga gatacctaca 4260

gcgtgagcta tgagaaagcg ccacgcttcc cgaagggaga aaggcggaca ggtatccggt 4320

aagcggcagg gtcggaacag gagagcgcac gagggagctt ccagggggaa acgcctggta 4380

tctttatagt cctgtcgggt ttcgccacct ctgacttgag cgtcgatttt tgtgatgctc 4440

gtcagggggg cggagcctat ggaaaaacgc cagcaacgcg gcctttttac ggttcctggc 4500

cttttgctgg ccttttgctc acatgttctt tcctgcgtta tcccctgatt ctgtggataa 4560

ccgtattacc gcctttgagt gagctgatac cgctcgccgc agccgaacga ccgagcgcag 4620

cgagtcagtg agcgaggaag cggaagagcg cccaatacgc aaaccgcctc tccccgcgcg 4680

ttggccgatt cattaatgca gctggcacga caggtttccc gactggaaag cgggcagtga 4740

gcgcaacgca attaatgtga gttagctcac tcattaggca ccccaggctt tacactttat 4800

gcttccggct cgtatgttgt gtggaattgt gagcggataa caatttcaca caggaaacag 4860

ctatgaccat gattacgcca gatttaatta ag 4892

<210> 32
<211> 4892
<212> DNA
<213> Artificial Sequence

<220>
<223> Description of Artificial Sequence: Synthetic
      polynucleotide

<400> 32
gccttaatta ggctgcgcgc tcgctcgctc actgaggccg cccgggcaaa gcccgggcgt      60

cgggcgacct ttggtcgccc ggcctcagtg agcgagcgag cgcgcagaga gggagtggcc     120

aactccatca ctaggggttc cttgtagtta atgattaacc cgccatgcta cttatctacc     180

agggtaatgg ggatcctcta gaactatagc tagtcgacat tgattattga ctagttatta     240

atagtaatca attacggggt cattagttca tagcccatat atggagttcc gcgttacata     300

acttacggta aatggcccgc ctggctgacc gcccaacgac ccccgcccat tgacgtcaat     360

aatgacgtat gttcccatag taacgccaat agggactttc cattgacgtc aatgggtgga     420

gtatttacgg taaactgccc acttggcagt acatcaagtg tatcatatgc caagtacgcc     480

ccctattgac gtcaatgacg gtaaatggcc cgcctggcat tatgcccagt acatgacctt     540

atgggacttt cctacttggc agtacatcta cgtattagtc atcgctatta ccatgtcgag     600

gccacgttct gcttcactct ccccatctcc cccccctccc cacccccaat tttgtattta     660

tttatttttt aattattttg tgcagcgatg ggggcggggg gggggggcgc gcgccaggcg     720

gggcggggcg gggcgagggg cggggcgggg cgaggcggag aggtgcggcg gcagccaatc     780

agagcggcgc gctccgaaag tttcctttta tggcgaggcg gcggcggcgg cggccctata     840

aaaagcgaag cgcgcggcgg gcgggagcaa gcttgaactg aaaaaccaga aagttaactg     900

gtaagtttag tctttttgtc ttttatttca ggtcccggat ccggtggtgg tgcaaatcaa     960

agaactgctc ctcagtggat gttgccttta cttctaggcc tgtacggaag tgttacttct    1020

gctctaaaag ctgcggaatt gtacccgcgg ccgatccacc ggtgccgcca ccatgtggac    1080

tctggggcga cgcgccgtgg ccgggctcct ggcatcccca tcccctgcgc aggcgcaaac    1140

cctcacacgg gttccacggc cagctgaact ggccccactg tgcggccggc ggggcctgag    1200

aacagatatt gacgcaacgt gcactcccag aagagccagc agcaaccaga gggggttgaa    1260

tcagatctgg aacgtcaaga agcagtcagt gtacctgatg aatctccgaa aaagcgggac    1320

ccttggtcac cccggatctc tcgatgaaac cacatatgaa cggctggccg aggaaacgct    1380

ggactccctg gccgagttct tcgaggacct ggcagacaag ccgtacacct tcgaagacta    1440

tgatgtttca ttcggaagtg gcgtgctgac ggtcaaactg ggaggcgatc tgggaaccta    1500

cgttataaac aaacagactc ccaataaaca gatctggttg tctagtccat ccagcggtcc    1560

taaacgctat gactggacgg gcaagaactg ggtctactcc cacgacggag ttagccttca    1620

cgaactgttg gccgcagagc tgacaaaggc gcttaagacg aaactggatc tctctagcct    1680

ggcctactcc ggcaaagatg cctagctcga ggacggggtg aactacgcct gaggatccga    1740

tctttttccc tctgccaaaa attatgggga catcatgaag ccccttgagc atctgacttc 1800

tggctaataa aggaaattta ttttcattgc aatagtgtgt tggaattttt tgtgtctctc 1860

actcggaagc aattcgttga tctgaatttc gaccacccat aatacccatt accctggtag 1920

ataagtagca tggcgggtta atcattaact acaaggaacc cctagtgatg gagttggcca 1980

ctccctctct gcgcgctcgc tcgctcactg aggccgggcg accaaaggtc gcccgacgcc 2040

cgggctttgc ccgggcggcc tcagtgagcg agcgagcgcg cagccttaat taacctaatt 2100

cactggccgt cgttttacaa cgtcgtgact gggaaaaccc tggcgttacc caacttaatc 2160

gccttgcagc acatccccct ttcgccagct ggcgtaatag cgaagaggcc cgcaccgatc 2220

gcccttccca acagttgcgc agcctgaatg gcgaatggga cgcgccctgt agcggcgcat 2280

taagcgcggc gggtgtggtg gttacgcgca gcgtgaccgc tacacttgcc agcgccctag 2340

cgcccgctcc tttcgctttc ttcccttcct ttctcgccac gttcgccggc tttccccgtc 2400

aagctctaaa tcgggggctc cctttagggt tccgatttag tgctttacgg cacctcgacc 2460

ccaaaaaact tgattagggt gatggttcac gtagtgggcc atcgccctga tagacggttt 2520

ttcgcccttt gacgttggag tccacgttct ttaatagtgg actcttgttc caaactggaa 2580

caacactcaa ccctatctcg gtctattctt ttgatttata agggattttg ccgatttcgg 2640

cctattggtt aaaaaatgag ctgatttaac aaaaatttaa cgcgaatttt aacaaaatat 2700

taacgcttac aatttaggtg gcacttttcg gggaaatgtg cgcggaaccc ctatttgttt 2760

atttttctaa atacattcaa atatgtatcc gctcatgaga caataaccct gataaatgct 2820

tcaataatat tgaaaaagga agagtatgag tattcaacat ttccgtgtcg cccttattcc 2880

cttttttgcg gcattttgcc ttcctgtttt tgctcaccca gaaacgctgg tgaaagtaaa 2940

agatgctgaa gatcagttgg gtgcacgagt gggttacatc gaactggatc tcaacagcgg 3000

taagatcctt gagagttttc gccccgaaga acgttttcca atgatgagca cttttaaagt 3060

tctgctatgt ggcgcggtat tatcccgtat tgacgccggg caagagcaac tcggtcgccg 3120

catacactat tctcagaatg acttggttga gtactcacca gtcacagaaa agcatcttac 3180

ggatggcatg acagtaagag aattatgcag tgctgccata accatgagtg ataacactgc 3240

ggccaactta cttctgacaa cgatcggagg accgaaggag ctaaccgctt ttttgcacaa 3300

catgggggat catgtaactc gccttgatcg ttgggaaccg gagctgaatg aagccatacc 3360

aaacgacgag cgtgacacca cgatgcctgt agcaatggca acaacgttgc gcaaactatt 3420

aactggcgaa ctacttactc tagcttcccg gcaacaatta atagactgga tggaggcgga 3480

taaagttgca ggaccacttc tgcgctcggc ccttccggct ggctggttta ttgctgataa 3540

atctggagcc ggtgagcgtg ggtctcgcgg tatcattgca gcactggggc cagatggtaa 3600

gccctcccgt atcgtagtta tctacacgac ggggagtcag gcaactatgg atgaacgaaa 3660

tagacagatc gctgagatag gtgcctcact gattaagcat tggtaactgt cagaccaagt 3720

ttactcatat atactttaga ttgatttaaa acttcatttt taatttaaaa ggatctaggt 3780

gaagatcctt tttgataatc tcatgaccaa aatcccttaa cgtgagtttt cgttccactg 3840

agcgtcagac cccgtagaaa agatcaaagg atcttcttga gatccttttt ttctgcgcgt 3900

aatctgctgc ttgcaaacaa aaaaaccacc gctaccagcg gtggtttgtt tgccggatca 3960

agagctacca actctttttc cgaaggtaac tggcttcagc agagcgcaga taccaaatac 4020

tgttcttcta gtgtagccgt agttaggcca ccacttcaag aactctgtag caccgcctac 4080

atacctcgct ctgctaatcc tgttaccagt ggctgctgcc agtggcgata agtcgtgtct 4140

taccgggttg gactcaagac gatagttacc ggataaggcg cagcggtcgg gctgaacggg 4200

gggttcgtgc acacagccca gcttggagcg aacgacctac accgaactga gatacctaca 4260

gcgtgagcta tgagaaagcg ccacgcttcc cgaagggaga aaggcggaca ggtatccggt 4320

aagcggcagg gtcggaacag gagagcgcac gagggagctt ccagggggaa acgcctggta 4380

tctttatagt cctgtcgggt ttcgccacct ctgacttgag cgtcgatttt tgtgatgctc 4440

gtcagggggg cggagcctat ggaaaaacgc cagcaacgcg gcctttttac ggttcctggc 4500

cttttgctgg ccttttgctc acatgttctt tcctgcgtta tcccctgatt ctgtggataa 4560

ccgtattacc gcctttgagt gagctgatac cgctcgccgc agccgaacga ccgagcgcag 4620

cgagtcagtg agcgaggaag cggaagagcg cccaatacgc aaaccgcctc tccccgcgcg 4680

ttggccgatt cattaatgca gctggcacga caggtttccc gactggaaag cgggcagtga 4740

gcgcaacgca attaatgtga gttagctcac tcattaggca ccccaggctt tacactttat 4800

gcttccggct cgtatgttgt gtggaattgt gagcggataa caatttcaca caggaaacag 4860

ctatgaccat gattacgcca gatttaatta ag 4892

<210> 33
<211> 4892
<212> DNA
<213> Artificial Sequence

<220>
<223> Description of Artificial Sequence: Synthetic
      polynucleotide

<400> 33
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtgccgcca ccatgtggac 1080

actggggagg agggccgtcg ctgggctgct ggcttcacca tcacccgctc aggctcagac 1140

cctgacaaga gtgccaaggc cagcagagct ggccccactg tgcgggcgga gaggactgcg 1200

aaccgacatc gatgctactt gtaccccaag gcgagcaagc tccaaccagc gagggctgaa 1260

ccagatttgg aatgtgaaga aacagtctgt ctacctgatg aatctgagaa agagcggcac 1320

tctgggacac cctggcagcc tggacgagac cacctacgag cggctggccg aggaaaccct 1380

ggattccctg gccgagttct ttgaagacct ggctgataag ccatacacct tcgaagacta 1440

tgacgtgagc ttcggcagcg gcgtgctgac agtcaaactg ggcggggacc tgggaacata 1500

cgtgatcaac aagcagactc ctaacaagca gatttggctg tctagtccct caagcggccc 1560

taagaggtat gactggacag ggaaaaactg ggtgtatagt cacgatggcg tctcactgca 1620

tgagctgctg gccgctgaac tgactaaggc actgaaaact aaactggacc tgagctccct 1680

ggcttactct gggaaagacg cataactcga ggacggggtg aactacgcct gaggatccga 1740

tctttttccc tctgccaaaa attatgggga catcatgaag ccccttgagc atctgacttc 1800

tggctaataa aggaaattta ttttcattgc aatagtgtgt tggaattttt tgtgtctctc 1860

actcggaagc aattcgttga tctgaatttc gaccacccat aatacccatt accctggtag 1920

ataagtagca tggcgggtta atcattaact acaaggaacc cctagtgatg gagttggcca 1980

ctccctctct gcgcgctcgc tcgctcactg aggccgggcg accaaaggtc gcccgacgcc 2040

cgggctttgc ccgggcggcc tcagtgagcg agcgagcgcg cagccttaat taacctaatt 2100

cactggccgt cgttttacaa cgtcgtgact gggaaaaccc tggcgttacc caacttaatc 2160

gccttgcagc acatccccct ttcgccagct ggcgtaatag cgaagaggcc cgcaccgatc 2220

gcccttccca acagttgcgc agcctgaatg gcgaatggga cgcgccctgt agcggcgcat 2280

taagcgcggc gggtgtggtg gttacgcgca gcgtgaccgc tacacttgcc agcgccctag 2340

cgcccgctcc tttcgctttc ttcccttcct ttctcgccac gttcgccggc tttccccgtc 2400

aagctctaaa tcgggggctc cctttagggt tccgatttag tgctttacgg cacctcgacc 2460

ccaaaaaact tgattagggt gatggttcac gtagtgggcc atcgccctga tagacggttt 2520

ttcgcccttt gacgttggag tccacgttct ttaatagtgg actcttgttc caaactggaa 2580

caacactcaa ccctatctcg gtctattctt ttgatttata agggattttg ccgatttcgg 2640

cctattggtt aaaaaatgag ctgatttaac aaaaatttaa cgcgaatttt aacaaaatat 2700

taacgcttac aatttaggtg gcacttttcg gggaaatgtg cgcggaaccc ctatttgttt 2760

atttttctaa atacattcaa atatgtatcc gctcatgaga caataaccct gataaatgct 2820

tcaataatat tgaaaaagga agagtatgag tattcaacat ttccgtgtcg cccttattcc 2880

cttttttgcg gcattttgcc ttcctgtttt tgctcaccca gaaacgctgg tgaaagtaaa 2940

agatgctgaa gatcagttgg gtgcacgagt gggttacatc gaactggatc tcaacagcgg 3000

taagatcctt gagagttttc gccccgaaga acgttttcca atgatgagca cttttaaagt 3060

tctgctatgt ggcgcggtat tatcccgtat tgacgccggg caagagcaac tcggtcgccg 3120

catacactat tctcagaatg acttggttga gtactcacca gtcacagaaa agcatcttac 3180

ggatggcatg acagtaagag aattatgcag tgctgccata accatgagtg ataacactgc 3240

ggccaactta cttctgacaa cgatcggagg accgaaggag ctaaccgctt ttttgcacaa 3300

catgggggat catgtaactc gccttgatcg ttgggaaccg gagctgaatg aagccatacc 3360

aaacgacgag cgtgacacca cgatgcctgt agcaatggca acaacgttgc gcaaactatt 3420

aactggcgaa ctacttactc tagcttcccg gcaacaatta atagactgga tggaggcgga 3480

taaagttgca ggaccacttc tgcgctcggc ccttccggct ggctggttta ttgctgataa 3540

atctggagcc ggtgagcgtg ggtctcgcgg tatcattgca gcactggggc cagatggtaa 3600

gccctcccgt atcgtagtta tctacacgac ggggagtcag gcaactatgg atgaacgaaa 3660

tagacagatc gctgagatag gtgcctcact gattaagcat tggtaactgt cagaccaagt 3720

ttactcatat atactttaga ttgatttaaa acttcatttt taatttaaaa ggatctaggt 3780

gaagatcctt tttgataatc tcatgaccaa aatcccttaa cgtgagtttt cgttccactg 3840

agcgtcagac cccgtagaaa agatcaaagg atcttcttga gatccttttt ttctgcgcgt 3900

aatctgctgc ttgcaaacaa aaaaaccacc gctaccagcg gtggtttgtt tgccggatca 3960

agagctacca actctttttc cgaaggtaac tggcttcagc agagcgcaga taccaaatac 4020

tgttcttcta gtgtagccgt agttaggcca ccacttcaag aactctgtag caccgcctac 4080

atacctcgct ctgctaatcc tgttaccagt ggctgctgcc agtggcgata agtcgtgtct 4140

taccgggttg gactcaagac gatagttacc ggataaggcg cagcggtcgg gctgaacggg 4200

gggttcgtgc acacagccca gcttggagcg aacgacctac accgaactga gatacctaca 4260

gcgtgagcta tgagaaagcg ccacgcttcc cgaagggaga aaggcggaca ggtatccggt 4320

aagcggcagg gtcggaacag gagagcgcac gagggagctt ccagggggaa acgcctggta 4380

tctttatagt cctgtcgggt ttcgccacct ctgacttgag cgtcgatttt tgtgatgctc 4440

gtcagggggg cggagcctat ggaaaaacgc cagcaacgcg gcctttttac ggttcctggc 4500

cttttgctgg ccttttgctc acatgttctt tcctgcgtta tcccctgatt ctgtggataa 4560

ccgtattacc gcctttgagt gagctgatac cgctcgccgc agccgaacga ccgagcgcag 4620

cgagtcagtg agcgaggaag cggaagagcg cccaatacgc aaaccgcctc tccccgcgcg 4680

ttggccgatt cattaatgca gctggcacga caggtttccc gactggaaag cgggcagtga 4740

gcgcaacgca attaatgtga gttagctcac tcattaggca ccccaggctt tacactttat 4800

gcttccggct cgtatgttgt gtggaattgt gagcggataa caatttcaca caggaaacag 4860

ctatgaccat gattacgcca gatttaatta ag 4892

<210> 34
<211> 4892
<212> DNA
<213> Artificial Sequence

<220>
<223> Description of Artificial Sequence: Synthetic
      polynucleotide

<400> 34
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtgccgcca ccatgtggac 1080

actgggcaga agggccgtgg ctgggctgct ggcatctccc tcccccgctc aggctcagac 1140

actgacccgc gtccctcggc cagcagagct ggccccactg tgcgggcgga gaggactgcg 1200

aaccgacatc gatgctactt gtaccccaag gcgagcaagc tccaaccagc gagggctgaa 1260

ccagatttgg aatgtgaaga aacagtctgt ctacctgatg aatctgagaa agagcggcac 1320

tctgggacac cctggcagcc tggacgagac cacctacgag cggctggccg aggaaaccct 1380

ggattccctg gccgagttct ttgaagacct ggctgataag ccatacacct tcgaagacta 1440

tgacgtgagc ttcggcagcg gcgtgctgac agtcaaactg ggcggggacc tgggaacata 1500

cgtgatcaac aagcagactc ctaacaagca gatttggctg tctagtccct caagcggccc 1560

taagaggtac gactggacag ggaaaaactg ggtgtatagt cacgatggcg tctcactgca 1620

tgagctgctg gccgctgaac tgactaaagc cctgaaaact aaactggacc tgtcatccct 1680

ggcatactct ggcaaggacg cataactcga ggacggggtg aactacgcct gaggatccga 1740

tctttttccc tctgccaaaa attatgggga catcatgaag ccccttgagc atctgacttc 1800

tggctaataa aggaaattta ttttcattgc aatagtgtgt tggaattttt tgtgtctctc 1860

actcggaagc aattcgttga tctgaatttc gaccacccat aatacccatt accctggtag 1920

ataagtagca tggcgggtta atcattaact acaaggaacc cctagtgatg gagttggcca 1980

ctccctctct gcgcgctcgc tcgctcactg aggccgggcg accaaaggtc gcccgacgcc 2040

cgggctttgc ccgggcggcc tcagtgagcg agcgagcgcg cagccttaat taacctaatt 2100

cactggccgt cgttttacaa cgtcgtgact gggaaaaccc tggcgttacc caacttaatc 2160

gccttgcagc acatccccct ttcgccagct ggcgtaatag cgaagaggcc cgcaccgatc 2220

gcccttccca acagttgcgc agcctgaatg gcgaatggga cgcgccctgt agcggcgcat 2280

taagcgcggc gggtgtggtg gttacgcgca gcgtgaccgc tacacttgcc agcgccctag 2340

cgcccgctcc tttcgctttc ttcccttcct ttctcgccac gttcgccggc tttccccgtc 2400

aagctctaaa tcgggggctc cctttagggt tccgatttag tgctttacgg cacctcgacc 2460

ccaaaaaact tgattagggt gatggttcac gtagtgggcc atcgccctga tagacggttt 2520

ttcgcccttt gacgttggag tccacgttct ttaatagtgg actcttgttc caaactggaa 2580

caacactcaa ccctatctcg gtctattctt ttgatttata agggattttg ccgatttcgg 2640

cctattggtt aaaaaatgag ctgatttaac aaaaatttaa cgcgaatttt aacaaaatat 2700

taacgcttac aatttaggtg gcacttttcg gggaaatgtg cgcggaaccc ctatttgttt 2760

atttttctaa atacattcaa atatgtatcc gctcatgaga caataaccct gataaatgct 2820

tcaataatat tgaaaaagga agagtatgag tattcaacat ttccgtgtcg cccttattcc 2880

cttttttgcg gcattttgcc ttcctgtttt tgctcaccca gaaacgctgg tgaaagtaaa 2940

agatgctgaa gatcagttgg gtgcacgagt gggttacatc gaactggatc tcaacagcgg 3000

taagatcctt gagagttttc gccccgaaga acgttttcca atgatgagca cttttaaagt 3060

tctgctatgt ggcgcggtat tatcccgtat tgacgccggg caagagcaac tcggtcgccg 3120

catacactat tctcagaatg acttggttga gtactcacca gtcacagaaa agcatcttac 3180

ggatggcatg acagtaagag aattatgcag tgctgccata accatgagtg ataacactgc 3240

ggccaactta cttctgacaa cgatcggagg accgaaggag ctaaccgctt ttttgcacaa 3300

catgggggat catgtaactc gccttgatcg ttgggaaccg gagctgaatg aagccatacc 3360

aaacgacgag cgtgacacca cgatgcctgt agcaatggca acaacgttgc gcaaactatt 3420

aactggcgaa ctacttactc tagcttcccg gcaacaatta atagactgga tggaggcgga 3480

taaagttgca ggaccacttc tgcgctcggc ccttccggct ggctggttta ttgctgataa 3540

atctggagcc ggtgagcgtg ggtctcgcgg tatcattgca gcactggggc cagatggtaa 3600

gccctcccgt atcgtagtta tctacacgac ggggagtcag gcaactatgg atgaacgaaa 3660

tagacagatc gctgagatag gtgcctcact gattaagcat tggtaactgt cagaccaagt 3720

ttactcatat atactttaga ttgatttaaa acttcatttt taatttaaaa ggatctaggt 3780

gaagatcctt tttgataatc tcatgaccaa aatcccttaa cgtgagtttt cgttccactg 3840

agcgtcagac cccgtagaaa agatcaaagg atcttcttga gatccttttt ttctgcgcgt 3900

aatctgctgc ttgcaaacaa aaaaaccacc gctaccagcg gtggtttgtt tgccggatca 3960

agagctacca actctttttc cgaaggtaac tggcttcagc agagcgcaga taccaaatac 4020

tgttcttcta gtgtagccgt agttaggcca ccacttcaag aactctgtag caccgcctac 4080

atacctcgct ctgctaatcc tgttaccagt ggctgctgcc agtggcgata agtcgtgtct 4140

taccgggttg gactcaagac gatagttacc ggataaggcg cagcggtcgg gctgaacggg 4200

gggttcgtgc acacagccca gcttggagcg aacgacctac accgaactga gatacctaca 4260

gcgtgagcta tgagaaagcg ccacgcttcc cgaagggaga aaggcggaca ggtatccggt 4320

aagcggcagg gtcggaacag gagagcgcac gagggagctt ccagggggaa acgcctggta 4380

tctttatagt cctgtcgggt ttcgccacct ctgacttgag cgtcgatttt tgtgatgctc 4440

gtcagggggg cggagcctat ggaaaaacgc cagcaacgcg gcctttttac ggttcctggc 4500

cttttgctgg ccttttgctc acatgttctt tcctgcgtta tcccctgatt ctgtggataa 4560

ccgtattacc gcctttgagt gagctgatac cgctcgccgc agccgaacga ccgagcgcag 4620

cgagtcagtg agcgaggaag cggaagagcg cccaatacgc aaaccgcctc tccccgcgcg 4680

ttggccgatt cattaatgca gctggcacga caggtttccc gactggaaag cgggcagtga 4740

gcgcaacgca attaatgtga gttagctcac tcattaggca ccccaggctt tacactttat 4800

gcttccggct cgtatgttgt gtggaattgt gagcggataa caatttcaca caggaaacag 4860

ctatgaccat gattacgcca gatttaatta ag 4892

<210> 35
<211> 4892
<212> DNA
<213> Artificial Sequence

<220>
<223> Description of Artificial Sequence: Synthetic
      polynucleotide

<400> 35
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtgccgcca ccatgtggac 1080

actcgggagg agggccgtgg ctgggctgct tgcttcacca tcacccgcac aggctcagac 1140

cctgacaagg gtcccaaggc cagctgagct ggccccactg tgcggtcgga gaggactgcg 1200

aaccgatatc gatgctacgt gtaccccaag gcgagcaagc agcaaccagc gagggctgaa 1260

ccagatttgg aatgtcaaga aacagagtgt ctacctgatg aatctccgaa agtccggcac 1320

tctgggtcac cctggcagcc tggacgaaac gacctacgag cggctggctg aggaaaccct 1380

ggattccctg gccgagttct tcgaagacct ggctgataag ccatacacct tcgaagacta 1440

tgacgtgagt ttcggcagcg gcgtgctgac agtgaaactg ggcggggacc tgggaactta 1500

cgttatcaac aagcagactc ctaataagca gatttggctg tctagtccct caagcggccc 1560

aaaaaggtat gactggacag gcaaaaactg ggtgtactcc cacgatggag tcagtctgca 1620

tgagcttctg gccgctgaac ttactaaggc gctgaaaact aaactggatc tgagctccct 1680

cgcttacagc gggaaagacg cataactcga ggacggggtg aactacgcct gaggatccga 1740

tctttttccc tctgccaaaa attatgggga catcatgaag ccccttgagc atctgacttc 1800

tggctaataa aggaaattta ttttcattgc aatagtgtgt tggaattttt tgtgtctctc 1860

actcggaagc aattcgttga tctgaatttc gaccacccat aatacccatt accctggtag 1920

ataagtagca tggcgggtta atcattaact acaaggaacc cctagtgatg gagttggcca 1980

ctccctctct gcgcgctcgc tcgctcactg aggccgggcg accaaaggtc gcccgacgcc 2040

cgggctttgc ccgggcggcc tcagtgagcg agcgagcgcg cagccttaat taacctaatt 2100

cactggccgt cgttttacaa cgtcgtgact gggaaaaccc tggcgttacc caacttaatc 2160

gccttgcagc acatccccct ttcgccagct ggcgtaatag cgaagaggcc cgcaccgatc 2220

gcccttccca acagttgcgc agcctgaatg gcgaatggga cgcgccctgt agcggcgcat 2280

taagcgcggc gggtgtggtg gttacgcgca gcgtgaccgc tacacttgcc agcgccctag 2340

cgcccgctcc tttcgctttc ttcccttcct ttctcgccac gttcgccggc tttccccgtc 2400

aagctctaaa tcgggggctc cctttagggt tccgatttag tgctttacgg cacctcgacc 2460

ccaaaaaact tgattagggt gatggttcac gtagtgggcc atcgccctga tagacggttt 2520

ttcgcccttt gacgttggag tccacgttct ttaatagtgg actcttgttc caaactggaa 2580

caacactcaa ccctatctcg gtctattctt ttgatttata agggattttg ccgatttcgg 2640

cctattggtt aaaaaatgag ctgatttaac aaaaatttaa cgcgaatttt aacaaaatat 2700

taacgcttac aatttaggtg gcacttttcg gggaaatgtg cgcggaaccc ctatttgttt 2760

atttttctaa atacattcaa atatgtatcc gctcatgaga caataaccct gataaatgct 2820

tcaataatat tgaaaaagga agagtatgag tattcaacat ttccgtgtcg cccttattcc 2880

cttttttgcg gcattttgcc ttcctgtttt tgctcaccca gaaacgctgg tgaaagtaaa 2940

agatgctgaa gatcagttgg gtgcacgagt gggttacatc gaactggatc tcaacagcgg 3000

taagatcctt gagagttttc gccccgaaga acgttttcca atgatgagca cttttaaagt 3060

tctgctatgt ggcgcggtat tatcccgtat tgacgccggg caagagcaac tcggtcgccg 3120

catacactat tctcagaatg acttggttga gtactcacca gtcacagaaa agcatcttac 3180

ggatggcatg acagtaagag aattatgcag tgctgccata accatgagtg ataacactgc 3240

ggccaactta cttctgacaa cgatcggagg accgaaggag ctaaccgctt ttttgcacaa 3300

catgggggat catgtaactc gccttgatcg ttgggaaccg gagctgaatg aagccatacc 3360

aaacgacgag cgtgacacca cgatgcctgt agcaatggca acaacgttgc gcaaactatt 3420

aactggcgaa ctacttactc tagcttcccg gcaacaatta atagactgga tggaggcgga 3480

taaagttgca ggaccacttc tgcgctcggc ccttccggct ggctggttta ttgctgataa 3540

atctggagcc ggtgagcgtg ggtctcgcgg tatcattgca gcactggggc cagatggtaa 3600

gccctcccgt atcgtagtta tctacacgac ggggagtcag gcaactatgg atgaacgaaa 3660

tagacagatc gctgagatag gtgcctcact gattaagcat tggtaactgt cagaccaagt 3720

ttactcatat atactttaga ttgatttaaa acttcatttt taatttaaaa ggatctaggt 3780

gaagatcctt tttgataatc tcatgaccaa aatcccttaa cgtgagtttt cgttccactg 3840

agcgtcagac cccgtagaaa agatcaaagg atcttcttga gatccttttt ttctgcgcgt 3900

aatctgctgc ttgcaaacaa aaaaaccacc gctaccagcg gtggtttgtt tgccggatca 3960

agagctacca actctttttc cgaaggtaac tggcttcagc agagcgcaga taccaaatac 4020

tgttcttcta gtgtagccgt agttaggcca ccacttcaag aactctgtag caccgcctac 4080

atacctcgct ctgctaatcc tgttaccagt ggctgctgcc agtggcgata agtcgtgtct 4140

taccgggttg gactcaagac gatagttacc ggataaggcg cagcggtcgg gctgaacggg 4200

gggttcgtgc acacagccca gcttggagcg aacgacctac accgaactga gatacctaca 4260

gcgtgagcta tgagaaagcg ccacgcttcc cgaagggaga aaggcggaca ggtatccggt 4320

aagcggcagg gtcggaacag gagagcgcac gagggagctt ccagggggaa acgcctggta 4380

tctttatagt cctgtcgggt ttcgccacct ctgacttgag cgtcgatttt tgtgatgctc 4440

gtcagggggg cggagcctat ggaaaaacgc cagcaacgcg gcctttttac ggttcctggc 4500

cttttgctgg ccttttgctc acatgttctt tcctgcgtta tcccctgatt ctgtggataa 4560

ccgtattacc gcctttgagt gagctgatac cgctcgccgc agccgaacga ccgagcgcag 4620

cgagtcagtg agcgaggaag cggaagagcg cccaatacgc aaaccgcctc tccccgcgcg 4680

ttggccgatt cattaatgca gctggcacga caggtttccc gactggaaag cgggcagtga 4740

gcgcaacgca attaatgtga gttagctcac tcattaggca ccccaggctt tacactttat 4800

gcttccggct cgtatgttgt gtggaattgt gagcggataa caatttcaca caggaaacag 4860

ctatgaccat gattacgcca gatttaatta ag 4892

<210> 36
<211> 4892
<212> DNA
<213> Artificial Sequence

<220>
<223> Description of Artificial Sequence: Synthetic
      polynucleotide

<400> 36
gccttaatta ggctgcgcgc tcgctcgctc actgaggccg cccgggcaaa gcccgggcgt      60

cgggcgacct ttggtcgccc ggcctcagtg agcgagcgag cgcgcagaga gggagtggcc     120

aactccatca ctaggggttc cttgtagtta atgattaacc cgccatgcta cttatctacc     180

agggtaatgg ggatcctcta gaactatagc tagtcgacat tgattattga ctagttatta     240

atagtaatca attacggggt cattagttca tagcccatat atggagttcc gcgttacata     300

acttacggta aatggcccgc ctggctgacc gcccaacgac ccccgcccat tgacgtcaat     360

aatgacgtat gttcccatag taacgccaat agggactttc cattgacgtc aatgggtgga     420

gtatttacgg taaactgccc acttggcagt acatcaagtg tatcatatgc caagtacgcc     480

ccctattgac gtcaatgacg gtaaatggcc cgcctggcat tatgcccagt acatgacctt     540

atgggacttt cctacttggc agtacatcta cgtattagtc atcgctatta ccatgtcgag     600

gccacgttct gcttcactct ccccatctcc cccccctccc cacccccaat tttgtattta     660

tttatttttt aattattttg tgcagcgatg ggggcggggg gggggggcgc gcgccaggcg     720

gggcggggcg gggcgagggg cggggcgggg cgaggcggag aggtgcggcg gcagccaatc     780

agagcggcgc gctccgaaag tttcctttta tggcgaggcg gcggcggcgg cggccctata     840

aaaagcgaag cgcgcggcgg gcgggagcaa gcttgaactg aaaaaccaga aagttaactg     900

gtaagtttag tctttttgtc ttttatttca ggtcccggat ccggtggtgg tgcaaatcaa     960

agaactgctc ctcagtggat gttgccttta cttctaggcc tgtacggaag tgttacttct    1020

gctctaaaag ctgcggaatt gtacccgcgg ccgatccacc ggtgccgcca ccatgtggac    1080

actggggagg agggccgtcg ctgggctgct ggcttcacct tcacccgctc aggctcagac    1140

cctcacaaga gtgcctaggc cagcagagct ggccccactc tgcgggcgga gaggactgcg    1200

aaccgacatc gatgcaacat gtacccctag gcgagcaagc tccaaccagc gaggcctgaa    1260

ccagatttgg aatgtgaaga aacagagtgt ctacctgatg aatctgagaa agagcggcac    1320

tctgggacac ccaggcagcc tggacgagac cacctacgag cggctggcag aggaaaccct    1380

ggattctttg gccgagttct ttgaagacct tgctgataag ccatacacat tcgaagacta    1440

tgacgtcagt ttcggaagcg gcgtgttgac agtcaaactg ggcggagacc tgggaacata    1500

cgtgatcaac aagcagactc ctaacaagca aatttggctt tctagtccat caagtggacc    1560

taagaggtat gactggacag ggaagaactg ggtgtacagc cacgatggcg tctctctgca 1620

tgagctgctg gccgctgaac tgactaaggc actgaaaact aaactggacc tgtcatccct 1680

ggcttattct gggaaagacg cataactcga ggacggggtg aactacgcct gaggatccga 1740

tctttttccc tctgccaaaa attatgggga catcatgaag ccccttgagc atctgacttc 1800

tggctaataa aggaaattta ttttcattgc aatagtgtgt tggaattttt tgtgtctctc 1860

actcggaagc aattcgttga tctgaatttc gaccacccat aatacccatt accctggtag 1920

ataagtagca tggcgggtta atcattaact acaaggaacc cctagtgatg gagttggcca 1980

ctccctctct gcgcgctcgc tcgctcactg aggccgggcg accaaaggtc gcccgacgcc 2040

cgggctttgc ccgggcggcc tcagtgagcg agcgagcgcg cagccttaat taacctaatt 2100

cactggccgt cgttttacaa cgtcgtgact gggaaaaccc tggcgttacc caacttaatc 2160

gccttgcagc acatccccct ttcgccagct ggcgtaatag cgaagaggcc cgcaccgatc 2220

gcccttccca acagttgcgc agcctgaatg gcgaatggga cgcgccctgt agcggcgcat 2280

taagcgcggc gggtgtggtg gttacgcgca gcgtgaccgc tacacttgcc agcgccctag 2340

cgcccgctcc tttcgctttc ttcccttcct ttctcgccac gttcgccggc tttccccgtc 2400

aagctctaaa tcgggggctc cctttagggt tccgatttag tgctttacgg cacctcgacc 2460

ccaaaaaact tgattagggt gatggttcac gtagtgggcc atcgccctga tagacggttt 2520

ttcgcccttt gacgttggag tccacgttct ttaatagtgg actcttgttc caaactggaa 2580

caacactcaa ccctatctcg gtctattctt ttgatttata agggattttg ccgatttcgg 2640

cctattggtt aaaaaatgag ctgatttaac aaaaatttaa cgcgaatttt aacaaaatat 2700

taacgcttac aatttaggtg gcacttttcg gggaaatgtg cgcggaaccc ctatttgttt 2760

atttttctaa atacattcaa atatgtatcc gctcatgaga caataaccct gataaatgct 2820

tcaataatat tgaaaaagga agagtatgag tattcaacat ttccgtgtcg cccttattcc 2880

cttttttgcg gcattttgcc ttcctgtttt tgctcaccca gaaacgctgg tgaaagtaaa 2940

agatgctgaa gatcagttgg gtgcacgagt gggttacatc gaactggatc tcaacagcgg 3000

taagatcctt gagagttttc gccccgaaga acgttttcca atgatgagca cttttaaagt 3060

tctgctatgt ggcgcggtat tatcccgtat tgacgccggg caagagcaac tcggtcgccg 3120

catacactat tctcagaatg acttggttga gtactcacca gtcacagaaa agcatcttac 3180

ggatggcatg acagtaagag aattatgcag tgctgccata accatgagtg ataacactgc 3240

ggccaactta cttctgacaa cgatcggagg accgaaggag ctaaccgctt ttttgcacaa 3300

catgggggat catgtaactc gccttgatcg ttgggaaccg gagctgaatg aagccatacc 3360

aaacgacgag cgtgacacca cgatgcctgt agcaatggca acaacgttgc gcaaactatt 3420

aactggcgaa ctacttactc tagcttcccg gcaacaatta atagactgga tggaggcgga 3480

taaagttgca ggaccacttc tgcgctcggc ccttccggct ggctggttta ttgctgataa 3540

atctggagcc ggtgagcgtg ggtctcgcgg tatcattgca gcactggggc cagatggtaa 3600

gccctcccgt atcgtagtta tctacacgac ggggagtcag gcaactatgg atgaacgaaa 3660

tagacagatc gctgagatag gtgcctcact gattaagcat tggtaactgt cagaccaagt 3720

ttactcatat atactttaga ttgatttaaa acttcatttt taatttaaaa ggatctaggt 3780

gaagatcctt tttgataatc tcatgaccaa aatcccttaa cgtgagtttt cgttccactg 3840

agcgtcagac cccgtagaaa agatcaaagg atcttcttga gatccttttt ttctgcgcgt 3900

aatctgctgc ttgcaaacaa aaaaaccacc gctaccagcg gtggtttgtt tgccggatca 3960

agagctacca actctttttc cgaaggtaac tggcttcagc agagcgcaga taccaaatac 4020

tgttcttcta gtgtagccgt agttaggcca ccacttcaag aactctgtag caccgcctac 4080

atacctcgct ctgctaatcc tgttaccagt ggctgctgcc agtggcgata agtcgtgtct 4140

taccgggttg gactcaagac gatagttacc ggataaggcg cagcggtcgg gctgaacggg 4200

gggttcgtgc acacagccca gcttggagcg aacgacctac accgaactga gatacctaca 4260

gcgtgagcta tgagaaagcg ccacgcttcc cgaagggaga aaggcggaca ggtatccggt 4320

aagcggcagg gtcggaacag gagagcgcac gagggagctt ccagggggaa acgcctggta 4380

tctttatagt cctgtcgggt ttcgccacct ctgacttgag cgtcgatttt tgtgatgctc 4440

gtcagggggg cggagcctat ggaaaaacgc cagcaacgcg gcctttttac ggttcctggc 4500

cttttgctgg ccttttgctc acatgttctt tcctgcgtta tcccctgatt ctgtggataa 4560

ccgtattacc gcctttgagt gagctgatac cgctcgccgc agccgaacga ccgagcgcag 4620

cgagtcagtg agcgaggaag cggaagagcg cccaatacgc aaaccgcctc tccccgcgcg 4680

ttggccgatt cattaatgca gctggcacga caggtttccc gactggaaag cgggcagtga 4740

gcgcaacgca attaatgtga gttagctcac tcattaggca ccccaggctt tacactttat 4800

gcttccggct cgtatgttgt gtggaattgt gagcggataa caatttcaca caggaaacag 4860

ctatgaccat gattacgcca gatttaatta ag 4892

<210> 37
<211> 4865
<212> DNA
<213> Artificial Sequence

<220>
<223> Description of Artificial Sequence: Synthetic
      polynucleotide

<400> 37
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120
aactccatca ctaggggttc cttgtagtta atgattaacc cgccatgcta cttatctacc 180
agggtaatgg ggatcctcta gaactatagc tagtcgacat tgattattga ctagttatta 240
atagtaatca attacggggt cattagttca tagcccatat atggagttcc gcgttacata 300
acttacggta aatggcccgc ctggctgacc gcccaacgac ccccgcccat tgacgtcaat 360
aatgacgtat gttcccatag taacgccaat agggactttc cattgacgtc aatgggtgga 420
gtatttacgg taaactgccc acttggcagt acatcaagtg tatcatatgc caagtacgcc 480
ccctattgac gtcaatgacg gtaaatggcc cgcctggcat tatgcccagt acatgacctt 540
atgggacttt cctacttggc agtacatcta cgtattagtc atcgctatta ccatgtcgag 600
gccacgttct gcttcactct ccccatctcc cccccctccc cacccccaat tttgtattta 660
tttatttttt aattattttg tgcagcgatg ggggcggggg gggggggcgc gcgccaggcg 720
gggcggggcg gggcgagggg cggggcgggg cgaggcggag aggtgcggcg gcagccaatc 780
agagcggcgc gctccgaaag tttcctttta tggcgaggcg gcggcggcgg cggccctata 840
aaaagcgaag cgcgcggcgg gcgggagcaa gcttgaactg aaaaaccaga aagttaactg 900
gtaagtttag tctttttgtc ttttatttca ggtcccggat ccggtggtgg tgcaaatcaa 960
agaactgctc ctcagtggat gttgccttta cttctaggcc tgtacggaag tgttacttct 1020
gctctaaaag ctgcggaatt gtacccgcgg ccgatccacc ggtgccgcca ccatggctgt 1080
agctgcagta aaatgggtga tgtcaaagag aactatcttg aaacatttat ttccagtcca 1140
aaatggagct ttatattgtg tttgtcatct gcgcaccgac atcgatgcga cctgcacgcc 1200
ccgccgcgca agttcgaacc aacgtggcct caaccagatt tggaatgtca aaaagcagag 1260
tgtctatttg atgaatttga ggaaatctgg aactttgggc cacccaggct ctctagatga 1320
gaccacctat gaaagactag cagaggaaac gctggactct ttagcagagt tttttgaaga 1380
ccttgcagac aagccataca cgtttgagga ctatgatgtc tcctttggga gtggtgtctt 1440
aactgtcaaa ctgggtggag atctaggaac ctatgtgatc aacaagcaga cgccaaacaa 1500
gcaaatctgg ctatcttctc catccagtgg acctaagcgt tatgactgga ctgggaaaaa 1560
ctgggtgttc tcccacgacg gcgtgtccct ccatgagctg ctggccgcag agctcactaa 1620
agccttaaaa accaaactgg acttgtcttg gttggcctat tccggaaaag atgcttgact 1680
cgaggacggg gtgaactacg cctgaggatc cgatcttttt ccctctgcca aaaattatgg 1740
ggacatcatg aagccccttg agcatctgac ttctggctaa taaaggaaat ttattttcat 1800
tgcaatagtg tgttggaatt ttttgtgtct ctcactcgga agcaattcgt tgatctgaat 1860
ttcgaccacc cataataccc attaccctgg tagataagta gcatggcggg ttaatcatta 1920
actacaagga acccctagtg atggagttgg ccactccctc tctgcgcgct cgctcgctca 1980

ctgaggccgg gcgaccaaag gtcgcccgac gcccgggctt tgcccgggcg gcctcagtga 2040

gcgagcgagc gcgcagcctt aattaaccta attcactggc cgtcgtttta caacgtcgtg 2100

actgggaaaa ccctggcgtt acccaactta atcgccttgc agcacatccc cctttcgcca 2160

gctggcgtaa tagcgaagag gcccgcaccg atcgcccttc ccaacagttg cgcagcctga 2220

atggcgaatg ggacgcgccc tgtagcggcg cattaagcgc ggcgggtgtg gtggttacgc 2280

gcagcgtgac cgctacactt gccagcgccc tagcgcccgc tcctttcgct ttcttccctt 2340

cctttctcgc cacgttcgcc ggctttcccc gtcaagctct aaatcggggg ctccctttag 2400

ggttccgatt tagtgcttta cggcacctcg accccaaaaa acttgattag ggtgatggtt 2460

cacgtagtgg gccatcgccc tgatagacgg tttttcgccc tttgacgttg gagtccacgt 2520

tctttaatag tggactcttg ttccaaactg gaacaacact caaccctatc tcggtctatt 2580

cttttgattt ataagggatt ttgccgattt cggcctattg gttaaaaaat gagctgattt 2640

aacaaaaatt taacgcgaat tttaacaaaa tattaacgct tacaatttag gtggcacttt 2700

tcggggaaat gtgcgcggaa cccctatttg tttatttttc taaatacatt caaatatgta 2760

tccgctcatg agacaataac cctgataaat gcttcaataa tattgaaaaa ggaagagtat 2820

gagtattcaa catttccgtg tcgcccttat tccctttttt gcggcatttt gccttcctgt 2880

ttttgctcac ccagaaacgc tggtgaaagt aaaagatgct gaagatcagt tgggtgcacg 2940

agtgggttac atcgaactgg atctcaacag cggtaagatc cttgagagtt ttcgccccga 3000

agaacgtttt ccaatgatga gcacttttaa agttctgcta tgtggcgcgg tattatcccg 3060

tattgacgcc gggcaagagc aactcggtcg ccgcatacac tattctcaga atgacttggt 3120

tgagtactca ccagtcacag aaaagcatct tacggatggc atgacagtaa gagaattatg 3180

cagtgctgcc ataaccatga gtgataacac tgcggccaac ttacttctga caacgatcgg 3240

aggaccgaag gagctaaccg cttttttgca caacatgggg gatcatgtaa ctcgccttga 3300

tcgttgggaa ccggagctga atgaagccat accaaacgac gagcgtgaca ccacgatgcc 3360

tgtagcaatg gcaacaacgt tgcgcaaact attaactggc gaactactta ctctagcttc 3420

ccggcaacaa ttaatagact ggatggaggc ggataaagtt gcaggaccac ttctgcgctc 3480

ggcccttccg gctggctggt ttattgctga taaatctgga gccggtgagc gtgggtctcg 3540

cggtatcatt gcagcactgg ggccagatgg taagccctcc cgtatcgtag ttatctacac 3600

gacggggagt caggcaacta tggatgaacg aaatagacag atcgctgaga taggtgcctc 3660

actgattaag cattggtaac tgtcagacca agtttactca tatatacttt agattgattt 3720

aaaacttcat ttttaattta aaaggatcta ggtgaagatc ctttttgata atctcatgac 3780

caaaatccct taacgtgagt tttcgttcca ctgagcgtca gaccccgtag aaaagatcaa 3840

aggatcttct tgagatcctt tttttctgcg cgtaatctgc tgcttgcaaa caaaaaaacc 3900

accgctacca gcggtggttt gtttgccgga tcaagagcta ccaactcttt ttccgaaggt 3960

aactggcttc agcagagcgc agataccaaa tactgttctt ctagtgtagc cgtagttagg 4020

ccaccacttc aagaactctg tagcaccgcc tacatacctc gctctgctaa tcctgttacc 4080

agtggctgct gccagtggcg ataagtcgtg tcttaccggg ttggactcaa gacgatagtt 4140

accggataag gcgcagcggt cgggctgaac ggggggttcg tgcacacagc ccagcttgga 4200

gcgaacgacc tacaccgaac tgagatacct acagcgtgag ctatgagaaa gcgccacgct 4260

tcccgaaggg agaaaggcgg acaggtatcc ggtaagcggc agggtcggaa caggagagcg 4320

cacgagggag cttccagggg gaaacgcctg gtatctttat agtcctgtcg ggtttcgcca 4380

cctctgactt gagcgtcgat ttttgtgatg ctcgtcaggg gggcggagcc tatggaaaaa 4440

cgccagcaac gcggcctttt tacggttcct ggccttttgc tggccttttg ctcacatgtt 4500

ctttcctgcg ttatcccctg attctgtgga taaccgtatt accgcctttg agtgagctga 4560

taccgctcgc cgcagccgaa cgaccgagcg cagcgagtca gtgagcgagg aagcggaaga 4620

gcgcccaata cgcaaaccgc ctctccccgc gcgttggccg attcattaat gcagctggca 4680

cgacaggttt cccgactgga aagcgggcag tgagcgcaac gcaattaatg tgagttagct 4740

cactcattag gcaccccagg ctttacactt tatgcttccg gctcgtatgt tgtgtggaat 4800

tgtgagcgga taacaatttc acacaggaaa cagctatgac catgattacg ccagatttaa 4860

ttaag 4865

<210> 38
<211> 4865
<212> DNA
<213> Artificial Sequence

<220>
<223> Description of Artificial Sequence: Synthetic
      polynucleotide

<400> 38
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtgccgcca ccatggctgt 1080

agctgcagta aaatgggtga tgtcaaagag aactatcttg aaacatttat ttccagtcca 1140

aaatggagct ttatattgtg tttgtcatct gcgaaccgac atcgatgcaa catgtacccc 1200

taggcgagca agctccaacc agcgaggcct gaaccagatt tggaatgtga agaaacagag 1260

tgtctacctg atgaatctga gaaagagcgg cactctggga cacccaggca gcctggacga 1320

gaccacctac gagcggctgg cagaggaaac cctggattct ttggccgagt tctttgaaga 1380

ccttgctgat aagccataca cattcgaaga ctatgacgtc agtttcggaa gcggcgtgtt 1440

gacagtcaaa ctgggcggag acctgggaac atacgtgatc aacaagcaga ctcctaacaa 1500

gcaaatttgg ctttctagtc catcaagtgg acctaagagg tatgactgga cagggaagaa 1560

ctgggtgtac agccacgatg gcgtctctct gcatgagctg ctggccgctg aactgactaa 1620

ggcactgaaa actaaactgg acctgtcatc cctggcttat tctgggaaag acgcataact 1680

cgaggacggg gtgaactacg cctgaggatc cgatcttttt ccctctgcca aaaattatgg 1740

ggacatcatg aagccccttg agcatctgac ttctggctaa taaaggaaat ttattttcat 1800

tgcaatagtg tgttggaatt ttttgtgtct ctcactcgga agcaattcgt tgatctgaat 1860

ttcgaccacc cataataccc attaccctgg tagataagta gcatggcggg ttaatcatta 1920

actacaagga acccctagtg atggagttgg ccactccctc tctgcgcgct cgctcgctca 1980

ctgaggccgg gcgaccaaag gtcgcccgac gcccgggctt tgcccgggcg gcctcagtga 2040

gcgagcgagc gcgcagcctt aattaaccta attcactggc cgtcgtttta caacgtcgtg 2100

actgggaaaa ccctggcgtt acccaactta atcgccttgc agcacatccc cctttcgcca 2160

gctggcgtaa tagcgaagag gcccgcaccg atcgcccttc ccaacagttg cgcagcctga 2220

atggcgaatg ggacgcgccc tgtagcggcg cattaagcgc ggcgggtgtg gtggttacgc 2280

gcagcgtgac cgctacactt gccagcgccc tagcgcccgc tcctttcgct ttcttccctt 2340

cctttctcgc cacgttcgcc ggctttcccc gtcaagctct aaatcggggg ctccctttag 2400

ggttccgatt tagtgcttta cggcacctcg accccaaaaa acttgattag ggtgatggtt 2460

cacgtagtgg gccatcgccc tgatagacgg tttttcgccc tttgacgttg gagtccacgt 2520

tctttaatag tggactcttg ttccaaactg gaacaacact caaccctatc tcggtctatt 2580

cttttgattt ataagggatt ttgccgattt cggcctattg gttaaaaaat gagctgattt 2640

aacaaaaatt taacgcgaat tttaacaaaa tattaacgct tacaatttag gtggcacttt 2700

tcggggaaat gtgcgcggaa cccctatttg tttatttttc taaatacatt caaatatgta 2760

tccgctcatg agacaataac cctgataaat gcttcaataa tattgaaaaa ggaagagtat 2820

gagtattcaa catttccgtg tcgcccttat tccctttttt gcggcatttt gccttcctgt 2880

ttttgctcac ccagaaacgc tggtgaaagt aaaagatgct gaagatcagt tgggtgcacg 2940

agtgggttac atcgaactgg atctcaacag cggtaagatc cttgagagtt ttcgccccga 3000

agaacgtttt ccaatgatga gcacttttaa agttctgcta tgtggcgcgg tattatcccg 3060

tattgacgcc gggcaagagc aactcggtcg ccgcatacac tattctcaga atgacttggt 3120

tgagtactca ccagtcacag aaaagcatct tacggatggc atgacagtaa gagaattatg 3180

cagtgctgcc ataaccatga gtgataacac tgcggccaac ttacttctga caacgatcgg 3240

aggaccgaag gagctaaccg cttttttgca caacatgggg gatcatgtaa ctcgccttga 3300

tcgttgggaa ccggagctga atgaagccat accaaacgac gagcgtgaca ccacgatgcc 3360

tgtagcaatg gcaacaacgt tgcgcaaact attaactggc gaactactta ctctagcttc 3420

ccggcaacaa ttaatagact ggatggaggc ggataaagtt gcaggaccac ttctgcgctc 3480

ggcccttccg gctggctggt ttattgctga taaatctgga gccggtgagc gtgggtctcg 3540

cggtatcatt gcagcactgg ggccagatgg taagccctcc cgtatcgtag ttatctacac 3600

gacggggagt caggcaacta tggatgaacg aaatagacag atcgctgaga taggtgcctc 3660

actgattaag cattggtaac tgtcagacca agtttactca tatatacttt agattgattt 3720

aaaacttcat ttttaattta aaaggatcta ggtgaagatc ctttttgata atctcatgac 3780

caaaatccct taacgtgagt tttcgttcca ctgagcgtca gaccccgtag aaaagatcaa 3840

aggatcttct tgagatcctt tttttctgcg cgtaatctgc tgcttgcaaa caaaaaaacc 3900

accgctacca gcggtggttt gtttgccgga tcaagagcta ccaactcttt ttccgaaggt 3960

aactggcttc agcagagcgc agataccaaa tactgttctt ctagtgtagc cgtagttagg 4020

ccaccacttc aagaactctg tagcaccgcc tacatacctc gctctgctaa tcctgttacc 4080

agtggctgct gccagtggcg ataagtcgtg tcttaccggg ttggactcaa gacgatagtt 4140

accggataag gcgcagcggt cgggctgaac ggggggttcg tgcacacagc ccagcttgga 4200

gcgaacgacc tacaccgaac tgagatacct acagcgtgag ctatgagaaa gcgccacgct 4260

tcccgaaggg agaaaggcgg acaggtatcc ggtaagcggc agggtcggaa caggagagcg 4320

cacgagggag cttccagggg gaaacgcctg gtatctttat agtcctgtcg ggtttcgcca 4380

cctctgactt gagcgtcgat ttttgtgatg ctcgtcaggg gggcggagcc tatggaaaaa 4440

cgccagcaac gcggcctttt tacggttcct ggccttttgc tggccttttg ctcacatgtt 4500

ctttcctgcg ttatcccctg attctgtgga taaccgtatt accgcctttg agtgagctga 4560

taccgctcgc cgcagccgaa cgaccgagcg cagcgagtca gtgagcgagg aagcggaaga 4620

gcgcccaata cgcaaaccgc ctctccccgc gcgttggccg attcattaat gcagctggca 4680

cgacaggttt cccgactgga aagcgggcag tgagcgcaac gcaattaatg tgagttagct 4740

cactcattag gcaccccagg ctttacactt tatgcttccg gctcgtatgt tgtgtggaat 4800

tgtgagcgga taacaatttc acacaggaaa cagctatgac catgattacg ccagatttaa 4860

ttaag 4865

<210> 39
<211> 4841
<212> DNA
<213> Artificial Sequence

<220>
<223> Description of Artificial Sequence: Synthetic
      polynucleotide

<400> 39
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtgccgcca ccatgttgag 1080

ccgggcagtg tgcggcacca gcaggcagct ggctccggct ttggggtatc tgggctccag 1140

gcagctgcgc accgacatcg atgcgacctg cacgccccgc cgcgcaagtt cgaaccaacg 1200

tggcctcaac cagatttgga atgtcaaaaa gcagagtgtc tatttgatga atttgaggaa 1260

atctggaact ttgggccacc caggctctct agatgagacc acctatgaaa gactagcaga 1320

ggaaacgctg gactctttag cagagttttt tgaagacctt gcagacaagc catacacgtt 1380

tgaggactat gatgtctcct ttgggagtgg tgtcttaact gtcaaactgg gtggagatct 1440

aggaacctat gtgatcaaca agcagacgcc aaacaagcaa atctggctat cttctccatc 1500

cagtggacct aagcgttatg actggactgg gaaaaactgg gtgttctccc acgacggcgt 1560

gtccctccat gagctgctgg ccgcagagct cactaaagcc ttaaaaacca aactggactt 1620

gtcttggttg gcctattccg gaaaagatgc ttgactcgag gacggggtga actacgcctg 1680

aggatccgat ctttttccct ctgccaaaaa ttatggggac atcatgaagc cccttgagca 1740

tctgacttct ggctaataaa ggaaatttat tttcattgca atagtgtgtt ggaatttttt 1800

gtgtctctca ctcggaagca attcgttgat ctgaatttcg accacccata atacccatta 1860

ccctggtaga taagtagcat ggcgggttaa tcattaacta caaggaaccc ctagtgatgg 1920

agttggccac tccctctctg cgcgctcgct cgctcactga ggccgggcga ccaaaggtcg 1980

cccgacgccc gggctttgcc cgggcggcct cagtgagcga gcgagcgcgc agccttaatt 2040

aacctaattc actggccgtc gttttacaac gtcgtgactg ggaaaaccct ggcgttaccc 2100

aacttaatcg ccttgcagca catccccctt tcgccagctg gcgtaatagc gaagaggccc 2160

gcaccgatcg cccttcccaa cagttgcgca gcctgaatgg cgaatggacg cgccctgta 2220

gcggcgcatt aagcgcggcg ggtgtggtgg ttacgcgcag cgtgaccgct acacttgcca 2280

gcgccctagc gcccgctcct ttcgctttct tcccttcctt tctcgccacg ttcgccggct 2340

ttccccgtca agctctaaat cgggggctcc ctttagggtt ccgatttagt gctttacggc 2400

acctcgaccc caaaaaactt gattagggtg atggttcacg tagtgggcca tcgccctgat 2460

agacggtttt tcgccctttg acgttggagt ccacgttctt taatagtgga ctcttgttcc 2520

aaactggaac aacactcaac cctatctcgg tctattcttt tgatttataa gggattttgc 2580

cgatttcggc ctattggtta aaaaatgagc tgatttaaca aaaatttaac gcgaatttta 2640

acaaaatatt aacgcttaca atttaggtgg cacttttcgg ggaaatgtgc gcggaacccc 2700

tatttgttta tttttctaaa tacattcaaa tatgtatccg ctcatgagac aataaccctg 2760

ataaatgctt caataatatt gaaaaaggaa gagtatgagt attcaacatt tccgtgtcgc 2820

ccttattccc ttttttgcgg cattttgcct tcctgttttt gctcacccag aaacgctggt 2880

gaaagtaaaa gatgctgaag atcagttggg tgcacgagtg ggttacatcg aactggatct 2940

caacagcggt aagatccttg agagttttcg ccccgaagaa cgttttccaa tgatgagcac 3000

ttttaaagtt ctgctatgtg gcgcggtatt atcccgtatt gacgccgggc aagagcaact 3060

cggtcgccgc atacactatt ctcagaatga cttggttgag tactcaccag tcacagaaaa 3120

gcatcttacg gatggcatga cagtaagaga attatgcagt gctgccataa ccatgagtga 3180

taacactgcg gccaacttac ttctgacaac gatcggagga ccgaaggagc taaccgcttt 3240

tttgcacaac atgggggatc atgtaactcg ccttgatcgt tgggaaccgg agctgaatga 3300

agccatacca aacgacgagc gtgacaccac gatgcctgta gcaatggcaa caacgttgcg 3360

caaactatta actggcgaac tacttactct agcttcccgg caacaattaa tagactggat 3420

ggaggcggat aaagttgcag gaccacttct gcgctcggcc cttccggctg gctggtttat 3480

tgctgataaa tctggagccg gtgagcgtgg gtctcgcggt atcattgcag cactggggcc 3540

agatggtaag ccctcccgta tcgtagttat ctacacgacg gggagtcagg caactatgga 3600

tgaacgaaat agacagatcg ctgagatagg tgcctcactg attaagcatt ggtaactgtc 3660

agaccaagtt tactcatata tactttagat tgatttaaaa cttcattttt aatttaaaag 3720

gatctaggtg aagatccttt ttgataatct catgaccaaa atcccttaac gtgagttttc 3780

gttccactga gcgtcagacc ccgtagaaaa gatcaaagga tcttcttgag atcctttttt 3840

tctgcgcgta atctgctgct tgcaaacaaa aaaaccaccg ctaccagcgg tggtttgttt 3900

gccggatcaa gagctaccaa ctctttttcc gaaggtaact ggcttcagca gagcgcagat 3960

accaaatact gttcttctag tgtagccgta gttaggccac cacttcaaga actctgtagc 4020

accgcctaca tacctcgctc tgctaatcct gttaccagtg gctgctgcca gtggcgataa 4080

gtcgtgtctt accgggttgg actcaagacg atagttaccg gataaggcgc agcggtcggg 4140

ctgaacgggg ggttcgtgca cacagcccag cttggagcga acgacctaca ccgaactgag 4200

atacctacag cgtgagctat gagaaagcgc cacgcttccc gaagggagaa aggcggacag 4260

gtatccggta agcggcaggg tcggaacagg agagcgcacg agggagcttc cagggggaaa 4320

cgcctggtat ctttatagtc ctgtcgggtt tcgccacctc tgacttgagc gtcgattttt 4380

gtgatgctcg tcaggggggc ggagcctatg gaaaaacgcc agcaacgcgg cctttttacg 4440

gttcctggcc ttttgctggc cttttgctca catgttcttt cctgcgttat cccctgattc 4500

tgtggataac cgtattaccg cctttgagtg agctgatacc gctcgccgca gccgaacgac 4560

cgagcgcagc gagtcagtga gcgaggaagc ggaagagcgc ccaatacgca aaccgcctct 4620

ccccgcgcgt tggccgattc attaatgcag ctggcacgac aggtttcccg actggaaagc 4680

gggcagtgag cgcaacgcaa ttaatgtgag ttagctcact cattaggcac cccaggcttt 4740

acactttatg cttccggctc gtatgttgtg tggaattgtg agcggataac aatttcacac 4800

aggaaacagc tatgaccatg attacgccag atttaattaa g 4841

<210> 40
<211> 5950
<212> DNA
<213> Artificial Sequence

<220>
<223> Description of Artificial Sequence: Synthetic
      polynucleotide

<400> 40
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

ctatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgggtcg 600

aggtgagccc cacgttctgc ttcactctcc ccatctcccc cccctcccca cccccaattt 660

tgtatttatt tattttttaa ttattttgtg cagcgatggg ggcggggggg gggggggcgc 720

gcgccaggcg gggcggggcg gggcgagggg cggggcgggg cgaggcggag aggtgcggcg 780

gcagccaatc agagcggcgc gctccgaaag tttcctttta tggcgaggcg gcggcggcgg 840

cggccctata aaaagcgaag cgcgcggcgg gcgggagtcg ctgcgttgcc ttcgccccgt 900

gccccgctcc gcgccgcctc gcgccgcccg ccccggctct gactgaccgc gttactccca 960

caggtgagcg ggcgggacgg cccttctcct ccgggctgta attagcgctt ggtttaatga 1020

cggctcgttt cttttctgtg gctgcgtgaa agccttaaag ggctccggga gggccctttg 1080

tgcggggggg agcggctcgg ggggtgcgtg cgtgtgtgtg tgcgtgggga gcgccgcgtg 1140

cggcccgcgc tgcccggcgg ctgtgagcgc tgcgggcgcg gcgcggggct ttgtgcgctc 1200

cgcgtgtgcg cgaggggagc gcggccgggg gcggtgcccc gcggtgcggg ggggctgcga 1260

ggggaacaaa ggctgcgtgc ggggtgtgtg cgtggggggg tgagcagggg gtgtgggcgc 1320

ggcggtcggg ctgtaacccc cccctgcacc cccctccccg agttgctgag cacggcccgg 1380

cttcgggtgc ggggctccgt gcggggcgtg gcgcggggct cgccgtgccg ggcggggggt 1440
ggcggcaggt gggggtgccg ggcggggcgg ggccgcctcg ggccggggag ggctcggggg 1500
aggggcgcgg cggccccgga gcgccggcgg ctgtcgaggc gcggcgagcc gcagccattg 1560
ccttttatgg taatcgtgcg agagggcgca gggacttcct ttgtcccaaa tctggcggag 1620
ccgaaatctg ggaggcgccg ccgcaccccc tctagcgggc gcgggcgaag cggtgcggcg 1680
ccggcaggaa ggaaatgggc ggggagggcc ttcgtgcgtc gccgcgccgc cgtccccttc 1740
tccatctcca gcctcggggc tgccgcaggg ggacggctgc cttcgggggg gacggggcag 1800
ggcggggttc ggcttctggc gtgtgaccgg cggctctaga gcctctgcta accatgttca 1860
tgccttcttc tttttcctac agctcctggg caacgtgctg gttgttgtgc tgtctcatca 1920
ttttggcaaa gaattaagct tgaactgaaa aaccagaaag ttaactggta agtttagtct 1980
ttttgtcttt tatttcaggt cccggatccg gtggtggtgc aaatcaaaga actgctcctc 2040
agtggatgtt gcctttactt ctaggcctgt acggaagtgt tacttctgct ctaaaagctg 2100
cggaattgta cccgcggccg atccaccggt atgtggactc tcgggcgccg cgcagtagcc 2160
ggcctcctgg cgtcacccag cccagcccag gcccagaccc tcacccgggt cccgcggccg 2220
gcagagttgg ccccactctg cggccgccgt ggcctgcgca ccgacatcga tgcgacctgc 2280
acgccccgcc gcgcaagttc gaaccaacgt ggcctcaacc agatttggaa tgtcaaaaag 2340
cagagtgtct atttgatgaa tttgaggaaa tctggaactt tgggccaccc aggctctcta 2400
gatgagacca cctatgaaag actagcagag gaaacgctgg actctttagc agagtttttt 2460
gaagaccttg cagacaagcc atacacgttt gaggactatg atgtctcctt tgggagtggt 2520
gtcttaactg tcaaactggg tggagatcta ggaacctatg tgatcaacaa gcagacgcca 2580
aacaagcaaa tctggctatc ttctccatcc agtggaccta agcgttatga ctggactggg 2640
aaaaactggg tgtactccca cgacggcgtg tccctccatg agctgctggc cgcagagctc 2700
actaaagcct taaaaaccaa actggacttg tcttccttgg cctattccgg aaaagatgct 2760
tgactcgagg acggggtgaa ctacgcctga ggatccgatc tttttccctc tgccaaaaat 2820
tatggggaca tcatgaagcc ccttgagcat ctgacttctg gctaataaag gaaatttatt 2880
ttcattgcaa tagtgtgttg gaattttttg tgtctctcac tcggaagcaa ttcgttgatc 2940
tgaatttcga ccacccataa tacccattac cctggtagat aagtagcatg gcgggttaat 3000
cattaactac aaggaacccc tagtgatgga gttggccact ccctctctgc gcgctcgctc 3060
gctcactgag gccgggcgac caaaggtcgc ccgacgcccg ggctttgccc gggcggcctc 3120
agtgagcgag cgagcgcgca gccttaatta acctaattca ctggccgtcg ttttacaacg 3180
tcgtgactgg gaaaaccctg gcgttaccca acttaatcgc cttgcagcac atcccccttt 3240
cgccagctgg cgtaatagcg aagaggcccg caccgatcgc ccttcccaac agttgcgcag 3300

cctgaatggc gaatgggacg cgccctgtag cggcgcatta agcgcggcgg gtgtggtggt 3360

tacgcgcagc gtgaccgcta cacttgccag cgccctagcg cccgctcctt tcgctttctt 3420

cccttccttt ctcgccacgt tcgccggctt tccccgtcaa gctctaaatc gggggctccc 3480

tttagggttc cgatttagtg ctttacggca cctcgacccc aaaaaacttg attagggtga 3540

tggttcacgt agtgggccat cgccctgata gacggttttt cgccctttga cgttggagtc 3600

cacgttcttt aatagtggac tcttgttcca aactggaaca acactcaacc ctatctcggt 3660

ctattctttt gatttataag ggattttgcc gatttcggcc tattggttaa aaaatgagct 3720

gatttaacaa aaatttaacg cgaattttaa caaaatatta acgcttacaa tttaggtggc 3780

acttttcggg gaaatgtgcg cggaacccct atttgtttat ttttctaaat acattcaaat 3840

atgtatccgc tcatgagaca ataaccctga taaatgcttc aataatattg aaaaaggaag 3900

agtatgagta ttcaacattt ccgtgtcgcc cttattccct tttttgcggc attttgcctt 3960

cctgtttttg ctcacccaga aacgctggtg aaagtaaaag atgctgaaga tcagttgggt 4020

gcacgagtgg gttacatcga actggatctc aacagcggta agatccttga gagttttcgc 4080

cccgaagaac gttttccaat gatgagcact tttaaagttc tgctatgtgg cgcggtatta 4140

tcccgtattg acgccgggca agagcaactc ggtcgccgca tacactattc tcagaatgac 4200

ttggttgagt actcaccagt cacagaaaag catcttacgg atggcatgac agtaagagaa 4260

ttatgcagtg ctgccataac catgagtgat aacactgcgg ccaacttact tctgacaacg 4320

atcggaggac cgaaggagct aaccgctttt ttgcacaaca tgggggatca tgtaactcgc 4380

cttgatcgtt gggaaccgga gctgaatgaa gccataccaa acgacgagcg tgacaccacg 4440

atgcctgtag caatggcaac aacgttgcgc aaactattaa ctggcgaact acttactcta 4500

gcttcccggc aacaattaat agactggatg gaggcggata aagttgcagg accacttctg 4560

cgctcggccc ttccggctgg ctggtttatt gctgataaat ctggagccgg tgagcgtggg 4620

tctcgcggta tcattgcagc actggggcca gatggtaagc cctcccgtat cgtagttatc 4680

tacacgacgg ggagtcaggc aactatggat gaacgaaata gacagatcgc tgagataggt 4740

gcctcactga ttaagcattg gtaactgtca gaccaagttt actcatatat actttagatt 4800

gatttaaaac ttcattttta atttaaaagg atctaggtga agatcctttt tgataatctc 4860

atgaccaaaa tcccttaacg tgagttttcg ttccactgag cgtcagaccc cgtagaaaag 4920

atcaaaggat cttcttgaga tccttttttt ctgcgcgtaa tctgctgctt gcaaacaaaa 4980

aaaccaccgc taccagcggt ggtttgtttg ccggatcaag agctaccaac tctttttccg 5040

aaggtaactg gcttcagcag agcgcagata ccaaatactg ttcttctagt gtagccgtag 5100

ttaggccacc acttcaagaa ctctgtagca ccgcctacat acctcgctct gctaatcctg 5160

ttaccagtgg ctgctgccag tggcgataag tcgtgtctta ccgggttgga ctcaagacga 5220

tagttaccgg ataaggcgca gcggtcgggc tgaacggggg gttcgtgcac acagcccagc 5280

ttggagcgaa cgacctacac cgaactgaga tacctacagc gtgagctatg agaaagcgcc 5340

acgcttcccg aagggagaaa ggcggacagg tatccggtaa gcggcagggt cggaacagga 5400

gagcgcacga gggagcttcc agggggaaac gcctggtatc tttatagtcc tgtcgggttt 5460

cgccacctct gacttgagcg tcgatttttg tgatgctcgt caggggggcg gagcctatgg 5520

aaaaacgcca gcaacgcggc ctttttacgg ttcctggcct tttgctggcc ttttgctcac 5580

atgttctttc ctgcgttatc ccctgattct gtggataacc gtattaccgc ctttgagtga 5640

gctgataccg ctcgccgcag ccgaacgacc gagcgcagcg agtcagtgag cgaggaagcg 5700

gaagagcgcc caatacgcaa accgcctctc cccgcgcgtt ggccgattca ttaatgcagc 5760

tggcacgaca ggtttcccga ctggaaagcg ggcagtgagc gcaacgcaat taatgtgagt 5820

tagctcactc attaggcacc ccaggcttta cactttatgc ttccggctcg tatgttgtgt 5880

ggaattgtga gcggataaca atttcacaca ggaaacagct atgaccatga ttacgccaga 5940

tttaattaag 5950

<210> 41
<211> 4748
<212> DNA
<213> Artificial Sequence

<220>
<223> Description of Artificial Sequence: Synthetic
      polynucleotide

<400> 41
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagg ttgacattga ttattgacta gttattaata 240

gtaatcaatt acggggtcat tagttcatag cccatatatg gagttccgcg ttacataact 300

tacggtaaat ggcccgcctg gctgaccgcc caacgacccc cgcccattga cgtcaataat 360

gacgtatgtt cccatagtaa cgccaatagg gactttccat tgacgtcaat gggtggagta 420

tttacggtaa actgcccact tggcagtaca tcaagtgtat catatgccaa gtacgccccc 480

tattgacgtc aatgacggta aatggcccgc ctggcattat gcccagtaca tgaccttatg 540

ggactttcct acttggcagt acatctacgt attagtcatc gctattacca tggtgatgcg 600

gttttggcag tacatcaatg ggcgtggata gcggtttgac tcacggggat ttccaagtct 660

ccaccccatt gacgtcaatg ggagtttgtt ttggcaccaa aatcaacggg actttccaaa 720

atgtcgtaac aactccgccc cattgacgca aatgggcggt aggcgtgtac ggtgggaggt 780
ctatataagc agagctctct ggctaactag agaacccact gcttactggc ttatcgaaat 840
taatacgact cactataggg agacccaagc tggctagcgt ttaaacttcg ggagcaagct 900
tggtaccgag ctcggatccg ccgccaccat gtggactctc gggcgccgcg cagtagccgg 960
cctcctggcg tcacccagcc cagcccaggc ccagaccctc acccgggtcc cgcggccggc 1020
agagttggcc ccactctgcg gccgccgtgg cctgcgcacc gacatcgatg cgacctgcac 1080
gccccgccgc gcaagttcga accaacgtgg cctcaaccag atttggaatg tcaaaaagca 1140
gagtgtctat ttgatgaatt tgaggaaatc tggaactttg ggccacccag gctctctaga 1200
tgagaccacc tatgaaagac tagcagagga aacgctggac tctttagcag agttttttga 1260
agaccttgca gacaagccat acacgtttga ggactatgat gtctcctttg ggagtggtgt 1320
cttaactgtc aaactgggtg gagatctagg aacctatgtg atcaacaagc agacgccaaa 1380
caagcaaatc tggctatctt ctccatccag tggacctaag cgttatgact ggactgggaa 1440
aaactgggtg tactcccacg acggcgtgtc cctccatgag ctgctggccg cagagctcac 1500
taaagcctta aaaaccaaac tggacttgtc ttccttggcc tattccggaa aagatgcttg 1560
actcgaggac ggggtgaact acgcctgagg atccgatctt tttccctctg ccaaaaatta 1620
tggggacatc atgaagcccc ttgagcatct gacttctggc taataaagga aatttatttt 1680
cattgcaata gtgtgttgga attttttgtg tctctcactc ggaagcaatt cgttgatctg 1740
aatttcgacc acccataata cccattaccc tggtagataa gtagcatggc gggttaatca 1800
ttaactacaa ggaaccccta gtgatggagt tggccactcc ctctctgcgc gctcgctcgc 1860
tcactgaggc cgggcgacca aaggtcgccc gacgcccggg ctttgcccgg gcggcctcag 1920
tgagcgagcg agcgcgcagc cttaattaac ctaattcact ggccgtcgtt ttacaacgtc 1980
gtgactggga aaaccctggc gttacccaac ttaatcgcct tgcagcacat ccccctttcg 2040
ccagctggcg taatagcgaa gaggcccgca ccgatcgccc ttcccaacag ttgcgcagcc 2100
tgaatggcga atgggacgcg ccctgtagcg gcgcattaag cgcggcgggt gtggtggtta 2160
cgcgcagcgt gaccgctaca cttgccagcg ccctagcgcc cgctcctttc gctttcttcc 2220
cttcctttct cgccacgttc gccggctttc cccgtcaagc tctaaatcgg gggctccctt 2280
tagggttccg atttagtgct ttacggcacc tcgaccccaa aaaacttgat tagggtgatg 2340
gttcacgtag tgggccatcg ccctgataga cggtttttcg ccctttgacg ttggagtcca 2400
cgttctttaa tagtggactc ttgttccaaa ctggaacaac actcaaccct atctcggtct 2460
attcttttga tttataaggg attttgccga tttcggccta ttggttaaaa aatgagctga 2520
tttaacaaaa atttaacgcg aattttaaca aaatattaac gcttacaatt taggtggcac 2580
ttttcgggga aatgtgcgcg gaacccctat ttgtttattt ttctaaatac attcaaatat 2640

gtatccgctc atgagacaat aaccctgata aatgcttcaa taatattgaa aaaggaagag 2700

tatgagtatt caacatttcc gtgtcgccct tattcccttt tttgcggcat tttgccttcc 2760

tgtttttgct cacccagaaa cgctggtgaa agtaaaagat gctgaagatc agttgggtgc 2820

acgagtgggt tacatcgaac tggatctcaa cagcggtaag atccttgaga gttttcgccc 2880

cgaagaacgt tttccaatga tgagcacttt taaagttctg ctatgtggcg cggtattatc 2940

ccgtattgac gccgggcaag agcaactcgg tcgccgcata cactattctc agaatgactt 3000

ggttgagtac tcaccagtca cagaaaagca tcttacggat ggcatgacag taagagaatt 3060

atgcagtgct gccataacca tgagtgataa cactgcggcc aacttacttc tgacaacgat 3120

cggaggaccg aaggagctaa ccgctttttt gcacaacatg ggggatcatg taactcgcct 3180

tgatcgttgg gaaccggagc tgaatgaagc cataccaaac gacgagcgtg acaccacgat 3240

gcctgtagca atggcaacaa cgttgcgcaa actattaact ggcgaactac ttactctagc 3300

ttcccggcaa caattaatag actggatgga ggcggataaa gttgcaggac cacttctgcg 3360

ctcggccctt ccggctggct ggtttattgc tgataaatct ggagccggtg agcgtgggtc 3420

tcgcggtatc attgcagcac tggggccaga tggtaagccc tcccgtatcg tagttatcta 3480

cacgacgggg agtcaggcaa ctatggatga acgaaataga cagatcgctg agataggtgc 3540

ctcactgatt aagcattggt aactgtcaga ccaagtttac tcatatatac tttagattga 3600

tttaaaactt catttttaat ttaaaaggat ctaggtgaag atcctttttg ataatctcat 3660

gaccaaaatc ccttaacgtg agttttcgtt ccactgagcg tcagaccccg tagaaaagat 3720

caaaggatct tcttgagatc ctttttttct gcgcgtaatc tgctgcttgc aaacaaaaaa 3780

accaccgcta ccagcggtgg tttgtttgcc ggatcaagag ctaccaactc tttttccgaa 3840

ggtaactggc ttcagcagag cgcagatacc aaatactgtt cttctagtgt agccgtagtt 3900

aggccaccac ttcaagaact ctgtagcacc gcctacatac ctcgctctgc taatcctgtt 3960

accagtggct gctgccagtg gcgataagtc gtgtcttacc gggttggact caagacgata 4020

gttaccggat aaggcgcagc ggtcgggctg aacggggggt tcgtgcacac agcccagctt 4080

ggagcgaacg acctacaccg aactgagata cctacagcgt gagctatgag aaagcgccac 4140

gcttcccgaa gggagaaagg cggacaggta tccggtaagc ggcagggtcg gaacaggaga 4200

gcgcacgagg gagcttccag ggggaaacgc ctggtatctt tatagtcctg tcgggtttcg 4260

ccacctctga cttgagcgtc gatttttgtg atgctcgtca ggggggcgga gcctatggaa 4320

aaacgccagc aacgcggcct ttttacggtt cctggccttt tgctggcctt ttgctcacat 4380

gttctttcct gcgttatccc ctgattctgt ggataaccgt attaccgcct ttgagtgagc 4440

tgataccgct cgccgcagcc gaacgaccga gcgcagcgag tcagtgagcg aggaagcgga 4500

agagcgccca atacgcaaac cgcctctccc cgcgcgttgg ccgattcatt aatgcagctg 4560

gcacgacagg tttcccgact ggaaagcggg cagtgagcgc aacgcaatta atgtgagtta 4620

gctcactcat taggcacccc aggctttaca ctttatgctt ccggctcgta tgttgtgtgg 4680

aattgtgagc ggataacaat ttcacacagg aaacagctat gaccatgatt acgccagatt 4740

taattaag 4748

<210> 42
<211> 4910
<212> DNA
<213> Artificial Sequence

<220>
<223> Description of Artificial Sequence: Synthetic
      polynucleotide

<400> 42
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagg ttgacattga ttattgacta gttattaata 240

gtaatcaatt acggggtcat tagttcatag cccatatatg gagttccgcg ttacataact 300

tacggtaaat ggcccgcctg gctgaccgcc caacgacccc cgcccattga cgtcaataat 360

gacgtatgtt cccatagtaa cgccaatagg gactttccat tgacgtcaat gggtggagta 420

tttacggtaa actgcccact tggcagtaca tcaagtgtat catatgccaa gtacgccccc 480

tattgacgtc aatgacggta aatggcccgc ctggcattat gcccagtaca tgaccttatg 540

ggactttcct acttggcagt acatctacgt attagtcatc gctattacca tggtgatgcg 600

gttttggcag tacatcaatg ggcgtggata gcggtttgac tcacggggat ttccaagtct 660

ccaccccatt gacgtcaatg ggagtttgtt ttggcaccaa aatcaacggg actttccaaa 720

atgtcgtaac aactccgccc cattgacgca aatgggcggt aggcgtgtac ggtgggaggt 780

ctatataagc agagctctct ggctaactag agaacccact gcttactggc ttatcgaaat 840

taatacgact cactataggg agacccaagc tggctagcgt ttaaacttcg ggagcaagct 900

tgaactgaaa aaccagaaag ttaactggta agtttagtct ttttgtcttt tatttcaggt 960

cccggatccg gtggtggtgc aaatcaaaga actgctcctc agtggatgtt gcctttactt 1020

ctaggcctgt acggaagtgt tacttctgct ctaaaagctg cggaattgta cccgcggccg 1080

atccaccggt atgtggactc tcgggcgccg cgcagtagcc ggcctcctgg cgtcacccag 1140

cccagcccag gcccagaccc tcacccgggt cccgcggccg gcagagttgg ccccactctg 1200

cggccgccgt ggcctgcgca ccgacatcga tgcgacctgc acgccccgcc gcgcaagttc 1260

gaaccaacgt ggcctcaacc agatttggaa tgtcaaaaag cagagtgtct atttgatgaa 1320
tttgaggaaa tctggaactt tgggccaccc aggctctcta gatgagacca cctatgaaag 1380
actagcagag gaaacgctgg actctttagc agagtttttt gaagaccttg cagacaagcc 1440
atacacgttt gaggactatg atgtctcctt tgggagtggt gtcttaactg tcaaactggg 1500
tggagatcta ggaacctatg tgatcaacaa gcagacgcca aacaagcaaa tctggctatc 1560
ttctccatcc agtggaccta agcgttatga ctggactggg aaaaactggg tgtactccca 1620
cgacggcgtg tccctccatg agctgctggc cgcagagctc actaaagcct taaaaaccaa 1680
actggacttg tcttccttgg cctattccgg aaaagatgct tgactcgagg acggggtgaa 1740
ctacgcctga ggatccgatc tttttccctc tgccaaaaat tatggggaca tcatgaagcc 1800
ccttgagcat ctgacttctg gctaataaag gaaatttatt ttcattgcaa tagtgtgttg 1860
gaattttttg tgtctctcac tcggaagcaa ttcgttgatc tgaatttcga ccacccataa 1920
tacccattac cctggtagat aagtagcatg gcgggttaat cattaactac aaggaacccc 1980
tagtgatgga gttggccact ccctctctgc gcgctcgctc gctcactgag gccgggcgac 2040
caaaggtcgc ccgacgcccg ggctttgccc gggcggcctc agtgagcgag cgagcgcgca 2100
gccttaatta acctaattca ctggccgtcg ttttacaacg tcgtgactgg gaaaaccctg 2160
gcgttaccca acttaatcgc cttgcagcac atcccccttt cgccagctgg cgtaatagcg 2220
aagaggcccg caccgatcgc ccttcccaac agttgcgcag cctgaatggc gaatgggacg 2280
cgccctgtag cggcgcatta agcgcggcgg gtgtggtggt tacgcgcagc gtgaccgcta 2340
cacttgccag cgccctagcg cccgctcctt tcgctttctt cccttccttt ctcgccacgt 2400
tcgccggctt tccccgtcaa gctctaaatc gggggctccc tttagggttc cgatttagtg 2460
ctttacggca cctcgacccc aaaaaacttg attagggtga tggttcacgt agtgggccat 2520
cgccctgata gacggttttt cgccctttga cgttggagtc cacgttcttt aatagtggac 2580
tcttgttcca aactggaaca acactcaacc ctatctcggt ctattctttt gatttataag 2640
ggattttgcc gatttcggcc tattggttaa aaaatgagct gatttaacaa aaatttaacg 2700
cgaattttaa caaaatatta acgcttacaa tttaggtggc acttttcggg gaaatgtgcg 2760
cggaacccct atttgtttat ttttctaaat acattcaaat atgtatccgc tcatgagaca 2820
ataaccctga taaatgcttc aataatattg aaaaaggaag agtatgagta ttcaacattt 2880
ccgtgtcgcc cttattccct tttttgcggc attttgcctt cctgtttttg ctcacccaga 2940
aacgctggtg aaagtaaaag atgctgaaga tcagttgggt gcacgagtgg gttacatcga 3000
actggatctc aacagcggta agatccttga gagttttcgc cccgaagaac gttttccaat 3060
gatgagcact tttaaagttc tgctatgtgg cgcggtatta tcccgtattg acgccgggca 3120
agagcaactc ggtcgccgca tacactattc tcagaatgac ttggttgagt actcaccagt 3180

cacagaaaag catcttacgg atggcatgac agtaagagaa ttatgcagtg ctgccataac 3240

catgagtgat aacactgcgg ccaacttact tctgacaacg atcggaggac cgaaggagct 3300

aaccgctttt ttgcacaaca tgggggatca tgtaactcgc cttgatcgtt gggaaccgga 3360

gctgaatgaa gccataccaa acgacgagcg tgacaccacg atgcctgtag caatggcaac 3420

aacgttgcgc aaactattaa ctggcgaact acttactcta gcttcccggc aacaattaat 3480

agactggatg gaggcggata aagttgcagg accacttctg cgctcggccc ttccggctgg 3540

ctggtttatt gctgataaat ctggagccgg tgagcgtggg tctcgcggta tcattgcagc 3600

actggggcca gatggtaagc cctcccgtat cgtagttatc tacacgacgg ggagtcaggc 3660

aactatggat gaacgaaata gacagatcgc tgagataggt gcctcactga ttaagcattg 3720

gtaactgtca gaccaagttt actcatatat actttagatt gatttaaaac ttcattttta 3780

atttaaaagg atctaggtga agatcctttt tgataatctc atgaccaaaa tcccttaacg 3840

tgagttttcg ttccactgag cgtcagaccc cgtagaaaag atcaaaggat cttcttgaga 3900

tccttttttt ctgcgcgtaa tctgctgctt gcaaacaaaa aaaccaccgc taccagcggt 3960

ggtttgtttg ccggatcaag agctaccaac tctttttccg aaggtaactg gcttcagcag 4020

agcgcagata ccaaatactg ttcttctagt gtagccgtag ttaggccacc acttcaagaa 4080

ctctgtagca ccgcctacat acctcgctct gctaatcctg ttaccagtgg ctgctgccag 4140

tggcgataag tcgtgtctta ccgggttgga ctcaagacga tagttaccgg ataaggcgca 4200

gcggtcgggc tgaacggggg gttcgtgcac acagcccagc ttggagcgaa cgacctacac 4260

cgaactgaga tacctacagc gtgagctatg agaaagcgcc acgcttcccg aagggagaaa 4320

ggcggacagg tatccggtaa gcggcagggt cggaacagga gagcgcacga gggagcttcc 4380

agggggaaac gcctggtatc tttatagtcc tgtcgggttt cgccacctct gacttgagcg 4440

tcgatttttg tgatgctcgt caggggggcg gagcctatgg aaaaacgcca gcaacgcggc 4500

ctttttacgg ttcctggcct tttgctggcc ttttgctcac atgttctttc ctgcgttatc 4560

ccctgattct gtggataacc gtattaccgc ctttgagtga gctgataccg ctcgccgcag 4620

ccgaacgacc gagcgcagcg agtcagtgag cgaggaagcg gaagagcgcc caatacgcaa 4680

accgcctctc cccgcgcgtt ggccgattca ttaatgcagc tggcacgaca ggtttcccga 4740

ctggaaagcg ggcagtgagc gcaacgcaat taatgtgagt tagctcactc attaggcacc 4800

ccaggcttta cactttatgc ttccggctcg tatgttgtgt ggaattgtga gcggataaca 4860

atttcacaca ggaaacagct atgaccatga ttacgccaga tttaattaag 4910

<210> 43
<211> 4910
<212> DNA

<213> Artificial Sequence

<220>
<223> Description of Artificial Sequence: Synthetic
      polynucleotide

<400> 43
gccttaatta ggctgcgcgc tcgctcgctc actgaggccg cccgggcaaa gcccgggcgt      60

cgggcgacct ttggtcgccc ggcctcagtg agcgagcgag cgcgcagaga gggagtggcc     120

aactccatca ctaggggttc cttgtagtta atgattaacc cgccatgcta cttatctacc     180

agggtaatgg ggatcctcta gaactatagg ttgacattga ttattgacta gttattaata     240

gtaatcaatt acggggtcat tagttcatag cccatatatg gagttccgcg ttacataact     300

tacggtaaat ggcccgcctg gctgaccgcc caacgacccc cgcccattga cgtcaataat     360

gacgtatgtt cccatagtaa cgccaatagg gactttccat tgacgtcaat gggtggagta     420

tttacggtaa actgcccact tggcagtaca tcaagtgtat catatgccaa gtacgccccc     480

tattgacgtc aatgacggta aatggcccgc ctggcattat gcccagtaca tgaccttatg     540

ggactttcct acttggcagt acatctacgt attagtcatc gctattacca tggtgatgcg     600

gttttggcag tacatcaatg ggcgtggata gcggtttgac tcacggggat ttccaagtct     660

ccaccccatt gacgtcaatg ggagtttgtt ttggcaccaa aatcaacggg actttccaaa     720

atgtcgtaac aactccgccc cattgacgca aatgggcggt aggcgtgtac ggtgggaggt     780

ctatataagc agagctctct ggctaactag agaacccact gcttactggc ttatcgaaat     840

taatacgact cactataggg agacccaagc tggctagcgt ttaaacttcg ggagcaagct     900

tgaactgaaa aaccagaaag ttaactggta agtttagtct ttttgtcttt tatttcaggt     960

cccggatccg gtggtggtgc aaatcaaaga actgctcctc agtggatgtt gcctttactt    1020

ctaggcctgt acggaagtgt tacttctgct ctaaaagctg cggaattgta cccgcggccg    1080

atccaccggt atgtggactc tgggtcgcag agcggtggca ggcttgctgg catctccgtc    1140

cccggctcag gcccagaccc tgacacgggt gcccaggccg gccgagctgg ctcctctgtg    1200

cggccggcgc ggccttagaa cagacattga cgcaacctgc acccccagaa gagccagttc    1260

caaccagcgc gggttgaatc agatctggaa cgtcaagaag cagagtgtgt accttatgaa    1320

tctccggaaa agtggcaccc tcggacaccc cggatccctc gatgaaacca cttatgagcg    1380

cctggccgag gaaaccctgg attccctggc tgagttcttc gaggacctgg ctgacaaacc    1440

ctacaccttc gaagactacg acgtttcctt cggaagtggg gtgctgacgg tcaaacttgg    1500

tggcgatctt ggaacctatg ttataaacaa gcagactccc aacaaacaga tctggctcag    1560

ctctccaagt tcaggaccca agaggtatga ctggacgggc aagaattggg tctattctca    1620

cgacggagtg agccttcatg aactgttggc tgccgaactg acaaaagccc ttaagacgaa    1680

actggacctg tcctcattgg cgtactctgg taaggatgcc taactcgagg acggggtgaa 1740

ctacgcctga ggatccgatc tttttccctc tgccaaaaat tatggggaca tcatgaagcc 1800

ccttgagcat ctgacttctg gctaataaag gaaatttatt ttcattgcaa tagtgtgttg 1860

gaattttttg tgtctctcac tcggaagcaa ttcgttgatc tgaatttcga ccacccataa 1920

tacccattac cctggtagat aagtagcatg gcgggttaat cattaactac aaggaacccc 1980

tagtgatgga gttggccact ccctctctgc gcgctcgctc gctcactgag gccgggcgac 2040

caaaggtcgc ccgacgcccg ggctttgccc gggcggcctc agtgagcgag cgagcgcgca 2100

gccttaatta acctaattca ctggccgtcg ttttacaacg tcgtgactgg gaaaaccctg 2160

gcgttaccca acttaatcgc cttgcagcac atcccccttt cgccagctgg cgtaatagcg 2220

aagaggcccg caccgatcgc ccttcccaac agttgcgcag cctgaatggc gaatgggacg 2280

cgccctgtag cggcgcatta agcgcggcgg gtgtggtggt tacgcgcagc gtgaccgcta 2340

cacttgccag cgccctagcg cccgctcctt tcgctttctt cccttccttt ctcgccacgt 2400

tcgccggctt tccccgtcaa gctctaaatc gggggctccc tttagggttc cgatttagtg 2460

ctttacggca cctcgacccc aaaaaacttg attagggtga tggttcacgt agtgggccat 2520

cgccctgata gacggttttt cgccctttga cgttggagtc cacgttcttt aatagtggac 2580

tcttgttcca aactggaaca acactcaacc ctatctcggt ctattctttt gatttataag 2640

ggattttgcc gatttcggcc tattggttaa aaaatgagct gatttaacaa aaatttaacg 2700

cgaattttaa caaaatatta acgcttacaa tttaggtggc acttttcggg gaaatgtgcg 2760

cggaacccct atttgtttat ttttctaaat acattcaaat atgtatccgc tcatgagaca 2820

ataaccctga taaatgcttc aataatattg aaaaaggaag agtatgagta ttcaacattt 2880

ccgtgtcgcc cttattccct tttttgcggc attttgcctt cctgtttttg ctcacccaga 2940

aacgctggtg aaagtaaaag atgctgaaga tcagttgggt gcacgagtgg gttacatcga 3000

actggatctc aacagcggta agatccttga gagttttcgc cccgaagaac gttttccaat 3060

gatgagcact tttaaagttc tgctatgtgg cgcggtatta tcccgtattg acgccgggca 3120

agagcaactc ggtcgccgca tacactattc tcagaatgac ttggttgagt actcaccagt 3180

cacagaaaag catcttacgg atggcatgac agtaagagaa ttatgcagtg ctgccataac 3240

catgagtgat aacactgcgg ccaacttact tctgacaacg atcggaggac cgaaggagct 3300

aaccgctttt ttgcacaaca tgggggatca tgtaactcgc cttgatcgtt gggaaccgga 3360

gctgaatgaa gccataccaa acgacgagcg tgacaccacg atgcctgtag caatggcaac 3420

aacgttgcgc aaactattaa ctggcgaact acttactcta gcttcccggc aacaattaat 3480

agactggatg gaggcggata aagttgcagg accacttctg cgctcggccc ttccggctgg 3540

ctggtttatt gctgataaat ctggagccgg tgagcgtggg tctcgcggta tcattgcagc 3600

actggggcca gatggtaagc cctcccgtat cgtagttatc tacacgacgg ggagtcaggc 3660

aactatggat gaacgaaata gacagatcgc tgagataggt gcctcactga ttaagcattg 3720

gtaactgtca gaccaagttt actcatatat actttagatt gatttaaaac ttcattttta 3780

atttaaaagg atctaggtga agatcctttt tgataatctc atgaccaaaa tcccttaacg 3840

tgagttttcg ttccactgag cgtcagaccc cgtagaaaag atcaaaggat cttcttgaga 3900

tccttttttt ctgcgcgtaa tctgctgctt gcaaacaaaa aaaccaccgc taccagcggt 3960

ggtttgtttg ccggatcaag agctaccaac tctttttccg aaggtaactg gcttcagcag 4020

agcgcagata ccaaatactg ttcttctagt gtagccgtag ttaggccacc acttcaagaa 4080

ctctgtagca ccgcctacat acctcgctct gctaatcctg ttaccagtgg ctgctgccag 4140

tggcgataag tcgtgtctta ccgggttgga ctcaagacga tagttaccgg ataaggcgca 4200

gcggtcgggc tgaacggggg gttcgtgcac acagcccagc ttggagcgaa cgacctacac 4260

cgaactgaga tacctacagc gtgagctatg agaaagcgcc acgcttcccg aagggagaaa 4320

ggcggacagg tatccggtaa gcggcagggt cggaacagga gagcgcacga gggagcttcc 4380

agggggaaac gcctggtatc tttatagtcc tgtcgggttt cgccacctct gacttgagcg 4440

tcgatttttg tgatgctcgt caggggggcg gagcctatgg aaaaacgcca gcaacgcggc 4500

ctttttacgg ttcctggcct tttgctggcc ttttgctcac atgttctttc ctgcgttatc 4560

ccctgattct gtggataacc gtattaccgc ctttgagtga gctgataccg ctcgccgcag 4620

ccgaacgacc gagcgcagcg agtcagtgag cgaggaagcg gaagagcgcc caatacgcaa 4680

accgcctctc cccgcgcgtt ggccgattca ttaatgcagc tggcacgaca ggtttcccga 4740

ctggaaagcg ggcagtgagc gcaacgcaat taatgtgagt tagctcactc attaggcacc 4800

ccaggcttta cactttatgc ttccggctcg tatgttgtgt ggaattgtga gcggataaca 4860

atttcacaca ggaaacagct atgaccatga ttacgccaga tttaattaag 4910

<210> 44
<211> 4910
<212> DNA
<213> Artificial Sequence

<220>
<223> Description of Artificial Sequence: Synthetic
      polynucleotide

<400> 44
   gccttaatta ggctgcgcgc tcgctcgctc actgaggccg cccgggcaaa gcccgggcgt 60
   cgggcgacct ttggtcgccc ggcctcagtg agcgagcgag cgcgcagaga gggagtggcc 120
   aactccatca ctaggggttc cttgtagtta atgattaacc cgccatgcta cttatctacc 180
   agggtaatgg ggatcctcta gaactatagg ttgacattga ttattgacta gttattaata 240
   gtaatcaatt acggggtcat tagttcatag cccatatatg gagttccgcg ttacataact 300
   tacggtaaat ggcccgcctg gctgaccgcc caacgacccc cgcccattga cgtcaataat 360
   gacgtatgtt cccatagtaa cgccaatagg gactttccat tgacgtcaat gggtggagta 420
   tttacggtaa actgcccact tggcagtaca tcaagtgtat catatgccaa gtacgccccc 480
   tattgacgtc aatgacggta aatggcccgc ctggcattat gcccagtaca tgaccttatg 540
   ggactttcct acttggcagt acatctacgt attagtcatc gctattacca tggtgatgcg 600
   gttttggcag tacatcaatg ggcgtggata gcggtttgac tcacggggat ttccaagtct 660
   ccaccccatt gacgtcaatg ggagtttgtt ttggcaccaa aatcaacggg actttccaaa 720
   atgtcgtaac aactccgccc cattgacgca aatgggcggt aggcgtgtac ggtgggaggt 780
   ctatataagc agagctctct ggctaactag agaacccact gcttactggc ttatcgaaat 840
   taatacgact cactataggg agacccaagc tggctagcgt ttaaacttcg ggagcaagct 900
   tgaactgaaa aaccagaaag ttaactggta agtttagtct ttttgtcttt tatttcaggt 960
   cccggatccg gtggtggtgc aaatcaaaga actgctcctc agtggatgtt gcctttactt 1020
   ctaggcctgt acggaagtgt tacttctgct ctaaaagctg cggaattgta cccgcggccg 1080
   atccaccggt atgtggactc tggggaggcg cgccgttgcc ggactcctgg catctccaag 1140
   cccagcgcag gctcaaaccc tcacgcgcgt tcccaggcca gccgagttgg cccccctttg 1200
   tggcaggcgc ggcctgagga cagatatcga cgccacatgc actcctcgcc gcgcctccag 1260
   caatcaaagg ggtttgaacc aaatttggaa cgtcaagaag caatcagtgt atctcatgaa 1320
   tctgagaaag agcggcacac tcggtcatcc cggaagtctg gacgaaacca catacgaacg 1380
   gctcgccgag gaaacgctgg atagcctggc cgagtttttc gaggacctgg ctgataaacc 1440
   gtatactttc gaagattatg acgtgtcatt cgggagcggc gtactgaccg tcaaattggg 1500
   cggtgacctg ggacatacgt tgattaacaa gcagactcct aataaacaga tttggttgag 1560
   ctctccttcc agcgggccta aacgctatga ttggacaggg aagaattggg tttacagtca 1620
   cgacggagtt agccttcacg agctcttggc agcggagctg acaaaggcgc tcaagaccaa 1680
   actcgacctg tcttcactcg cctattcagg caaagacgcc tgactcgagg acggggtgaa 1740
   ctacgcctga ggatccgatc tttttccctc tgccaaaaat tatggggaca tcatgaagcc 1800
   ccttgagcat ctgacttctg gctaataaag gaaatttatt ttcattgcaa tagtgtgttg 1860
   gaattttttg tgtctctcac tcggaagcaa ttcgttgatc tgaatttcga ccacccataa 1920
   tacccattac cctggtagat aagtagcatg gcgggttaat cattaactac aaggaacccc 1980
   tagtgatgga gttggccact ccctctctgc gcgctcgctc gctcactgag gccgggcgac 2040
   caaaggtcgc ccgacgcccg ggctttgccc gggcggcctc agtgagcgag cgagcgcgca 2100
   gccttaatta acctaattca ctggccgtcg ttttacaacg tcgtgactgg gaaaaccctg 2160
   gcgttaccca acttaatcgc cttgcagcac atcccccttt cgccagctgg cgtaatagcg 2220
   aagaggcccg caccgatcgc ccttcccaac agttgcgcag cctgaatggc gaatgggacg 2280
   cgccctgtag cggcgcatta agcgcggcgg gtgtggtggt tacgcgcagc gtgaccgcta 2340
   cacttgccag cgccctagcg cccgctcctt tcgctttctt cccttccttt ctcgccacgt 2400
   tcgccggctt tccccgtcaa gctctaaatc gggggctccc tttagggttc cgatttagtg 2460
   ctttacggca cctcgacccc aaaaaacttg attagggtga tggttcacgt agtgggccat 2520
   cgccctgata gacggttttt cgccctttga cgttggagtc cacgttcttt aatagtggac 2580
   tcttgttcca aactggaaca acactcaacc ctatctcggt ctattctttt gatttataag 2640
   ggattttgcc gatttcggcc tattggttaa aaaatgagct gatttaacaa aaatttaacg 2700
   cgaattttaa caaaatatta acgcttacaa tttaggtggc acttttcggg gaaatgtgcg 2760
   cggaacccct atttgtttat ttttctaaat acattcaaat atgtatccgc tcatgagaca 2820
   ataaccctga taaatgcttc aataatattg aaaaaggaag agtatgagta ttcaacattt 2880
   ccgtgtcgcc cttattccct tttttgcggc attttgcctt cctgtttttg ctcacccaga 2940
   aacgctggtg aaagtaaaag atgctgaaga tcagttgggt gcacgagtgg gttacatcga 3000
   actggatctc aacagcggta agatccttga gagttttcgc cccgaagaac gttttccaat 3060
   gatgagcact tttaaagttc tgctatgtgg cgcggtatta tcccgtattg acgccgggca 3120
   agagcaactc ggtcgccgca tacactattc tcagaatgac ttggttgagt actcaccagt 3180
   cacagaaaag catcttacgg atggcatgac agtaagagaa ttatgcagtg ctgccataac 3240
   catgagtgat aacactgcgg ccaacttact tctgacaacg atcggaggac cgaaggagct 3300
   aaccgctttt ttgcacaaca tgggggatca tgtaactcgc cttgatcgtt gggaaccgga 3360
   gctgaatgaa gccataccaa acgacgagcg tgacaccacg atgcctgtag caatggcaac 3420
   aacgttgcgc aaactattaa ctggcgaact acttactcta gcttcccggc aacaattaat 3480
   agactggatg gaggcggata aagttgcagg accacttctg cgctcggccc ttccggctgg 3540
   ctggtttatt gctgataaat ctggagccgg tgagcgtggg tctcgcggta tcattgcagc 3600
   actggggcca gatggtaagc cctcccgtat cgtagttatc tacacgacgg ggagtcaggc 3660
   aactatggat gaacgaaata gacagatcgc tgagataggt gcctcactga ttaagcattg 3720
   gtaactgtca gaccaagttt actcatatat actttagatt gatttaaaac ttcattttta 3780
   atttaaaagg atctaggtga agatcctttt tgataatctc atgaccaaaa tcccttaacg 3840
   tgagttttcg ttccactgag cgtcagaccc cgtagaaaag atcaaaggat cttcttgaga 3900
   tccttttttt ctgcgcgtaa tctgctgctt gcaaacaaaa aaaccaccgc taccagcggt 3960
   ggtttgtttg ccggatcaag agctaccaac tctttttccg aaggtaactg gcttcagcag 4020
   agcgcagata ccaaatactg ttcttctagt gtagccgtag ttaggccacc acttcaagaa 4080
   ctctgtagca ccgcctacat acctcgctct gctaatcctg ttaccagtgg ctgctgccag 4140
   tggcgataag tcgtgtctta ccgggttgga ctcaagacga tagttaccgg ataaggcgca 4200
   gcggtcgggc tgaacggggg gttcgtgcac acagcccagc ttggagcgaa cgacctacac 4260
   cgaactgaga tacctacagc gtgagctatg agaaagcgcc acgcttcccg aagggagaaa 4320
   ggcggacagg tatccggtaa gcggcagggt cggaacagga gagcgcacga gggagcttcc 4380
   agggggaaac gcctggtatc tttatagtcc tgtcgggttt cgccacctct gacttgagcg 4440
   tcgatttttg tgatgctcgt caggggggcg gagcctatgg aaaaacgcca gcaacgcggc 4500
   ctttttacgg ttcctggcct tttgctggcc ttttgctcac atgttctttc ctgcgttatc 4560
   ccctgattct gtggataacc gtattaccgc ctttgagtga gctgataccg ctcgccgcag 4620
   ccgaacgacc gagcgcagcg agtcagtgag cgaggaagcg gaagagcgcc caatacgcaa 4680
   accgcctctc cccgcgcgtt ggccgattca ttaatgcagc tggcacgaca ggtttcccga 4740
   ctggaaagcg ggcagtgagc gcaacgcaat taatgtgagt tagctcactc attaggcacc 4800
   ccaggcttta cactttatgc ttccggctcg tatgttgtgt ggaattgtga gcggataaca 4860
   atttcacaca ggaaacagct atgaccatga ttacgccaga tttaattaag 4910

<210> 45
<211> 4910
<212> DNA
<213> Artificial Sequence

<220>
<223> Description of Artificial Sequence: Synthetic
      polynucleotide

<400> 45
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagg ttgacattga ttattgacta gttattaata 240

gtaatcaatt acggggtcat tagttcatag cccatatatg gagttccgcg ttacataact 300

tacggtaaat ggcccgcctg gctgaccgcc caacgacccc cgcccattga cgtcaataat 360

gacgtatgtt cccatagtaa cgccaatagg gactttccat tgacgtcaat gggtggagta 420

tttacggtaa actgcccact tggcagtaca tcaagtgtat catatgccaa gtacgccccc 480

tattgacgtc aatgacggta aatggcccgc ctggcattat gcccagtaca tgaccttatg 540

ggactttcct acttggcagt acatctacgt attagtcatc gctattacca tggtgatgcg 600

gttttggcag tacatcaatg ggcgtggata gcggtttgac tcacggggat ttccaagtct 660

ccaccccatt gacgtcaatg ggagtttgtt ttggcaccaa aatcaacggg actttccaaa 720

atgtcgtaac aactccgccc cattgacgca aatgggcggt aggcgtgtac ggtgggaggt 780

ctatataagc agagctctct ggctaactag agaacccact gcttactggc ttatcgaaat 840

taatacgact cactataggg agacccaagc tggctagcgt ttaaacttcg ggagcaagct 900

tgaactgaaa aaccagaaag ttaactggta agtttagtct ttttgtcttt tatttcaggt 960

cccggatccg gtggtggtgc aaatcaaaga actgctcctc agtggatgtt gcctttactt 1020

ctaggcctgt acggaagtgt tacttctgct ctaaaagctg cggaattgta cccgcggccg 1080

atccaccggt atgtggacac tcgggcgacg cgccgtggct gggctgcttg cttccccaag 1140

tcccgcacag gcgcaaaccc tcacaagggt cccacggcca gctgaattgg ccccgctgtg 1200

cggtcggagg ggcctgcgaa cagatataga cgcaacgtgc acccccagaa gagcaagcag 1260

caaccagaga ggcctcaatc agatttggaa cgtcaagaaa cagagtgtct atctgatgaa 1320

cctccgcaaa tcagggaccc ttggtcatcc gggatctctt gatgaaacga cctatgaacg 1380

gctggctgag gagacgcttg actccctggc agagttcttc gaggatctgg ctgataagcc 1440

atacacgttc gaggactatg atgtttcttt tggttctggc gtcctgacgg tgaaactcgg 1500

aggggatctg ggcacttacg ttatcaacaa acagactccc aataaacaga tttggttgtc 1560

tagtcctagc agtggcccaa aacgctatga ctggacgggc aagaactggg tgtactccca 1620

cgatggagtt agcctgcacg aacttctggc cgctgagctt actaaagcgc tgaagactaa 1680

gctggatctc tccagcctcg catacagcgg caaagacgcc tagctcgagg acggggtgaa 1740

ctacgcctga ggatccgatc tttttccctc tgccaaaaat tatggggaca tcatgaagcc 1800

ccttgagcat ctgacttctg gctaataaag gaaatttatt ttcattgcaa tagtgtgttg 1860

gaattttttg tgtctctcac tcggaagcaa ttcgttgatc tgaatttcga ccacccataa 1920

tacccattac cctggtagat aagtagcatg gcgggttaat cattaactac aaggaacccc 1980

tagtgatgga gttggccact ccctctctgc gcgctcgctc gctcactgag gccgggcgac 2040

caaaggtcgc ccgacgcccg ggctttgccc gggcggcctc agtgagcgag cgagcgcgca 2100

gccttaatta acctaattca ctggccgtcg ttttacaacg tcgtgactgg gaaaaccctg 2160

gcgttaccca acttaatcgc cttgcagcac atcccccttt cgccagctgg cgtaatagcg 2220

aagaggcccg caccgatcgc ccttcccaac agttgcgcag cctgaatggc gaatgggacg 2280

cgccctgtag cggcgcatta agcgcggcgg gtgtggtggt tacgcgcagc gtgaccgcta 2340

cacttgccag cgccctagcg cccgctcctt tcgctttctt cccttccttt ctcgccacgt 2400

tcgccggctt tccccgtcaa gctctaaatc gggggctccc tttagggttc cgatttagtg 2460

ctttacggca cctcgacccc aaaaaacttg attagggtga tggttcacgt agtgggccat 2520

cgccctgata gacggttttt cgccctttga cgttggagtc cacgttcttt aatagtggac 2580
tcttgttcca aactggaaca acactcaacc ctatctcggt ctattctttt gatttataag 2640
ggattttgcc gatttcggcc tattggttaa aaaatgagct gatttaacaa aaatttaacg 2700
cgaattttaa caaaatatta acgcttacaa tttaggtggc acttttcggg gaaatgtgcg 2760
cggaacccct atttgtttat ttttctaaat acattcaaat atgtatccgc tcatgagaca 2820
ataaccctga taaatgcttc aataatattg aaaaaggaag agtatgagta ttcaacattt 2880
ccgtgtcgcc cttattccct tttttgcggc attttgcctt cctgtttttg ctcacccaga 2940
aacgctggtg aaagtaaaag atgctgaaga tcagttgggt gcacgagtgg gttacatcga 3000
actggatctc aacagcggta agatccttga gagttttcgc cccgaagaac gttttccaat 3060
gatgagcact tttaaagttc tgctatgtgg cgcggtatta tcccgtattg acgccgggca 3120
agagcaactc ggtcgccgca tacactattc tcagaatgac ttggttgagt actcaccagt 3180
cacagaaaag catcttacgg atggcatgac agtaagagaa ttatgcagtg ctgccataac 3240
catgagtgat aacactgcgg ccaacttact tctgacaacg atcggaggac cgaaggagct 3300
aaccgctttt ttgcacaaca tgggggatca tgtaactcgc cttgatcgtt gggaaccgga 3360
gctgaatgaa gccataccaa acgacgagcg tgacaccacg atgcctgtag caatggcaac 3420
aacgttgcgc aaactattaa ctggcgaact acttactcta gcttcccggc aacaattaat 3480
agactggatg gaggcggata aagttgcagg accacttctg cgctcggccc ttccggctgg 3540
ctggtttatt gctgataaat ctggagccgg tgagcgtggg tctcgcggta tcattgcagc 3600
actggggcca gatggtaagc cctcccgtat cgtagttatc tacacgacgg ggagtcaggc 3660
aactatggat gaacgaaata gacagatcgc tgagataggt gcctcactga ttaagcattg 3720
gtaactgtca gaccaagttt actcatatat actttagatt gatttaaaac ttcattttta 3780
atttaaaagg atctaggtga agatcctttt tgataatctc atgaccaaaa tcccttaacg 3840
tgagttttcg ttccactgag cgtcagaccc cgtagaaaag atcaaaggat cttcttgaga 3900
tccttttttt ctgcgcgtaa tctgctgctt gcaaacaaaa aaaccaccgc taccagcggt 3960
ggtttgtttg ccggatcaag agctaccaac tctttttccg aaggtaactg gcttcagcag 4020
agcgcagata ccaaatactg ttcttctagt gtagccgtag ttaggccacc acttcaagaa 4080
ctctgtagca ccgcctacat acctcgctct gctaatcctg ttaccagtgg ctgctgccag 4140
tggcgataag tcgtgtctta ccgggttgga ctcaagacga tagttaccgg ataaggcgca 4200
gcggtcgggc tgaacggggg gttcgtgcac acagcccagc ttggagcgaa cgacctacac 4260
cgaactgaga tacctacagc gtgagctatg agaaagcgcc acgcttcccg aagggagaaa 4320
ggcggacagg tatccggtaa gcggcagggt cggaacagga gagcgcacga gggagcttcc 4380
agggggaaac gcctggtatc tttatagtcc tgtcgggttt cgccacctct gacttgagcg 4440

tcgatttttg tgatgctcgt caggggggcg gagcctatgg aaaaacgcca gcaacgcggc 4500

ctttttacgg ttcctggcct tttgctggcc ttttgctcac atgttctttc ctgcgttatc 4560

ccctgattct gtggataacc gtattaccgc ctttgagtga gctgataccg ctcgccgcag 4620

ccgaacgacc gagcgcagcg agtcagtgag cgaggaagcg gaagagcgcc caatacgcaa 4680

accgcctctc cccgcgcgtt ggccgattca ttaatgcagc tggcacgaca ggtttcccga 4740

ctggaaagcg ggcagtgagc gcaacgcaat taatgtgagt tagctcactc attaggcacc 4800

ccaggcttta cactttatgc ttccggctcg tatgttgtgt ggaattgtga gcggataaca 4860

atttcacaca ggaaacagct atgaccatga ttacgccaga tttaattaag 4910

<210> 46
<211> 4910
<212> DNA
<213> Artificial Sequence

<220>
<223> Description of Artificial Sequence: Synthetic
      polynucleotide

<400> 46
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagg ttgacattga ttattgacta gttattaata 240

gtaatcaatt acggggtcat tagttcatag cccatatatg gagttccgcg ttacataact 300

tacggtaaat ggcccgcctg gctgaccgcc caacgacccc cgcccattga cgtcaataat 360

gacgtatgtt cccatagtaa cgccaatagg gactttccat tgacgtcaat gggtggagta 420

tttacggtaa actgcccact tggcagtaca tcaagtgtat catatgccaa gtacgccccc 480

tattgacgtc aatgacggta aatggcccgc ctggcattat gcccagtaca tgaccttatg 540

ggactttcct acttggcagt acatctacgt attagtcatc gctattacca tggtgatgcg 600

gttttggcag tacatcaatg ggcgtggata gcggtttgac tcacggggat ttccaagtct 660

ccaccccatt gacgtcaatg ggagtttgtt ttggcaccaa aatcaacggg actttccaaa 720

atgtcgtaac aactccgccc cattgacgca aatgggcggt aggcgtgtac ggtgggaggt 780

ctatataagc agagctctct ggctaactag agaacccact gcttactggc ttatcgaaat 840

taatacgact cactataggg agacccaagc tggctagcgt ttaaacttcg ggagcaagct 900

tgaactgaaa aaccagaaag ttaactggta agtttagtct ttttgtcttt tatttcaggt 960

cccggatccg gtggtggtgc aaatcaaaga actgctcctc agtggatgtt gcctttactt 1020

ctaggcctgt acggaagtgt tacttctgct ctaaaagctg cggaattgta cccgcggccg 1080

atccaccggt atgtggactc tgggtcgacg cgccgtagcc ggtctcttgg cctccccatc 1140

ccctgcgcag gcacagaccc ttacacgagt tccacggccc gctgaactgg caccactctg 1200

cggacgccgg ggcctgagaa ccgatattga cgctacgtgc actccccgac gagcctcttc 1260

taatcagagg gggctcaacc agatctggaa cgtgaagaag cagtcagtct acttgatgaa 1320

tctgagaaag agcgggaccc ttgggcaccc cgggtctctc gacgagacta catacgagag 1380

gctggccgag gagactttgg actctctcgc cgaattcttc gaggatttgg cagacaagcc 1440

gtacaccttt gaagactacg atgtctcatt cggatcaggg gtgctgacgg ttaaactggg 1500

gggcgatctc ggaacctacg tcataaacaa acagacaccc aataagcaga tctggttgtc 1560

tagcccatca tccggtccta aacggtacga ctggaccggc aagaactggg tctactccca 1620

tgacggagtc agtcttcatg aactgcttgc cgcagagctg accaaggctc ttaagacgaa 1680

actggatctc tctagcctgg cctactccgg gaaggatgca tagctcgagg acggggtgaa 1740

ctacgcctga ggatccgatc tttttccctc tgccaaaaat tatggggaca tcatgaagcc 1800

ccttgagcat ctgacttctg gctaataaag gaaatttatt ttcattgcaa tagtgtgttg 1860

gaattttttg tgtctctcac tcggaagcaa ttcgttgatc tgaatttcga ccacccataa 1920

tacccattac cctggtagat aagtagcatg gcgggttaat cattaactac aaggaacccc 1980

tagtgatgga gttggccact ccctctctgc gcgctcgctc gctcactgag gccgggcgac 2040

caaaggtcgc ccgacgcccg ggctttgccc gggcggcctc agtgagcgag cgagcgcgca 2100

gccttaatta acctaattca ctggccgtcg ttttacaacg tcgtgactgg gaaaaccctg 2160

gcgttaccca acttaatcgc cttgcagcac atcccccttt cgccagctgg cgtaatagcg 2220

aagaggcccg caccgatcgc ccttcccaac agttgcgcag cctgaatggc gaatgggacg 2280

cgccctgtag cggcgcatta agcgcggcgg gtgtggtggt tacgcgcagc gtgaccgcta 2340

cacttgccag cgccctagcg cccgctcctt tcgctttctt cccttccttt ctcgccacgt 2400

tcgccggctt tccccgtcaa gctctaaatc gggggctccc tttagggttc cgatttagtg 2460

ctttacggca cctcgacccc aaaaaacttg attagggtga tggttcacgt agtgggccat 2520

cgccctgata gacggttttt cgccctttga cgttggagtc cacgttcttt aatagtggac 2580

tcttgttcca aactggaaca acactcaacc ctatctcggt ctattctttt gatttataag 2640

ggattttgcc gatttcggcc tattggttaa aaaatgagct gatttaacaa aaatttaacg 2700

cgaattttaa caaaatatta acgcttacaa tttaggtggc acttttcggg gaaatgtgcg 2760

cggaacccct atttgtttat ttttctaaat acattcaaat atgtatccgc tcatgagaca 2820

ataaccctga taaatgcttc aataatattg aaaaaggaag agtatgagta ttcaacattt 2880

ccgtgtcgcc cttattccct tttttgcggc attttgcctt cctgtttttg ctcacccaga 2940

aacgctggtg aaagtaaaag atgctgaaga tcagttgggt gcacgagtgg gttacatcga 3000

actggatctc aacagcggta agatccttga gagttttcgc cccgaagaac gttttccaat 3060

gatgagcact tttaaagttc tgctatgtgg cgcggtatta tcccgtattg acgccgggca 3120

agagcaactc ggtcgccgca tacactattc tcagaatgac ttggttgagt actcaccagt 3180

cacagaaaag catcttacgg atggcatgac agtaagagaa ttatgcagtg ctgccataac 3240

catgagtgat aacactgcgg ccaacttact tctgacaacg atcggaggac cgaaggagct 3300

aaccgctttt ttgcacaaca tgggggatca tgtaactcgc cttgatcgtt gggaaccgga 3360

gctgaatgaa gccataccaa acgacgagcg tgacaccacg atgcctgtag caatggcaac 3420

aacgttgcgc aaactattaa ctggcgaact acttactcta gcttcccggc aacaattaat 3480

agactggatg gaggcggata aagttgcagg accacttctg cgctcggccc ttccggctgg 3540

ctggtttatt gctgataaat ctggagccgg tgagcgtggg tctcgcggta tcattgcagc 3600

actggggcca gatggtaagc cctcccgtat cgtagttatc tacacgacgg ggagtcaggc 3660

aactatggat gaacgaaata gacagatcgc tgagataggt gcctcactga ttaagcattg 3720

gtaactgtca gaccaagttt actcatatat actttagatt gatttaaaac ttcattttta 3780

atttaaaagg atctaggtga agatcctttt tgataatctc atgaccaaaa tcccttaacg 3840

tgagttttcg ttccactgag cgtcagaccc cgtagaaaag atcaaaggat cttcttgaga 3900

tccttttttt ctgcgcgtaa tctgctgctt gcaaacaaaa aaaccaccgc taccagcggt 3960

ggtttgtttg ccggatcaag agctaccaac tctttttccg aaggtaactg gcttcagcag 4020

agcgcagata ccaaatactg ttcttctagt gtagccgtag ttaggccacc acttcaagaa 4080

ctctgtagca ccgcctacat acctcgctct gctaatcctg ttaccagtgg ctgctgccag 4140

tggcgataag tcgtgtctta ccgggttgga ctcaagacga tagttaccgg ataaggcgca 4200

gcggtcgggc tgaacggggg gttcgtgcac acagcccagc ttggagcgaa cgacctacac 4260

cgaactgaga tacctacagc gtgagctatg agaaagcgcc acgcttcccg aagggagaaa 4320

ggcggacagg tatccggtaa gcggcagggt cggaacagga gagcgcacga gggagcttcc 4380

agggggaaac gcctggtatc tttatagtcc tgtcgggttt cgccacctct gacttgagcg 4440

tcgatttttg tgatgctcgt caggggggcg gagcctatgg aaaaacgcca gcaacgcggc 4500

ctttttacgg ttcctggcct tttgctggcc ttttgctcac atgttctttc ctgcgttatc 4560

ccctgattct gtggataacc gtattaccgc ctttgagtga gctgataccg ctcgccgcag 4620

ccgaacgacc gagcgcagcg agtcagtgag cgaggaagcg gaagagcgcc caatacgcaa 4680

accgcctctc cccgcgcgtt ggccgattca ttaatgcagc tggcacgaca ggtttcccga 4740

ctggaaagcg ggcagtgagc gcaacgcaat taatgtgagt tagctcactc attaggcacc 4800

ccaggcttta cactttatgc ttccggctcg tatgttgtgt ggaattgtga gcggataaca 4860

atttcacaca ggaaacagct atgaccatga ttacgccaga tttaattaag 4910

<210> 47
<211> 4919
<212> DNA
<213> Artificial Sequence

<220>
<223> Description of Artificial Sequence: Synthetic
      polynucleotide

<400> 47
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagg ttgacattga ttattgacta gttattaata 240

gtaatcaatt acggggtcat tagttcatag cccatatatg gagttccgcg ttacataact 300

tacggtaaat ggcccgcctg gctgaccgcc caacgacccc cgcccattga cgtcaataat 360

gacgtatgtt cccatagtaa cgccaatagg gactttccat tgacgtcaat gggtggagta 420

tttacggtaa actgcccact tggcagtaca tcaagtgtat catatgccaa gtacgccccc 480

tattgacgtc aatgacggta aatggcccgc ctggcattat gcccagtaca tgaccttatg 540

ggactttcct acttggcagt acatctacgt attagtcatc gctattacca tggtgatgcg 600

gttttggcag tacatcaatg ggcgtggata gcggtttgac tcacggggat ttccaagtct 660

ccaccccatt gacgtcaatg ggagtttgtt ttggcaccaa aatcaacggg actttccaaa 720

atgtcgtaac aactccgccc cattgacgca aatgggcggt aggcgtgtac ggtgggaggt 780

ctatataagc agagctctct ggctaactag agaacccact gcttactggc ttatcgaaat 840

taatacgact cactataggg agacccaagc tggctagcgt ttaaacttcg ggagcaagct 900

tgaactgaaa aaccagaaag ttaactggta agtttagtct ttttgtcttt tatttcaggt 960

cccggatccg gtggtggtgc aaatcaaaga actgctcctc agtggatgtt gcctttactt 1020

ctaggcctgt acggaagtgt tacttctgct ctaaaagctg cggaattgta cccgcggccg 1080

atccaccggt gccgccacca tgtggactct cgggcgccgc gcagtagccg gcctcctggc 1140

gtcacccagc ccagcccagg cccagaccct cacccgggtc ccgcggccgg cagagttggc 1200

cccactctgc ggccgccgtg gcctgcgcac cgacatcgat gcgacctgca cgccccgccg 1260

cgcaagttcg aaccaacgtg gcctcaacca gatttggaat gtcaaaaagc agagtgtcta 1320

tttgatgaat ttgaggaaat ctggaacttt gggccaccca ggctctctag atgagaccac 1380

ctatgaaaga ctagcagagg aaacgctgga ctctttagca gagttttttg aagaccttgc 1440

agacaagcca tacacgtttg aggactatga tgtctccttt gggagtggtg tcttaactgt 1500

caaactgggt ggagatctag gaacctatgt gatcaacaag cagacgccaa acaagcaaat 1560

ctggctatct tctccatcca gtggacctaa gcgttatgac tggactggga aaaactgggt 1620

gtactcccac gacggcgtgt ccctccatga gctgctggcc gcagagctca ctaaagcctt 1680

aaaaaccaaa ctggacttgt cttccttggc ctattccgga aaagatgctt gactcgagga 1740

cggggtgaac tacgcctgag gatccgatct ttttccctct gccaaaaatt atggggacat 1800

catgaagccc cttgagcatc tgacttctgg ctaataaagg aaatttattt tcattgcaat 1860

agtgtgttgg aattttttgt gtctctcact cggaagcaat tcgttgatct gaatttcgac 1920

cacccataat acccattacc ctggtagata agtagcatgg cgggttaatc attaactaca 1980

aggaacccct agtgatggag ttggccactc cctctctgcg cgctcgctcg ctcactgagg 2040

ccgggcgacc aaaggtcgcc cgacgcccgg gctttgcccg ggcggcctca gtgagcgagc 2100

gagcgcgcag ccttaattaa cctaattcac tggccgtcgt tttacaacgt cgtgactggg 2160

aaaaccctgg cgttacccaa cttaatcgcc ttgcagcaca tccccctttc gccagctggc 2220

gtaatagcga agaggcccgc accgatcgcc cttcccaaca gttgcgcagc ctgaatggcg 2280

aatgggacgc gccctgtagc ggcgcattaa gcgcggcggg tgtggtggtt acgcgcagcg 2340

tgaccgctac acttgccagc gccctagcgc ccgctccttt cgctttcttc ccttcctttc 2400

tcgccacgtt cgccggcttt ccccgtcaag ctctaaatcg ggggctccct ttagggttcc 2460

gatttagtgc tttacggcac ctcgacccca aaaaacttga ttagggtgat ggttcacgta 2520

gtgggccatc gccctgatag acggtttttc gccctttgac gttggagtcc acgttcttta 2580

atagtggact cttgttccaa actggaacaa cactcaaccc tatctcggtc tattcttttg 2640

atttataagg gattttgccg atttcggcct attggttaaa aaatgagctg atttaacaaa 2700

aatttaacgc gaattttaac aaaatattaa cgcttacaat ttaggtggca cttttcgggg 2760

aaatgtgcgc ggaaccccta tttgtttatt tttctaaata cattcaaata tgtatccgct 2820

catgagacaa taaccctgat aaatgcttca ataatattga aaaaggaaga gtatgagtat 2880

tcaacatttc cgtgtcgccc ttattccctt ttttgcggca ttttgccttc ctgtttttgc 2940

tcacccagaa acgctggtga aagtaaaaga tgctgaagat cagttgggtg cacgagtggg 3000

ttacatcgaa ctggatctca acagcggtaa gatccttgag agttttcgcc ccgaagaacg 3060

ttttccaatg atgagcactt ttaaagttct gctatgtggc gcggtattat cccgtattga 3120

cgccgggcaa gagcaactcg gtcgccgcat acactattct cagaatgact tggttgagta 3180

ctcaccagtc acagaaaagc atcttacgga tggcatgaca gtaagagaat tatgcagtgc 3240

tgccataacc atgagtgata acactgcggc caacttactt ctgacaacga tcggaggacc 3300

gaaggagcta accgcttttt tgcacaacat gggggatcat gtaactcgcc ttgatcgttg 3360

ggaaccggag ctgaatgaag ccataccaaa cgacgagcgt gacaccacga tgcctgtagc 3420

aatggcaaca acgttgcgca aactattaac tggcgaacta cttactctag cttcccggca 3480

acaattaata gactggatgg aggcggataa agttgcagga ccacttctgc gctcggccct 3540

tccggctggc tggtttattg ctgataaatc tggagccggt gagcgtgggt ctcgcggtat 3600

cattgcagca ctggggccag atggtaagcc ctcccgtatc gtagttatct acacgacggg 3660

gagtcaggca actatggatg aacgaaatag acagatcgct gagataggtg cctcactgat 3720

taagcattgg taactgtcag accaagttta ctcatatata ctttagattg atttaaaact 3780

tcatttttaa tttaaaagga tctaggtgaa gatccttttt gataatctca tgaccaaaat 3840

cccttaacgt gagttttcgt tccactgagc gtcagacccc gtagaaaaga tcaaaggatc 3900

ttcttgagat cctttttttc tgcgcgtaat ctgctgcttg caaacaaaaa aaccaccgct 3960

accagcggtg gtttgtttgc cggatcaaga gctaccaact ctttttccga aggtaactgg 4020

cttcagcaga gcgcagatac caaatactgt tcttctagtg tagccgtagt taggccacca 4080

cttcaagaac tctgtagcac cgcctacata cctcgctctg ctaatcctgt taccagtggc 4140

tgctgccagt ggcgataagt cgtgtcttac cgggttggac tcaagacgat agttaccgga 4200

taaggcgcag cggtcgggct gaacgggggg ttcgtgcaca cagcccagct tggagcgaac 4260

gacctacacc gaactgagat acctacagcg tgagctatga gaaagcgcca cgcttcccga 4320

agggagaaag gcggacaggt atccggtaag cggcagggtc ggaacaggag agcgcacgag 4380

ggagcttcca gggggaaacg cctggtatct ttatagtcct gtcgggtttc gccacctctg 4440

acttgagcgt cgatttttgt gatgctcgtc aggggggcgg agcctatgga aaaacgccag 4500

caacgcggcc tttttacggt tcctggcctt ttgctggcct tttgctcaca tgttctttcc 4560

tgcgttatcc cctgattctg tggataaccg tattaccgcc tttgagtgag ctgataccgc 4620

tcgccgcagc cgaacgaccg agcgcagcga gtcagtgagc gaggaagcgg aagagcgccc 4680

aatacgcaaa ccgcctctcc ccgcgcgttg gccgattcat taatgcagct ggcacgacag 4740

gtttcccgac tggaaagcgg gcagtgagcg caacgcaatt aatgtgagtt agctcactca 4800

ttaggcaccc caggctttac actttatgct tccggctcgt atgttgtgtg gaattgtgag 4860

cggataacaa tttcacacag gaaacagcta tgaccatgat tacgccagat ttaattaag 4919

<210> 48
<211> 5586
<212> DNA
<213> Artificial Sequence

<220>
<223> Description of Artificial Sequence: Synthetic
      polynucleotide

<400> 48

gccttaatta ggctgcgcgc tcgctcgctc actgaggccg cccgggcaaa gcccgggcgt 60
cgggcgacct ttggtcgccc ggcctcagtg agcgagcgag cgcgcagaga gggagtggcc 120
aactccatca ctaggggttc cttgtagtta atgattaacc cgccatgcta cttatctacc 180
agggtaatgg ggatcctcta gaactatagc tagtcgacga gctcccaggc tcaagtgatc 240
ctcccgcctc agcctcctga gtagctgaga tcacaggcat gcactaccac actcggctca 300
catttgacat cctctaaagc atatataaaa tgtgaagaaa actttcacaa tttgcatccc 360
tttgtaatat gtaacagaaa taaaattctc ttttaaaatc tatcaacaat aggcaaggca 420
cggtggctca cgcctgtcgt ctcagcactt tgtgaggccc aggcgggcag atcgtttgag 480
cctagaagtt caagaccacc ctgggcaaca tagcgaaacc ccctttctac aaaaaataca 540
aaaactagct gggtgtggtg gtgcacacct gtagtcccag ctacttggaa ggctgaaatg 600
ggaagactgc ttgagcccgg gagggagaag ttgcagtaag ccaggaccac accactgcac 660
tccagcctgg gcaacagagt gagactctgt ctcaaacaaa caaataaatg aggcgggtgg 720
atcacgaggt cagtagatcg agaccatcct ggctaacacg gtgaaacccg tctctactaa 780
aaaaaaaaaa aaatacaaaa aattagccag gcatggtggc gggcgcctgt agtcccagtt 840
actcgggagg ctgaggcagg agaatggcgt gaaaccggga ggcagagctt gcagtgagcc 900
gagatcgcac cactgccctc cagcctgggc gacagagcga gactccgtct caatcaatca 960
atcaatcaat aaaatctatt aacaatattt attgtgcact taacaggaac atgccctgtc 1020
caaaaaaaac tttacagggc ttaactcatt ttatccttac cacaatccta tgaagtagga 1080
acttttataa aacgcatttt ataaacaagg cacagagagg ttaattaact tgccctctgg 1140
tcacacagct aggaagtggg cagagtacag atttacacaa ggcatccgtc tcctggcccc 1200
acatacccaa ctgctgtaaa cccataccgg cggccaagca gcctcaattt gtgcatgcac 1260
ccacttccca gcaagacagc agctcccaag ttcctcctgt ttagaatttt agaagcggcg 1320
ggccaccagg ctgcagtctc ccttgggtca ggggtcctgg ttgcactccg tgctttgcac 1380
aaagcaggct ctccattttt gttaaatgca cgaatagtgc taagctggga agttcttcct 1440
gaggtctaac ctctagctgc tcccccacag aagagtgcct gcggccagtg gccaccaggg 1500
gtcgccgcag cacccagcgc tggagggcgg agcgggcggc agacccggag cagcaagtgg 1560
actctctcga gaagcttgaa ctgaaaaacc agaaagttaa ctggtaagtt tagtcttttt 1620
gtcttttatt tcaggtcccg gatccggtgg tggtgcaaat caaagaactg ctcctcagtg 1680
gatgttgcct ttacttctag gcctgtacgg aagtgttact tctgctctaa aagctgcgga 1740
attgtacccg cggccgatcc accggtatgt ggactctcgg gcgccgcgca gtagccggcc 1800
tcctggcgtc acccagccca gcccaggccc agaccctcac ccgggtcccg cggccggcag 1860
agttggcccc actctgcggc cgccgtggcc tgcgcaccga catcgatgcg acctgcacgc 1920

cccgccgcgc aagttcgaac caacgtggcc tcaaccagat ttggaatgtc aaaaagcaga 1980
gtgtctattt gatgaatttg aggaaatctg gaactttggg ccacccaggc tctctagatg 2040
agaccaccta tgaaagacta gcagaggaaa cgctggactc tttagcagag ttttttgaag 2100
accttgcaga caagccatac acgtttgagg actatgatgt ctcctttggg agtggtgtct 2160
taactgtcaa actgggtgga gatctaggaa cctatgtgat caacaagcag acgccaaaca 2220
agcaaatctg gctatcttct ccatccagtg gacctaagcg ttatgactgg actgggaaaa 2280
actgggtgta ctcccacgac ggcgtgtccc tccatgagct gctggccgca gagctcacta 2340
aagccttaaa aaccaaactg gacttgtctt ccttggccta ttccggaaaa gatgcttgac 2400
tcgaggacgg ggtgaactac gcctgaggat ccgatctttt tccctctgcc aaaaattatg 2460
gggacatcat gaagcccctt gagcatctga cttctggcta ataaaggaaa tttattttca 2520
ttgcaatagt gtgttggaat tttttgtgtc tctcactcgg aagcaattcg ttgatctgaa 2580
tttcgaccac ccataatacc cattaccctg gtagataagt agcatggcgg gttaatcatt 2640
aactacaagg aacccctagt gatggagttg gccactccct ctctgcgcgc tcgctcgctc 2700
actgaggccg ggcgaccaaa ggtcgcccga cgcccgggct ttgcccgggc ggcctcagtg 2760
agcgagcgag cgcgcagcct taattaacct aattcactgg ccgtcgtttt acaacgtcgt 2820
gactgggaaa accctggcgt tacccaactt aatcgccttg cagcacatcc ccctttcgcc 2880
agctggcgta atagcgaaga ggcccgcacc gatcgccctt cccaacagtt gcgcagcctg 2940
aatggcgaat gggacgcgcc ctgtagcggc gcattaagcg cggcgggtgt ggtggttacg 3000
cgcagcgtga ccgctacact tgccagcgcc ctagcgcccg ctcctttcgc tttcttccct 3060
tcctttctcg ccacgttcgc cggctttccc cgtcaagctc taaatcgggg gctcccttta 3120
gggttccgat ttagtgcttt acggcacctc gaccccaaaa aacttgatta gggtgatggt 3180
tcacgtagtg ggccatcgcc ctgatagacg gtttttcgcc ctttgacgtt ggagtccacg 3240
ttctttaata gtggactctt gttccaaact ggaacaacac tcaaccctat ctcggtctat 3300
tcttttgatt tataagggat tttgccgatt tcggcctatt ggttaaaaaa tgagctgatt 3360
taacaaaaat ttaacgcgaa ttttaacaaa atattaacgc ttacaattta ggtggcactt 3420
ttcggggaaa tgtgcgcgga acccctattt gtttattttt ctaaatacat tcaaatatgt 3480
atccgctcat gagacaataa ccctgataaa tgcttcaata atattgaaaa aggaagagta 3540
tgagtattca acatttccgt gtcgccctta ttcccttttt tgcggcattt tgccttcctg 3600
tttttgctca cccagaaacg ctggtgaaag taaaagatgc tgaagatcag ttgggtgcac 3660
gagtgggtta catcgaactg gatctcaaca gcggtaagat ccttgagagt tttcgccccg 3720
aagaacgttt tccaatgatg agcactttta aagttctgct atgtggcgcg gtattatccc 3780

gtattgacgc cgggcaagag caactcggtc gccgcataca ctattctcag aatgacttgg 3840

ttgagtactc accagtcaca gaaaagcatc ttacggatgg catgacagta agagaattat 3900

gcagtgctgc cataaccatg agtgataaca ctgcggccaa cttacttctg acaacgatcg 3960

gaggaccgaa ggagctaacc gcttttttgc acaacatggg ggatcatgta actcgccttg 4020

atcgttggga accggagctg aatgaagcca taccaaacga cgagcgtgac accacgatgc 4080

ctgtagcaat ggcaacaacg ttgcgcaaac tattaactgg cgaactactt actctagctt 4140

cccggcaaca attaatagac tggatggagg cggataaagt tgcaggacca cttctgcgct 4200

cggcccttcc ggctggctgg tttattgctg ataaatctgg agccggtgag cgtgggtctc 4260

gcggtatcat tgcagcactg gggccagatg gtaagccctc ccgtatcgta gttatctaca 4320

cgacggggag tcaggcaact atggatgaac gaaatagaca gatcgctgag ataggtgcct 4380

cactgattaa gcattggtaa ctgtcagacc aagtttactc atatatactt tagattgatt 4440

taaaacttca tttttaattt aaaaggatct aggtgaagat cctttttgat aatctcatga 4500

ccaaaatccc ttaacgtgag ttttcgttcc actgagcgtc agaccccgta gaaaagatca 4560

aaggatcttc ttgagatcct ttttttctgc gcgtaatctg ctgcttgcaa acaaaaaaac 4620

caccgctacc agcggtggtt tgtttgccgg atcaagagct accaactctt tttccgaagg 4680

taactggctt cagcagagcg cagataccaa atactgttct tctagtgtag ccgtagttag 4740

gccaccactt caagaactct gtagcaccgc ctacatacct cgctctgcta atcctgttac 4800

cagtggctgc tgccagtggc gataagtcgt gtcttaccgg gttggactca agacgatagt 4860

taccggataa ggcgcagcgg tcgggctgaa cggggggttc gtgcacacag cccagcttgg 4920

agcgaacgac ctacaccgaa ctgagatacc tacagcgtga gctatgagaa agcgccacgc 4980

ttcccgaagg gagaaaggcg gacaggtatc cggtaagcgg cagggtcgga acaggagagc 5040

gcacgaggga gcttccaggg ggaaacgcct ggtatcttta tagtcctgtc gggtttcgcc 5100

acctctgact tgagcgtcga tttttgtgat gctcgtcagg ggggcggagc ctatggaaaa 5160

acgccagcaa cgcggccttt ttacggttcc tggccttttg ctggcctttt gctcacatgt 5220

tctttcctgc gttatcccct gattctgtgg ataaccgtat taccgccttt gagtgagctg 5280

ataccgctcg ccgcagccga acgaccgagc gcagcgagtc agtgagcgag gaagcggaag 5340

agcgcccaat acgcaaaccg cctctccccg cgcgttggcc gattcattaa tgcagctggc 5400

acgacaggtt tcccgactgg aaagcgggca gtgagcgcaa cgcaattaat gtgagttagc 5460

tcactcatta ggcaccccag gctttacact ttatgcttcc ggctcgtatg ttgtgtggaa 5520

ttgtgagcgg ataacaattt cacacaggaa acagctatga ccatgattac gccagattta 5580

attaag 5586

<210> 49
<211> 5586
<212> DNA
<213> Artificial Sequence

<220>
<223> Description of Artificial Sequence: Synthetic
      polynucleotide

<400> 49
gccttaatta ggctgcgcgc tcgctcgctc actgaggccg cccgggcaaa gcccgggcgt      60

cgggcgacct ttggtcgccc ggcctcagtg agcgagcgag cgcgcagaga gggagtggcc     120

aactccatca ctaggggttc cttgtagtta atgattaacc cgccatgcta cttatctacc     180

agggtaatgg ggatcctcta gaactatagc tagtcgacga gctcccaggc tcaagtgatc     240

ctcccgcctc agcctcctga gtagctgaga tcacaggcat gcactaccac actcggctca     300

catttgacat cctctaaagc atatataaaa tgtgaagaaa actttcacaa tttgcatccc     360

tttgtaatat gtaacagaaa taaaattctc ttttaaaatc tatcaacaat aggcaaggca     420

cggtggctca cgcctgtcgt ctcagcactt tgtgaggccc aggcgggcag atcgtttgag     480

cctagaagtt caagaccacc ctgggcaaca tagcgaaacc ccctttctac aaaaaataca     540

aaaactagct gggtgtggtg gtgcacacct gtagtcccag ctacttggaa ggctgaaatg     600

ggaagactgc ttgagcccgg gagggagaag ttgcagtaag ccaggaccac accactgcac     660

tccagcctgg gcaacagagt gagactctgt ctcaaacaaa caaataaatg aggcgggtgg     720

atcacgaggt cagtagatcg agaccatcct ggctaacacg gtgaaacccg tctctactaa     780

aaaaaaaaaa aaatacaaaa aattagccag gcatggtggc gggcgcctgt agtcccagtt     840

actcgggagg ctgaggcagg agaatggcgt gaaaccggga ggcagagctt gcagtgagcc     900

gagatcgcac cactgccctc cagcctgggc gacagagcga gactccgtct caatcaatca     960

atcaatcaat aaaatctatt aacaatattt attgtgcact taacaggaac atgccctgtc    1020

caaaaaaaac tttacagggc ttaactcatt ttatccttac cacaatccta tgaagtagga    1080

acttttataa aacgcatttt ataaacaagg cacagagagg ttaattaact tgccctctgg    1140

tcacacagct aggaagtggg cagagtacag atttacacaa ggcatccgtc tcctggcccc    1200

acatacccaa ctgctgtaaa cccataccgg cggccaagca gcctcaattt gtgcatgcac    1260

ccacttccca gcaagacagc agctcccaag ttcctcctgt ttagaatttt agaagcggcg    1320

ggccaccagg ctgcagtctc ccttgggtca ggggtcctgg ttgcactccg tgctttgcac    1380

aaagcaggct ctccattttt gttaaatgca cgaatagtgc taagctggga agttcttcct    1440

gaggtctaac ctctagctgc tcccccacag aagagtgcct gcggccagtg gccaccaggg    1500

gtcgccgcag cacccagcgc tggagggcgg agcgggcggc agacccggag cagcaagtgg    1560

actctctcga gaagcttgaa ctgaaaaacc agaaagttaa ctggtaagtt tagtcttttt    1620

gtcttttatt tcaggtcccg gatccggtgg tggtgcaaat caaagaactg ctcctcagtg 1680

gatgttgcct ttacttctag gcctgtacgg aagtgttact tctgctctaa aagctgcgga 1740

attgtacccg cggccgatcc accggtatgt ggactctggg tcgcagagcg gtggcaggct 1800

tgctggcatc tccgtccccg gctcaggccc agaccctgac acgggtgccc aggccggccg 1860

agctggctcc tctgtgcggc cggcgcggcc ttagaacaga cattgacgca acctgcaccc 1920

ccagaagagc cagttccaac cagcgcgggt tgaatcagat ctggaacgtc aagaagcaga 1980

gtgtgtacct tatgaatctc cggaaaagtg gcaccctcgg acaccccgga tccctcgatg 2040

aaaccactta tgagcgcctg gccgaggaaa ccctggattc cctggctgag ttcttcgagg 2100

acctggctga caaaccctac accttcgaag actacgacgt ttccttcgga agtggggtgc 2160

tgacggtcaa acttggtggc gatcttggaa cctatgttat aaacaagcag actcccaaca 2220

aacagatctg gctcagctct ccaagttcag gacccaagag gtatgactgg acgggcaaga 2280

attgggtcta ttctcacgac ggagtgagcc ttcatgaact gttggctgcc gaactgacaa 2340

aagcccttaa gacgaaactg gacctgtcct cattggcgta ctctggtaag gatgcctaac 2400

tcgaggacgg ggtgaactac gcctgaggat ccgatctttt tccctctgcc aaaaattatg 2460

gggacatcat gaagcccctt gagcatctga cttctggcta ataaaggaaa tttattttca 2520

ttgcaatagt gtgttggaat tttttgtgtc tctcactcgg aagcaattcg ttgatctgaa 2580

tttcgaccac ccataatacc cattaccctg gtagataagt agcatggcgg gttaatcatt 2640

aactacaagg aacccctagt gatggagttg gccactccct ctctgcgcgc tcgctcgctc 2700

actgaggccg ggcgaccaaa ggtcgcccga cgcccgggct ttgcccgggc ggcctcagtg 2760

agcgagcgag cgcgcagcct taattaacct aattcactgg ccgtcgtttt acaacgtcgt 2820

gactgggaaa accctggcgt tacccaactt aatcgccttg cagcacatcc ccctttcgcc 2880

agctggcgta atagcgaaga ggcccgcacc gatcgccctt cccaacagtt gcgcagcctg 2940

aatggcgaat gggacgcgcc ctgtagcggc gcattaagcg cggcgggtgt ggtggttacg 3000

cgcagcgtga ccgctacact tgccagcgcc ctagcgcccg ctcctttcgc tttcttccct 3060

tcctttctcg ccacgttcgc cggctttccc cgtcaagctc taaatcgggg gctcccttta 3120

gggttccgat ttagtgcttt acggcacctc gaccccaaaa aacttgatta gggtgatggt 3180

tcacgtagtg ggccatcgcc ctgatagacg gtttttcgcc ctttgacgtt ggagtccacg 3240

ttctttaata gtggactctt gttccaaact ggaacaacac tcaaccctat ctcggtctat 3300

tcttttgatt tataagggat tttgccgatt tcggcctatt ggttaaaaaa tgagctgatt 3360

taacaaaaat ttaacgcgaa ttttaacaaa atattaacgc ttacaattta ggtggcactt 3420

ttcggggaaa tgtgcgcgga acccctattt gtttattttt ctaaatacat tcaaatatgt 3480

atccgctcat gagacaataa ccctgataaa tgcttcaata atattgaaaa aggaagagta 3540

tgagtattca acatttccgt gtcgccctta ttcccttttt tgcggcattt tgccttcctg 3600

tttttgctca cccagaaacg ctggtgaaag taaaagatgc tgaagatcag ttgggtgcac 3660

gagtgggtta catcgaactg gatctcaaca gcggtaagat ccttgagagt tttcgccccg 3720

aagaacgttt tccaatgatg agcactttta aagttctgct atgtggcgcg gtattatccc 3780

gtattgacgc cgggcaagag caactcggtc gccgcataca ctattctcag aatgacttgg 3840

ttgagtactc accagtcaca gaaaagcatc ttacggatgg catgacagta agagaattat 3900

gcagtgctgc cataaccatg agtgataaca ctgcggccaa cttacttctg acaacgatcg 3960

gaggaccgaa ggagctaacc gcttttttgc acaacatggg ggatcatgta actcgccttg 4020

atcgttggga accggagctg aatgaagcca taccaaacga cgagcgtgac accacgatgc 4080

ctgtagcaat ggcaacaacg ttgcgcaaac tattaactgg cgaactactt actctagctt 4140

cccggcaaca attaatagac tggatggagg cggataaagt tgcaggacca cttctgcgct 4200

cggcccttcc ggctggctgg tttattgctg ataaatctgg agccggtgag cgtgggtctc 4260

gcggtatcat tgcagcactg gggccagatg gtaagccctc ccgtatcgta gttatctaca 4320

cgacggggag tcaggcaact atggatgaac gaaatagaca gatcgctgag ataggtgcct 4380

cactgattaa gcattggtaa ctgtcagacc aagtttactc atatatactt tagattgatt 4440

taaaacttca tttttaattt aaaaggatct aggtgaagat cctttttgat aatctcatga 4500

ccaaaatccc ttaacgtgag ttttcgttcc actgagcgtc agaccccgta gaaaagatca 4560

aaggatcttc ttgagatcct ttttttctgc gcgtaatctg ctgcttgcaa acaaaaaaac 4620

caccgctacc agcggtggtt tgtttgccgg atcaagagct accaactctt tttccgaagg 4680

taactggctt cagcagagcg cagataccaa atactgttct tctagtgtag ccgtagttag 4740

gccaccactt caagaactct gtagcaccgc ctacatacct cgctctgcta atcctgttac 4800

cagtggctgc tgccagtggc gataagtcgt gtcttaccgg gttggactca agacgatagt 4860

taccggataa ggcgcagcgg tcgggctgaa cggggggttc gtgcacacag cccagcttgg 4920

agcgaacgac ctacaccgaa ctgagatacc tacagcgtga gctatgagaa agcgccacgc 4980

ttcccgaagg gagaaaggcg gacaggtatc cggtaagcgg cagggtcgga acaggagagc 5040

gcacgaggga gcttccaggg ggaaacgcct ggtatcttta tagtcctgtc gggtttcgcc 5100

acctctgact tgagcgtcga tttttgtgat gctcgtcagg ggggcggagc ctatggaaaa 5160

acgccagcaa cgcggccttt ttacggttcc tggccttttg ctggcctttt gctcacatgt 5220

tctttcctgc gttatcccct gattctgtgg ataaccgtat taccgccttt gagtgagctg 5280

ataccgctcg ccgcagccga acgaccgagc gcagcgagtc agtgagcgag gaagcggaag 5340

agcgcccaat acgcaaaccg cctctccccg cgcgttggcc gattcattaa tgcagctggc 5400

acgacaggtt tcccgactgg aaagcgggca gtgagcgcaa cgcaattaat gtgagttagc 5460

tcactcatta ggcaccccag gctttacact ttatgcttcc ggctcgtatg ttgtgtggaa 5520

ttgtgagcgg ataacaattt cacacaggaa acagctatga ccatgattac gccagattta 5580

attaag 5586

<210> 50
<211> 5586
<212> DNA
<213> Artificial Sequence

<220>
<223> Description of Artificial Sequence: Synthetic
      polynucleotide

<400> 50
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacga gctcccaggc tcaagtgatc 240

ctcccgcctc agcctcctga gtagctgaga tcacaggcat gcactaccac actcggctca 300

catttgacat cctctaaagc atatataaaa tgtgaagaaa actttcacaa tttgcatccc 360

tttgtaatat gtaacagaaa taaaattctc ttttaaaatc tatcaacaat aggcaaggca 420

cggtggctca cgcctgtcgt ctcagcactt tgtgaggccc aggcgggcag atcgtttgag 480

cctagaagtt caagaccacc ctgggcaaca tagcgaaacc ccctttctac aaaaaataca 540

aaaactagct gggtgtggtg gtgcacacct gtagtcccag ctacttggaa ggctgaaatg 600

ggaagactgc ttgagcccgg gagggagaag ttgcagtaag ccaggaccac accactgcac 660

tccagcctgg gcaacagagt gagactctgt ctcaaacaaa caaataaatg aggcgggtgg 720

atcacgaggt cagtagatcg agaccatcct ggctaacacg gtgaaacccg tctctactaa 780

aaaaaaaaaa aaatacaaaa aattagccag gcatggtggc gggcgcctgt agtcccagtt 840

actcgggagg ctgaggcagg agaatggcgt gaaaccggga ggcagagctt gcagtgagcc 900

gagatcgcac cactgccctc cagcctgggc gacagagcga gactccgtct caatcaatca 960

atcaatcaat aaaatctatt aacaatattt attgtgcact taacaggaac atgccctgtc 1020

caaaaaaaac tttacagggc ttaactcatt ttatccttac cacaatccta tgaagtagga 1080

acttttataa aacgcatttt ataaacaagg cacagagagg ttaattaact tgccctctgg 1140

tcacacagct aggaagtggg cagagtacag atttacacaa ggcatccgtc tcctggcccc 1200

acatacccaa ctgctgtaaa cccataccgg cggccaagca gcctcaattt gtgcatgcac 1260

ccacttccca gcaagacagc agctcccaag ttcctcctgt ttagaatttt agaagcggcg 1320

ggccaccagg ctgcagtctc ccttgggtca ggggtcctgg ttgcactccg tgctttgcac 1380

aaagcaggct ctccattttt gttaaatgca cgaatagtgc taagctggga agttcttcct 1440

gaggtctaac ctctagctgc tcccccacag aagagtgcct gcggccagtg gccaccaggg 1500

gtcgccgcag cacccagcgc tggagggcgg agcgggcggc agacccggag cagcaagtgg 1560

actctctcga gaagcttgaa ctgaaaaacc agaaagttaa ctggtaagtt tagtcttttt 1620

gtcttttatt tcaggtcccg gatccggtgg tggtgcaaat caaagaactg ctcctcagtg 1680

gatgttgcct ttacttctag gcctgtacgg aagtgttact tctgctctaa aagctgcgga 1740

attgtacccg cggccgatcc accggtatgt ggactctggg gaggcgcgcc gttgccggac 1800

tcctggcatc tccaagccca gcgcaggctc aaaccctcac gcgcgttccc aggccagccg 1860

agttggcccc cctttgtggc aggcgcggcc tgaggacaga tatcgacgcc acatgcactc 1920

ctcgccgcgc ctccagcaat caaaggggtt tgaaccaaat ttggaacgtc aagaagcaat 1980

cagtgtatct catgaatctg agaaagagcg gcacactcgg tcatcccgga agtctggacg 2040

aaaccacata cgaacggctc gccgaggaaa cgctggatag cctggccgag tttttcgagg 2100

acctggctga taaaccgtat actttcgaag attatgacgt gtcattcggg agcggcgtac 2160

tgaccgtcaa attgggcggt gacctgggga catacgtgat taacaagcag actcctaata 2220

aacagatttg gttgagctct ccttccagcg ggcctaaacg ctatgattgg acagggaaga 2280

attgggttta cagtcacgac ggagttagcc ttcacgagct cttggcagcg gagctgacaa 2340

aggcgctcaa gaccaaactc gacctgtctt cactcgccta ttcaggcaaa gacgcctgac 2400

tcgaggacgg ggtgaactac gcctgaggat ccgatctttt tccctctgcc aaaaattatg 2460

gggacatcat gaagcccctt gagcatctga cttctggcta ataaaggaaa tttattttca 2520

ttgcaatagt gtgttggaat tttttgtgtc tctcactcgg aagcaattcg ttgatctgaa 2580

tttcgaccac ccataatacc cattaccctg gtagataagt agcatggcgg gttaatcatt 2640

aactacaagg aacccctagt gatggagttg gccactccct ctctgcgcgc tcgctcgctc 2700

actgaggccg ggcgaccaaa ggtcgcccga cgcccgggct ttgcccgggc ggcctcagtg 2760

agcgagcgag cgcgcagcct taattaacct aattcactgg ccgtcgtttt acaacgtcgt 2820

gactgggaaa accctggcgt tacccaactt aatcgccttg cagcacatcc ccctttcgcc 2880

agctggcgta atagcgaaga ggcccgcacc gatcgccctt cccaacagtt gcgcagcctg 2940

aatggcgaat gggacgcgcc ctgtagcggc gcattaagcg cggcgggtgt ggtggttacg 3000

cgcagcgtga ccgctacact tgccagcgcc ctagcgcccg ctcctttcgc tttcttccct 3060

tcctttctcg ccacgttcgc cggctttccc cgtcaagctc taaatcgggg gctcccttta 3120

gggttccgat ttagtgcttt acggcacctc gaccccaaaa aacttgatta gggtgatggt 3180

tcacgtagtg ggccatcgcc ctgatagacg gtttttcgcc ctttgacgtt ggagtccacg 3240

ttctttaata gtggactctt gttccaaact ggaacaacac tcaaccctat ctcggtctat 3300

tcttttgatt tataagggat tttgccgatt tcggcctatt ggttaaaaaa tgagctgatt 3360

taacaaaaat ttaacgcgaa ttttaacaaa atattaacgc ttacaattta ggtggcactt 3420

ttcggggaaa tgtgcgcgga acccctattt gtttattttt ctaaatacat tcaaatatgt 3480

atccgctcat gagacaataa ccctgataaa tgcttcaata atattgaaaa aggaagagta 3540

tgagtattca acatttccgt gtcgccctta ttcccttttt tgcggcattt tgccttcctg 3600

tttttgctca cccagaaacg ctggtgaaag taaaagatgc tgaagatcag ttgggtgcac 3660

gagtgggtta catcgaactg gatctcaaca gcggtaagat ccttgagagt tttcgccccg 3720

aagaacgttt tccaatgatg agcactttta aagttctgct atgtggcgcg gtattatccc 3780

gtattgacgc cgggcaagag caactcggtc gccgcataca ctattctcag aatgacttgg 3840

ttgagtactc accagtcaca gaaaagcatc ttacggatgg catgacagta agagaattat 3900

gcagtgctgc cataaccatg agtgataaca ctgcggccaa cttacttctg acaacgatcg 3960

gaggaccgaa ggagctaacc gcttttttgc acaacatggg ggatcatgta actcgccttg 4020

atcgttggga accggagctg aatgaagcca taccaaacga cgagcgtgac accacgatgc 4080

ctgtagcaat ggcaacaacg ttgcgcaaac tattaactgg cgaactactt actctagctt 4140

cccggcaaca attaatagac tggatggagg cggataaagt tgcaggacca cttctgcgct 4200

cggcccttcc ggctggctgg tttattgctg ataaatctgg agccggtgag cgtgggtctc 4260

gcggtatcat tgcagcactg gggccagatg gtaagccctc ccgtatcgta gttatctaca 4320

cgacggggag tcaggcaact atggatgaac gaaatagaca gatcgctgag ataggtgcct 4380

cactgattaa gcattggtaa ctgtcagacc aagtttactc atatatactt tagattgatt 4440

taaaacttca tttttaattt aaaaggatct aggtgaagat cctttttgat aatctcatga 4500

ccaaaatccc ttaacgtgag ttttcgttcc actgagcgtc agaccccgta gaaaagatca 4560

aaggatcttc ttgagatcct ttttttctgc gcgtaatctg ctgcttgcaa acaaaaaaac 4620

caccgctacc agcggtggtt tgtttgccgg atcaagagct accaactctt tttccgaagg 4680

taactggctt cagcagagcg cagataccaa atactgttct tctagtgtag ccgtagttag 4740

gccaccactt caagaactct gtagcaccgc ctacatacct cgctctgcta atcctgttac 4800

cagtggctgc tgccagtggc gataagtcgt gtcttaccgg gttggactca agacgatagt 4860

taccggataa ggcgcagcgg tcgggctgaa cggggggttc gtgcacacag cccagcttgg 4920

agcgaacgac ctacaccgaa ctgagatacc tacagcgtga gctatgagaa agcgccacgc 4980

ttcccgaagg gagaaaggcg gacaggtatc cggtaagcgg cagggtcgga acaggagagc 5040

gcacgaggga gcttccaggg ggaaacgcct ggtatcttta tagtcctgtc gggtttcgcc 5100

acctctgact tgagcgtcga tttttgtgat gctcgtcagg ggggcggagc ctatggaaaa 5160

acgccagcaa cgcggccttt ttacggttcc tggccttttg ctggcctttt gctcacatgt 5220

tctttcctgc gttatcccct gattctgtgg ataaccgtat taccgccttt gagtgagctg 5280

ataccgctcg ccgcagccga acgaccgagc gcagcgagtc agtgagcgag gaagcggaag 5340

agcgcccaat acgcaaaccg cctctccccg cgcgttggcc gattcattaa tgcagctggc 5400

acgacaggtt tcccgactgg aaagcgggca gtgagcgcaa cgcaattaat gtgagttagc 5460

tcactcatta ggcaccccag gctttacact ttatgcttcc ggctcgtatg ttgtgtggaa 5520

ttgtgagcgg ataacaattt cacacaggaa acagctatga ccatgattac gccagattta 5580

attaag 5586

<210> 51
<211> 5586
<212> DNA
<213> Artificial Sequence

<220>
<223> Description of Artificial Sequence: Synthetic
      polynucleotide

<400> 51
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacga gctcccaggc tcaagtgatc 240

ctcccgcctc agcctcctga gtagctgaga tcacaggcat gcactaccac actcggctca 300

catttgacat cctctaaagc atatataaaa tgtgaagaaa actttcacaa tttgcatccc 360

tttgtaatat gtaacagaaa taaaattctc ttttaaaatc tatcaacaat aggcaaggca 420

cggtggctca cgcctgtcgt ctcagcactt tgtgaggccc aggcgggcag atcgtttgag 480

cctagaagtt caagaccacc ctgggcaaca tagcgaaacc ccctttctac aaaaaataca 540

aaaactagct gggtgtggtg gtgcacacct gtagtcccag ctacttggaa ggctgaaatg 600

ggaagactgc ttgagcccgg gagggagaag ttgcagtaag ccaggaccac accactgcac 660

tccagcctgg gcaacagagt gagactctgt ctcaaacaaa caaataaatg aggcgggtgg 720

atcacgaggt cagtagatcg agaccatcct ggctaacacg gtgaaacccg tctctactaa 780

aaaaaaaaaa aaatacaaaa aattagccag gcatggtggc gggcgcctgt agtcccagtt 840

actcgggagg ctgaggcagg agaatggcgt gaaaccggga ggcagagctt gcagtgagcc 900

gagatcgcac cactgccctc cagcctgggc gacagagcga gactccgtct caatcaatca 960

atcaatcaat aaaatctatt aacaatattt attgtgcact taacaggaac atgccctgtc 1020

caaaaaaaac tttacagggc ttaactcatt ttatccttac cacaatccta tgaagtagga 1080

acttttataa aacgcatttt ataaacaagg cacagagagg ttaattaact tgccctctgg 1140

tcacacagct aggaagtggg cagagtacag atttacacaa ggcatccgtc tcctggcccc 1200

acatacccaa ctgctgtaaa cccataccgg cggccaagca gcctcaattt gtgcatgcac 1260

ccacttccca gcaagacagc agctcccaag ttcctcctgt ttagaatttt agaagcggcg 1320

ggccaccagg ctgcagtctc ccttgggtca ggggtcctgg ttgcactccg tgctttgcac 1380

aaagcaggct ctccattttt gttaaatgca cgaatagtgc taagctggga agttcttcct 1440

gaggtctaac ctctagctgc tcccccacag aagagtgcct gcggccagtg gccaccaggg 1500

gtcgccgcag cacccagcgc tggagggcgg agcgggcggc agacccggag cagcaagtgg 1560

actctctcga gaagcttgaa ctgaaaaacc agaaagttaa ctggtaagtt tagtcttttt 1620

gtcttttatt tcaggtcccg gatccggtgg tggtgcaaat caaagaactg ctcctcagtg 1680

gatgttgcct ttacttctag gcctgtacgg aagtgttact tctgctctaa aagctgcgga 1740

attgtacccg cggccgatcc accggtatgt ggacactcgg gcgacgcgcc gtggctgggc 1800

tgcttgcttc cccaagtccc gcacaggcgc aaaccctcac aagggtccca cggccagctg 1860

aattggcccc gctgtgcggt cggaggggcc tgcgaacaga tatagacgca acgtgcaccc 1920

ccagaagagc aagcagcaac cagagaggcc tcaatcagat ttggaacgtc aagaaacaga 1980

gtgtctatct gatgaacctc cgcaaatcag ggacccttgg tcatccggga tctcttgatg 2040

aaacgaccta tgaacggctg gctgaggaga cgcttgactc cctggcagag ttcttcgagg 2100

atctggctga taagccatac acgttcgagg actatgatgt ttcttttggt tctggcgtcc 2160

tgacggtgaa actcggaggg gatctgggca cttacgttat caacaaacag actcccaata 2220

aacagatttg gttgtctagt cctagcagtg gcccaaaacg ctatgactgg acgggcaaga 2280

actgggtgta ctcccacgat ggagttagcc tgcacgaact tctggccgct gagcttacta 2340

aagcgctgaa gactaagctg gatctctcca gcctcgcata cagcggcaaa gacgcctagc 2400

tcgaggacgg ggtgaactac gcctgaggat ccgatctttt tccctctgcc aaaaattatg 2460

gggacatcat gaagcccctt gagcatctga cttctggcta ataaaggaaa tttattttca 2520

ttgcaatagt gtgttggaat tttttgtgtc tctcactcgg aagcaattcg ttgatctgaa 2580

tttcgaccac ccataatacc cattaccctg gtagataagt agcatggcgg gttaatcatt 2640

aactacaagg aacccctagt gatggagttg gccactccct ctctgcgcgc tcgctcgctc 2700

actgaggccg ggcgaccaaa ggtcgcccga cgcccgggct ttgcccgggc ggcctcagtg 2760

agcgagcgag cgcgcagcct taattaacct aattcactgg ccgtcgtttt acaacgtcgt 2820

gactgggaaa accctggcgt tacccaactt aatcgccttg cagcacatcc ccctttcgcc 2880

agctggcgta atagcgaaga ggcccgcacc gatcgccctt cccaacagtt gcgcagcctg 2940

aatggcgaat gggacgcgcc ctgtagcggc gcattaagcg cggcgggtgt ggtggttacg 3000

cgcagcgtga ccgctacact tgccagcgcc ctagcgcccg ctcctttcgc tttcttccct 3060

tcctttctcg ccacgttcgc cggctttccc cgtcaagctc taaatcgggg gctcccttta 3120

gggttccgat ttagtgcttt acggcacctc gaccccaaaa aacttgatta gggtgatggt 3180

tcacgtagtg ggccatcgcc ctgatagacg gtttttcgcc ctttgacgtt ggagtccacg 3240

ttctttaata gtggactctt gttccaaact ggaacaacac tcaaccctat ctcggtctat 3300

tcttttgatt tataagggat tttgccgatt tcggcctatt ggttaaaaaa tgagctgatt 3360

taacaaaaat ttaacgcgaa ttttaacaaa atattaacgc ttacaattta ggtggcactt 3420

ttcggggaaa tgtgcgcgga acccctattt gtttattttt ctaaatacat tcaaatatgt 3480

atccgctcat gagacaataa ccctgataaa tgcttcaata atattgaaaa aggaagagta 3540

tgagtattca acatttccgt gtcgccctta ttcccttttt tgcggcattt tgccttcctg 3600

tttttgctca cccagaaacg ctggtgaaag taaaagatgc tgaagatcag ttgggtgcac 3660

gagtgggtta catcgaactg gatctcaaca gcggtaagat ccttgagagt tttcgccccg 3720

aagaacgttt tccaatgatg agcactttta aagttctgct atgtggcgcg gtattatccc 3780

gtattgacgc cgggcaagag caactcggtc gccgcataca ctattctcag aatgacttgg 3840

ttgagtactc accagtcaca gaaaagcatc ttacggatgg catgacagta agagaattat 3900

gcagtgctgc cataaccatg agtgataaca ctgcggccaa cttacttctg acaacgatcg 3960

gaggaccgaa ggagctaacc gcttttttgc acaacatggg ggatcatgta actcgccttg 4020

atcgttggga accggagctg aatgaagcca taccaaacga cgagcgtgac accacgatgc 4080

ctgtagcaat ggcaacaacg ttgcgcaaac tattaactgg cgaactactt actctagctt 4140

cccggcaaca attaatagac tggatggagg cggataaagt tgcaggacca cttctgcgct 4200

cggcccttcc ggctggctgg tttattgctg ataaatctgg agccggtgag cgtgggtctc 4260

gcggtatcat tgcagcactg gggccagatg gtaagccctc ccgtatcgta gttatctaca 4320

cgacggggag tcaggcaact atggatgaac gaaatagaca gatcgctgag ataggtgcct 4380

cactgattaa gcattggtaa ctgtcagacc aagtttactc atatatactt tagattgatt 4440

taaaacttca tttttaattt aaaaggatct aggtgaagat cctttttgat aatctcatga 4500

ccaaaatccc ttaacgtgag ttttcgttcc actgagcgtc agaccccgta gaaaagatca 4560

aaggatcttc ttgagatcct ttttttctgc gcgtaatctg ctgcttgcaa acaaaaaaac 4620

caccgctacc agcggtggtt tgtttgccgg atcaagagct accaactctt tttccgaagg 4680

taactggctt cagcagagcg cagataccaa atactgttct tctagtgtag ccgtagttag 4740

gccaccactt caagaactct gtagcaccgc ctacatacct cgctctgcta atcctgttac 4800

cagtggctgc tgccagtggc gataagtcgt gtcttaccgg gttggactca agacgatagt 4860

taccggataa ggcgcagcgg tcgggctgaa cggggggttc gtgcacacag cccagcttgg 4920

agcgaacgac ctacaccgaa ctgagatacc tacagcgtga gctatgagaa agcgccacgc 4980

ttcccgaagg gagaaaggcg gacaggtatc cggtaagcgg cagggtcgga acaggagagc 5040

gcacgaggga gcttccaggg ggaaacgcct ggtatcttta tagtcctgtc gggtttcgcc 5100

acctctgact tgagcgtcga tttttgtgat gctcgtcagg ggggcggagc ctatggaaaa 5160

acgccagcaa cgcggccttt ttacggttcc tggccttttg ctggcctttt gctcacatgt 5220

tctttcctgc gttatcccct gattctgtgg ataaccgtat taccgccttt gagtgagctg 5280

ataccgctcg ccgcagccga acgaccgagc gcagcgagtc agtgagcgag gaagcggaag 5340

agcgcccaat acgcaaaccg cctctccccg cgcgttggcc gattcattaa tgcagctggc 5400

acgacaggtt tcccgactgg aaagcgggca gtgagcgcaa cgcaattaat gtgagttagc 5460

tcactcatta ggcaccccag gctttacact ttatgcttcc ggctcgtatg ttgtgtggaa 5520

ttgtgagcgg ataacaattt cacacaggaa acagctatga ccatgattac gccagattta 5580

attaag 5586

<210> 52
<211> 5586
<212> DNA
<213> Artificial Sequence

<220>
<223> Description of Artificial Sequence: Synthetic
      polynucleotide

<400> 52
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacga gctcccaggc tcaagtgatc 240

ctcccgcctc agcctcctga gtagctgaga tcacaggcat gcactaccac actcggctca 300

catttgacat cctctaaagc atatataaaa tgtgaagaaa actttcacaa tttgcatccc 360

tttgtaatat gtaacagaaa taaaattctc ttttaaaatc tatcaacaat aggcaaggca 420

cggtggctca cgcctgtcgt ctcagcactt tgtgaggccc aggcgggcag atcgtttgag 480

cctagaagtt caagaccacc ctgggcaaca tagcgaaacc ccctttctac aaaaaataca 540

aaaactagct gggtgtggtg gtgcacacct gtagtcccag ctacttggaa ggctgaaatg 600

ggaagactgc ttgagcccgg gagggagaag ttgcagtaag ccaggaccac accactgcac 660

tccagcctgg gcaacagagt gagactctgt ctcaaacaaa caaataaatg aggcgggtgg 720

atcacgaggt cagtagatcg agaccatcct ggctaacacg gtgaaacccg tctctactaa 780

aaaaaaaaaa aaatacaaaa aattagccag gcatggtggc gggcgcctgt agtcccagtt 840

actcgggagg ctgaggcagg agaatggcgt gaaccgggga ggcagagctt gcagtgagcc 900

gagatcgcac cactgccctc cagcctgggc gacagagcga gactccgtct caatcaatca 960

atcaatcaat aaaatctatt aacaatattt attgtgcact taacaggaac atgccctgtc 1020

caaaaaaaac tttacagggc ttaactcatt ttatccttac cacaatccta tgaagtagga 1080

acttttataa aacgcatttt ataaacaagg cacagagagg ttaattaact tgccctctgg 1140

tcacacagct aggaagtggg cagagtacag atttacacaa ggcatccgtc tcctggcccc 1200

acatacccaa ctgctgtaaa cccataccgg cggccaagca gcctcaattt gtgcatgcac 1260

ccacttccca gcaagacagc agctcccaag ttcctcctgt ttagaatttt agaagcggcg 1320

ggccaccagg ctgcagtctc ccttgggtca ggggtcctgg ttgcactccg tgctttgcac 1380

aaagcaggct ctccattttt gttaaatgca cgaatagtgc taagctggga agttcttcct 1440

gaggtctaac ctctagctgc tcccccacag aagagtgcct gcggccagtg gccaccaggg 1500

gtcgccgcag cacccagcgc tggagggcgg agcgggcggc agacccggag cagcaagtgg 1560

actctctcga gaagcttgaa ctgaaaaacc agaaagttaa ctggtaagtt tagtcttttt 1620

gtcttttatt tcaggtcccg gatccggtgg tggtgcaaat caaagaactg ctcctcagtg 1680

gatgttgcct ttacttctag gcctgtacgg aagtgttact tctgctctaa aagctgcgga 1740

attgtacccg cggccgatcc accggtatgt ggactctggg tcgacgcgcc gtagccggtc 1800

tcttggcctc cccatcccct gcgcaggcac agacccttac acgagttcca cggcccgctg 1860

aactggcacc actctgcgga cgccggggcc tgagaaccga tattgacgct acgtgcactc 1920

cccgacgagc ctcttctaat cagagggggc tcaaccagat ctggaacgtg aagaagcagt 1980

cagtctactt gatgaatctg agaaagagcg ggacccttgg gcaccccggg tctctcgacg 2040

agactacata cgagaggctg gccgaggaga ctttggactc tctcgccgaa ttcttcgagg 2100

atttggcaga caagccgtac acctttgaag actacgatgt ctcattcgga tcaggggtgc 2160

tgacggttaa actggggggc gatctcggaa cctacgtcat aaacaaacag acacccaata 2220

agcagatctg gttgtctagc ccatcatccg gtcctaaacg gtacgactgg accggcaaga 2280

actgggtcta ctcccatgac ggagtcagtc ttcatgaact gcttgccgca gagctgacca 2340

aggctcttaa gacgaaactg gatctctcta gcctggccta ctccgggaag gatgcatagc 2400

tcgaggacgg ggtgaactac gcctgaggat ccgatctttt tccctctgcc aaaaattatg 2460

gggacatcat gaagcccctt gagcatctga cttctggcta ataaaggaaa tttattttca 2520

ttgcaatagt gtgttggaat tttttgtgtc tctcactcgg aagcaattcg ttgatctgaa 2580

tttcgaccac ccataatacc cattaccctg gtagataagt agcatggcgg gttaatcatt 2640
aactacaagg aacccctagt gatggagttg gccactccct ctctgcgcgc tcgctcgctc 2700
actgaggccg ggcgaccaaa ggtcgcccga cgcccgggct ttgcccgggc ggcctcagtg 2760
agcgagcgag cgcgcagcct taattaacct aattcactgg ccgtcgtttt acaacgtcgt 2820
gactgggaaa accctggcgt tacccaactt aatcgccttg cagcacatcc ccctttcgcc 2880
agctggcgta atagcgaaga ggcccgcacc gatcgccctt cccaacagtt gcgcagcctg 2940
aatggcgaat gggacgcgcc ctgtagcggc gcattaagcg cggcgggtgt ggtggttacg 3000
cgcagcgtga ccgctacact tgccagcgcc ctagcgcccg ctcctttcgc tttcttccct 3060
tcctttctcg ccacgttcgc cggctttccc cgtcaagctc taaatcgggg gctcccttta 3120
gggttccgat ttagtgcttt acggcacctc gaccccaaaa aacttgatta gggtgatggt 3180
tcacgtagtg ggccatcgcc ctgatagacg gtttttcgcc ctttgacgtt ggagtccacg 3240
ttctttaata gtggactctt gttccaaact ggaacaacac tcaaccctat ctcggtctat 3300
tcttttgatt tataagggat tttgccgatt tcggcctatt ggttaaaaaa tgagctgatt 3360
taacaaaaat ttaacgcgaa ttttaacaaa atattaacgc ttacaattta ggtggcactt 3420
ttcggggaaa tgtgcgcgga acccctattt gtttattttt ctaaatacat tcaaatatgt 3480
atccgctcat gagacaataa ccctgataaa tgcttcaata atattgaaaa aggaagagta 3540
tgagtattca acatttccgt gtcgccctta ttcccttttt tgcggcattt tgccttcctg 3600
tttttgctca cccagaaacg ctggtgaaag taaaagatgc tgaagatcag ttgggtgcac 3660
gagtgggtta catcgaactg gatctcaaca gcggtaagat ccttgagagt tttcgccccg 3720
aagaacgttt tccaatgatg agcactttta aagttctgct atgtggcgcg gtattatccc 3780
gtattgacgc cgggcaagag caactcggtc gccgcataca ctattctcag aatgacttgg 3840
ttgagtactc accagtcaca gaaaagcatc ttacggatgg catgacagta agagaattat 3900
gcagtgctgc cataaccatg agtgataaca ctgcggccaa cttacttctg acaacgatcg 3960
gaggaccgaa ggagctaacc gcttttttgc acaacatggg ggatcatgta actcgccttg 4020
atcgttggga accggagctg aatgaagcca taccaaacga cgagcgtgac accacgatgc 4080
ctgtagcaat ggcaacaacg ttgcgcaaac tattaactgg cgaactactt actctagctt 4140
cccggcaaca attaatagac tggatggagg cggataaagt tgcaggacca cttctgcgct 4200
cggcccttcc ggctggctgg tttattgctg ataaatctgg agccggtgag cgtgggtctc 4260
gcggtatcat tgcagcactg gggccagatg gtaagccctc ccgtatcgta gttatctaca 4320
cgacggggag tcaggcaact atggatgaac gaaatagaca gatcgctgag ataggtgcct 4380
cactgattaa gcattggtaa ctgtcagacc aagtttactc atatatactt tagattgatt 4440
taaaacttca tttttaattt aaaaggatct aggtgaagat cctttttgat aatctcatga 4500

ccaaaatccc ttaacgtgag ttttcgttcc actgagcgtc agaccccgta gaaaagatca 4560

aaggatcttc ttgagatcct ttttttctgc gcgtaatctg ctgcttgcaa acaaaaaaac 4620

caccgctacc agcggtggtt tgtttgccgg atcaagagct accaactctt tttccgaagg 4680

taactggctt cagcagagcg cagataccaa atactgttct tctagtgtag ccgtagttag 4740

gccaccactt caagaactct gtagcaccgc ctacatacct cgctctgcta atcctgttac 4800

cagtggctgc tgccagtggc gataagtcgt gtcttaccgg gttggactca agacgatagt 4860

taccggataa ggcgcagcgg tcgggctgaa cggggggttc gtgcacacag cccagcttgg 4920

agcgaacgac ctacaccgaa ctgagatacc tacagcgtga gctatgagaa agcgccacgc 4980

ttcccgaagg gagaaaggcg gacaggtatc cggtaagcgg cagggtcgga acaggagagc 5040

gcacgaggga gcttccaggg ggaaacgcct ggtatcttta tagtcctgtc gggtttcgcc 5100

acctctgact tgagcgtcga tttttgtgat gctcgtcagg ggggcggagc ctatggaaaa 5160

acgccagcaa cgcggccttt ttacggttcc tggccttttg ctggcctttt gctcacatgt 5220

tctttcctgc gttatcccct gattctgtgg ataaccgtat taccgccttt gagtgagctg 5280

ataccgctcg ccgcagccga acgaccgagc gcagcgagtc agtgagcgag gaagcggaag 5340

agcgcccaat acgcaaaccg cctctccccg cgcgttggcc gattcattaa tgcagctggc 5400

acgacaggtt tcccgactgg aaagcgggca gtgagcgcaa cgcaattaat gtgagttagc 5460

tcactcatta ggcaccccag gctttacact ttatgcttcc ggctcgtatg ttgtgtggaa 5520

ttgtgagcgg ataacaattt cacacaggaa acagctatga ccatgattac gccagattta 5580

attaag 5586

<210> 53
<211> 6434
<212> DNA
<213> Artificial Sequence

<220>
<223> Description of Artificial Sequence: Synthetic
      polynucleotide

<400> 53
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggacgcgtcc cacctccctc tctgtgctgg gactcacaga gggagacctc 240

aggaggcagt ctgtccatca catgtccaaa tgcagagcat accctgggct gggcgcagtg 300

gcgcacaact gtaattccag cactttggga ggctgatgtg gaaggatcac ttgagcccag 360

aagttctaga ccagcctggg caacatggca agaccctatc tctacaaaaa aagttaaaaa 420

atcagccacg tgtggtgaca cacacctgta gtcccagcta ttcaggaggc tgaggtgagg 480

ggatcactta aggctgggag gttgaggctg cagtgagtcg tggttgcgcc actgcactcc 540

agcctgggca acagtgagac cctgtctcaa aagacaaaaa aaaaaaaaaa aaaaaaaaga 600

acatatcctg gtgtggagta ggggacgctg ctctgacaga ggctcggggg cctgagctgg 660

ctctgtgagc tggggaggag gcagacagcc aggccttgtc tgcaagcaga cctggcagca 720

ttgggctggc cgccccccag ggcctcctct tcatgcccag tgaatgactc accttggcac 780

agacacaatg ttcggggtgg gcacagtgcc tgcttcccgc cgcaccccag cccccctcaa 840

atgccttccg agaagcccat tgagcagggg gcttgcattg caccccagcc tgacagcctg 900

gcatcttggg ataaaagcag cacagccccc taggggctgc ccttgctgtg tggcgccacc 960

ggcggtggag aacaaggctc tattcagcct gtgcccagga aaggggatca ggggatgccc 1020

aggcatggac agtgggtggc agggggggag aggagggctg tctgcttccc agaagtccaa 1080

ggacacaaat gggtgagggg actgggcagg gttctgaccc tgtgggacca gagtggaggg 1140

cgtagatgga cctgaagtct ccagggacaa cagggcccag gtctcaggct cctagttggg 1200

cccagtggct ccagcgtttc caaacccatc catccccaga ggttcttccc atctctccag 1260

gctgatgtgt gggaactcga ggaaataaat ctccagtggg agacggaggg gtggccaggg 1320

aaacggggcg ctgcaggaat aaagacgagc cagcacagcc agctcatgtg taacggcttt 1380

gtggagctgt caaggcctgg tctctgggag agaggcacag ggaggccaga caaggaaggg 1440

gtgacctgga gggacagatc caggggctaa agtcctgata aggcaagaga gtgccggccc 1500

cctcttgccc tatcaggacc tccactgcca catagaggcc atgattgacc cttagacaaa 1560

gggctggtgt ccaatcccag cccccagccc cagaactcca gggaatgaat gggcagagag 1620

caggaatgtg ggacatctgt gttcaaggga aggactccag gagtctgctg ggaatgaggc 1680

ctagtaggaa atgaggtggc ccttgagggt acagaacagg ttcattcttc gccaaattcc 1740

cagcaccttg caggcactta cagctgagtg agataatgcc tgggttatga aatcaaaaag 1800

ttggaaagca ggtcagaggt catctggtac agcccttcct tccctttttt tttttttttt 1860

tttgtgagac aaggtctctc tctgttgccc aggctggagt ggcgcaaaca cagctcactg 1920

cagcctcaac ctactgggct caagcaatcc tccagcctca gcctcccaaa gtgctgggat 1980

tacaagcatg agccacccca ctcagccctt tccttccttt ttaattgatg cataataatt 2040

gtaagtattc atcatggtcc aaccaaccct ttcttgaccc accttcctag agagagggtc 2100

ctcttgcttc agcggtcagg gccccagacc catggtctgg ctccaggtac cacctgcctc 2160

atgcaggagt tggcgtgccc aggaagctct gcctctgggc acagtgacct cagtggggtg 2220

aggggagctc tccccatagc tgggctgcgg cccaacccca ccccctcagg ctatgccagg 2280

gggtgttgcc aggggcaccc gggcatcgcc agtctagccc actccttcat aaagccctcg 2340
catcccagga gcgagcagag ccagagcagg ttggagagga gacgcatcac ctccgctgct 2400
cgccgggatc ctcgggagca agcttgaact gaaaaaccag aaagttaact ggtaagttta 2460
gtctttttgt cttttatttc aggtcccgga tccggtggtg gtgcaaatca aagaactgct 2520
cctcagtgga tgttgccttt acttctaggc ctgtacggaa gtgttacttc tgctctaaaa 2580
gctgcggaat tgtacccgcg gccgatccac cggtatgtgg actctcgggc gccgcgcagt 2640
agccggcctc ctggcgtcac ccagcccggc ccaggcccag accctcaccc gggtcccgcg 2700
gccggcagag ttggccccac tctgcggccg ccgtggcctg cgcaccgaca tcgatgcgac 2760
ctgcacgccc cgccgcgcaa gttcgaacca acgtggcctc aaccagattt ggaatgtcaa 2820
aaagcagagt gtctatttga tgaatttgag gaaatctgga actttgggcc acccaggctc 2880
tctagatgag accacctatg aaagactagc agaggaaacg ctggactctt tagcagagtt 2940
ttttgaagac cttgcagaca agccatacac gtttgaggac tatgatgtct cctttgggag 3000
tggtgtctta actgtcaaac tgggtggaga tctaggaacc tatgtgatca acaagcagac 3060
gccaaacaag caaatctggc tatcttctcc atccagtgga cctaagcgtt atgactggac 3120
tgggaaaaac tgggtgttct cccacgacgg cgtgtccctc catgagctgc tggccgcaga 3180
gctcactaaa gccttaaaaa ccaaactgga cttgtcttgg ttggcctatt ccggaaaaga 3240
tgcttgactc gaggacgggg tgaactacgc ctgaggatcc gatctttttc cctctgccaa 3300
aaattatggg gacatcatga agccccttga gcatctgact tctggctaat aaaggaaatt 3360
tattttcatt gcaatagtgt gttggaattt tttgtgtctc tcactcggaa gcaattcgtt 3420
gatctgaatt tcgaccaccc ataataccca ttaccctggt agataagtag catggcgggt 3480
taatcattaa ctacaaggaa cccctagtga tggagttggc cactccctct ctgcgcgctc 3540
gctcgctcac tgaggccggg cgaccaaagg tcgcccgacg cccgggcttt gcccgggcgg 3600
cctcagtgag cgagcgagcg cgcagcctta attaacctaa ttcactggcc gtcgttttac 3660
aacgtcgtga ctgggaaaac cctggcgtta cccaacttaa tcgccttgca gcacatcccc 3720
ctttcgccag ctggcgtaat agcgaagagg cccgcaccga tcgcccttcc caacagttgc 3780
gcagcctgaa tggcgaatgg gacgcgccct gtagcggcgc attaagcgcg gcgggtgtgg 3840
tggttacgcg cagcgtgacc gctacacttg ccagcgccct agcgcccgct cctttcgctt 3900
tcttcccttc ctttctcgcc acgttcgccg gctttccccg tcaagctcta aatcgggggc 3960
tccctttagg gttccgattt agtgctttac ggcacctcga ccccaaaaaa cttgattagg 4020
gtgatggttc acgtagtggg ccatcgccct gatagacggt ttttcgccct ttgacgttgg 4080
agtccacgtt ctttaatagt ggactcttgt tccaaactgg aacaacactc aaccctatct 4140
cggtctattc ttttgattta taagggattt tgccgatttc ggcctattgg ttaaaaaatg 4200

agctgattta acaaaaattt aacgcgaatt ttaacaaaat attaacgctt acaatttagg 4260

tggcactttt cggggaaatg tgcgcggaac ccctatttgt ttatttttct aaatacattc 4320

aaatatgtat ccgctcatga gacaataacc ctgataaatg cttcaataat attgaaaaag 4380

gaagagtatg agtattcaac atttccgtgt cgcccttatt cccttttttg cggcattttg 4440

ccttcctgtt tttgctcacc cagaaacgct ggtgaaagta aaagatgctg aagatcagtt 4500

gggtgcacga gtgggttaca tcgaactgga tctcaacagc ggtaagatcc ttgagagttt 4560

tcgccccgaa gaacgttttc caatgatgag cacttttaaa gttctgctat gtggcgcggt 4620

attatcccgt attgacgccg ggcaagagca actcggtcgc cgcatacact attctcagaa 4680

tgacttggtt gagtactcac cagtcacaga aaagcatctt acggatggca tgacagtaag 4740

agaattatgc agtgctgcca taaccatgag tgataacact gcggccaact tacttctgac 4800

aacgatcgga ggaccgaagg agctaaccgc ttttttgcac aacatggggg atcatgtaac 4860

tcgccttgat cgttgggaac cggagctgaa tgaagccata ccaaacgacg agcgtgacac 4920

cacgatgcct gtagcaatgg caacaacgtt gcgcaaacta ttaactggcg aactacttac 4980

tctagcttcc cggcaacaat taatagactg gatggaggcg gataaagttg caggaccact 5040

tctgcgctcg gcccttccgg ctggctggtt tattgctgat aaatctggag ccggtgagcg 5100

tgggtctcgc ggtatcattg cagcactggg gccagatggt aagccctccc gtatcgtagt 5160

tatctacacg acggggagtc aggcaactat ggatgaacga aatagacaga tcgctgagat 5220

aggtgcctca ctgattaagc attggtaact gtcagaccaa gtttactcat atatacttta 5280

gattgattta aaacttcatt tttaatttaa aaggatctag gtgaagatcc tttttgataa 5340

tctcatgacc aaaatccctt aacgtgagtt ttcgttccac tgagcgtcag accccgtaga 5400

aaagatcaaa ggatcttctt gagatccttt ttttctgcgc gtaatctgct gcttgcaaac 5460

aaaaaaacca ccgctaccag cggtggtttg tttgccggat caagagctac caactctttt 5520

tccgaaggta actggcttca gcagagcgca gataccaaat actgttcttc tagtgtagcc 5580

gtagttaggc caccacttca agaactctgt agcaccgcct acatacctcg ctctgctaat 5640

cctgttacca gtggctgctg ccagtggcga taagtcgtgt cttaccgggt tggactcaag 5700

acgatagtta ccggataagg cgcagcggtc gggctgaacg gggggttcgt gcacacagcc 5760

cagcttggag cgaacgacct acaccgaact gagataccta cagcgtgagc tatgagaaag 5820

cgccacgctt cccgaaggga gaaaggcgga caggtatccg gtaagcggca gggtcggaac 5880

aggagagcgc acgagggagc ttccaggggg aaacgcctgg tatctttata gtcctgtcgg 5940

gtttcgccac ctctgacttg agcgtcgatt tttgtgatgc tcgtcagggg ggcggagcct 6000

atggaaaaac gccagcaacg cggccttttt acggttcctg gccttttgct ggccttttgc 6060

tcacatgttc tttcctgcgt tatcccctga ttctgtggat aaccgtatta ccgcctttga 6120

gtgagctgat accgctcgcc gcagccgaac gaccgagcgc agcgagtcag tgagcgagga 6180

agcggaagag cgcccaatac gcaaaccgcc tctccccgcg cgttggccga ttcattaatg 6240

cagctggcac gacaggtttc ccgactggaa agcgggcagt gagcgcaacg caattaatgt 6300

gagttagctc actcattagg caccccaggc tttacacttt atgcttccgg ctcgtatgtt 6360

gtgtggaatt gtgagcggat aacaatttca cacaggaaac agctatgacc atgattacgc 6420

cagatttaat taag 6434

<210> 54
<211> 4790
<212> DNA
<213> Artificial Sequence

<220>
<223> Description of Artificial Sequence: Synthetic
      polynucleotide

<400> 54
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacta gtatctgcag agggccctgc 240

gtatgagtgc aagtgggttt taggaccagg atgaggcggg gtgggggtgc ctacctgacg 300

accgaccccg acccactgga caagcaccca acccccattc cccaaattgc gcatccccta 360

tcagagaggg ggaggggaaa caggatgcgg cgaggcgcgt gcgcactgcc agcttcagca 420

ccgcggacag tgccttcgcc cccgcctggc ggcgcgcgcc accgccgcct cagcactgaa 480

ggcgcgctga cgtcactcgc cggtcccccg caaactcccc ttcccggcca ccttggtcgc 540

gtccgcgccg ccgccggccc agccggaccg caccacgcga ggcgcgagat aggggggcac 600

gggcgcgacc atctgcgctg cggcgccggc gactcagcgc tgcctcagtc tgcggtgggc 660

agcggaggag tcgtgtcgtg cctgagagcg cagctgtgct cctgggcacc gcgcagtccg 720

cccccgcggc tcctggccag accaccccta ggaccccctg ccccaagtcg cagccaagct 780

tgaactgaaa aaccagaaag ttaactggta agtttagtct ttttgtcttt tatttcaggt 840

cccggatccg gtggtggtgc aaatcaaaga actgctcctc agtggatgtt gcctttactt 900

ctaggcctgt acggaagtgt tacttctgct ctaaaagctg cggaattgta cccgcggccg 960

atccaccggt atgtggactc tcgggcgccg cgcagtagcc ggcctcctgg cgtcacccag 1020

cccagcccag gcccagaccc tcacccgggt cccgcggccg gcagagttgg ccccactctg 1080

cggccgccgt ggcctgcgca ccgacatcga tgcgacctgc acgccccgcc gcgcaagttc 1140

gaaccaacgt ggcctcaacc agatttggaa tgtcaaaaag cagagtgtct atttgatgaa 1200

tttgaggaaa tctggaactt tgggccaccc aggctctcta gatgagacca cctatgaaag 1260

actagcagag gaaacgctgg actctttagc agagtttttt gaagaccttg cagacaagcc 1320

atacacgttt gaggactatg atgtctcctt tgggagtggt gtcttaactg tcaaactggg 1380

tggagatcta ggaacctatg tgatcaacaa gcagacgcca aacaagcaaa tctggctatc 1440

ttctccatcc agtggaccta agcgttatga ctggactggg aaaaactggg tgtactccca 1500

cgacggcgtg tccctccatg agctgctggc cgcagagctc actaaagcct taaaaaccaa 1560

actggacttg tcttccttgg cctattccgg aaaagatgct tgactcgagg acggggtgaa 1620

ctacgcctga ggatccgatc tttttccctc tgccaaaaat tatggggaca tcatgaagcc 1680

ccttgagcat ctgacttctg gctaataaag gaaatttatt ttcattgcaa tagtgtgttg 1740

gaattttttg tgtctctcac tcggaagcaa ttcgttgatc tgaatttcga ccacccataa 1800

tacccattac cctggtagat aagtagcatg gcgggttaat cattaactac aaggaacccc 1860

tagtgatgga gttggccact ccctctctgc gcgctcgctc gctcactgag gccgggcgac 1920

caaaggtcgc ccgacgcccg ggctttgccc gggcggcctc agtgagcgag cgagcgcgca 1980

gccttaatta acctaattca ctggccgtcg ttttacaacg tcgtgactgg gaaaaccctg 2040

gcgttaccca acttaatcgc cttgcagcac atcccccttt cgccagctgg cgtaatagcg 2100

aagaggcccg caccgatcgc ccttcccaac agttgcgcag cctgaatggc gaatgggacg 2160

cgccctgtag cggcgcatta agcgcggcgg gtgtggtggt tacgcgcagc gtgaccgcta 2220

cacttgccag cgccctagcg cccgctcctt tcgctttctt cccttccttt ctcgccacgt 2280

tcgccggctt tccccgtcaa gctctaaatc gggggctccc tttagggttc cgatttagtg 2340

ctttacggca cctcgacccc aaaaaacttg attagggtga tggttcacgt agtgggccat 2400

cgccctgata gacggttttt cgccctttga cgttggagtc cacgttcttt aatagtggac 2460

tcttgttcca aactggaaca acactcaacc ctatctcggt ctattctttt gatttataag 2520

ggattttgcc gatttcggcc tattggttaa aaaatgagct gatttaacaa aaatttaacg 2580

cgaattttaa caaaatatta acgcttacaa tttaggtggc acttttcggg gaaatgtgcg 2640

cggaacccct atttgtttat ttttctaaat acattcaaat atgtatccgc tcatgagaca 2700

ataaccctga taaatgcttc aataatattg aaaaaggaag agtatgagta ttcaacattt 2760

ccgtgtcgcc cttattccct tttttgcggc attttgcctt cctgtttttg ctcacccaga 2820

aacgctggtg aaagtaaaag atgctgaaga tcagttgggt gcacgagtgg gttacatcga 2880

actggatctc aacagcggta agatccttga gagttttcgc cccgaagaac gttttccaat 2940

gatgagcact tttaaagttc tgctatgtgg cgcggtatta tcccgtattg acgccgggca 3000

agagcaactc ggtcgccgca tacactattc tcagaatgac ttggttgagt actcaccagt 3060

cacagaaaag catcttacgg atggcatgac agtaagagaa ttatgcagtg ctgccataac 3120

catgagtgat aacactgcgg ccaacttact tctgacaacg atcggaggac cgaaggagct 3180

aaccgctttt ttgcacaaca tgggggatca tgtaactcgc cttgatcgtt gggaaccgga 3240

gctgaatgaa gccataccaa acgacgagcg tgacaccacg atgcctgtag caatggcaac 3300

aacgttgcgc aaactattaa ctggcgaact acttactcta gcttcccggc aacaattaat 3360

agactggatg gaggcggata aagttgcagg accacttctg cgctcggccc ttccggctgg 3420

ctggtttatt gctgataaat ctggagccgg tgagcgtggg tctcgcggta tcattgcagc 3480

actggggcca gatggtaagc cctcccgtat cgtagttatc tacacgacgg ggagtcaggc 3540

aactatggat gaacgaaata gacagatcgc tgagataggt gcctcactga ttaagcattg 3600

gtaactgtca gaccaagttt actcatatat actttagatt gatttaaaac ttcattttta 3660

atttaaaagg atctaggtga agatcctttt tgataatctc atgaccaaaa tcccttaacg 3720

tgagttttcg ttccactgag cgtcagaccc cgtagaaaag atcaaaggat cttcttgaga 3780

tccttttttt ctgcgcgtaa tctgctgctt gcaaacaaaa aaaccaccgc taccagcggt 3840

ggtttgtttg ccggatcaag agctaccaac tctttttccg aaggtaactg gcttcagcag 3900

agcgcagata ccaaatactg ttcttctagt gtagccgtag ttaggccacc acttcaagaa 3960

ctctgtagca ccgcctacat acctcgctct gctaatcctg ttaccagtgg ctgctgccag 4020

tggcgataag tcgtgtctta ccgggttgga ctcaagacga tagttaccgg ataaggcgca 4080

gcggtcgggc tgaacggggg gttcgtgcac acagcccagc ttggagcgaa cgacctacac 4140

cgaactgaga tacctacagc gtgagctatg agaaagcgcc acgcttcccg aagggagaaa 4200

ggcggacagg tatccggtaa gcggcagggt cggaacagga gagcgcacga gggagcttcc 4260

agggggaaac gcctggtatc tttatagtcc tgtcgggttt cgccacctct gacttgagcg 4320

tcgatttttg tgatgctcgt caggggggcg gagcctatgg aaaaacgcca gcaacgcggc 4380

ctttttacgg ttcctggcct tttgctggcc ttttgctcac atgttctttc ctgcgttatc 4440

ccctgattct gtggataacc gtattaccgc ctttgagtga gctgataccg ctcgccgcag 4500

ccgaacgacc gagcgcagcg agtcagtgag cgaggaagcg gaagagcgcc caatacgcaa 4560

accgcctctc cccgcgcgtt ggccgattca ttaatgcagc tggcacgaca ggtttcccga 4620

ctggaaagcg ggcagtgagc gcaacgcaat taatgtgagt tagctcactc attaggcacc 4680

ccaggcttta cactttatgc ttccggctcg tatgttgtgt ggaattgtga gcggataaca 4740

atttcacaca ggaaacagct atgaccatga ttacgccaga tttaattaag 4790

<210> 55
<211> 4880
<212> DNA

<213> Artificial Sequence

<220>
<223> Description of Artificial Sequence: Synthetic
      polynucleotide

<400> 55
gccttaatta ggctgcgcgc tcgctcgctc actgaggccg cccgggcaaa gcccgggcgt      60

cgggcgacct ttggtcgccc ggcctcagtg agcgagcgag cgcgcagaga gggagtggcc     120

aactccatca ctaggggttc cttgtagtta atgattaacc cgccatgcta cttatctacc     180

agggtaatgg ggatcctcta gaactatagc tagtcgacga gtcaatggga aaaacccatt     240

ggagccaagt acactgactc aatagggact ttccattggg ttttgcccag tacataaggt     300

caataggggg tgagtcaaca ggaaagtccc attggagcca agtacattga gtcaataggg     360

actttccaat gggttttgcc cagtacataa ggtcaatggg aggtaagcca atgggttttt     420

cccattactg acatgtatac tgagtcatta gggactttcc aatgggtttt gcccagtaca     480

taaggtcaat aggggtgaat caacaggaaa gtcccattgg agccaagtac actgagtcaa     540

tagggacttt ccattgggtt ttgcccagta caaaaggtca atagggggtg agtcaatggg     600

tttttcccat tattggcaca tacataaggt caataggggt gactagtgga gaagagcatg     660

cttgagggct gagtgcccct cagtgggcag agagcacatg gcccacagtc cctgagaagt     720

tggggggagg ggtgggcaat tgaactggtg cctagagaag gtggggcttg ggtaaactgg     780

gaaagtgatg tggtgtactg gctccacctt tttccccagg gtgggggaga accatatata     840

agtgcagtag tctctgtgaa cattcaagct tgaactgaaa aaccagaaag ttaactggta     900

agtttagtct ttttgtcttt tatttcaggt cccggatccg gtggtggtgc aaatcaaaga     960

actgctcctc agtggatgtt gcctttactt ctaggcctgt acggaagtgt tacttctgct    1020

ctaaaagctg cggaattgta cccgcggccg atccaccggt atgtggactc tcgggcgccg    1080

cgcagtagcc ggcctcctgg cgtcacccag cccagcccag gcccagaccc tcacccgggt    1140

cccgcggccg gcagagttgg ccccactctg cggccgccgt ggcctgcgca ccgacatcga    1200

tgcgacctgc acgccccgcc gcgcaagttc gaaccaacgt ggcctcaacc agatttggaa    1260

tgtcaaaaag cagagtgtct atttgatgaa tttgaggaaa tctggaactt tgggccaccc    1320

aggctctcta gatgagacca cctatgaaag actagcagag gaaacgctgg actctttagc    1380

agagtttttt gaagaccttg cagacaagcc atacacgttt gaggactatg atgtctcctt    1440

tgggagtggt gtcttaactg tcaaactggg tggagatcta ggaacctatg tgatcaacaa    1500

gcagacgcca aacaagcaaa tctggctatc ttctccatcc agtggaccta agcgttatga    1560

ctggactggg aaaaactggg tgtactccca cgacggcgtg tccctccatg agctgctggc    1620

cgcagagctc actaaagcct taaaaaccaa actggacttg tcttccttgg cctattccgg    1680

aaaagatgct tgactcgagg acggggtgaa ctacgcctga ggatccgatc tttttccctc 1740

tgccaaaaat tatggggaca tcatgaagcc ccttgagcat ctgacttctg gctaataaag 1800

gaaatttatt ttcattgcaa tagtgtgttg gaattttttg tgtctctcac tcggaagcaa 1860

ttcgttgatc tgaatttcga ccacccataa tacccattac cctggtagat aagtagcatg 1920

gcgggttaat cattaactac aaggaacccc tagtgatgga gttggccact ccctctctgc 1980

gcgctcgctc gctcactgag gccgggcgac caaaggtcgc ccgacgcccg ggctttgccc 2040

gggcggcctc agtgagcgag cgagcgcgca gccttaatta acctaattca ctggccgtcg 2100

ttttacaacg tcgtgactgg gaaaaccctg gcgttaccca acttaatcgc cttgcagcac 2160

atcccccttt cgccagctgg cgtaatagcg aagaggcccg caccgatcgc ccttcccaac 2220

agttgcgcag cctgaatggc gaatgggacg cgccctgtag cggcgcatta agcgcggcgg 2280

gtgtggtggt tacgcgcagc gtgaccgcta cacttgccag cgccctagcg cccgctcctt 2340

tcgctttctt cccttccttt ctcgccacgt tcgccggctt tccccgtcaa gctctaaatc 2400

gggggctccc tttagggttc cgatttagtg ctttacggca cctcgacccc aaaaaacttg 2460

attagggtga tggttcacgt agtgggccat cgccctgata gacggttttt cgccctttga 2520

cgttggagtc cacgttcttt aatagtggac tcttgttcca aactggaaca acactcaacc 2580

ctatctcggt ctattctttt gatttataag ggattttgcc gatttcggcc tattggttaa 2640

aaaatgagct gatttaacaa aaatttaacg cgaattttaa caaaatatta acgcttacaa 2700

tttaggtggc acttttcggg gaaatgtgcg cggaacccct atttgtttat ttttctaaat 2760

acattcaaat atgtatccgc tcatgagaca ataaccctga taaatgcttc aataatattg 2820

aaaaaggaag agtatgagta ttcaacattt ccgtgtcgcc cttattccct tttttgcggc 2880

attttgcctt cctgtttttg ctcacccaga aacgctggtg aaagtaaaag atgctgaaga 2940

tcagttgggt gcacgagtgg gttacatcga actggatctc aacagcggta agatccttga 3000

gagttttcgc cccgaagaac gttttccaat gatgagcact tttaaagttc tgctatgtgg 3060

cgcggtatta tcccgtattg acgccgggca agagcaactc ggtcgccgca tacactattc 3120

tcagaatgac ttggttgagt actcaccagt cacagaaaag catcttacgg atggcatgac 3180

agtaagagaa ttatgcagtg ctgccataac catgagtgat aacactgcgg ccaacttact 3240

tctgacaacg atcggaggac cgaaggagct aaccgctttt ttgcacaaca tgggggatca 3300

tgtaactcgc cttgatcgtt gggaaccgga gctgaatgaa gccataccaa acgacgagcg 3360

tgacaccacg atgcctgtag caatggcaac aacgttgcgc aaactattaa ctggcgaact 3420

acttactcta gcttcccggc aacaattaat agactggatg gaggcggata aagttgcagg 3480

accacttctg cgctcggccc ttccggctgg ctggtttatt gctgataaat ctggagccgg 3540

tgagcgtggg tctcgcggta tcattgcagc actggggcca gatggtaagc cctcccgtat 3600

cgtagttatc tacacgacgg ggagtcaggc aactatggat gaacgaaata gacagatcgc 3660

tgagataggt gcctcactga ttaagcattg gtaactgtca gaccaagttt actcatatat 3720

actttagatt gatttaaaac ttcattttta atttaaaagg atctaggtga agatcctttt 3780

tgataatctc atgaccaaaa tcccttaacg tgagttttcg ttccactgag cgtcagaccc 3840

cgtagaaaag atcaaaggat cttcttgaga tccttttttt ctgcgcgtaa tctgctgctt 3900

gcaaacaaaa aaaccaccgc taccagcggt ggtttgtttg ccggatcaag agctaccaac 3960

tctttttccg aaggtaactg gcttcagcag agcgcagata ccaaatactg ttcttctagt 4020

gtagccgtag ttaggccacc acttcaagaa ctctgtagca ccgcctacat acctcgctct 4080

gctaatcctg ttaccagtgg ctgctgccag tggcgataag tcgtgtctta ccgggttgga 4140

ctcaagacga tagttaccgg ataaggcgca gcggtcgggc tgaacggggg gttcgtgcac 4200

acagcccagc ttggagcgaa cgacctacac cgaactgaga tacctacagc gtgagctatg 4260

agaaagcgcc acgcttcccg aagggagaaa ggcggacagg tatccggtaa gcggcagggt 4320

cggaacagga gagcgcacga gggagcttcc agggggaaac gcctggtatc tttatagtcc 4380

tgtcgggttt cgccacctct gacttgagcg tcgatttttg tgatgctcgt caggggggcg 4440

gagcctatgg aaaaacgcca gcaacgcggc ctttttacgg ttcctggcct tttgctggcc 4500

ttttgctcac atgttctttc ctgcgttatc ccctgattct gtggataacc gtattaccgc 4560

ctttgagtga gctgataccg ctcgccgcag ccgaacgacc gagcgcagcg agtcagtgag 4620

cgaggaagcg gaagagcgcc caatacgcaa accgcctctc cccgcgcgtt ggccgattca 4680

ttaatgcagc tggcacgaca ggtttcccga ctggaaagcg ggcagtgagc gcaacgcaat 4740

taatgtgagt tagctcactc attaggcacc ccaggcttta cactttatgc ttccggctcg 4800

tatgttgtgt ggaattgtga gcggataaca atttcacaca ggaaacagct atgaccatga 4860

ttacgccaga tttaattaag 4880

<210> 56
<211> 4881
<212> DNA
<213> Artificial Sequence

<220>
<223> Description of Artificial Sequence: Synthetic
      polynucleotide

<400> 56
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacga gtcaatggga aaaacccatt 240

ggagccaagt acactgactc aatagggact ttccattggg ttttgcccag tacataaggt 300

caataggggg tgagtcaaca ggaaagtccc attggagcca agtacattga gtcaataggg 360

actttccaat gggttttgcc cagtacataa ggtcaatggg aggtaagcca atgggttttt 420

cccattactg acatgtatac tgagtcatta gggactttcc aatgggtttt gcccagtaca 480

taaggtcaat aggggtgaat caacaggaaa gtcccattgg agccaagtac actgagtcaa 540

tagggacttt ccattgggtt ttgcccagta caaaaggtca atagggggtg agtcaatggg 600

tttttcccat tattggcaca tacataaggt caataggggt gactagtcag tgggcagagc 660

gcacatcgcc cacagtcccc gagaagttgg ggggaggggt cggcaattga accggtgcct 720

agagaaggtg gcgcggggta aactgggaaa gtgatgtcgt gtactggctc cgcctttttc 780

ccgagggtgg gggagaaccg tatataagtg cagtagtcgc cgtgaacgtt ctttttcgca 840

acgggtttgc cgccagaaca cagctgaagc ttgaactgaa aaaccagaaa gttaactggt 900

aagtttagtc tttttgtctt ttatttcagg tcccggatcc ggtggtggtg caaatcaaag 960

aactgctcct cagtggatgt tgcctttact tctaggcctg tacggaagtg ttacttctgc 1020

tctaaaagct gcggaattgt acccgcggcc gatccaccgg tatgtggact ctcgggcgcc 1080

gcgcagtagc cggcctcctg gcgtcaccca gcccagccca ggcccagacc ctcacccggg 1140

tcccgcggcc ggcagagttg gccccactct gcggccgccg tggcctgcgc accgacatcg 1200

atgcgacctg cacgccccgc cgcgcaagtt cgaaccaacg tggcctcaac cagatttgga 1260

atgtcaaaaa gcagagtgtc tatttgatga atttgaggaa atctggaact ttgggccacc 1320

caggctctct agatgagacc acctatgaaa gactagcaga ggaaacgctg gactctttag 1380

cagagttttt tgaagacctt gcagacaagc catacacgtt tgaggactat gatgtctcct 1440

ttgggagtgg tgtcttaact gtcaaactgg gtggagatct aggaacctat gtgatcaaca 1500

agcagacgcc aaacaagcaa atctggctat cttctccatc cagtggacct aagcgttatg 1560

actggactgg gaaaaactgg gtgtactccc acgacggcgt gtccctccat gagctgctgg 1620

ccgcagagct cactaaagcc ttaaaaacca aactggactt gtcttccttg gcctattccg 1680

gaaaagatgc ttgactcgag gacggggtga actacgcctg aggatccgat ctttttccct 1740

ctgccaaaaa ttatggggac atcatgaagc cccttgagca tctgacttct ggctaataaa 1800

ggaaatttat tttcattgca atagtgtgtt ggaatttttt gtgtctctca ctcggaagca 1860

attcgttgat ctgaatttcg accacccata ataccattta ccctggtaga taagtagcat 1920

ggcgggttaa tcattaacta caaggaaccc ctagtgatgg agttggccac tccctctctg 1980

cgcgctcgct cgctcactga ggccgggcga ccaaaggtcg cccgacgccc gggctttgcc 2040

cgggcggcct cagtgagcga gcgagcgcgc agccttaatt aacctaattc actggccgtc 2100

gttttacaac gtcgtgactg ggaaaaccct ggcgttaccc aacttaatcg ccttgcagca 2160

catccccctt tcgccagctg gcgtaatagc gaagaggccc gcaccgatcg cccttcccaa 2220

cagttgcgca gcctgaatgg cgaatgggac gcgccctgta gcggcgcatt aagcgcggcg 2280

ggtgtggtgg ttacgcgcag cgtgaccgct acacttgcca gcgccctagc gcccgctcct 2340

ttcgctttct tcccttcctt tctcgccacg ttcgccggct ttccccgtca agctctaaat 2400

cgggggctcc ctttagggtt ccgatttagt gctttacggc acctcgaccc caaaaaactt 2460

gattagggtg atggttcacg tagtgggcca tcgccctgat agacggtttt tcgccctttg 2520

acgttggagt ccacgttctt taatagtgga ctcttgttcc aaactggaac aacactcaac 2580

cctatctcgg tctattcttt tgatttataa gggattttgc cgatttcggc ctattggtta 2640

aaaaatgagc tgatttaaca aaaatttaac gcgaatttta acaaaatatt aacgcttaca 2700

atttaggtgg cacttttcgg ggaaatgtgc gcggaacccc tatttgttta tttttctaaa 2760

tacattcaaa tatgtatccg ctcatgagac aataaccctg ataaatgctt caataatatt 2820

gaaaaaggaa gagtatgagt attcaacatt tccgtgtcgc ccttattccc ttttttgcgg 2880

cattttgcct tcctgttttt gctcacccag aaacgctggt gaaagtaaaa gatgctgaag 2940

atcagttggg tgcacgagtg ggttacatcg aactggatct caacagcggt aagatccttg 3000

agagttttcg ccccgaagaa cgttttccaa tgatgagcac ttttaaagtt ctgctatgtg 3060

gcgcggtatt atcccgtatt gacgccgggc aagagcaact cggtcgccgc atacactatt 3120

ctcagaatga cttggttgag tactcaccag tcacagaaaa gcatcttacg gatggcatga 3180

cagtaagaga attatgcagt gctgccataa ccatgagtga taacactgcg gccaacttac 3240

ttctgacaac gatcggagga ccgaaggagc taaccgcttt tttgcacaac atgggggatc 3300

atgtaactcg ccttgatcgt tgggaaccgg agctgaatga agccatacca aacgacgagc 3360

gtgacaccac gatgcctgta gcaatggcaa caacgttgcg caaactatta actggcgaac 3420

tacttactct agcttcccgg caacaattaa tagactggat ggaggcggat aaagttgcag 3480

gaccacttct gcgctcggcc cttccggctg gctggtttat tgctgataaa tctggagccg 3540

gtgagcgtgg gtctcgcggt atcattgcag cactggggcc agatggtaag ccctcccgta 3600

tcgtagttat ctacacgacg gggagtcagg caactatgga tgaacgaaat agacagatcg 3660

ctgagatagg tgcctcactg attaagcatt ggtaactgtc agaccaagtt tactcatata 3720

tactttagat tgatttaaaa cttcattttt aatttaaaag gatctaggtg aagatccttt 3780

ttgataatct catgaccaaa atcccttaac gtgagttttc gttccactga gcgtcagacc 3840

ccgtagaaaa gatcaaagga tcttcttgag atcctttttt tctgcgcgta atctgctgct 3900

tgcaaacaaa aaaaccaccg ctaccagcgg tggtttgttt gccggatcaa gagctaccaa 3960

ctctttttcc gaaggtaact ggcttcagca gagcgcagat accaaatact gttcttctag 4020

tgtagccgta gttaggccac cacttcaaga actctgtagc accgcctaca tacctcgctc 4080

tgctaatcct gttaccagtg gctgctgcca gtggcgataa gtcgtgtctt accgggttgg 4140

actcaagacg atagttaccg gataaggcgc agcggtcggg ctgaacgggg ggttcgtgca 4200

cacagcccag cttggagcga acgacctaca ccgaactgag atacctacag cgtgagctat 4260

gagaaagcgc cacgcttccc gaagggagaa aggcggacag gtatccggta agcggcaggg 4320

tcggaacagg agagcgcacg agggagcttc cagggggaaa cgcctggtat ctttatagtc 4380

ctgtcgggtt tcgccacctc tgacttgagc gtcgattttt gtgatgctcg tcaggggggc 4440

ggagcctatg gaaaaacgcc agcaacgcgg cctttttacg gttcctggcc ttttgctggc 4500

cttttgctca catgttcttt cctgcgttat cccctgattc tgtggataac cgtattaccg 4560

cctttgagtg agctgatacc gctcgccgca gccgaacgac cgagcgcagc gagtcagtga 4620

gcgaggaagc ggaagagcgc ccaatacgca aaccgcctct ccccgcgcgt tggccgattc 4680

attaatgcag ctggcacgac aggtttcccg actggaaagc gggcagtgag cgcaacgcaa 4740

ttaatgtgag ttagctcact cattaggcac cccaggcttt acactttatg cttccggctc 4800

gtatgttgtg tggaattgtg agcggataac aatttcacac aggaaacagc tatgaccatg 4860

attacgccag atttaattaa g 4881

<210> 57
<211> 4746
<212> DNA
<213> Artificial Sequence

<220>
<223> Description of Artificial Sequence: Synthetic
      polynucleotide

<400> 57
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagt ccacggggtt ggggttgcgc cttttccaag 240

gcagccctgg gtttgcgcag ggacgcggct gctctgggcg tggttccggg aaacgcagcg 300

gcgccgaccc tgggtctcgc acattcttca cgtccgttcg cagcgtcacc cggatcttcg 360

ccgctaccct tgtgggcccc ccggcgacgc ttcctcgtcc gcccctaagt cgggaaggtt 420

ccttgcggtt cgcggcgtgc cggacgtgac aaacggaagc cgcacgtctc actagtaccc 480

tcgcagacgg acagcgccag ggagcaatgg cagcgcgccg accgcgatgg gctgtggcca 540

atagcggctg ctcagcaggg cgcgccgaga gcagcggccg ggaaggggcg gtgcgggagg 600

cggggtgtgg ggcggtagtg tgggccctgt tcctgcccgc gcggtgttcc gcattctgca 660

agcctccgga gcgcacgtcg gcagtcggct ccctcgttga ccgaatcacc gacctctctc 720

cccacgggag caagcttgaa ctgaaaaacc agaaagttaa ctggtaagtt tagtcttttt 780

gtcttttatt tcaggtcccg gatccggtgg tggtgcaaat caaagaactg ctcctcagtg 840

gatgttgcct ttacttctag gcctgtacgg aagtgttact tctgctctaa aagctgcgga 900

attgtacccg cggccgatcc accggtatgt ggactctcgg gcgccgcgca gtagccggcc 960

tcctggcgtc acccagccca gcccaggccc agaccctcac ccgggtcccg cggccggcag 1020

agttggcccc actctgcggc cgccgtggcc tgcgcaccga catcgatgcg acctgcacgc 1080

cccgccgcgc aagttcgaac caacgtggcc tcaaccagat ttggaatgtc aaaaagcaga 1140

gtgtctattt gatgaatttg aggaaatctg gaactttggg ccacccaggc tctctagatg 1200

agaccaccta tgaaagacta gcagaggaaa cgctggactc tttagcagag ttttttgaag 1260

accttgcaga caagccatac acgtttgagg actatgatgt ctcctttggg agtggtgtct 1320

taactgtcaa actgggtgga gatctaggaa cctatgtgat caacaagcag acgccaaaca 1380

agcaaatctg gctatcttct ccatccagtg gacctaagcg ttatgactgg actgggaaaa 1440

actgggtgta ctcccacgac ggcgtgtccc tccatgagct gctggccgca gagctcacta 1500

aagccttaaa aaccaaactg gacttgtctt ccttggccta ttccggaaaa gatgcttgac 1560

tcgaggacgg ggtgaactac gcctgaggat ccgatctttt tccctctgcc aaaaattatg 1620

gggacatcat gaagcccctt gagcatctga cttctggcta ataaaggaaa tttattttca 1680

ttgcaatagt gtgttggaat tttttgtgtc tctcactcgg aagcaattcg ttgatctgaa 1740

tttcgaccac ccataatacc cattaccctg gtagataagt agcatggcgg gttaatcatt 1800

aactacaagg aacccctagt gatggagttg gccactccct ctctgcgcgc tcgctcgctc 1860

actgaggccg ggcgaccaaa ggtcgcccga cgcccgggct ttgcccgggc ggcctcagtg 1920

agcgagcgag cgcgcagcct taattaacct aattcactgg ccgtcgtttt acaacgtcgt 1980

gactgggaaa accctggcgt tacccaactt aatcgccttg cagcacatcc ccctttcgcc 2040

agctggcgta atagcgaaga ggcccgcacc gatcgccctt cccaacagtt gcgcagcctg 2100

aatggcgaat gggacgcgcc ctgtagcggc gcattaagcg cggcgggtgt ggtggttacg 2160

cgcagcgtga ccgctacact tgccagcgcc ctagcgcccg ctcctttcgc tttcttccct 2220

tcctttctcg ccacgttcgc cggctttccc cgtcaagctc taaatcgggg gctcccttta 2280

gggttccgat ttagtgcttt acggcacctc gaccccaaaa aacttgatta gggtgatggt 2340

tcacgtagtg ggccatcgcc ctgatagacg gtttttcgcc ctttgacgtt ggagtccacg 2400

ttctttaata gtggactctt gttccaaact ggaacaacac tcaaccctat ctcggtctat 2460

tcttttgatt tataagggat tttgccgatt tcggcctatt ggttaaaaaa tgagctgatt 2520

taacaaaaat ttaacgcgaa ttttaacaaa atattaacgc ttacaattta ggtggcactt 2580

ttcggggaaa tgtgcgcgga acccctattt gtttattttt ctaaatacat tcaaatatgt 2640

atccgctcat gagacaataa ccctgataaa tgcttcaata atattgaaaa aggaagagta 2700

tgagtattca acatttccgt gtcgccctta ttcccttttt tgcggcattt tgccttcctg 2760

tttttgctca cccagaaacg ctggtgaaag taaaagatgc tgaagatcag ttgggtgcac 2820

gagtgggtta catcgaactg gatctcaaca gcggtaagat ccttgagagt tttcgccccg 2880

aagaacgttt tccaatgatg agcactttta aagttctgct atgtggcgcg gtattatccc 2940

gtattgacgc cgggcaagag caactcggtc gccgcataca ctattctcag aatgacttgg 3000

ttgagtactc accagtcaca gaaaagcatc ttacggatgg catgacagta agagaattat 3060

gcagtgctgc cataaccatg agtgataaca ctgcggccaa cttacttctg acaacgatcg 3120

gaggaccgaa ggagctaacc gcttttttgc acaacatggg ggatcatgta actcgccttg 3180

atcgttggga accggagctg aatgaagcca taccaaacga cgagcgtgac accacgatgc 3240

ctgtagcaat ggcaacaacg ttgcgcaaac tattaactgg cgaactactt actctagctt 3300

cccggcaaca attaatagac tggatggagg cggataaagt tgcaggacca cttctgcgct 3360

cggcccttcc ggctggctgg tttattgctg ataaatctgg agccggtgag cgtgggtctc 3420

gcggtatcat tgcagcactg gggccagatg gtaagccctc ccgtatcgta gttatctaca 3480

cgacggggag tcaggcaact atggatgaac gaaatagaca gatcgctgag ataggtgcct 3540

cactgattaa gcattggtaa ctgtcagacc aagtttactc atatatactt tagattgatt 3600

taaaacttca tttttaattt aaaaggatct aggtgaagat cctttttgat aatctcatga 3660

ccaaaatccc ttaacgtgag ttttcgttcc actgagcgtc agaccccgta gaaaagatca 3720

aaggatcttc ttgagatcct ttttttctgc gcgtaatctg ctgcttgcaa acaaaaaaac 3780

caccgctacc agcggtggtt tgtttgccgg atcaagagct accaactctt tttccgaagg 3840

taactggctt cagcagagcg cagataccaa atactgttct tctagtgtag ccgtagttag 3900

gccaccactt caagaactct gtagcaccgc ctacatacct cgctctgcta atcctgttac 3960

cagtggctgc tgccagtggc gataagtcgt gtcttaccgg gttggactca agacgatagt 4020

taccggataa ggcgcagcgg tcgggctgaa cggggggttc gtgcacacag cccagcttgg 4080

agcgaacgac ctacaccgaa ctgagatacc tacagcgtga gctatgagaa agcgccacgc 4140

ttcccgaagg gagaaaggcg gacaggtatc cggtaagcgg cagggtcgga acaggagagc 4200

gcacgaggga gcttccaggg ggaaacgcct ggtatcttta tagtcctgtc gggtttcgcc 4260

acctctgact tgagcgtcga tttttgtgat gctcgtcagg ggggcggagc ctatggaaaa 4320

acgccagcaa cgcggccttt ttacggttcc tggccttttg ctggcctttt gctcacatgt 4380

tctttcctgc gttatcccct gattctgtgg ataaccgtat taccgccttt gagtgagctg 4440

ataccgctcg ccgcagccga acgaccgagc gcagcgagtc agtgagcgag gaagcggaag 4500

agcgcccaat acgcaaaccg cctctccccg cgcgttggcc gattcattaa tgcagctggc 4560

acgacaggtt tcccgactgg aaagcgggca gtgagcgcaa cgcaattaat gtgagttagc 4620

tcactcatta ggcaccccag gctttacact ttatgcttcc ggctcgtatg ttgtgtggaa 4680

ttgtgagcgg ataacaattt cacacaggaa acagctatga ccatgattac gccagattta 4740

attaag 4746

<210> 58
<211> 4746
<212> DNA
<213> Artificial Sequence

<220>
<223> Description of Artificial Sequence: Synthetic
      polynucleotide

<400> 58
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagt ccacggggtt ggggttgcgc cttttccaag 240

gcagccctgg gtttgcgcag ggacgcggct gctctgggcg tggttccggg aaacgcagcg 300

gcgccgaccc tgggtctcgc acattcttca cgtccgttcg cagcgtcacc cggatcttcg 360

ccgctaccct tgtgggcccc ccggcgacgc ttcctcgtcc gcccctaagt cgggaaggtt 420

ccttgcggtt cgcggcgtgc cggacgtgac aaacggaagc cgcacgtctc actagtaccc 480

tcgcagacgg acagcgccag ggagcaatgg cagcgcgccg accgcgatgg gctgtggcca 540

atagcggctg ctcagcaggg cgcgccgaga gcagcggccg ggaaggggcg gtgcgggagg 600

cggggtgtgg ggcggtagtg tgggccctgt tcctgcccgc gcggtgttcc gcattctgca 660

agcctccgga gcgcacgtcg gcagtcggct ccctcgttga ccgaatcacc gacctctctc 720

cccacgggag caagcttgaa ctgaaaaacc agaaagttaa ctggtaagtt tagtcttttt 780

gtcttttatt tcaggtcccg gatccggtgg tggtgcaaat caaagaactg ctcctcagtg 840

gatgttgcct ttacttctag gcctgtacgg aagtgttact tctgctctaa aagctgcgga 900

attgtacccg cggccgatcc accggtatgt ggactctggg tcgcagagcg gtggcaggct 960

tgctggcatc tccgtccccg gctcaggccc agaccctgac acgggtgccc aggccggccg 1020

agctggctcc tctgtgcggc cggcgcggcc ttagaacaga cattgacgca acctgcaccc 1080

ccagaagagc cagttccaac cagcgcgggt tgaatcagat ctggaacgtc aagaagcaga 1140

gtgtgtacct tatgaatctc cggaaaagtg gcaccctcgg acaccccgga tccctcgatg 1200

aaaccactta tgagcgcctg gccgaggaaa ccctggattc cctggctgag ttcttcgagg 1260

acctggctga caaaccctac accttcgaag actacgacgt ttccttcgga agtggggtgc 1320

tgacggtcaa acttggtggc gatcttggaa cctatgttat aaacaagcag actcccaaca 1380

aacagatctg gctcagctct ccaagttcag gacccaagag gtatgactgg acgggcaaga 1440

attgggtcta ttctcacgac ggagtgagcc ttcatgaact gttggctgcc gaactgacaa 1500

aagcccttaa gacgaaactg gacctgtcct cattggcgta ctctggtaag gatgcctaac 1560

tcgaggacgg ggtgaactac gcctgaggat ccgatctttt tccctctgcc aaaaattatg 1620

gggacatcat gaagcccctt gagcatctga cttctggcta ataaaggaaa tttattttca 1680

ttgcaatagt gtgttggaat tttttgtgtc tctcactcgg aagcaattcg ttgatctgaa 1740

tttcgaccac ccataatacc cattaccctg gtagataagt agcatggcgg gttaatcatt 1800

aactacaagg aacccctagt gatggagttg gccactccct ctctgcgcgc tcgctcgctc 1860

actgaggccg ggcgaccaaa ggtcgcccga cgcccgggct ttgcccgggc ggcctcagtg 1920

agcgagcgag cgcgcagcct taattaacct aattcactgg ccgtcgtttt acaacgtcgt 1980

gactgggaaa accctggcgt tacccaactt aatcgccttg cagcacatcc ccctttcgcc 2040

agctggcgta atagcgaaga ggcccgcacc gatcgccctt cccaacagtt gcgcagcctg 2100

aatggcgaat gggacgcgcc ctgtagcggc gcattaagcg cggcgggtgt ggtggttacg 2160

cgcagcgtga ccgctacact tgccagcgcc ctagcgcccg ctcctttcgc tttcttccct 2220

tcctttctcg ccacgttcgc cggctttccc cgtcaagctc taaatcgggg gctcccttta 2280

gggttccgat ttagtgcttt acggcacctc gaccccaaaa aacttgatta gggtgatggt 2340

tcacgtagtg ggccatcgcc ctgatagacg gtttttcgcc ctttgacgtt ggagtccacg 2400

ttctttaata gtggactctt gttccaaact ggaacaacac tcaaccctat ctcggtctat 2460

tcttttgatt tataagggat tttgccgatt tcggcctatt ggttaaaaaa tgagctgatt 2520

taacaaaaat ttaacgcgaa ttttaacaaa atattaacgc ttacaattta ggtggcactt 2580

ttcggggaaa tgtgcgcgga acccctattt gtttattttt ctaaatacat tcaaatatgt 2640

atccgctcat gagacaataa ccctgataaa tgcttcaata atattgaaaa aggaagagta 2700

tgagtattca acatttccgt gtcgccctta ttcccttttt tgcggcattt tgccttcctg 2760

tttttgctca cccagaaacg ctggtgaaag taaaagatgc tgaagatcag ttgggtgcac 2820

gagtgggtta catcgaactg gatctcaaca gcggtaagat ccttgagagt tttcgccccg 2880

aagaacgttt tccaatgatg agcactttta aagttctgct atgtggcgcg gtattatccc 2940

gtattgacgc cgggcaagag caactcggtc gccgcataca ctattctcag aatgacttgg 3000

ttgagtactc accagtcaca gaaaagcatc ttacggatgg catgacagta agagaattat 3060

gcagtgctgc cataaccatg agtgataaca ctgcggccaa cttacttctg acaacgatcg 3120

gaggaccgaa ggagctaacc gcttttttgc acaacatggg ggatcatgta actcgccttg 3180

atcgttggga accggagctg aatgaagcca taccaaacga cgagcgtgac accacgatgc 3240

ctgtagcaat ggcaacaacg ttgcgcaaac tattaactgg cgaactactt actctagctt 3300

cccggcaaca attaatagac tggatggagg cggataaagt tgcaggacca cttctgcgct 3360

cggcccttcc ggctggctgg tttattgctg ataaatctgg agccggtgag cgtgggtctc 3420

gcggtatcat tgcagcactg gggccagatg gtaagccctc ccgtatcgta gttatctaca 3480

cgacggggag tcaggcaact atggatgaac gaaatagaca gatcgctgag ataggtgcct 3540

cactgattaa gcattggtaa ctgtcagacc aagtttactc atatatactt tagattgatt 3600

taaaacttca tttttaattt aaaaggatct aggtgaagat cctttttgat aatctcatga 3660

ccaaaatccc ttaacgtgag ttttcgttcc actgagcgtc agaccccgta gaaaagatca 3720

aaggatcttc ttgagatcct ttttttctgc gcgtaatctg ctgcttgcaa acaaaaaaac 3780

caccgctacc agcggtggtt tgtttgccgg atcaagagct accaactctt tttccgaagg 3840

taactggctt cagcagagcg cagataccaa atactgttct tctagtgtag ccgtagttag 3900

gccaccactt caagaactct gtagcaccgc ctacatacct cgctctgcta atcctgttac 3960

cagtggctgc tgccagtggc gataagtcgt gtcttaccgg gttggactca agacgatagt 4020

taccggataa ggcgcagcgg tcgggctgaa cggggggttc gtgcacacag cccagcttgg 4080

agcgaacgac ctacaccgaa ctgagatacc tacagcgtga gctatgagaa agcgccacgc 4140

ttcccgaagg gagaaaggcg gacaggtatc cggtaagcgg cagggtcgga acaggagagc 4200

gcacgaggga gcttccaggg ggaaacgcct ggtatcttta tagtcctgtc gggtttcgcc 4260

acctctgact tgagcgtcga tttttgtgat gctcgtcagg ggggcggagc ctatggaaaa 4320

acgccagcaa cgcggccttt ttacggttcc tggccttttg ctggcctttt gctcacatgt 4380

tctttcctgc gttatcccct gattctgtgg ataaccgtat taccgccttt gagtgagctg 4440

ataccgctcg ccgcagccga acgaccgagc gcagcgagtc agtgagcgag gaagcggaag 4500

agcgcccaat acgcaaaccg cctctccccg cgcgttggcc gattcattaa tgcagctggc 4560

acgacaggtt tcccgactgg aaagcgggca gtgagcgcaa cgcaattaat gtgagttagc 4620

tcactcatta ggcaccccag gctttacact ttatgcttcc ggctcgtatg ttgtgtggaa 4680

ttgtgagcgg ataacaattt cacacaggaa acagctatga ccatgattac gccagattta 4740

attaag 4746

<210> 59
<211> 4746
<212> DNA
<213> Artificial Sequence

<220>
<223> Description of Artificial Sequence: Synthetic
      polynucleotide

<400> 59
gccttaatta ggctgcgcgc tcgctcgctc actgaggccg cccgggcaaa gcccgggcgt      60

cgggcgacct ttggtcgccc ggcctcagtg agcgagcgag cgcgcagaga gggagtggcc     120

aactccatca ctaggggttc cttgtagtta atgattaacc cgccatgcta cttatctacc     180

agggtaatgg ggatcctcta gaactatagt ccacggggtt ggggttgcgc cttttccaag     240

gcagccctgg gtttgcgcag ggacgcggct gctctgggcg tggttccggg aaacgcagcg     300

gcgccgaccc tgggtctcgc acattcttca cgtccgttcg cagcgtcacc cggatcttcg     360

ccgctaccct tgtgggcccc ccggcgacgc ttcctcgtcc gcccctaagt cgggaaggtt     420

ccttgcggtt cgcggcgtgc cggacgtgac aaacggaagc cgcacgtctc actagtaccc     480

tcgcagacgg acagcgccag ggagcaatgg cagcgcgccg accgcgatgg gctgtggcca     540

atagcggctg ctcagcaggg cgcgccgaga gcagcggccg ggaaggggcg gtgcgggagg     600

cggggtgtgg ggcggtagtg tgggccctgt tcctgcccgc gcggtgttcc gcattctgca     660

agcctccgga gcgcacgtcg gcagtcggct ccctcgttga ccgaatcacc gacctctctc     720

cccacgggag caagcttgaa ctgaaaaacc agaaagttaa ctggtaagtt tagtcttttt     780

gtcttttatt tcaggtcccg gatccggtgg tggtgcaaat caaagaactg ctcctcagtg     840

gatgttgcct ttacttctag gcctgtacgg aagtgttact tctgctctaa aagctgcgga     900

attgtacccg cggccgatcc accggtatgt ggactctggg gaggcgcgcc gttgccggac     960

tcctggcatc tccaagccca gcgcaggctc aaaccctcac gcgcgttccc aggccagccg    1020

agttggcccc cctttgtggc aggcgcggcc tgaggacaga tatcgacgcc acatgcactc    1080

ctcgccgcgc ctccagcaat caaaggggtt tgaaccaaat ttggaacgtc aagaagcaat    1140

cagtgtatct catgaatctg agaaagagcg gcacactcgg tcatcccgga agtctggacg    1200

aaaccacata cgaacggctc gccgaggaaa cgctggatag cctggccgag tttttcgagg    1260

acctggctga taaaccgtat actttcgaag attatgacgt gtcattcggg agcggcgtac    1320

tgaccgtcaa attgggcggt gacctgggga catacgtgat taacaagcag actcctaata    1380

aacagatttg gttgagctct ccttccagcg ggcctaaacg ctatgattgg acagggaaga    1440

attgggttta cagtcacgac ggagttagcc ttcacgagct cttggcagcg gagctgacaa    1500

aggcgctcaa gaccaaactc gacctgtctt cactcgccta ttcaggcaaa gacgcctgac    1560

tcgaggacgg ggtgaactac gcctgaggat ccgatctttt tccctctgcc aaaaattatg    1620

gggacatcat gaagcccctt gagcatctga cttctggcta ataaaggaaa tttattttca    1680

ttgcaatagt gtgttggaat tttttgtgtc tctcactcgg aagcaattcg ttgatctgaa    1740

tttcgaccac ccataatacc cattaccctg gtagataagt agcatggcgg gttaatcatt 1800

aactacaagg aacccctagt gatggagttg gccactccct ctctgcgcgc tcgctcgctc 1860

actgaggccg ggcgaccaaa ggtcgcccga cgcccgggct ttgcccgggc ggcctcagtg 1920

agcgagcgag cgcgcagcct taattaacct aattcactgg ccgtcgtttt acaacgtcgt 1980

gactgggaaa accctggcgt tacccaactt aatcgccttg cagcacatcc ccctttcgcc 2040

agctggcgta atagcgaaga ggcccgcacc gatcgccctt cccaacagtt gcgcagcctg 2100

aatggcgaat gggacgcgcc ctgtagcggc gcattaagcg cggcgggtgt ggtggttacg 2160

cgcagcgtga ccgctacact tgccagcgcc ctagcgcccg ctcctttcgc tttcttccct 2220

tcctttctcg ccacgttcgc cggctttccc cgtcaagctc taaatcgggg gctcccttta 2280

gggttccgat ttagtgcttt acggcacctc gaccccaaaa aacttgatta gggtgatggt 2340

tcacgtagtg ggccatcgcc ctgatagacg gtttttcgcc ctttgacgtt ggagtccacg 2400

ttctttaata gtggactctt gttccaaact ggaacaacac tcaaccctat ctcggtctat 2460

tcttttgatt tataagggat tttgccgatt tcggcctatt ggttaaaaaa tgagctgatt 2520

taacaaaaat ttaacgcgaa ttttaacaaa atattaacgc ttacaattta ggtggcactt 2580

ttcggggaaa tgtgcgcgga acccctattt gtttattttt ctaaatacat tcaaatatgt 2640

atccgctcat gagacaataa ccctgataaa tgcttcaata atattgaaaa aggaagagta 2700

tgagtattca acatttccgt gtcgccctta ttcccttttt tgcggcattt tgccttcctg 2760

tttttgctca cccagaaacg ctggtgaaag taaaagatgc tgaagatcag ttgggtgcac 2820

gagtgggtta catcgaactg gatctcaaca gcggtaagat ccttgagagt tttcgccccg 2880

aagaacgttt tccaatgatg agcactttta aagttctgct atgtggcgcg gtattatccc 2940

gtattgacgc cgggcaagag caactcggtc gccgcataca ctattctcag aatgacttgg 3000

ttgagtactc accagtcaca gaaaagcatc ttacggatgg catgacagta agagaattat 3060

gcagtgctgc cataaccatg agtgataaca ctgcggccaa cttacttctg acaacgatcg 3120

gaggaccgaa ggagctaacc gcttttttgc acaacatggg ggatcatgta actcgccttg 3180

atcgttggga accggagctg aatgaagcca taccaaacga cgagcgtgac accacgatgc 3240

ctgtagcaat ggcaacaacg ttgcgcaaac tattaactgg cgaactactt actctagctt 3300

cccggcaaca attaatagac tggatggagg cggataaagt tgcaggacca cttctgcgct 3360

cggcccttcc ggctggctgg tttattgctg ataaatctgg agccggtgag cgtgggtctc 3420

gcggtatcat tgcagcactg gggccagatg gtaagccctc ccgtatcgta gttatctaca 3480

cgacggggag tcaggcaact atggatgaac gaaatagaca gatcgctgag ataggtgcct 3540

cactgattaa gcattggtaa ctgtcagacc aagtttactc atatatactt tagattgatt 3600

taaaacttca tttttaattt aaaaggatct aggtgaagat cctttttgat aatctcatga 3660

ccaaaatccc ttaacgtgag ttttcgttcc actgagcgtc agaccccgta gaaaagatca 3720

aaggatcttc ttgagatcct ttttttctgc gcgtaatctg ctgcttgcaa acaaaaaaac 3780

caccgctacc agcggtggtt tgtttgccgg atcaagagct accaactctt tttccgaagg 3840

taactggctt cagcagagcg cagataccaa atactgttct tctagtgtag ccgtagttag 3900

gccaccactt caagaactct gtagcaccgc ctacatacct cgctctgcta atcctgttac 3960

cagtggctgc tgccagtggc gataagtcgt gtcttaccgg gttggactca agacgatagt 4020

taccggataa ggcgcagcgg tcgggctgaa cggggggttc gtgcacacag cccagcttgg 4080

agcgaacgac ctacaccgaa ctgagatacc tacagcgtga gctatgagaa agcgccacgc 4140

ttcccgaagg gagaaaggcg gacaggtatc cggtaagcgg cagggtcgga acaggagagc 4200

gcacgaggga gcttccaggg ggaaacgcct ggtatcttta tagtcctgtc gggtttcgcc 4260

acctctgact tgagcgtcga tttttgtgat gctcgtcagg ggggcggagc ctatggaaaa 4320

acgccagcaa cgcggccttt ttacggttcc tggccttttg ctggcctttt gctcacatgt 4380

tctttcctgc gttatcccct gattctgtgg ataaccgtat taccgccttt gagtgagctg 4440

ataccgctcg ccgcagccga acgaccgagc gcagcgagtc agtgagcgag gaagcggaag 4500

agcgcccaat acgcaaaccg cctctccccg cgcgttggcc gattcattaa tgcagctggc 4560

acgacaggtt tcccgactgg aaagcgggca gtgagcgcaa cgcaattaat gtgagttagc 4620

tcactcatta ggcaccccag gctttacact ttatgcttcc ggctcgtatg ttgtgtggaa 4680

ttgtgagcgg ataacaattt cacacaggaa acagctatga ccatgattac gccagattta 4740

attaag 4746

<210> 60
<211> 4746
<212> DNA
<213> Artificial Sequence

<220>
<223> Description of Artificial Sequence: Synthetic
      polynucleotide

<400> 60
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagt ccacggggtt ggggttgcgc cttttccaag 240

gcagccctgg gtttgcgcag ggacgcggct gctctgggcg tggttccggg aaacgcagcg 300

gcgccgaccc tgggtctcgc acattcttca cgtccgttcg cagcgtcacc cggatcttcg 360

ccgctaccct tgtgggcccc ccggcgacgc ttcctcgtcc gcccctaagt cgggaaggtt 420

ccttgcggtt cgcggcgtgc cggacgtgac aaacggaagc cgcacgtctc actagtaccc 480

tcgcagacgg acagcgccag ggagcaatgg cagcgcgccg accgcgatgg gctgtggcca 540

atagcggctg ctcagcaggg cgcgccgaga gcagcggccg ggaaggggcg gtgcgggagg 600

cggggtgtgg ggcggtagtg tgggccctgt tcctgcccgc gcggtgttcc gcattctgca 660

agcctccgga gcgcacgtcg gcagtcggct ccctcgttga ccgaatcacc gacctctctc 720

cccacgggag caagcttgaa ctgaaaaacc agaaagttaa ctggtaagtt tagtcttttt 780

gtcttttatt tcaggtcccg gatccggtgg tggtgcaaat caaagaactg ctcctcagtg 840

gatgttgcct ttacttctag gcctgtacgg aagtgttact tctgctctaa aagctgcgga 900

attgtacccg cggccgatcc accggtatgt ggacactcgg gcgacgcgcc gtggctgggc 960

tgcttgcttc cccaagtccc gcacaggcgc aaaccctcac aagggtccca cggccagctg 1020

aattggcccc gctgtgcggt cggaggggcc tgcgaacaga tatagacgca acgtgcaccc 1080

ccagaagagc aagcagcaac cagagaggcc tcaatcagat ttggaacgtc aagaaacaga 1140

gtgtctatct gatgaacctc cgcaaatcag ggacccttgg tcatccggga tctcttgatg 1200

aaacgaccta tgaacggctg gctgaggaga cgcttgactc cctggcagag ttcttcgagg 1260

atctggctga taagccatac acgttcgagg actatgatgt ttcttttggt tctggcgtcc 1320

tgacggtgaa actcggaggg gatctgggca cttacgttat caacaaacag actcccaata 1380

aacagatttg gttgtctagt cctagcagtg gcccaaaacg ctatgactgg acgggcaaga 1440

actgggtgta ctcccacgat ggagttagcc tgcacgaact tctggccgct gagcttacta 1500

aagcgctgaa gactaagctg gatctctcca gcctcgcata cagcggcaaa gacgcctagc 1560

tcgaggacgg ggtgaactac gcctgaggat ccgatctttt tccctctgcc aaaaattatg 1620

gggacatcat gaagcccctt gagcatctga cttctggcta ataaaggaaa tttattttca 1680

ttgcaatagt gtgttggaat tttttgtgtc tctcactcgg aagcaattcg ttgatctgaa 1740

tttcgaccac ccataatacc cattaccctg gtagataagt agcatggcgg gttaatcatt 1800

aactacaagg aacccctagt gatggagttg gccactccct ctctgcgcgc tcgctcgctc 1860

actgaggccg ggcgaccaaa ggtcgcccga cgcccgggct ttgcccgggc ggcctcagtg 1920

agcgagcgag cgcgcagcct taattaacct aattcactgg ccgtcgtttt acaacgtcgt 1980

gactgggaaa accctggcgt tacccaactt aatcgccttg cagcacatcc ccctttcgcc 2040

agctggcgta atagcgaaga ggcccgcacc gatcgccctt cccaacagtt gcgcagcctg 2100

aatggcgaat gggacgcgcc ctgtagcggc gcattaagcg cggcgggtgt ggtggttacg 2160

cgcagcgtga ccgctacact tgccagcgcc ctagcgcccg ctcctttcgc tttcttccct 2220

tcctttctcg ccacgttcgc cggctttccc cgtcaagctc taaatcgggg gctcccttta 2280

gggttccgat ttagtgcttt acggcacctc gaccccaaaa aacttgatta gggtgatggt 2340

tcacgtagtg ggccatcgcc ctgatagacg gtttttcgcc ctttgacgtt ggagtccacg 2400

ttctttaata gtggactctt gttccaaact ggaacaacac tcaaccctat ctcggtctat 2460

tcttttgatt tataagggat tttgccgatt tcggcctatt ggttaaaaaa tgagctgatt 2520

taacaaaaat ttaacgcgaa ttttaacaaa atattaacgc ttacaattta ggtggcactt 2580

ttcggggaaa tgtgcgcgga acccctattt gtttattttt ctaaatacat tcaaatatgt 2640

atccgctcat gagacaataa ccctgataaa tgcttcaata atattgaaaa aggaagagta 2700

tgagtattca acatttccgt gtcgccctta ttcccttttt tgcggcattt tgccttcctg 2760

tttttgctca cccagaaacg ctggtgaaag taaaagatgc tgaagatcag ttgggtgcac 2820

gagtgggtta catcgaactg gatctcaaca gcggtaagat ccttgagagt tttcgccccg 2880

aagaacgttt tccaatgatg agcactttta aagttctgct atgtggcgcg gtattatccc 2940

gtattgacgc cgggcaagag caactcggtc gccgcataca ctattctcag aatgacttgg 3000

ttgagtactc accagtcaca gaaaagcatc ttacggatgg catgacagta agagaattat 3060

gcagtgctgc cataaccatg agtgataaca ctgcggccaa cttacttctg acaacgatcg 3120

gaggaccgaa ggagctaacc gcttttttgc acaacatggg ggatcatgta actcgccttg 3180

atcgttggga accggagctg aatgaagcca taccaaacga cgagcgtgac accacgatgc 3240

ctgtagcaat ggcaacaacg ttgcgcaaac tattaactgg cgaactactt actctagctt 3300

cccggcaaca attaatagac tggatggagg cggataaagt tgcaggacca cttctgcgct 3360

cggcccttcc ggctggctgg tttattgctg ataaatctgg agccggtgag cgtgggtctc 3420

gcggtatcat tgcagcactg gggccagatg gtaagccctc ccgtatcgta gttatctaca 3480

cgacggggag tcaggcaact atggatgaac gaaatagaca gatcgctgag ataggtgcct 3540

cactgattaa gcattggtaa ctgtcagacc aagtttactc atatatactt tagattgatt 3600

taaaacttca tttttaattt aaaaggatct aggtgaagat cctttttgat aatctcatga 3660

ccaaaatccc ttaacgtgag ttttcgttcc actgagcgtc agaccccgta gaaaagatca 3720

aaggatcttc ttgagatcct ttttttctgc gcgtaatctg ctgcttgcaa acaaaaaaac 3780

caccgctacc agcggtggtt tgtttgccgg atcaagagct accaactctt tttccgaagg 3840

taactggctt cagcagagcg cagataccaa atactgttct tctagtgtag ccgtagttag 3900

gccaccactt caagaactct gtagcaccgc ctacatacct cgctctgcta atcctgttac 3960

cagtggctgc tgccagtggc gataagtcgt gtcttaccgg gttggactca agacgatagt 4020

taccggataa ggcgcagcgg tcgggctgaa cggggggttc gtgcacacag cccagcttgg 4080

agcgaacgac ctacaccgaa ctgagatacc tacagcgtga gctatgagaa agcgccacgc 4140

ttcccgaagg gagaaaggcg gacaggtatc cggtaagcgg cagggtcgga acaggagagc 4200

gcacgaggga gcttccaggg ggaaacgcct ggtatcttta tagtcctgtc gggtttcgcc 4260

acctctgact tgagcgtcga tttttgtgat gctcgtcagg ggggcggagc ctatggaaaa 4320

acgccagcaa cgcggccttt ttacggttcc tggccttttg ctggcctttt gctcacatgt 4380

tctttcctgc gttatcccct gattctgtgg ataaccgtat taccgccttt gagtgagctg 4440

ataccgctcg ccgcagccga acgaccgagc gcagcgagtc agtgagcgag gaagcggaag 4500

agcgcccaat acgcaaaccg cctctccccg cgcgttggcc gattcattaa tgcagctggc 4560

acgacaggtt tcccgactgg aaagcgggca gtgagcgcaa cgcaattaat gtgagttagc 4620

tcactcatta ggcaccccag gctttacact ttatgcttcc ggctcgtatg ttgtgtggaa 4680

ttgtgagcgg ataacaattt cacacaggaa acagctatga ccatgattac gccagattta 4740

attaag 4746

<210> 61
<211> 4746
<212> DNA
<213> Artificial Sequence

<220>
<223> Description of Artificial Sequence: Synthetic
      polynucleotide

<400> 61
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagt ccacggggtt ggggttgcgc cttttccaag 240

gcagccctgg gtttgcgcag ggacgcggct gctctgggcg tggttccggg aaacgcagcg 300

gcgccgaccc tgggtctcgc acattcttca cgtccgttcg cagcgtcacc cggatcttcg 360

ccgctaccct tgtgggcccc ccggcgacgc ttcctcgtcc gcccctaagt cgggaaggtt 420

ccttgcggtt cgcggcgtgc cggacgtgac aaacggaagc cgcacgtctc actagtaccc 480

tcgcagacgg acagcgccag ggagcaatgg cagcgcgccg accgcgatgg gctgtggcca 540

atagcggctg ctcagcaggg cgcgccgaga gcagcggccg ggaaggggcg gtgcgggagg 600

cggggtgtgg ggcggtagtg tgggccctgt tcctgcccgc gcggtgttcc gcattctgca 660

agcctccgga gcgcacgtcg gcagtcggct ccctcgttga ccgaatcacc gacctctctc 720

cccacgggag caagcttgaa ctgaaaaacc agaaagttaa ctggtaagtt tagtcttttt 780

gtcttttatt tcaggtcccg gatccggtgg tggtgcaaat caaagaactg ctcctcagtg 840

gatgttgcct ttacttctag gcctgtacgg aagtgttact tctgctctaa aagctgcgga 900

attgtacccg cggccgatcc accggtatgt ggactctggg tcgacgcgcc gtagccggtc 960

tcttggcctc cccatcccct gcgcaggcac agacccttac acgagttcca cggcccgctg 1020

aactggcacc actctgcgga cgccggggcc tgagaaccga tattgacgct acgtgcactc 1080

cccgacgagc ctcttctaat cagagggggc tcaaccagat ctggaacgtg aagaagcagt 1140

cagtctactt gatgaatctg agaaagagcg ggacccttgg gcaccccggg tctctcgacg 1200

agactacata cgagaggctg gccgaggaga ctttggactc tctcgccgaa ttcttcgagg 1260

atttggcaga caagccgtac acctttgaag actacgatgt ctcattcgga tcaggggtgc 1320

tgacggttaa actggggggc gatctcggaa cctacgtcat aaacaaacag acacccaata 1380

agcagatctg gttgtctagc ccatcatccg gtcctaaacg gtacgactgg accggcaaga 1440

actgggtcta ctcccatgac ggagtcagtc ttcatgaact gcttgccgca gagctgacca 1500

aggctcttaa gacgaaactg gatctctcta gcctggccta ctccgggaag gatgcatagc 1560

tcgaggacgg ggtgaactac gcctgaggat ccgatctttt tccctctgcc aaaaattatg 1620

gggacatcat gaagcccctt gagcatctga cttctggcta ataaaggaaa tttattttca 1680

ttgcaatagt gtgttggaat tttttgtgtc tctcactcgg aagcaattcg ttgatctgaa 1740

tttcgaccac ccataatacc cattaccctg gtagataagt agcatggcgg gttaatcatt 1800

aactacaagg aacccctagt gatggagttg gccactccct ctctgcgcgc tcgctcgctc 1860

actgaggccg ggcgaccaaa ggtcgcccga cgcccgggct ttgcccgggc ggcctcagtg 1920

agcgagcgag cgcgcagcct taattaacct aattcactgg ccgtcgtttt acaacgtcgt 1980

gactgggaaa accctggcgt tacccaactt aatcgccttg cagcacatcc ccctttcgcc 2040

agctggcgta atagcgaaga ggcccgcacc gatcgccctt cccaacagtt gcgcagcctg 2100

aatggcgaat gggacgcgcc ctgtagcggc gcattaagcg cggcgggtgt ggtggttacg 2160

cgcagcgtga ccgctacact tgccagcgcc ctagcgcccg ctcctttcgc tttcttccct 2220

tcctttctcg ccacgttcgc cggctttccc cgtcaagctc taaatcgggg gctcccttta 2280

gggttccgat ttagtgcttt acggcacctc gaccccaaaa aacttgatta gggtgatggt 2340

tcacgtagtg ggccatcgcc ctgatagacg gtttttcgcc ctttgacgtt ggagtccacg 2400

ttctttaata gtggactctt gttccaaact ggaacaacac tcaaccctat ctcggtctat 2460

tcttttgatt tataagggat tttgccgatt tcggcctatt ggttaaaaaa tgagctgatt 2520

taacaaaaat ttaacgcgaa ttttaacaaa atattaacgc ttacaattta ggtggcactt 2580

ttcggggaaa tgtgcgcgga acccctattt gtttattttt ctaaatacat tcaaatatgt 2640

atccgctcat gagacaataa ccctgataaa tgcttcaata atattgaaaa aggaagagta 2700

tgagtattca acatttccgt gtcgccctta ttcccttttt tgcggcattt tgccttcctg 2760

tttttgctca cccagaaacg ctggtgaaag taaaagatgc tgaagatcag ttgggtgcac 2820

gagtgggtta catcgaactg gatctcaaca gcggtaagat ccttgagagt tttcgccccg 2880

aagaacgttt tccaatgatg agcactttta aagttctgct atgtggcgcg gtattatccc 2940

gtattgacgc cgggcaagag caactcggtc gccgcataca ctattctcag aatgacttgg 3000

ttgagtactc accagtcaca gaaaagcatc ttacggatgg catgacagta agagaattat 3060

gcagtgctgc cataaccatg agtgataaca ctgcggccaa cttacttctg acaacgatcg 3120

gaggaccgaa ggagctaacc gcttttttgc acaacatggg ggatcatgta actcgccttg 3180

atcgttggga accggagctg aatgaagcca taccaaacga cgagcgtgac accacgatgc 3240

ctgtagcaat ggcaacaacg ttgcgcaaac tattaactgg cgaactactt actctagctt 3300

cccggcaaca attaatagac tggatggagg cggataaagt tgcaggacca cttctgcgct 3360

cggcccttcc ggctggctgg tttattgctg ataaatctgg agccggtgag cgtgggtctc 3420

gcggtatcat tgcagcactg gggccagatg gtaagccctc ccgtatcgta gttatctaca 3480

cgacggggag tcaggcaact atggatgaac gaaatagaca gatcgctgag ataggtgcct 3540

cactgattaa gcattggtaa ctgtcagacc aagtttactc atatatactt tagattgatt 3600

taaaacttca tttttaattt aaaaggatct aggtgaagat cctttttgat aatctcatga 3660

ccaaaatccc ttaacgtgag ttttcgttcc actgagcgtc agaccccgta gaaaagatca 3720

aaggatcttc ttgagatcct ttttttctgc gcgtaatctg ctgcttgcaa acaaaaaaac 3780

caccgctacc agcggtggtt tgtttgccgg atcaagagct accaactctt tttccgaagg 3840

taactggctt cagcagagcg cagataccaa atactgttct tctagtgtag ccgtagttag 3900

gccaccactt caagaactct gtagcaccgc ctacatacct cgctctgcta atcctgttac 3960

cagtggctgc tgccagtggc gataagtcgt gtcttaccgg gttggactca agacgatagt 4020

taccggataa ggcgcagcgg tcgggctgaa cggggggttc gtgcacacag cccagcttgg 4080

agcgaacgac ctacaccgaa ctgagatacc tacagcgtga gctatgagaa agcgccacgc 4140

ttcccgaagg gagaaaggcg gacaggtatc cggtaagcgg cagggtcgga acaggagagc 4200

gcacgaggga gcttccaggg ggaaacgcct ggtatcttta tagtcctgtc gggtttcgcc 4260

acctctgact tgagcgtcga tttttgtgat gctcgtcagg ggggcggagc ctatggaaaa 4320

acgccagcaa cgcggccttt ttacggttcc tggccttttg ctggcctttt gctcacatgt 4380

tctttcctgc gttatcccct gattctgtgg ataaccgtat taccgccttt gagtgagctg 4440

ataccgctcg ccgcagccga acgaccgagc gcagcgagtc agtgagcgag gaagcggaag 4500

agcgcccaat acgcaaaccg cctctccccg cgcgttggcc gattcattaa tgcagctggc 4560

acgacaggtt tcccgactgg aaagcgggca gtgagcgcaa cgcaattaat gtgagttagc 4620

tcactcatta ggcaccccag gctttacact ttatgcttcc ggctcgtatg ttgtgtggaa 4680

ttgtgagcgg ataacaattt cacacaggaa acagctatga ccatgattac gccagattta 4740

attaag 4746

<210> 62
<211> 4550
<212> DNA
<213> Artificial Sequence

<220>
<223> Description of Artificial Sequence: Synthetic
      polynucleotide

<400> 62
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacgg tgtggaaagt ccccaggctc 240

cccagcaggc agaagtatgc aaagcatgca tctcaattag tcagcaacca ggtgtggaaa 300

gtccccaggc tccccagcag gcagaagtat gcaaagcatg catctcaatt agtcagcaac 360

catagtcccg cccctaactc cgcccatccc gcccctaact ccgcccagtt ccgcccattc 420

tccgccccat ggctgactaa ttttttttat ttatgcagag gccgaggccg cctcggcctc 480

tgagctattc cagaagtagt gaggaggctt ttttggaggc ctaggctttt gcaaaaagct 540

tgaactgaaa aaccagaaag ttaactggta agtttagtct ttttgtcttt tatttcaggt 600

cccggatccg gtggtggtgc aaatcaaaga actgctcctc agtggatgtt gcctttactt 660

ctaggcctgt acggaagtgt tacttctgct ctaaaagctg cggaattgta cccgcggccg 720

atccaccggt atgtggactc tcgggcgccg cgcagtagcc ggcctcctgg cgtcacccag 780

cccagcccag gcccagaccc tcacccgggt cccgcggccg gcagagttgg ccccactctg 840

cggccgccgt ggcctgcgca ccgacatcga tgcgacctgc acgccccgcc gcgcaagttc 900

gaaccaacgt ggcctcaacc agatttggaa tgtcaaaaag cagagtgtct atttgatgaa 960

tttgaggaaa tctggaactt tgggccaccc aggctctcta gatgagacca cctatgaaag 1020

actagcagag gaaacgctgg actctttagc agagtttttt gaagaccttg cagacaagcc 1080

atacacgttt gaggactatg atgtctcctt tgggagtggt gtcttaactg tcaaactggg 1140

tggagatcta ggaacctatg tgatcaacaa gcagacgcca aacaagcaaa tctggctatc 1200

ttctccatcc agtggaccta agcgttatga ctggactggg aaaaactggg tgtactccca 1260

cgacggcgtg tccctccatg agctgctggc cgcagagctc actaaagcct taaaaaccaa 1320

actggacttg tcttccttgg cctattccgg aaaagatgct tgactcgagg acggggtgaa 1380

ctacgcctga ggatccgatc tttttccctc tgccaaaaat tatggggaca tcatgaagcc 1440

ccttgagcat ctgacttctg gctaataaag gaaatttatt ttcattgcaa tagtgtgttg 1500

gaattttttg tgtctctcac tcggaagcaa ttcgttgatc tgaatttcga ccacccataa 1560

tacccattac cctggtagat aagtagcatg gcgggttaat cattaactac aaggaacccc 1620

tagtgatgga gttggccact ccctctctgc gcgctcgctc gctcactgag gccgggcgac 1680

caaaggtcgc ccgacgcccg ggctttgccc gggcggcctc agtgagcgag cgagcgcgca 1740

gccttaatta acctaattca ctggccgtcg ttttacaacg tcgtgactgg gaaaaccctg 1800

gcgttaccca acttaatcgc cttgcagcac atcccccttt cgccagctgg cgtaatagcg 1860

aagaggcccg caccgatcgc ccttcccaac agttgcgcag cctgaatggc gaatgggacg 1920

cgccctgtag cggcgcatta agcgcggcgg gtgtggtggt tacgcgcagc gtgaccgcta 1980

cacttgccag cgccctagcg cccgctcctt tcgctttctt cccttccttt ctcgccacgt 2040

tcgccggctt tccccgtcaa gctctaaatc gggggctccc tttagggttc cgatttagtg 2100

ctttacggca cctcgacccc aaaaaacttg attagggtga tggttcacgt agtgggccat 2160

cgccctgata gacggttttt cgccctttga cgttggagtc cacgttcttt aatagtggac 2220

tcttgttcca aactggaaca acactcaacc ctatctcggt ctattctttt gatttataag 2280

ggattttgcc gatttcggcc tattggttaa aaaatgagct gatttaacaa aaatttaacg 2340

cgaattttaa caaaatatta acgcttacaa tttaggtggc acttttcggg gaaatgtgcg 2400

cggaacccct atttgtttat ttttctaaat acattcaaat atgtatccgc tcatgagaca 2460

ataaccctga taaatgcttc aataatattg aaaaaggaag agtatgagta ttcaacattt 2520

ccgtgtcgcc cttattccct tttttgcggc attttgcctt cctgtttttg ctcacccaga 2580

aacgctggtg aaagtaaaag atgctgaaga tcagttgggt gcacgagtgg gttacatcga 2640

actggatctc aacagcggta agatccttga gagttttcgc cccgaagaac gttttccaat 2700

gatgagcact tttaaagttc tgctatgtgg cgcggtatta tcccgtattg acgccgggca 2760

agagcaactc ggtcgccgca tacactattc tcagaatgac ttggttgagt actcaccagt 2820

cacagaaaag catcttacgg atggcatgac agtaagagaa ttatgcagtg ctgccataac 2880

catgagtgat aacactgcgg ccaacttact tctgacaacg atcggaggac cgaaggagct 2940

aaccgctttt ttgcacaaca tgggggatca tgtaactcgc cttgatcgtt gggaaccgga 3000

gctgaatgaa gccataccaa acgacgagcg tgacaccacg atgcctgtag caatggcaac 3060

aacgttgcgc aaactattaa ctggcgaact acttactcta gcttcccggc aacaattaat 3120

agactggatg gaggcggata aagttgcagg accacttctg cgctcggccc ttccggctgg 3180

ctggtttatt gctgataaat ctggagccgg tgagcgtggg tctcgcggta tcattgcagc 3240

actggggcca gatggtaagc cctcccgtat cgtagttatc tacacgacgg ggagtcaggc 3300

aactatggat gaacgaaata gacagatcgc tgagataggt gcctcactga ttaagcattg 3360

gtaactgtca gaccaagttt actcatatat actttagatt gatttaaaac ttcattttta 3420

atttaaaagg atctaggtga agatcctttt tgataatctc atgaccaaaa tcccttaacg 3480

tgagttttcg ttccactgag cgtcagaccc cgtagaaaag atcaaaggat cttcttgaga 3540

tccttttttt ctgcgcgtaa tctgctgctt gcaaacaaaa aaaccaccgc taccagcggt 3600

ggtttgtttg ccggatcaag agctaccaac tctttttccg aaggtaactg gcttcagcag 3660

agcgcagata ccaaatactg ttcttctagt gtagccgtag ttaggccacc acttcaagaa 3720

ctctgtagca ccgcctacat acctcgctct gctaatcctg ttaccagtgg ctgctgccag 3780

tggcgataag tcgtgtctta ccgggttgga ctcaagacga tagttaccgg ataaggcgca 3840

gcggtcgggc tgaacggggg gttcgtgcac acagcccagc ttggagcgaa cgacctacac 3900

cgaactgaga tacctacagc gtgagctatg agaaagcgcc acgcttcccg aagggagaaa 3960

ggcggacagg tatccggtaa gcggcagggt cggaacagga gagcgcacga gggagcttcc 4020

agggggaaac gcctggtatc tttatagtcc tgtcgggttt cgccacctct gacttgagcg 4080

tcgatttttg tgatgctcgt caggggggcg gagcctatgg aaaaacgcca gcaacgcggc 4140

ctttttacgg ttcctggcct tttgctggcc ttttgctcac atgttctttc ctgcgttatc 4200

ccctgattct gtggataacc gtattaccgc ctttgagtga gctgataccg ctcgccgcag 4260

ccgaacgacc gagcgcagcg agtcagtgag cgaggaagcg gaagagcgcc caatacgcaa 4320

accgcctctc cccgcgcgtt ggccgattca ttaatgcagc tggcacgaca ggtttcccga 4380

ctggaaagcg ggcagtgagc gcaacgcaat taatgtgagt tagctcactc attaggcacc 4440

ccaggcttta cactttatgc ttccggctcg tatgttgtgt ggaattgtga gcggataaca 4500

atttcacaca ggaaacagct atgaccatga ttacgccaga tttaattaag 4550

<210> 63
<211> 4824
<212> DNA
<213> Artificial Sequence

<220>
<223> Description of Artificial Sequence: Synthetic
      polynucleotide

<400> 63
ctgcgcgctc gctcgctcac tgaggccgcc cgggcaaagc ccgggcgtcg ggcgaccttt 60

ggtcgcccgg cctcagtgag cgagcgagcg cgcagagagg gagtgtagcc atgctctagg 120

aagatcaatt caattcacgc gtcgacattg attattgact agttattaat agtaatcaat 180

tacggggtca ttagttcata gcccatatat ggagttccgc gttacataac ttacggtaaa 240

tggcccgcct ggctgaccgc ccaacgaccc ccgcccattg acgtcaataa tgacgtatgt 300

tcccatagta acgccaatag ggactttcca ttgacgtcaa tgggtggagt atttacggta 360

aactgcccac ttggcagtac atcaagtgta tcatatgcca agtacgcccc ctattgacgt 420

caatgacggt aaatggcccg cctggcatta tgcccagtac atgaccttat gggactttcc 480

tacttggcag tacatctacg tattagtcat cgctattacc atgtcgaggc cacgttctgc 540

ttcactctcc ccatctcccc cccctcccca cccccaattt tgtatttatt tattttttaa 600

ttattttgtg cagcgatggg ggcggggggg gggggcgcgc gccaggcggg gcggggcggg 660

gcgaggggcg gggcggggcg aggcggagag gtgcggcggc agccaatcag agcggcgcgc 720

tccgaaagtt tccttttatg gcgaggcggc ggcggcggcg gccctataaa aagcgaagcg 780

cgcggcgggc gggagcaagc ttgaactgaa aaaccagaaa gttaactggt aagtttagtc 840

tttttgtctt ttatttcagg tcccggatcc ggtggtggtg caaatcaaag aactgctcct 900

cagtggatgt tgcctttact tctaggcctg tacggaagtg ttacttctgc tctaaaagct 960

gcggaattgt acccgcggcc gatccaccgg tatgtggact ctcgggcgcc gcgcagtagc 1020

cggcctcctg gcgtcaccca gcccagccca ggcccagacc ctcacccggg tcccgcggcc 1080

ggcagagttg gccccactct gcggccgccg tggcctgcgc accgacatcg atgcgacctg 1140

cacgccccgc cgcgcaagtt cgaaccaacg tggcctcaac cagatttgga atgtcaaaaa 1200

gcagagtgtc tatttgatga atttgaggaa atctggaact ttgggccacc caggctctct 1260

agatgagacc acctatgaaa gactagcaga ggaaacgctg gactctttag cagagttttt 1320

tgaagacctt gcagacaagc catacacgtt tgaggactat gatgtctcct ttgggagtgg 1380

tgtcttaact gtcaaactgg gtggagatct aggaacctat gtgatcaaca agcagacgcc 1440

aaacaagcaa atctggctat cttctccatc cagtggacct aagcgttatg actggactgg 1500

gaaaaactgg gtgtactccc acgacggcgt gtccctccat gagctgctgg ccgcagagct 1560

cactaaagcc ttaaaaacca aactggactt gtcttccttg gcctattccg gaaaagatgc 1620

ttgagcggcc ctagcgttta aacgggccct ctagagtcga cccgggcggc ctcgaggacg 1680

gggtgaacta cgcctgagga tccgatcttt ttccctctgc caaaaattat ggggacatca 1740

tgaagcccct tgagcatctg acttctggct aataaaggaa atttattttc attgcaatag 1800

tgtgttggaa ttttttgtgt ctctcactcg gcctaggtag ataagtagca tggcgggtta 1860

atcattaact acaaggaacc cctagtgatg gagttggcca ctccctctct gcgcgctcgc 1920

tcgctcactg aggccgggcg accaaaggtc gcccgacgcc cgggctttgc ccgggcggcc 1980

tcagtgagcg agcgagcgcg cagccttaat taacctaatt cactggccgt cgttttacaa 2040

cgtcgtgact gggaaaaccc tggcgttacc caacttaatc gccttgcagc acatccccct 2100

ttcgccagct ggcgtaatag cgaagaggcc cgcaccgatc gcccttccca acagttgcgc 2160

agcctgaatg gcgaatggga cgcgccctgt agcggcgcat taagcgcggc gggtgtggtg 2220

gttacgcgca gcgtgaccgc tacacttgcc agcgccctag cgcccgctcc tttcgctttc 2280

ttcccttcct ttctcgccac gttcgccggc tttccccgtc aagctctaaa tcgggggctc 2340

cctttagggt tccgatttag tgctttacgg cacctcgacc ccaaaaaact tgattagggt 2400

gatggttcac gtagtgggcc atcgccctga tagacggttt ttcgcccttt gacgttggag 2460

tccacgttct ttaatagtgg actcttgttc caaactggaa caacactcaa ccctatctcg 2520

gtctattctt ttgatttata agggattttg ccgatttcgg cctattggtt aaaaaatgag 2580

ctgatttaac aaaaatttaa cgcgaatttt aacaaaatat taacgcttac aatttaggtg 2640

gcacttttcg gggaaatgtg cgcggaaccc ctatttgttt atttttctaa atacattcaa 2700

atatgtatcc gctcatgaga caataaccct gataaatgct tcaataatat tgaaaaagga 2760

agagtatgag tattcaacat ttccgtgtcg cccttattcc cttttttgcg gcattttgcc 2820

ttcctgtttt tgctcaccca gaaacgctgg tgaaagtaaa agatgctgaa gatcagttgg 2880

gtgcacgagt gggttacatc gaactggatc tcaacagcgg taagatcctt gagagttttc 2940

gccccgaaga acgttttcca atgatgagca cttttaaagt tctgctatgt ggcgcggtat 3000

tatcccgtat tgacgccggg caagagcaac tcggtcgccg catacactat tctcagaatg 3060

acttggttga gtactcacca gtcacagaaa agcatcttac ggatggcatg acagtaagag 3120

aattatgcag tgctgccata accatgagtg ataacactgc ggccaactta cttctgacaa 3180

cgatcggagg accgaaggag ctaaccgctt ttttgcacaa catgggggat catgtaactc 3240

gccttgatcg ttgggaaccg gagctgaatg aagccatacc aaacgacgag cgtgacacca 3300

cgatgcctgt agcaatggca acaacgttgc gcaaactatt aactggcgaa ctacttactc 3360

tagcttcccg gcaacaatta atagactgga tggaggcgga taaagttgca ggaccacttc 3420

tgcgctcggc ccttccggct ggctggttta ttgctgataa atctggagcc ggtgagcgtg 3480

ggtctcgcgg tatcattgca gcactggggc cagatggtaa gccctcccgt atcgtagtta 3540

tctacacgac ggggagtcag gcaactatgg atgaacgaaa tagacagatc gctgagatag 3600

gtgcctcact gattaagcat tggtaactgt cagaccaagt ttactcatat atactttaga 3660

ttgatttaaa acttcatttt taatttaaaa ggatctaggt gaagatcctt tttgataatc 3720

tcatgaccaa aatcccttaa cgtgagtttt cgttccactg agcgtcagac cccgtagaaa 3780

agatcaaagg atcttcttga gatccttttt ttctgcgcgt aatctgctgc ttgcaaacaa 3840

aaaaaccacc gctaccagcg gtggtttgtt tgccggatca agagctacca actctttttc 3900

cgaaggtaac tggcttcagc agagcgcaga taccaaatac tgttcttcta gtgtagccgt 3960

agttaggcca ccacttcaag aactctgtag caccgcctac atacctcgct ctgctaatcc 4020

tgttaccagt ggctgctgcc agtggcgata agtcgtgtct taccgggttg gactcaagac 4080

gatagttacc ggataaggcg cagcggtcgg gctgaacggg gggttcgtgc acacagccca 4140

gcttggagcg aacgacctac accgaactga gatacctaca gcgtgagcta tgagaaagcg 4200

ccacgcttcc cgaagggaga aaggcggaca ggtatccggt aagcggcagg gtcggaacag 4260

gagagcgcac gagggagctt ccagggggaa acgcctggta tctttatagt cctgtcgggt 4320

ttcgccacct ctgacttgag cgtcgatttt tgtgatgctc gtcagggggg cggagcctat 4380

ggaaaaacgc cagcaacgcg gcctttttac ggttcctggc cttttgctgg ccttttgctc 4440

acatgttctt tcctgcgtta tcccctgatt ctgtggataa ccgtattacc gcctttgagt 4500

gagctgatac cgctcgccgc agccgaacga ccgagcgcag cgagtcagtg agcgaggaag 4560

cggaagagcg cccaatacgc aaaccgcctc tccccgcgcg ttggccgatt cattaatgca 4620

gctggcacga caggtttccc gactggaaag cgggcagtga gcgcaacgca attaatgtga 4680

gttagctcac tcattaggca ccccaggctt tacactttat gcttccggct cgtatgttgt 4740

gtggaattgt gagcggataa caatttcaca caggaaacag ctatgaccat gattacgcca 4800

gatttaatta aggccttaat tagg 4824

<210> 64
<211> 4805
<212> DNA
<213> Artificial Sequence

<220>
<223> Description of Artificial Sequence: Synthetic
      polynucleotide

<400> 64
ctgcgcgctc gctcgctcac tgaggccgcc cgggcaaagc ccgggcgtcg ggcgaccttt 60

ggtcgcccgg cctcagtgag cgagcgagcg cgcagagagg gagtgtagcc atgctctagg 120

aagatcaatt caattcacgc gtcgacattg attattgact agttattaat agtaatcaat 180

tacggggtca ttagttcata gcccatatat ggagttccgc gttacataac ttacggtaaa 240

tggcccgcct ggctgaccgc ccaacgaccc ccgcccattg acgtcaataa tgacgtatgt 300

tcccatagta acgccaatag ggactttcca ttgacgtcaa tgggtggagt atttacggta 360

aactgcccac ttggcagtac atcaagtgta tcatatgcca agtacgcccc ctattgacgt 420

caatgacggt aaatggcccg cctggcatta tgcccagtac atgaccttat gggactttcc 480

tacttggcag tacatctacg tattagtcat cgctattacc atgtcgaggc cacgttctgc 540

ttcactctcc ccatctcccc cccctcccca cccccaattt tgtatttatt tattttttaa 600

ttattttgtg cagcgatggg ggcggggggg gggggcgcgc gccaggcggg gcggggcggg 660

gcgaggggcg gggcggggcg aggcggagag gtgcggcggc agccaatcag agcggcgcgc 720

tccgaaagtt tccttttatg gcgaggcggc ggcggcggcg gccctataaa aagcgaagcg 780

cgcggcgggc gggagcaagc ttgaactgaa aaaccagaaa gttaactggt aagtttagtc 840

tttttgtctt ttatttcagg tcccggatcc ggtggtggtg caaatcaaag aactgctcct 900

cagtggatgt tgcctttact tctaggcctg tacggaagtg ttacttctgc tctaaaagct 960

gcggaattgt acccgcggcc gatccaccgg tatgtggact ctcgggcgcc gcgcagtagc 1020

cggcctcctg gcgtcaccca gcccagccca ggcccagacc ctcacccggg tcccgcggcc 1080

ggcagagttg gccccactct gcggccgccg tggcctgcgc accgacatcg atgcgacctg 1140

cacgccccgc cgcgcaagtt cgaaccaacg tggcctcaac cagatttgga atgtcaaaaa 1200

gcagagtgtc tatttgatga atttgaggaa atctggaact ttgggccacc caggctctct 1260

agatgagacc acctatgaaa gactagcaga ggaaacgctg gactctttag cagagttttt 1320

tgaagacctt gcagacaagc catacacgtt tgaggactat gatgtctcct ttgggagtgg 1380

tgtcttaact gtcaaactgg gtggagatct aggaacctat gtgatcaaca agcagacgcc 1440

aaacaagcaa atctggctat cttctccatc cagtggacct aagcgttatg actggactgg 1500

gaaaaactgg gtgtactccc acgacggcgt gtccctccat gagctgctgg ccgcagagct 1560

cactaaagcc ttaaaaacca aactggactt gtcttccttg gcctattccg gaaaagatgc 1620

ttatccttat gacgtgcctg actatgcctg actcgaggac ggggtgaact acgcctgagg 1680

atccgatctt tttccctctg ccaaaaatta tggggacatc atgaagcccc ttgagcatct 1740

gacttctggc taataaagga aatttatttt cattgcaata gtgtgttgga attttttgtg 1800

tctctcactc ggcctaggta gataagtagc atggcgggtt aatcattaac tacaaggaac 1860

ccctagtgat ggagttggcc actccctctc tgcgcgctcg ctcgctcact gaggccgggc 1920

gaccaaaggt cgcccgacgc ccgggctttg cccgggcggc ctcagtgagc gagcgagcgc 1980

gcagccttaa ttaacctaat tcactggccg tcgttttaca acgtcgtgac tgggaaaacc 2040

ctggcgttac ccaacttaat cgccttgcag cacatccccc tttcgccagc tggcgtaata 2100

gcgaagaggc ccgcaccgat cgcccttccc aacagttgcg cagcctgaat ggcgaatggg 2160

acgcgccctg tagcggcgca ttaagcgcgg cgggtgtggt ggttacgcgc agcgtgaccg 2220

ctacacttgc cagcgcccta gcgcccgctc ctttcgcttt cttcccttcc tttctcgcca 2280

cgttcgccgg ctttccccgt caagctctaa atcgggggct ccctttaggg ttccgattta 2340

gtgctttacg gcacctcgac cccaaaaaac ttgattaggg tgatggttca cgtagtgggc 2400

catcgccctg atagacggtt tttcgccctt tgacgttgga gtccacgttc tttaatagtg 2460

gactcttgtt ccaaactgga acaacactca accctatctc ggtctattct tttgatttat 2520

aagggatttt gccgatttcg gcctattggt taaaaaatga gctgatttaa caaaaattta 2580

acgcgaattt taacaaaata ttaacgctta caatttaggt ggcacttttc ggggaaatgt 2640

gcgcggaacc cctatttgtt tatttttcta aatacattca aatatgtatc cgctcatgag 2700

acaataaccc tgataaatgc ttcaataata ttgaaaaagg aagagtatga gtattcaaca 2760

tttccgtgtc gcccttattc ccttttttgc ggcattttgc cttcctgttt ttgctcaccc 2820

agaaacgctg gtgaaagtaa aagatgctga agatcagttg ggtgcacgag tgggttacat 2880

cgaactggat ctcaacagcg gtaagatcct tgagagtttt cgccccgaag aacgttttcc 2940

aatgatgagc acttttaaag ttctgctatg tggcgcggta ttatcccgta ttgacgccgg 3000

gcaagagcaa ctcggtcgcc gcatacacta ttctcagaat gacttggttg agtactcacc 3060

agtcacagaa aagcatctta cggatggcat gacagtaaga gaattatgca gtgctgccat 3120

aaccatgagt gataacactg cggccaactt acttctgaca acgatcggag gaccgaagga 3180

gctaaccgct tttttgcaca acatggggga tcatgtaact cgccttgatc gttgggaacc 3240

ggagctgaat gaagccatac caaacgacga gcgtgacacc acgatgcctg tagcaatggc 3300

aacaacgttg cgcaaactat taactggcga actacttact ctagcttccc ggcaacaatt 3360

aatagactgg atggaggcgg ataaagttgc aggaccactt ctgcgctcgg cccttccggc 3420

tggctggttt attgctgata aatctggagc cggtgagcgt gggtctcgcg gtatcattgc 3480

agcactgggg ccagatggta agccctcccg tatcgtagtt atctacacga cggggagtca 3540

ggcaactatg gatgaacgaa atagacagat cgctgagata ggtgcctcac tgattaagca 3600

ttggtaactg tcagaccaag tttactcata tatactttag attgatttaa aacttcattt 3660

ttaatttaaa aggatctagg tgaagatcct ttttgataat ctcatgacca aaatccctta 3720

acgtgagttt tcgttccact gagcgtcaga ccccgtagaa aagatcaaag gatcttcttg 3780

agatcctttt tttctgcgcg taatctgctg cttgcaaaca aaaaaaccac cgctaccagc 3840

ggtggtttgt ttgccggatc aagagctacc aactcttttt ccgaaggtaa ctggcttcag 3900

cagagcgcag ataccaaata ctgttcttct agtgtagccg tagttaggcc accacttcaa 3960

gaactctgta gcaccgccta catacctcgc tctgctaatc ctgttaccag tggctgctgc 4020

cagtggcgat aagtcgtgtc ttaccgggtt ggactcaaga cgatagttac cggataaggc 4080

gcagcggtcg ggctgaacgg ggggttcgtg cacacagccc agcttggagc gaacgaccta 4140

caccgaactg agatacctac agcgtgagct atgagaaagc gccacgcttc ccgaagggag 4200

aaaggcggac aggtatccgg taagcggcag ggtcggaaca ggagagcgca cgagggagct 4260

tccaggggga aacgcctggt atctttatag tcctgtcggg tttcgccacc tctgacttga 4320

gcgtcgattt ttgtgatgct cgtcaggggg gcggagccta tggaaaaacg ccagcaacgc 4380

ggccttttta cggttcctgg ccttttgctg gccttttgct cacatgttct ttcctgcgtt 4440

atcccctgat tctgtggata accgtattac cgcctttgag tgagctgata ccgctcgccg 4500

cagccgaacg accgagcgca gcgagtcagt gagcgaggaa gcggaagagc gcccaatacg 4560

caaaccgcct ctccccgcgc gttggccgat tcattaatgc agctggcacg acaggtttcc 4620

cgactggaaa gcgggcagtg agcgcaacgc aattaatgtg agttagctca ctcattaggc 4680

accccaggct ttacacttta tgcttccggc tcgtatgttg tgtggaattg tgagcggata 4740

acaatttcac acaggaaaca gctatgacca tgattacgcc agatttaatt aaggccttaa 4800

ttagg 4805

<210> 65
<211> 4850
<212> DNA
<213> Artificial Sequence

<220>
<223> Description of Artificial Sequence: Synthetic
      polynucleotide

<400> 65
ctgcgcgctc gctcgctcac tgaggccgcc cgggcaaagc ccgggcgtcg ggcgaccttt 60

ggtcgcccgg cctcagtgag cgagcgagcg cgcagagagg gagtgtagcc atgctctagg 120

aagatcaatt caattcacgc gtcgacattg attattgact agttattaat agtaatcaat 180

tacggggtca ttagttcata gcccatatat ggagttccgc gttacataac ttacggtaaa 240

tggcccgcct ggctgaccgc ccaacgaccc ccgcccattg acgtcaataa tgacgtatgt 300

tcccatagta acgccaatag ggactttcca ttgacgtcaa tgggtggagt atttacggta 360

aactgcccac ttggcagtac atcaagtgta tcatatgcca agtacgcccc ctattgacgt 420

caatgacggt aaatggcccg cctggcatta tgcccagtac atgaccttat gggactttcc 480

tacttggcag tacatctacg tattagtcat cgctattacc atgtcgaggc cacgttctgc 540

ttcactctcc ccatctcccc cccctcccca cccccaattt tgtatttatt tattttttaa 600

ttattttgtg cagcgatggg ggcggggggg gggggcgcgc gccaggcggg gcggggcggg 660

gcgaggggcg gggcggggcg aggcggagag gtgcggcggc agccaatcag agcggcgcgc 720

tccgaaagtt tccttttatg gcgaggcggc ggcggcggcg gccctataaa aagcgaagcg 780

cgcggcgggc gggagcaagc ttgaactgaa aaaccagaaa gttaactggt aagtttagtc 840

tttttgtctt ttatttcagg tcccggatcc ggtggtggtg caaatcaaag aactgctcct 900

cagtggatgt tgcctttact tctaggcctg tacggaagtg ttacttctgc tctaaaagct 960

gcggaattgt acccgcggcc gatccaccgg tatgtggact ctcgggcgcc gcgcagtagc 1020

cggcctcctg gcgtcaccca gcccagccca ggcccagacc ctcacccggg tcccgcggcc 1080

ggcagagttg gccccactct gcggccgccg tggcctgcgc accgacatcg atgcgacctg 1140

cacgccccgc cgcgcaagtt cgaaccaacg tggcctcaac cagatttgga atgtcaaaaa 1200

gcagagtgtc tatttgatga atttgaggaa atctggaact ttgggccacc caggctctct 1260

agatgagacc acctatgaaa gactagcaga ggaaacgctg gactctttag cagagttttt 1320

tgaagacctt gcagacaagc catacacgtt tgaggactat gatgtctcct ttgggagtgg 1380

tgtcttaact gtcaaactgg gtggagatct aggaacctat gtgatcaaca agcagacgcc 1440

aaacaagcaa atctggctat cttctccatc cagtggacct aagcgttatg actggactgg 1500

gaaaaactgg gtgtactccc acgacggcgt gtccctccat gagctgctgg ccgcagagct 1560

cactaaagcc ttaaaaacca aactggactt gtcttccttg gcctattccg gaaaagatgc 1620

tggatccgac tacaaagacc atgacggtga ttataaagat catgacatcg actacaagga 1680

tgacgatgac aagtagctcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggcct 1860

aggtagataa gtagcatggc gggttaatca ttaactacaa ggaaccccta gtgatggagt 1920

tggccactcc ctctctgcgc gctcgctcgc tcactgaggc cgggcgacca aaggtcgccc 1980

gacgcccggg ctttgcccgg gcggcctcag tgagcgagcg agcgcgcagc cttaattaac 2040

ctaattcact ggccgtcgtt ttacaacgtc gtgactggga aaaccctggc gttacccaac 2100

ttaatcgcct tgcagcacat ccccctttcg ccagctggcg taatagcgaa gaggcccgca 2160

ccgatcgccc ttcccaacag ttgcgcagcc tgaatggcga atgggacgcg ccctgtagcg 2220

gcgcattaag cgcggcgggt gtggtggtta cgcgcagcgt gaccgctaca cttgccagcg 2280

ccctagcgcc cgctcctttc gctttcttcc cttcctttct cgccacgttc gccggctttc 2340

cccgtcaagc tctaaatcgg gggctccctt tagggttccg atttagtgct ttacggcacc 2400

tcgaccccaa aaaacttgat tagggtgatg gttcacgtag tgggccatcg ccctgataga 2460

cggtttttcg ccctttgacg ttggagtcca cgttctttaa tagtggactc ttgttccaaa 2520

ctggaacaac actcaaccct atctcggtct attcttttga tttataaggg attttgccga 2580

tttcggccta ttggttaaaa aatgagctga tttaacaaaa atttaacgcg aattttaaca 2640

aaatattaac gcttacaatt taggtggcac ttttcgggga aatgtgcgcg gaacccctat 2700

ttgtttattt ttctaaatac attcaaatat gtatccgctc atgagacaat aaccctgata 2760

aatgcttcaa taatattgaa aaaggaagag tatgagtatt caacatttcc gtgtcgccct 2820

tattcccttt tttgcggcat tttgccttcc tgtttttgct cacccagaaa cgctggtgaa 2880

agtaaaagat gctgaagatc agttgggtgc acgagtgggt tacatcgaac tggatctcaa 2940

cagcggtaag atccttgaga gttttcgccc cgaagaacgt tttccaatga tgagcacttt 3000

taaagttctg ctatgtggcg cggtattatc ccgtattgac gccgggcaag agcaactcgg 3060

tcgccgcata cactattctc agaatgactt ggttgagtac tcaccagtca cagaaaagca 3120

tcttacggat ggcatgacag taagagaatt atgcagtgct gccataacca tgagtgataa 3180

cactgcggcc aacttacttc tgacaacgat cggaggaccg aaggagctaa ccgctttttt 3240

gcacaacatg ggggatcatg taactcgcct tgatcgttgg gaaccggagc tgaatgaagc 3300

cataccaaac gacgagcgtg acaccacgat gcctgtagca atggcaacaa cgttgcgcaa 3360

actattaact ggcgaactac ttactctagc ttcccggcaa caattaatag actggatgga 3420

ggcggataaa gttgcaggac cacttctgcg ctcggccctt ccggctggct ggtttattgc 3480

tgataaatct ggagccggtg agcgtgggtc tcgcggtatc attgcagcac tggggccaga 3540

tggtaagccc tcccgtatcg tagttatcta cacgacgggg agtcaggcaa ctatggatga 3600

acgaaataga cagatcgctg agataggtgc ctcactgatt aagcattggt aactgtcaga 3660

ccaagtttac tcatatatac tttagattga tttaaaactt catttttaat ttaaaaggat 3720

ctaggtgaag atcctttttg ataatctcat gaccaaaatc ccttaacgtg agttttcgtt 3780

ccactgagcg tcagaccccg tagaaaagat caaaggatct tcttgagatc ctttttttct 3840

gcgcgtaatc tgctgcttgc aaacaaaaaa accaccgcta ccagcggtgg tttgtttgcc 3900

ggatcaagag ctaccaactc tttttccgaa ggtaactggc ttcagcagag cgcagatacc 3960

aaatactgtt cttctagtgt agccgtagtt aggccaccac ttcaagaact ctgtagcacc 4020

gcctacatac ctcgctctgc taatcctgtt accagtggct gctgccagtg gcgataagtc 4080

gtgtcttacc gggttggact caagacgata gttaccggat aaggcgcagc ggtcgggctg 4140

aacggggggt tcgtgcacac agcccagctt ggagcgaacg acctacaccg aactgagata 4200

cctacagcgt gagctatgag aaagcgccac gcttcccgaa gggagaaagg cggacaggta 4260

tccggtaagc ggcagggtcg gaacaggaga gcgcacgagg gagcttccag ggggaaacgc 4320

ctggtatctt tatagtcctg tcgggtttcg ccacctctga cttgagcgtc gatttttgtg 4380

atgctcgtca ggggggcgga gcctatggaa aaacgccagc aacgcggcct ttttacggtt 4440

cctggccttt tgctggcctt ttgctcacat gttctttcct gcgttatccc ctgattctgt 4500

ggataaccgt attaccgcct ttgagtgagc tgataccgct cgccgcagcc gaacgaccga 4560

gcgcagcgag tcagtgagcg aggaagcgga agagcgccca atacgcaaac cgcctctccc 4620

cgcgcgttgg ccgattcatt aatgcagctg gcacgacagg tttcccgact ggaaagcggg 4680

cagtgagcgc aacgcaatta atgtgagtta gctcactcat taggcacccc aggctttaca 4740

ctttatgctt ccggctcgta tgttgtgtgg aattgtgagc ggataacaat ttcacacagg 4800

aaacagctat gaccatgatt acgccagatt taattaaggc cttaattagg 4850

<210> 66
<211> 4886
<212> DNA
<213> Artificial Sequence

<220>

<223> Description of Artificial Sequence: Synthetic
      polynucleotide

<400> 66
ctgcgcgctc gctcgctcac tgaggccgcc cgggcaaagc ccgggcgtcg ggcgaccttt      60

ggtcgcccgg cctcagtgag cgagcgagcg cgcagagagg gagtgtagcc atgctctagg     120

aagatcaatt caattcacgc gtcgacattg attattgact agttattaat agtaatcaat     180

tacggggtca ttagttcata gcccatatat ggagttccgc gttacataac ttacggtaaa     240

tggcccgcct ggctgaccgc ccaacgaccc ccgcccattg acgtcaataa tgacgtatgt     300

tcccatagta acgccaatag ggactttcca ttgacgtcaa tgggtggagt atttacggta     360

aactgcccac ttggcagtac atcaagtgta tcatatgcca agtacgcccc ctattgacgt     420

caatgacggt aaatggcccg cctggcatta tgcccagtac atgaccttat gggactttcc     480

tacttggcag tacatctacg tattagtcat cgctattacc atgtcgaggc cacgttctgc     540

ttcactctcc ccatctcccc cccctcccca cccccaattt tgtatttatt tattttttaa     600

ttattttgtg cagcgatggg ggcggggggg gggggcgcgc gccaggcggg gcggggcggg     660

gcgaggggcg gggcggggcg aggcggagag gtgcggcggc agccaatcag agcggcgcgc     720

tccgaaagtt tccttttatg gcgaggcggc ggcggcggcg gccctataaa aagcgaagcg     780

cgcggcgggc gggagcaagc ttgaactgaa aaaccagaaa gttaactggt aagtttagtc     840

tttttgtctt ttatttcagg tcccggatcc ggtggtggtg caaatcaaag aactgctcct     900

cagtggatgt tgcctttact tctaggcctg tacggaagtg ttacttctgc tctaaaagct     960

gcggaattgt acccgcggcc gatccaccgg tatgtggact ctcgggcgcc gcgcagtagc    1020

cggcctcctg gcgtcaccca gcccagccca ggcccagacc ctcacccggg tcccgcggcc    1080

ggcagagttg gccccactct gcggccgccg tggcctgcgc accgacatcg atgcgacctg    1140

cacgccccgc cgcgcaagtt cgaaccaacg tggcctcaac cagatttgga atgtcaaaaa    1200

gcagagtgtc tatttgatga atttgaggaa atctggaact ttgggccacc caggctctct    1260

agatgagacc acctatgaaa gactagcaga ggaaacgctg gactctttag cagagttttt    1320

tgaagacctt gcagacaagc catacacgtt tgaggactat gatgtctcct ttgggagtgg    1380

tgtcttaact gtcaaactgg gtggagatct aggaacctat gtgatcaaca agcagacgcc    1440

aaacaagcaa atctggctat cttctccatc cagtggacct aagcgttatg actggactgg    1500

gaaaaactgg gtgtactccc acgacggcgt gtccctccat gagctgctgg ccgcagagct    1560

cactaaagcc ttaaaaacca aactggactt gtcttccttg gcctattccg gaaaagatgc    1620

tggatccgaa caaaaactca tctcagaaga ggatctggga tccgactaca aagaccatga    1680

cggtgattat aaagatcatg acatcgacta caaggatgac gatgacaagt agctcgagga    1740

cggggtgaac tacgcctgag gatccgatct ttttccctct gccaaaaatt atggggacat    1800

catgaagccc cttgagcatc tgacttctgg ctaataaagg aaatttattt tcattgcaat 1860

agtgtgttgg aattttttgt gtctctcact cggcctaggt agataagtag catggcgggt 1920

taatcattaa ctacaaggaa cccctagtga tggagttggc cactccctct ctgcgcgctc 1980

gctcgctcac tgaggccggg cgaccaaagg tcgcccgacg cccgggcttt gcccgggcgg 2040

cctcagtgag cgagcgagcg cgcagcctta attaacctaa ttcactggcc gtcgttttac 2100

aacgtcgtga ctgggaaaac cctggcgtta cccaacttaa tcgccttgca gcacatcccc 2160

ctttcgccag ctggcgtaat agcgaagagg cccgcaccga tcgcccttcc caacagttgc 2220

gcagcctgaa tggcgaatgg gacgcgccct gtagcggcgc attaagcgcg gcgggtgtgg 2280

tggttacgcg cagcgtgacc gctacacttg ccagcgccct agcgcccgct cctttcgctt 2340

tcttcccttc ctttctcgcc acgttcgccg gctttccccg tcaagctcta aatcgggggc 2400

tccctttagg gttccgattt agtgctttac ggcacctcga ccccaaaaaa cttgattagg 2460

gtgatggttc acgtagtggg ccatcgccct gatagacggt ttttcgccct ttgacgttgg 2520

agtccacgtt ctttaatagt ggactcttgt tccaaactgg aacaacactc aaccctatct 2580

cggtctattc ttttgattta taagggattt tgccgatttc ggcctattgg ttaaaaaatg 2640

agctgattta acaaaaattt aacgcgaatt ttaacaaaat attaacgctt acaatttagg 2700

tggcactttt cggggaaatg tgcgcggaac ccctatttgt ttatttttct aaatacattc 2760

aaatatgtat ccgctcatga gacaataacc ctgataaatg cttcaataat attgaaaaag 2820

gaagagtatg agtattcaac atttccgtgt cgcccttatt cccttttttg cggcattttg 2880

ccttcctgtt tttgctcacc cagaaacgct ggtgaaagta aaagatgctg aagatcagtt 2940

gggtgcacga gtgggttaca tcgaactgga tctcaacagc ggtaagatcc ttgagagttt 3000

tcgccccgaa gaacgttttc caatgatgag cacttttaaa gttctgctat gtggcgcggt 3060

attatcccgt attgacgccg ggcaagagca actcggtcgc cgcatacact attctcagaa 3120

tgacttggtt gagtactcac cagtcacaga aaagcatctt acggatggca tgacagtaag 3180

agaattatgc agtgctgcca taaccatgag tgataacact gcggccaact tacttctgac 3240

aacgatcgga ggaccgaagg agctaaccgc ttttttgcac aacatggggg atcatgtaac 3300

tcgccttgat cgttgggaac cggagctgaa tgaagccata ccaaacgacg agcgtgacac 3360

cacgatgcct gtagcaatgg caacaacgtt gcgcaaacta ttaactggcg aactacttac 3420

tctagcttcc cggcaacaat taatagactg gatggaggcg gataaagttg caggaccact 3480

tctgcgctcg gcccttccgg ctggctggtt tattgctgat aaatctggag ccggtgagcg 3540

tgggtctcgc ggtatcattg cagcactggg gccagatggt aagccctccc gtatcgtagt 3600

tatctacacg acggggagtc aggcaactat ggatgaacga aatagacaga tcgctgagat 3660

aggtgcctca ctgattaagc attggtaact gtcagaccaa gtttactcat atatacttta 3720

gattgattta aaacttcatt tttaatttaa aaggatctag gtgaagatcc tttttgataa 3780

tctcatgacc aaaatccctt aacgtgagtt ttcgttccac tgagcgtcag accccgtaga 3840

aaagatcaaa ggatcttctt gagatccttt ttttctgcgc gtaatctgct gcttgcaaac 3900

aaaaaaacca ccgctaccag cggtggtttg tttgccggat caagagctac caactctttt 3960

tccgaaggta actggcttca gcagagcgca gataccaaat actgttcttc tagtgtagcc 4020

gtagttaggc caccacttca agaactctgt agcaccgcct acatacctcg ctctgctaat 4080

cctgttacca gtggctgctg ccagtggcga taagtcgtgt cttaccgggt tggactcaag 4140

acgatagtta ccggataagg cgcagcggtc gggctgaacg gggggttcgt gcacacagcc 4200

cagcttggag cgaacgacct acaccgaact gagataccta cagcgtgagc tatgagaaag 4260

cgccacgctt cccgaaggga gaaaggcgga caggtatccg gtaagcggca gggtcggaac 4320

aggagagcgc acgagggagc ttccaggggg aaacgcctgg tatctttata gtcctgtcgg 4380

gtttcgccac ctctgacttg agcgtcgatt tttgtgatgc tcgtcagggg ggcggagcct 4440

atggaaaaac gccagcaacg cggccttttt acggttcctg gccttttgct ggccttttgc 4500

tcacatgttc tttcctgcgt tatcccctga ttctgtggat aaccgtatta ccgcctttga 4560

gtgagctgat accgctcgcc gcagccgaac gaccgagcgc agcgagtcag tgagcgagga 4620

agcggaagag cgcccaatac gcaaaccgcc tctccccgcg cgttggccga ttcattaatg 4680

cagctggcac gacaggtttc ccgactggaa agcgggcagt gagcgcaacg caattaatgt 4740

gagttagctc actcattagg caccccaggc tttacacttt atgcttccgg ctcgtatgtt 4800

gtgtggaatt gtgagcggat aacaatttca cacaggaaac agctatgacc atgattacgc 4860

cagatttaat taaggcctta attagg 4886

<210> 67
<211> 4808
<212> DNA
<213> Artificial Sequence

<220>
<223> Description of Artificial Sequence: Synthetic
      polynucleotide

<400> 67
ctgcgcgctc gctcgctcac tgaggccgcc cgggcaaagc ccgggcgtcg ggcgaccttt 60

ggtcgcccgg cctcagtgag cgagcgagcg cgcagagagg gagtgtagcc atgctctagg 120

aagatcaatt caattcacgc gtcgacattg attattgact agttattaat agtaatcaat 180

tacggggtca ttagttcata gcccatatat ggagttccgc gttacataac ttacggtaaa 240

tggcccgcct ggctgaccgc ccaacgaccc ccgcccattg acgtcaataa tgacgtatgt 300

tcccatagta acgccaatag ggactttcca ttgacgtcaa tgggtggagt atttacggta 360

aactgcccac ttggcagtac atcaagtgta tcatatgcca agtacgcccc ctattgacgt 420

caatgacggt aaatggcccg cctggcatta tgcccagtac atgaccttat gggactttcc 480

tacttggcag tacatctacg tattagtcat cgctattacc atgtcgaggc cacgttctgc 540

ttcactctcc ccatctcccc cccctcccca cccccaattt tgtatttatt tattttttaa 600

ttattttgtg cagcgatggg ggcggggggg gggggcgcgc gccaggcggg gcggggcggg 660

gcgaggggcg gggcggggcg aggcggagag gtgcggcggc agccaatcag agcggcgcgc 720

tccgaaagtt tccttttatg gcgaggcggc ggcggcggcg gccctataaa aagcgaagcg 780

cgcggcgggc gggagcaagc ttgaactgaa aaaccagaaa gttaactggt aagtttagtc 840

tttttgtctt ttatttcagg tcccggatcc ggtggtggtg caaatcaaag aactgctcct 900

cagtggatgt tgcctttact tctaggcctg tacggaagtg ttacttctgc tctaaaagct 960

gcggaattgt acccgcggcc gatccaccgg tatgtggact ctcgggcgcc gcgcagtagc 1020

cggcctcctg gcgtcaccca gcccagccca ggcccagacc ctcacccggg tcccgcggcc 1080

ggcagagttg gccccactct gcggccgccg tggcctgcgc accgacatcg atgcgacctg 1140

cacgccccgc cgcgcaagtt cgaaccaacg tggcctcaac cagatttgga atgtcaaaaa 1200

gcagagtgtc tatttgatga atttgaggaa atctggaact ttgggccacc caggctctct 1260

agatgagacc acctatgaaa gactagcaga ggaaacgctg gactctttag cagagttttt 1320

tgaagacctt gcagacaagc catacacgtt tgaggactat gatgtctcct ttgggagtgg 1380

tgtcttaact gtcaaactgg gtggagatct aggaacctat gtgatcaaca agcagacgcc 1440

aaacaagcaa atctggctat cttctccatc cagtggacct aagcgttatg actggactgg 1500

gaaaaactgg gtgtactccc acgacggcgt gtccctccat gagctgctgg ccgcagagct 1560

cactaaagcc ttaaaaacca aactggactt gtcttccttg gcctattccg gaaaagatgc 1620

tgaacaaaaa ctcatctcag aagaggatct gtagctcgag gacggggtga actacgcctg 1680

aggatccgat ctttttccct ctgccaaaaa ttatggggac atcatgaagc cccttgagca 1740

tctgacttct ggctaataaa ggaaatttat tttcattgca atagtgtgtt ggaatttttt 1800

gtgtctctca ctcggcctag gtagataagt agcatggcgg gttaatcatt aactacaagg 1860

aacccctagt gatggagttg gccactccct ctctgcgcgc tcgctcgctc actgaggccg 1920

ggcgaccaaa ggtcgcccga cgcccgggct ttgcccgggc ggcctcagtg agcgagcgag 1980

cgcgcagcct taattaacct aattcactgg ccgtcgtttt acaacgtcgt gactgggaaa 2040

accctggcgt tacccaactt aatcgccttg cagcacatcc ccctttcgcc agctggcgta 2100

atagcgaaga ggcccgcacc gatcgccctt cccaacagtt gcgcagcctg aatggcgaat 2160

gggacgcgcc ctgtagcggc gcattaagcg cggcgggtgt ggtggttacg cgcagcgtga 2220

ccgctacact tgccagcgcc ctagcgcccg ctcctttcgc tttcttccct tcctttctcg 2280

ccacgttcgc cggctttccc cgtcaagctc taaatcgggg gctcccttta gggttccgat 2340

ttagtgcttt acggcacctc gaccccaaaa aacttgatta gggtgatggt tcacgtagtg 2400

ggccatcgcc ctgatagacg gtttttcgcc ctttgacgtt ggagtccacg ttctttaata 2460

gtggactctt gttccaaact ggaacaacac tcaaccctat ctcggtctat tcttttgatt 2520

tataagggat tttgccgatt tcggcctatt ggttaaaaaa tgagctgatt taacaaaaat 2580

ttaacgcgaa ttttaacaaa atattaacgc ttacaattta ggtggcactt ttcggggaaa 2640

tgtgcgcgga acccctattt gtttattttt ctaaatacat tcaaatatgt atccgctcat 2700

gagacaataa ccctgataaa tgcttcaata atattgaaaa aggaagagta tgagtattca 2760

acatttccgt gtcgccctta ttcccttttt tgcggcattt tgccttcctg tttttgctca 2820

cccagaaacg ctggtgaaag taaaagatgc tgaagatcag ttgggtgcac gagtgggtta 2880

catcgaactg gatctcaaca gcggtaagat ccttgagagt tttcgccccg aagaacgttt 2940

tccaatgatg agcactttta aagttctgct atgtggcgcg gtattatccc gtattgacgc 3000

cgggcaagag caactcggtc gccgcataca ctattctcag aatgacttgg ttgagtactc 3060

accagtcaca gaaaagcatc ttacggatgg catgacagta agagaattat gcagtgctgc 3120

cataaccatg agtgataaca ctgcggccaa cttacttctg acaacgatcg gaggaccgaa 3180

ggagctaacc gcttttttgc acaacatggg ggatcatgta actcgccttg atcgttggga 3240

accggagctg aatgaagcca taccaaacga cgagcgtgac accacgatgc ctgtagcaat 3300

ggcaacaacg ttgcgcaaac tattaactgg cgaactactt actctagctt cccggcaaca 3360

attaatagac tggatggagg cggataaagt tgcaggacca cttctgcgct cggcccttcc 3420

ggctggctgg tttattgctg ataaatctgg agccggtgag cgtgggtctc gcggtatcat 3480

tgcagcactg gggccagatg gtaagccctc ccgtatcgta gttatctaca cgacggggag 3540

tcaggcaact atggatgaac gaaatagaca gatcgctgag ataggtgcct cactgattaa 3600

gcattggtaa ctgtcagacc aagtttactc atatatactt tagattgatt taaaacttca 3660

tttttaattt aaaaggatct aggtgaagat cctttttgat aatctcatga ccaaaatccc 3720

ttaacgtgag ttttcgttcc actgagcgtc agaccccgta gaaaagatca aaggatcttc 3780

ttgagatcct ttttttctgc gcgtaatctg ctgcttgcaa acaaaaaaac caccgctacc 3840

agcggtggtt tgtttgccgg atcaagagct accaactctt tttccgaagg taactggctt 3900

cagcagagcg cagataccaa atactgttct tctagtgtag ccgtagttag gccaccactt 3960

caagaactct gtagcaccgc ctacatacct cgctctgcta atcctgttac cagtggctgc 4020

tgccagtggc gataagtcgt gtcttaccgg gttggactca agacgatagt taccggataa 4080

ggcgcagcgg tcgggctgaa cggggggttc gtgcacacag cccagcttgg agcgaacgac 4140

ctacaccgaa ctgagatacc tacagcgtga gctatgagaa agcgccacgc ttcccgaagg 4200

gagaaaggcg gacaggtatc cggtaagcgg cagggtcgga acaggagagc gcacgaggga 4260

gcttccaggg ggaaacgcct ggtatcttta tagtcctgtc gggtttcgcc acctctgact 4320

tgagcgtcga tttttgtgat gctcgtcagg ggggcggagc ctatggaaaa acgccagcaa 4380

cgcggccttt ttacggttcc tggccttttg ctggcctttt gctcacatgt tctttcctgc 4440

gttatcccct gattctgtgg ataaccgtat taccgccttt gagtgagctg ataccgctcg 4500

ccgcagccga acgaccgagc gcagcgagtc agtgagcgag gaagcggaag agcgcccaat 4560

acgcaaaccg cctctccccg cgcgttggcc gattcattaa tgcagctggc acgacaggtt 4620

tcccgactgg aaagcgggca gtgagcgcaa cgcaattaat gtgagttagc tcactcatta 4680

ggcaccccag gctttacact ttatgcttcc ggctcgtatg ttgtgtggaa ttgtgagcgg 4740

ataacaattt cacacaggaa acagctatga ccatgattac gccagattta attaaggcct 4800

taattagg 4808

<210> 68
<211> 4886
<212> DNA
<213> Artificial Sequence

<220>
<223> Description of Artificial Sequence: Synthetic
      polynucleotide

<400> 68
ctgcgcgctc gctcgctcac tgaggccgcc cgggcaaagc ccgggcgtcg ggcgaccttt 60

ggtcgcccgg cctcagtgag cgagcgagcg cgcagagagg gagtgtagcc atgctctagg 120

aagatcaatt caattcacgc gtcgacattg attattgact agttattaat agtaatcaat 180

tacggggtca ttagttcata gcccatatat ggagttccgc gttacataac ttacggtaaa 240

tggcccgcct ggctgaccgc ccaacgaccc ccgcccattg acgtcaataa tgacgtatgt 300

tcccatagta acgccaatag ggactttcca ttgacgtcaa tgggtggagt atttacggta 360

aactgcccac ttggcagtac atcaagtgta tcatatgcca agtacgcccc ctattgacgt 420

caatgacggt aaatggcccg cctggcatta tgcccagtac atgaccttat gggactttcc 480

tacttggcag tacatctacg tattagtcat cgctattacc atgtcgaggc cacgttctgc 540

ttcactctcc ccatctcccc cccctcccca cccccaattt tgtatttatt tattttttaa 600

ttattttgtg cagcgatggg ggcggggggg gggggcgcgc gccaggcggg gcggggcggg 660

gcgaggggcg gggcggggcg aggcggagag gtgcggcggc agccaatcag agcggcgcgc 720

tccgaaagtt tccttttatg gcgaggcggc ggcggcggcg gccctataaa aagcgaagcg 780

cgcggcgggc gggagcaagc ttgaactgaa aaaccagaaa gttaactggt aagtttagtc 840
tttttgtctt ttatttcagg tcccggatcc ggtggtggtg caaatcaaag aactgctcct 900
cagtggatgt tgcctttact tctaggcctg tacggaagtg ttacttctgc tctaaaagct 960
gcggaattgt acccgcggcc gatccaccgg tatgtggact ctcgggcgcc gcgcagtagc 1020
cggcctcctg gcgtcaccca gcccagccca ggcccagacc ctcacccggg tcccgcggcc 1080
ggcagagttg gccccactct gcggccgccg tggcctgcgc accgacatcg atgcgacctg 1140
cacgccccgc cgcgcaagtt cgaaccaacg tggcctcaac cagatttgga atgtcaaaaa 1200
gcagagtgtc tatttgatga atttgaggaa atctggaact ttgggccacc caggctctct 1260
agatgagacc acctatgaaa gactagcaga ggaaacgctg gactctttag cagagttttt 1320
tgaagacctt gcagacaagc catacacgtt tgaggactat gatgtctcct ttgggagtgg 1380
tgtcttaact gtcaaactgg gtggagatct aggaacctat gtgatcaaca agcagacgcc 1440
aaacaagcaa atctggctat cttctccatc cagtggacct aagcgttatg actggactgg 1500
gaaaaactgg gtgtactccc acgacggcgt gtccctccat gagctgctgg ccgcagagct 1560
cactaaagcc ttaaaaacca aactggactt gtcttccttg gcctattccg gaaaagatgc 1620
tggatccgac tacaaagacc atgacggtga ttataaagat catgacatcg actacaagga 1680
tgacgatgac aagggatccg aacaaaaact catctcagaa gaggatctgt agctcgagga 1740
cggggtgaac tacgcctgag gatccgatct ttttccctct gccaaaaatt atggggacat 1800
catgaagccc cttgagcatc tgacttctgg ctaataaagg aaatttattt tcattgcaat 1860
agtgtgttgg aattttttgt gtctctcact cggcctaggt agataagtag catggcgggt 1920
taatcattaa ctacaaggaa cccctagtga tggagttggc cactccctct ctgcgcgctc 1980
gctcgctcac tgaggccggg cgaccaaagg tcgcccgacg cccgggcttt gcccgggcgg 2040
cctcagtgag cgagcgagcg cgcagcctta attaacctaa ttcactggcc gtcgttttac 2100
aacgtcgtga ctgggaaaac cctggcgtta cccaacttaa tcgccttgca gcacatcccc 2160
ctttcgccag ctggcgtaat agcgaagagg cccgcaccga tcgcccttcc caacagttgc 2220
gcagcctgaa tggcgaatgg gacgcgccct gtagcggcgc attaagcgcg gcgggtgtgg 2280
tggttacgcg cagcgtgacc gctacacttg ccagcgccct agcgcccgct cctttcgctt 2340
tcttcccttc ctttctcgcc acgttcgccg gctttccccg tcaagctcta aatcgggggc 2400
tccctttagg gttccgattt agtgctttac ggcacctcga ccccaaaaaa cttgattagg 2460
gtgatggttc acgtagtggg ccatcgccct gatagacggt ttttcgccct ttgacgttgg 2520
agtccacgtt ctttaatagt ggactcttgt tccaaactgg aacaacactc aaccctatct 2580
cggtctattc ttttgattta taagggattt tgccgatttc ggcctattgg ttaaaaaatg 2640
agctgattta acaaaaattt aacgcgaatt ttaacaaaat attaacgctt acaatttagg 2700

tggcactttt cggggaaatg tgcgcggaac ccctatttgt ttatttttct aaatacattc 2760

aaatatgtat ccgctcatga gacaataacc ctgataaatg cttcaataat attgaaaaag 2820

gaagagtatg agtattcaac atttccgtgt cgcccttatt cccttttttg cggcattttg 2880

ccttcctgtt tttgctcacc cagaaacgct ggtgaaagta aaagatgctg aagatcagtt 2940

gggtgcacga gtgggttaca tcgaactgga tctcaacagc ggtaagatcc ttgagagttt 3000

tcgccccgaa gaacgttttc caatgatgag cacttttaaa gttctgctat gtggcgcggt 3060

attatcccgt attgacgccg ggcaagagca actcggtcgc cgcatacact attctcagaa 3120

tgacttggtt gagtactcac cagtcacaga aaagcatctt acggatggca tgacagtaag 3180

agaattatgc agtgctgcca taaccatgag tgataacact gcggccaact tacttctgac 3240

aacgatcgga ggaccgaagg agctaaccgc ttttttgcac aacatggggg atcatgtaac 3300

tcgccttgat cgttgggaac cggagctgaa tgaagccata ccaaacgacg agcgtgacac 3360

cacgatgcct gtagcaatgg caacaacgtt gcgcaaacta ttaactggcg aactacttac 3420

tctagcttcc cggcaacaat taatagactg gatggaggcg gataaagttg caggaccact 3480

tctgcgctcg gcccttccgg ctggctggtt tattgctgat aaatctggag ccggtgagcg 3540

tgggtctcgc ggtatcattg cagcactggg gccagatggt aagccctccc gtatcgtagt 3600

tatctacacg acggggagtc aggcaactat ggatgaacga aatagacaga tcgctgagat 3660

aggtgcctca ctgattaagc attggtaact gtcagaccaa gtttactcat atatacttta 3720

gattgattta aaacttcatt tttaatttaa aaggatctag gtgaagatcc tttttgataa 3780

tctcatgacc aaaatccctt aacgtgagtt ttcgttccac tgagcgtcag accccgtaga 3840

aaagatcaaa ggatcttctt gagatccttt ttttctgcgc gtaatctgct gcttgcaaac 3900

aaaaaaacca ccgctaccag cggtggtttg tttgccggat caagagctac caactctttt 3960

tccgaaggta actggcttca gcagagcgca gataccaaat actgttcttc tagtgtagcc 4020

gtagttaggc caccacttca agaactctgt agcaccgcct acatacctcg ctctgctaat 4080

cctgttacca gtggctgctg ccagtggcga taagtcgtgt cttaccgggt tggactcaag 4140

acgatagtta ccggataagg cgcagcggtc gggctgaacg gggggttcgt gcacacagcc 4200

cagcttggag cgaacgacct acaccgaact gagataccta cagcgtgagc tatgagaaag 4260

cgccacgctt cccgaaggga gaaaggcgga caggtatccg gtaagcggca gggtcggaac 4320

aggagagcgc acgagggagc ttccaggggg aaacgcctgg tatctttata gtcctgtcgg 4380

gtttcgccac ctctgacttg agcgtcgatt tttgtgatgc tcgtcagggg ggcggagcct 4440

atggaaaaac gccagcaacg cggccttttt acggttcctg gccttttgct ggccttttgc 4500

tcacatgttc tttcctgcgt tatcccctga ttctgtggat aaccgtatta ccgcctttga 4560

gtgagctgat accgctcgcc gcagccgaac gaccgagcgc agcgagtcag tgagcgagga 4620

agcggaagag cgcccaatac gcaaaccgcc tctccccgcg cgttggccga ttcattaatg 4680

cagctggcac gacaggtttc ccgactggaa agcgggcagt gagcgcaacg caattaatgt 4740

gagttagctc actcattagg caccccaggc tttacacttt atgcttccgg ctcgtatgtt 4800

gtgtggaatt gtgagcggat aacaatttca cacaggaaac agctatgacc atgattacgc 4860

cagatttaat taaggcctta attagg 4886

<210> 69
<211> 4778
<212> DNA
<213> Artificial Sequence

<220>
<223> Description of Artificial Sequence: Synthetic
      polynucleotide

<400> 69
ctgcgcgctc gctcgctcac tgaggccgcc cgggcaaagc ccgggcgtcg ggcgaccttt 60

ggtcgcccgg cctcagtgag cgagcgagcg cgcagagagg gagtgtagcc atgctctagg 120

aagatcaatt caattcacgc gtcgacattg attattgact agttattaat agtaatcaat 180

tacggggtca ttagttcata gcccatatat ggagttccgc gttacataac ttacggtaaa 240

tggcccgcct ggctgaccgc ccaacgaccc ccgcccattg acgtcaataa tgacgtatgt 300

tcccatagta acgccaatag ggactttcca ttgacgtcaa tgggtggagt atttacggta 360

aactgcccac ttggcagtac atcaagtgta tcatatgcca agtacgcccc ctattgacgt 420

caatgacggt aaatggcccg cctggcatta tgcccagtac atgaccttat gggactttcc 480

tacttggcag tacatctacg tattagtcat cgctattacc atgtcgaggc cacgttctgc 540

ttcactctcc ccatctcccc cccctcccca cccccaattt tgtatttatt tattttttaa 600

ttattttgtg cagcgatggg ggcggggggg gggggcgcgc gccaggcggg gcggggcggg 660

gcgaggggcg gggcggggcg aggcggagag gtgcggcggc agccaatcag agcggcgcgc 720

tccgaaagtt tccttttatg gcgaggcggc ggcggcggcg gccctataaa aagcgaagcg 780

cgcggcgggc gggagcaagc ttgaactgaa aaaccagaaa gttaactggt aagtttagtc 840

tttttgtctt ttatttcagg tcccggatcc ggtggtggtg caaatcaaag aactgctcct 900

cagtggatgt tgcctttact tctaggcctg tacggaagtg ttacttctgc tctaaaagct 960

gcggaattgt acccgcggcc gatccaccgg tatgtggaca ctcgggcgac gcgccgtggc 1020

tgggctgctt gcttccccaa gtcccgcaca ggcgcaaacc ctcacaaggg tcccacggcc 1080

agctgaattg gccccgctgt gcggtcggag gggcctgcga acagatatag acgcaacgtg 1140

cacccccaga agagcaagca gcaaccagag aggcctcaat cagatttgga acgtcaagaa 1200

acagagtgtc tatctgatga acctccgcaa atcagggacc cttggtcatc cgggatctct 1260

tgatgaaacg acctatgaac ggctggctga ggagacgctt gactccctgg cagagttctt 1320

cgaggatctg gctgataagc catacacgtt cgaggactat gatgtttctt ttggttctgg 1380

cgtcctgacg gtgaaactcg gaggggatct gggcacttac gttatcaaca aacagactcc 1440

caataaacag atttggttgt ctagtcctag cagtggccca aaacgctatg actggacggg 1500

caagaactgg gtgtactccc acgatggagt tagcctgcac gaacttctgg ccgctgagct 1560

tactaaagcg ctgaagacta agctggatct ctccagcctc gcatacagcg gcaaagacgc 1620

ctagctcgag gacggggtga actacgcctg aggatccgat ctttttccct ctgccaaaaa 1680

ttatggggac atcatgaagc cccttgagca tctgacttct ggctaataaa ggaaatttat 1740

tttcattgca atagtgtgtt ggaatttttt gtgtctctca ctcggcctag gtagataagt 1800

agcatggcgg gttaatcatt aactacaagg aacccctagt gatggagttg gccactccct 1860

ctctgcgcgc tcgctcgctc actgaggccg ggcgaccaaa ggtcgcccga cgcccgggct 1920

ttgcccgggc ggcctcagtg agcgagcgag cgcgcagcct taattaacct aattcactgg 1980

ccgtcgtttt acaacgtcgt gactgggaaa accctggcgt tacccaactt aatcgccttg 2040

cagcacatcc ccctttcgcc agctggcgta atagcgaaga ggcccgcacc gatcgccctt 2100

cccaacagtt gcgcagcctg aatggcgaat gggacgcgcc ctgtagcggc gcattaagcg 2160

cggcgggtgt ggtggttacg cgcagcgtga ccgctacact tgccagcgcc ctagcgcccg 2220

ctcctttcgc tttcttccct tcctttctcg ccacgttcgc cggctttccc cgtcaagctc 2280

taaatcgggg gctcccttta gggttccgat ttagtgcttt acggcacctc gaccccaaaa 2340

aacttgatta gggtgatggt tcacgtagtg ggccatcgcc ctgatagacg gtttttcgcc 2400

ctttgacgtt ggagtccacg ttctttaata gtggactctt gttccaaact ggaacaacac 2460

tcaaccctat ctcggtctat tcttttgatt tataagggat tttgccgatt tcggcctatt 2520

ggttaaaaaa tgagctgatt taacaaaaat ttaacgcgaa ttttaacaaa atattaacgc 2580

ttacaattta ggtggcactt ttcggggaaa tgtgcgcgga acccctattt gtttattttt 2640

ctaaatacat tcaaatatgt atccgctcat gagacaataa ccctgataaa tgcttcaata 2700

atattgaaaa aggaagagta tgagtattca acatttccgt gtcgccctta ttcccttttt 2760

tgcggcattt tgccttcctg tttttgctca cccagaaacg ctggtgaaag taaaagatgc 2820

tgaagatcag ttgggtgcac gagtgggtta catcgaactg gatctcaaca gcggtaagat 2880

ccttgagagt tttcgccccg aagaacgttt tccaatgatg agcactttta aagttctgct 2940

atgtggcgcg gtattatccc gtattgacgc cgggcaagag caactcggtc gccgcataca 3000

ctattctcag aatgacttgg ttgagtactc accagtcaca gaaaagcatc ttacggatgg 3060

catgacagta agagaattat gcagtgctgc cataaccatg agtgataaca ctgcggccaa 3120

cttacttctg acaacgatcg gaggaccgaa ggagctaacc gcttttttgc acaacatggg 3180

ggatcatgta actcgccttg atcgttggga accggagctg aatgaagcca taccaaacga 3240

cgagcgtgac accacgatgc ctgtagcaat ggcaacaacg ttgcgcaaac tattaactgg 3300

cgaactactt actctagctt cccggcaaca attaatagac tggatggagg cggataaagt 3360

tgcaggacca cttctgcgct cggcccttcc ggctggctgg tttattgctg ataaatctgg 3420

agccggtgag cgtgggtctc gcggtatcat tgcagcactg gggccagatg gtaagccctc 3480

ccgtatcgta gttatctaca cgacggggag tcaggcaact atggatgaac gaaatagaca 3540

gatcgctgag ataggtgcct cactgattaa gcattggtaa ctgtcagacc aagtttactc 3600

atatatactt tagattgatt taaaacttca tttttaattt aaaaggatct aggtgaagat 3660

cctttttgat aatctcatga ccaaaatccc ttaacgtgag ttttcgttcc actgagcgtc 3720

agaccccgta gaaaagatca aaggatcttc ttgagatcct ttttttctgc gcgtaatctg 3780

ctgcttgcaa acaaaaaaac caccgctacc agcggtggtt tgtttgccgg atcaagagct 3840

accaactctt tttccgaagg taactggctt cagcagagcg cagataccaa atactgttct 3900

tctagtgtag ccgtagttag gccaccactt caagaactct gtagcaccgc ctacatacct 3960

cgctctgcta atcctgttac cagtggctgc tgccagtggc gataagtcgt gtcttaccgg 4020

gttggactca agacgatagt taccggataa ggcgcagcgg tcgggctgaa cggggggttc 4080

gtgcacacag cccagcttgg agcgaacgac ctacaccgaa ctgagatacc tacagcgtga 4140

gctatgagaa agcgccacgc ttcccgaagg gagaaaggcg gacaggtatc cggtaagcgg 4200

cagggtcgga acaggagagc gcacgaggga gcttccaggg ggaaacgcct ggtatcttta 4260

tagtcctgtc gggtttcgcc acctctgact tgagcgtcga tttttgtgat gctcgtcagg 4320

ggggcggagc ctatggaaaa acgccagcaa cgcggccttt ttacggttcc tggccttttg 4380

ctggcctttt gctcacatgt tctttcctgc gttatcccct gattctgtgg ataaccgtat 4440

taccgccttt gagtgagctg ataccgctcg ccgcagccga acgaccgagc gcagcgagtc 4500

agtgagcgag gaagcggaag agcgcccaat acgcaaaccg cctctccccg cgcgttggcc 4560

gattcattaa tgcagctggc acgacaggtt tcccgactgg aaagcgggca gtgagcgcaa 4620

cgcaattaat gtgagttagc tcactcatta ggcaccccag gctttacact ttatgcttcc 4680

ggctcgtatg ttgtgtggaa ttgtgagcgg ataacaattt cacacaggaa acagctatga 4740

ccatgattac gccagattta attaaggcct taattagg 4778

<210> 70
<211> 4778
<212> DNA
<213> Artificial Sequence

<220>
<223> Description of Artificial Sequence: Synthetic
      polynucleotide

<400> 70
ctgcgcgctc gctcgctcac tgaggccgcc cgggcaaagc ccgggcgtcg ggcgaccttt      60

ggtcgcccgg cctcagtgag cgagcgagcg cgcagagagg gagtgtagcc atgctctagg     120

aagatcaatt caattcacgc gtcgacattg attattgact agttattaat agtaatcaat     180

tacggggtca ttagttcata gcccatatat ggagttccgc gttacataac ttacggtaaa     240

tggcccgcct ggctgaccgc ccaacgaccc ccgcccattg acgtcaataa tgacgtatgt     300

tcccatagta acgccaatag ggactttcca ttgacgtcaa tgggtggagt atttacggta     360

aactgcccac ttggcagtac atcaagtgta tcatatgcca agtacgcccc ctattgacgt     420

caatgacggt aaatggcccg cctggcatta tgcccagtac atgaccttat gggactttcc     480

tacttggcag tacatctacg tattagtcat cgctattacc atgtcgaggc cacgttctgc     540

ttcactctcc ccatctcccc cccctcccca cccccaattt tgtatttatt tattttttaa     600

ttattttgtg cagcgatggg ggcggggggg gggggcgcgc gccaggcggg gcggggcggg     660

gcgaggggcg gggcggggcg aggcggagag gtgcggcggc agccaatcag agcggcgcgc     720

tccgaaagtt tccttttatg gcgaggcggc ggcggcggcg gccctataaa aagcgaagcg     780

cgcggcgggc gggagcaagc ttgaactgaa aaaccagaaa gttaactggt aagtttagtc     840

tttttgtctt ttatttcagg tcccggatcc ggtggtggtg caaatcaaag aactgctcct     900

cagtggatgt tgcctttact tctaggcctg tacggaagtg ttacttctgc tctaaaagct     960

gcggaattgt acccgcggcc gatccaccgg tatgtggact ctgggtcgac gcgccgtagc    1020

cggtctcttg gcctccccat cccctgcgca ggcacagacc cttacacgag ttccacggcc    1080

cgctgaactg gcaccactct gcggacgccg gggcctgaga accgatattg acgctacgtg    1140

cactccccga cgagcctctt ctaatcagag ggggctcaac cagatctgga acgtgaagaa    1200

gcagtcagtc tacttgatga atctgagaaa gagcgggacc cttgggcacc ccgggtctct    1260

cgacgagact acatacgaga ggctggccga ggagactttg gactctctcg ccgaattctt    1320

cgaggatttg gcagacaagc cgtacacctt tgaagactac gatgtctcat tcggatcagg    1380

ggtgctgacg gttaaactgg ggggcgatct cggaacctac gtcataaaca aacagacacc    1440

caataagcag atctggttgt ctagcccatc atccggtcct aaacggtacg actggaccgg    1500

caagaactgg gtctactccc atgacggagt cagtcttcat gaactgcttg ccgcagagct    1560

gaccaaggct cttaagacga aactggatct ctctagcctg gcctactccg ggaaggatgc    1620

atagctcgag gacggggtga actacgcctg aggatccgat ctttttccct ctgccaaaaa    1680

ttatggggac atcatgaagc cccttgagca tctgacttct ggctaataaa ggaaatttat    1740

tttcattgca atagtgtgtt ggaatttttt gtgtctctca ctcggcctag gtagataagt 1800

agcatggcgg gttaatcatt aactacaagg aacccctagt gatggagttg gccactccct 1860

ctctgcgcgc tcgctcgctc actgaggccg ggcgaccaaa ggtcgcccga cgcccgggct 1920

ttgcccgggc ggcctcagtg agcgagcgag cgcgcagcct taattaacct aattcactgg 1980

ccgtcgtttt acaacgtcgt gactgggaaa accctggcgt tacccaactt aatcgccttg 2040

cagcacatcc ccctttcgcc agctggcgta atagcgaaga ggcccgcacc gatcgccctt 2100

cccaacagtt gcgcagcctg aatggcgaat gggacgcgcc ctgtagcggc gcattaagcg 2160

cggcgggtgt ggtggttacg cgcagcgtga ccgctacact tgccagcgcc ctagcgcccg 2220

ctcctttcgc tttcttccct tcctttctcg ccacgttcgc cggctttccc cgtcaagctc 2280

taaatcgggg gctcccttta gggttccgat ttagtgcttt acggcacctc gaccccaaaa 2340

aacttgatta gggtgatggt tcacgtagtg ggccatcgcc ctgatagacg gtttttcgcc 2400

ctttgacgtt ggagtccacg ttctttaata gtggactctt gttccaaact ggaacaacac 2460

tcaaccctat ctcggtctat tcttttgatt tataagggat tttgccgatt tcggcctatt 2520

ggttaaaaaa tgagctgatt taacaaaaat ttaacgcgaa ttttaacaaa atattaacgc 2580

ttacaattta ggtggcactt ttcggggaaa tgtgcgcgga acccctattt gtttattttt 2640

ctaaatacat tcaaatatgt atccgctcat gagacaataa ccctgataaa tgcttcaata 2700

atattgaaaa aggaagagta tgagtattca acatttccgt gtcgccctta ttcccttttt 2760

tgcggcattt tgccttcctg tttttgctca cccagaaacg ctggtgaaag taaaagatgc 2820

tgaagatcag ttgggtgcac gagtgggtta catcgaactg gatctcaaca gcggtaagat 2880

ccttgagagt tttcgccccg aagaacgttt tccaatgatg agcactttta aagttctgct 2940

atgtggcgcg gtattatccc gtattgacgc cgggcaagag caactcggtc gccgcataca 3000

ctattctcag aatgacttgg ttgagtactc accagtcaca gaaaagcatc ttacggatgg 3060

catgacagta agagaattat gcagtgctgc cataaccatg agtgataaca ctgcggccaa 3120

cttacttctg acaacgatcg gaggaccgaa ggagctaacc gcttttttgc acaacatggg 3180

ggatcatgta actcgccttg atcgttggga accggagctg aatgaagcca taccaaacga 3240

cgagcgtgac accacgatgc ctgtagcaat ggcaacaacg ttgcgcaaac tattaactgg 3300

cgaactactt actctagctt cccggcaaca attaatagac tggatggagg cggataaagt 3360

tgcaggacca cttctgcgct cggcccttcc ggctggctgg tttattgctg ataaatctgg 3420

agccggtgag cgtgggtctc gcggtatcat tgcagcactg gggccagatg gtaagccctc 3480

ccgtatcgta gttatctaca cgacggggag tcaggcaact atggatgaac gaaatagaca 3540

gatcgctgag ataggtgcct cactgattaa gcattggtaa ctgtcagacc aagtttactc 3600

atatatactt tagattgatt taaaacttca tttttaattt aaaaggatct aggtgaagat 3660

cctttttgat aatctcatga ccaaaatccc ttaacgtgag ttttcgttcc actgagcgtc 3720

agaccccgta gaaaagatca aaggatcttc ttgagatcct ttttttctgc gcgtaatctg 3780

ctgcttgcaa acaaaaaaac caccgctacc agcggtggtt tgtttgccgg atcaagagct 3840

accaactctt tttccgaagg taactggctt cagcagagcg cagataccaa atactgttct 3900

tctagtgtag ccgtagttag gccaccactt caagaactct gtagcaccgc ctacatacct 3960

cgctctgcta atcctgttac cagtggctgc tgccagtggc gataagtcgt gtcttaccgg 4020

gttggactca agacgatagt taccggataa ggcgcagcgg tcgggctgaa cggggggttc 4080

gtgcacacag cccagcttgg agcgaacgac ctacaccgaa ctgagatacc tacagcgtga 4140

gctatgagaa agcgccacgc ttcccgaagg gagaaaggcg gacaggtatc cggtaagcgg 4200

cagggtcgga acaggagagc gcacgaggga gcttccaggg ggaaacgcct ggtatcttta 4260

tagtcctgtc gggtttcgcc acctctgact tgagcgtcga tttttgtgat gctcgtcagg 4320

ggggcggagc ctatggaaaa acgccagcaa cgcggccttt ttacggttcc tggccttttg 4380

ctggcctttt gctcacatgt tctttcctgc gttatcccct gattctgtgg ataaccgtat 4440

taccgccttt gagtgagctg ataccgctcg ccgcagccga acgaccgagc gcagcgagtc 4500

agtgagcgag gaagcggaag agcgcccaat acgcaaaccg cctctccccg cgcgttggcc 4560

gattcattaa tgcagctggc acgacaggtt tcccgactgg aaagcgggca gtgagcgcaa 4620

cgcaattaat gtgagttagc tcactcatta ggcaccccag gctttacact ttatgcttcc 4680

ggctcgtatg ttgtgtggaa ttgtgagcgg ataacaattt cacacaggaa acagctatga 4740

ccatgattac gccagattta attaaggcct taattagg 4778

<210> 71
<211> 4778
<212> DNA
<213> Artificial Sequence

<220>
<223> Description of Artificial Sequence: Synthetic
      polynucleotide

<400> 71
ctgcgcgctc gctcgctcac tgaggccgcc cgggcaaagc ccgggcgtcg ggcgaccttt 60

ggtcgcccgg cctcagtgag cgagcgagcg cgcagagagg gagtgtagcc atgctctagg 120

aagatcaatt caattcacgc gtcgacattg attattgact agttattaat agtaatcaat 180

tacggggtca ttagttcata gcccatatat ggagttccgc gttacataac ttacggtaaa 240

tggcccgcct ggctgaccgc ccaacgaccc ccgcccattg acgtcaataa tgacgtatgt 300

tcccatagta acgccaatag ggactttcca ttgacgtcaa tgggtggagt atttacggta 360

aactgcccac ttggcagtac atcaagtgta tcatatgcca agtacgcccc ctattgacgt 420

caatgacggt aaatggcccg cctggcatta tgcccagtac atgaccttat gggactttcc 480

tacttggcag tacatctacg tattagtcat cgctattacc atgtcgaggc cacgttctgc 540

ttcactctcc ccatctcccc cccctcccca cccccaattt tgtatttatt tattttttaa 600

ttattttgtg cagcgatggg ggcggggggg gggggcgcgc gccaggcggg gcggggcggg 660

gcgaggggcg gggcggggcg aggcggagag gtgcggcggc agccaatcag agcggcgcgc 720

tccgaaagtt tccttttatg gcgaggcggc ggcggcggcg gccctataaa aagcgaagcg 780

cgcggcgggc gggagcaagc ttgaactgaa aaaccagaaa gttaactggt aagtttagtc 840

tttttgtctt ttatttcagg tcccggatcc ggtggtggtg caaatcaaag aactgctcct 900

cagtggatgt tgcctttact tctaggcctg tacggaagtg ttacttctgc tctaaaagct 960

gcggaattgt acccgcggcc gatccaccgg tatgtggact ctcgggcgac gcgccgtagc 1020

cggtctcctg gcttccccaa gccctgcaca ggcacagacc ctcacacggg tcccacggcc 1080

agctgaattg gccccactct gcggtcgccg gggcctgcga accgatatag acgcaacgtg 1140

cactccccga cgagcaagtt ccaaccagag agggctcaac cagatttgga acgtcaagaa 1200

gcagagtgtc tatttgatga atctgagaaa atccgggacc cttgggcacc ccggatctct 1260

tgatgaaact acctatgaaa ggctggccga ggagacgctg gactctctgg cagagttctt 1320

cgaggatctg gcagacaagc catacacgtt tgaggactat gatgtctctt ttggatctgg 1380

cgtcctgacg gttaaactgg gaggggatct cggaacctac gttatcaaca aacagactcc 1440

caataagcag atctggttgt ctagtccatc cagtggtcct aaacgctatg actggacggg 1500

caagaactgg gtgtactccc acgacggagt tagccttcat gaactgctgg ccgcagagct 1560

gactaaagct cttaagacta aactggatct ctctagcctg gcctactccg ggaaagatgc 1620

ctagctcgag gacggggtga actacgcctg aggatccgat ctttttccct ctgccaaaaa 1680

ttatggggac atcatgaagc cccttgagca tctgacttct ggctaataaa ggaaatttat 1740

tttcattgca atagtgtgtt ggaatttttt gtgtctctca ctcggcctag gtagataagt 1800

agcatggcgg gttaatcatt aactacaagg aacccctagt gatggagttg gccactccct 1860

ctctgcgcgc tcgctcgctc actgaggccg ggcgaccaaa ggtcgcccga cgcccgggct 1920

ttgcccgggc ggcctcagtg agcgagcgag cgcgcagcct taattaacct aattcactgg 1980

ccgtcgtttt acaacgtcgt gactgggaaa accctggcgt tacccaactt aatcgccttg 2040

cagcacatcc ccctttcgcc agctggcgta atagcgaaga ggcccgcacc gatcgccctt 2100

cccaacagtt gcgcagcctg aatggcgaat gggacgcgcc ctgtagcggc gcattaagcg 2160

cggcgggtgt ggtggttacg cgcagcgtga ccgctacact tgccagcgcc ctagcgcccg 2220

ctcctttcgc tttcttccct tcctttctcg ccacgttcgc cggctttccc cgtcaagctc 2280

taaatcgggg gctcccttta gggttccgat ttagtgcttt acggcacctc gaccccaaaa 2340

aacttgatta gggtgatggt tcacgtagtg ggccatcgcc ctgatagacg gtttttcgcc 2400

ctttgacgtt ggagtccacg ttctttaata gtggactctt gttccaaact ggaacaacac 2460

tcaaccctat ctcggtctat tcttttgatt tataagggat tttgccgatt tcggcctatt 2520

ggttaaaaaa tgagctgatt taacaaaaat ttaacgcgaa ttttaacaaa atattaacgc 2580

ttacaattta ggtggcactt ttcggggaaa tgtgcgcgga acccctattt gtttattttt 2640

ctaaatacat tcaaatatgt atccgctcat gagacaataa ccctgataaa tgcttcaata 2700

atattgaaaa aggaagagta tgagtattca acatttccgt gtcgccctta ttcccttttt 2760

tgcggcattt tgccttcctg tttttgctca cccagaaacg ctggtgaaag taaaagatgc 2820

tgaagatcag ttgggtgcac gagtgggtta catcgaactg gatctcaaca gcggtaagat 2880

ccttgagagt tttcgccccg aagaacgttt tccaatgatg agcactttta aagttctgct 2940

atgtggcgcg gtattatccc gtattgacgc cgggcaagag caactcggtc gccgcataca 3000

ctattctcag aatgacttgg ttgagtactc accagtcaca gaaaagcatc ttacggatgg 3060

catgacagta agagaattat gcagtgctgc cataaccatg agtgataaca ctgcggccaa 3120

cttacttctg acaacgatcg gaggaccgaa ggagctaacc gcttttttgc acaacatggg 3180

ggatcatgta actcgccttg atcgttggga accggagctg aatgaagcca taccaaacga 3240

cgagcgtgac accacgatgc ctgtagcaat ggcaacaacg ttgcgcaaac tattaactgg 3300

cgaactactt actctagctt cccggcaaca attaatagac tggatggagg cggataaagt 3360

tgcaggacca cttctgcgct cggcccttcc ggctggctgg tttattgctg ataaatctgg 3420

agccggtgag cgtgggtctc gcggtatcat tgcagcactg gggccagatg gtaagccctc 3480

ccgtatcgta gttatctaca cgacggggag tcaggcaact atggatgaac gaaatagaca 3540

gatcgctgag ataggtgcct cactgattaa gcattggtaa ctgtcagacc aagtttactc 3600

atatatactt tagattgatt taaaacttca tttttaattt aaaaggatct aggtgaagat 3660

cctttttgat aatctcatga ccaaaatccc ttaacgtgag ttttcgttcc actgagcgtc 3720

agaccccgta gaaaagatca aaggatcttc ttgagatcct ttttttctgc gcgtaatctg 3780

ctgcttgcaa acaaaaaaac caccgctacc agcggtggtt tgtttgccgg atcaagagct 3840

accaactctt tttccgaagg taactggctt cagcagagcg cagataccaa atactgttct 3900

tctagtgtag ccgtagttag gccaccactt caagaactct gtagcaccgc ctacatacct 3960

cgctctgcta atcctgttac cagtggctgc tgccagtggc gataagtcgt gtcttaccgg 4020

gttggactca agacgatagt taccggataa ggcgcagcgg tcgggctgaa cggggggttc 4080

gtgcacacag cccagcttgg agcgaacgac ctacaccgaa ctgagatacc tacagcgtga 4140

gctatgagaa agcgccacgc ttcccgaagg gagaaaggcg gacaggtatc cggtaagcgg 4200

cagggtcgga acaggagagc gcacgaggga gcttccaggg ggaaacgcct ggtatcttta 4260

tagtcctgtc gggtttcgcc acctctgact tgagcgtcga tttttgtgat gctcgtcagg 4320

ggggcggagc ctatggaaaa acgccagcaa cgcggccttt ttacggttcc tggccttttg 4380

ctggcctttt gctcacatgt tctttcctgc gttatcccct gattctgtgg ataaccgtat 4440

taccgccttt gagtgagctg ataccgctcg ccgcagccga acgaccgagc gcagcgagtc 4500

agtgagcgag gaagcggaag agcgcccaat acgcaaaccg cctctccccg cgcgttggcc 4560

gattcattaa tgcagctggc acgacaggtt tcccgactgg aaagcgggca gtgagcgcaa 4620

cgcaattaat gtgagttagc tcactcatta ggcaccccag gctttacact ttatgcttcc 4680

ggctcgtatg ttgtgtggaa ttgtgagcgg ataacaattt cacacaggaa acagctatga 4740

ccatgattac gccagattta attaaggcct taattagg 4778

<210> 72
<211> 4778
<212> DNA
<213> Artificial Sequence

<220>
<223> Description of Artificial Sequence: Synthetic
      polynucleotide

<400> 72
ctgcgcgctc gctcgctcac tgaggccgcc cgggcaaagc ccgggcgtcg ggcgaccttt 60

ggtcgcccgg cctcagtgag cgagcgagcg cgcagagagg gagtgtagcc atgctctagg 120

aagatcaatt caattcacgc gtcgacattg attattgact agttattaat agtaatcaat 180

tacggggtca ttagttcata gcccatatat ggagttccgc gttacataac ttacggtaaa 240

tggcccgcct ggctgaccgc ccaacgaccc ccgcccattg acgtcaataa tgacgtatgt 300

tcccatagta acgccaatag ggactttcca ttgacgtcaa tgggtggagt atttacggta 360

aactgcccac ttggcagtac atcaagtgta tcatatgcca agtacgcccc ctattgacgt 420

caatgacggt aaatggcccg cctggcatta tgcccagtac atgaccttat gggactttcc 480

tacttggcag tacatctacg tattagtcat cgctattacc atgtcgaggc cacgttctgc 540

ttcactctcc ccatctcccc cccctcccca cccccaattt tgtatttatt tattttttaa 600

ttattttgtg cagcgatggg ggcggggggg gggggcgcgc gccaggcggg gcggggcggg 660

gcgaggggcg gggcggggcg aggcggagag gtgcggcggc agccaatcag agcggcgcgc 720

tccgaaagtt tccttttatg gcgaggcggc ggcggcggcg gccctataaa aagcgaagcg 780

cgcggcgggc gggagcaagc ttgaactgaa aaaccagaaa gttaactggt aagtttagtc 840

tttttgtctt ttatttcagg tcccggatcc ggtggtggtg caaatcaaag aactgctcct 900

cagtggatgt tgcctttact tctaggcctg tacggaagtg ttacttctgc tctaaaagct 960

gcggaattgt acccgcggcc gatccaccgg tatgtggact ctggggcgac gcgccgtggc 1020

cgggctcctg gcatccccat cccctgcgca ggcgcaaacc ctcacacggg ttccacggcc 1080

agctgaactg gccccactgt gcggccggcg gggcctgaga acagatattg acgcaacgtg 1140

cactcccaga agagccagca gcaaccagag ggggttgaat cagatctgga acgtcaagaa 1200

gcagtcagtg tacctgatga atctccgaaa aagcgggacc cttggtcacc ccggatctct 1260

cgatgaaacc acatatgaac ggctggccga ggaaacgctg gactccctgg ccgagttctt 1320

cgaggacctg gcagacaagc cgtacacctt cgaagactat gatgtttcat tcggaagtgg 1380

cgtgctgacg gtcaaactgg gaggcgatct gggaacctac gttataaaca aacagactcc 1440

caataaacag atctggttgt ctagtccatc cagcggtcct aaacgctatg actggacggg 1500

caagaactgg gtctactccc acgacggagt tagccttcac gaactgttgg ccgcagagct 1560

gacaaaggcg cttaagacga aactggatct ctctagcctg gcctactccg gcaaagatgc 1620

ctagctcgag gacggggtga actacgcctg aggatccgat ctttttccct ctgccaaaaa 1680

ttatggggac atcatgaagc cccttgagca tctgacttct ggctaataaa ggaaatttat 1740

tttcattgca atagtgtgtt ggaatttttt gtgtctctca ctcggcctag gtagataagt 1800

agcatggcgg gttaatcatt aactacaagg aacccctagt gatggagttg gccactccct 1860

ctctgcgcgc tcgctcgctc actgaggccg ggcgaccaaa ggtcgcccga cgcccgggct 1920

ttgcccgggc ggcctcagtg agcgagcgag cgcgcagcct taattaacct aattcactgg 1980

ccgtcgtttt acaacgtcgt gactgggaaa accctggcgt tacccaactt aatcgccttg 2040

cagcacatcc ccctttcgcc agctggcgta atagcgaaga ggcccgcacc gatcgccctt 2100

cccaacagtt gcgcagcctg aatggcgaat gggacgcgcc ctgtagcggc gcattaagcg 2160

cggcgggtgt ggtggttacg cgcagcgtga ccgctacact tgccagcgcc ctagcgcccg 2220

ctcctttcgc tttcttccct tcctttctcg ccacgttcgc cggctttccc cgtcaagctc 2280

taaatcgggg gctcccttta gggttccgat ttagtgcttt acggcacctc gaccccaaaa 2340

aacttgatta gggtgatggt tcacgtagtg ggccatcgcc ctgatagacg gtttttcgcc 2400

ctttgacgtt ggagtccacg ttctttaata gtggactctt gttccaaact ggaacaacac 2460

tcaaccctat ctcggtctat tcttttgatt tataagggat tttgccgatt tcggcctatt 2520

ggttaaaaaa tgagctgatt taacaaaaat ttaacgcgaa ttttaacaaa atattaacgc 2580

ttacaattta ggtggcactt ttcggggaaa tgtgcgcgga acccctattt gtttattttt 2640

ctaaatacat tcaaatatgt atccgctcat gagacaataa ccctgataaa tgcttcaata 2700

atattgaaaa aggaagagta tgagtattca acatttccgt gtcgccctta ttcccttttt 2760

tgcggcattt tgccttcctg tttttgctca cccagaaacg ctggtgaaag taaaagatgc 2820

tgaagatcag ttgggtgcac gagtgggtta catcgaactg gatctcaaca gcggtaagat 2880

ccttgagagt tttcgccccg aagaacgttt tccaatgatg agcactttta aagttctgct 2940

atgtggcgcg gtattatccc gtattgacgc cgggcaagag caactcggtc gccgcataca 3000

ctattctcag aatgacttgg ttgagtactc accagtcaca gaaaagcatc ttacggatgg 3060

catgacagta agagaattat gcagtgctgc cataaccatg agtgataaca ctgcggccaa 3120

cttacttctg acaacgatcg gaggaccgaa ggagctaacc gcttttttgc acaacatggg 3180

ggatcatgta actcgccttg atcgttggga accggagctg aatgaagcca taccaaacga 3240

cgagcgtgac accacgatgc ctgtagcaat ggcaacaacg ttgcgcaaac tattaactgg 3300

cgaactactt actctagctt cccggcaaca attaatagac tggatggagg cggataaagt 3360

tgcaggacca cttctgcgct cggcccttcc ggctggctgg tttattgctg ataaatctgg 3420

agccggtgag cgtgggtctc gcggtatcat tgcagcactg gggccagatg gtaagccctc 3480

ccgtatcgta gttatctaca cgacggggag tcaggcaact atggatgaac gaaatagaca 3540

gatcgctgag ataggtgcct cactgattaa gcattggtaa ctgtcagacc aagtttactc 3600

atatatactt tagattgatt taaaacttca tttttaattt aaaaggatct aggtgaagat 3660

cctttttgat aatctcatga ccaaaatccc ttaacgtgag ttttcgttcc actgagcgtc 3720

agaccccgta gaaaagatca aaggatcttc ttgagatcct ttttttctgc gcgtaatctg 3780

ctgcttgcaa acaaaaaaac caccgctacc agcggtggtt tgtttgccgg atcaagagct 3840

accaactctt tttccgaagg taactggctt cagcagagcg cagataccaa atactgttct 3900

tctagtgtag ccgtagttag gccaccactt caagaactct gtagcaccgc ctacatacct 3960

cgctctgcta atcctgttac cagtggctgc tgccagtggc gataagtcgt gtcttaccgg 4020

gttggactca agacgatagt taccggataa ggcgcagcgg tcgggctgaa cggggggttc 4080

gtgcacacag cccagcttgg agcgaacgac ctacaccgaa ctgagatacc tacagcgtga 4140

gctatgagaa agcgccacgc ttcccgaagg gagaaaggcg gacaggtatc cggtaagcgg 4200

cagggtcgga acaggagagc gcacgaggga gcttccaggg ggaaacgcct ggtatcttta 4260

tagtcctgtc gggtttcgcc acctctgact tgagcgtcga tttttgtgat gctcgtcagg 4320

ggggcggagc ctatggaaaa acgccagcaa cgcggccttt ttacggttcc tggccttttg 4380

ctggcctttt gctcacatgt tctttcctgc gttatcccct gattctgtgg ataaccgtat 4440

taccgccttt gagtgagctg ataccgctcg ccgcagccga acgaccgagc gcagcgagtc 4500

agtgagcgag gaagcggaag agcgcccaat acgcaaaccg cctctccccg cgcgttggcc 4560

gattcattaa tgcagctggc acgacaggtt tcccgactgg aaagcgggca gtgagcgcaa 4620

cgcaattaat gtgagttagc tcactcatta ggcaccccag gctttacact ttatgcttcc 4680

ggctcgtatg ttgtgtggaa ttgtgagcgg ataacaattt cacacaggaa acagctatga 4740

ccatgattac gccagattta attaaggcct taattagg 4778

<210> 73
<211> 4854
<212> DNA
<213> Artificial Sequence

<220>
<223> Description of Artificial Sequence: Synthetic
      polynucleotide

<400> 73
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtcgac attgattatt gactagttat taatagtaat 240

caattacggg gtcattagtt catagcccat atatggagtt ccgcgttaca taacttacgg 300

taaatggccc gcctggctga ccgcccaacg acccccgccc attgacgtca ataatgacgt 360

atgttcccat agtaacgcca atagggactt tccattgacg tcaatgggtg gagtatttac 420

ggtaaactgc ccacttggca gtacatcaag tgtatcatat gccaagtacg ccccctattg 480

acgtcaatga cggtaaatgg cccgcctggc attatgccca gtacatgacc ttatgggact 540

ttcctacttg gcagtacatc tacgtattag tcatcgctat taccatggtg atgcggtttt 600

ggcagtacat caatgggcgt ggatagcggt ttgactcacg gggatttcca agtctccacc 660

ccattgacgt caatgggagt ttgttttggc accaaaatca acgggacttt ccaaaatgtc 720

gtaacaactc cgccccattg acgcaaatgg gcggtaggcg tgtacggtgg gaggtctata 780

taagcagagc tctctggcta actagagaac ccactgctta ctggcttatc gaaattaata 840

cgactcacta tagggagacc caagctggct agcgtttaaa cttcgggagc aagcttgaac 900

tgaaaaacca gaaagttaac tggtaagttt agtctttttg tcttttattt caggtcccgg 960

atccggtggt ggtgcaaatc aaagaactgc tcctcagtgg atgttgcctt tacttctagg 1020

cctgtacgga agtgttactt ctgctctaaa agctgcggaa ttgtacccgc ggccgatcca 1080

ccggtatgtg gactctcggg cgccgcgcag tagccggcct cctggcgtca cccagcccag 1140

cccaggccca gaccctcacc cgggtcccgc ggccggcaga gttggcccca ctctgcggcc 1200

gccgtggcct gcgcaccgac atcgatgcga cctgcacgcc ccgccgcgca agttcgaacc 1260

aacgtggcct caaccagatt tggaatgtca aaaagcagag tgtctatttg atgaatttga 1320

ggaaatctgg aactttgggc cacccaggct ctctagatga gaccacctat gaaagactag 1380

cagaggaaac gctggactct ttagcagagt tttttgaaga ccttgcagac aagccataca 1440

cgtttgagga ctatgatgtc tcctttggga gtggtgtctt aactgtcaaa ctgggtggag 1500

atctaggaac ctatgtgatc aacaagcaga cgccaaacaa gcaaatctgg ctatcttctc 1560

catccagtgg acctaagcgt tatgactgga ctgggaaaaa ctgggtgtac tcccacgacg 1620

gcgtgtccct ccatgagctg ctggccgcag agctcactaa agccttaaaa accaaactgg 1680

acttgtcttc cttggcctat tccggaaaag atgcttgagc ggccctagcg tttaaacggg 1740

ccctctagag tcgacccggg cggcctcgag gacggggtga actacgcctg aggatccgat 1800

ctttttccct ctgccaaaaa ttatggggac atcatgaagc cccttgagca tctgacttct 1860

ggctaataaa ggaaatttat tttcattgca atagtgtgtt ggaatttttt gtgtctctca 1920

ctcggcctag gtagataagt agcatggcgg gttaatcatt aactacaagg aacccctagt 1980

gatggagttg gccactccct ctctgcgcgc tcgctcgctc actgaggccg ggcgaccaaa 2040

ggtcgcccga cgcccgggct ttgcccgggc ggcctcagtg agcgagcgag cgcgcagcct 2100

taattaacct aattcactgg ccgtcgtttt acaacgtcgt gactgggaaa accctggcgt 2160

tacccaactt aatcgccttg cagcacatcc ccctttcgcc agctggcgta atagcgaaga 2220

ggcccgcacc gatcgccctt cccaacagtt gcgcagcctg aatggcgaat gggacgcgcc 2280

ctgtagcggc gcattaagcg cggcgggtgt ggtggttacg cgcagcgtga ccgctacact 2340

tgccagcgcc ctagcgcccg ctcctttcgc tttcttccct tcctttctcg ccacgttcgc 2400

cggctttccc cgtcaagctc taaatcgggg gctcccttta gggttccgat ttagtgcttt 2460

acggcacctc gaccccaaaa aacttgatta gggtgatggt tcacgtagtg ggccatcgcc 2520

ctgatagacg gtttttcgcc ctttgacgtt ggagtccacg ttctttaata gtggactctt 2580

gttccaaact ggaacaacac tcaaccctat ctcggtctat tcttttgatt tataagggat 2640

tttgccgatt tcggcctatt ggttaaaaaa tgagctgatt taacaaaaat ttaacgcgaa 2700

ttttaacaaa atattaacgc ttacaattta ggtggcactt ttcggggaaa tgtgcgcgga 2760

acccctattt gtttattttt ctaaatacat tcaaatatgt atccgctcat gagacaataa 2820

ccctgataaa tgcttcaata atattgaaaa aggaagagta tgagtattca acatttccgt 2880

gtcgccctta ttcccttttt tgcggcattt tgccttcctg tttttgctca cccagaaacg 2940

ctggtgaaag taaaagatgc tgaagatcag ttgggtgcac gagtgggtta catcgaactg 3000

gatctcaaca gcggtaagat ccttgagagt tttcgccccg aagaacgttt tccaatgatg 3060

agcactttta aagttctgct atgtggcgcg gtattatccc gtattgacgc cgggcaagag 3120

caactcggtc gccgcataca ctattctcag aatgacttgg ttgagtactc accagtcaca 3180

gaaaagcatc ttacggatgg catgacagta agagaattat gcagtgctgc cataaccatg 3240

agtgataaca ctgcggccaa cttacttctg acaacgatcg gaggaccgaa ggagctaacc 3300

gcttttttgc acaacatggg ggatcatgta actcgccttg atcgttggga accggagctg 3360

aatgaagcca taccaaacga cgagcgtgac accacgatgc ctgtagcaat ggcaacaacg 3420

ttgcgcaaac tattaactgg cgaactactt actctagctt cccggcaaca attaatagac 3480

tggatggagg cggataaagt tgcaggacca cttctgcgct cggcccttcc ggctggctgg 3540

tttattgctg ataaatctgg agccggtgag cgtgggtctc gcggtatcat tgcagcactg 3600

gggccagatg gtaagccctc ccgtatcgta gttatctaca cgacggggag tcaggcaact 3660

atggatgaac gaaatagaca gatcgctgag ataggtgcct cactgattaa gcattggtaa 3720

ctgtcagacc aagtttactc atatatactt tagattgatt taaaacttca tttttaattt 3780

aaaaggatct aggtgaagat cctttttgat aatctcatga ccaaaatccc ttaacgtgag 3840

ttttcgttcc actgagcgtc agaccccgta gaaaagatca aaggatcttc ttgagatcct 3900

ttttttctgc gcgtaatctg ctgcttgcaa acaaaaaaac caccgctacc agcggtggtt 3960

tgtttgccgg atcaagagct accaactctt tttccgaagg taactggctt cagcagagcg 4020

cagataccaa atactgttct tctagtgtag ccgtagttag gccaccactt caagaactct 4080

gtagcaccgc ctacatacct cgctctgcta atcctgttac cagtggctgc tgccagtggc 4140

gataagtcgt gtcttaccgg gttggactca agacgatagt taccggataa ggcgcagcgg 4200

tcgggctgaa cggggggttc gtgcacacag cccagcttgg agcgaacgac ctacaccgaa 4260

ctgagatacc tacagcgtga gctatgagaa agcgccacgc ttcccgaagg gagaaaggcg 4320

gacaggtatc cggtaagcgg cagggtcgga acaggagagc gcacgaggga gcttccaggg 4380

ggaaacgcct ggtatcttta tagtcctgtc gggtttcgcc acctctgact tgagcgtcga 4440

tttttgtgat gctcgtcagg ggggcggagc ctatggaaaa acgccagcaa cgcggccttt 4500

ttacggttcc tggccttttg ctggcctttt gctcacatgt tctttcctgc gttatcccct 4560

gattctgtgg ataaccgtat taccgccttt gagtgagctg ataccgctcg ccgcagccga 4620

acgaccgagc gcagcgagtc agtgagcgag gaagcggaag agcgcccaat acgcaaaccg 4680

cctctccccg cgcgttggcc gattcattaa tgcagctggc acgacaggtt tcccgactgg 4740

aaagcgggca gtgagcgcaa cgcaattaat gtgagttagc tcactcatta ggcaccccag 4800

gctttacact ttatgcttcc ggctcgtatg ttgtgtggaa ttgtgagcgg ataa 4854

<210> 74
<211> 4808
<212> DNA
<213> Artificial Sequence

<220>
<223> Description of Artificial Sequence: Synthetic
      polynucleotide

<400> 74
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtcgac attgattatt gactagttat taatagtaat 240

caattacggg gtcattagtt catagcccat atatggagtt ccgcgttaca taacttacgg 300

taaatggccc gcctggctga ccgcccaacg acccccgccc attgacgtca ataatgacgt 360

atgttcccat agtaacgcca atagggactt tccattgacg tcaatgggtg gagtatttac 420

ggtaaactgc ccacttggca gtacatcaag tgtatcatat gccaagtacg ccccctattg 480

acgtcaatga cggtaaatgg cccgcctggc attatgccca gtacatgacc ttatgggact 540

ttcctacttg gcagtacatc tacgtattag tcatcgctat taccatggtg atgcggtttt 600

ggcagtacat caatgggcgt ggatagcggt ttgactcacg gggatttcca agtctccacc 660

ccattgacgt caatgggagt ttgttttggc accaaaatca acgggacttt ccaaaatgtc 720

gtaacaactc cgccccattg acgcaaatgg gcggtaggcg tgtacggtgg gaggtctata 780

taagcagagc tctctggcta actagagaac ccactgctta ctggcttatc gaaattaata 840

cgactcacta tagggagacc caagctggct agcgtttaaa cttcgggagc aagcttgaac 900

tgaaaaacca gaaagttaac tggtaagttt agtctttttg tcttttattt caggtcccgg 960

atccggtggt ggtgcaaatc aaagaactgc tcctcagtgg atgttgcctt tacttctagg 1020

cctgtacgga agtgttactt ctgctctaaa agctgcggaa ttgtacccgc ggccgatcca 1080

ccggtatgtg gacactcggg cgacgcgccg tggctgggct gcttgcttcc ccaagtcccg 1140

cacaggcgca aaccctcaca agggtcccac ggccagctga attggccccg ctgtgcggtc 1200

ggagggggct gcgaacagat atagacgcaa cgtgcacccc cagaagagca agcagcaacc 1260

agagaggcct caatcagatt tggaacgtca agaaacagag tgtctatctg atgaacctcc 1320

gcaaatcagg gacccttggt catccgggat ctcttgatga aacgacctat gaacggctgg 1380

ctgaggagac gcttgactcc ctggcagagt tcttcgagga tctggctgat aagccataca 1440

cgttcgagga ctatgatgtt tcttttggtt ctggcgtcct gacggtgaaa ctcggagggg 1500

atctgggcac ttacgttatc aacaaacaga ctcccaataa acagatttgg ttgtctagtc 1560

ctagcagtgg cccaaaacgc tatgactgga cgggcaagaa ctgggtgtac tcccacgatg 1620

gagttagcct gcacgaactt ctggccgctg agcttactaa agcgctgaag actaagctgg 1680

atctctccag cctcgcatac agcggcaaag acgcctagct cgaggacggg gtgaactacg 1740

cctgaggatc cgatcttttt ccctctgcca aaaattatgg ggacatcatg aagccccttg 1800

agcatctgac ttctggctaa taaaggaaat ttattttcat tgcaatagtg tgttggaatt 1860

ttttgtgtct ctcactcggc ctaggtagat aagtagcatg gcgggttaat cattaactac 1920

aaggaacccc tagtgatgga gttggccact ccctctctgc gcgctcgctc gctcactgag 1980

gccgggcgac caaaggtcgc ccgacgcccg ggctttgccc gggcggcctc agtgagcgag 2040

cgagcgcgca gccttaatta acctaattca ctggccgtcg ttttacaacg tcgtgactgg 2100

gaaaaccctg gcgttaccca acttaatcgc cttgcagcac atcccccttt cgccagctgg 2160

cgtaatagcg aagaggcccg caccgatcgc ccttcccaac agttgcgcag cctgaatggc 2220

gaatgggacg cgccctgtag cggcgcatta agcgcggcgg gtgtggtggt tacgcgcagc 2280

gtgaccgcta cacttgccag cgccctagcg cccgctcctt tcgctttctt cccttccttt 2340

ctcgccacgt tcgccggctt tccccgtcaa gctctaaatc gggggctccc tttagggttc 2400

cgatttagtg ctttacggca cctcgacccc aaaaaacttg attagggtga tggttcacgt 2460

agtgggccat cgccctgata gacggttttt cgccctttga cgttggagtc cacgttcttt 2520

aatagtggac tcttgttcca aactggaaca acactcaacc ctatctcggt ctattctttt 2580

gatttataag ggattttgcc gatttcggcc tattggttaa aaaatgagct gatttaacaa 2640

aaatttaacg cgaattttaa caaaatatta acgcttacaa tttaggtggc acttttcggg 2700

gaaatgtgcg cggaacccct atttgtttat ttttctaaat acattcaaat atgtatccgc 2760

tcatgagaca ataaccctga taaatgcttc aataatattg aaaaaggaag agtatgagta 2820

ttcaacattt ccgtgtcgcc cttattccct tttttgcggc attttgcctt cctgtttttg 2880

ctcacccaga aacgctggtg aaagtaaaag atgctgaaga tcagttgggt gcacgagtgg 2940

gttacatcga actggatctc aacagcggta agatccttga gagttttcgc cccgaagaac 3000

gttttccaat gatgagcact tttaaagttc tgctatgtgg cgcggtatta tcccgtattg 3060

acgccgggca agagcaactc ggtcgccgca tacactattc tcagaatgac ttggttgagt 3120

actcaccagt cacagaaaag catcttacgg atggcatgac agtaagagaa ttatgcagtg 3180

ctgccataac catgagtgat aacactgcgg ccaacttact tctgacaacg atcggaggac 3240

cgaaggagct aaccgctttt ttgcacaaca tgggggatca tgtaactcgc cttgatcgtt 3300

gggaaccgga gctgaatgaa gccataccaa acgacgagcg tgacaccacg atgcctgtag 3360

caatggcaac aacgttgcgc aaactattaa ctggcgaact acttactcta gcttcccggc 3420

aacaattaat agactggatg gaggcggata aagttgcagg accacttctg cgctcggccc 3480

ttccggctgg ctggtttatt gctgataaat ctggagccgg tgagcgtggg tctcgcggta 3540

tcattgcagc actggggcca gatggtaagc cctcccgtat cgtagttatc tacacgacgg 3600

ggagtcaggc aactatggat gaacgaaata gacagatcgc tgagataggt gcctcactga 3660

ttaagcattg gtaactgtca gaccaagttt actcatatat actttagatt gatttaaaac 3720

ttcattttta atttaaaagg atctaggtga agatcctttt tgataatctc atgaccaaaa 3780

tcccttaacg tgagttttcg ttccactgag cgtcagaccc cgtagaaaag atcaaaggat 3840

cttcttgaga tccttttttt ctgcgcgtaa tctgctgctt gcaaacaaaa aaaccaccgc 3900

taccagcggt ggtttgtttg ccggatcaag agctaccaac tctttttccg aaggtaactg 3960

gcttcagcag agcgcagata ccaaatactg ttcttctagt gtagccgtag ttaggccacc 4020

acttcaagaa ctctgtagca ccgcctacat acctcgctct gctaatcctg ttaccagtgg 4080

ctgctgccag tggcgataag tcgtgtctta ccgggttgga ctcaagacga tagttaccgg 4140

ataaggcgca gcggtcgggc tgaacggggg gttcgtgcac acagcccagc ttggagcgaa 4200

cgacctacac cgaactgaga tacctacagc gtgagctatg agaaagcgcc acgcttcccg 4260

aagggagaaa ggcggacagg tatccggtaa gcggcagggt cggaacagga gagcgcacga 4320

gggagcttcc agggggaaac gcctggtatc tttatagtcc tgtcgggttt cgccacctct 4380

gacttgagcg tcgatttttg tgatgctcgt caggggggcg gagcctatgg aaaaacgcca 4440

gcaacgcggc ctttttacgg ttcctggcct tttgctggcc ttttgctcac atgttctttc 4500

ctgcgttatc ccctgattct gtggataacc gtattaccgc ctttgagtga gctgataccg 4560

ctcgccgcag ccgaacgacc gagcgcagcg agtcagtgag cgaggaagcg gaagagcgcc 4620

caatacgcaa accgcctctc cccgcgcgtt ggccgattca ttaatgcagc tggcacgaca 4680

ggtttcccga ctggaaagcg ggcagtgagc gcaacgcaat taatgtgagt tagctcactc 4740

attaggcacc ccaggcttta cactttatgc ttccggctcg tatgttgtgt ggaattgtga 4800

gcggataa 4808

<210> 75
<211> 4808
<212> DNA
<213> Artificial Sequence

<220>
<223> Description of Artificial Sequence: Synthetic
      polynucleotide

<400> 75
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtcgac attgattatt gactagttat taatagtaat 240

caattacggg gtcattagtt catagcccat atatggagtt ccgcgttaca taacttacgg 300

taaatggccc gcctggctga ccgcccaacg acccccgccc attgacgtca ataatgacgt 360

atgttcccat agtaacgcca atagggactt tccattgacg tcaatgggtg gagtatttac 420

ggtaaactgc ccacttggca gtacatcaag tgtatcatat gccaagtacg ccccctattg 480

acgtcaatga cggtaaatgg cccgcctggc attatgccca gtacatgacc ttatgggact 540

ttcctacttg gcagtacatc tacgtattag tcatcgctat taccatggtg atgcggtttt 600

ggcagtacat caatgggcgt ggatagcggt ttgactcacg gggatttcca agtctccacc 660

ccattgacgt caatgggagt ttgttttggc accaaaatca acgggacttt ccaaaatgtc 720

gtaacaactc cgccccattg acgcaaatgg gcggtaggcg tgtacggtgg gaggtctata 780

taagcagagc tctctggcta actagagaac ccactgctta ctggcttatc gaaattaata 840

cgactcacta tagggagacc caagctggct agcgtttaaa cttcgggagc aagcttgaac 900

tgaaaaacca gaaagttaac tggtaagttt agtctttttg tcttttattt caggtcccgg 960

atccggtggt ggtgcaaatc aaagaactgc tcctcagtgg atgttgcctt tacttctagg 1020

cctgtacgga agtgttactt ctgctctaaa agctgcggaa ttgtacccgc ggccgatcca 1080

ccggtatgtg gactctgggt cgacgcgccg tagccggtct cttggcctcc ccatcccctg 1140

cgcaggcaca gacccttaca cgagttccac ggcccgctga actggcacca ctctgcggac 1200

gccggggcct gagaaccgat attgacgcta cgtgcactcc ccgacgagcc tcttctaatc 1260

agagggggct caaccagatc tggaacgtga agaagcagtc agtctacttg atgaatctga 1320

gaaagagcgg gacccttggg caccccgggt ctctcgacga gactacatac gagaggctgg 1380

ccgaggagac tttggactct ctcgccgaat tcttcgagga tttggcagac aagccgtaca 1440

cctttgaaga ctacgatgtc tcattcggat caggggtgct gacggttaaa ctggggggcg 1500

atctcggaac ctacgtcata aacaaacaga cacccaataa gcagatctgg ttgtctagcc 1560

catcatccgg tcctaaacgg tacgactgga ccggcaagaa ctgggtctac tcccatgacg 1620

gagtcagtct tcatgaactg cttgccgcag agctgaccaa ggctcttaag acgaaactgg 1680

atctctctag cctggcctac tccgggaagg atgcatagct cgaggacggg gtgaactacg 1740

cctgaggatc cgatcttttt ccctctgcca aaaattatgg ggacatcatg aagccccttg 1800

agcatctgac ttctggctaa taaaggaaat ttattttcat tgcaatagtg tgttggaatt 1860

ttttgtgtct ctcactcggc ctaggtagat aagtagcatg gcgggttaat cattaactac 1920

aaggaacccc tagtgatgga gttggccact ccctctctgc gcgctcgctc gctcactgag 1980

gccgggcgac caaaggtcgc ccgacgcccg ggctttgccc gggcggcctc agtgagcgag 2040

cgagcgcgca gccttaatta acctaattca ctggccgtcg ttttacaacg tcgtgactgg 2100

gaaaaccctg gcgttaccca acttaatcgc cttgcagcac atcccccttt cgccagctgg 2160

cgtaatagcg aagaggcccg caccgatcgc ccttcccaac agttgcgcag cctgaatggc 2220

gaatgggacg cgccctgtag cggcgcatta agcgcggcgg gtgtggtggt tacgcgcagc 2280

gtgaccgcta cacttgccag cgccctagcg cccgctcctt tcgctttctt cccttccttt 2340

ctcgccacgt tcgccggctt tccccgtcaa gctctaaatc gggggctccc tttagggttc 2400

cgatttagtg ctttacggca cctcgacccc aaaaaacttg attagggtga tggttcacgt 2460

agtgggccat cgccctgata gacggttttt cgccctttga cgttggagtc cacgttcttt 2520

aatagtggac tcttgttcca aactggaaca acactcaacc ctatctcggt ctattctttt 2580

gatttataag ggattttgcc gatttcggcc tattggttaa aaaatgagct gatttaacaa 2640

aaatttaacg cgaattttaa caaaatatta acgcttacaa tttaggtggc acttttcggg 2700

gaaatgtgcg cggaacccct atttgtttat ttttctaaat acattcaaat atgtatccgc 2760

tcatgagaca ataaccctga taaatgcttc aataatattg aaaaaggaag agtatgagta 2820

ttcaacattt ccgtgtcgcc cttattccct tttttgcggc attttgcctt cctgtttttg 2880

ctcacccaga aacgctggtg aaagtaaaag atgctgaaga tcagttgggt gcacgagtgg 2940

gttacatcga actggatctc aacagcggta agatccttga gagttttcgc cccgaagaac 3000

gttttccaat gatgagcact tttaaagttc tgctatgtgg cgcggtatta tcccgtattg 3060

acgccgggca agagcaactc ggtcgccgca tacactattc tcagaatgac ttggttgagt 3120

actcaccagt cacagaaaag catcttacgg atggcatgac agtaagagaa ttatgcagtg 3180

ctgccataac catgagtgat aacactgcgg ccaacttact tctgacaacg atcggaggac 3240

cgaaggagct aaccgctttt ttgcacaaca tgggggatca tgtaactcgc cttgatcgtt 3300

gggaaccgga gctgaatgaa gccataccaa acgacgagcg tgacaccacg atgcctgtag 3360

caatggcaac aacgttgcgc aaactattaa ctggcgaact acttactcta gcttcccggc 3420

aacaattaat agactggatg gaggcggata aagttgcagg accacttctg cgctcggccc 3480

ttccggctgg ctggtttatt gctgataaat ctggagccgg tgagcgtggg tctcgcggta 3540

tcattgcagc actggggcca gatggtaagc cctcccgtat cgtagttatc tacacgacgg 3600

ggagtcaggc aactatggat gaacgaaata gacagatcgc tgagataggt gcctcactga 3660

ttaagcattg gtaactgtca gaccaagttt actcatatat actttagatt gatttaaaac 3720

ttcattttta atttaaaagg atctaggtga agatcctttt tgataatctc atgaccaaaa 3780

tcccttaacg tgagttttcg ttccactgag cgtcagaccc cgtagaaaag atcaaaggat 3840

cttcttgaga tccttttttt ctgcgcgtaa tctgctgctt gcaaacaaaa aaaccaccgc 3900

taccagcggt ggtttgtttg ccggatcaag agctaccaac tctttttccg aaggtaactg 3960

gcttcagcag agcgcagata ccaaatactg ttcttctagt gtagccgtag ttaggccacc 4020

acttcaagaa ctctgtagca ccgcctacat acctcgctct gctaatcctg ttaccagtgg 4080

ctgctgccag tggcgataag tcgtgtctta ccgggttgga ctcaagacga tagttaccgg 4140

ataaggcgca gcggtcgggc tgaacggggg gttcgtgcac acagcccagc ttggagcgaa 4200

cgacctacac cgaactgaga tacctacagc gtgagctatg agaaagcgcc acgcttcccg 4260

aagggagaaa ggcggacagg tatccggtaa gcggcagggt cggaacagga gagcgcacga 4320

gggagcttcc agggggaaac gcctggtatc tttatagtcc tgtcgggttt cgccacctct 4380

gacttgagcg tcgatttttg tgatgctcgt caggggggcg gagcctatgg aaaaacgcca 4440

gcaacgcggc ctttttacgg ttcctggcct tttgctggcc ttttgctcac atgttctttc 4500

ctgcgttatc ccctgattct gtggataacc gtattaccgc ctttgagtga gctgataccg 4560

ctcgccgcag ccgaacgacc gagcgcagcg agtcagtgag cgaggaagcg gaagagcgcc 4620

caatacgcaa accgcctctc cccgcgcgtt ggccgattca ttaatgcagc tggcacgaca 4680

ggtttcccga ctggaaagcg ggcagtgagc gcaacgcaat taatgtgagt tagctcactc 4740

attaggcacc ccaggcttta cactttatgc ttccggctcg tatgttgtgt ggaattgtga 4800

gcggataa 4808

<210> 76
<211> 4808
<212> DNA
<213> Artificial Sequence

<220>
<223> Description of Artificial Sequence: Synthetic
      polynucleotide

<400> 76
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtcgac attgattatt gactagttat taatagtaat 240

caattacggg gtcattagtt catagcccat atatggagtt ccgcgttaca taacttacgg 300

taaatggccc gcctggctga ccgcccaacg acccccgccc attgacgtca ataatgacgt 360

atgttcccat agtaacgcca atagggactt tccattgacg tcaatgggtg gagtatttac 420

ggtaaactgc ccacttggca gtacatcaag tgtatcatat gccaagtacg ccccctattg 480

acgtcaatga cggtaaatgg cccgcctggc attatgccca gtacatgacc ttatgggact 540

ttcctacttg gcagtacatc tacgtattag tcatcgctat taccatggtg atgcggtttt 600

ggcagtacat caatgggcgt ggatagcggt ttgactcacg gggatttcca agtctccacc 660

ccattgacgt caatgggagt ttgttttggc accaaaatca acgggacttt ccaaaatgtc 720

gtaacaactc cgccccattg acgcaaatgg gcggtaggcg tgtacggtgg gaggtctata 780

taagcagagc tctctggcta actagagaac ccactgctta ctggcttatc gaaattaata 840

cgactcacta tagggagacc caagctggct agcgtttaaa cttcgggagc aagcttgaac 900

tgaaaaacca gaaagttaac tggtaagttt agtctttttg tcttttattt caggtcccgg 960

atccggtggt ggtgcaaatc aaagaactgc tcctcagtgg atgttgcctt tacttctagg 1020

cctgtacgga agtgttactt ctgctctaaa agctgcggaa ttgtacccgc ggccgatcca 1080

ccggtatgtg gactctcggg cgacgcgccg tagccggtct cctggcttcc ccaagccctg 1140

cacaggcaca gaccctcaca cgggtcccac ggccagctga attggcccca ctctgcggtc 1200

gccggggcct gcgaaccgat atagacgcaa cgtgcactcc ccgacgagca agttccaacc 1260

agagagggct caaccagatt tggaacgtca agaagcagag tgtctatttg atgaatctga 1320

gaaaatccgg gacccttggg caccccggat ctcttgatga aactacctat gaaaggctgg 1380

ccgaggagac gctggactct ctggcagagt tcttcgagga tctggcagac aagccataca 1440

cgtttgagga ctatgatgtc tcttttggat ctggcgtcct gacggttaaa ctgggagggg 1500

atctcggaac ctacgttatc aacaaacaga ctcccaataa gcagatctgg ttgtctagtc 1560

catccagtgg tcctaaacgc tatgactgga cgggcaagaa ctgggtgtac tcccacgacg 1620

gagttagcct tcatgaactg ctggccgcag agctgactaa agctcttaag actaaactgg 1680

atctctctag cctggcctac tccgggaaag atgcctagct cgaggacggg gtgaactacg 1740

cctgaggatc cgatcttttt ccctctgcca aaaattatgg ggacatcatg aagccccttg 1800

agcatctgac ttctggctaa taaaggaaat ttattttcat tgcaatagtg tgttggaatt 1860

ttttgtgtct ctcactcggc ctaggtagat aagtagcatg gcgggttaat cattaactac 1920

aaggaacccc tagtgatgga gttggccact ccctctctgc gcgctcgctc gctcactgag 1980

gccgggcgac caaaggtcgc ccgacgcccg ggctttgccc gggcggcctc agtgagcgag 2040

cgagcgcgca gccttaatta acctaattca ctggccgtcg ttttacaacg tcgtgactgg 2100

gaaaaccctg gcgttaccca acttaatcgc cttgcagcac atcccccttt cgccagctgg 2160

cgtaatagcg aagaggcccg caccgatcgc ccttcccaac agttgcgcag cctgaatggc 2220

gaatgggacg cgccctgtag cggcgcatta agcgcggcgg gtgtggtggt tacgcgcagc 2280

gtgaccgcta cacttgccag cgccctagcg cccgctcctt tcgctttctt cccttccttt 2340

ctcgccacgt tcgccggctt tccccgtcaa gctctaaatc gggggctccc tttagggttc 2400

cgatttagtg ctttacggca cctcgacccc aaaaaacttg attagggtga tggttcacgt 2460

agtgggccat cgccctgata gacggttttt cgccctttga cgttggagtc cacgttcttt 2520

aatagtggac tcttgttcca aactggaaca acactcaacc ctatctcggt ctattctttt 2580

gatttataag ggattttgcc gatttcggcc tattggttaa aaaatgagct gatttaacaa 2640

aaatttaacg cgaattttaa caaaatatta acgcttacaa tttaggtggc acttttcggg 2700

gaaatgtgcg cggaacccct atttgtttat ttttctaaat acattcaaat atgtatccgc 2760

tcatgagaca ataaccctga taaatgcttc aataatattg aaaaaggaag agtatgagta 2820

ttcaacattt ccgtgtcgcc cttattccct tttttgcggc attttgcctt cctgtttttg 2880

ctcacccaga aacgctggtg aaagtaaaag atgctgaaga tcagttgggt gcacgagtgg 2940

gttacatcga actggatctc aacagcggta agatccttga gagttttcgc cccgaagaac 3000

gttttccaat gatgagcact tttaaagttc tgctatgtgg cgcggtatta tcccgtattg 3060

acgccgggca agagcaactc ggtcgccgca tacactattc tcagaatgac ttggttgagt 3120

actcaccagt cacagaaaag catcttacgg atggcatgac agtaagagaa ttatgcagtg 3180

ctgccataac catgagtgat aacactgcgg ccaacttact tctgacaacg atcggaggac 3240

cgaaggagct aaccgctttt ttgcacaaca tgggggatca tgtaactcgc cttgatcgtt 3300

gggaaccgga gctgaatgaa gccataccaa acgacgagcg tgacaccacg atgcctgtag 3360

caatggcaac aacgttgcgc aaactattaa ctggcgaact acttactcta gcttcccggc 3420

aacaattaat agactggatg gaggcggata aagttgcagg accacttctg cgctcggccc 3480

ttccggctgg ctggtttatt gctgataaat ctggagccgg tgagcgtggg tctcgcggta 3540

tcattgcagc actggggcca gatggtaagc cctcccgtat cgtagttatc tacacgacgg 3600

ggagtcaggc aactatggat gaacgaaata gacagatcgc tgagataggt gcctcactga 3660

ttaagcattg gtaactgtca gaccaagttt actcatatat actttagatt gatttaaaac 3720

ttcattttta atttaaaagg atctaggtga agatcctttt tgataatctc atgaccaaaa 3780

tcccttaacg tgagttttcg ttccactgag cgtcagaccc cgtagaaaag atcaaaggat 3840

cttcttgaga tccttttttt ctgcgcgtaa tctgctgctt gcaaacaaaa aaaccaccgc 3900

taccagcggt ggtttgtttg ccggatcaag agctaccaac tctttttccg aaggtaactg 3960

gcttcagcag agcgcagata ccaaatactg ttcttctagt gtagccgtag ttaggccacc 4020

acttcaagaa ctctgtagca ccgcctacat acctcgctct gctaatcctg ttaccagtgg 4080

ctgctgccag tggcgataag tcgtgtctta ccgggttgga ctcaagacga tagttaccgg 4140

ataaggcgca gcggtcgggc tgaacggggg gttcgtgcac acagcccagc ttggagcgaa 4200

cgacctacac cgaactgaga tacctacagc gtgagctatg agaaagcgcc acgcttcccg 4260

aagggagaaa ggcggacagg tatccggtaa gcggcagggt cggaacagga gagcgcacga 4320

gggagcttcc agggggaaac gcctggtatc tttatagtcc tgtcgggttt cgccacctct 4380

gacttgagcg tcgatttttg tgatgctcgt caggggggcg gagcctatgg aaaaacgcca 4440

gcaacgcggc ctttttacgg ttcctggcct tttgctggcc ttttgctcac atgttctttc 4500

ctgcgttatc ccctgattct gtggataacc gtattaccgc ctttgagtga gctgataccg 4560

ctcgccgcag ccgaacgacc gagcgcagcg agtcagtgag cgaggaagcg gaagagcgcc 4620

caatacgcaa accgcctctc cccgcgcgtt ggccgattca ttaatgcagc tggcacgaca 4680

ggtttcccga ctggaaagcg ggcagtgagc gcaacgcaat taatgtgagt tagctcactc 4740

attaggcacc ccaggcttta cactttatgc ttccggctcg tatgttgtgt ggaattgtga 4800

gcggataa                                                          4808

<210> 77
<211> 4808
<212> DNA
<213> Artificial Sequence

<220>
<223> Description of Artificial Sequence: Synthetic
      polynucleotide

<400> 77
caatttcaca caggaaacag ctatgaccat gattacgcca gatttaatta aggccttaat      60

taggctgcgc gctcgctcgc tcactgaggc cgcccgggca aagcccgggc gtcgggcgac     120

ctttggtcgc ccggcctcag tgagcgagcg agcgcgcaga gagggagtgt agccatgctc     180

taggaagatc aattcaattc acgcgtcgac attgattatt gactagttat taatagtaat     240

caattacggg gtcattagtt catagcccat atatggagtt ccgcgttaca taacttacgg     300

taaatggccc gcctggctga ccgcccaacg acccccgccc attgacgtca ataatgacgt     360

atgttcccat agtaacgcca atagggactt tccattgacg tcaatgggtg gagtatttac     420

ggtaaactgc ccacttggca gtacatcaag tgtatcatat gccaagtacg ccccctattg     480

acgtcaatga cggtaaatgg cccgcctggc attatgccca gtacatgacc ttatgggact     540

ttcctacttg gcagtacatc tacgtattag tcatcgctat taccatggtg atgcggtttt     600

ggcagtacat caatgggcgt ggatagcggt ttgactcacg gggatttcca agtctccacc     660

ccattgacgt caatgggagt ttgttttggc accaaaatca acgggacttt ccaaaatgtc     720

gtaacaactc cgccccattg acgcaaatgg gcggtaggcg tgtacggtgg gaggtctata     780

taagcagagc tctctggcta actagagaac ccactgctta ctggcttatc gaaattaata     840

cgactcacta tagggagacc caagctggct agcgtttaaa cttcgggagc aagcttgaac     900

tgaaaaacca gaaagttaac tggtaagttt agtctttttg tcttttattt caggtcccgg     960

atccggtggt ggtgcaaatc aaagaactgc tcctcagtgg atgttgcctt tacttctagg    1020

cctgtacgga agtgttactt ctgctctaaa agctgcggaa ttgtacccgc ggccgatcca    1080

ccggtatgtg gactctgggg cgacgcgccg tggccgggct cctggcatcc ccatcccctg    1140

cgcaggcgca aaccctcaca cgggttccac ggccagctga actggcccca ctgtgcggcc    1200

ggcggggcct gagaacagat attgacgcaa cgtgcactcc cagaagagcc agcagcaacc    1260

agagggggtt gaatcagatc tggaacgtca agaagcagtc agtgtacctg atgaatctcc    1320

gaaaaagcgg gacccttggt caccccggat ctctcgatga aaccacatat gaacggctgg    1380

ccgaggaaac gctggactcc ctggccgagt tcttcgagga cctggcagac aagccgtaca    1440

ccttcgaaga ctatgatgtt tcattcggaa gtggcgtgct gacggtcaaa ctgggaggcg    1500

atctgggaac ctacgttata aacaaacaga ctcccaataa acagatctgg ttgtctagtc 1560
catccagcgg tcctaaacgc tatgactgga cgggcaagaa ctgggtctac tcccacgacg 1620
gagttagcct tcacgaactg ttggccgcag agctgacaaa ggcgcttaag acgaaactgg 1680
atctctctag cctggcctac tccggcaaag atgcctagct cgaggacggg gtgaactacg 1740
cctgaggatc cgatcttttt ccctctgcca aaaattatgg ggacatcatg aagccccttg 1800
agcatctgac ttctggctaa taaaggaaat ttattttcat tgcaatagtg tgttggaatt 1860
ttttgtgtct ctcactcggc ctaggtagat aagtagcatg gcgggttaat cattaactac 1920
aaggaacccc tagtgatgga gttggccact ccctctctgc gcgctcgctc gctcactgag 1980
gccgggcgac caaaggtcgc ccgacgcccg ggctttgccc gggcggcctc agtgagcgag 2040
cgagcgcgca gccttaatta acctaattca ctggccgtcg ttttacaacg tcgtgactgg 2100
gaaaaccctg gcgttaccca acttaatcgc cttgcagcac atcccccttt cgccagctgg 2160
cgtaatagcg aagaggcccg caccgatcgc ccttcccaac agttgcgcag cctgaatggc 2220
gaatgggacg cgccctgtag cggcgcatta agcgcggcgg gtgtggtggt tacgcgcagc 2280
gtgaccgcta cacttgccag cgccctagcg cccgctcctt tcgctttctt cccttccttt 2340
ctcgccacgt tcgccggctt tccccgtcaa gctctaaatc gggggctccc tttagggttc 2400
cgatttagtg ctttacggca cctcgacccc aaaaaacttg attagggtga tggttcacgt 2460
agtgggccat cgccctgata gacggttttt cgccctttga cgttggagtc cacgttcttt 2520
aatagtggac tcttgttcca aactggaaca acactcaacc ctatctcggt ctattctttt 2580
gatttataag ggattttgcc gatttcggcc tattggttaa aaaatgagct gatttaacaa 2640
aaatttaacg cgaattttaa caaaatatta acgcttacaa tttaggtggc acttttcggg 2700
gaaatgtgcg cggaacccct atttgtttat ttttctaaat acattcaaat atgtatccgc 2760
tcatgagaca ataaccctga taaatgcttc aataatattg aaaaaggaag agtatgagta 2820
ttcaacattt ccgtgtcgcc cttattccct tttttgcggc attttgcctt cctgtttttg 2880
ctcacccaga aacgctggtg aaagtaaaag atgctgaaga tcagttgggt gcacgagtgg 2940
gttacatcga actggatctc aacagcggta agatccttga gagttttcgc cccgaagaac 3000
gttttccaat gatgagcact tttaaagttc tgctatgtgg cgcggtatta tcccgtattg 3060
acgccgggca agagcaactc ggtcgccgca tacactattc tcagaatgac ttggttgagt 3120
actcaccagt cacagaaaag catcttacgg atggcatgac agtaagagaa ttatgcagtg 3180
ctgccataac catgagtgat aacactgcgg ccaacttact tctgacaacg atcggaggac 3240
cgaaggagct aaccgctttt ttgcacaaca tgggggatca tgtaactcgc cttgatcgtt 3300
gggaaccgga gctgaatgaa gccataccaa acgacgagcg tgacaccacg atgcctgtag 3360
caatggcaac aacgttgcgc aaactattaa ctggcgaact acttactcta gcttcccggc 3420

aacaattaat agactggatg gaggcggata aagttgcagg accacttctg cgctcggccc 3480

ttccggctgg ctggtttatt gctgataaat ctggagccgg tgagcgtggg tctcgcggta 3540

tcattgcagc actggggcca gatggtaagc cctcccgtat cgtagttatc tacacgacgg 3600

ggagtcaggc aactatggat gaacgaaata gacagatcgc tgagataggt gcctcactga 3660

ttaagcattg gtaactgtca gaccaagttt actcatatat actttagatt gatttaaaac 3720

ttcattttta atttaaaagg atctaggtga agatcctttt tgataatctc atgaccaaaa 3780

tcccttaacg tgagttttcg ttccactgag cgtcagaccc cgtagaaaag atcaaaggat 3840

cttcttgaga tccttttttt ctgcgcgtaa tctgctgctt gcaaacaaaa aaaccaccgc 3900

taccagcggt ggtttgtttg ccggatcaag agctaccaac tctttttccg aaggtaactg 3960

gcttcagcag agcgcagata ccaaatactg ttcttctagt gtagccgtag ttaggccacc 4020

acttcaagaa ctctgtagca ccgcctacat acctcgctct gctaatcctg ttaccagtgg 4080

ctgctgccag tggcgataag tcgtgtctta ccgggttgga ctcaagacga tagttaccgg 4140

ataaggcgca gcggtcgggc tgaacggggg gttcgtgcac acagcccagc ttggagcgaa 4200

cgacctacac cgaactgaga tacctacagc gtgagctatg agaaagcgcc acgcttcccg 4260

aagggagaaa ggcggacagg tatccggtaa gcggcagggt cggaacagga gagcgcacga 4320

gggagcttcc agggggaaac gcctggtatc tttatagtcc tgtcgggttt cgccacctct 4380

gacttgagcg tcgatttttg tgatgctcgt caggggggcg gagcctatgg aaaaacgcca 4440

gcaacgcggc ctttttacgg ttcctggcct tttgctggcc ttttgctcac atgttctttc 4500

ctgcgttatc ccctgattct gtggataacc gtattaccgc ctttgagtga gctgataccg 4560

ctcgccgcag ccgaacgacc gagcgcagcg agtcagtgag cgaggaagcg gaagagcgcc 4620

caatacgcaa accgcctctc cccgcgcgtt ggccgattca ttaatgcagc tggcacgaca 4680

ggtttcccga ctggaaagcg ggcagtgagc gcaacgcaat taatgtgagt tagctcactc 4740

attaggcacc ccaggcttta cactttatgc ttccggctcg tatgttgtgt ggaattgtga 4800

gcggataa 4808

<210> 78
<211> 4863
<212> DNA
<213> Artificial Sequence

<220>
<223> Description of Artificial Sequence: Synthetic
      polynucleotide

<400> 78
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtcgac attgattatt gactagttat taatagtaat 240

caattacggg gtcattagtt catagcccat atatggagtt ccgcgttaca taacttacgg 300

taaatggccc gcctggctga ccgcccaacg acccccgccc attgacgtca ataatgacgt 360

atgttcccat agtaacgcca atagggactt tccattgacg tcaatgggtg gagtatttac 420

ggtaaactgc ccacttggca gtacatcaag tgtatcatat gccaagtacg ccccctattg 480

acgtcaatga cggtaaatgg cccgcctggc attatgccca gtacatgacc ttatgggact 540

ttcctacttg gcagtacatc tacgtattag tcatcgctat taccatggtg atgcggtttt 600

ggcagtacat caatgggcgt ggatagcggt ttgactcacg gggatttcca agtctccacc 660

ccattgacgt caatgggagt ttgttttggc accaaaatca acgggacttt ccaaaatgtc 720

gtaacaactc cgccccattg acgcaaatgg gcggtaggcg tgtacggtgg gaggtctata 780

taagcagagc tctctggcta actagagaac ccactgctta ctggcttatc gaaattaata 840

cgactcacta tagggagacc caagctggct agcgtttaaa cttcgggagc aagcttgaac 900

tgaaaaacca gaaagttaac tggtaagttt agtctttttg tcttttattt caggtcccgg 960

atccggtggt ggtgcaaatc aaagaactgc tcctcagtgg atgttgcctt tacttctagg 1020

cctgtacgga agtgttactt ctgctctaaa agctgcggaa ttgtacccgc ggccgatcca 1080

ccggtgccgc caccatgtgg actctcgggc gccgcgcagt agccggcctc ctggcgtcac 1140

ccagcccagc ccaggcccag accctcaccc gggtcccgcg gccggcagag ttggccccac 1200

tctgcggccg ccgtggcctg cgcaccgaca tcgatgcgac ctgcacgccc cgccgcgcaa 1260

gttcgaacca acgtggcctc aaccagattt ggaatgtcaa aaagcagagt gtctatttga 1320

tgaatttgag gaaatctgga actttgggcc acccaggctc tctagatgag accacctatg 1380

aaagactagc agaggaaacg ctggactctt tagcagagtt ttttgaagac cttgcagaca 1440

agccatacac gtttgaggac tatgatgtct cctttgggag tggtgtctta actgtcaaac 1500

tgggtggaga tctaggaacc tatgtgatca acaagcagac gccaaacaag caaatctggc 1560

tatcttctcc atccagtgga cctaagcgtt atgactggac tgggaaaaac tgggtgtact 1620

cccacgacgg cgtgtccctc catgagctgc tggccgcaga gctcactaaa gccttaaaaa 1680

ccaaactgga cttgtcttcc ttggcctatt ccggaaaaga tgcttgagcg gccctagcgt 1740

ttaaacgggc cctctagagt cgacccgggc ggcctcgagg acggggtgaa ctacgcctga 1800

ggatccgatc tttttccctc tgccaaaaat tatggggaca tcatgaagcc ccttgagcat 1860

ctgacttctg gctaataaag gaaatttatt ttcattgcaa tagtgtgttg gaattttttg 1920

tgtctctcac tcggcctagg tagataagta gcatggcggg ttaatcatta actacaagga 1980

acccctagtg atggagttgg ccactccctc tctgcgcgct cgctcgctca ctgaggccgg 2040

gcgaccaaag gtcgcccgac gcccgggctt tgcccgggcg gcctcagtga gcgagcgagc 2100

gcgcagcctt aattaaccta attcactggc cgtcgtttta caacgtcgtg actgggaaaa 2160

ccctggcgtt acccaactta atcgccttgc agcacatccc cctttcgcca gctggcgtaa 2220

tagcgaagag gcccgcaccg atcgcccttc ccaacagttg cgcagcctga atggcgaatg 2280

ggacgcgccc tgtagcggcg cattaagcgc ggcgggtgtg gtggttacgc gcagcgtgac 2340

cgctacactt gccagcgccc tagcgcccgc tcctttcgct ttcttccctt cctttctcgc 2400

cacgttcgcc ggctttcccc gtcaagctct aaatcggggg ctccctttag ggttccgatt 2460

tagtgcttta cggcacctcg accccaaaaa acttgattag ggtgatggtt cacgtagtgg 2520

gccatcgccc tgatagacgg tttttcgccc tttgacgttg gagtccacgt tctttaatag 2580

tggactcttg ttccaaactg gaacaacact caaccctatc tcggtctatt cttttgattt 2640

ataagggatt ttgccgattt cggcctattg gttaaaaaat gagctgattt aacaaaaatt 2700

taacgcgaat tttaacaaaa tattaacgct tacaatttag gtggcacttt tcggggaaat 2760

gtgcgcggaa cccctatttg tttatttttc taaatacatt caaatatgta tccgctcatg 2820

agacaataac cctgataaat gcttcaataa tattgaaaaa ggaagagtat gagtattcaa 2880

catttccgtg tcgcccttat tccctttttt gcggcatttt gccttcctgt ttttgctcac 2940

ccagaaacgc tggtgaaagt aaaagatgct gaagatcagt tgggtgcacg agtgggttac 3000

atcgaactgg atctcaacag cggtaagatc cttgagagtt ttcgccccga agaacgtttt 3060

ccaatgatga gcacttttaa agttctgcta tgtggcgcgg tattatcccg tattgacgcc 3120

gggcaagagc aactcggtcg ccgcatacac tattctcaga atgacttggt tgagtactca 3180

ccagtcacag aaaagcatct tacggatggc atgacagtaa gagaattatg cagtgctgcc 3240

ataaccatga gtgataacac tgcggccaac ttacttctga caacgatcgg aggaccgaag 3300

gagctaaccg cttttttgca caacatgggg gatcatgtaa ctcgccttga tcgttgggaa 3360

ccggagctga atgaagccat accaaacgac gagcgtgaca ccacgatgcc tgtagcaatg 3420

gcaacaacgt tgcgcaaact attaactggc gaactactta ctctagcttc ccggcaacaa 3480

ttaatagact ggatggaggc ggataaagtt gcaggaccac ttctgcgctc ggcccttccg 3540

gctggctggt ttattgctga taaatctgga gccggtgagc gtgggtctcg cggtatcatt 3600

gcagcactgg ggccagatgg taagccctcc cgtatcgtag ttatctacac gacggggagt 3660

caggcaacta tggatgaacg aaatagacag atcgctgaga taggtgcctc actgattaag 3720

cattggtaac tgtcagacca agtttactca tatatacttt agattgattt aaaacttcat 3780

ttttaattta aaaggatcta ggtgaagatc ctttttgata atctcatgac caaaatccct 3840

taacgtgagt tttcgttcca ctgagcgtca gaccccgtag aaaagatcaa aggatcttct 3900

tgagatcctt tttttctgcg cgtaatctgc tgcttgcaaa caaaaaaacc accgctacca 3960

gcggtggttt gtttgccgga tcaagagcta ccaactcttt ttccgaaggt aactggcttc 4020

agcagagcgc agataccaaa tactgttctt ctagtgtagc cgtagttagg ccaccacttc 4080

aagaactctg tagcaccgcc tacatacctc gctctgctaa tcctgttacc agtggctgct 4140

gccagtggcg ataagtcgtg tcttaccggg ttggactcaa gacgatagtt accggataag 4200

gcgcagcggt cgggctgaac ggggggttcg tgcacacagc ccagcttgga gcgaacgacc 4260

tacaccgaac tgagatacct acagcgtgag ctatgagaaa gcgccacgct tcccgaaggg 4320

agaaaggcgg acaggtatcc ggtaagcggc agggtcggaa caggagagcg cacgagggag 4380

cttccagggg gaaacgcctg gtatctttat agtcctgtcg ggtttcgcca cctctgactt 4440

gagcgtcgat ttttgtgatg ctcgtcaggg gggcggagcc tatggaaaaa cgccagcaac 4500

gcggcctttt tacggttcct ggccttttgc tggccttttg ctcacatgtt ctttcctgcg 4560

ttatcccctg attctgtgga taaccgtatt accgcctttg agtgagctga taccgctcgc 4620

cgcagccgaa cgaccgagcg cagcgagtca gtgagcgagg aagcggaaga gcgcccaata 4680

cgcaaaccgc ctctccccgc gcgttggccg attcattaat gcagctggca cgacaggttt 4740

cccgactgga aagcgggcag tgagcgcaac gcaattaatg tgagttagct cactcattag 4800

gcaccccagg ctttacactt tatgcttccg gctcgtatgt tgtgtggaat tgtgagcgga 4860

taa 4863

<210> 79
<211> 5533
<212> DNA
<213> Artificial Sequence

<220>
<223> Description of Artificial Sequence: Synthetic
      polynucleotide

<400> 79
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtacta tagctagagc tcccaggctc aagtgatcct 240

cccgcctcag cctcctgagt agctgagatc acaggcatgc actaccacac tcggctcaca 300

tttgacatcc tctaaagcat atataaaatg tgaagaaaac tttcacaatt tgcatccctt 360

tgtaatatgt aacagaaata aaattctctt ttaaaatcta tcaacaatag gcaaggcacg 420

gtggctcacg cctgtcgtct cagcactttg tgaggcccag gcgggcagat cgtttgagcc 480

tagaagttca agaccaccct gggcaacata gcgaaacccc ctttctacaa aaaatacaaa 540

aactagctgg gtgtggtggt gcacacctgt agtcccagct acttggaagg ctgaaatggg 600

aagactgctt gagcccggga gggagaagtt gcagtaagcc aggaccacac cactgcactc 660

cagcctgggc aacagagtga gactctgtct caaacaaaca aataaatgag gcgggtggat 720

cacgaggtca gtagatcgag accatcctgg ctaacacggt gaaacccgtc tctactaaaa 780

aaaaaaaaaa atacaaaaaa ttagccaggc atggtggcgg gcgcctgtag tcccagttac 840

tcgggaggct gaggcaggag aatggcgtga aaccgggagg cagagcttgc agtgagccga 900

gatcgcacca ctgccctcca gcctgggcga cagagcgaga ctccgtctca atcaatcaat 960

caatcaataa aatctattaa caatatttat tgtgcactta acaggaacat gccctgtcca 1020

aaaaaaactt tacagggctt aactcatttt atccttacca caatcctatg aagtaggaac 1080

ttttataaaa cgcattttat aaacaaggca cagagaggtt aattaacttg ccctctggtc 1140

acacagctag gaagtgggca gagtacagat ttacacaagg catccgtctc ctggccccac 1200

atacccaact gctgtaaacc cataccggcg gccaagcagc ctcaatttgt gcatgcaccc 1260

acttcccagc aagacagcag ctcccaagtt cctcctgttt agaattttag aagcggcggg 1320

ccaccaggct gcagtctccc ttgggtcagg ggtcctggtt gcactccgtg ctttgcacaa 1380

agcaggctct ccatttttgt taaatgcacg aatagtgcta agctgggaag ttcttcctga 1440

ggtctaacct ctagctgctc ccccacagaa gagtgcctgc ggccagtggc caccaggggt 1500

cgccgcagca cccagcgctg gagggcggag cgggcggcag acccggagca gcaagtggac 1560

tctctcgaga agcttgaact gaaaaaccag aaagttaact ggtaagttta gtctttttgt 1620

cttttatttc aggtcccgga tccggtggtg gtgcaaatca aagaactgct cctcagtgga 1680

tgttgccttt acttctaggc ctgtacggaa gtgttacttc tgctctaaaa gctgcggaat 1740

tgtacccgcg gccgatccac cggtatgtgg actctcgggc gccgcgcagt agccggcctc 1800

ctggcgtcac ccagcccagc ccaggcccag accctcaccc gggtcccgcg gccggcagag 1860

ttggccccac tctgcggccg ccgtggcctg cgcaccgaca tcgatgcgac ctgcacgccc 1920

cgccgcgcaa gttcgaacca acgtggcctc aaccagattt ggaatgtcaa aaagcagagt 1980

gtctatttga tgaatttgag gaaatctgga actttgggcc acccaggctc tctagatgag 2040

accacctatg aaagactagc agaggaaacg ctggactctt tagcagagtt ttttgaagac 2100

cttgcagaca agccatacac gtttgaggac tatgatgtct cctttgggag tggtgtctta 2160

actgtcaaac tgggtggaga tctaggaacc tatgtgatca acaagcagac gccaaacaag 2220

caaatctggc tatcttctcc atccagtgga cctaagcgtt atgactggac tgggaaaaac 2280

tgggtgtact cccacgacgg cgtgtccctc catgagctgc tggccgcaga gctcactaaa 2340

gccttaaaaa ccaaactgga cttgtcttcc ttggcctatt ccggaaaaga tgcttgagcg 2400

gccctagcgt ttaaacgggc cctctagagt cgacccgggc ggcctcgagg acggggtgaa 2460

ctacgcctga ggatccgatc tttttccctc tgccaaaaat tatggggaca tcatgaagcc 2520

ccttgagcat ctgacttctg gctaataaag gaaatttatt ttcattgcaa tagtgtgttg 2580

gaattttttg tgtctctcac tcggcctagg tagataagta gcatggcggg ttaatcatta 2640

actacaagga acccctagtg atggagttgg ccactccctc tctgcgcgct cgctcgctca 2700

ctgaggccgg gcgaccaaag gtcgcccgac gcccgggctt tgcccgggcg gcctcagtga 2760

gcgagcgagc gcgcagcctt aattaaccta attcactggc cgtcgtttta caacgtcgtg 2820

actgggaaaa ccctggcgtt acccaactta atcgccttgc agcacatccc cctttcgcca 2880

gctggcgtaa tagcgaagag gcccgcaccg atcgcccttc ccaacagttg cgcagcctga 2940

atggcgaatg ggacgcgccc tgtagcggcg cattaagcgc ggcgggtgtg gtggttacgc 3000

gcagcgtgac cgctacactt gccagcgccc tagcgcccgc tcctttcgct ttcttccctt 3060

cctttctcgc cacgttcgcc ggctttcccc gtcaagctct aaatcggggg ctccctttag 3120

ggttccgatt tagtgcttta cggcacctcg accccaaaaa acttgattag ggtgatggtt 3180

cacgtagtgg gccatcgccc tgatagacgg tttttcgccc tttgacgttg gagtccacgt 3240

tctttaatag tggactcttg ttccaaactg gaacaacact caaccctatc tcggtctatt 3300

cttttgattt ataagggatt ttgccgattt cggcctattg gttaaaaaat gagctgattt 3360

aacaaaaatt taacgcgaat tttaacaaaa tattaacgct tacaatttag gtggcacttt 3420

tcggggaaat gtgcgcggaa cccctatttg tttatttttc taaatacatt caaatatgta 3480

tccgctcatg agacaataac cctgataaat gcttcaataa tattgaaaaa ggaagagtat 3540

gagtattcaa catttccgtg tcgcccttat tccctttttt gcggcatttt gccttcctgt 3600

ttttgctcac ccagaaacgc tggtgaaagt aaaagatgct gaagatcagt tgggtgcacg 3660

agtgggttac atcgaactgg atctcaacag cggtaagatc cttgagagtt ttcgccccga 3720

agaacgtttt ccaatgatga gcacttttaa agttctgcta tgtggcgcgg tattatcccg 3780

tattgacgcc gggcaagagc aactcggtcg ccgcatacac tattctcaga atgacttggt 3840

tgagtactca ccagtcacag aaaagcatct tacggatggc atgacagtaa gagaattatg 3900

cagtgctgcc ataaccatga gtgataacac tgcggccaac ttacttctga caacgatcgg 3960

aggaccgaag gagctaaccg cttttttgca caacatgggg gatcatgtaa ctcgccttga 4020

tcgttgggaa ccggagctga atgaagccat accaaacgac gagcgtgaca ccacgatgcc 4080

tgtagcaatg gcaacaacgt tgcgcaaact attaactggc gaactactta ctctagcttc 4140

ccggcaacaa ttaatagact ggatggaggc ggataaagtt gcaggaccac ttctgcgctc 4200

ggcccttccg gctggctggt ttattgctga taaatctgga gccggtgagc gtgggtctcg 4260

cggtatcatt gcagcactgg ggccagatgg taagccctcc cgtatcgtag ttatctacac 4320

gacggggagt caggcaacta tggatgaacg aaatagacag atcgctgaga taggtgcctc 4380

actgattaag cattggtaac tgtcagacca agtttactca tatatacttt agattgattt 4440

aaaacttcat ttttaattta aaaggatcta ggtgaagatc ctttttgata atctcatgac 4500

caaaatccct taacgtgagt tttcgttcca ctgagcgtca gaccccgtag aaaagatcaa 4560

aggatcttct tgagatcctt tttttctgcg cgtaatctgc tgcttgcaaa caaaaaaacc 4620

accgctacca gcggtggttt gtttgccgga tcaagagcta ccaactcttt ttccgaaggt 4680

aactggcttc agcagagcgc agataccaaa tactgttctt ctagtgtagc cgtagttagg 4740

ccaccacttc aagaactctg tagcaccgcc tacatacctc gctctgctaa tcctgttacc 4800

agtggctgct gccagtggcg ataagtcgtg tcttaccggg ttggactcaa gacgatagtt 4860

accggataag gcgcagcggt cgggctgaac ggggggttcg tgcacacagc ccagcttgga 4920

gcgaacgacc tacaccgaac tgagatacct acagcgtgag ctatgagaaa gcgccacgct 4980

tcccgaaggg agaaaggcgg acaggtatcc ggtaagcggc agggtcggaa caggagagcg 5040

cacgagggag cttccagggg gaaacgcctg gtatctttat agtcctgtcg ggtttcgcca 5100

cctctgactt gagcgtcgat ttttgtgatg ctcgtcaggg gggcggagcc tatggaaaaa 5160

cgccagcaac gcggcctttt tacggttcct ggccttttgc tggccttttg ctcacatgtt 5220

ctttcctgcg ttatcccctg attctgtgga taaccgtatt accgcctttg agtgagctga 5280

taccgctcgc cgcagccgaa cgaccgagcg cagcgagtca gtgagcgagg aagcggaaga 5340

gcgcccaata cgcaaaccgc ctctccccgc gcgttggccg attcattaat gcagctggca 5400

cgacaggttt cccgactgga aagcgggcag tgagcgcaac gcaattaatg tgagttagct 5460

cactcattag gcaccccagg ctttacactt tatgcttccg gctcgtatgt tgtgtggaat 5520

tgtgagcgga taa 5533

<210> 80
<211> 5487
<212> DNA
<213> Artificial Sequence

<220>
<223> Description of Artificial Sequence: Synthetic
      polynucleotide

<400> 80
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtacta tagctagagc tcccaggctc aagtgatcct 240

cccgcctcag cctcctgagt agctgagatc acaggcatgc actaccacac tcggctcaca 300

tttgacatcc tctaaagcat atataaaatg tgaagaaaac tttcacaatt tgcatccctt 360

tgtaatatgt aacagaaata aaattctctt ttaaaatcta tcaacaatag gcaaggcacg 420

gtggctcacg cctgtcgtct cagcactttg tgaggcccag gcgggcagat cgtttgagcc 480

tagaagttca agaccaccct gggcaacata gcgaaacccc ctttctacaa aaaatacaaa 540

aactagctgg gtgtggtggt gcacacctgt agtcccagct acttggaagg ctgaaatggg 600

aagactgctt gagcccggga gggagaagtt gcagtaagcc aggaccacac cactgcactc 660

cagcctgggc aacagagtga gactctgtct caaacaaaca aataaatgag gcgggtggat 720

cacgaggtca gtagatcgag accatcctgg ctaacacggt gaaacccgtc tctactaaaa 780

aaaaaaaaaa atacaaaaaa ttagccaggc atggtggcgg gcgcctgtag tcccagttac 840

tcgggaggct gaggcaggag aatggcgtga aaccgggagg cagagcttgc agtgagccga 900

gatcgcacca ctgccctcca gcctgggcga cagagcgaga ctccgtctca atcaatcaat 960

caatcaataa aatctattaa caatatttat tgtgcactta acaggaacat gccctgtcca 1020

aaaaaaactt tacagggctt aactcatttt atccttacca caatcctatg aagtaggaac 1080

ttttataaaa cgcattttat aaacaaggca cagagaggtt aattaacttg ccctctggtc 1140

acacagctag gaagtgggca gagtacagat ttacacaagg catccgtctc ctggccccac 1200

atacccaact gctgtaaacc cataccggcg gccaagcagc ctcaatttgt gcatgcaccc 1260

acttcccagc aagacagcag ctcccaagtt cctcctgttt agaattttag aagcggcggg 1320

ccaccaggct gcagtctccc ttgggtcagg ggtcctggtt gcactccgtg ctttgcacaa 1380

agcaggctct ccatttttgt taaatgcacg aatagtgcta agctgggaag ttcttcctga 1440

ggtctaacct ctagctgctc ccccacagaa gagtgcctgc ggccagtggc caccaggggt 1500

cgccgcagca cccagcgctg gagggcggag cgggcggcag acccggagca gcaagtggac 1560

tctctcgaga agcttgaact gaaaaaccag aaagttaact ggtaagttta gtctttttgt 1620

cttttatttc aggtcccgga tccggtggtg gtgcaaatca aagaactgct cctcagtgga 1680

tgttgccttt acttctaggc ctgtacggaa gtgttacttc tgctctaaaa gctgcggaat 1740

tgtacccgcg gccgatccac cggtatgtgg acactcgggc gacgcgccgt ggctgggctg 1800

cttgcttccc caagtcccgc acaggcgcaa accctcacaa gggtcccacg gccagctgaa 1860

ttggccccgc tgtgcggtcg gaggggcctg cgaacagata tagacgcaac gtgcaccccc 1920

agaagagcaa gcagcaacca gagaggcctc aatcagattt ggaacgtcaa gaaacagagt 1980

gtctatctga tgaacctccg caaatcaggg acccttggtc atccgggatc tcttgatgaa 2040

acgacctatg aacggctggc tgaggagacg cttgactccc tggcagagtt cttcgaggat 2100

ctggctgata agccatacac gttcgaggac tatgatgttt cttttggttc tggcgtcctg 2160

acggtgaaac tcggagggga tctgggcact tacgttatca acaaacagac tcccaataaa 2220

cagatttggt tgtctagtcc tagcagtggc ccaaaacgct atgactggac gggcaagaac 2280

tgggtgtact cccacgatgg agttagcctg cacgaacttc tggccgctga gcttactaaa 2340

gcgctgaaga ctaagctgga tctctccagc ctcgcataca gcggcaaaga cgcctagctc 2400

gaggacgggg tgaactacgc ctgaggatcc gatctttttc cctctgccaa aaattatggg 2460

gacatcatga agccccttga gcatctgact tctggctaat aaaggaaatt tattttcatt 2520

gcaatagtgt gttggaattt tttgtgtctc tcactcggcc taggtagata agtagcatgg 2580

cgggttaatc attaactaca aggaacccct agtgatggag ttggccactc cctctctgcg 2640

cgctcgctcg ctcactgagg ccgggcgacc aaaggtcgcc cgacgcccgg gctttgcccg 2700

ggcggcctca gtgagcgagc gagcgcgcag ccttaattaa cctaattcac tggccgtcgt 2760

tttacaacgt cgtgactggg aaaaccctgg cgttacccaa cttaatcgcc ttgcagcaca 2820

tccccctttc gccagctggc gtaatagcga agaggcccgc accgatcgcc cttcccaaca 2880

gttgcgcagc ctgaatggcg aatgggacgc gccctgtagc ggcgcattaa gcgcggcggg 2940

tgtggtggtt acgcgcagcg tgaccgctac acttgccagc gccctagcgc ccgctccttt 3000

cgctttcttc ccttcctttc tcgccacgtt cgccggcttt ccccgtcaag ctctaaatcg 3060

ggggctccct ttagggttcc gatttagtgc tttacggcac ctcgacccca aaaaacttga 3120

ttagggtgat ggttcacgta gtgggccatc gccctgatag acggtttttc gccctttgac 3180

gttggagtcc acgttcttta atagtggact cttgttccaa actggaacaa cactcaaccc 3240

tatctcggtc tattcttttg atttataagg gattttgccg atttcggcct attggttaaa 3300

aaatgagctg atttaacaaa aatttaacgc gaattttaac aaaatattaa cgcttacaat 3360

ttaggtggca cttttcgggg aaatgtgcgc ggaaccccta tttgtttatt tttctaaata 3420

cattcaaata tgtatccgct catgagacaa taaccctgat aaatgcttca ataatattga 3480

aaaaggaaga gtatgagtat tcaacatttc cgtgtcgccc ttattccctt ttttgcggca 3540

ttttgccttc ctgtttttgc tcacccagaa acgctggtga aagtaaaaga tgctgaagat 3600

cagttgggtg cacgagtggg ttacatcgaa ctggatctca acagcggtaa gatccttgag 3660

agttttcgcc ccgaagaacg ttttccaatg atgagcactt ttaaagttct gctatgtggc 3720

gcggtattat cccgtattga cgccgggcaa gagcaactcg gtcgccgcat acactattct 3780

cagaatgact tggttgagta ctcaccagtc acagaaaagc atcttacgga tggcatgaca 3840

gtaagagaat tatgcagtgc tgccataacc atgagtgata acactgcggc caacttactt 3900

ctgacaacga tcggaggacc gaaggagcta accgcttttt tgcacaacat gggggatcat 3960

gtaactcgcc ttgatcgttg ggaaccggag ctgaatgaag ccataccaaa cgacgagcgt 4020

gacaccacga tgcctgtagc aatggcaaca acgttgcgca aactattaac tggcgaacta 4080

cttactctag cttcccggca acaattaata gactggatgg aggcggataa agttgcagga 4140

ccacttctgc gctcggccct tccggctggc tggtttattg ctgataaatc tggagccggt 4200

gagcgtgggt ctcgcggtat cattgcagca ctggggccag atggtaagcc ctcccgtatc 4260

gtagttatct acacgacggg gagtcaggca actatggatg aacgaaatag acagatcgct 4320

gagataggtg cctcactgat taagcattgg taactgtcag accaagttta ctcatatata 4380

ctttagattg atttaaaact tcatttttaa tttaaaagga tctaggtgaa gatccttttt 4440

gataatctca tgaccaaaat cccttaacgt gagttttcgt tccactgagc gtcagacccc 4500

gtagaaaaga tcaaaggatc ttcttgagat cctttttttc tgcgcgtaat ctgctgcttg 4560

caaacaaaaa aaccaccgct accagcggtg gtttgtttgc cggatcaaga gctaccaact 4620

ctttttccga aggtaactgg cttcagcaga gcgcagatac caaatactgt tcttctagtg 4680

tagccgtagt taggccacca cttcaagaac tctgtagcac cgcctacata cctcgctctg 4740

ctaatcctgt taccagtggc tgctgccagt ggcgataagt cgtgtcttac cgggttggac 4800

tcaagacgat agttaccgga taaggcgcag cggtcgggct gaacgggggg ttcgtgcaca 4860

cagcccagct tggagcgaac gacctacacc gaactgagat acctacagcg tgagctatga 4920

gaaagcgcca cgcttcccga agggagaaag gcggacaggt atccggtaag cggcagggtc 4980

ggaacaggag agcgcacgag ggagcttcca gggggaaacg cctggtatct ttatagtcct 5040

gtcgggtttc gccacctctg acttgagcgt cgatttttgt gatgctcgtc aggggggcgg 5100

agcctatgga aaaacgccag caacgcggcc tttttacggt tcctggcctt ttgctggcct 5160

tttgctcaca tgttctttcc tgcgttatcc cctgattctg tggataaccg tattaccgcc 5220

tttgagtgag ctgataccgc tcgccgcagc cgaacgaccg agcgcagcga gtcagtgagc 5280

gaggaagcgg aagagcgccc aatacgcaaa ccgcctctcc ccgcgcgttg gccgattcat 5340

taatgcagct ggcacgacag gtttcccgac tggaaagcgg gcagtgagcg caacgcaatt 5400

aatgtgagtt agctcactca ttaggcaccc caggctttac actttatgct tccggctcgt 5460

atgttgtgtg gaattgtgag cggataa 5487

<210> 81
<211> 5487
<212> DNA
<213> Artificial Sequence

<220>
<223> Description of Artificial Sequence: Synthetic
      polynucleotide

<400> 81
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtacta tagctagagc tcccaggctc aagtgatcct 240

cccgcctcag cctcctgagt agctgagatc acaggcatgc actaccacac tcggctcaca 300

tttgacatcc tctaaagcat atataaaatg tgaagaaaac tttcacaatt tgcatccctt 360

tgtaatatgt aacagaaata aaattctctt ttaaaatcta tcaacaatag gcaaggcacg 420

gtggctcacg cctgtcgtct cagcactttg tgaggcccag gcgggcagat cgtttgagcc 480

tagaagttca agaccaccct gggcaacata gcgaaacccc ctttctacaa aaaatacaaa 540

aactagctgg gtgtggtggt gcacacctgt agtcccagct acttggaagg ctgaaatggg 600

aagactgctt gagcccggga gggagaagtt gcagtaagcc aggaccacac cactgcactc 660

cagcctgggc aacagagtga gactctgtct caaacaaaca aataaatgag gcgggtggat 720

cacgaggtca gtagatcgag accatcctgg ctaacacggt gaaacccgtc tctactaaaa 780

aaaaaaaaaa atacaaaaaa ttagccaggc atggtggcgg gcgcctgtag tcccagttac 840

tcgggaggct gaggcaggag aatggcgtga aaccgggagg cagagcttgc agtgagccga 900

gatcgcacca ctgccctcca gcctgggcga cagagcgaga ctccgtctca atcaatcaat 960

caatcaataa aatctattaa caatatttat tgtgcactta acaggaacat gccctgtcca 1020

aaaaaaactt tacagggctt aactcatttt atccttacca caatcctatg aagtaggaac 1080

ttttataaaa cgcattttat aaacaaggca cagagaggtt aattaacttg ccctctggtc 1140

acacagctag gaagtgggca gagtacagat ttacacaagg catccgtctc ctggccccac 1200

atacccaact gctgtaaacc cataccggcg gccaagcagc ctcaatttgt gcatgcaccc 1260

acttcccagc aagacagcag ctcccaagtt cctcctgttt agaattttag aagcggcggg 1320

ccaccaggct gcagtctccc ttgggtcagg ggtcctggtt gcactccgtg ctttgcacaa 1380

agcaggctct ccatttttgt taaatgcacg aatagtgcta agctgggaag ttcttcctga 1440

ggtctaacct ctagctgctc ccccacagaa gagtgcctgc ggccagtggc caccaggggt 1500

cgccgcagca cccagcgctg gagggcggag cgggcggcag acccggagca gcaagtggac 1560

tctctcgaga agcttgaact gaaaaaccag aaagttaact ggtaagttta gtctttttgt 1620

cttttatttc aggtcccgga tccggtggtg gtgcaaatca aagaactgct cctcagtgga 1680

tgttgccttt acttctaggc ctgtacggaa gtgttacttc tgctctaaaa gctgcggaat 1740

tgtacccgcg gccgatccac cggtatgtgg actctgggtc gacgcgccgt agccggtctc 1800

ttggcctccc catcccctgc gcaggcacag acccttacac gagttccacg gcccgctgaa 1860

ctggcaccac tctgcggacg ccggggcctg agaaccgata ttgacgctac gtgcactccc 1920

cgacgagcct cttctaatca gagggggctc aaccagatct ggaacgtgaa gaagcagtca 1980

gtctacttga tgaatctgag aaagagcggg acccttgggc accccgggtc tctcgacgag 2040

actacatacg agaggctggc cgaggagact ttggactctc tcgccgaatt cttcgaggat 2100

ttggcagaca agccgtacac ctttgaagac tacgatgtct cattcggatc aggggtgctg 2160

acggttaaac tggggggcga tctcggaacc tacgtcataa acaaacagac acccaataag 2220

cagatctggt tgtctagccc atcatccggt cctaaacggt acgactggac cggcaagaac 2280

tgggtctact cccatgacgg agtcagtctt catgaactgc ttgccgcaga gctgaccaag 2340

gctcttaaga cgaaactgga tctctctagc ctggcctact ccgggaagga tgcatagctc 2400

gaggacgggg tgaactacgc ctgaggatcc gatctttttc cctctgccaa aaattatggg 2460

gacatcatga agccccttga gcatctgact tctggctaat aaaggaaatt tattttcatt 2520

gcaatagtgt gttggaattt tttgtgtctc tcactcggcc taggtagata agtagcatgg 2580

cgggttaatc attaactaca aggaacccct agtgatggag ttggccactc cctctctgcg 2640

cgctcgctcg ctcactgagg ccgggcgacc aaaggtcgcc cgacgcccgg gctttgcccg 2700

ggcggcctca gtgagcgagc gagcgcgcag ccttaattaa cctaattcac tggccgtcgt 2760

tttacaacgt cgtgactggg aaaaccctgg cgttacccaa cttaatcgcc ttgcagcaca 2820

tccccctttc gccagctggc gtaatagcga agaggcccgc accgatcgcc cttcccaaca 2880

gttgcgcagc ctgaatggcg aatgggacgc gccctgtagc ggcgcattaa gcgcggcggg 2940

tgtggtggtt acgcgcagcg tgaccgctac acttgccagc gccctagcgc ccgctccttt 3000

cgctttcttc ccttcctttc tcgccacgtt cgccggcttt ccccgtcaag ctctaaatcg 3060

ggggctccct ttagggttcc gatttagtgc tttacggcac ctcgacccca aaaaacttga 3120

ttagggtgat ggttcacgta gtgggccatc gccctgatag acggtttttc gccctttgac 3180

gttggagtcc acgttcttta atagtggact cttgttccaa actggaacaa cactcaaccc 3240

tatctcggtc tattcttttg atttataagg gattttgccg atttcggcct attggttaaa 3300

aaatgagctg atttaacaaa aatttaacgc gaattttaac aaaatattaa cgcttacaat 3360

ttaggtggca cttttcgggg aaatgtgcgc ggaaccccta tttgtttatt tttctaaata 3420

cattcaaata tgtatccgct catgagacaa taaccctgat aaatgcttca ataatattga 3480

aaaaggaaga gtatgagtat tcaacatttc cgtgtcgccc ttattccctt ttttgcggca 3540

ttttgccttc ctgtttttgc tcacccagaa acgctggtga aagtaaaaga tgctgaagat 3600

cagttgggtg cacgagtggg ttacatcgaa ctggatctca acagcggtaa gatccttgag 3660

agttttcgcc ccgaagaacg ttttccaatg atgagcactt ttaaagttct gctatgtggc 3720

gcggtattat cccgtattga cgccgggcaa gagcaactcg gtcgccgcat acactattct 3780

cagaatgact tggttgagta ctcaccagtc acagaaaagc atcttacgga tggcatgaca 3840

gtaagagaat tatgcagtgc tgccataacc atgagtgata acactgcggc caacttactt 3900

ctgacaacga tcggaggacc gaaggagcta accgcttttt tgcacaacat gggggatcat 3960

gtaactcgcc ttgatcgttg ggaaccggag ctgaatgaag ccataccaaa cgacgagcgt 4020

gacaccacga tgcctgtagc aatggcaaca acgttgcgca aactattaac tggcgaacta 4080

cttactctag cttcccggca acaattaata gactggatgg aggcggataa agttgcagga 4140

ccacttctgc gctcggccct tccggctggc tggtttattg ctgataaatc tggagccggt 4200

gagcgtgggt ctcgcggtat cattgcagca ctggggccag atggtaagcc ctcccgtatc 4260

gtagttatct acacgacggg gagtcaggca actatggatg aacgaaatag acagatcgct 4320

gagataggtg cctcactgat taagcattgg taactgtcag accaagttta ctcatatata 4380

ctttagattg atttaaaact tcatttttaa tttaaaagga tctaggtgaa gatccttttt 4440

gataatctca tgaccaaaat cccttaacgt gagttttcgt tccactgagc gtcagacccc 4500

gtagaaaaga tcaaaggatc ttcttgagat cctttttttc tgcgcgtaat ctgctgcttg 4560

caaacaaaaa aaccaccgct accagcggtg gtttgtttgc cggatcaaga gctaccaact 4620

ctttttccga aggtaactgg cttcagcaga gcgcagatac caaatactgt tcttctagtg 4680

tagccgtagt taggccacca cttcaagaac tctgtagcac cgcctacata cctcgctctg 4740

ctaatcctgt taccagtggc tgctgccagt ggcgataagt cgtgtcttac cgggttggac 4800

tcaagacgat agttaccgga taaggcgcag cggtcgggct gaacgggggg ttcgtgcaca 4860

cagcccagct tggagcgaac gacctacacc gaactgagat acctacagcg tgagctatga 4920

gaaagcgcca cgcttcccga agggagaaag gcggacaggt atccggtaag cggcagggtc 4980

ggaacaggag agcgcacgag ggagcttcca gggggaaacg cctggtatct ttatagtcct 5040

gtcgggtttc gccacctctg acttgagcgt cgatttttgt gatgctcgtc aggggggcgg 5100

agcctatgga aaaacgccag caacgcggcc tttttacggt tcctggcctt ttgctggcct 5160

tttgctcaca tgttctttcc tgcgttatcc cctgattctg tggataaccg tattaccgcc 5220

tttgagtgag ctgataccgc tcgccgcagc cgaacgaccg agcgcagcga gtcagtgagc 5280

gaggaagcgg aagagcgccc aatacgcaaa ccgcctctcc ccgcgcgttg gccgattcat 5340

taatgcagct ggcacgacag gtttcccgac tggaaagcgg gcagtgagcg caacgcaatt 5400

aatgtgagtt agctcactca ttaggcaccc caggctttac actttatgct tccggctcgt 5460

atgttgtgtg gaattgtgag cggataa 5487

<210> 82
<211> 5487
<212> DNA
<213> Artificial Sequence

<220>
<223> Description of Artificial Sequence: Synthetic

polynucleotide

<400> 82
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtacta tagctagagc tcccaggctc aagtgatcct 240

cccgcctcag cctcctgagt agctgagatc acaggcatgc actaccacac tcggctcaca 300

tttgacatcc tctaaagcat atataaaatg tgaagaaaac tttcacaatt tgcatccctt 360

tgtaatatgt aacagaaata aaattctctt ttaaaatcta tcaacaatag gcaaggcacg 420

gtggctcacg cctgtcgtct cagcactttg tgaggcccag gcgggcagat cgtttgagcc 480

tagaagttca agaccaccct gggcaacata gcgaaacccc ctttctacaa aaaatacaaa 540

aactagctgg gtgtggtggt gcacacctgt agtcccagct acttggaagg ctgaaatggg 600

aagactgctt gagcccggga gggagaagtt gcagtaagcc aggaccacac cactgcactc 660

cagcctgggc aacagagtga gactctgtct caaacaaaca aataaatgag gcgggtggat 720

cacgaggtca gtagatcgag accatcctgg ctaacacggt gaaacccgtc tctactaaaa 780

aaaaaaaaaa atacaaaaaa ttagccaggc atggtggcgg gcgcctgtag tcccagttac 840

tcgggaggct gaggcaggag aatggcgtga aaccgggagg cagagcttgc agtgagccga 900

gatcgcacca ctgccctcca gcctgggcga cagagcgaga ctccgtctca atcaatcaat 960

caatcaataa aatctattaa caatatttat tgtgcactta acaggaacat gccctgtcca 1020

aaaaaaactt tacagggctt aactcatttt atccttacca caatcctatg aagtaggaac 1080

ttttataaaa cgcattttat aaacaaggca cagagaggtt aattaacttg ccctctggtc 1140

acacagctag gaagtgggca gagtacagat ttacacaagg catccgtctc ctggccccac 1200

atacccaact gctgtaaacc cataccggcg gccaagcagc ctcaatttgt gcatgcaccc 1260

acttcccagc aagacagcag ctcccaagtt cctcctgttt agaattttag aagcggcggg 1320

ccaccaggct gcagtctccc ttgggtcagg ggtcctggtt gcactccgtg ctttgcacaa 1380

agcaggctct ccatttttgt taaatgcacg aatagtgcta agctgggaag ttcttcctga 1440

ggtctaacct ctagctgctc ccccacagaa gagtgcctgc ggccagtggc caccaggggt 1500

cgccgcagca cccagcgctg gagggcggag cgggcggcag acccggagca gcaagtggac 1560

tctctcgaga agcttgaact gaaaaaccag aaagttaact ggtaagttta gtctttttgt 1620

cttttatttc aggtcccgga tccggtggtg gtgcaaatca aagaactgct cctcagtgga 1680

tgttgccttt acttctaggc ctgtacggaa gtgttacttc tgctctaaaa gctgcggaat 1740

tgtacccgcg gccgatccac cggtatgtgg actctcgggc gacgcgccgt agccggtctc 1800

ctggcttccc caagccctgc acaggcacag accctcacac gggtcccacg gccagctgaa 1860

ttggccccac tctgcggtcg ccggggcctg cgaaccgata tagacgcaac gtgcactccc 1920

cgacgagcaa gttccaacca gagagggctc aaccagattt ggaacgtcaa gaagcagagt 1980

gtctatttga tgaatctgag aaaatccggg acccttgggc accccggatc tcttgatgaa 2040

actacctatg aaaggctggc cgaggagacg ctggactctc tggcagagtt cttcgaggat 2100

ctggcagaca agccatacac gtttgaggac tatgatgtct cttttggatc tggcgtcctg 2160

acggttaaac tgggagggga tctcggaacc tacgttatca acaaacagac tcccaataag 2220

cagatctggt tgtctagtcc atccagtggt cctaaacgct atgactggac gggcaagaac 2280

tgggtgtact cccacgacgg agttagcctt catgaactgc tggccgcaga gctgactaaa 2340

gctcttaaga ctaaactgga tctctctagc ctggcctact ccgggaaaga tgcctagctc 2400

gaggacgggg tgaactacgc ctgaggatcc gatctttttc cctctgccaa aaattatggg 2460

gacatcatga agccccttga gcatctgact tctggctaat aaaggaaatt tattttcatt 2520

gcaatagtgt gttggaattt tttgtgtctc tcactcggcc taggtagata agtagcatgg 2580

cgggttaatc attaactaca aggaacccct agtgatggag ttggccactc cctctctgcg 2640

cgctcgctcg ctcactgagg ccgggcgacc aaaggtcgcc cgacgcccgg gctttgcccg 2700

ggcggcctca gtgagcgagc gagcgcgcag ccttaattaa cctaattcac tggccgtcgt 2760

tttacaacgt cgtgactggg aaaaccctgg cgttacccaa cttaatcgcc ttgcagcaca 2820

tccccctttc gccagctggc gtaatagcga agaggcccgc accgatcgcc cttcccaaca 2880

gttgcgcagc ctgaatggcg aatgggacgc gccctgtagc ggcgcattaa gcgcggcggg 2940

tgtggtggtt acgcgcagcg tgaccgctac acttgccagc gccctagcgc ccgctccttt 3000

cgctttcttc ccttcctttc tcgccacgtt cgccggcttt ccccgtcaag ctctaaatcg 3060

ggggctccct ttagggttcc gatttagtgc tttacggcac ctcgacccca aaaaacttga 3120

ttagggtgat ggttcacgta gtgggccatc gccctgatag acggtttttc gccctttgac 3180

gttggagtcc acgttcttta atagtggact cttgttccaa actggaacaa cactcaaccc 3240

tatctcggtc tattcttttg atttataagg gattttgccg atttcggcct attggttaaa 3300

aaatgagctg atttaacaaa aatttaacgc gaattttaac aaaatattaa cgcttacaat 3360

ttaggtggca cttttcgggg aaatgtgcgc ggaaccccta tttgtttatt tttctaaata 3420

cattcaaata tgtatccgct catgagacaa taaccctgat aaatgcttca ataatattga 3480

aaaaggaaga gtatgagtat tcaacatttc cgtgtcgccc ttattccctt ttttgcggca 3540

ttttgccttc ctgtttttgc tcacccagaa acgctggtga aagtaaaaga tgctgaagat 3600

cagttgggtg cacgagtggg ttacatcgaa ctggatctca acagcggtaa gatccttgag 3660

agttttcgcc ccgaagaacg ttttccaatg atgagcactt ttaaagttct gctatgtggc 3720

gcggtattat cccgtattga cgccgggcaa gagcaactcg gtcgccgcat acactattct 3780

cagaatgact tggttgagta ctcaccagtc acagaaaagc atcttacgga tggcatgaca 3840

gtaagagaat tatgcagtgc tgccataacc atgagtgata acactgcggc caacttactt 3900

ctgacaacga tcggaggacc gaaggagcta accgcttttt tgcacaacat gggggatcat 3960

gtaactcgcc ttgatcgttg ggaaccggag ctgaatgaag ccataccaaa cgacgagcgt 4020

gacaccacga tgcctgtagc aatggcaaca acgttgcgca aactattaac tggcgaacta 4080

cttactctag cttcccggca acaattaata gactggatgg aggcggataa agttgcagga 4140

ccacttctgc gctcggccct tccggctggc tggtttattg ctgataaatc tggagccggt 4200

gagcgtgggt ctcgcggtat cattgcagca ctggggccag atggtaagcc ctcccgtatc 4260

gtagttatct acacgacggg gagtcaggca actatggatg aacgaaatag acagatcgct 4320

gagataggtg cctcactgat taagcattgg taactgtcag accaagttta ctcatatata 4380

ctttagattg atttaaaact tcatttttaa tttaaaagga tctaggtgaa gatccttttt 4440

gataatctca tgaccaaaat cccttaacgt gagttttcgt tccactgagc gtcagacccc 4500

gtagaaaaga tcaaaggatc ttcttgagat cctttttttc tgcgcgtaat ctgctgcttg 4560

caaacaaaaa aaccaccgct accagcggtg gtttgtttgc cggatcaaga gctaccaact 4620

ctttttccga aggtaactgg cttcagcaga gcgcagatac caaatactgt tcttctagtg 4680

tagccgtagt taggccacca cttcaagaac tctgtagcac cgcctacata cctcgctctg 4740

ctaatcctgt taccagtggc tgctgccagt ggcgataagt cgtgtcttac cgggttggac 4800

tcaagacgat agttaccgga taaggcgcag cggtcgggct gaacgggggg ttcgtgcaca 4860

cagcccagct tggagcgaac gacctacacc gaactgagat acctacagcg tgagctatga 4920

gaaagcgcca cgcttcccga agggagaaag gcggacaggt atccggtaag cggcagggtc 4980

ggaacaggag agcgcacgag ggagcttcca gggggaaacg cctggtatct ttatagtcct 5040

gtcgggtttc gccacctctg acttgagcgt cgatttttgt gatgctcgtc aggggggcgg 5100

agcctatgga aaaacgccag caacgcggcc tttttacggt tcctggcctt ttgctggcct 5160

tttgctcaca tgttctttcc tgcgttatcc cctgattctg tggataaccg tattaccgcc 5220

tttgagtgag ctgataccgc tcgccgcagc cgaacgaccg agcgcagcga gtcagtgagc 5280

gaggaagcgg aagagcgccc aatacgcaaa ccgcctctcc ccgcgcgttg gccgattcat 5340

taatgcagct ggcacgacag gtttcccgac tggaaagcgg gcagtgagcg caacgcaatt 5400

aatgtgagtt agctcactca ttaggcaccc caggctttac actttatgct tccggctcgt 5460

atgttgtgtg gaattgtgag cggataa 5487

<210> 83

<211> 5487
<212> DNA
<213> Artificial Sequence

<220>
<223> Description of Artificial Sequence: Synthetic
      polynucleotide

<400> 83
caatttcaca caggaaacag ctatgaccat gattacgcca gatttaatta aggccttaat      60

taggctgcgc gctcgctcgc tcactgaggc cgcccgggca aagcccgggc gtcgggcgac     120

ctttggtcgc ccggcctcag tgagcgagcg agcgcgcaga gagggagtgt agccatgctc     180

taggaagatc aattcaattc acgcgtacta tagctagagc tcccaggctc aagtgatcct     240

cccgcctcag cctcctgagt agctgagatc acaggcatgc actaccacac tcggctcaca     300

tttgacatcc tctaaagcat atataaaatg tgaagaaaac tttcacaatt tgcatccctt     360

tgtaatatgt aacagaaata aaattctctt ttaaaatcta tcaacaatag gcaaggcacg     420

gtggctcacg cctgtcgtct cagcactttg tgaggcccag gcgggcagat cgtttgagcc     480

tagaagttca agaccaccct gggcaacata gcgaaacccc ctttctacaa aaaatacaaa     540

aactagctgg gtgtggtggt gcacacctgt agtcccagct acttggaagg ctgaaatggg     600

aagactgctt gagcccggga gggagaagtt gcagtaagcc aggaccacac cactgcactc     660

cagcctgggc aacagagtga gactctgtct caaacaaaca aataaatgag gcgggtggat     720

cacgaggtca gtagatcgag accatcctgg ctaacacggt gaaacccgtc tctactaaaa     780

aaaaaaaaaa atacaaaaaa ttagccaggc atggtggcgg gcgcctgtag tcccagttac     840

tcgggaggct gaggcaggag aatggcgtga aaccgggagg cagagcttgc agtgagccga     900

gatcgcacca ctgccctcca gcctgggcga cagagcgaga ctccgtctca atcaatcaat     960

caatcaataa aatctattaa caatatttat tgtgcactta acaggaacat gccctgtcca    1020

aaaaaaactt tacagggctt aactcatttt atccttacca caatcctatg aagtaggaac    1080

ttttataaaa cgcattttat aaacaaggca cagagaggtt aattaacttg ccctctggtc    1140

acacagctag gaagtgggca gagtacagat ttacacaagg catccgtctc ctggccccac    1200

atacccaact gctgtaaacc cataccggcg gccaagcagc ctcaatttgt gcatgcaccc    1260

acttcccagc aagacagcag ctcccaagtt cctcctgttt agaattttag aagcggcggg    1320

ccaccaggct gcagtctccc ttgggtcagg ggtcctggtt gcactccgtg ctttgcacaa    1380

agcaggctct ccatttttgt taaatgcacg aatagtgcta agctgggaag ttcttcctga    1440

ggtctaacct ctagctgctc ccccacagaa gagtgcctgc ggccagtggc caccaggggt    1500

cgccgcagca cccagcgctg gagggcggag cgggcggcag acccggagca gcaagtggac    1560

tctctcgaga agcttgaact gaaaaaccag aaagttaact ggtaagttta gtctttttgt    1620

cttttatttc aggtcccgga tccggtggtg gtgcaaatca aagaactgct cctcagtgga 1680

tgttgccttt acttctaggc ctgtacggaa gtgttacttc tgctctaaaa gctgcggaat 1740

tgtacccgcg gccgatccac cggtatgtgg actctggggc gacgcgccgt ggccgggctc 1800

ctggcatccc catcccctgc gcaggcgcaa accctcacac gggttccacg gccagctgaa 1860

ctggccccac tgtgcggccg gcggggcctg agaacagata ttgacgcaac gtgcactccc 1920

agaagagcca gcagcaacca gagggggttg aatcagatct ggaacgtcaa gaagcagtca 1980

gtgtacctga tgaatctccg aaaaagcggg acccttggtc accccggatc tctcgatgaa 2040

accacatatg aacggctggc cgaggaaacg ctggactccc tggccgagtt cttcgaggac 2100

ctggcagaca agccgtacac cttcgaagac tatgatgttt cattcggaag tggcgtgctg 2160

acggtcaaac tgggaggcga tctgggaacc tacgttataa acaaacagac tcccaataaa 2220

cagatctggt tgtctagtcc atccagcggt cctaaacgct atgactggac gggcaagaac 2280

tgggtctact cccacgacgg agttagcctt cacgaactgt tggccgcaga gctgacaaag 2340

gcgcttaaga cgaaactgga tctctctagc ctggcctact ccggcaaaga tgcctagctc 2400

gaggacgggg tgaactacgc ctgaggatcc gatctttttc cctctgccaa aaattatggg 2460

gacatcatga agccccttga gcatctgact tctggctaat aaaggaaatt tattttcatt 2520

gcaatagtgt gttggaattt tttgtgtctc tcactcggcc taggtagata agtagcatgg 2580

cgggttaatc attaactaca aggaacccct agtgatggag ttggccactc cctctctgcg 2640

cgctcgctcg ctcactgagg ccgggcgacc aaaggtcgcc cgacgcccgg gctttgcccg 2700

ggcggcctca gtgagcgagc gagcgcgcag ccttaattaa cctaattcac tggccgtcgt 2760

tttacaacgt cgtgactggg aaaaccctgg cgttacccaa cttaatcgcc ttgcagcaca 2820

tccccctttc gccagctggc gtaatagcga agaggcccgc accgatcgcc cttcccaaca 2880

gttgcgcagc ctgaatggcg aatgggacgc gccctgtagc ggcgcattaa gcgcggcggg 2940

tgtggtggtt acgcgcagcg tgaccgctac acttgccagc gccctagcgc ccgctccttt 3000

cgctttcttc ccttcctttc tcgccacgtt cgccggcttt ccccgtcaag ctctaaatcg 3060

ggggctccct ttagggttcc gatttagtgc tttacggcac ctcgacccca aaaaacttga 3120

ttagggtgat ggttcacgta gtgggccatc gccctgatag acggtttttc gccctttgac 3180

gttggagtcc acgttcttta atagtggact cttgttccaa actggaacaa cactcaaccc 3240

tatctcggtc tattcttttg atttataagg gattttgccg atttcggcct attggttaaa 3300

aaatgagctg atttaacaaa aatttaacgc gaattttaac aaaatattaa cgcttacaat 3360

ttaggtggca cttttcgggg aaatgtgcgc ggaaccccta tttgtttatt tttctaaata 3420

cattcaaata tgtatccgct catgagacaa taaccctgat aaatgcttca ataatattga 3480

aaaaggaaga gtatgagtat tcaacatttc cgtgtcgccc ttattccctt ttttgcggca 3540

ttttgccttc ctgtttttgc tcacccagaa acgctggtga aagtaaaaga tgctgaagat 3600

cagttgggtg cacgagtggg ttacatcgaa ctggatctca acagcggtaa gatccttgag 3660

agttttcgcc ccgaagaacg ttttccaatg atgagcactt ttaaagttct gctatgtggc 3720

gcggtattat cccgtattga cgccgggcaa gagcaactcg gtcgccgcat acactattct 3780

cagaatgact tggttgagta ctcaccagtc acagaaaagc atcttacgga tggcatgaca 3840

gtaagagaat tatgcagtgc tgccataacc atgagtgata acactgcggc caacttactt 3900

ctgacaacga tcggaggacc gaaggagcta accgcttttt tgcacaacat gggggatcat 3960

gtaactcgcc ttgatcgttg ggaaccggag ctgaatgaag ccataccaaa cgacgagcgt 4020

gacaccacga tgcctgtagc aatggcaaca acgttgcgca aactattaac tggcgaacta 4080

cttactctag cttcccggca acaattaata gactggatgg aggcggataa agttgcagga 4140

ccacttctgc gctcggccct tccggctggc tggtttattg ctgataaatc tggagccggt 4200

gagcgtgggt ctcgcggtat cattgcagca ctggggccag atggtaagcc ctcccgtatc 4260

gtagttatct acacgacggg gagtcaggca actatggatg aacgaaatag acagatcgct 4320

gagataggtg cctcactgat taagcattgg taactgtcag accaagttta ctcatatata 4380

ctttagattg atttaaaact tcatttttaa tttaaaagga tctaggtgaa gatccttttt 4440

gataatctca tgaccaaaat cccttaacgt gagttttcgt tccactgagc gtcagacccc 4500

gtagaaaaga tcaaaggatc ttcttgagat cctttttttc tgcgcgtaat ctgctgcttg 4560

caaacaaaaa aaccaccgct accagcggtg gtttgtttgc cggatcaaga gctaccaact 4620

ctttttccga aggtaactgg cttcagcaga gcgcagatac caaatactgt tcttctagtg 4680

tagccgtagt taggccacca cttcaagaac tctgtagcac cgcctacata cctcgctctg 4740

ctaatcctgt taccagtggc tgctgccagt ggcgataagt cgtgtcttac cgggttggac 4800

tcaagacgat agttaccgga taaggcgcag cggtcgggct gaacgggggg ttcgtgcaca 4860

cagcccagct tggagcgaac gacctacacc gaactgagat acctacagcg tgagctatga 4920

gaaagcgcca cgcttcccga agggagaaag gcggacaggt atccggtaag cggcagggtc 4980

ggaacaggag agcgcacgag ggagcttcca gggggaaacg cctggtatct ttatagtcct 5040

gtcgggtttc gccacctctg acttgagcgt cgatttttgt gatgctcgtc aggggggcgg 5100

agcctatgga aaaacgccag caacgcggcc tttttacggt tcctggcctt ttgctggcct 5160

tttgctcaca tgttctttcc tgcgttatcc cctgattctg tggataaccg tattaccgcc 5220

tttgagtgag ctgataccgc tcgccgcagc cgaacgaccg agcgcagcga gtcagtgagc 5280

gaggaagcgg aagagcgccc aatacgcaaa ccgcctctcc ccgcgcgttg gccgattcat 5340

taatgcagct ggcacgacag gtttcccgac tggaaagcgg gcagtgagcg caacgcaatt 5400

aatgtgagtt agctcactca ttaggcaccc caggctttac actttatgct tccggctcgt 5460

atgttgtgtg gaattgtgag cggataa 5487

<210> 84
<211> 6391
<212> DNA
<213> Artificial Sequence

<220>
<223> Description of Artificial Sequence: Synthetic
      polynucleotide

<400> 84
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtccca cctccctctc tgtgctggga ctcacagagg 240

gagacctcag gaggcagtct gtccatcaca tgtccaaatg cagagcatac cctgggctgg 300

gcgcagtggc gcacaactgt aattccagca ctttgggagg ctgatgtgga aggatcactt 360

gagcccagaa gttctagacc agcctgggca acatggcaag accctatctc tacaaaaaaa 420

gttaaaaaat cagccacgtg tggtgacaca cacctgtagt cccagctatt caggaggctg 480

aggtgagggg atcacttaag gctgggaggt tgaggctgca gtgagtcgtg gttgcgccac 540

tgcactccag cctgggcaac agtgagaccc tgtctcaaaa gacaaaaaaa aaaaaaaaaa 600

aaaaaagaac atatcctggt gtggagtagg ggacgctgct ctgacagagg ctcgggggcc 660

tgagctggct ctgtgagctg gggaggaggc agacagccag gccttgtctg caagcagacc 720

tggcagcatt gggctggccg ccccccaggg cctcctcttc atgcccagtg aatgactcac 780

cttggcacag acacaatgtt cggggtgggc acagtgcctg cttcccgccg caccccagcc 840

cccctcaaat gccttccgag aagcccattg agcagggggc ttgcattgca ccccagcctg 900

acagcctggc atcttgggat aaaagcagca cagcccccta ggggctgccc ttgctgtgtg 960

gcgccaccgg cggtggagaa caaggctcta ttcagcctgt gcccaggaaa ggggatcagg 1020

ggatgcccag gcatggacag tgggtggcag ggggggagag gagggctgtc tgcttcccag 1080

aagtccaagg acacaaatgg gtgaggggac tgggcagggt tctgaccctg tgggaccaga 1140

gtggagggcg tagatggacc tgaagtctcc agggacaaca gggcccaggt ctcaggctcc 1200

tagttgggcc cagtggctcc agcgtttcca aacccatcca tccccagagg ttcttcccat 1260

ctctccaggc tgatgtgtgg gaactcgagg aaataaatct ccagtgggag acggaggggt 1320

ggccagggaa acggggcgct gcaggaataa agacgagcca gcacagccag ctcatgtgta 1380

acggctttgt ggagctgtca aggcctggtc tctgggagag aggcacaggg aggccagaca 1440

aggaaggggt gacctggagg gacagatcca ggggctaaag tcctgataag gcaagagagt 1500

gccggccccc tcttgcccta tcaggacctc cactgccaca tagaggccat gattgaccct 1560

tagacaaagg gctggtgtcc aatcccagcc cccagcccca gaactccagg gaatgaatgg 1620

gcagagagca ggaatgtggg acatctgtgt tcaagggaag gactccagga gtctgctggg 1680

aatgaggcct agtaggaaat gaggtggccc ttgagggtac agaacaggtt cattcttcgc 1740

caaattccca gcaccttgca ggcacttaca gctgagtgag ataatgcctg ggttatgaaa 1800

tcaaaaagtt ggaaagcagg tcagaggtca tctggtacag cccttccttc cctttttttt 1860

tttttttttt tgtgagacaa ggtctctctc tgttgcccag gctggagtgg cgcaaacaca 1920

gctcactgca gcctcaacct actgggctca agcaatcctc cagcctcagc ctcccaaagt 1980

gctgggatta caagcatgag ccaccccact cagccctttc cttccttttt aattgatgca 2040

taataattgt aagtattcat catggtccaa ccaacccttt cttgacccac cttcctagag 2100

agagggtcct cttgcttcag cggtcagggc cccagaccca tggtctggct ccaggtacca 2160

cctgcctcat gcaggagttg gcgtgcccag gaagctctgc ctctgggcac agtgacctca 2220

gtggggtgag gggagctctc cccatagctg ggctgcggcc caaccccacc ccctcaggct 2280

atgccagggg gtgttgccag gggcacccgg gcatcgccag tctagcccac tccttcataa 2340

agccctcgca tcccaggagc gagcagagcc agagcaggtt ggagaggaga cgcatcacct 2400

ccgctgctcg ccgggatcct cgggagcaag cttgaactga aaaaccagaa agttaactgg 2460

taagtttagt ctttttgtct tttatttcag gtcccggatc cggtggtggt gcaaatcaaa 2520

gaactgctcc tcagtggatg ttgcctttac ttctaggcct gtacggaagt gttacttctg 2580

ctctaaaagc tgcggaattg tacccgcggc cgatccaccg gtatgtggac tctcgggcgc 2640

cgcgcagtag ccggcctcct ggcgtcaccc agcccagccc aggcccagac cctcacccgg 2700

gtcccgcggc cggcagagtt ggccccactc tgcggccgcc gtggcctgcg caccgacatc 2760

gatgcgacct gcacgccccg ccgcgcaagt tcgaaccaac gtggcctcaa ccagatttgg 2820

aatgtcaaaa agcagagtgt ctatttgatg aatttgagga aatctggaac tttgggccac 2880

ccaggctctc tagatgagac cacctatgaa agactagcag aggaaacgct ggactcttta 2940

gcagagtttt ttgaagacct tgcagacaag ccatacacgt ttgaggacta tgatgtctcc 3000

tttgggagtg gtgtcttaac tgtcaaactg ggtggagatc taggaaccta tgtgatcaac 3060

aagcagacgc caaacaagca aatctggcta tcttctccat ccagtggacc taagcgttat 3120

gactggactg ggaaaaactg ggtgtactcc cacgacggcg tgtccctcca tgagctgctg 3180

gccgcagagc tcactaaagc cttaaaaacc aaactggact tgtcttcctt ggcctattcc 3240

ggaaaagatg cttgagcggc cctagcgttt aaacgggccc tctagagtcg acccgggcgg 3300

cctcgaggac ggggtgaact acgcctgagg atccgatctt tttccctctg ccaaaaatta 3360

tggggacatc atgaagcccc ttgagcatct gacttctggc taataaagga aatttatttt 3420

cattgcaata gtgtgttgga attttttgtg tctctcactc ggcctaggta gataagtagc 3480

atggcgggtt aatcattaac tacaaggaac ccctagtgat ggagttggcc actccctctc 3540

tgcgcgctcg ctcgctcact gaggccgggc gaccaaaggt cgcccgacgc ccgggctttg 3600

cccgggcggc ctcagtgagc gagcgagcgc gcagccttaa ttaacctaat tcactggccg 3660

tcgttttaca acgtcgtgac tgggaaaacc ctggcgttac ccaacttaat cgccttgcag 3720

cacatccccc tttcgccagc tggcgtaata gcgaagaggc ccgcaccgat cgcccttccc 3780

aacagttgcg cagcctgaat ggcgaatggg acgcgccctg tagcggcgca ttaagcgcgg 3840

cgggtgtggt ggttacgcgc agcgtgaccg ctacacttgc cagcgcccta gcgcccgctc 3900

ctttcgcttt cttcccttcc tttctcgcca cgttcgccgg ctttccccgt caagctctaa 3960

atcgggggct ccctttaggg ttccgattta gtgctttacg gcacctcgac cccaaaaaac 4020

ttgattaggg tgatggttca cgtagtgggc catcgccctg atagacggtt tttcgccctt 4080

tgacgttgga gtccacgttc tttaatagtg gactcttgtt ccaaactgga acaacactca 4140

accctatctc ggtctattct tttgatttat aagggatttt gccgatttcg gcctattggt 4200

taaaaaatga gctgatttaa caaaaattta acgcgaattt taacaaaata ttaacgctta 4260

caatttaggt ggcacttttc ggggaaatgt gcgcggaacc cctatttgtt tatttttcta 4320

aatacattca aatatgtatc cgctcatgag acaataaccc tgataaatgc ttcaataata 4380

ttgaaaaagg aagagtatga gtattcaaca tttccgtgtc gcccttattc ccttttttgc 4440

ggcattttgc cttcctgttt ttgctcaccc agaaacgctg gtgaaagtaa aagatgctga 4500

agatcagttg ggtgcacgag tgggttacat cgaactggat ctcaacagcg gtaagatcct 4560

tgagagtttt cgccccgaag aacgttttcc aatgatgagc acttttaaag ttctgctatg 4620

tggcgcggta ttatcccgta ttgacgccgg gcaagagcaa ctcggtcgcc gcatacacta 4680

ttctcagaat gacttggttg agtactcacc agtcacagaa aagcatctta cggatggcat 4740

gacagtaaga gaattatgca gtgctgccat aaccatgagt gataacactg cggccaactt 4800

acttctgaca acgatcggag gaccgaagga gctaaccgct tttttgcaca acatggggga 4860

tcatgtaact cgccttgatc gttgggaacc ggagctgaat gaagccatac caaacgacga 4920

gcgtgacacc acgatgcctg tagcaatggc aacaacgttg cgcaaactat taactggcga 4980

actacttact ctagcttccc ggcaacaatt aatagactgg atggaggcgg ataaagttgc 5040

aggaccactt ctgcgctcgg cccttccggc tggctggttt attgctgata aatctggagc 5100

cggtgagcgt gggtctcgcg gtatcattgc agcactgggg ccagatggta agccctcccg 5160

tatcgtagtt atctacacga cggggagtca ggcaactatg gatgaacgaa atagacagat 5220

cgctgagata ggtgcctcac tgattaagca ttggtaactg tcagaccaag tttactcata 5280

tatactttag attgatttaa aacttcattt ttaatttaaa aggatctagg tgaagatcct 5340

ttttgataat ctcatgacca aaatccctta acgtgagttt tcgttccact gagcgtcaga 5400

ccccgtagaa aagatcaaag gatcttcttg agatcctttt tttctgcgcg taatctgctg 5460

cttgcaaaca aaaaaaccac cgctaccagc ggtggtttgt ttgccggatc aagagctacc 5520

aactcttttt ccgaaggtaa ctggcttcag cagagcgcag ataccaaata ctgttcttct 5580

agtgtagccg tagttaggcc accacttcaa gaactctgta gcaccgccta catacctcgc 5640

tctgctaatc ctgttaccag tggctgctgc cagtggcgat aagtcgtgtc ttaccgggtt 5700

ggactcaaga cgatagttac cggataaggc gcagcggtcg ggctgaacgg ggggttcgtg 5760

cacacagccc agcttggagc gaacgaccta caccgaactg agatacctac agcgtgagct 5820

atgagaaagc gccacgcttc ccgaagggag aaaggcggac aggtatccgg taagcggcag 5880

ggtcggaaca ggagagcgca cgagggagct tccaggggga aacgcctggt atctttatag 5940

tcctgtcggg tttcgccacc tctgacttga gcgtcgattt ttgtgatgct cgtcaggggg 6000

gcggagccta tggaaaaacg ccagcaacgc ggccttttta cggttcctgg ccttttgctg 6060

gccttttgct cacatgttct ttcctgcgtt atcccctgat tctgtggata accgtattac 6120

cgcctttgag tgagctgata ccgctcgccg cagccgaacg accgagcgca gcgagtcagt 6180

gagcgaggaa gcggaagagc gcccaatacg caaaccgcct ctccccgcgc gttggccgat 6240

tcattaatgc agctggcacg acaggtttcc cgactggaaa gcgggcagtg agcgcaacgc 6300

aattaatgtg agttagctca ctcattaggc accccaggct ttacacttta tgcttccggc 6360

tcgtatgttg tgtggaattg tgagcggata a 6391

<210> 85
<211> 4699
<212> DNA
<213> Artificial Sequence

<220>
<223> Description of Artificial Sequence: Synthetic
      polynucleotide

<400> 85
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtacta tagtccacgg ggttggggtt gcgccttttc 240

caaggcagcc ctgggtttgc gcagggacgc ggctgctctg ggcgtggttc cgggaaacgc 300

agcggcgccg accctgggtc tcgcacattc ttcacgtccg ttcgcagcgt cacccggatc 360

ttcgccgcta cccttgtggg ccccccggcg acgcttcctc gtccgcccct aagtcgggaa 420

ggttccttgc ggttcgcggc gtgccggacg tgacaaacgg aagccgcacg tctcactagt 480

accctcgcag acggacagcg ccagggagca atggcagcgc gccgaccgcg atgggctgtg 540

gccaatagcg gctgctcagc agggcgcgcc gagagcagcg gccgggaagg ggcggtgcgg 600

gaggcggggt gtggggcggt agtgtgggcc ctgttcctgc ccgcgcggtg ttccgcattc 660

tgcaagcctc cggagcgcac gtcggcagtc ggctccctcg ttgaccgaat caccgacctc 720

tctccccacg ggagcaagct tgaactgaaa aaccagaaag ttaactggta agtttagtct 780

ttttgtcttt tatttcaggt cccggatccg gtggtggtgc aaatcaaaga actgctcctc 840

agtggatgtt gcctttactt ctaggcctgt acggaagtgt tacttctgct ctaaaagctg 900

cggaattgta cccgcggccg atccaccggt atgtggactc tcgggcgccg cgcagtagcc 960

ggcctcctgg cgtcacccag cccagcccag gcccagaccc tcacccgggt cccgcggccg 1020

gcagagttgg ccccactctg cggccgccgt ggcctgcgca ccgacatcga tgcgacctgc 1080

acgccccgcc gcgcaagttc gaaccaacgt ggcctcaacc agatttggaa tgtcaaaaag 1140

cagagtgtct atttgatgaa tttgaggaaa tctggaactt tgggccaccc aggctctcta 1200

gatgagacca cctatgaaag actagcagag gaaacgctgg actctttagc agagtttttt 1260

gaagaccttg cagacaagcc atacacgttt gaggactatg atgtctcctt tgggagtggt 1320

gtcttaactg tcaaactggg tggagatcta ggaacctatg tgatcaacaa gcagacgcca 1380

aacaagcaaa tctggctatc ttctccatcc agtggaccta agcgttatga ctggactggg 1440

aaaaactggg tgtactccca cgacggcgtg tccctccatg agctgctggc cgcagagctc 1500

actaaagcct taaaaaccaa actggacttg tcttccttgg cctattccgg aaaagatgct 1560

tgagcggccc tagcgtttaa acgggccctc tagagtcgac ccgggcggcc tcgaggacgg 1620

ggtgaactac gcctgaggat ccgatctttt tccctctgcc aaaaattatg gggacatcat 1680

gaagcccctt gagcatctga cttctggcta ataaaggaaa tttattttca ttgcaatagt 1740

gtgttggaat tttttgtgtc tctcactcgg cctaggtaga taagtagcat ggcgggttaa 1800

tcattaacta caaggaaccc ctagtgatgg agttggccac tccctctctg cgcgctcgct 1860

cgctcactga ggccgggcga ccaaaggtcg cccgacgccc gggctttgcc cgggcggcct 1920

cagtgagcga gcgagcgcgc agccttaatt aacctaattc actggccgtc gttttacaac 1980

gtcgtgactg ggaaaaccct ggcgttaccc aacttaatcg ccttgcagca catccccctt 2040

tcgccagctg gcgtaatagc gaagaggccc gcaccgatcg cccttcccaa cagttgcgca 2100

gcctgaatgg cgaatggacg cgccctgtag cggcgcatta agcgcggcgg gtgtggtgg 2160

ttacgcgcag cgtgaccgct acacttgcca gcgccctagc gcccgctcct ttcgctttct 2220

tcccttcctt tctcgccacg ttcgccggct ttccccgtca agctctaaat cgggggctcc 2280

ctttagggtt ccgatttagt gctttacggc acctcgaccc caaaaaactt gattagggtg 2340

atggttcacg tagtgggcca tcgccctgat agacggtttt tcgccctttg acgttggagt 2400

ccacgttctt taatagtgga ctcttgttcc aaactggaac aacactcaac cctatctcgg 2460

tctattcttt tgatttataa gggattttgc cgatttcggc ctattggtta aaaaatgagc 2520

tgatttaaca aaaatttaac gcgaatttta acaaaatatt aacgcttaca atttaggtgg 2580

cacttttcgg ggaaatgtgc gcggaacccc tatttgttta tttttctaaa tacattcaaa 2640

tatgtatccg ctcatgagac aataaccctg ataaatgctt caataatatt gaaaaaggaa 2700

gagtatgagt attcaacatt tccgtgtcgc ccttattccc ttttttgcgg cattttgcct 2760

tcctgttttt gctcacccag aaacgctggt gaaagtaaaa gatgctgaag atcagttggg 2820

tgcacgagtg ggttacatcg aactggatct caacagcggt aagatccttg agagttttcg 2880

ccccgaagaa cgttttccaa tgatgagcac ttttaaagtt ctgctatgtg gcgcggtatt 2940

atcccgtatt gacgccgggc aagagcaact cggtcgccgc atacactatt ctcagaatga 3000

cttggttgag tactcaccag tcacagaaaa gcatcttacg gatggcatga cagtaagaga 3060

attatgcagt gctgccataa ccatgagtga taacactgcg gccaacttac ttctgacaac 3120

gatcggagga ccgaaggagc taaccgcttt tttgcacaac atgggggatc atgtaactcg 3180

ccttgatcgt tgggaaccgg agctgaatga agccatacca aacgacgagc gtgacaccac 3240

gatgcctgta gcaatggcaa caacgttgcg caaactatta actggcgaac tacttactct 3300

agcttcccgg caacaattaa tagactggat ggaggcggat aaagttgcag gaccacttct 3360

gcgctcggcc cttccggctg gctggtttat tgctgataaa tctggagccg gtgagcgtgg 3420

gtctcgcggt atcattgcag cactggggcc agatggtaag ccctcccgta tcgtagttat 3480

ctacacgacg gggagtcagg caactatgga tgaacgaaat agacagatcg ctgagatagg 3540

tgcctcactg attaagcatt ggtaactgtc agaccaagtt tactcatata tactttagat 3600

tgatttaaaa cttcattttt aatttaaaag gatctaggtg aagatccttt ttgataatct 3660

catgaccaaa atcccttaac gtgagttttc gttccactga gcgtcagacc ccgtagaaaa 3720

gatcaaagga tcttcttgag atcctttttt tctgcgcgta atctgctgct tgcaaacaaa 3780

aaaaccaccg ctaccagcgg tggtttgttt gccggatcaa gagctaccaa ctctttttcc 3840

gaaggtaact ggcttcagca gagcgcagat accaaatact gttcttctag tgtagccgta 3900

gttaggccac cacttcaaga actctgtagc accgcctaca tacctcgctc tgctaatcct 3960

gttaccagtg gctgctgcca gtggcgataa gtcgtgtctt accgggttgg actcaagacg 4020

atagttaccg gataaggcgc agcggtcggg ctgaacgggg ggttcgtgca cacagcccag 4080

cttggagcga acgacctaca ccgaactgag atacctacag cgtgagctat gagaaagcgc 4140

cacgcttccc gaagggagaa aggcggacag gtatccggta agcggcaggg tcggaacagg 4200

agagcgcacg agggagcttc cagggggaaa cgcctggtat ctttatagtc ctgtcgggtt 4260

tcgccacctc tgacttgagc gtcgattttt gtgatgctcg tcaggggggc ggagcctatg 4320

gaaaaacgcc agcaacgcgg cctttttacg gttcctggcc ttttgctggc cttttgctca 4380

catgttcttt cctgcgttat cccctgattc tgtggataac cgtattaccg cctttgagtg 4440

agctgatacc gctcgccgca gccgaacgac cgagcgcagc gagtcagtga gcgaggaagc 4500

ggaagagcgc ccaatacgca aaccgcctct ccccgcgcgt tggccgattc attaatgcag 4560

ctggcacgac aggtttcccg actggaaagc gggcagtgag cgcaacgcaa ttaatgtgag 4620

ttagctcact cattaggcac cccaggcttt acactttatg cttccggctc gtatgttgtg 4680

tggaattgtg agcggataa 4699

<210> 86
<211> 4653
<212> DNA
<213> Artificial Sequence

<220>
<223> Description of Artificial Sequence: Synthetic
      polynucleotide

<400> 86
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtacta tagtccacgg ggttggggtt gcgccttttc 240

caaggcagcc ctgggtttgc gcagggacgc ggctgctctg ggcgtggttc cgggaaacgc 300

agcggcgccg accctgggtc tcgcacattc ttcacgtccg ttcgcagcgt cacccggatc 360

ttcgccgcta cccttgtggg ccccccggcg acgcttcctc gtccgcccct aagtcgggaa 420

ggttccttgc ggttcgcggc gtgccggacg tgacaaacgg aagccgcacg tctcactagt 480

accctcgcag acggacagcg ccagggagca atggcagcgc gccgaccgcg atgggctgtg 540

gccaatagcg gctgctcagc agggcgcgcc gagagcagcg gccgggaagg ggcggtgcgg 600

gaggcggggt gtggggcggt agtgtgggcc ctgttcctgc ccgcgcggtg ttccgcattc 660

tgcaagcctc cggagcgcac gtcggcagtc ggctccctcg ttgaccgaat caccgacctc 720

tctccccacg ggagcaagct tgaactgaaa aaccagaaag ttaactggta agtttagtct 780

ttttgtcttt tatttcaggt cccggatccg gtggtggtgc aaatcaaaga actgctcctc 840

agtggatgtt gcctttactt ctaggcctgt acggaagtgt tacttctgct ctaaaagctg 900

cggaattgta cccgcggccg atccaccggt atgtggacac tcgggcgacg cgccgtggct 960

gggctgcttg cttccccaag tcccgcacag gcgcaaaccc tcacaagggt cccacggcca 1020

gctgaattgg ccccgctgtg cggtcggagg ggcctgcgaa cagatataga cgcaacgtgc 1080

acccccagaa gagcaagcag caaccagaga ggcctcaatc agatttggaa cgtcaagaaa 1140

cagagtgtct atctgatgaa cctccgcaaa tcagggaccc ttggtcatcc gggatctctt 1200

gatgaaacga cctatgaacg gctggctgag gagacgcttg actccctggc agagttcttc 1260

gaggatctgg ctgataagcc atacacgttc gaggactatg atgtttcttt tggttctggc 1320

gtcctgacgg tgaaactcgg aggggatctg ggcacttacg ttatcaacaa acagactccc 1380

aataaacaga tttggttgtc tagtcctagc agtggcccaa aacgctatga ctggacgggc 1440

aagaactggg tgtactccca cgatggagtt agcctgcacg aacttctggc cgctgagctt 1500

actaaagcgc tgaagactaa gctggatctc tccagcctcg catacagcgg caaagacgcc 1560

tagctcgagg acggggtgaa ctacgcctga ggatccgatc tttttccctc tgccaaaaat 1620

tatggggaca tcatgaagcc ccttgagcat ctgacttctg gctaataaag gaaatttatt 1680

ttcattgcaa tagtgtgttg gaattttttg tgtctctcac tcggcctagg tagataagta 1740

gcatggcggg ttaatcatta actacaagga acccctagtg atggagttgg ccactccctc 1800

tctgcgcgct cgctcgctca ctgaggccgg gcgaccaaag gtcgcccgac gcccgggctt 1860

tgcccgggcg gcctcagtga gcgagcgagc gcgcagcctt aattaaccta attcactggc 1920

cgtcgtttta caacgtcgtg actgggaaaa ccctggcgtt acccaactta atcgccttgc 1980

agcacatccc cctttcgcca gctggcgtaa tagcgaagag gcccgcaccg atcgcccttc 2040

ccaacagttg cgcagcctga atggcgaatg ggacgcgccc tgtagcggcg cattaagcgc 2100

ggcgggtgtg gtggttacgc gcagcgtgac cgctacactt gccagcgccc tagcgcccgc 2160

tcctttcgct ttcttccctt cctttctcgc cacgttcgcc ggctttcccc gtcaagctct 2220

aaatcggggg ctccctttag ggttccgatt tagtgcttta cggcacctcg accccaaaaa 2280

acttgattag ggtgatggtt cacgtagtgg gccatcgccc tgatagacgg tttttcgccc 2340

tttgacgttg gagtccacgt tctttaatag tggactcttg ttccaaactg gaacaacact 2400

caaccctatc tcggtctatt cttttgattt ataagggatt ttgccgattt cggcctattg 2460

gttaaaaaat gagctgattt aacaaaaatt taacgcgaat tttaacaaaa tattaacgct 2520

tacaatttag gtggcacttt tcggggaaat gtgcgcggaa cccctatttg tttatttttc 2580

taaatacatt caaatatgta tccgctcatg agacaataac cctgataaat gcttcaataa 2640

tattgaaaaa ggaagagtat gagtattcaa catttccgtg tcgcccttat tccctttttt 2700

gcggcatttt gccttcctgt ttttgctcac ccagaaacgc tggtgaaagt aaaagatgct 2760

gaagatcagt tgggtgcacg agtgggttac atcgaactgg atctcaacag cggtaagatc 2820

cttgagagtt ttcgccccga agaacgtttt ccaatgatga gcacttttaa agttctgcta 2880

tgtggcgcgg tattatcccg tattgacgcc gggcaagagc aactcggtcg ccgcatacac 2940

tattctcaga atgacttggt tgagtactca ccagtcacag aaaagcatct tacggatggc 3000

atgacagtaa gagaattatg cagtgctgcc ataaccatga gtgataacac tgcggccaac 3060

ttacttctga caacgatcgg aggaccgaag gagctaaccg cttttttgca caacatgggg 3120

gatcatgtaa ctcgccttga tcgttgggaa ccggagctga atgaagccat accaaacgac 3180

gagcgtgaca ccacgatgcc tgtagcaatg gcaacaacgt tgcgcaaact attaactggc 3240

gaactactta ctctagcttc ccggcaacaa ttaatagact ggatggaggc ggataaagtt 3300

gcaggaccac ttctgcgctc ggcccttccg gctggctggt ttattgctga taaatctgga 3360

gccggtgagc gtgggtctcg cggtatcatt gcagcactgg ggccagatgg taagccctcc 3420

cgtatcgtag ttatctacac gacggggagt caggcaacta tggatgaacg aaatagacag 3480

atcgctgaga taggtgcctc actgattaag cattggtaac tgtcagacca agtttactca 3540

tatatacttt agattgattt aaaacttcat ttttaattta aaaggatcta ggtgaagatc 3600

ctttttgata atctcatgac caaaatccct taacgtgagt tttcgttcca ctgagcgtca 3660

gaccccgtag aaaagatcaa aggatcttct tgagatcctt tttttctgcg cgtaatctgc 3720

tgcttgcaaa caaaaaaacc accgctacca gcggtggttt gtttgccgga tcaagagcta 3780

ccaactcttt ttccgaaggt aactggcttc agcagagcgc agataccaaa tactgttctt 3840

ctagtgtagc cgtagttagg ccaccacttc aagaactctg tagcaccgcc tacatacctc 3900

gctctgctaa tcctgttacc agtggctgct gccagtggcg ataagtcgtg tcttaccggg 3960

ttggactcaa gacgatagtt accggataag gcgcagcggt cgggctgaac ggggggttcg 4020

tgcacacagc ccagcttgga gcgaacgacc tacaccgaac tgagatacct acagcgtgag 4080

ctatgagaaa gcgccacgct tcccgaaggg agaaaggcgg acaggtatcc ggtaagcggc 4140

agggtcggaa caggagagcg cacgagggag cttccagggg gaaacgcctg gtatctttat 4200

agtcctgtcg ggtttcgcca cctctgactt gagcgtcgat ttttgtgatg ctcgtcaggg 4260

gggcggagcc tatggaaaaa cgccagcaac gcggcctttt tacggttcct ggccttttgc 4320

tggccttttg ctcacatgtt ctttcctgcg ttatcccctg attctgtgga taaccgtatt 4380

accgcctttg agtgagctga taccgctcgc cgcagccgaa cgaccgagcg cagcgagtca 4440

gtgagcgagg aagcggaaga gcgcccaata cgcaaaccgc ctctccccgc gcgttggccg 4500

attcattaat gcagctggca cgacaggttt cccgactgga aagcgggcag tgagcgcaac 4560

gcaattaatg tgagttagct cactcattag gcaccccagg ctttacactt tatgcttccg 4620

gctcgtatgt tgtgtggaat tgtgagcgga taa 4653

<210> 87

<211> 4653
<212> DNA
<213> Artificial Sequence

<220>
<223> Description of Artificial Sequence: Synthetic
      polynucleotide

<400> 87
caatttcaca caggaaacag ctatgaccat gattacgcca gatttaatta aggccttaat      60

taggctgcgc gctcgctcgc tcactgaggc cgcccgggca aagcccgggc gtcgggcgac     120

ctttggtcgc ccggcctcag tgagcgagcg agcgcgcaga gagggagtgt agccatgctc     180

taggaagatc aattcaattc acgcgtacta tagtccacgg ggttggggtt gcgccttttc     240

caaggcagcc ctgggtttgc gcagggacgc ggctgctctg ggcgtggttc cgggaaacgc     300

agcggcgccg accctgggtc tcgcacattc ttcacgtccg ttcgcagcgt cacccggatc     360

ttcgccgcta cccttgtggg ccccccggcg acgcttcctc gtccgcccct aagtcgggaa     420

ggttccttgc ggttcgcggc gtgccggacg tgacaaacgg aagccgcacg tctcactagt     480

accctcgcag acggacagcg ccagggagca atggcagcgc gccgaccgcg atgggctgtg     540

gccaatagcg gctgctcagc agggcgcgcc gagagcagcg gccgggaagg ggcggtgcgg     600

gaggcggggt gtggggcggt agtgtgggcc ctgttcctgc ccgcgcggtg ttccgcattc     660

tgcaagcctc cggagcgcac gtcggcagtc ggctccctcg ttgaccgaat caccgacctc     720

tctccccacg ggagcaagct tgaactgaaa aaccagaaag ttaactggta agtttagtct     780

ttttgtcttt tatttcaggt cccggatccg gtggtggtgc aaatcaaaga actgctcctc     840

agtggatgtt gcctttactt ctaggcctgt acggaagtgt tacttctgct ctaaaagctg     900

cggaattgta cccgcggccg atccaccggt atgtggactc tgggtcgacg cgccgtagcc     960

ggtctcttgg cctccccatc ccctgcgcag gcacagaccc ttacacgagt tccacggccc    1020

gctgaactgg caccactctg cggacgccgg ggcctgagaa ccgatattga cgctacgtgc    1080

actccccgac gagcctcttc taatcagagg gggctcaacc agatctggaa cgtgaagaag    1140

cagtcagtct acttgatgaa tctgagaaag agcgggaccc ttgggcaccc cgggtctctc    1200

gacgagacta catacgagag gctggccgag gagactttgg actctctcgc cgaattcttc    1260

gaggatttgg cagacaagcc gtacaccttt gaagactacg atgtctcatt cggatcaggg    1320

gtgctgacgg ttaaactggg gggcgatctc ggaacctacg tcataaacaa acagacaccc    1380

aataagcaga tctggttgtc tagcccatca tccggtccta aacggtacga ctggaccggc    1440

aagaactggg tctactccca tgacggagtc agtcttcatg aactgcttgc cgcagagctg    1500

accaaggctc ttaagacgaa actggatctc tctagcctgg cctactccgg gaaggatgca    1560

tagctcgagg acggggtgaa ctacgcctga ggatccgatc tttttccctc tgccaaaaat    1620

tatggggaca tcatgaagcc ccttgagcat ctgacttctg gctaataaag gaaatttatt 1680

ttcattgcaa tagtgtgttg gaattttttg tgtctctcac tcggcctagg tagataagta 1740

gcatggcggg ttaatcatta actacaagga acccctagtg atggagttgg ccactccctc 1800

tctgcgcgct cgctcgctca ctgaggccgg gcgaccaaag gtcgcccgac gcccgggctt 1860

tgcccgggcg gcctcagtga gcgagcgagc gcgcagcctt aattaaccta attcactggc 1920

cgtcgtttta caacgtcgtg actgggaaaa ccctggcgtt acccaactta atcgccttgc 1980

agcacatccc cctttcgcca gctggcgtaa tagcgaagag gcccgcaccg atcgcccttc 2040

ccaacagttg cgcagcctga atggcgaatg ggacgcgccc tgtagcggcg cattaagcgc 2100

ggcgggtgtg gtggttacgc gcagcgtgac cgctacactt gccagcgccc tagcgcccgc 2160

tcctttcgct ttcttccctt cctttctcgc cacgttcgcc ggctttcccc gtcaagctct 2220

aaatcggggg ctccctttag ggttccgatt tagtgcttta cggcacctcg accccaaaaa 2280

acttgattag ggtgatggtt cacgtagtgg gccatcgccc tgatagacgg tttttcgccc 2340

tttgacgttg gagtccacgt tctttaatag tggactcttg ttccaaactg gaacaacact 2400

caaccctatc tcggtctatt cttttgattt ataagggatt ttgccgattt cggcctattg 2460

gttaaaaaat gagctgattt aacaaaaatt taacgcgaat tttaacaaaa tattaacgct 2520

tacaatttag gtggcacttt tcggggaaat gtgcgcggaa cccctatttg tttatttttc 2580

taaatacatt caaatatgta tccgctcatg agacaataac cctgataaat gcttcaataa 2640

tattgaaaaa ggaagagtat gagtattcaa catttccgtg tcgcccttat tccctttttt 2700

gcggcatttt gccttcctgt ttttgctcac ccagaaacgc tggtgaaagt aaaagatgct 2760

gaagatcagt tgggtgcacg agtgggttac atcgaactgg atctcaacag cggtaagatc 2820

cttgagagtt ttcgccccga agaacgtttt ccaatgatga gcacttttaa agttctgcta 2880

tgtggcgcgg tattatcccg tattgacgcc gggcaagagc aactcggtcg ccgcatacac 2940

tattctcaga atgacttggt tgagtactca ccagtcacag aaaagcatct tacggatggc 3000

atgacagtaa gagaattatg cagtgctgcc ataaccatga gtgataacac tgcggccaac 3060

ttacttctga caacgatcgg aggaccgaag gagctaaccg cttttttgca caacatgggg 3120

gatcatgtaa ctcgccttga tcgttgggaa ccggagctga atgaagccat accaaacgac 3180

gagcgtgaca ccacgatgcc tgtagcaatg gcaacaacgt tgcgcaaact attaactggc 3240

gaactactta ctctagcttc ccggcaacaa ttaatagact ggatggaggc ggataaagtt 3300

gcaggaccac ttctgcgctc ggcccttccg gctggctggt ttattgctga taaatctgga 3360

gccggtgagc gtgggtctcg cggtatcatt gcagcactgg ggccagatgg taagccctcc 3420

cgtatcgtag ttatctacac gacggggagt caggcaacta tggatgaacg aaatagacag 3480

atcgctgaga taggtgcctc actgattaag cattggtaac tgtcagacca agtttactca 3540

tatatacttt agattgattt aaaacttcat ttttaattta aaaggatcta ggtgaagatc 3600

ctttttgata atctcatgac caaaatccct taacgtgagt tttcgttcca ctgagcgtca 3660

gaccccgtag aaaagatcaa aggatcttct tgagatcctt tttttctgcg cgtaatctgc 3720

tgcttgcaaa caaaaaaacc accgctacca gcggtggttt gtttgccgga tcaagagcta 3780

ccaactcttt ttccgaaggt aactggcttc agcagagcgc agataccaaa tactgttctt 3840

ctagtgtagc cgtagttagg ccaccacttc aagaactctg tagcaccgcc tacatacctc 3900

gctctgctaa tcctgttacc agtggctgct gccagtggcg ataagtcgtg tcttaccggg 3960

ttggactcaa gacgatagtt accggataag gcgcagcggt cgggctgaac ggggggttcg 4020

tgcacacagc ccagcttgga gcgaacgacc tacaccgaac tgagatacct acagcgtgag 4080

ctatgagaaa gcgccacgct tcccgaaggg agaaaggcgg acaggtatcc ggtaagcggc 4140

agggtcggaa caggagagcg cacgagggag cttccagggg gaaacgcctg gtatctttat 4200

agtcctgtcg ggtttcgcca cctctgactt gagcgtcgat ttttgtgatg ctcgtcaggg 4260

gggcggagcc tatggaaaaa cgccagcaac gcggcctttt tacggttcct ggccttttgc 4320

tggccttttg ctcacatgtt ctttcctgcg ttatcccctg attctgtgga taaccgtatt 4380

accgcctttg agtgagctga taccgctcgc cgcagccgaa cgaccgagcg cagcgagtca 4440

gtgagcgagg aagcggaaga gcgcccaata cgcaaaccgc ctctccccgc gcgttggccg 4500

attcattaat gcagctggca cgacaggttt cccgactgga aagcgggcag tgagcgcaac 4560

gcaattaatg tgagttagct cactcattag gcaccccagg ctttacactt tatgcttccg 4620

gctcgtatgt tgtgtggaat tgtgagcgga taa 4653

<210> 88
<211> 4653
<212> DNA
<213> Artificial Sequence

<220>
<223> Description of Artificial Sequence: Synthetic
      polynucleotide

<400> 88
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtacta tagtccacgg ggttggggtt gcgccttttc 240

caaggcagcc ctgggtttgc gcagggacgc ggctgctctg ggcgtggttc cgggaaacgc 300

agcggcgccg accctgggtc tcgcacattc ttcacgtccg ttcgcagcgt cacccggatc 360

ttcgccgcta cccttgtggg ccccccggcg acgcttcctc gtccgcccct aagtcgggaa 420

ggttccttgc ggttcgcggc gtgccggacg tgacaaacgg aagccgcacg tctcactagt 480

accctcgcag acggacagcg ccagggagca atggcagcgc gccgaccgcg atgggctgtg 540

gccaatagcg gctgctcagc agggcgcgcc gagagcagcg gccgggaagg ggcggtgcgg 600

gaggcggggt gtggggcggt agtgtgggcc ctgttcctgc ccgcgcggtg ttccgcattc 660

tgcaagcctc cggagcgcac gtcggcagtc ggctccctcg ttgaccgaat caccgacctc 720

tctccccacg ggagcaagct tgaactgaaa aaccagaaag ttaactggta agtttagtct 780

ttttgtcttt tatttcaggt cccggatccg gtggtggtgc aaatcaaaga actgctcctc 840

agtggatgtt gcctttactt ctaggcctgt acggaagtgt tacttctgct ctaaaagctg 900

cggaattgta cccgcggccg atccaccggt atgtggactc tcgggcgacg cgccgtagcc 960

ggtctcctgg cttccccaag ccctgcacag gcacagaccc tcacacgggt cccacggcca 1020

gctgaattgg ccccactctg cggtcgccgg ggcctgcgaa ccgatataga cgcaacgtgc 1080

actccccgac gagcaagttc caaccagaga gggctcaacc agatttggaa cgtcaagaag 1140

cagagtgtct atttgatgaa tctgagaaaa tccgggaccc ttgggcaccc cggatctctt 1200

gatgaaacta cctatgaaag gctggccgag gagacgctgg actctctggc agagttcttc 1260

gaggatctgg cagacaagcc atacacgttt gaggactatg atgtctcttt tggatctggc 1320

gtcctgacgg ttaaactggg aggggatctc ggaacctacg ttatcaacaa acagactccc 1380

aataagcaga tctggttgtc tagtccatcc agtggtccta aacgctatga ctggacgggc 1440

aagaactggg tgtactccca cgacggagtt agccttcatg aactgctggc cgcagagctg 1500

actaaagctc ttaagactaa actggatctc tctagcctgg cctactccgg gaaagatgcc 1560

tagctcgagg acggggtgaa ctacgcctga ggatccgatc tttttccctc tgccaaaaat 1620

tatggggaca tcatgaagcc ccttgagcat ctgacttctg gctaataaag gaaatttatt 1680

ttcattgcaa tagtgtgttg gaattttttg tgtctctcac tcggcctagg tagataagta 1740

gcatggcggg ttaatcatta actacaagga acccctagtg atggagttgg ccactccctc 1800

tctgcgcgct cgctcgctca ctgaggccgg gcgaccaaag gtcgcccgac gcccgggctt 1860

tgcccgggcg gcctcagtga gcgagcgagc gcgcagcctt aattaaccta attcactggc 1920

cgtcgtttta caacgtcgtg actgggaaaa ccctggcgtt acccaactta atcgccttgc 1980

agcacatccc cctttcgcca gctggcgtaa tagcgaagag gcccgcaccg atcgcccttc 2040

ccaacagttg cgcagcctga atggcgaatg ggacgcgccc tgtagcggcg cattaagcgc 2100

ggcgggtgtg gtggttacgc gcagcgtgac cgctacactt gccagcgccc tagcgcccgc 2160

tcctttcgct ttcttccctt cctttctcgc cacgttcgcc ggctttcccc gtcaagctct 2220

aaatcggggg ctccctttag ggttccgatt tagtgcttta cggcacctcg accccaaaaa 2280

acttgattag ggtgatggtt cacgtagtgg gccatcgccc tgatagacgg tttttcgccc 2340

tttgacgttg gagtccacgt tctttaatag tggactcttg ttccaaactg gaacaacact 2400

caaccctatc tcggtctatt cttttgattt ataagggatt ttgccgattt cggcctattg 2460

gttaaaaaat gagctgattt aacaaaaatt taacgcgaat tttaacaaaa tattaacgct 2520

tacaatttag gtggcacttt tcggggaaat gtgcgcggaa cccctatttg tttatttttc 2580

taaatacatt caaatatgta tccgctcatg agacaataac cctgataaat gcttcaataa 2640

tattgaaaaa ggaagagtat gagtattcaa catttccgtg tcgcccttat tccctttttt 2700

gcggcatttt gccttcctgt ttttgctcac ccagaaacgc tggtgaaagt aaaagatgct 2760

gaagatcagt tgggtgcacg agtgggttac atcgaactgg atctcaacag cggtaagatc 2820

cttgagagtt ttcgccccga agaacgtttt ccaatgatga gcacttttaa agttctgcta 2880

tgtggcgcgg tattatcccg tattgacgcc gggcaagagc aactcggtcg ccgcatacac 2940

tattctcaga atgacttggt tgagtactca ccagtcacag aaaagcatct tacggatggc 3000

atgacagtaa gagaattatg cagtgctgcc ataaccatga gtgataacac tgcggccaac 3060

ttacttctga caacgatcgg aggaccgaag gagctaaccg cttttttgca caacatgggg 3120

gatcatgtaa ctcgccttga tcgttgggaa ccggagctga atgaagccat accaaacgac 3180

gagcgtgaca ccacgatgcc tgtagcaatg gcaacaacgt tgcgcaaact attaactggc 3240

gaactactta ctctagcttc ccggcaacaa ttaatagact ggatggaggc ggataaagtt 3300

gcaggaccac ttctgcgctc ggcccttccg gctggctggt ttattgctga taaatctgga 3360

gccggtgagc gtgggtctcg cggtatcatt gcagcactgg ggccagatgg taagccctcc 3420

cgtatcgtag ttatctacac gacggggagt caggcaacta tggatgaacg aaatagacag 3480

atcgctgaga taggtgcctc actgattaag cattggtaac tgtcagacca agtttactca 3540

tatatacttt agattgattt aaaacttcat ttttaattta aaaggatcta ggtgaagatc 3600

ctttttgata atctcatgac caaaatccct taacgtgagt tttcgttcca ctgagcgtca 3660

gaccccgtag aaaagatcaa aggatcttct tgagatcctt tttttctgcg cgtaatctgc 3720

tgcttgcaaa caaaaaaacc accgctacca gcggtggttt gtttgccgga tcaagagcta 3780

ccaactcttt ttccgaaggt aactggcttc agcagagcgc agataccaaa tactgttctt 3840

ctagtgtagc cgtagttagg ccaccacttc aagaactctg tagcaccgcc tacatacctc 3900

gctctgctaa tcctgttacc agtggctgct gccagtggcg ataagtcgtg tcttaccggg 3960

ttggactcaa gacgatagtt accggataag gcgcagcggt cgggctgaac ggggggttcg 4020

tgcacacagc ccagcttgga gcgaacgacc tacaccgaac tgagatacct acagcgtgag 4080

ctatgagaaa gcgccacgct tcccgaaggg agaaaggcgg acaggtatcc ggtaagcggc 4140

agggtcggaa caggagagcg cacgagggag cttccagggg gaaacgcctg gtatctttat 4200

agtcctgtcg ggtttcgcca cctctgactt gagcgtcgat ttttgtgatg ctcgtcaggg 4260

gggcggagcc tatggaaaaa cgccagcaac gcggcctttt tacggttcct ggccttttgc 4320

tggccttttg ctcacatgtt ctttcctgcg ttatcccctg attctgtgga taaccgtatt 4380

accgcctttg agtgagctga taccgctcgc cgcagccgaa cgaccgagcg cagcgagtca 4440

gtgagcgagg aagcggaaga gcgcccaata cgcaaaccgc ctctccccgc gcgttggccg 4500

attcattaat gcagctggca cgacaggttt cccgactgga aagcgggcag tgagcgcaac 4560

gcaattaatg tgagttagct cactcattag gcaccccagg ctttacactt tatgcttccg 4620

gctcgtatgt tgtgtggaat tgtgagcgga taa 4653

<210> 89
<211> 4653
<212> DNA
<213> Artificial Sequence

<220>
<223> Description of Artificial Sequence: Synthetic
      polynucleotide

<400> 89
caatttcaca caggaaacag ctatgaccat gattacgcca gatttaatta aggccttaat 60

taggctgcgc gctcgctcgc tcactgaggc cgcccgggca aagcccgggc gtcgggcgac 120

ctttggtcgc ccggcctcag tgagcgagcg agcgcgcaga gagggagtgt agccatgctc 180

taggaagatc aattcaattc acgcgtacta tagtccacgg ggttggggtt gcgccttttc 240

caaggcagcc ctgggtttgc gcagggacgc ggctgctctg ggcgtggttc cgggaaacgc 300

agcggcgccg accctgggtc tcgcacattc ttcacgtccg ttcgcagcgt cacccggatc 360

ttcgccgcta cccttgtggg ccccccggcg acgcttcctc gtccgcccct aagtcgggaa 420

ggttccttgc ggttcgcggc gtgccggacg tgacaaacgg aagccgcacg tctcactagt 480

accctcgcag acggacagcg ccagggagca atggcagcgc gccgaccgcg atgggctgtg 540

gccaatagcg gctgctcagc agggcgcgcc gagagcagcg gccgggaagg ggcggtgcgg 600

gaggcggggt gtggggcggt agtgtgggcc ctgttcctgc ccgcgcggtg ttccgcattc 660

tgcaagcctc cggagcgcac gtcggcagtc ggctccctcg ttgaccgaat caccgacctc 720

tctccccacg ggagcaagct tgaactgaaa aaccagaaag ttaactggta agtttagtct 780

ttttgtcttt tatttcaggt cccggatccg gtggtggtgc aaatcaaaga actgctcctc 840

agtggatgtt gcctttactt ctaggcctgt acggaagtgt tacttctgct ctaaaagctg 900

cggaattgta cccgcggccg atccaccggt atgtggactc tggggcgacg cgccgtggcc 960

gggctcctgg catccccatc ccctgcgcag gcgcaaaccc tcacacgggt tccacggcca 1020

gctgaactgg ccccactgtg cggccggcgg ggcctgagaa cagatattga cgcaacgtgc 1080

actcccagaa gagccagcag caaccagagg gggttgaatc agatctggaa cgtcaagaag 1140

cagtcagtgt acctgatgaa tctccgaaaa agcgggaccc ttggtcaccc cggatctctc 1200

gatgaaacca catatgaacg gctggccgag gaaacgctgg actccctggc cgagttcttc 1260

gaggacctgg cagacaagcc gtacaccttc gaagactatg atgtttcatt cggaagtggc 1320

gtgctgacgg tcaaactggg aggcgatctg ggaacctacg ttataaacaa acagactccc 1380

aataaacaga tctggttgtc tagtccatcc agcggtccta aacgctatga ctggacgggc 1440

aagaactggg tctactccca cgacggagtt agccttcacg aactgttggc cgcagagctg 1500

acaaaggcgc ttaagacgaa actggatctc tctagcctgg cctactccgg caaagatgcc 1560

tagctcgagg acggggtgaa ctacgcctga ggatccgatc tttttccctc tgccaaaaat 1620

tatggggaca tcatgaagcc ccttgagcat ctgacttctg gctaataaag gaaatttatt 1680

ttcattgcaa tagtgtgttg gaattttttg tgtctctcac tcggcctagg tagataagta 1740

gcatggcggg ttaatcatta actacaagga acccctagtg atggagttgg ccactccctc 1800

tctgcgcgct cgctcgctca ctgaggccgg gcgaccaaag gtcgcccgac gcccgggctt 1860

tgcccgggcg gcctcagtga gcgagcgagc gcgcagcctt aattaaccta attcactggc 1920

cgtcgtttta caacgtcgtg actgggaaaa ccctggcgtt acccaactta atcgccttgc 1980

agcacatccc cctttcgcca gctggcgtaa tagcgaagag gcccgcaccg atcgcccttc 2040

ccaacagttg cgcagcctga atggcgaatg ggacgcgccc tgtagcggcg cattaagcgc 2100

ggcgggtgtg gtggttacgc gcagcgtgac cgctacactt gccagcgccc tagcgcccgc 2160

tcctttcgct ttcttccctt cctttctcgc cacgttcgcc ggctttcccc gtcaagctct 2220

aaatcggggg ctccctttag ggttccgatt tagtgcttta cggcacctcg accccaaaaa 2280

acttgattag ggtgatggtt cacgtagtgg gccatcgccc tgatagacgg tttttcgccc 2340

tttgacgttg gagtccacgt tctttaatag tggactcttg ttccaaactg gaacaacact 2400

caaccctatc tcggtctatt cttttgattt ataagggatt ttgccgattt cggcctattg 2460

gttaaaaaat gagctgattt aacaaaaatt taacgcgaat tttaacaaaa tattaacgct 2520

tacaatttag gtggcacttt tcggggaaat gtgcgcggaa cccctatttg tttatttttc 2580

taaatacatt caaatatgta tccgctcatg agacaataac cctgataaat gcttcaataa 2640

tattgaaaaa ggaagagtat gagtattcaa catttccgtg tcgcccttat tccctttttt 2700

gcggcatttt gccttcctgt ttttgctcac ccagaaacgc tggtgaaagt aaaagatgct 2760

gaagatcagt tgggtgcacg agtgggttac atcgaactgg atctcaacag cggtaagatc 2820

cttgagagtt ttcgccccga agaacgtttt ccaatgatga gcacttttaa agttctgcta 2880

tgtggcgcgg tattatcccg tattgacgcc gggcaagagc aactcggtcg ccgcatacac 2940

tattctcaga atgacttggt tgagtactca ccagtcacag aaaagcatct tacggatggc 3000

atgacagtaa gagaattatg cagtgctgcc ataaccatga gtgataacac tgcggccaac 3060

ttacttctga caacgatcgg aggaccgaag gagctaaccg cttttttgca caacatgggg 3120

gatcatgtaa ctcgccttga tcgttgggaa ccggagctga atgaagccat accaaacgac 3180

gagcgtgaca ccacgatgcc tgtagcaatg gcaacaacgt tgcgcaaact attaactggc 3240

gaactactta ctctagcttc ccggcaacaa ttaatagact ggatggaggc ggataaagtt 3300

gcaggaccac ttctgcgctc ggcccttccg gctggctggt ttattgctga taaatctgga 3360

gccggtgagc gtgggtctcg cggtatcatt gcagcactgg ggccagatgg taagccctcc 3420

cgtatcgtag ttatctacac gacggggagt caggcaacta tggatgaacg aaatagacag 3480

atcgctgaga taggtgcctc actgattaag cattggtaac tgtcagacca agtttactca 3540

tatatacttt agattgattt aaaacttcat ttttaattta aaaggatcta ggtgaagatc 3600

ctttttgata atctcatgac caaaatccct taacgtgagt tttcgttcca ctgagcgtca 3660

gaccccgtag aaaagatcaa aggatcttct tgagatcctt tttttctgcg cgtaatctgc 3720

tgcttgcaaa caaaaaaacc accgctacca gcggtggttt gtttgccgga tcaagagcta 3780

ccaactcttt ttccgaaggt aactggcttc agcagagcgc agataccaaa tactgttctt 3840

ctagtgtagc cgtagttagg ccaccacttc aagaactctg tagcaccgcc tacatacctc 3900

gctctgctaa tcctgttacc agtggctgct gccagtggcg ataagtcgtg tcttaccggg 3960

ttggactcaa gacgatagtt accggataag gcgcagcggt cgggctgaac ggggggttcg 4020

tgcacacagc ccagcttgga gcgaacgacc tacaccgaac tgagatacct acagcgtgag 4080

ctatgagaaa gcgccacgct tcccgaaggg agaaaggcgg acaggtatcc ggtaagcggc 4140

agggtcggaa caggagagcg cacgagggag cttccagggg gaaacgcctg gtatctttat 4200

agtcctgtcg ggtttcgcca cctctgactt gagcgtcgat ttttgtgatg ctcgtcaggg 4260

gggcggagcc tatggaaaaa cgccagcaac gcggcctttt tacggttcct ggccttttgc 4320

tggccttttg ctcacatgtt ctttcctgcg ttatcccctg attctgtgga taaccgtatt 4380

accgcctttg agtgagctga taccgctcgc cgcagccgaa cgaccgagcg cagcgagtca 4440

gtgagcgagg aagcggaaga gcgcccaata cgcaaaccgc ctctccccgc gcgttggccg 4500

attcattaat gcagctggca cgacaggttt cccgactgga aagcgggcag tgagcgcaac 4560

gcaattaatg tgagttagct cactcattag gcaccccagg ctttacactt tatgcttccg 4620

gctcgtatgt tgtgtggaat tgtgagcgga taa 4653

<210> 90
<211> 4883
<212> DNA
<213> Artificial Sequence

<220>
<223> Description of Artificial Sequence: Synthetic
      polynucleotide

<400> 90
gccttaatta ggctgcgcgc tcgctcgctc actgaggccg cccgggcaaa gcccgggcgt      60

cgggcgacct ttggtcgccc ggcctcagtg agcgagcgag cgcgcagaga gggagtggcc     120

aactccatca ctaggggttc cttgtagtta atgattaacc cgccatgcta cttatctacc     180

agggtaatgg ggatcctcta gaactatagc tagtcgacat tgattattga ctagttatta     240

atagtaatca attacggggt cattagttca tagcccatat atggagttcc gcgttacata     300

acttacggta aatggcccgc ctggctgacc gcccaacgac ccccgcccat tgacgtcaat     360

aatgacgtat gttcccatag taacgccaat agggactttc cattgacgtc aatgggtgga     420

gtatttacgg taaactgccc acttggcagt acatcaagtg tatcatatgc caagtacgcc     480

ccctattgac gtcaatgacg gtaaatggcc cgcctggcat tatgcccagt acatgacctt     540

atgggacttt cctacttggc agtacatcta cgtattagtc atcgctatta ccatgtcgag     600

gccacgttct gcttcactct ccccatctcc cccccctccc cacccccaat tttgtattta     660

tttatttttt aattattttg tgcagcgatg ggggcggggg gggggggcgc gcgccaggcg     720

gggcggggcg gggcgagggg cggggcgggg cgaggcggag aggtgcggcg gcagccaatc     780

agagcggcgc gctccgaaag tttcctttta tggcgaggcg gcggcggcgg cggccctata     840

aaaagcgaag cgcgcggcgg gcgggagcaa gcttgaactg aaaaaccaga aagttaactg     900

gtaagtttag tctttttgtc ttttatttca ggtcccggat ccggtggtgg tgcaaatcaa     960

agaactgctc ctcagtggat gttgccttta cttctaggcc tgtacggaag tgttacttct    1020

gctctaaaag ctgcggaatt gtacccgcgg ccgatccacc ggtatgtgga ctctcgggcg    1080

ccgcgcagta gccggcctcc tggcgtcacc cagcccagcc caggcccaga ccctcacccg    1140

ggtcccgcgg ccggcagagt tggccccact ctgcggccgc cgtggcctgc gcaccgacat    1200

cgatgcgacc tgcacgcccc gccgcgcaag ttcgaaccaa cgtggcctca accagatttg    1260

gaatgtcaaa aagcagagtg tctatttgat gaatttgagg aaatctggaa ctttgggcca    1320

cccaggctct ctagatgaga ccacctatga aagactagca gaggaaacgc tggactcttt    1380

agcagagttt tttgaagacc ttgcagacaa gccatacacg tttgaggact atgatgtctc    1440

ctttgggagt ggtgtcttaa ctgtcaaact gggtggagat ctaggaacct atgtgatcaa    1500

caagcagacg ccaaacaagc aaatctggct atcttctcca tccagtggac ctaagcgtta    1560

tgactggact gggaaaaact gggtgtactc ccacgacggc gtgtccctcc atgagctgct    1620

ggccgcagag ctcactaaag ccttaaaaac caaactggac ttgtcttcct tggcctattc    1680

cggaaaagat gcttgactcg aggacggggt gaactacgcc tgaggatccg atctttttcc    1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 91
<211> 4829
<212> DNA
<213> Artificial Sequence

<220>
<223> Description of Artificial Sequence: Synthetic
      polynucleotide

<400> 91
ctgcgcgctc gctcgctcac tgaggccgcc cgggcaaagc ccgggcgtcg ggcgaccttt 60

ggtcgcccgg cctcagtgag cgagcgagcg cgcagagagg gagtgtagcc atgctctagg 120

aagatcaatt cggtacaatt cacgcgtcga cattgattat tgactagtta ttaatagtaa 180

tcaattacgg ggtcattagt tcatagccca tatatggagt tccgcgttac ataacttacg 240

gtaaatggcc cgcctggctg accgcccaac gacccccgcc cattgacgtc aataatgacg 300

tatgttccca tagtaacgcc aatagggact ttccattgac gtcaatgggt ggagtattta 360

cggtaaactg cccacttggc agtacatcaa gtgtatcata tgccaagtac gccccctatt 420

gacgtcaatg acggtaaatg gcccgcctgg cattatgccc agtacatgac cttatgggac 480

tttcctactt ggcagtacat ctacgtatta gtcatcgcta ttaccatgtc gaggccacgt 540

tctgcttcac tctccccatc tcccccccct ccccaccccc aattttgtat ttatttattt 600

tttaattatt ttgtgcagcg atgggggcgg gggggggggg cgcgcgccag gcggggcggg 660

gcggggcgag gggcggggcg gggcgaggcg gagaggtgcg gcggcagcca atcagagcgg 720

cgcgctccga aagtttcctt ttatggcgag gcggcggcgg cggcggccct ataaaaagcg 780

aagcgcgcgg cgggcgggag caagcttgaa ctgaaaaacc agaaagttaa ctggtaagtt 840

tagtcttttt gtcttttatt tcaggtcccg gatccggtgg tggtgcaaat caaagaactg 900

ctcctcagtg gatgttgcct ttacttctag gcctgtacgg aagtgttact tctgctctaa 960

aagctgcgga attgtacccg cggccgatcc accggtatgt ggactctcgg gcgccgcgca 1020

gtagccggcc tcctggcgtc acccagccca gcccaggccc agaccctcac ccgggtcccg 1080

cggccggcag agttggcccc actctgcggc cgccgtggcc tgcgcaccga catcgatgcg 1140

acctgcacgc cccgccgcgc aagttcgaac caacgtggcc tcaaccagat ttggaatgtc 1200

aaaaagcaga gtgtctattt gatgaatttg aggaaatctg gaactttggg ccacccaggc 1260

tctctagatg agaccaccta tgaaagacta gcagaggaaa cgctggactc tttagcagag 1320

ttttttgaag accttgcaga caagccatac acgtttgagg actatgatgt ctcctttggg 1380

agtggtgtct taactgtcaa actgggtgga gatctaggaa cctatgtgat caacaagcag 1440

acgccaaaca agcaaatctg gctatcttct ccatccagtg gacctaagcg ttatgactgg 1500

actgggaaaa actgggtgta ctcccacgac ggcgtgtccc tccatgagct gctggccgca 1560

gagctcacta aagccttaaa aaccaaactg gacttgtctt ccttggccta ttccggaaaa 1620

gatgcttgag cggccctagc gtttaaacgg gccctctaga gtcgacccgg gcggcctcga 1680

ggacggggtg aactacgcct gaggatccga tctttttccc tctgccaaaa attatgggga 1740

catcatgaag ccccttgagc atctgacttc tggctaataa aggaaattta ttttcattgc 1800

aatagtgtgt tggaattttt tgtgtctctc actcggccta ggtagataag tagcatggcg 1860

ggttaatcat taactacaag gaacccctag tgatggagtt ggccactccc tctctgcgcg 1920

ctcgctcgct cactgaggcc gggcgaccaa aggtcgcccg acgcccgggc tttgcccggg 1980

cggcctcagt gagcgagcga gcgcgcagcc ttaattaacc taattcactg gccgtcgttt 2040

tacaacgtcg tgactgggaa aaccctggcg ttacccaact taatcgcctt gcagcacatc 2100

cccctttcgc cagctggcgt aatagcgaag aggcccgcac cgatcgccct tcccaacagt 2160

tgcgcagcct gaatggcgaa tgggacgcgc cctgtagcgg cgcattaagc gcggcgggtg 2220

tggtggttac gcgcagcgtg accgctacac ttgccagcgc cctagcgccc gctcctttcg 2280

ctttcttccc ttcctttctc gccacgttcg ccggctttcc ccgtcaagct ctaaatcggg 2340

ggctcccttt agggttccga tttagtgctt tacggcacct cgaccccaaa aaacttgatt 2400

agggtgatgg ttcacgtagt gggccatcgc cctgatagac ggtttttcgc cctttgacgt 2460

tggagtccac gttctttaat agtggactct tgttccaaac tggaacaaca ctcaacccta 2520

tctcggtcta ttcttttgat ttataaggga ttttgccgat ttcggcctat tggttaaaaa 2580

atgagctgat ttaacaaaaa tttaacgcga attttaacaa aatattaacg cttacaattt 2640

aggtggcact tttcggggaa atgtgcgcgg aacccctatt tgtttatttt tctaaataca 2700

ttcaaatatg tatccgctca tgagacaata accctgataa atgcttcaat aatattgaaa 2760

aaggaagagt atgagtattc aacatttccg tgtcgccctt attccctttt ttgcggcatt 2820

ttgccttcct gtttttgctc acccagaaac gctggtgaaa gtaaaagatg ctgaagatca 2880

gttgggtgca cgagtgggtt acatcgaact ggatctcaac agcggtaaga tccttgagag 2940

ttttcgcccc gaagaacgtt ttccaatgat gagcactttt aaagttctgc tatgtggcgc 3000

ggtattatcc cgtattgacg ccgggcaaga gcaactcggt cgccgcatac actattctca 3060

gaatgacttg gttgagtact caccagtcac agaaaagcat cttacggatg gcatgacagt 3120

aagagaatta tgcagtgctg ccataaccat gagtgataac actgcggcca acttacttct 3180

gacaacgatc ggaggaccga aggagctaac cgcttttttg cacaacatgg gggatcatgt 3240

aactcgcctt gatcgttggg aaccggagct gaatgaagcc ataccaaacg acgagcgtga 3300

caccacgatg cctgtagcaa tggcaacaac gttgcgcaaa ctattaactg gcgaactact 3360

tactctagct tcccggcaac aattaataga ctggatggag gcggataaag ttgcaggacc 3420

acttctgcgc tcggcccttc cggctggctg gtttattgct gataaatctg gagccggtga 3480

gcgtgggtct cgcggtatca ttgcagcact ggggccagat ggtaagccct cccgtatcgt 3540

agttatctac acgacgggga gtcaggcaac tatggatgaa cgaaatagac agatcgctga 3600

gataggtgcc tcactgatta agcattggta actgtcagac caagtttact catatatact 3660

ttagattgat ttaaaacttc atttttaatt taaaaggatc taggtgaaga tcctttttga 3720

taatctcatg accaaaatcc cttaacgtga gttttcgttc cactgagcgt cagaccccgt 3780

agaaaagatc aaaggatctt cttgagatcc tttttttctg cgcgtaatct gctgcttgca 3840

aacaaaaaaa ccaccgctac cagcggtggt ttgtttgccg gatcaagagc taccaactct 3900

ttttccgaag gtaactggct tcagcagagc gcagatacca aatactgttc ttctagtgta 3960

gccgtagtta ggccaccact tcaagaactc tgtagcaccg cctacatacc tcgctctgct 4020

aatcctgtta ccagtggctg ctgccagtgg cgataagtcg tgtcttaccg ggttggactc 4080

aagacgatag ttaccggata aggcgcagcg gtcgggctga acggggggtt cgtgcacaca 4140

gcccagcttg gagcgaacga cctacaccga actgagatac ctacagcgtg agctatgaga 4200

aagcgccacg cttcccgaag ggagaaaggc ggacaggtat ccggtaagcg gcagggtcgg 4260

aacaggagag cgcacgaggg agcttccagg gggaaacgcc tggtatcttt atagtcctgt 4320

cgggtttcgc cacctctgac ttgagcgtcg atttttgtga tgctcgtcag gggggcggag 4380

cctatggaaa aacgccagca acgcggcctt tttacggttc ctggcctttt gctggccttt 4440

tgctcacatg ttctttcctg cgttatcccc tgattctgtg gataaccgta ttaccgcctt 4500

tgagtgagct gataccgctc gccgcagccg aacgaccgag cgcagcgagt cagtgagcga 4560

ggaagcggaa gagcgcccaa tacgcaaacc gcctctcccc gcgcgttggc cgattcatta 4620

atgcagctgg cacgacaggt ttcccgactg gaaagcgggc agtgagcgca acgcaattaa 4680

tgtgagttag ctcactcatt aggcacccca ggctttacac tttatgcttc cggctcgtat 4740

gttgtgtgga attgtgagcg gataacaatt tcacacagga aacagctatg accatgatta 4800

cgccagattt aattaaggcc ttaattagg 4829

<210> 92
<211> 4883
<212> DNA
<213> Artificial Sequence

<220>
<223> Description of Artificial Sequence: Synthetic
      polynucleotide

<400> 92
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780
agagcggcgc gctccgaaag tttcctttta tggcgaggcg gcggcggcgg cggccctata 840
aaaagcgaag cgcgcggcgg gcgggagcaa gcttgaactg aaaaaccaga aagttaactg 900
gtaagtttag tctttttgtc ttttatttca ggtcccggat ccggtggtgg tgcaaatcaa 960
agaactgctc ctcagtggat gttgccttta cttctaggcc tgtacggaag tgttacttct 1020
gctctaaaag ctgcggaatt gtacccgcgg ccgatccacc ggtatgtgga ctctcgggcg 1080
ccgcgcagta gccggcctcc tggcgtcacc cagcccggcc caggcccaga ccctcacccg 1140
ggtcccgcgg ccggcagagt tggccccact ctgcggccgc cgtggcctgc gcaccgacat 1200
cgatgcgacc tgcacgcccc gccgcgcaag ttcgaaccaa cgtggcctca accagatttg 1260
gaatgtcaaa aagcagagtg tctatttgat gaatttgagg aaatctggaa ctttgggcca 1320
cccaggctct ctagatgaga ccacctatga aagactagca gaggaaacgc tggactcttt 1380
agcagagttt tttgaagacc ttgcagacaa gccatacacg tttgaggact atgatgtctc 1440
ctttgggagt ggtgtcttaa ctgtcaaact gggtggagat ctaggaacct atgtgatcaa 1500
caagcagacg ccaaacaagc aaatctggct atcttctcca tccagtggac ctaagcgtta 1560
tgactggact gggaaaaact gggtgttctc ccacgacggc gtgtccctcc atgagctgct 1620
ggccgcagag ctcactaaag ccttaaaaac caaactggac ttgtcttggt tggcctattc 1680
cggaaaagat gcttgactcg aggacggggt gaactacgcc tgaggatccg atctttttcc 1740
ctctgccaaa aattatgggg acatcatgaa gccccttgag catctgactt ctggctaata 1800
aaggaaattt attttcattg caatagtgtg ttggaatttt ttgtgtctct cactcggaag 1860
caattcgttg atctgaattt cgaccaccca taatacccat taccctggta gataagtagc 1920
atggcgggtt aatcattaac tacaaggaac ccctagtgat ggagttggcc actccctctc 1980
tgcgcgctcg ctcgctcact gaggccgggc gaccaaaggt cgcccgacgc ccgggctttg 2040
cccgggcggc ctcagtgagc gagcgagcgc gcagccttaa ttaacctaat tcactggccg 2100
tcgttttaca acgtcgtgac tgggaaaacc ctggcgttac ccaacttaat cgccttgcag 2160
cacatccccc tttcgccagc tggcgtaata gcgaagaggc ccgcaccgat cgcccttccc 2220
aacagttgcg cagcctgaat ggcgaatggg acgcgccctg tagcggcgca ttaagcgcgg 2280
cgggtgtggt ggttacgcgc agcgtgaccg ctacacttgc cagcgcccta gcgcccgctc 2340
ctttcgcttt cttcccttcc tttctcgcca cgttcgccgg ctttccccgt caagctctaa 2400
atcgggggct ccctttaggg ttccgattta gtgctttacg gcacctcgac cccaaaaaac 2460
ttgattaggg tgatggttca cgtagtgggc catcgccctg atagacggtt tttcgccctt 2520
tgacgttgga gtccacgttc tttaatagtg gactcttgtt ccaaactgga acaacactca 2580
accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 93
<211> 4883
<212> DNA
<213> Artificial Sequence

<220>
<223> Description of Artificial Sequence: Synthetic
      polynucleotide

<400> 93
gccttaatta ggctgcgcgc tcgctcgctc actgaggccg cccgggcaaa gcccgggcgt 60

cgggcgacct ttggtcgccc ggcctcagtg agcgagcgag cgcgcagaga gggagtggcc 120

aactccatca ctaggggttc cttgtagtta atgattaacc cgccatgcta cttatctacc 180

agggtaatgg ggatcctcta gaactatagc tagtcgacat tgattattga ctagttatta 240

atagtaatca attacggggt cattagttca tagcccatat atggagttcc gcgttacata 300

acttacggta aatggcccgc ctggctgacc gcccaacgac ccccgcccat tgacgtcaat 360

aatgacgtat gttcccatag taacgccaat agggactttc cattgacgtc aatgggtgga 420

gtatttacgg taaactgccc acttggcagt acatcaagtg tatcatatgc caagtacgcc 480

ccctattgac gtcaatgacg gtaaatggcc cgcctggcat tatgcccagt acatgacctt 540

atgggacttt cctacttggc agtacatcta cgtattagtc atcgctatta ccatgtcgag 600

gccacgttct gcttcactct ccccatctcc cccccctccc cacccccaat tttgtattta 660

tttatttttt aattattttg tgcagcgatg ggggcggggg gggggggcgc gcgccaggcg 720

gggcggggcg gggcgagggg cggggcgggg cgaggcggag aggtgcggcg gcagccaatc 780

agagcggcgc gctccgaaag tttcctttta tggcgaggcg gcggcggcgg cggccctata 840

aaaagcgaag cgcgcggcgg gcgggagcaa gcttgaactg aaaaaccaga aagttaactg 900

gtaagtttag tctttttgtc ttttatttca ggtcccggat ccggtggtgg tgcaaatcaa 960

agaactgctc ctcagtggat gttgccttta cttctaggcc tgtacggaag tgttacttct 1020

gctctaaaag ctgcggaatt gtacccgcgg ccgatccacc ggtatgtgga ctctcgggcg 1080

ccgcgcagta gccggcctcc tggcgtcacc cagcccagcc caggcccaga ccctcacccg 1140

ggtcccgcgg ccggcagagt tggccccact ctgcggccgc cgtggcctgc gcaccgacat 1200

cgatgcgacc tgcacgcccc gccgcgcaag ttcgaaccaa cgtggcctca accagatttg 1260

gaatgtcaaa aagcagagtg tctatttgat gaatttgagg aaatctggaa ctttgggcca 1320

cccaggctct ctagatgaga ccacctatga aagactagca gaggaaacgc tggactcttt 1380

agcagagttt tttgaagacc ttgcagacaa gccatacacg tttgaggact atgatgtctc 1440

ctttgggagt ggtgtcttaa ctgtcaaact gggtggagat ctaggaacct atgtgatcaa 1500

caagcagacg ccaaacaagc aaatctggct atcttctcca tccagtggac ctaagcgtta 1560

tgactggact gggaaaaact gggtgtactc ccacgacggc gtgtccctcc atgagctgct 1620

ggccgcagag ctcactaaag ccttaaaaac caaactggac ttgtcttcct tggcctattc 1680

cggaaaagat gcttgactcg aggacggggt gaactacgcc tgaggatccg atctttttcc 1740

ctctgccaaa aattatgggg acatcatgaa gccccttgag catctgactt ctggctaata 1800

aaggaaattt attttcattg caatagtgtg ttggaatttt ttgtgtctct cactcggaag 1860

caattcgttg atctgaattt cgaccaccca taatacccat taccctggta gataagtagc 1920

atggcgggtt aatcattaac tacaaggaac ccctagtgat ggagttggcc actccctctc 1980

tgcgcgctcg ctcgctcact gaggccgggc gaccaaaggt cgcccgacgc ccgggctttg 2040

cccgggcggc ctcagtgagc gagcgagcgc gcagccttaa ttaacctaat tcactggccg 2100

tcgttttaca acgtcgtgac tgggaaaacc ctggcgttac ccaacttaat cgccttgcag 2160

cacatccccc tttcgccagc tggcgtaata gcgaagaggc ccgcaccgat cgcccttccc 2220

aacagttgcg cagcctgaat ggcgaatggg acgcgccctg tagcggcgca ttaagcgcgg 2280

cgggtgtggt ggttacgcgc agcgtgaccg ctacacttgc cagcgcccta gcgcccgctc 2340

ctttcgcttt cttcccttcc tttctcgcca cgttcgccgg ctttccccgt caagctctaa 2400

atcgggggct ccctttaggg ttccgattta gtgctttacg gcacctcgac cccaaaaaac 2460

ttgattaggg tgatggttca cgtagtgggc catcgccctg atagacggtt tttcgccctt 2520

tgacgttgga gtccacgttc tttaatagtg gactcttgtt ccaaactgga acaacactca 2580

accctatctc ggtctattct tttgatttat aagggatttt gccgatttcg gcctattggt 2640

taaaaaatga gctgatttaa caaaaattta acgcgaattt taacaaaata ttaacgctta 2700

caatttaggt ggcacttttc ggggaaatgt gcgcggaacc cctatttgtt tatttttcta 2760

aatacattca aatatgtatc cgctcatgag acaataaccc tgataaatgc ttcaataata 2820

ttgaaaaagg aagagtatga gtattcaaca tttccgtgtc gcccttattc ccttttttgc 2880

ggcattttgc cttcctgttt ttgctcaccc agaaacgctg gtgaaagtaa aagatgctga 2940

agatcagttg ggtgcacgag tgggttacat cgaactggat ctcaacagcg gtaagatcct 3000

tgagagtttt cgccccgaag aacgttttcc aatgatgagc acttttaaag ttctgctatg 3060

tggcgcggta ttatcccgta ttgacgccgg gcaagagcaa ctcggtcgcc gcatacacta 3120

ttctcagaat gacttggttg agtactcacc agtcacagaa aagcatctta cggatggcat 3180

gacagtaaga gaattatgca gtgctgccat aaccatgagt gataacactg cggccaactt 3240

acttctgaca acgatcggag gaccgaagga gctaaccgct tttttgcaca acatggggga 3300

tcatgtaact cgccttgatc gttgggaacc ggagctgaat gaagccatac caaacgacga 3360

gcgtgacacc acgatgcctg tagcaatggc aacaacgttg cgcaaactat taactggcga 3420

actacttact ctagcttccc ggcaacaatt aatagactgg atggaggcgg ataaagttgc 3480

aggaccactt ctgcgctcgg cccttccggc tggctggttt attgctgata aatctggagc 3540

cggtgagcgt gggtctcgcg gtatcattgc agcactgggg ccagatggta agccctcccg 3600

tatcgtagtt atctacacga cggggagtca ggcaactatg gatgaacgaa atagacagat 3660

cgctgagata ggtgcctcac tgattaagca ttggtaactg tcagaccaag tttactcata 3720

tatactttag attgatttaa aacttcattt ttaatttaaa aggatctagg tgaagatcct 3780

ttttgataat ctcatgacca aaatccctta acgtgagttt tcgttccact gagcgtcaga 3840

ccccgtagaa aagatcaaag gatcttcttg agatcctttt tttctgcgcg taatctgctg 3900

cttgcaaaca aaaaaaccac cgctaccagc ggtggtttgt ttgccggatc aagagctacc 3960

aactcttttt ccgaaggtaa ctggcttcag cagagcgcag ataccaaata ctgttcttct 4020

agtgtagccg tagttaggcc accacttcaa gaactctgta gcaccgccta catacctcgc 4080

tctgctaatc ctgttaccag tggctgctgc cagtggcgat aagtcgtgtc ttaccgggtt 4140

ggactcaaga cgatagttac cggataaggc gcagcggtcg ggctgaacgg ggggttcgtg 4200

cacacagccc agcttggagc gaacgaccta caccgaactg agatacctac agcgtgagct 4260

atgagaaagc gccacgcttc ccgaagggag aaaggcggac aggtatccgg taagcggcag 4320

ggtcggaaca ggagagcgca cgagggagct tccaggggga aacgcctggt atctttatag 4380

tcctgtcggg tttcgccacc tctgacttga gcgtcgattt ttgtgatgct cgtcaggggg 4440

gcggagccta tggaaaaacg ccagcaacgc ggccttttta cggttcctgg ccttttgctg 4500

gccttttgct cacatgttct ttcctgcgtt atcccctgat tctgtggata accgtattac 4560

cgcctttgag tgagctgata ccgctcgccg cagccgaacg accgagcgca gcgagtcagt 4620

gagcgaggaa gcggaagagc gcccaatacg caaaccgcct ctccccgcgc gttggccgat 4680

tcattaatgc agctggcacg acaggtttcc cgactggaaa gcgggcagtg agcgcaacgc 4740

aattaatgtg agttagctca ctcattaggc accccaggct ttacacttta tgcttccggc 4800

tcgtatgttg tgtggaattg tgagcggata acaatttcac acaggaaaca gctatgacca 4860

tgattacgcc agatttaatt aag 4883

<210> 94
<211> 2101
<212> DNA
<213> Artificial Sequence

<220>
<223> Description of Artificial Sequence: Synthetic
      polynucleotide

<400> 94
ctgcgcgctc gctcgctcac tgaggccgcc cgggcaaagc ccgggcgtcg ggcgaccttt      60

ggtcgcccgg cctcagtgag cgagcgagcg cgcagagagg gagtgtagcc atgctctagg     120

aagatcaatt cggtacaatt cacgcgtcga cattgattat tgactctggt cgttacataa     180

cttacggtaa atggcccgcc tggctgaccg cccaacgacc ccgcccattg acgtcaataa     240

tgacgtatgt tcccatagta acgccaatag ggactttcca ttgacgtcaa tgggtggagt     300

atttacggta aactgcccac ttggcagtac atcaagtgta tcatatgcca agtacgcccc     360

ctattgacgt caatgacggt aaatggcccg cctggcatta tgcccagtac atgaccttat     420

gggactttcc tacttggcag tacatctact cgaggccacg ttctgcttca ctctccccat     480

ctcccccccc tccccacccc caattttgta tttatttatt ttttaattat tttgtgcagc     540

gatgggggcg gggggggggg gggggggcgc gcgccaggcg gggcggggcg gggcgagggg     600

ggcggggcgg ggcgaggcgg agaggtgcgg cggcagccaa tcagagcggc gcgctccgaa     660

agtttccttt tatggcgagg cggcggcggc ggcggcccta taaaaagcga agcgcgcggc     720

gggcgggagc gggatcagcc accgcggtgg cggccctaga gtcgatcgag gaactgaaaa     780

accagaaagt taactggtaa gtttagtctt tttgtctttt atttcaggtc ccggatccgg     840

tggtggtgca aatcaaagaa ctgctcctca gtggatgttg cctttacttc taggcctgta     900

cggaagtgtt acttctgctc taaaagctgc ggaattgtac ccgcggccga tccaccggtc     960

gccaccatgg tgagcaaggg cgaggagctg ttcaccgggg tggtgcccat cctggtcgag    1020

ctggacggcg acgtaaacgg ccacaagttc agcgtgtccg gcgagggcga gggcgatgcc    1080

acctacggca agctgaccct gaagttcatc tgcaccaccg gcaagctgcc cgtgccctgg    1140

cccaccctcg tgaccaccct gacctacggc gtgcagtgct tcagccgcta ccccgaccac    1200

atgaagcagc acgacttctt caagtccgcc atgcccgaag gctacgtcca ggagcgcacc    1260

atcttcttca aggacgacgg caactacaag acccgcgccg aggtgaagtt cgagggcgac    1320

accctggtga accgcatcga gctgaagggc atcgacttca aggaggacgg caacatcctg    1380

gggcacaagc tggagtacaa ctacaacagc cacaacgtct atatcatggc cgacaagcag    1440

aagaacggca tcaaggtgaa cttcaagatc cgccacaaca tcgaggacgg cagcgtgcag    1500

ctcgccgacc actaccagca gaacaccccc atcggcgacg gccccgtgct gctgcccgac    1560

aaccactacc tgagcaccca gtccgccctg agcaaagacc ccaacgagaa gcgcgatcac 1620

atggtcctgc tggagttcgt gaccgccgcc gggatcactc tcggcatgga cgagctgtac 1680

aagtaaagcg gccatcaagc ttatcgatac cgtcgactag agctcgctga tcagcctcga 1740

ctgtgccttc tagttgccag ccatctgttg tttgcccctc ccccgtgcct tccttgaccc 1800

tggaaggtgc cactcccact gtcctttcct aataaaatga ggaaattgca tcgcattgtc 1860

tgagtaggtg tcattctatt ctggggggtg gggtggggca ggacagcaag ggggaggatt 1920

gggaagacaa ttaggtagat aagtagcatg gcgggttaat cattaactac aaggaacccc 1980

tagtgatgga gttggccact ccctctctgc gcgctcgctc gctcactgag gccgggcgac 2040

caaaggtcgc ccgacgcccg ggctttgccc gggcggcctc agtgagcgag cgagcgcgca 2100

g 2101

## Description

### CROSS REFERENCE TO RELATED APPLICATIONS

This application claims the benefit of U.S. Provisional Patent Application No. 62/104,101, entitled Methods of Delivery to the Central Nervous System, filed January 16, 2015, U.S. Provisional Patent Application No. 62/152,185, entitled Methods of Delivery to the Central Nervous System, filed April 24, 2015, U.S. Provisional Patent Application No. 62/152,283, entitled Central Nervous System Targeting Polynucleotides, filed April 24, 2015, U.S. Provisional Patent Application No. 62/159,888, entitled Methods of Delivery to the Central Nervous System, filed May 11, 2015, U.S. Provisional Patent Application No. 62/159,882, entitled Central Nervous System Targeting Polynucleotides, filed May 11, 2015; the contents of each of which are herein incorporated by reference in their entirety.

### REFERENCE TO THE SEQUENCE LISTING

The present application is being filed along with a Sequence Listing in electronic format. The Sequence Listing is provided as a file entitled 1022PCT.txt created on January 15, 2016 which is 635,539 bytes in size. The information in the electronic format of the sequence listing is incorporated herein by reference in its entirety.

### FIELD OF THE INVENTION

The present invention relates to compositions, methods and processes for the formulation and for the administration of a gene therapy agent using parvovirus e.g., adeno-associated virus (AAV) to the CNS, CNS tissues, CNS structures or CNS cells.

### BACKGROUND OF THE INVENTION

Use of adeno-associated virus (AAV) to deliver therapeutic agents (i.e., transgenes) to the central nervous system offers a means to achieve a widespread distribution of delivered genes in the CNS. Tissue of the CNS is highly heterogeneous and consists of different cell types including different types of neurons (e.g. excitatory and inhibitory neurons) and glial cells (e.g., oligodendrocytes, astrocytes and microglia). The characterization of different AAV capsid serotypes reveals that different AAV serotypes have different efficiency of transduction to different CNS tissues (e.g., cervical spinal cord and hippocampus) and cells (e.g., neurons or glial cells).

Studies, such as those referenced herein examining the targeting of specific tissues and cell types of the CNS by AAV capsids address one part of the problem of effective clinical treatment of CNS disorders by AAV delivery of therapeutic transgenes. The appropriate expression of the therapeutic transgene encoding the delivered payload, both temporally and spatially within the desired cell type, is critical to achieving the desired ameliorative effect. The properties of regulatory elements that drive expression of exogenous payloads from AAV genomes have not been well characterized.

On this background there remains, however, much work to be done to optimize delivery of therapeutic agents to the central nervous system. Better understanding and optimizing delivery parameters for viral particle distribution as described herein will lead to safer and more effective gene therapy. AAVs have emerged as one of the most widely studied and utilized viral particles for gene transfer to mammalian cells. See, e.g., Tratschin et al., Mol. Cell Biol., 5(11):3251-3260 (1985) and Grimm et al., Hum. Gene Ther., 10(15):2445-2450 (1999).

The present invention addresses the need for new technologies by providing AAV-based compositions and complexes which go beyond those of the art by providing for administration and/or delivery of recombinant adeno-associated viral particles in the treatment of diseases or disorders of the CNS, CNS tissues and/or CNS structures.

While delivery is exemplified in the AAV context, other viral vectors, non-viral vectors, nanoparticles, or liposomes may be similarly used to deliver the therapeutic transgenes and include, but are not limited to, vector genomes of any of the AAV serotypes or other parvoviral viral delivery vehicles or lentivirus, etc. The observations and teachings extend to any macromolecular structure, including modified cells, introduced into the CNS in the manner as described herein.

### SUMMARY OF THE INVENTION

The present invention provides AAV capsid serotypes with specific CNS cell type tropism, expression levels and bio-distribution in the CNS. Additionally, the present invention provides regulatory elements and codon optimization of the AAV genome useful *in vitro* and *in vivo* in both cell lines and primary CNS cell types. Accordingly, the present invention provides novel AAV particles with novel combinations of capsid and/or payload that target specific cells and/or tissue in a particular anatomic location in the CNS.

Described herein are compositions, methods, processes, kits and devices for the design, preparation, manufacture and/or formulation of AAV particles. In some embodiments, payloads, such as but not limited to AAV polynucleotides, may be encoded by payload constructs or contained within plasmids or vectors or recombinant adeno-associated viruses (AAVs).

The present invention also provides administration and/or delivery methods for vectors and viral particles, e.g., AAV particles, for the treatment or amelioration of diseases or disorders of the CNS. Such methods may involve the inhibition of gene expression, gene replacement or gene activation. Such outcomes are achieved by utilizing the methods and compositions taught herein.

The details of various embodiments of the invention are set forth in the description below. Other features, objects, and advantages of the invention will be apparent from the description and the drawings, and from the claims.

### BRIEF DESCRIPTION OF THE DRAWINGS

FIG. 1A-1D shows slab 1 and slab 2 individual and average frataxin expression on day 28 in mice treated via intracerebroventricular (ICV) or intracisternal (CM) administration.
FIG. 2A-2H shows frataxin mRNA expression levels in the CNS and other peripheral organs in tested monkeys after intrathecal (IT) L1 bolus dosing of each capsids.
FIG. 3A-3F shows frataxin expression profiles by the capsid serotypes across rostro-caudal spinal cord segments and dorsal root ganglia (DRG) in monkeys.
FIG. 4A-4B shows frataxin mRNA expression in dentate nucleus and liver for all tested capsids in monkeys.

### DETAILED DESCRIPTION OF THE INVENTION

Viruses of the Parvoviridae family are small non-enveloped icosahedral capsid viruses characterized by a single stranded DNA genome. Parvoviridae family viruses consist of two subfamilies: Parvovirinae, which infect vertebrates, and Densovirinae, which infect invertebrates. This virus family is used as a biological tool due to a relatively simple structure that may be easily manipulated with standard molecular biology techniques. The genome of the virus may be modified to contain a minimum of components for the assembly of a functional recombinant virus, or viral particle, which is loaded with or engineered to express or deliver a desired nucleic acid construct or payload, e.g., a transgene, polypeptide-encoding polynucleotide or modulatory nucleic acid, which may be delivered to a target cell, tissue or organism.

The parvoviruses and other members of the Parvoviridae family are generally described in Kenneth I. Berns, "Parvoviridae: The Viruses and Their Replication," Chapter 69 in FIELDS VIROLOGY (3d Ed. 1996), the contents of which is incorporated by reference in its entirety.

The Parvoviridae family comprises the Dependovirus genus which includes adeno-associated viruses (AAVs) capable of replication in vertebrate hosts including, but not limited to, human, primate, bovine, canine, equine, and ovine species.

The details of one or more embodiments of the invention are set forth in the accompanying description below. Other features, objects and advantages of the invention will be apparent from the description. In the description, the singular forms also include the plural unless the context clearly dictates otherwise. Unless defined otherwise, all technical and scientific terms used herein have the same meaning as commonly understood by one of ordinary skill in the art to which this invention belongs. In the case of conflict, the present description will control.

### Parvovirus, e.g. AAV particle payload: transgenes, polypeptide-encoding polynucleotides and/or modulatory nucleic acids

In some embodiments, the present invention provides administration and/or delivery methods for vectors and viral particles, e.g., AAV particles, for the treatment and/or amelioration of diseases or disorders of the CNS (e.g., Friedreich's ataxia).

AAV particles of the present invention may comprise a nucleic acid sequence encoding at least one "payload." As used herein, a "payload" or "payload construct" refers to one or more polynucleotides or polynucleotide regions encoded by or within a viral genome or an expression product of such polynucleotide or polynucleotide region, e.g., a transgene, a polynucleotide encoding a polypeptide or multi-polypeptide or a modulatory nucleic acid or regulatory nucleic acid.

The payload may comprise any nucleic acid known in the art which is useful for modulating the expression (by supplementation or gene replacement or by inhibition using a modulatory nucleic acid) in a target cell transduced or contacted with the AAV particle carrying the payload.

The payload construct may comprise a combination of coding and non-coding nucleic acid sequences.

In one embodiment, the nucleic acid sequence of the AAV particle may be a payload construct.

In one embodiment, the payload construct encodes more than one payload. As a non-limiting example, a payload construct encoding more than one payload may be replicated and packaged into a viral particle. A target cell transduced with a viral particle comprising more than one payload may express each of the payloads in a single cell.

In some embodiments, the payload construct may encode a coding or non-coding RNA.

In one embodiment, the payload is a polypeptide which may be a peptide or protein. A protein encoded by the payload construct may comprise a secreted protein, an intracellular protein, an extracellular protein, and/or a membrane protein. The encoded proteins may be structural or functional. Proteins encoded by the payload construct include, but are not limited to, mammalian proteins. The AAV particles described herein may be useful in the fields of human disease, antibodies, viruses, veterinary applications and a variety of *in vivo* and *in vitro* settings.

In some embodiments, AAV particles described herein are useful in the field of medicine for the treatment, palliation and/or amelioration of conditions or diseases such as, but not limited to, blood, cardiovascular, CNS, and/or genetic disorders.

In some embodiments, AAV particles in accordance with the present invention may be used for the treatment of disorders, and/or conditions, including but not limited to, neurological disorders (*e.g*. Alzheimer's disease, Huntington's disease; autism; Parkinson's disease; Spinal muscular atrophy, Friedreich's ataxia). In one embodiment, the payload encodes a messenger RNA (mRNA). As used herein, the term "messenger RNA" (mRNA) refers to any polynucleotide which encodes a polypeptide of interest and which is capable of being translated to produce the encoded polypeptide of interest *in vitro, in vivo, in situ,* or *ex vivo.*

The components of an mRNA include, but are not limited to, a coding region, a 5'UTR, a 3'UTR, a 5' cap and a poly-A tail. In some embodiments, the encoded mRNA or any portion of the AAV genome may be codon optimized.

In one embodiment the protein encoded by the payload construct is between 50-5000 amino acids in length. In some embodiments the protein encoded is between 50-2000 amino acids in length. In some embodiments the protein encoded is between 50-1000 amino acids in length. In some embodiments the protein encoded is between 50-1500 amino acids in length. In some embodiments the protein encoded is between 50-1000 amino acids in length. In some embodiments the protein encoded is between 50-800 amino acids in length. In some embodiments the protein encoded is between 50-600 amino acids in length. In some embodiments the protein encoded is between 50-400 amino acids in length. In some embodiments the protein encoded is between 50-200 amino acids in length. In some embodiments the protein encoded is between 50-100 amino acids in length.

In some embodiments the peptide encoded by the payload construct is between 4-50 amino acids in length. In one embodiment, the shortest length of a region of the payload of the present invention encoding a peptide can be the length that is sufficient to encode for a tetrapeptide, a pentapeptide, a hexapeptide, a heptapeptide, an octapeptide, a nonapeptide, or a decapeptide. In another embodiment, the length may be sufficient to encode a peptide of 2-30 amino acids, e.g. 5-30, 10-30, 2-25, 5-25, 10-25, or 10-20 amino acids. The length may be sufficient to encode for a peptide of at least 11, 12, 13, 14, 15, 17, 20, 25 or 30 amino acids, or a peptide that is no longer than 50 amino acids, e.g. no longer than 35, 30, 25, 20, 17, 15, 14, 13, 12, 11 or 10 amino acids.

In one embodiment, an AAV particle comprises at least one polynucleotide, e.g., viral genome, encoding at least one payload.

In one embodiment, the AAV particle comprises an AAV capsid selected from the groups consisting of AAV1, AAV2, AAV3, AAV4, AAV5, AAV6, AAV7, AAV8, AAV9, AAV10, and AAV11, AAV12, AAVrh8, AAVrh10, AAV-DJ, and AAV-DJ/8 capsid serotypes, or variants thereof (e.g., AAV3A and AAV3B).

In one embodiment, the serotype which may be useful in the present invention may be AAVDJ8 (or AAV-DJ8). The amino acid sequence of AAVDJ8 may comprise two or more mutations in order to remove the heparin binding domain (HBD). As a non-limiting example, the AAV-DJ sequence described as SEQ ID NO: 1 in US Patent No. 7,588,772, the contents of which are herein incorporated by reference in their entirety, may comprise two mutations: (1) R587Q where arginine (R; arg) at amino acid 587 is changed to glutamine (Q; Gln) and (2) R590T where arginine (R; Arg) at amino acid 590 is changed to threonine (T; Thr). As another non-limiting example, may comprise three mutations: (1) K406R where lysine (K; Lys) at amino acid 406 is changed to arginine (R; Arg), (2) R587Q where arginine (R; Arg) at amino acid 587 is changed to glutamine (Q; Gln) and (3) R590T where arginine (R; Arg) at amino acid 590 is changed to threonine (T; Thr).

### Modulatory nucleic acids

An RNA encoded by the viral genome or DNA of the payload construct may also comprise a tRNA, rRNA, tmRNA, miRNA, RNAi, siRNA, piRNA, shRNA antisense RNA, double stranded RNA, snRNA, snoRNA, and long non-coding RNA (ncRNA). Examples of such IncRNA molecules and RNAi constructs designed to target such IncRNA any of which may be encoded in the payload constructs are taught in International Publication, WO2012/018881 A2, the content of which is incorporated by reference in their entirety.

In one embodiment, the payload construct encodes a microRNA or miRNA, or engineered precursors thereof, as the payload. These payloads along with siRNA, shRNA, antisense molecules and the like may also be referred to as "modulatory nucleic acid" payloads.

MicroRNAs (or miRNA) are 19-25 nucleotide long noncoding RNAs that bind to the 3'UTR of nucleic acid molecules and down-regulate gene expression either by reducing nucleic acid molecule stability or by inhibiting translation. The payload constructs described herein may encode one or more microRNA target sequences, microRNA sequences, or microRNA seeds, or any known precursors thereof such as pre- or pri-microRNAs. Such sequences may correspond to any known microRNA such as those taught in US Publication US2005/0261218 and US Publication US2005/0059005, the content of which is incorporated herein by reference in their entirety.

A microRNA sequence comprises a "seed" region, i.e., a sequence in the region of positions 2-8 of the mature microRNA, which sequence has perfect Watson-Crick complementarity to the miRNA target sequence. A microRNA seed may comprise positions 2-8 or 2-7 of the mature microRNA. In some embodiments, a microRNA seed may comprise 7 nucleotides (e.g., nucleotides 2-8 of the mature microRNA), wherein the seed-complementary site in the corresponding miRNA target is flanked by an adenine (A) opposed to microRNA position 1. In some embodiments, a microRNA seed may comprise 6 nucleotides (e.g., nucleotides 2-7 of the mature microRNA), wherein the seed-complementary site in the corresponding miRNA target is flanked by an adenine (A) opposed to microRNA position 1. See for example, Grimson A, Farh KK, Johnston WK, Garrett-Engele P, Lim LP, Bartel DP; Mol Cell. 2007 Jul 6; 27(1):91-105; each of which is herein incorporated by reference in their entirety. The bases of the microRNA seed have complete complementarity with the target sequence.

When designed to inhibit or silence a gene, the AAV particles comprising payload constructs of the present invention may also encode a payload which may be processed to produce a siRNA, miRNA or other double stranded (ds) or single stranded (ss) gene modulatory nucleic acids or motifs.

Accordingly, the encoded siRNA duplexes or dsRNA can be used to inhibit gene expression in a cell, in particular cells of the CNS. In some aspects, the inhibition of gene expression refers to an inhibition by at least about 20%, preferably by at least about 30%, 40%, 50%, 60%, 70%, 80%, 85%, 90%, 95% and 100%. Accordingly, the protein product of the targeted gene may be inhibited by at least about 20%, preferably by at least about 30%, 40%, 50%, 60%, 70%, 80%, 85%, 90%, 95% and 100%. The gene can be either a wild type gene or a mutated gene with at least one mutation. Accordingly, the targeted protein is either wild type protein or a mutated protein with at least one mutation.

In some embodiments, the present invention provides methods for treating, or ameliorating a disease or condition associated with abnormal gene and/or protein in a subject in need of treatment, the method comprising administering to the subject any effective amount of at least one AAV particle encoding an siRNA duplex targeting the gene, delivering duplex into targeted cells, inhibiting the gene expression and protein production, and ameliorating symptoms of the disease or condition in the subject.

### Gene replacement or activation

When designed to increase the expression of a gene or replace a gene, AAV particles may comprise a viral genome comprising a payload construct which encodes a normal gene to replace a mutated, defective or nonfunctional copy of that gene in the recipient.

### Functional payloads

In one embodiment, a payload may comprise polypeptides that serve as marker proteins to assess cell transformation and expression, fusion proteins, polypeptides having a desired biological activity, gene products that can complement a genetic defect, RNA molecules, transcription factors, and other gene products that are of interest in regulation and/or expression. In another embodiment, a payload may comprise nucleotide sequences that provide a desired effect or regulatory function (e.g., transposons, transcription factors).

The encoded payload may comprise a gene therapy product. A gene therapy product may include, but is not limited to, a polypeptide, RNA molecule, or other gene product that, when expressed in a target cell, provides a desired therapeutic effect. In some embodiments, a gene therapy product may comprise a substitute for a non-functional gene that is absent or mutated.

A payload construct encoding a payload may comprise or encode a selectable marker. A selectable marker may comprise a gene sequence or a protein encoded by a gene sequence expressed in a host cell that allows for the identification, selection, and/or purification of the host cell from a population of cells that may or may not express the selectable marker. In one embodiment, the selectable marker provides resistance to survive a selection process that would otherwise kill the host cell, such as treatment with an antibiotic. In another embodiment, an antibiotic selectable marker may comprise one or more antibiotic resistance factors, including but not limited to neomycin resistance (e.g., neo), hygromycin resistance, kanamycin resistance, and/or puromycin resistance.

In some embodiments, any nucleic acid sequence encoding a polypeptide can be used as a selectable marker comprising recognition by a specific antibody.

In some embodiments, a payload construct encoding a payload may comprise a selectable marker including, but not limited to, β-lactamase, luciferase, β-galactosidase, or any other reporter gene as that term is understood in the art, including cell-surface markers, such as CD4 or the truncated nerve growth factor (NGFR) (for GFP, see WO 96/23810; Heim et al., Current Biology 2:178-182 (1996); Heim et al., Proc. Natl. Acad. Sci. USA (1995); or Heim et al., Science 373:663-664 (1995); for β-lactamase, see WO 96/30540); the contents of each of which are herein incorporated by reference in their entirety.

In some embodiments, a payload construct encoding a selectable marker may comprise a fluorescent protein. A fluorescent protein as herein described may comprise any fluorescent marker including but not limited to green, yellow, and/or red fluorescent protein (GFP, YFP, and/or RFP).

In accordance with the invention, a payload comprising a nucleic acid for expression of a payload in a target cell will be incorporated into the viral genome and produced located between two ITR sequences, or on either side of an asymmetrical ITR engineered with two D regions.

A payload construct encoding one or more payloads for expression in a target cell may comprise one or more payload or non-payload nucleotide sequences operably linked to at least one target cell-compatible promoter. A person skilled in the art may recognize that a target cell may require a specific promoter including but not limited to a promoter that is species specific, inducible, tissue-specific, or cell cycle-specific Parr et al., Nat. Med.3:1145-9 (1997).

### Promoters

A person skilled in the art may recognize that expression in a target cell may require a specific promoter, including but not limited to, a promoter that is species specific, inducible, tissue-specific, or cell cycle-specific (Parr et al., Nat. Med.3:1145-9 (1997); the contents of which are herein incorporated by reference in its entirety).

In one embodiment, the promoter is deemed to be efficient for expression of the payload encoded in the viral genome of the AAV particle.

In one embodiment, the promoter is a promoter deemed to be efficient for driving expression in the cell being targeted.

In one embodiment, the promoter provides expression of a payload for a period of time in targeted tissues such as, but not limited to, nervous system tissues. Expression from a promoter may be for a period of 1 hour, 2, hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 1 day, 2 days, 3 days, 4 days, 5 days, 6 days, 1 week, 8 days, 9 days, 10 days, 11 days, 12 days, 13 days, 2 weeks, 15 days, 16 days, 17 days, 18 days, 19 days, 20 days, 3 weeks, 22 days, 23 days, 24 days, 25 days, 26 days, 27 days, 28 days, 29 days, 30 days, 31 days, 1 month, 2 months, 3 months, 4 months, 5 months, 6 months, 7 months, 8 months, 9 months, 10 months, 11 months, 1 year, 13 months, 14 months, 15 months, 16 months, 17 months, 18 months, 19 months, 20 months, 21 months, 22 months, 23 months, 2 years, 3 years, 4 years, 5 years, 6 years, 7 years, 8 years, 9 years, 10 years or more than 10 years. Expression may be for 1-5 hours, 1-12 hours, 1-2 days, 1-5 days, 1-2 weeks, 1-3 weeks, 1-4 weeks, 1-2 months, 1-4 months, 1-6 months, 2-6 months, 3-6 months, 3-9 months, 4-8 months, 6-12 months, 1-2 years, 1-5 years, 2-5 years, 3-6 years, 3-8 years, 4-8 years or 5-10 years. In one embodiment, the promoter is a weak promoter for sustained expression of a payload in nervous tissues. In one embodiment, the promoter is a weak promoter for sustained frataxin expression in nervous system tissue such as, but not limited to, neuronal tissue and glial tissue.

In one embodiment, the Friedreich's ataxia (FRDA) promoter is used in the viral genomes of the AAV particles described herein.

In one embodiment, there is a region located approximately ∼5 kb upstream of the first exon of the encoded payload, more specifically, there is a 17 bp region located approximately 4.9 kb upstream of the first exon of the encoded fraxtaxin gene in order to allow for expression with the FRDA promoter (See e.g., Puspasari et al. Long Range Regulation of Human FXN Gene Expression, PLOS ONE, 2011; the contents of which is herein incorporated by reference in its entirety).

In one embodiment, the promoter may be a promoter which is less than 1 kb. The promoter may have a length of 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800 or more than 800. The promoter may have a length between 200-300, 200-400, 200-500, 200-600, 200-700, 200-800, 300-400, 300-500, 300-600, 300-700, 300-800, 400-500, 400-600, 400-700, 400-800, 500-600, 500-700, 500-800, 600-700, 600-800 or 700-800.

In one embodiment, the promoter may be a combination of two or more components of the same or different promoters such as, but not limited to, CMV and CBA. Each component may have a length of 200, 210, 220, 230, 240, 250, 260, 270, 280, 290, 300, 310, 320, 330, 340, 350, 360, 370, 380, 381, 382, 383, 384, 385, 386, 387, 388, 389, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800 or more than 800. Each component may have a length between 200-300, 200-400, 200-500, 200-600, 200-700, 200-800, 300-400, 300-500, 300-600, 300-700, 300-800, 400-500, 400-600, 400-700, 400-800, 500-600, 500-700, 500-800, 600-700, 600-800 or 700-800. In one embodiment, the promoter is a combination of a 382 nucleotide CMV-enhancer sequence and a 260 nucleotide CBA-promoter sequence.

In one embodiment, the payload construct of the viral genome comprises at least one element to enhance the transgene target specificity and expression (See e.g., Powell et al. Viral Expression Cassette Elements to Enhance Transgene Target Specificity and Expression in Gene Therapy, 2015; the contents of which are herein incorporated by reference in its entirety). Non-limiting examples of elements to enhance the transgene target specificity and expression include promoters, endogenous miRNAs, post-transcriptional regulatory elements (PREs), polyadenylation (PolyA) signal sequences and upstream enhancers (USEs), CMV enhancers and introns.

Promoters which promote expression in most tissues include, but are not limited to, human elongation factor 1α-subunit (EF1α), immediate-early cytomegalovirus (CMV), chicken β-actin (CBA) and its derivative CAG, the β glucuronidase (GUSB), or ubiquitin C (UBC). Tissue-specific expression elements can be used to restrict expression to certain cell types such as, but not limited to, nervous system promoters which can be used to restrict expression to neurons, astrocytes, or oligodendrocytes. Non-limiting example of tissue-specific expression elements for neurons include neuron-specific enolase (NSE), platelet-derived growth factor (PDGF), platelet-derived growth factor B-chain (PDGF-β), the synapsin (Syn), the methyl-CpG binding protein 2 (MeCP2), Ca²⁺/calmodulin-dependent protein kinase II (CaMKII), metabotropic glutamate receptor 2 (mGluR2), NFL, NFH, nβ2, PPE, Enk and EAAT2 promoters. A non-limiting example of a tissue-specific expression elements for astrocytes include the glial fibrillary acidic protein (GFAP) and EAAT2 promoters. A non-limiting example of a tissue-specific expression element for oligodendrocytes includes the myelin basic protein (MBP) promoter.

In one embodiment, the encoded payload construct comprises a ubiquitous promoter. Non-limiting examples of ubiquitous promoters include CMV, CBA (including derivatives CAG, CBh, etc.), EF-1α, PGK, UBC, GUSB (hGBp), and UCOE (promoter of HNRPA2B1-CBX3).

Yu et al. (Molecular Pain 2011, 7:63; the contents of which are herein incorporated by reference in its entirety) evaluated the expression of eGFP under the CAG, EFIα, PGK and UBC promoters in rat DRG cells and primary DRG cells using lentiviral vectors and found that UBC showed weaker expression than the other 3 promoters and there was only 10-12% glia expression seen for all promoters. Soderblom et al. (E. Neuro 2015; the contents of which are herein incorporated by reference in its entirety) evaluated the expression of eGFP in AAV8 with CMV and UBC promoters and AAV2 with the CMV promoter after injection in the motor cortex. Intranasal administration of a plasmid containing a UBC or EFIα promoter showed a sustained airway expression greater than the expression with the CMV promoter (See e.g., Gill et al., Gene Therapy 2001, Vol. 8, 1539-1546; the contents of which are herein incorporated by reference in its entirety). Husain et al. (Gene Therapy 2009; the contents of which are herein incorporated by reference in its entirety) evaluated a HβH construct with a hGUSB promoter, a HSV-1LAT promoter and a NSE promoter and found that the HβH construct showed weaker expression than NSE in mice brain. Passini and Wolfe (J. Virol. 2001, 12382-12392, the contents of which are herein incorporated by reference in its entirety) evaluated the long term effects of the HβH vector following an intraventricular injection in neonatal mice and found that there was sustained expression for at least 1 year. Low expression in all brain regions was found by Xu et al. (Gene Therapy 2001, 8, 1323-1332; the contents of which are herein incorporated by reference in its entirety) when NF-L and NF-H promoters were used as compared to the CMV-lacZ, CMV-luc, EF, GFAP, hENK, nAChR, PPE, PPE + wpre, NSE (0.3 kb), NSE (1.8 kb) and NSE (1.8 kb + wpre). Xu et al. found that the promoter activity in descending order was NSE (1.8 kb), EF, NSE (0.3 kb), GFAP, CMV, hENK, PPE, NFL and NFH. NFL is a 650 nucleotide promoter and NFH is a 920 nucleotide promoter which are both absent in the liver but NFH is abundant in the sensory proprioceptive neurons, brain and spinal cord and NFH is present in the heart. Scn8a is a 470 nucleotide promoter which expresses throughout the DRG, spinal cord and brain with particularly high expression seen in the hippocampal neurons and cerebellar Purkinje cells, cortex, thalamus and hypothalamus (See e.g., Drews et al. Identification of evolutionary conserved, functional noncoding elements in the promoter region of the sodium channel gene SCN8A, Mamm Genome (2007) 18:723-731; and Raymond et al. Expression of Alternatively Spliced Sodium Channel α-subunit genes, Journal of Biological Chemistry (2004) 279(44) 46234-46241; the contents of each of which are herein incorporated by reference in their entireties).

Any of the promoters taught by Yu, Soderblom, Gill, Husain, Passini, Xu, Drews or Raymond may be used in the present inventions.

In one embodiment, the encoded payload construct comprises a promoter which is not cell specific.

In one embodiment, the encoded payload construct comprises an ubiquitin c (UBC) promoter. The UBC promoter may have a size of 300-350 nucleotides. As a non-limiting example, the UBC promoter is 332 nucleotides.

In one embodiment, the encoded payload construct comprises a β-glucuronidase (GUSB) promoter. The GUSB promoter may have a size of 350-400 nucleotides. As a non-limiting example, the GUSB promoter is 378 nucleotides. As a non-limiting example, the construct may be AAV-promoter-CMV/globin intron-hFXN-RBG, where the AAV may be self-complementary and the AAV may be the DJ serotype.

In one embodiment, the payload construct comprises a neurofilament (NFL) promoter. The NFL promoter may have a size of 600-700 nucleotides. As a non-limiting example, the NFL promoter is 650 nucleotides. As a non-limiting example, the construct may be AAV-promoter-CMV/globin intron-hFXN-RBG, where the AAV may be self-complementary and the AAV may be the DJ serotype.

In one embodiment, the payload construct comprises a neurofilament heavy (NFH) promoter. The NFH promoter may have a size of 900-950 nucleotides. As a non-limiting example, the NFH promoter is 920 nucleotides. As a non-limiting example, the construct may be AAV-promoter-CMV/globin intron-hFXN-RBG, where the AAV may be self-complementary and the AAV may be the DJ serotype.

In one embodiment, the payload construct comprises a scn8a promoter. The scn8a promoter may have a size of 450-500 nucleotides. As a non-limiting example, the scn8a promoter is 470 nucleotides. As a non-limiting example, the construct may be AAV-promoter-CMV/globin intron-hFXN-RBG, where the AAV may be self-complementary and the AAV may be the DJ serotype.

In one embodiment, the payload construct comprises a frataxin (FXN) promoter.

In one embodiment, the payload construct comprises a phosphoglycerate kinase 1 (PGK) promoter.

In one embodiment, the payload construct comprises a chicken β-actin (CBA) promoter.

In one embodiment, the payload construct comprises an immediate-early cytomegalovirus (CMV) promoter.

In one embodiment, the payload construct comprises a liver or a skeletal muscle promoter. Non-limiting examples of liver promoters include hAAT and TBG. Non-limiting examples of skeletal muscle promoters include Desmin, MCK and C5-12.

In one embodiment, the payload construct comprises an enhancer element, a promoter and/or a 5'UTR intron. The enhancer element, also referred to herein as an "enhancer," may be, but is not limited to, a CMV enhancer, the promoter may be, but is not limited to, a CMV, CBA, UBC, GUSB, NSE, Synapsin, MeCP2, and GFAP promoter and the 5'UTR/intron may be, but is not limited to, SV40, and CBA-MVM. As a non-limiting example, the enhancer, promoter and/or intron used in combination may be: (1) CMV enhancer, CMV promoter, SV40 5'UTR intron; (2) CMV enhancer, CBA promoter, SV 40 5'UTR intron; (3) CMV enhancer, CBA promoter, CBA-MVM 5'UTR intron; (4) UBC promoter; (5) GUSB promoter; (6) NSE promoter; (7) Synapsin promoter; (8) MeCP2 promoter and (9) GFAP promoter.

In one embodiment, the payload construct has an engineered promoter.

In one embodiment, the payload construct comprises a promoter which provides expression for at least 1 month, 2 months, 3 months, 4 months, 5 months, 6 months, 7 months, 8 months, 9 months, 10 months, 11 months, 1 year, 2 years, 3 years 4 years, 5 years, 6 years, 7 years, 8 years, 9 years, 10 years, 11 years, 12 years, 13 years, 14 years, 15 years, 16 years, 17 years, 18 years, 19 years, 20 years, 21 years, 22 years, 23 years, 24 years, 25 years, 26 years, 27 years, 28 years, 29 years, 30 years, 31 years, 32 years, 33 years, 34 years, 35 years, 36 years, 37 years, 38 years, 39 years, 40 years, 41 years, 42 years, 43 years, 44 years, 45 years, 46 years, 47 years, 48 years, 49 years, 50 years, 55 years, 60 years, 65 years, or more than 65 years.

### Introns

In one embodiment, the payload construct comprises at least one element to enhance the transgene expression such as one or more introns or portions thereof. Non-limiting examples of introns include, MVM (67-97 bps), F.IX truncated intron 1 (300 bps), β-globin SD/immunoglobulin heavy chain splice acceptor (250 bps), adenovirus splice donor/immunoglobin splice acceptor (500 bps), SV40 late splice donor/splice acceptor (19S/16S) (180 bps) and hybrid adenovirus splice donor/IgG splice acceptor (230 bps).

In one embodiment, the intron or intron portion may be 100-500 nucleotides in length. The intron may have a length of 80, 90, 100, 110, 120, 130, 140, 150, 160, 170, 171, 172, 173, 174, 175, 176, 177, 178, 179, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490 or 500. The intron may have a length between 80-100, 80-120, 80-140, 80-160, 80-180, 80-200, 80-250, 80-300, 80-350, 80-400, 80-450, 80-500, 200-300, 200-400, 200-500, 300-400, 300-500, or 400-500.

### Viral Production

The present disclosure provides a method for the generation of parvovirus particle, e.g., AAV particle, by viral genome replication in a viral replication cell comprising contacting the viral replication cell with a payload construct vector and a viral construct vector.

The present disclosure provides a method for producing an AAV particle having enhanced (increased, improved) transduction efficiency comprising the steps of: 1) co-transfecting competent bacterial cells with a bacmid vector and either a viral construct vector and/or payload construct vector, 2) isolating the resultant viral construct vector and payload construct vector and separately transfecting viral replication cells, 3) isolating and purifying resultant payload and viral construct particles comprising viral construct vector or payload construct vector, 4) co-infecting a viral replication cell with both the payload construct vector and viral construct vector, 5) harvesting and purifying the viral particle comprising a parvoviral genome. Production methods are further disclosed in commonly owned and co-pending International Publication No. WO2015191508, the contents of which are herein incorporated by reference in their entirety.

### Particles and capsids

The invention also provides nucleic acids encoding the mutated or modified virus capsids and capsid proteins of the invention. In some embodiments the capsids are engineered according to the methods of co-owned and co-pending International Publication No. WO2015191508, the contents of which are herein incorporated by reference in their entirety.

Further provided are particles comprising the nucleic acids and cells (in vivo or in culture) comprising the nucleic acids and/or particles of the invention. Suitable particles include without limitation viral particles (e.g., adenovirus, AAV, herpes virus, vaccinia, poxviruses, baculoviruses, and the like), plasmids, phage, YACs, BACs, and the like as are well known in the art. Such nucleic acids, particles and cells can be used, for example, as reagents (e.g., helper packaging constructs or packaging cells) for the production of modified virus capsids or virus particles as described herein.

The molecules of the invention which contain payload constructs include any genetic element (vector) which may be delivered to a host cell, e.g., naked DNA, plasmid, phage, transposon, cosmid, episome, a protein in a non-viral delivery vehicle (e.g., a lipid-based carrier), virus, etc., which transfers the sequences carried thereon. The methods used to construct any embodiment of this invention are known to those with skill in nucleic acid manipulation and include genetic engineering, recombinant engineering, and synthetic techniques. See, e.g., Sambrook et al, Molecular Cloning: A Laboratory Manual, Cold Spring Harbor Press, Cold Spring Harbor, N.Y.

The transgene or payload can be carried on any suitable vector, e.g., a plasmid, which is delivered to a host cell. The plasmids useful in this invention may be engineered such that they are suitable for replication and, optionally, integration in prokaryotic cells, mammalian cells, or both. These plasmids contain sequences permitting replication of the transgene in eukaryotes and/or prokaryotes and selection markers for these systems. Selectable markers or reporter genes may include sequences encoding geneticin, hygromicin or purimycin resistance, among others. The plasmids may also contain certain selectable reporters or marker genes that can be used to signal the presence of the vector in bacterial cells, such as ampicillin resistance. Other components of the plasmid may include an origin of replication and an amplicon, such as the amplicon system employing the Epstein Barr virus nuclear antigen. This amplicon system, or other similar amplicon components permit high copy episomal replication in the cells. Preferably, the molecule carrying the transgene or payload is transfected into the cell, where it may exist transiently. Alternatively, the transgene may be stably integrated into the genome of the host cell, either chromosomally or as an episome. In certain embodiments, the transgene may be present in multiple copies, optionally in head-to-head, head-to-tail, or tail-to-tail concatamers. Suitable transfection techniques are known and may readily be utilized to deliver the transgene to the host cell.

In one embodiment, the AAV particles of the invention may be a single-stranded AAV (ssAAV) or a self-complementary AAV (scAAV) described herein or known in the art.

### AAV capsid serotypes

In some embodiments, viral vectors and viral particles produced according to the present invention may target to deliver and/or to transfer a payload of interest to specific population of cells in specific anatomical regions (e.g., dopaminergic (DAergic) neurons in the Substantia Nigra (SN)) in the central nervous system).

In some embodiments, viral vectors and viral particles of the present invention may be packaged in a capsid structure or may be capsid free. Such capsid free viral vector donor and/or acceptor sequences such as AAV, are described in, for example, US Publication 20140107186, the content of which is incorporated by reference in its entirety.

In some embodiments, viral vectors and viral particles produced according to the present invention may comprise different capsid proteins, either naturally occurring and/or recombinant, including, but not limited to, AAV1, AAV2, AAV3, AAV4, AAV5, AAV6, AAV7, AAV8, AAV9, AAV10, and AAV11, AAV12, AAVrh8, AAVrh10, AAV-DJ, and AAV-DJ/8 capsid serotypes, or variants thereof (e.g., AAV3A and AAV3B). Nucleic acid sequences encoding one or more AAV capsid proteins useful in the present invention are disclosed in the commonly owned International Publication No. WO2015191508, the contents of which are herein incorporated by reference in their entirety.

In some embodiments, viral vectors and viral particles produced according to the present invention may comprise hybrid serotypes with enhanced transduction to specific cell types of interest in the central nervous system, prolonged transgene expression and/or a safety profile. The hybrid serotypes may be generated by transcapsidation, adsorption of bi-specific antibody to capsid surface, mosaic capsid, and chimeric capsid, and/or other capsid protein modifications.

The naturally occurring AAV Cap gene expresses VP1, VP2, and VP3 capsid proteins are encoded by a single open reading frame of the Cap gene under control of the p40 promoter. In one embodiment, nucleotide sequences encoding VP1, VP2 and VP3 proteins and/or amino acid sequences of AAV VP capsid proteins may be modified for increased efficiency to target to the central nervous system (e.g., CNS tissue tropism). Any of the VP genes of the serotypes selected from, but not limited to, AAV1, AAV2, AAV3, AAV4, AAV5, AAV6, AAV7, AAV8, AAV9, AAV10, and AAV11, AAV12, AAVrh8, AAVrh10, AAV-DJ, and AAV-DJ/8 capsid serotypes, or variants thereof (e.g., AAV3A and AAV3B) may be modified.

In some embodiments, viral vectors and viral particles of the present invention may be further modified toward a specific therapeutic application. The modification may be done by rational mutagenesis of capsid proteins (see, e.g., Pulicherla et al., Mol Ther, 2011, 19: 1070-1078), incorporation of peptide ligands to the capsid, for example a peptide derived from an NMDA receptor agonist for enhanced retrograde transport (Xu et al., Virology, 2005, 341: 203-214), and directed evolution to produce new AAV variants for increased CNS transduction.

In one embodiment, viral vectors and viral particles of the present invention may comprise capsid proteins having sequences of SEQ ID NOs: 1 and 3, which have increased tropism to the brain, of International Publication No. WO2014160092, the content of which is incorporated herein by reference in its entirety.

In one embodiment, viral vectors and viral particles of the present invention may comprise capsid proteins which may target to oligodendrocytes in the central nervous system. The capsid proteins may comprise AAV capsid coding sequence of SEQ ID NO: 1 or AAV capsid proteins comprising amino acid sequences of SEQ ID NOs: 2 to 4 of International Publication No. WO2014052789, the content of which is herein incorporated by reference in its entirety.

In one embodiment, viral vectors and viral particles of the present invention may comprise capsid proteins having increased capacity to cross the blood-brain barrier in CNS as disclosed in US Pat. No: 8,927,514, the content of which is incorporated herein by reference in its entirety. The amino acid sequences and nucleic acid sequences of such capsid proteins may include, but are not limited to, SEQ ID NOs: 2-17 and SEQ ID NOs: 25-33, respectively, of US Pat. No: 8,927,514.

In some embodiments, viral vectors and viral particles of the present invention may comprise AAV2 capsid proteins or variants thereof. Viral particles with AAV2 capsid proteins have been shown to deliver genes to neurons effectively in the brain, retina and spinal cord. In one embodiment, AAV2 capsid proteins may be further modified such as addition of a targeting peptide to the capsid proteins that targets an AAV particle to brain vascular endothelium as disclosed in US Pat. NOs: 6,691,948 and 8, 299,215, the contents of each of which are herein incorporated by reference in their entirety. Such viral particles may be used to deliver a functional payload of interest to treat a brain disease such as mucopolysaccharide (MPS).

In some embodiments, viral vectors and viral particles of the present invention may comprise AAV5 capsid proteins or variants thereof. Viral particles with AAV5 capsid proteins can transduce neurons in various regions of the CNS, including the cortex, the hippocampus (HPC), cerebellum, substantia nigra (SN), striatum, globus pallidus, and spinal cord (Burger C et al., Mol Ther., 2004, 10(2): 302-317; Liu G et al., Mol Ther. 2007, 15(2): 242-247; and Colle M et al., Hum, Mol. Genet. 2010, 19(1): 147-158). In one embodiment, viral particles having AA5 capsid proteins with increased transduction to cells in CNS may be those particles from US. Pat. NO: 7,056,502, the content of which is incorporated herein by reference in its entirety.

In some embodiments, viral vectors and viral particles of the present invention may comprise AAV6 capsid proteins or variants thereof. Recombinant AAV6 serotype can target motor neurons in the spinal cord by Intracerebroventricular (ICV) injection (Dirren E et al., Hum Gene Ther., 2014, 25(2): 109-120). In addition, a study from San Sebastian et al indicated that AAV6 serotype can be retrogradely transported from terminals to neuronal cell bodies in the rat brain (San Sebastian et al, Gen Ther., 2014, 20(12): 1178-1183).

In some embodiments, viral vectors and viral particles of the present invention may comprise AAV8 capsid proteins or variants thereof. Viral particles with AAV8 capsid proteins can transduce neurons, for example in hippocampus (Klein RL et al., Mol Ther., 2006, 13(3): 517-527). In one embodiment, AAV8 capsid proteins may comprise the amino acid sequence of SEQ ID NO: 2 of US Pat. NO: 8,318,480, the content of which is herein incorporated by reference in its entirety.

In some embodiments, viral vectors and viral particles of the present invention may comprise AAV9 capsid proteins or variants thereof. AAV9 capsid serotype mediated gene delivery has been observed in the brain with efficient and long-term expression of transgene after intraparenchymal injections to the CNS (Klein RL et al., Eur J Neurosci., 2008, 27: 1615-1625). AAV9 serotype can produce robust and wide-scale neuronal transduction throughout the CNS after a peripheral, systemic (e.g., intravenous) administration in neonatal subjects (Foust KD et al., Nat. Biotechnol., 2009, 27: 59-65; and Duque S et al, Mol Ther., 2009, 17: 1187-1196). Intrathecal (intra-cisterna magna routes) administration of AAV9 serotypes can also produce widespread spinal expression. In one embodiment, AAV9 serotype may comprise an AAV capsid protein having the amino acid sequence of SEQ ID NO: 2 of US Pat. No: 7,198,951, the content of which is incorporated herein by reference in its entirety. In another aspect, AAV 9 serotype may comprise VP1 capsid proteins of SEQ ID NOs: 2, 4 or 6 in which at least one of surface-exposed tyrosine residues in the amino acid sequence is substituted with another amino acid residue, as disclosed in US patent publication No. US20130224836, the content of which is incorporated herein by reference in its entirety.

In some embodiments, viral vectors and viral particles of the present invention may comprise AAVrh10 capsid proteins or variants thereof. Viral particles comprising AAVrh10 capsid proteins can target neurons, other cells as well, in the spinal cord after intrathecal (IT) administration. In one embodiment, AAVrh10 capsid proteins may comprise the amino acid sequence of SEQ ID NO: 81 of EP patent NO: 2341068.

In some embodiments, viral vectors and viral particles of the present invention may comprise AAVDJ capsid proteins, AAVDJ/8 capsid proteins, or variants thereof. Holehonnur et al showed that AVDJ/8 serotype can target neurons within the Basal and Lateral Amygdala (BLA) (Holennur R et al., BMC Neurosci, 2014, Feb 18:15:28). In one embodiment, AAVDJ capsid proteins and/or AAVDJ/8 capsid proteins may comprise an amino acid sequence comprising a first region that is derived from a first AAV serotype (e.g., AAV2), a second region that is derived from a second AAV serotype (e.g., AAV8), and a third region that is derived from a third AAV serotype (e.g., AAV9), wherein the first, second and third region may include any amino acid sequences that are disclosed in this description.

In some embodiment, viral vectors and viral particles produced according to the present invention may comprise single stranded DNA viral genomes (ssAAVs) or self-complementary AAV genomes (scAAVs). scAAV genomes contain both DNA strands which anneal together to form double stranded DNA. By skipping second strand synthesis, scAAVs allow for rapid expression in the cell.

In one embodiment, the AAV particles of the invention comprise the formula AAV-promoter-transgene. The AAV may be an ssAAV or a scAAV described herein or known in the art.

### Delivery to the CNS

Factors affecting delivery of payloads by parvovirus, e.g., AAV particles to cells of the central nervous system (e.g., parenchyma) as provided by the invention may comprise, but are not limited to, infusion parameters and devices, spatial orientation of the subject, composition physiochemical properties, and viral physiochemical and biochemical properties.

In one embodiment, delivery of payloads by adeno-associated virus (AAV) particles to cells of the central nervous system (e.g., parenchyma) comprises prolonged infusion into cerebrospinal fluid (CSF). CSF is produced by specialized ependymal cells that comprise the choroid plexus located in the ventricles of the brain. CSF produced within the brain then circulates and surrounds the central nervous system including the brain and spinal cord. CSF continually circulates around the central nervous system, including the ventricles of the brain and subarachnoid space that surrounds both the brain and spinal cord, while maintaining a homeostatic balance of production and reabsorption into the vascular system. The entire volume of CSF is replaced approximately four to six times per day or approximately once every four hours, though values for individuals may vary.

In one embodiment, the AAV particles may be delivered by a route to bypass the liver metabolism.

In one embodiment, the AAV particles may be delivered to reduce degradation of the AAV particles and/or degradation of the formulation in the blood.

In one embodiment, the AAV particles may be delivered to bypass anatomical blockages such as, but not limited to the blood brain barrier.

In one embodiment, the AAV particles may be formulated and delivered to a subject by a route which increases the speed of drug effect as compared to oral delivery.

In one embodiment, the AAV particles may be delivery by a method to provide uniform transduction of the spinal cord and dorsal root ganglion (DRG). As a non-limiting example, the AAV particles may be delivered using intrathecal infusion. The intrathecal infusion may be a bolus infusion or it may be a continuous infusion. As another non-limiting example, the AAV particles are delivered using continuous intrathecal infusion over a period of about 10 hours.

In one embodiment, the AAV particles may be delivered to a subject via a single route administration.

In one embodiment, the AAV particles may be delivered to a subject via a multi-site route of administration. For example, a subject may be administered the AAV particles at 2, 3, 4, 5 or more than 5 sites.

In one embodiment, the AAV particles may be formulated. As a non-limiting example the baricity and/or osmolarity of the formulation may be optimized to ensure optimal drug distribution in the central nervous system or a region or component of the central nervous system.

In one embodiment, a subject may be administered the AAV particles described herein using a catheter. The catheter may be placed in the lumbar region or the cervical region of a subject. As a non-limiting example, the catheter may be placed in the lumbar region of the subject. As another non-limiting example, the catheter may be placed in the cervical region of the subject. As yet another non-limiting example, the catheter may be placed in the high cervical region of the subject. As used herein, the "high cervical region" refers to the region of the spinal cord comprising the cervical vertebrae C1, C2, C3 and C4 or any subset thereof.

In one embodiment, a subject may be administered the AAV particles described herein using a bolus infusion. As used herein, a "bolus infusion" means a single and rapid infusion of a substance or composition.

In one embodiment, a subject may be administered the AAV particles described herein using sustained delivery over a period of minutes, hours or days. The infusion rate may be changed depending on the subject, distribution, formulation or another delivery parameter known to those in the art.

In one embodiment, the intracranial pressure may be evaluated prior to administration. The route, volume, AAV particle concentration, infusion duration and/or vector titer may be optimized based on the intracranial pressure of a subject.

In one embodiment, the AAV particles described herein may be delivered by a method which allows even distribution of the AAV particles along the CNS taking into account cerebrospinal fluid (CSF) dynamics. While not wishing to be bound by theory, CSF turnover (TO) occurs approximately 6 times/day or every 4 hours and thus continuous delivery of the AAV particles at a fixed rate, may lead to AAV particles which have distributed throughout the CNS.

In one embodiment, AAV particles are delivered taking into account the oscillating movement of the CSF around the spinal cord. Vortexes are formed by the oscillating movement of the CSF around the cord and these individual vortices combine to form vortex arrays. The arrays combine to form fluid paths for movement of the AAV particles along the spinal cord.

In one embodiment, the delivery method and duration is chosen to provide broad transduction in the spinal cord. As a non-limiting example, intrathecal delivery is used to provide broad transduction along the rostral-caudal length of the spinal cord. As another non-limiting example, multi-site infusions provide a more uniform transduction along the rostral-caudal length of the spinal cord. As yet another non-limiting example, prolonged infusions provide a more uniform transduction along the rostral-caudal length of the spinal cord.

In one embodiment, delivery of AAV particles comprising a viral genome encoding a payload described herein to sensory neurons in the dorsal root ganglion (DRG), ascending spinal cord sensory tracts, and cerebellum will lead to an increased expression of the encoded payload. The increased expression may lead to improved survival and function of various cell types.

In one embodiment, delivery of AAV particles comprising a nucleic acid sequence encoding frataxin to sensory neurons in the dorsal root ganglion (DRG), ascending spinal cord sensory tracts, and cerebellum leads to an increased expression of frataxin. The increased expression of frataxin then leads to improved survival, ataxia (balance) and gait, sensory capability, coordination of movement and strength, functional capacity and quality of life and/or improved function of various cell types.

In one embodiment, the AAV particles may be delivered by systemic delivery. As a non-limiting example, the systemic delivery may be by intravascular administration.

In one embodiment, the AAV particles may be delivered by injection into the CSF pathway. Non-limiting examples of delivery to the CSF pathway include intrathecal and intracerebroventricular administration.

In one embodiment, the AAV particles may be delivered by direct injection into the brain. As a non-limiting example, the brain delivery may be by intrastriatal administration.

### Intracerebroventricular (ICV) Infusion

In one embodiment, delivery of AAV particles to cells of the central nervous system (e.g., parenchyma) is performed by intracerebroventricular (ICV) prolonged infusion. ICV prolonged infusion comprises delivery by injection into the ventricular system of the brain. ICV prolonged infusion may comprise delivery to any of the ventricles of the brain, including, but not limited to, either of the two lateral ventricles left and right, third ventricle, and/or fourth ventricle. ICV prolonged infusion may comprise delivery to any of the foramina, or channels that connect the ventricles, including, but not limited to, interventricular foramina, also called the foramina of Monroe, cerebral aqueduct, and/or central canal. ICV prolonged infusion may comprise delivery to any of the apertures of the ventricular system including, but not limited to, the median aperture (aka foramen of Magendie), right lateral aperture, and/or left lateral aperture (aka foramina of Lushka). In one embodiment, ICV prolonged infusion comprises delivery to the perivascular space in the brain.

### Intrathecal (IT) Infusion

It has been discovered that prolonged continuous intrathecal (IT) infusion leads to stable AAV particle levels within the cerebral spinal fluid (CSF) circulating around the brain and spinal cord by maintaining favorable concentration gradients for AAV particle movement into the parenchyma. Prolonged IT infusion provides increased exposure that favors tissue interactions with AAV by extracellular and intraneuronal processes. In one embodiment extracellular 'paravascular capture' comprises the inward movement of AAV along blood vessels. In one embodiment, AAV may move along the outside of neural axons including, but not limited to, nerves such as the dorsal and ventral roots that transect the IT space and are bathed by CSF. Intraneuronal exposure comprises uptake and transport within and along the interior of axons towards (retrograde) or away from (anterograde) the neuronal cell body; AAV has been shown to move in both directions dependent on the serotype.

Stable levels of AAV particles and concentrations gradients induced by prolonged IT infusion increases the total area of spinal cord exposed to efficacious AAV particle concentrations. Consequently, prolonged exposure to the spinal cord will allow for a single site of delivery for widespread neuraxial transfection. As a non-limiting example, the even distribution across targeted neuraxis may avoid hot spots of transduction.

Prolonged IT infusion additionally allows for slower, more controlled infusion that yields more reproducible results. In contrast, bolus delivery can lead to wastage of AAV drug product and 'hot spots' comprising uneven, high levels of transduction along the spinal cord or adjacent dorsal root ganglion.

The large size of AAV particles, about 25nm diameter, leads to steric hindrance wherein there is a limit to the number of AAV particles that may access tissue binding sites and achieve subsequent uptake into cells at any given point in time. Bolus delivery of high numbers of AAV particles over a short period of infusion makes it impossible for much of the delivered AAV dose to reach binding sites for uptake into neurons, astrocytes, oligodendrocytes, microglia and other CNS cells. In contrast, prolonged continuous IT infusion may provide more even and complete distribution of AAV along the neuraxis as AAV concentration reaches equilibrium, thereby reducing the risk of steric hindrance due to the large size of AAV as well as providing a longer timeframe for uptake of AAV into neural cells, tissues, and structures.

This extended timeframe allows for AAV levels in the spinal cord to approach steady state, i.e., the maximum possible level of particles in the CSF for a given infusion rate and concentration. Steady state for AAV levels is reached when the amount of AAV infused into the CSF is equal to AAV clearance rate. The longer that AAV is delivered at or near steady state, the longer there is maintained a favorable diffusion gradient from CSF into parenchyma, which maximizes the opportunity for particles to be transported via extra- and intra-neuronal routes.

While not wishing to be bound by theory, since CSF turnover (TO) occurs approximately 6 times/day or every 4 hours, the steady state described here which is dependent on continuous delivery of AAV at a fixed rate, would predict that AAV particles move and distribute based on CSF flow dynamics, and that overall binding of AAV to tissue is small compared to total AAV particle dose.

The present invention provides administration and/or delivery methods for vectors and viral particles, e.g., AAV particles or AAV, for the treatment or amelioration of diseases or disorders of the CNS. Such methods may involve the inhibition of gene expression, gene replacement or gene activation. Such outcomes are achieved by utilizing the infusion methods taught herein.

In one embodiment, delivery of AAV particles to cells of the central nervous system (e.g., parenchyma) is performed by intrathecal (IT) prolonged infusion. IT prolonged infusion comprises delivery by injection into the subarachnoid space, between the arachnoid membrane and pia mater, which comprises the channels through which CSF circulates. IT prolonged infusion comprises delivery to any area of the subarachnoid space including, but not limited to, perivascular space and the subarachnoid space along the entire length of the spinal cord and surrounding the brain.

### The Spinal Cord

The human spinal cord was first mapped by Bruce, 1901 (Bruce, A., 1901. A Topographical Atlas of the Spinal Cord. Williams and Norgate, London, Available from: www.archive.org/details/cu31924024791406 [24.07.14]) and later by others including Sengul et al., 2013 (Sengul, G., Watson, C., Tanaka, I., Paxinos, G., 2013. Atlas of the Spinal Cord of the Rat, Mouse, Marmoset, Rhesus, and Human. Elsevier Academic Press, San Diego), the contents of each of which is herein incorporated by reference in its entirety. The spinal cord can be divided into 5 regions, into an organization which is derived from the adjacent vertebrae: cervical, thoracic, lumbar, sacral, and coccygeal (caudal) as described in Watson et al., 2015 (Neuroscience Research 93 (2015) 164-175 The spinal cord of the common marmoset (Callithrix jacchus) Charles Watson, Gulgun Senguld, Ikuko Tanakae, Zoltan Rusznakb, and Hironobu Tokunoe), and Pardo et al., 2012 (Toxicologic Pathology, 40: 624-636, 2012 "Technical Guide for Nervous System Sampling of the Cynomolgus Monkey for General Toxicity Studies" Ingrid D. Pardo, Robert H. Garman, Klaus Weber, Walter F. Bobrowski, Jerry F. Hardisty, And Daniel Morton), the contents of each of which is herein incorporated by reference in its entirety. The segments in each region and their numbering are shown in Table 1.

In some instanced, rhesus and cynomolgus monkey each have the same number of segments in each region. Rhesus monkey and Cynomolgus monkeys have 7 or 8 segments in the cervical region. Humans have 7 or 8 segments in the cervical region. Humans, Rhesus monkeys and Cynomolgus monkeys have 12 thoracic segments. Humans have 5 lumbar segments while Rhesus and Cynomolgus monkeys have 7 lumbar segments. The sacral region includes 5 segments in humans, but three segments in Cynomolgus monkey and Rhesus monkey. The coccygeal region has 3 segments in rhesus monkey and cynomolgus monkey, and one segment in human.

**Table 1. Spinal cord segments in human, cynomolgus and rhesus monkeys**

| **Spinal Cord Region** | **Human** | **Cynomolgus Monkey** | **Rhesus Monkey** |
|---|---|---|---|
| Cervical | C1-C7 | C1-C7 | C1-C7 |
| Thoracic | T1-T12 | T1-T12 | T1-T12 |
| Lumbar | L1-L5 | L1-L7 | L1-L7 |
| Sacral | S1-S5 | S1-S3 | S1-S3 |
| Coccygeal (caudal) | Col | Col-3 | Col-3 |

Additionally, the spinal cord can also be divided into six regions anatomically and functionally (Sengul et al., 2013 (Sengul, G., Watson, C., Tanaka, I., Paxinos, G., 2013. Atlas of the Spinal Cord of the Rat, Mouse, Marmoset, Rhesus, and Human. Elsevier Academic Press, San Diego), and also Watson et al., Neuroscience Research 93:164-175 (2015)). These regions are the neck muscle region, the upper limb muscle region, the sympathetic outflow region, the lower limb muscle region, the parasympathetic outflow region, and the tail muscle region. These six regions also correlate with territories defined by gene expression during development (see, e.g., Watson et al., supra). The six regions can be defined histologically by the presence or absence of 2 features, the lateral motor column (LMC) and the preganglionic (intermediolateral) column (PGC) (Watson et al., 2015, incorporated herein by reference in its entirety). The limb enlargements are characterized by the presence of a lateral motor column (LMC) and the autonomic regions containing a preganglionic column (PGC). The neck (prebrachial) and tail (caudal) regions have neither an LMC nor a PGC. The limb enlargements and the sympathetic outflow region are marked by particular patterns of hox gene expression in the mouse and chicken, further supporting the division of the spinal cord into these functional regions. Table 2 maps the C, T, L, S and Co designations described in Table 1 to the functional regions according to Sengul et al. and Watson et al. and maps the functional equivalents for Human, Rhesus Monkey, and Japanese Monkey (another macaque). Note: S1 in Rhesus Monkey and L7 in Japanese monkey is located in both crural and postcrural regions.

**Table 2. Spinal cord regions and sections by function**

| **Spinal Cord Region** | **Human** | **Rhesus Monkey** | **Japanese Monkey** |
|---|---|---|---|
| Neck Muscle Region (prebrachial region) | C1-C4 (according to Bruce) | C1-C4 (according to Sengul et al.) | C1-C3 (as described in Watson et al.) |
| | C1-C3 (according to Sengul et al.) | | |
| Upper limb Region (brachial region) | C5-T1 (according to Bruce) | C5-T1 (according to Sengul et al.) | C4-C8 (as described in Watson et al.) |
| | C4-T1 (according to Sengul et al.) | | |
| Sympathetic outflow region (postbrachial region) | T2-L1 (according to Bruce) | T2-L3 (according to Sengul et al.) | T1-L2 (as described in Watson et al.) |
| | T2-L1 (according to Sengul et al.) | | |
| Lower limb muscle region (crural region) | L2-S2 (according to Bruce) | L4-S1 (according to Sengul et al.) | L3-L7 (as described in Watson et al.) |
| | L3-S2 (according to Sengul et al.) | | |
| Parasympathetic outflow region (postcrural region) | S3-S4 (according to Bruce) | S1-S3 (according to Sengul et al.) | L7-S3 (as described in Watson et al.) |
| | S3-S5 (according to Sengul et al.) | | |
| Tail muscle region (caudal region) | S5-Col (according to Bruce) | Col-Co3 (according to Sengul et al.) | Col-Co3 (as described in Watson et al.) |
| | Col (according to Sengul et al.) | | |

In one embodiment, the catheter for intrathecal delivery may be located in the cervical region. The AAV particles may be delivered in a continuous or bolus infusion.

In one embodiment, the catheter for intrathecal delivery may be located in the lumbar region. The AAV particles may be delivered in a continuous or bolus infusion.

In one embodiment, if continuous delivery of the AAV particles is used, the continuous infusion may be for 1 hour, 2, hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours or more than 24 hours.

In one embodiment, the catheter may be in located at one site in the spine for delivery. As a non-limiting example, the location may be in the cervical or the lumbar region. The AAV particles may be delivered in a continuous or bolus infusion.

In one embodiment, the catheter may be located at more than one site in the spine for multi-site delivery. The AAV particles may be delivered in a continuous and/or bolus infusion. Each site of delivery may be a different dosing regimen or the same dosing regimen may be used for each site of delivery. As a non-limiting example, the sites of delivery may be in the cervical and the lumbar region. As another non-limiting example, the sites of delivery may be in the cervical region. As another non-limiting example, the sites of delivery may be in the lumbar region.

In one embodiment, a subject may be analyzed for spinal anatomy and pathology prior to delivery of the AAV particles described herein. As a non-limiting example, a subject with scoliosis may have a different dosing regimen and/or catheter location compared to a subject without scoliosis.

In one embodiment, the orientation of the spine subject during delivery of the AAV particles may be vertical to the ground.

In another embodiment, the orientation of the spine of the subject during delivery of the AAV particles may be horizontal to the ground.

In one embodiment, the spine of the subject may be at an angle as compared to the ground during the delivery of the AAV particles subject. The angle of the spine of the subject as compared to the ground may be at least 10, 20, 30, 40, 50, 60, 70, 80, 90, 100, 110, 120, 130, 140, 150 or 180 degrees.

In one embodiment, a subject may be delivered the AAV particles herein using two or more delivery routes. As a non-limiting example, the delivery routes may be intrathecal administration and intracerebroventricular administration.

In one embodiment, a subject may be delivered the AAV particles herein at more than one site. As a non-limiting example, the delivery may be a multi-site intrathecal delivery using a bolus injection.

In one embodiment, a subject may be delivered the AAV particles described herein by intrathecal delivery in the lumbar region via a 10 hour bolus injection.

In one embodiment, subjects such as mammals (e.g., non-human primates (NHPs)) are administered by intrathecal (IT) or intracerebroventricular (ICV) infusion the AAV particles described herein. The AAV particles may comprise scAAV or ssAAV, of any of the serotypes described herein, comprising a payload (e.g., a transgene). The dose may be 1x10¹³ to 3x10¹³ vg per subject. The subject may be administered a dose of the AAV particles over an extended period of time such as, but not limited to, 10 ml over 10 hours. The subjects may be evaluated 14-30 days (e.g., 14, 21, 28, or 30 days) after administration to determine the expression of the payload in the subject. Further, the subject may be evaluated prior to administration and after administration to determine changes in behavior and activity such as, but not limited to, tremors, lethargic behavior, motor deficits in limbs, strength, spinal reflex deficits, food consumption. (For AAV9 in Non Human Primates (Cyno) see: Samaranch et al. Human Gene Therapy 23:382-389 April 2012, Samaranch et al. Human Gene Therapy 24: 526-532 May 2013, Samaranch et al. Molecular Therapy 22(2) 329-337 February 2014, Gray et al. Gene Ther. 20(4) 450-459 April 2013; the contents of each of which are herein incorporated by reference in their entireties).

Cross-sections may be labeled according to vertebral segmentation numbering and/or spinal segment numbering. From the mid-thoracic region through the sacral region, the spinal cord is compressed relative to the vertebrae, resulting in a difference of vertebral and spinal levels.

In some embodiments IT prolonged infusion comprises delivery to the cervical, thoracic, and or lumbar regions of the spine. As used herein, IT prolonged infusion into the spine is defined by the vertebral level at the site of prolonged infusion. In some embodiments IT prolonged infusion comprises delivery to the cervical region of the spine at any location including, but not limited to C1, C2, C3, C4, C5, C6, C7, and/or C8. In some embodiments IT prolonged infusion comprises delivery to the thoracic region of the spine at any location including, but not limited to T1, T2, T3, T3, T4, T5, T6, T7, T8, T9, T10, T11, and/or T12. In some embodiments IT prolonged infusion comprises delivery to the lumbar region of the spine at any location including, but not limited to L1, L2, L3, L3, L4, L5, and/or L6. In some embodiments IT prolonged infusion comprises delivery to the sacral region of the spine at any location including, but not limited to S1, S2, S3, S4, or S5. In some embodiments, delivery by IT prolonged infusion comprises one or more than one site of prolonged infusion.

In some embodiments, delivery by IT prolonged infusion may comprise 1, 2, 3, 4, 5, 6, 7, 8, 9, 10, 11, 12, 13, 14, 15, 16, 17, 18, 19, 20, 21, 22, 23, 24, or 25 sites of prolonged infusion. In one embodiment, delivery by IT prolonged infusion comprises at least three sites of prolonged infusion. In one embodiment, delivery by IT prolonged infusion consists of three sites of prolonged infusion. In one embodiment, delivery by IT prolonged infusion comprises three sites of prolonged infusion at C1, T1, and L1.

In one embodiment, intrathecal administration delivers AAV particles to targeted regions of the CNS. Non-limiting examples of regions of the CNS to deliver AAV particles include dorsal root ganglion, dentate nucleus-cerebellum and the auditory pathway.

In one embodiment, intrathecal administration of AAV particles provides peripheral exposure which is as low as possible or a moderate level that is beneficial. As a non-limiting example, intrathecal administration of AAV particles shows almost no peripheral exposure to the liver. As another non-limiting example, intrathecal administration of AAV particles provides an expression level to the heart which treat and/or prevents cardiomyopathies. As yet another non-limiting example, intrathecal administration of AAV particles provides an expression level to the pancreas which treats and/or prevents diabetes.

### Infusion parameters and volume

In some embodiments, infusion volume, duration of infusion, infusion patterns and rates for delivery of AAV particles to cells of the central nervous system (e.g., parenchyma) may be determined and regulated. In one embodiment, delivery of AAV particles to cells of the central nervous system (e.g., parenchyma) comprises infusion of up to 1 mL. The infusion may be at least 0.1 mL, 0.2 mL, 0.3 mL, 0.4 mL, 0.5 mL, 0.6 mL, 0.7 mL, 0.8 mL, 0.9 mL, 1 mL or the infusion may be 0.1 - 0.2 mL, 0.1 - 0.3 mL, 0.1 - 0.4 mL, 0.1 - 0.5 mL, 0.1 - 0.6 mL, 0.1 - 0.7 mL, 0.1 - 0.8 mL, 0.1 - 0.9 mL, 0.1 - 1 mL, 0.2 - 0.3 mL, 0.2 - 0.4 mL, 0.2 - 0.5 mL, 0.2 - 0.6 mL, 0.2 - 0.7 mL, 0.2 - 0.8 mL, 0.2 - 0.9 mL, 0.2 - 1 mL, 0.3 - 0.4 mL, 0.3 - 0.5 mL, 0.3 - 0.6 mL, 0.3 - 0.7 mL, 0.3 - 0.8 mL, 0.3 - 0.9 mL, 0.3 - 1 mL, 0.4 - 0.5 mL, 0.4 - 0.6 mL, 0.4 - 0.7 mL, 0.4 - 0.8 mL, 0.4 - 0.9 mL, 0.4 - 1 mL, 0.5 - 0.6 mL, 0.5 - 0.7 mL, 0.5 - 0.8 mL, 0.5 - 0.9 mL, 0.5 - 1 mL, 0.6 - 0.7 mL, 0.6 - 0.8 mL, 0.6 - 0.9 mL, 0.6 - 1 mL, 0.7 - 0.8 mL, 0.7 - 0.9 mL, 0.7 - 1 mL, 0.8 - 0.9 mL, 0.8 - 1 mL, or 0.9 - 1 mL.

In one embodiment, delivery of AAV particles to cells of the central nervous system (e.g., parenchyma) comprises infusion of between about 1 mL to about 120 mL. The infusion may be 1 - 5 mL, 1 - 10 mL, 1 - 15 mL, 1 - 20 mL, 1 - 25 mL, 1- 30 mL, 1 - 35 mL, 1 - 40 mL, 1 - 45 mL, 1 - 50 mL, 1 - 55 mL, 1 - 60 mL, 1 - 65 mL, 1 - 70 mL, 1 - 75 mL, 1 - 80 mL, 1 - 85 mL, 1 - 90 mL, 1 - 95 mL, 1 - 100 mL, 1 - 105 mL, 1 - 110 mL, 1 - 115 mL, 1 - 120 mL, 5 - 10 mL, 5 - 15 mL, 5 - 20 mL, 5 - 25 mL, 1- 30 mL, 5 - 35 mL, 5 - 40 mL, 5 - 45 mL, 5 - 50 mL, 5 - 55 mL, 5 - 60 mL, 5 - 65 mL, 5 - 70 mL, 5 - 75 mL, 5 - 80 mL, 5 - 85 mL, 5 - 90 mL, 5 - 95 mL, 5 - 100 mL, 5 - 105 mL, 5 - 110 mL, 5 - 115 mL, 1 - 120 mL, 10 - 15 mL, 10 - 20 mL, 10 - 25 mL, 10 - 30 mL, 10 - 35 mL, 10 - 40 mL, 10 - 45 mL, 10 - 50 mL, 10 - 55 mL, 10 - 60 mL, 10 - 65 mL, 10 - 70 mL, 10 - 75 mL, 10 - 80 mL, 10 - 85 mL, 10 - 90 mL, 10 - 95 mL, 10 - 100 mL, 10 - 105 mL, 10 - 110 mL, 10 - 115 mL, 10 - 120 mL 15 - 20 mL, 15 - 25 mL, 15 - 30 mL, 15 - 35 mL, 15 - 40 mL, 15 - 45 mL, 15 - 50 mL, 15 - 55 mL, 15 - 60 mL, 15 - 65 mL, 15 - 70 mL, 15 - 75 mL, 15 - 80 mL, 15 - 85 mL, 15 - 90 mL, 15 - 95 mL, 15 - 100 mL, 15 - 105 mL, 15 - 110 mL, 15 - 115 mL, 15 - 120 mL, 20 - 25 mL, 20 - 30 mL, 20 - 35 mL, 20 - 40 mL, 20 - 45 mL, 20 - 50 mL, 20 - 55 mL, 20 - 60 mL, 20 - 65 mL, 20 - 70 mL, 20 - 75 mL, 20 - 80 mL, 20 - 85 mL, 20 - 90 mL, 20 - 95 mL, 20 - 100 mL, 20 - 105 mL, 20 - 110 mL, 20 - 115 mL, 20 - 120 mL, 25 - 30 mL, 25 - 35 mL, 25 - 40 mL, 25 - 45 mL, 25 - 50 mL, 25 - 55 mL, 25 - 60 mL, 25 - 65 mL, 25 - 70 mL, 25 - 75 mL, 25 - 80 mL, 25 - 85 mL, 25 - 90 mL, 25 - 95 mL, 25 - 100 mL, 25 - 105 mL, 25 - 110 mL, 25 - 115 mL, 25 - 120 mL, 30 - 35 mL, 30 - 40 mL, 30 - 45 mL, 30 - 50 mL, 30 - 55 mL, 30 - 60 mL, 30 - 65 mL, 30 - 70 mL, 30 - 75 mL, 30 - 80 mL, 30 - 85 mL, 30 - 90 mL, 30 - 95 mL, 30 - 100 mL, 30 - 105 mL, 30 - 110 mL, 30 - 115 mL, 30 - 120 mL, 35 - 40 mL, 35 - 45 mL, 35 - 50 mL, 35 - 55 mL, 35 - 60 mL, 35 - 65 mL, 35 - 70 mL, 35 - 75 mL, 35 - 80 mL, 35 - 85 mL, 35 - 90 mL, 35 - 95 mL, 35 - 100 mL, 35 - 105 mL, 35 - 110 mL, 35 - 115 mL, 35 - 120 mL, 40 - 45 mL, 40 - 50 mL, 40 - 55 mL, 40 - 60 mL, 40 - 65 mL, 40 - 70 mL, 40 - 75 mL, 40 - 80 mL, 40 - 85 mL, 40 - 90 mL, 40 - 95 mL, 40 - 100 mL, 40 - 105 mL, 40 - 110 mL, 40 - 115 mL, 40 - 120 mL, 45 - 50 mL, 45 - 55 mL, 45 - 60 mL, 45 - 65 mL, 45 - 70 mL, 45 - 75 mL, 45 - 80 mL, 45 - 85 mL, 45 - 90 mL, 45 - 95 mL, 45 - 100 mL, 45 - 105 mL, 45 - 110 mL, 45 - 115 mL, 45 - 120 mL, 50 - 55 mL, 50 - 60 mL, 50 - 65 mL, 50 - 70 mL, 50 - 75 mL, 50 - 80 mL, 50 - 85 mL, 50 - 90 mL, 50 - 95 mL, 50 - 100 mL, 50 - 105 mL, 50 - 110 mL, 50 - 115 mL, 50 - 120 mL, 55 - 60 mL, 55 - 65 mL, 55 - 70 mL, 55 - 75 mL, 55 - 80 mL, 55 - 85 mL, 55 - 90 mL, 55 - 95 mL, 55 - 100 mL, 55 - 105 mL, 55 - 110 mL, 55 - 115 mL, 55 - 120 mL, 60 - 65 mL, 60 - 70 mL, 60 - 75 mL, 60 - 80 mL, 60 - 85 mL, 60 - 90 mL, 60 - 95 mL, 60 - 100 mL, 60 - 105 mL, 60 - 110 mL, 60 - 115 mL, 60 - 120 mL, 65 - 70 mL, 65 - 75 mL, 65 - 80 mL, 65 - 85 mL, 65 - 90 mL, 65 - 95 mL, 65 - 100 mL, 65 - 105 mL, 65 - 110 mL, 65 - 115 mL, 65 - 120 mL, 70 - 75 mL, 70 - 80 mL, 70 - 85 mL, 70 - 90 mL, 70 - 95 mL, 70 - 100 mL, 70 - 105 mL, 70 - 110 mL, 70 - 115 mL, 70 - 120 mL, 75 - 80 mL, 75 - 85 mL, 75 - 90 mL, 75 - 95 mL, 75 - 100 mL, 75 - 105 mL, 75 - 110 mL, 75 - 115 mL, 75 - 120 mL, 80 - 85 mL, 80 - 90 mL, 80 - 95 mL, 80 - 100 mL, 80 - 105 mL, 80 - 110 mL, 80 - 115 mL, 80 - 120 mL, 85 - 90 mL, 85 - 95 mL, 85 - 100 mL, 85 - 105 mL, 85 - 110 mL, 85 - 115 mL, 85 - 120 mL, 90 - 95 mL, 90 - 100 mL, 90 - 105 mL, 90 - 110 mL, 90 - 115 mL, 90 - 120 mL, 95 - 100 mL, 95 - 105 mL, 95 - 110 mL, 95 - 115 mL, 95 - 120 mL, 100 - 105 mL, 100 - 110 mL, 100 - 115 mL, 100 - 120 mL, 105 - 110 mL, 105 - 115 mL, 105 - 120 mL, 110 - 115 mL, 110 - 120 mL, or 115 - 120 mL.

In one embodiment, delivery of AAV particles to cells of the central nervous system (e.g., parenchyma) may comprise an infusion of about 0.1, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 mL. In one embodiment, delivery of AAV particles to cells of the central nervous system (e.g., parenchyma) comprises of infusion of 1 mL.

In one embodiment, delivery of AAV particles to cells of the central nervous system (e.g., parenchyma) comprises of infusion of at least 1 mL. In one embodiment, delivery of AAV particles to cells of the central nervous system (e.g., parenchyma) comprises infusion of at least 3 mL. In one embodiment, delivery of AAV particles to cells of the central nervous system (e.g., parenchyma) comprises of infusion of 3 mL. In one embodiment, delivery of AAV particles to cells of the central nervous system (e.g., parenchyma) comprises infusion of at least 10 mL. In one embodiment, delivery of AAV particles to cells of the central nervous system (e.g., parenchyma) consists of infusion of 10 mL.

In one embodiment, the serotype of the AAV particles described herein may depend on the desired distribution, transduction efficiency and cellular targeting required. As described by Sorrentino et al. (comprehensive map of CNS transduction by eight adeno-associated virus serotypes upon cerebrospinal fluid administration in pigs, Molecular Therapy accepted article preview online 07 December 2015; doi:10.1038/mt.2015.212; the contents of which are herein incorporated by reference in its entirety), AAV serotypes provided different distributions, transduction efficiencies and cellular targeting. In order to provide the desired efficacy, the AAV serotype needs to be selected that best matches not only the cells to be targeted but also the desired transduction efficiency and distribution.

### Duration of Infusion: Bolus Infusion

In one embodiment, delivery of AAV particles to cells of the central nervous system (e.g., parenchyma) comprises infusion by bolus injection with a duration of less than 30 minutes. In one embodiment, infusion by bolus injection comprises injection with a duration of less than 20 minutes. In one embodiment, infusion by bolus injection comprises injection with a duration of less than 10 minutes. In one embodiment, infusion by bolus injection comprises injection with a duration of less than 10 seconds. In one embodiment, infusion by bolus injection comprises injection with a duration of between 10 seconds to 10 minutes. In one embodiment, infusion by bolus injection comprises injection with a duration of 10 minutes. In one embodiment, infusion by bolus injection consists of injection with a duration of 10 minutes.

In one embodiment, delivery of AAV particles to cells of the central nervous system (e.g., parenchyma) comprises infusion by at least one bolus injection. In one embodiment, delivery may comprise infusion by 1, 2, 3, 4, 5, 6, 7, 8, 9, or 10 bolus injections. In one embodiment, delivery may comprise infusion by at least three bolus injections. In one embodiment, delivery comprises infusion by three bolus injections. In one embodiment, delivery of AAV to cells of the central nervous system (e.g., parenchyma) consists of infusion by three bolus injections.

In one embodiment, delivery of AAV particles to cells of the central nervous system (e.g., parenchyma) comprising infusion of more than one bolus injection further comprises an interval of at least one hour between injections. In one embodiment, delivery of AAV particles to cells of the central nervous system (e.g., parenchyma) comprising infusion of more than one bolus injection may further comprise an interval of 1, 2, 3, 4, 5, 6, 7, 8, 9, 10, 11, 12, 13, 14, 15, 16, 17, 18, 19, 20, 21, 22, 23, 24, 30, 36, 42, 48, 54, 60, 66, 72, 78, 84, 90, 96, 108, or 120 hour(s) between injections. In one embodiment, delivery comprising infusion of more than one bolus injection further comprises an interval of one hour between injections. In one embodiment, delivery consists of infusion by three bolus injections at an interval of one hour.

In one embodiment, DRG and/or cortical brain expression may be higher with shorter, high concentration infusions.

### Prolonged Infusion

In one embodiment, delivery of AAV particles to cells of the central nervous system (e.g., parenchyma) comprises prolonged infusion of pharmaceutically acceptable composition comprising AAV particles over a duration of at least 10 minutes. In one embodiment, delivery comprises prolonged infusion over a duration of between 30 minutes and 60 minutes. In one embodiment, delivery may comprise prolonged infusion of over a duration of 0.17, 0.33, 0.5, 0.67, 0.83, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, or 125 hour(s). In one embodiment, delivery comprises prolonged infusion over a duration of one hour. In one embodiment, delivery of AAV particles to cells of the central nervous system (e.g., parenchyma) consists of prolonged infusion over a duration of one hour.

In one embodiment, delivery of AAV particles to cells of the central nervous system (e.g., parenchyma) comprises prolonged infusion over a duration of 10 hours. In one embodiment, delivery of AAV particles to cells of the central nervous system (e.g., parenchyma) consists of prolonged infusion over a duration of 10 hours. In one embodiment, prolonged infusion may yield more homogenous levels of protein expression across the spinal cord, as compared to bolus dosing at one or multiple sites. In one embodiment, dentate nucleus expression may increase with prolonged infusions.

### Single and Multiple Rounds of Dosing

In one embodiment, delivery of AAV particles to cells of the central nervous system (e.g., parenchyma) comprises prolonged infusion of at least one dose. In one embodiment, delivery comprises prolonged infusion of one dose. In one embodiment, delivery of AAV to cells of the central nervous system (e.g., parenchyma) may comprise prolonged infusion of 1, 2, 3, 4, 5, 6, 7, 8, 9, or 10 dose(s).

### Interval of Dosing

In one embodiment, delivery of AAV particles to cells of the central nervous system (e.g., parenchyma) comprising prolonged infusion of more than one dose further comprises an interval of at least one hour between doses. In one embodiment, delivery may comprise an interval of 1, 2, 3, 4, 5, 6, 7, 8, 9, 10, 11, 12, 13, 14, 15, 16, 17, 18, 19, 20, 21, 22, 23, 24, 30, 36, 42, 48, 54, 60, 66, 72, 78, 84, 90, 96, 108, or 120 hour(s) between doses. In one embodiment, delivery comprises an interval of 24 hours between doses. In one embodiment, delivery consists of three prolonged infusion doses at an interval of 24 hours.

### Infusion Patterns: Simple (Constant)

In one embodiment, delivery of AAV particles to cells of the central nervous system (e.g., parenchyma) may comprise a constant rate of prolonged infusion. As used herein, a "constant rate" is a rate that stays about the same during the prolonged infusion.

### Infusion Patterns: Ramped

In one embodiment, delivery of AAV particles to cells of the central nervous system (e.g., parenchyma) may comprise a ramped rate of prolonged infusion where the rate either increases or decreases over time. As a non-limiting example, the rate of prolonged infusion increases over time. As another non-limiting example, the rate of prolonged infusion decreases over time.

### Infusion Patterns: Complex

In one embodiment, delivery of AAV particles to cells of the central nervous system (e.g., parenchyma) may comprise a complex rate of prolonged infusion wherein the rate of prolonged infusion alternates between high and low rates of prolonged infusion over time.

### Prolonged Infusion Rate

In one embodiment, delivery of AAV particles to cells of the central nervous system (e.g., parenchyma) comprises a rate of delivery may be defined by [VG/hour = mL/hour ^{∗} VG/mL] wherein VG is viral genomes, VG/mL is composition concentration, and mL/hour is rate of prolonged infusion. In accordance with the present invention, the

In one embodiment, delivery of AAV to cells of the central nervous system (e.g., parenchyma) may comprise a rate of prolonged infusion between about 0.1 mL/hour and about 25.0 mL/hour (or higher if CSF pressure does not increase to dangerous levels). In some embodiments, delivery may comprise a rate of prolonged infusion of about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, 12.0, 12.1, 12.2, 12.3, 12.4, 12.5, 12.6, 12.7, 12.8, 12.9, 13.0, 13.1, 13.2, 13.3, 13.4, 13.5, 13.6, 13.7, 13.8, 13.9, 14.0, 14.1, 14.2, 14.3, 14.4, 14.5, 14.6, 14.7, 14.8, 14.9, 15.0, 15.1, 15.2, 15.3, 15.4, 15.5, 15.6, 15.7, 15.8, 15.9, 16.0, 16.1, 16.2, 16.3, 16.4, 16.5, 16.6, 16.7, 16.8, 16.9, 17.0, 17.1, 17.2, 17.3, 17.4, 17.5, 17.6, 17.7, 17.8, 17.9, 18.0, 18.1, 18.2, 18.3, 18.4, 18.5, 18.6, 18.7, 18.8, 18.9, 19.0, 19.1, 19.2, 19.3, 19.4, 19.5, 19.6, 19.7, 19.8, 19.9, 20.0, 20.1, 20.2, 20.3, 20.4, 20.5, 20.6, 20.7, 20.8, 20.9, 21.0, 21.1, 21.2, 21.3, 21.4, 21.5, 21.6, 21.7, 21.8, 21.9, 22.0, 22.1, 22.2, 22.3, 22.4, 22.5, 22.6, 22.7, 22.8, 22.9, 23.0, 23.1, 23.2, 23.3, 23.4, 23.5, 23.6, 23.7, 23.8, 23.9, 24.0, 24.1, 24.2, 24.3, 24.4, 24.5, 24.6, 24.7, 24.8, 24.9, or 25.0 mL/hour. In some embodiments, delivery may comprise a rate of prolonged infusion of about 10, 20 30, 40, or 50 mL/hr. In one embodiment, delivery of AAV particles to cells of the central nervous system (e.g., parenchyma) comprises a rate of prolonged infusion of 1.0 mL/hour. In one embodiment, delivery consists of a rate of prolonged infusion of 1.0 mL/hour. In one embodiment, delivery of AAV to cells of the central nervous system (e.g., parenchyma) comprises a rate of prolonged infusion of 1.5 mL/hour. In one embodiment, delivery of AAV particles to cells of the central nervous system (e.g., parenchyma) consists of a rate of prolonged infusion of 1.5 mL/hour.

### Prolonged Infusion Dosing: Total Dose

In one embodiment, delivery of AAV particles to cells of the central nervous system (e.g., parenchyma) comprises prolonged infusion of at least one dose, or two or more doses. The interval between doses may be at least one hour, or between 1 hour and 120 hours. In one embodiment, the total dose of viral genomes delivered to cells of the central nervous system (e.g., parenchyma) defined by the equation [Total Dose VG = VG/mL ^{∗} mL ^{∗} # of doses] wherein VG is viral genomes and VG/mL is viral genome concentration. In accordance with the present invention, the total dose may be between about 1x10⁶ VG and about 1x10¹⁶ VG.

### Infusion compositions

In some embodiments, a composition comprising AAV particles delivered to cells of the central nervous system (e.g., parenchyma) may have a certain range of concentrations, pH, baricity (i.e. density of solution), osmolarity, temperature, and other physiochemical and biochemical properties that benefit the delivery of AAV particles to cells of the central nervous system (e.g., parenchyma).

In one embodiment, delivery of AAV particles to cells of the central nervous system (e.g., parenchyma) may comprise a total dose between about 1x10⁶ VG and about 1x10¹⁶ VG. In some embodiments, delivery may comprise a total dose of about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6 x10⁷, 7 x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 1.9x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, 9x10¹⁰, 1x10¹¹, 2x10¹¹, 2.5x10¹¹, 3x10¹¹, 4x10¹¹, 5x10¹¹, 6x10¹¹, 7x10¹¹, 8x10¹¹, 9x10¹¹, 1x10¹², 2x10¹², 3x10¹², 4x10¹², 5x10¹², 6x10¹², 7x10¹², 8x10¹², 9x10¹², 1x10¹³, 2x10¹³, 3x10¹³, 4x10¹³, 5x10¹³, 6x10¹³, 7x10¹³, 8x10¹³, 9x10¹³, 1x10¹⁴, 2x10¹⁴, 3x10¹⁴, 4x10¹⁴, 5x10¹⁴, 6x10¹⁴, 7x10¹⁴, 8x10¹⁴, 9x10¹⁴, 1x10¹⁵, 2x10¹⁵, 3x10¹⁵, 4x10¹⁵, 5x10¹⁵, 6x10¹⁵, 7x10¹⁵, 8x10¹⁵, 9x10¹⁵, or 1x10¹⁶ VG. As a non-limiting example, the total dose is 1x10¹³ VG. As another non-limiting example, the total dose is 3x10¹³ VG. As another non-limiting example, the total dose is 3.73x10¹⁰ VG. As another non-limiting example, the total dose is 1.9x10¹⁰ VG. As another non-limiting example, the total dose is 2.5x10¹¹ VG. As another non-limiting example, the total dose is 5x10¹¹ VG. As another non-limiting example, the total dose is 1x10¹² VG. As another non-limiting example, the total dose is 5x10¹² VG.

In one embodiment, delivery of AAV particles to cells of the central nervous system (e.g., parenchyma) comprises a total dose of 5x10¹⁰ VG. In one embodiment, delivery consists of a total dose of 5x10¹⁰ VG. In one embodiment, delivery comprises a total dose of 3x10¹³ VG. In one embodiment, delivery of AAV to cells of the central nervous system (e.g., parenchyma) consists of a total dose of 3x10¹³ VG.

### Pressure

In one embodiment, delivery of AAV particles to cells of the central nervous system (e.g., parenchyma) may comprise a rate of prolonged infusion wherein the rate of prolonged infusion exceeds the rate of CSF absorption. In some embodiments, CSF pressure may increase wherein the rate of delivery is greater than the rate of clearance. In one embodiment, increased CSF pressure may increase delivery of AAV particles to cells of the central nervous system (e.g., parenchyma of brain and spinal cord). In one embodiment, delivery of AAV to cells of the central nervous system (e.g., parenchyma) may comprise an increase in sustained CSF pressure between about 1% and about 25%. In some embodiments, delivery may comprise an increase in sustained CSF pressure of about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, or 25%.

Although the descriptions of pharmaceutical compositions, e.g., AAV comprising a payload to be delivered, provided herein are principally directed to pharmaceutical compositions which are suitable for administration to humans, it will be understood by the skilled artisan that such compositions are generally suitable for administration to any other animal, e.g., to non-human animals, e.g. non-human mammals. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation. Subjects to which administration of the pharmaceutical compositions is contemplated include, but are not limited to, humans and/or other primates; mammals, including commercially relevant mammals such as cattle, pigs, horses, sheep, cats, dogs, mice, and/or rats; and/or birds, including commercially relevant birds such as poultry, chickens, ducks, geese, and/or turkeys.

In some embodiments, compositions are administered to humans, human patients or subjects. For the purposes of the present disclosure, the phrase "active ingredient" generally refers either to the viral particle carrying the payload or to the payload delivered by the viral particle as described herein.

Formulations of the AAV pharmaceutical compositions described herein may be prepared by any method known or hereafter developed in the art of pharmacology. In general, such preparatory methods include the step of bringing the active ingredient into association with an excipient and/or one or more other accessory ingredients, and then, if necessary and/or desirable, dividing, shaping and/or packaging the product into a desired single- or multi-dose unit.

Relative amounts of the active ingredient, the pharmaceutically acceptable excipient, and/or any additional ingredients in a pharmaceutical composition in accordance with the invention will vary, depending upon the identity, size, and/or condition of the subject treated and further depending upon the route by which the composition is to be administered.

### Prolonged Infusion Composition Concentration

In one embodiment, delivery of AAV particles to cells of the central nervous system (e.g., parenchyma) may comprise a composition concentration between about 1x10⁶ VG/mL and about 1x10¹⁶ VG/mL. In some embodiments, delivery may comprise a composition concentration of about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, 9x10¹⁰, 1x10¹¹, 2x10¹¹, 3x10¹¹, 4x10¹¹, 5x10¹¹, 6x10¹¹, 7x10¹¹, 8x10¹¹, 9x10¹¹, 1x10¹², 2x10¹², 3x10¹², 4x10¹², 5x10¹², 6x10¹², 7x10¹², 8x10¹², 9x10¹², 1x10¹³, 2x10¹³, 3x10¹³, 4x10¹³, 5x10¹³, 6x10¹³, 7x10¹³, 8x10¹³, 9x10¹³, 1x10¹⁴, 2x10¹⁴, 3x10¹⁴, 4x10¹⁴, 5x10¹⁴, 6x10¹⁴, 7x10¹⁴, 8x10¹⁴, 9x10¹⁴, 1x10¹⁵, 2x10¹⁵, 3x10¹⁵, 4x10¹⁵, 5x10¹⁵, 6x10¹⁵, 7x10¹⁵, 8x10¹⁵, 9x10¹⁵, or 1x10¹⁶ VG/mL. In one embodiment, delivery comprises a composition concentration of 1x10¹³ VG/mL. In one embodiment, delivery consists of a composition concentration of 1x10¹³ VG/mL. In one embodiment, delivery comprises a composition concentration of 3x10¹² VG/mL. In one embodiment, delivery consists of a composition concentration of 3x10¹² VG/mL.

### Composition pH

In one embodiment, delivery of AAV to cells of the central nervous system (e.g., parenchyma) comprises a buffered composition of between pH 4.5 and 8.0. In some embodiments, delivery may comprise a buffered composition of about pH 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0. In one embodiment, delivery comprises a buffered composition of pH 7.4, which is considered physiological pH. In one embodiment, delivery comprises a buffered composition of pH 7.0. In one embodiment, buffer strength, or ability to hold pH, is relatively very low allowing the infused composition to quickly adjust to the prevailing physiological pH of the CSF (∼pH7.4).

### Composition Baricity

It is known in the art that CSF comprises a baricity, or density of solution, of approximately 1g/mL at 37°C. In one embodiment, delivery of AAV particles to cells of the central nervous system (e.g., parenchyma) comprises an isobaric composition wherein the baricity of the composition at 37°C is approximately 1g/mL. In one embodiment, delivery comprises a hypobaric composition wherein the baricity of the composition at 37°C is less than 1g/mL. In one embodiment, delivery comprises a hyperbaric composition wherein the baricity of the composition at 37°C is greater than 1g/mL. In one embodiment, delivery comprises a hyperbaric composition wherein the baricity of the composition at 37°C is increased by addition of approximately 5% to 8% dextrose. In one embodiment, delivery comprises a hyperbaric composition wherein the baricity of the composition at 37° C is increased by addition of 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, or 8.0% dextrose.

### Composition Osmolarity

In one embodiment, delivery of AAV particles to cells of the central nervous system (e.g., parenchyma) comprises co-administration of intravenous agents that increase serum osmolarity. As used herein, "co-administered" means the administration of two or more components. Co-administration refers to the administration of two or more components simultaneously or with a time lapse between administration such as 1 second, 5 seconds, 10 seconds, 15 seconds, 30 seconds, 45 seconds, 1 minute, 2 minutes, 3 minutes, 4 minutes, 5 minutes, 6 minutes, 7 minutes, 8 minutes, 9 minutes, 10 minutes, 11 minutes, 12 minutes, 13 minutes, 14 minutes, 15 minutes, 16 minutes, 17 minutes, 18 minutes, 19 minutes, 20 minutes, 21 minutes, 22 minutes, 23 minutes, 24 minutes, 25 minutes, 26 minutes, 27 minutes, 28 minutes, 29 minutes, 30 minutes, 31 minutes, 32 minutes, 33 minutes, 34 minutes, 35 minutes, 36 minutes, 37 minutes, 38 minutes, 39 minutes, 40 minutes, 41 minutes, 42 minutes, 43 minutes, 44 minutes, 45 minutes, 46 minutes, 47 minutes, 48 minutes, 49 minutes, 50 minutes, 51 minutes, 52 minutes, 53 minutes, 54 minutes, 55 minutes, 56 minutes, 57 minutes, 58 minutes, 59 minutes, 1 hour, 1.5 hours, 2 hours, 2.5 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 1 day, 1.5 days, 2 days, or more than 3 days.

In one embodiment, delivery comprises co-administration of intravenous mannitol. In one embodiment, delivery comprises co-administration of approximately 0.25 to 1.0 g/kg intravenous mannitol. In one embodiment, delivery comprises co-administration of 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.50, 0.51, 0.52, 0.53, 0.54, 0.55, 0.56, 0.57, 0.58, 0.59, 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99, 1.00 g/ kg intravenous mannitol.

### Composition Temperature

In one embodiment, delivery of AAV particles to cells of the central nervous system (e.g., parenchyma) comprises a composition wherein the temperature of the composition is 37°C. In one embodiment, delivery comprises a composition wherein the temperature of the composition is between approximately 20°C and 26°C. In one embodiment, delivery comprises a composition wherein the temperature of the composition is approximately 20.0°C, 20.1°C, 20.2°C, 20.3°C, 20.4°C, 20.5°C, 20.6°C, 20.7°C, 20.8°C, 20.9°C, 21.0°C, 21.1°C, 21.2°C, 21.3°C, 21.4°C, 21.5°C, 21.6°C, 21.7°C, 21.8°C, 21.9°C, 22.0°C, 22.1°C, 22.2°C, 22.3°C, 22.4°C, 22.5°C, 22.6°C, 22.7°C, 22.8°C, 22.9°C, 23.0°C, 23.1°C, 23.2°C, 23.3°C, 23.4°C, 23.5°C, 23.6°C, 23.7°C, 23.8°C, 23.9°C, 24.0°C, 24.1°C, 24.2°C, 24.3°C, 24.4°C, 24.5°C, 24.6°C, 24.7°C, 24.8°C, 24.9°C, 25.0°C, 25.1°C, 25.2°C, 25.3°C, 25.4°C, 25.5°C, 25.6°C, 25.7°C, 25.8°C, 25.9°C, or 26.0°C.

### Drug Physiochemical & Biochemical Properties

In one embodiment, delivery of parvovirus e.g., AAV particles to cells of the central nervous system (e.g., parenchyma) comprises a composition wherein the AAV capsid is hydrophilic. In one embodiment, delivery comprises a composition wherein the AAV capsid is lipophilic.

In one embodiment, delivery of AAV particles to cells of the central nervous system (e.g., parenchyma) comprises a composition wherein the AAV capsid targets a specific receptor. In one embodiment, delivery of AAV particles to cells of the central nervous system (e.g., parenchyma) comprises a composition wherein the AAV capsid further comprises a specific ligand.

In some embodiments, delivery of AAV particles to cells of the central nervous system (e.g., parenchyma) comprises a composition wherein the AAV capsid may be, but is not limited to, AAV1, AAV2, AAV3, AAV4, AAV5, AAV6, AAV7, AAV8, AAV9, AAV10, AAV11, AAV12, AAVrh8, AAVrh10, AAV-DJ, and/or AAV-DJ8.

In one embodiment, delivery of AAV particles to cells of the central nervous system (e.g., parenchyma) comprises a composition wherein the AAV further comprises a self-complementary (SC) genome. In one embodiment, delivery comprises a composition wherein the AAV further comprises a single stranded (SS) genome.

In one embodiment, a self-complementary (sc) vector may be used to yield higher expression than the corresponding single stranded vector.

In one embodiment, delivery of AAV particles to cells of the central nervous system (e.g., parenchyma) comprises a composition wherein the AAV genome further comprises a cell specific promoter region. In one embodiment, delivery comprises a composition wherein the AAV genome further comprises a ubiquitous promoter region.

### Spatial Orientation: Body Angle

In one embodiment, delivery of AAV particles to cells of the central nervous system (e.g., parenchyma) comprises administration to a horizontal subject. In one embodiment, delivery comprises administration to a vertical subject. In one embodiment, delivery comprises administration to a subject at an angle between approximately horizontal 0° to about vertical 90°. In one embodiment, delivery comprises administration to a subject at an angle of 0°, 1°, 2°, 3°, 4°, 5°, 6°, 7°, 8°, 9°, 10°, 11°, 12°, 13°, 14°, 15°, 16°, 17°, 18°, 19°, 20°, 21°, 22°, 23°, 24°, 25°, 26°, 27°, 28°, 29°, 30°, 31°, 32°, 33°, 34°, 35°, 36°, 37°, 38°, 39°, 40°, 41°, 42°, 43°, 44°, 45°, 46°, 47°, 48°, 49°, 50°, 51°, 52°, 53°, 54°, 55°, 56°, 57°, 58°, 59°, 60°, 61°, 62°, 63°, 64°, 65°, 66°, 67°, 68°, 69°, 70°, 71°, 72°, 73°, 74°, 75°, 76°, 77°, 78°, 79°, 80°, 81°, 82°, 83°, 84°, 85°, 86°, 87°, 88°, 89°, 90°.

### Spatial Orientation: Change in the Orientation/Slope of Subject Body Position over Time

In one embodiment, delivery of AAV particles to cells of the central nervous system (e.g., parenchyma) comprises administration to a subject wherein the angle of the subject changes over time from horizontal to vertical head up or vertical head down. In one embodiment, delivery comprises administration to a subject wherein the angle of the subject changes over time from vertical to horizontal.

In one embodiment, delivery comprises administration to a subject wherein the angle of the subject changes over time in two planes from vertical to horizontal as well as rotation around the long axis of the body. In combination, any % angle of the body can be realized between horizontal to vertical and rotationally left or right.

### Delivery Devices

In some embodiments, delivery of AAV particles to cells of the central nervous system (e.g., parenchyma) comprises a prolonged infusion pump or device. In some embodiments, the device may be a pump or comprise a catheter for administration of compositions of the invention across the blood brain barrier. Such devices include but are not limited to a pressurized olfactory delivery device, iontophoresis devices, multi-layered microfluidic devices, and the like. Such devices may be portable or stationary. They may be implantable or externally tethered to the body or combinations thereof.

Devices for administration may be employed for delivery of AAV particles to cells of the central nervous system (e.g., parenchyma) according to the present invention according to single, multi- or split-dosing regimens taught herein.

Method and devices known in the art for multi-administration to cells, organs and tissues are contemplated for use in conjunction with the methods and compositions disclosed herein as embodiments of the present invention. These include, for example, those methods and devices having multiple needles, hybrid devices employing for example lumens or catheters as well as devices utilizing heat, electric current or radiation driven mechanisms.

In one embodiment, the AAV particles may be delivered using an infusion port described herein and/or one that is known in the art.

In one embodiment, the AAV particles may be delivered using an infusion pump and/or an infusion port. The infusion pump and/or the infusion port may be one described herein or one known in the art such as, but not limited to, SYNCHROMED® II by Medtronic. The infusion pump may be programmed at a fixed rate or a variable rate for controlled delivery. The stability of the AAV particles and formulations thereof as well as the leachable materials should be evaluated prior to use.

In one embodiment, the devices described herein to deliver to a subject the above-described AAV particles may also include a tip protection device (e.g., for catheters and/or stereotactic fixtures of microcatheters). Non-limiting examples of protection devices are described in US Patent Publication No. US20140371711 and International Patent Publication No. WO2014204954, the contents of each of which are herein incorporated by reference in their entireties. The tip protection device may include an elongate body having a central lumen extending longitudinally therethrough, the lumen being sized and configured to slidably receive a catheter, and a locking mechanism configured to selectively maintain the elongate body in a fixed longitudinal position relative to a catheter inserted through the central lumen.

In one embodiment, the AAV particles may be delivered to a subject using a convection-enhanced delivery device. Non-limiting examples of targeted delivery of drugs using convection are described in US Patent Publication Nos. US20100217228, US20130035574 and US20130035660 and International Patent Publication No. WO2013019830 and WO2008144585, the contents of each of which are herein incorporated by reference in their entireties. The convection-enhanced delivery device may be a microfluidic catheter device that may be suitable for targeted delivery of drugs via convection, including devices capable of multi-directional drug delivery, devices that control fluid pressure and velocity using the venturi effect, and devices that include conformable balloons. As a non-limiting example, the convention-enhanced delivery device uses the venturi effect for targeted delivery of drugs as described in US Patent Publication No. US20130035574, the contents of which are herein incorporation by reference in its entirety. As another non-limiting example, the convention-enhanced delivery device uses the conformable balloons for targeted delivery of drugs as described in US Patent Publication No. US20130035660, the contents of which are herein incorporation by reference in its entirety. As another non-limiting example, the convection enhanced delivery device may be a CED catheter from Medgenesis Therapeutix such as those described in International Patent Publication No. WO2008144585 and US Patent No. US20100217228, the contents of each of which are herein incorporated by reference in their entireties. As another non-limiting example, the AAV particles may be in a liposomal composition for convection enhanced delivery such as the liposomal compositions from Medgenesis Therapeutix described in International Patent Publication No. WO2010057317 and US Patent No. US20110274625, the contents of each of which are herein incorporated by reference in their entireties, which may comprise a molar ratio of DSPC:DSPG:CHOL of 7:2:1.

In one embodiment, the catheter may be a neuromodulation catheter. Non-limiting examples of neuromodulation catheters include those taught in US Patent Application No. US20150209104 and International Publication Nos. WO2015143372, WO2015113027, WO2014189794 and WO2014150989, the contents of each of which are herein incorporated by reference in their entireties.

In one embodiment, the AAV particles may be delivered using an injection device which has a basic form of a stiff tube with holes of a selectable size at selectable places along the tube. This is a device which may be customized depending on the subject or the fluid being delivered. As anon-limiting example, the injection device which comprises a stiff tube with holes of a selectable size and location may be any of the devices described in US Patent Nos. 6,464,662, 6,572,579 and International Patent Publication No. WO2002007809, the contents of each of which are herein incorporated by reference in their entireties.

In one embodiment, the AAV particles may be delivered to a subject who is using or who has used a treatment stimulator for brain diseases. Non-limiting examples include treatment stimulators from THERATAXIS™ and the treatment stimulators described in International Patent Publication No. WO2008144232, the contents of which are herein incorporated by reference in its entirety.

In one embodiment, the AAV particles may be delivered to a defined area using a medical device which comprises a sealing system proximal to the delivery end of the device. Non-limiting examples of medical device with can deliver AAV particles to a defined area includes US Patent No. 7,998,128, US Patent Application No. US20100030102 and International Patent Publication No. WO2007133776, the contents of each of which are herein incorporated by reference in their entireties.

In one embodiment, the AAV particle may be delivered over an extended period of time using an extended delivery device. Non-limiting examples of extended delivery devices are described in International Patent Publication Nos. WO2015017609 and WO2014100157, US Patent No. 8,992,458, and US Patent Publication Nos. US20150038949, US20150133887 and US20140171902, the contents of each of which are herein incorporated by reference in their entireties. As a non-limiting example, the devices used to deliver the AAV particles are CED devices with various features for reducing or preventing backflow as in International Patent Publication No. WO2015017609 and US Patent Publication No. US20150038949, the contents of each of which are herein incorporated by reference in their entireties. As another non-limiting example, the devices used to deliver the AAV particles are CED devices which include a bullet-shaped nose proximal to a distal fluid outlet where the bullet-shaped nose forms a good seal with surrounding tissue and helps reduce or prevent backflow of infused fluid as described in US Patent No. 8,992,458, US Patent Publication Nos. US20150133887 and US20140171902 and International Patent Publication No. WO2014100157, the contents of each of which are herein incorporated by reference by their entireties. As another non-limiting example, the catheter may be made using micro-electro-mechanical systems (MEMS) technology to reduce backflow as described by Brady et al. (Journal of Neuroscience Methods 229 (2014) 76-83), the contents of which are herein incorporated by reference in its entirety.

In one embodiment, the AAV particles may be delivered using an implantable delivery device. Non-limiting examples of implantable devices are described by and sold by Codman Neuro Sciences (Le Locle, CH). The implantable device may be an implantable pump such as, but not limited to, those described in US Patent Nos. 8,747,391, 7,931,642, 7,637,897, and 6,755,814 and US Patent Publication No. US20100069891, the contents of each of which are herein incorporated by reference in their entireties. The implantable device (e.g., a fluidic system) may have the flow rate accuracy of the device optimized by the methods described in US Patent No. 8,740,182 and 8,240,635, and US Patent Publication No. US20120283703, the contents of each of which are herein incorporated by reference in its entirety. As a non-limiting example, the duty cycle of the valve of a system may be optimized to achieve the desired flow rate. The implantable device may have an electrokinetic actuator for adjusting, controlling or programming fine titration of fluid flow through a valve mechanism without intermixing between the electrolyte and fluid. As a non-limiting example, the electrokinetic actuator may be any of those described in US Patent No. 8,231,563 and US Patent Publication No. US20120283703, the contents of which are herein incorporated by reference in its entirety. Fluids of an implantable infusion pump may be monitored using methods known in the art and those taught in US Patent No. 7,725,272, the contents of which are herein incorporated by reference in its entirety.

In one embodiment, the delivery of the AAV particles in a subject may be determined and/or predicted using the prediction methods described in International Patent Publication No. WO2001085230, the contents of which are herein incorporated by reference in its entirety.

In one embodiment, a subject may be imaged prior to, during and/or after administration of the AAV particles. The imaging method may be a method known in the art and/or described herein. As a non-limiting example, the imaging method which may be used to classify brain tissue includes the medical image processing method described in US Patent Nos. 7,848,543, 9,101,282 and EP Application No. EP1768041, the contents of each of which are herein incorporated by reference in their entireties. As yet another non-limiting example, the physiological states and the effects of treatment of a neurological disease in a subject may be tracked using the methods described in US Patent Publication No. US20090024181, the contents of which are herein incorporated by reference in its entirety.

In one embodiment, a device may be used to deliver the AAV particles where the device creates one or more channels, tunnels or grooves in tissue in order to increase hydraulic conductivity. These channels, tunnels or grooves will allow the AAV particles to flow and produce a predictable infusion pattern. Non-limiting examples of this device is described in US Patent No. 8,083,720, US Patent Application No. US20110106009, and International Publication No. WO2009151521, the contents of each of which are herein incorporated by reference in its entirety.

In one embodiment, the flow of a composition comprising the AAV particles may be controlled using acoustic waveform outside the target area. Non-limiting examples of devices, methods and controls for using sonic guidance to control molecules is described in US Patent Application No. US20120215157, US Patent No. US 8,545,405, International Patent Publication Nos. WO2010096495 and WO2010080701, the contents of each of which are herein incorporated by reference in their entireties.

In one embodiment, the flow of a composition comprising the AAV particles may be modeled prior to administration using the methods and apparatus described in US Patent No. 6,549,803 and 8,406,850 and US Patent Application No. US20080292160, the content of each of which is incorporated by reference in their entireties. As a non-limiting example, the physiological parameters defining edema induced upon infusion of fluid from an intraparenchymally placed catheter may be estimated using the methods described in US Patent No. 8,406,850 and US Patent Application No. US20080292160, the contents of which is herein incorporated by reference in its entirety.

In one embodiment, a surgical alignment device may be used to deliver the AAV particles to a subject. The surgical alignment device may be a device described herein and/or is known in the art. As a non-limiting example, the surgical alignment device may be controlled remotely (i.e., robotic) such as the alignment devices described in US Patent Nos. 7,366,561 and 8,083,753, the contents of each of which is incorporated by reference in their entireties.

In one embodiment, an intraparenchymal (IPA) catheter from Alcyone may be used to deliver the AAV particles described herein.

In another embodiment, an intraparenchymal catheter from Atanse may be used to deliver the AAV particles described herein.

In one embodiment, the distribution of the AAV particles described herein may be evaluated using imaging technology from Therataxis and/or Brain Lab.

### Treatment and pharmaceutical compositions

### Formulation

The AAV particles of the invention can be formulated using one or more excipients to: (1) increase stability; (2) increase cell transfection or transduction; (3) permit the sustained or delayed release; (4) alter the biodistribution (e.g., target the viral particle to specific tissues or cell types); (5) increase the translation of encoded protein *in vivo*; (6) alter the release profile of encoded protein *in vivo* and/or (7) allow for regulatable expression of the payload.

Formulations of the present invention can include, without limitation, saline, liposomes, lipid nanoparticles, polymers, peptides, proteins, cells transfected with viral vectors (e.g., for transplantation into a subject) and combinations thereof.

Formulations of the pharmaceutical compositions described herein may be prepared by any method known or hereafter developed in the art of pharmacology. In general, such preparatory methods include the step of associating the active ingredient with an excipient and/or one or more other accessory ingredients.

In one embodiment, the AAV particles of the invention may be formulated in PBS with 0.001% of pluronic acid (F-68) at a pH of about 7.0.

A pharmaceutical composition in accordance with the present disclosure may be prepared, packaged, and/or sold in bulk, as a single unit dose, and/or as a plurality of single unit doses. As used herein, a "unit dose" refers to a discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient (e.g. AAV particle), the pharmaceutically acceptable excipient, and/or any additional ingredients in a pharmaceutical composition in accordance with the present disclosure may vary, depending upon the identity, size, and/or condition of the subject being treated and further depending upon the route by which the composition is to be administered. For example, the composition may comprise between 0.1% and 99% (w/w) of the active ingredient. By way of example, the composition may comprise between 0.1% and 100%, e.g., between .5 and 50%, between 1-30%, between 5-80%, at least 80% (w/w) active ingredient.

In some embodiments, the AAV particle formulations described herein may contain a nucleic acid encoding at least one payload. As a non-limiting example, the formulations may contain a nucleic acid encoding 1, 2, 3, 4 or 5 payloads. In one embodiment the formulation may contain a nucleic acid encoding a payload construct encoding proteins selected from categories such as, but not limited to, human proteins, veterinary proteins, bacterial proteins, biological proteins, antibodies, immunogenic proteins, therapeutic peptides and proteins, secreted proteins, plasma membrane proteins, cytoplasmic and cytoskeletal proteins, intracellular membrane bound proteins, nuclear proteins, proteins associated with human disease and/or proteins associated with non-human diseases. In one embodiment, the formulation contains at least three payload constructs encoding proteins.

The formulations of the invention can include one or more excipients, each in an amount that together increases the stability of the AAV particle, increases cell transfection or transduction by the viral particle, increases the expression of viral particle encoded protein, and/or alters the release profile of AAV particle encoded proteins. In some embodiments, a pharmaceutically acceptable excipient may be at least 95%, at least 96%, at least 97%, at least 98%, at least 99%, or 100% pure. In some embodiments, an excipient is approved for use for humans and for veterinary use. In some embodiments, an excipient may be approved by United States Food and Drug Administration. In some embodiments, an excipient may be of pharmaceutical grade. In some embodiments, an excipient may meet the standards of the United States Pharmacopoeia (USP), the European Pharmacopoeia (EP), the British Pharmacopoeia, and/or the International Pharmacopoeia.

Excipients, which, as used herein, includes, but is not limited to, any and all solvents, dispersion media, diluents, or other liquid vehicles, dispersion or suspension aids, surface active agents, isotonic agents, thickening or emulsifying agents, preservatives, and the like, as suited to the particular dosage form desired. Various excipients for formulating pharmaceutical compositions and techniques for preparing the composition are known in the art (see Remington: The Science and Practice of Pharmacy, 21st Edition, A. R. Gennaro, Lippincott, Williams & Wilkins, Baltimore, MD, 2006; incorporated herein by reference in its entirety). The use of a conventional excipient medium may be contemplated within the scope of the present disclosure, except insofar as any conventional excipient medium may be incompatible with a substance or its derivatives, such as by producing any undesirable biological effect or otherwise interacting in a deleterious manner with any other component(s) of the pharmaceutical composition.

### Inactive Ingredients

In some embodiments, AAV formulations may comprise at least one excipient which is an inactive ingredient. As used herein, the term "inactive ingredient" refers to one or more agents that do not contribute to the activity of the pharmaceutical composition included in formulations. In some embodiments, all, none or some of the inactive ingredients which may be used in the formulations of the present invention may be approved by the US Food and Drug Administration (FDA).

Formulations of AAV particles disclosed herein may include cations or anions. In one embodiment, the formulations include metal cations such as, but not limited to, Zn2+, Ca2+, Cu2+, Mg+ and combinations thereof.

### Administration

The AAV particles of the present invention may be administered by any route which results in a therapeutically effective outcome. These include, but are not limited to epidural, peridural, subdural (in particular delivery of AAV over one or more targeted regions of the neocortex), intracerebral (into the cerebrum), intracerebroventricular (into the cerebral ventricles), intrathecal (into the spinal canal or within the cerebrospinal fluid at any level of the cerebrospinal axis), intradiscal (within a disc), intradural (within or beneath the dura), intraspinal (within the vertebral column), caudal block, diagnostic, nerve block, or spinal. In specific embodiments, compositions may be administered in a way which allows them cross the blood-brain barrier, vascular barrier, or other epithelial barrier. In one embodiment, a formulation for a route of administration may include at least one inactive ingredient.

In one embodiment, the AAV particles may be delivered by systemic delivery. As a non-limiting example, the systemic delivery may be by intravascular administration.

In one embodiment, the AAV particles may be delivered by injection into the CSF pathway. Non-limiting examples of delivery to the CSF pathway include intrathecal and intracerebroventricular administration.

In one embodiment, the AAV particles may be delivered by direct injection into the brain. As a non-limiting example, the brain delivery may be by intrastriatal administration.

In one embodiment, the AAV particles may delivered to a subject using a device to deliver the AAV particles and a head fixation assembly. The head fixation assembly may be, but is not limited to, any of the head fixation assemblies sold by MRI interventions. As a non-limiting example, the head fixation assembly may be any of the assemblies described in US Patent Nos. 8,099,150, 8,548,569 and 9,031,636 and International Patent Publication Nos. WO201108495 and WO2014014585, the contents of each of which are incorporated by reference in their entireties. A head fixation assembly may be used in combination with an MRI compatible drill such as, but not limited to, the MRI compatible drills described in International Patent Publication No. WO2013181008 and US Patent Publication No. US20130325012, the contents of which are herein incorporated by reference in its entirety.

In one embodiment, the AAV particles may be delivered using a method, system and/or computer program for positioning apparatus to a target point on a subject to deliver the AAV particles. As a non-limiting example, the method, system and/or computer program may be the methods, systems and/or computer programs described in US Patent No. 8, 340,743, the contents of which are herein incorporated by reference in its entirety. The method may include: determining a target point in the body and a reference point, wherein the target point and the reference point define a planned trajectory line (PTL) extending through each; determining a visualization plane, wherein the PTL intersects the visualization plane at a sighting point; mounting the guide device relative to the body to move with respect to the PTL, wherein the guide device does not intersect the visualization plane; determining a point of intersection (GPP) between the guide axis and the visualization plane; and aligning the GPP with the sighting point in the visualization plane.

In one embodiment, the AAV particles may be delivered using an MRI-guided device. Non-limiting examples of MRI-guided devices are described in US Patent Nos. 9,055,884, 9,042,958, 8,886,288, 8,768,433, 8,396,532, 8,369,930, 8,374,677 and 8,175,677 and US Patent Application No. US20140024927 the contents of each of which are herein incorporated by reference in their entireties. As a non-limiting example, the MRI-guided device may be able to provide data in real time such as those described in US Patent Nos. 8,886,288 and 8,768,433, the contents of each of which is herein incorporated by reference in its entirety. As another non-limiting example, the MRI-guided device or system may be used with a targeting cannula such as the systems described in US Patent Nos. 8,175,677 and 8,374,677, the contents of each of which are herein incorporated by reference in their entireties. As yet another non-limiting example, the MRI-guided device includes a trajectory guide frame for guiding an interventional device as described, for example, in US Patent No. 9,055,884 and US Patent Application No. US20140024927, the contents of each of which are herein incorporated by reference in their entireties.

In one embodiment the AAV particles may be delivered using an MRI-compatible tip assembly. Non-limiting examples of MRI-compatible tip assemblies are described in US Patent Publication No. US20140275980, the contents of which is herein incorporated by reference in its entirety.

In one embodiment, the AAV particles may be delivered using a cannula which is MRI-compatible. Non-limiting examples of MRI-compatible cannulas include those taught in International Patent Publication No. WO2011130107, the contents of which are herein incorporated by reference in its entirety.

In one embodiment, the AAV particles may be delivered using a catheter which is MRI-compatible. Non-limiting examples of MRI-compatible catheters include those taught in International Patent Publication No. WO2012116265, US Patent Publication No. 8,825,133 and US Patent Publication No. US20140024909, the contents of each of which are herein incorporated by reference in their entireties.

In one embodiment, the AAV particles may be delivered using a device with an elongated tubular body and a diaphragm as described in US Patent Publication Nos. US20140276582 and US20140276614, the contents of each of which are herein incorporated by reference in their entireties.

In one embodiment, the AAV particles may be delivered using an MRI compatible localization and/or guidance system such as, but not limited to, those described in US Patent Publication Nos. US20150223905 and US20150230871, the contents of each of which are herein incorporated by reference in their entireties. As a non-limiting example, the MRI compatible localization and/or guidance systems may comprise a mount adapted for fixation to a patient, a targeting cannula with a lumen configured to attach to the mount so as to be able to controllably translate in at least three dimensions, and an elongate probe configured to snugly advance via slide and retract in the targeting cannula lumen, the elongate probe comprising at least one of a stimulation or recording electrode.

In one embodiment, the AAV particles may be delivered to a subject using a trajectory frame as described in US Patent Publication Nos. US20150031982 and US20140066750 and International Patent Publication Nos. WO2015057807 and WO2014039481, the contents of each of which are herein incorporated by reference in their entireties.

In one embodiment, the AAV particles may be delivered to a subject to improve and/or correct mitochondrial dysfunction.

In one embodiment, the AAV particles may be delivered to a subject to preserve neurons. The neurons may be primary and/or secondary sensor neurons.

In one embodiment, administration of the AAV particles may preserve and/or correct function in the sensory pathways.

In one embodiment, administration of the AAV particles may protect central pathways from degeneration. As a non-limiting example, the degeneration is later onset degeneration of auditory pathways.

### Dosing

The present invention provides methods of administering AAV particles in accordance with the invention to a subject in need thereof. AAV particle pharmaceutical, imaging, diagnostic, or prophylactic compositions thereof, may be administered to a subject using any amount and any route of administration effective for preventing, treating, diagnosing, or imaging a disease, disorder, and/or condition (e.g., a disease, disorder, and/or condition relating to working memory deficits). The exact amount required will vary from subject to subject, depending on the species, age, and general condition of the subject, the severity of the disease, the particular composition, its mode of administration, its mode of activity, and the like. Compositions in accordance with the invention are typically formulated in unit dosage form for ease of administration and uniformity of dosage. It will be understood, however, that the total daily usage of the compositions of the present invention may be decided by the attending physician within the scope of sound medical judgment. The specific therapeutically effective, prophylactically effective, or appropriate imaging dose level for any particular patient will depend upon a variety of factors including the disorder being treated and the severity of the disorder; the activity of the specific payload employed; the specific composition employed; the age, body weight, general health, sex and diet of the patient; the time of administration, route of administration, and rate of excretion of the specific payload employed; the duration of the treatment; drugs used in combination or coincidental with the specific payload employed; and like factors well known in the medical arts.

In certain embodiments, AAV particle pharmaceutical compositions in accordance with the present invention may be administered at dosage levels sufficient to deliver from about 0.0001 mg/kg to about 100 mg/kg, from about 0.001 mg/kg to about 0.05 mg/kg, from about 0.005 mg/kg to about 0.05 mg/kg, from about 0.001 mg/kg to about 0.005 mg/kg, from about 0.05 mg/kg to about 0.5 mg/kg, from about 0.01 mg/kg to about 50 mg/kg, from about 0.1 mg/kg to about 40 mg/kg, from about 0.5 mg/kg to about 30 mg/kg, from about 0.01 mg/kg to about 10 mg/kg, from about 0.1 mg/kg to about 10 mg/kg, or from about 1 mg/kg to about 25 mg/kg, of subject body weight per day, one or more times a day, to obtain the desired therapeutic, diagnostic, prophylactic, or imaging effect. It will be understood that the above dosing concentrations may be converted to vg or viral genomes per kg or into total viral genomes administered by one of skill in the art.

As used herein, a "single unit dose" is a dose of any therapeutic administered in one dose/at one time/single route/single point of contact, i.e., single administration event. As used herein, a "total daily dose" is an amount given or prescribed in 24 hour period. It may be administered as a single unit dose. The viral particles may be formulated in buffer only or in a formulation described herein.

In one embodiment, delivery of the AAV particles described herein results in minimal serious adverse events (SAEs) as a result of the delivery of the AAV particles.

In one embodiment, a subject has had a low incidence of mild to moderate adverse events (AEs) near the time of the administration of the AAV particles. The subject may have had a low incidence of mild to moderate AEs within minutes (e.g., 1, 5, 10, 15, 20, 25, 30, 35, 40, 45, 50, 55 or 60 minutes), hours (1, 2, 3, 4, 5, 6, 7, 8, 9, 10, 11, 12, 13, 14, 15, 16, 17, 18, 19, 20, 21, 22, 23, or 24 hours) or days (1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 days).

In one embodiment, delivery of AAV particles to cells of the central nervous system (e.g., parenchyma) may comprise a total dose between about 1x10⁶ VG and about 1x10¹⁶ VG. In some embodiments, delivery may comprise a total dose of about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 1.9x10¹⁰, 2x10¹⁰, 3x10¹⁰, 3.73x10¹⁰, 4x10¹⁰, 5x10¹⁰, 6x10¹⁰, 7x10¹⁰, 8x10¹⁰, 9x10¹⁰, 1x10¹¹, 2x10¹¹, 2.5x10¹¹, 3x10¹¹, 4x10¹¹, 5x10¹¹, 6x10¹¹, 7x10¹¹, 8x10¹¹, 9x10¹¹, 1x10¹², 2x10¹², 3x10¹², 4x10¹², 5x10¹², 6x10¹², 7x10¹², 8x10¹², 9x10¹², 1x10¹³, 2x10¹³, 3x10¹³, 4x10¹³, 5x10¹³, 6x10¹³, 7x10¹³, 8x10¹³, 9x10¹³, 1x10¹⁴, 2x10¹⁴, 3x10¹⁴, 4x10¹⁴, 5x10¹⁴, 6x10¹⁴, 7x10¹⁴, 8x10¹⁴, 9x10¹⁴, 1x10¹⁵, 2x10¹⁵, 3x10¹⁵, 4x10¹⁵, 5x10¹⁵, 6x10¹⁵, 7x10¹⁵, 8x10¹⁵, 9x10¹⁵, or 1x10¹⁶ VG. As a non-limiting example, the total dose is 1x10¹³ VG. As another non-limiting example, the total dose is 3x10¹³ VG. As another non-limiting example, the total dose is 3.73x10¹⁰VG. As another non-limiting example, the total dose is 1.9x10¹⁰ VG. As another non-limiting example, the total dose is 2.5x10¹¹ VG. As another non-limiting example, the total dose is 5x10¹¹ VG. As another non-limiting example, the total dose is 1x10¹² VG. As another non-limiting example, the total dose is 5x10¹² VG.

In one embodiment, delivery of AAV particles to cells of the central nervous system (e.g., parenchyma) may comprise a composition concentration between about 1x10⁶ VG/mL and about 1x10¹⁶ VG/mL. In some embodiments, delivery may comprise a composition concentration of about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, 9x10¹⁰, 1x10¹¹, 2x10¹¹, 3x10¹¹, 4x10¹¹, 5x10¹¹, 6x10¹¹, 7x10¹¹, 8x10¹¹, 9x10¹¹, 1x10¹², 2x10¹², 3x10¹², 4x10¹², 5x10¹², 6x10¹², 7x10¹², 8x10¹², 9x10¹², 1x10¹³, 2x10¹³, 3x10¹³, 4x10¹³, 5x10¹³, 6x10¹³, 7x10¹³, 8x10¹³, 9x10¹³, 1x10¹⁴, 2x10¹⁴, 3x10¹⁴, 4x10¹⁴, 5x10¹⁴, 6x10¹⁴, 7x10¹⁴, 8x10¹⁴, 9x10¹⁴, 1x10¹⁵, 2x10¹⁵, 3x10¹⁵, 4x10¹⁵, 5x10¹⁵, 6x10¹⁵, 7x10¹⁵, 8x10¹⁵, 9x10¹⁵, or 1x10¹⁶ VG/mL. In one embodiment, the delivery comprises a composition concentration of 1x10¹³ VG/mL. In one embodiment, the delivery comprises a composition concentration of 3x10¹² VG/mL

In one embodiment, delivery of AAV particles to cells of the central nervous system (e.g., parenchyma) may comprise a composition concentration between about 1x10⁶ VG/uL and about 1x10¹⁶ VG/uL. In some embodiments, delivery may comprise a composition concentration of about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, 9x10¹⁰, 1x10¹¹, 2x10¹¹, 3x10¹¹, 4x10¹¹, 5x10¹¹, 6x10¹¹, 7x10¹¹, 8x10¹¹, 9x10¹¹, 1x10¹², 2x10¹², 3x10¹², 4x10¹², 5x10¹², 6x10¹², 7x10¹², 8x10¹², 9x10¹², 1x10¹³, 2x10¹³, 3x10¹³, 4x10¹³, 5x10¹³, 6x10¹³, 7x10¹³, 8x10¹³, 9x10¹³, 1x10¹⁴, 2x10¹⁴, 3x10¹⁴, 4x10¹⁴, 5x10¹⁴, 6x10¹⁴, 7x10¹⁴, 8x10¹⁴, 9x10¹⁴, 1x10¹⁵, 2x10¹⁵, 3x10¹⁵, 4x10¹⁵, 5x10¹⁵, 6x10¹⁵, 7x10¹⁵, 8x10¹⁵, 9x10¹⁵, or 1x10¹⁶ VG/uL. In one embodiment, the delivery comprises a composition concentration of 1x10¹³ VG/uL. In one embodiment, the delivery comprises a composition concentration of 3x10¹² VG/uL. In one embodiment, the delivery comprises a composition concentration of 1.9x10¹⁰ VG/10 uL. In one embodiment, the delivery comprises a composition concentration of 2.5x10¹¹ VG/100 uL. In one embodiment, the delivery comprises a composition concentration of 5x10¹¹ VG/100 uL.

In one embodiment, the dosage delivered to a subject may take into account the amount of backflow of the substance. As a non-limiting example, the method for determining the backflow of a substance or fluid along a track of a delivery device is described in US Patent Nos. 7,742,630, 7,715,902 and European Publication No. EP1788498, the contents of each of which is herein incorporated by reference in their entireties. As a non-limiting example, a method of reducing the amount of backflow which is described in US Patent Publication No. US20140243783, the contents of which are herein incorporated by reference in its entirety, may be used to reduce the backflow from the administration of composition comprising AAV particles described herein.

### Combinations

The AAV particles may be used in combination with one or more other therapeutic, prophylactic, diagnostic, or imaging agents. By "in combination with," it is not intended to imply that the agents must be administered at the same time and/or formulated for delivery together, although these methods of delivery are within the scope of the present disclosure. Compositions can be administered concurrently with, prior to, or subsequent to, one or more other desired therapeutics or medical procedures. In general, each agent will be administered at a dose and/or on a time schedule determined for that agent. In some embodiments, the present disclosure encompasses the delivery of pharmaceutical, prophylactic, diagnostic, or imaging compositions in combination with agents that may improve their bioavailability, reduce and/or modify their metabolism, inhibit their excretion, and/or modify their distribution within the body.

In one embodiment, the AAV particles may be delivered via intracerebroventricular (ICV) and/or intrathecal (IT) infusion and therapeutic agent may also be delivered to a subject via intravascular limb infusion in order to deliver the therapeutic agent to the skeletal muscle. Delivery of adeno-associate virus by intravascular limb infusion is described by Gruntman and Flotte (Human Gene Therapy Clinical Development, Vol. 26(3), 2015 159-164; the contents of which is herein incorporated by reference in its entirety).

### Delivery to Cells

The present disclosure provides a method of delivering to a cell or tissue any of the above-described AAV particle, comprising contacting the cell or tissue with said AAV particle or contacting the cell or tissue with a particle comprising said AAV particle, or contacting the cell or tissue with any of the described compositions, including pharmaceutical compositions. The method of delivering the AAV particle to a cell or tissue can be accomplished in *vitro, ex vivo*, or *in vivo.*

### Delivery to Subjects

The present disclosure additionally provides a method of delivering to a subject, including a mammalian subject, any of the above-described AAV particle comprising administering to the subject said AAV particle, or administering to the subject a particle comprising said AAV particle, or administering to the subject any of the described compositions, including pharmaceutical compositions.

### Measurement of Expression

Expression of payloads from viral genomes may be determined using various methods known in the art such as, but not limited to immunochemistry (e.g., IHC) or in situ hybridization (ISH). In one embodiment, transgenes delivered in different AAV capsids may have different expression levels in Dorsal Root Ganglion (DRG).

### CNS diseases

The present disclosure provides a method for treating a disease, disorder and/or condition in a mammalian subject, including a human subject, comprising administering to the subject any of the viral particles e.g., AAV, AAV particle or AAV genomes described herein (i.e., viral genomes or "VG") or administering to the subject a particle comprising said AAV particle or AAV genome, or administering to the subject any of the described compositions, including pharmaceutical compositions. In one embodiment, the disease, disorder and/or condition is a neurological disease, disorder and/or condition. The CNS diseases may be diseases that affect any component of the brain (including the cerebral hemispheres, diencephalon, brain stem, and cerebellum) or the spinal cord.

In some embodiments, AAV particles of the present invention, through delivery of a function payload that is a therapeutic product that can modulate the level or function of a gene product in the CNS, may be used to treat a neurodegenerative diseases and/or diseases or disorders that are characteristic with neurodegeneration, neuromuscular diseases, lysosomal diseases, trauma, bone marrow injuries, pain (including neuropathic pain), cancers of the nervous system, demyelinating diseases, autoimmune diseases of the nervous system, neurotoxic syndromes, sleeping disorders genetic brain disorders and developmental CNS disorders. A functional payload may alleviate or reduce symptoms that result from abnormal level and/or function of a gene product (e.g., an absence or defect in a protein) in a subject in need thereof or that otherwise confers a benefit to a CNS disorder in a subject in need thereof.

As non-limiting examples, therapeutic products delivered by AAV particles of the present invention may include, but are not limited to, growth and trophic factors, cytokines, hormones, neurotransmitters, enzymes, anti-apoptotic factors, angiogenic factors, and any protein known to be mutated in pathological disorders such as the "survival of motor neuron " protein (SMN); antisense RNA or RNAi targeting messenger RNAs coding for proteins having a therapeutic interest in any of CNS diseases discussed herein; or microRNAs that function in gene silencing and post-transcriptionally regulation of gene expression in the CNS (e.g., brain specific Mir-128a, See Adlakha and Saini, Molecular cancer, 2014, 13:33).

The growth and trophic factors may include, but are not limited to brain-derived growth factor (BDNF), epidermal growth factor (EGF), basic Fibroblast growth factor (bFGF), Ciliary neurotrophic factor (CNTF), corticotropin-releasing factor (CRF), Glial cell line derived growth factor (GDNF), Insulin-like growth factor-1 (IGF-1), nerve growth factor (NGF), neurotrophin-3 (NT-3), neurotrophin-4 (NT-4), and vascular endothelial growth factor (VEGF). Cytokines may include interleukin-10 (IL-10), interleukin-6, Interleukin-8, chemokine CXCL12 (SDF-1), TGF-beta, and Growth and differentiation factor (GDF-1/10).

In some embodiments, the neurological disorders may be neurodegenerative disorders including, but not limited to, Alzheimer's Diseases (AD); Amyotrophic lateral sclerosis (ALS); Creutzfeldt-Jakob Disease ; Huntingtin's disease (HD); Friedreich's ataxia (FA); Parkinson Disease (PD); Multiple System Atrophy (MSA); Spinal Muscular Atrophy (SMA), Multiple Sclerosis (MS); Primary progressive aphasia; Progressive supranuclear palsy; Dementia; Brain Cancer, Degenerative Nerve Diseases, Encephalitis, Epilepsy, Genetic Brain Disorders that cause neurodegeneration, *Retinitis pigmentosa* (RP), Head and Brain Malformations, Hydrocephalus, Stroke, Prion disease, Infantile neuronal ceroid lipofuscinosis (INCL) (a neurodegenerative disease of children caused by a deficiency in the lysosomal enzyme palmitoyl protein thioesterase-1 (PPT1)), and others.

In some embodiments, AAV particles of the present invention may be used to treat diseases that are associated with impairments of the growth and development of the CNS, i.e., neurodevelopmental disorders. In some aspects, such neurodevelopmental disorders may be caused by genetic mutations, including but not limited to, Fragile X syndrome (caused by mutations in FMR1 gene), Down syndrome (caused by trisomy of chromosome 21), Rett syndrome, Williams syndrome, Angelman syndrome, Smith-Magenis syndrome, ATR-X syndrome, Barth syndrome, Immune dysfunction and/or infectious diseases during infancy such as Sydenham's chorea, Schizophrenia Congenital toxoplasmosis, Congenital rubella syndrome, Metabolic disorders such as diabetes mellitus and phenylketonuria; nutritional defects and/or brain trauma, Autism and autism spectrum.

In some embodiments, AAV particles of the present invention, may be used to treat a tumor in the CNS, including but not limited to, acoustic neuroma, Astrocytoma (Grades I, II, III and IV), Chordoma, CNS Lymphoma, Craniopharyngioma, Gliomas (e.g., brain stem glioma, ependymoma, optical nerve glioma, subependymoma), Medulloblastoma, Meningioma, Metastatic brain tumors, Oligodendroglioma, Pituitary Tumors, Primitive neuroectodermal (PNET), and Schwannoma.

In some embodiments, the neurological disorders may be functional neurological disorders with motor and/or sensory symptoms which have neurological origin in the CNS. As non-limiting examples, functional neurological disorders may be chronic pain, seizures, speech problems, involuntary movements, and sleep disturbances.

In some embodiments, the neurological disorders may be white matter disorders (a group of diseases that affects nerve fibers in the CNS) including but not limited to, Pelizaeus-Merzbacher disease, Hypomyelination with atrophy of basal ganglia and cerebellum, Aicardi-Goutières syndrome, Megalencephalic leukoencephalopathy with subcortical cysts, Congenital muscular dystrophies, Myotonic dystrophy, Wilson disease, Lowe syndrome, Sjögren-Larsson syndrome, PIBD or Tay syndrome, Cockayne's disease, erebrotendinous xanthomatosis, Zellweger syndrome, Neonatal adrenoleukodystrophy, Infantile Refsum disease, Zellweger-like syndrome, Pseudo-Zellweger syndrome, Pseudo-neonatal adrenoleukodystrophy, Bifunctional protein deficiency, X-linked adrenoleukodystrophy and adrenomyeloneuropathy and Refsum disease.

In some embodiments, the neurological disorders may be lysosomal storage disorders (LSDs) caused by the inability of cells in the CNS to break down metabolic end products, including but not limited to Niemann-Pick disease (a LSD resulting from inherited deficiency in acid sphingomyelinase (ASM); Metachromatic leukodystrophy (MLD) (a LSD characterized by accumulation of sulfatides in glial cells and neurons, the result of an inherited deficiency of arylsulfatase A (ARSA)); Globoid-cell leukodystrophy (GLD) (a LSD caused by mutations in galactosylceramidase); Fabry disease (a LSD caused by mutations in the alpha-galactosidase A *(GLA)* gene); Gaucher disease (caused by mutations in the beta-glucocerebrosidase (GBA) gene); GM1/GM2 gangliosidosis; Mucopolysaccharidoses disorder; Pompe disease; and Neuronal ceroid lipofuscinosis.

In one embodiment, the neurological disease, disorder and/or condition is Parkinson's disease. In one embodiment, the polynucleotide used to treat Parkinson's disease comprises any one of SEQ ID NOs 1-93 wherein the payload is replaced by AADC or any other payload known in the art for treating Parkinson's disease. As a non-limiting example, the payload may be a sequence such as NM_001082971.1 (GI: 132814447), NM_000790.3 (GI: 132814459), NM_001242886.1 (GI: 338968913), NM_001242887.1 (GI: 338968916), NM_001242888.1 (GI: 338968918), NM_001242889.1 (GI: 338968920), NM_001242890.1 (GI: 338968922) and fragment or variants thereof.

In another embodiment, the neurological disease, disorder and/or condition is Friedreich's Ataxia. In one embodiment, the delivery of the AAV particles may halt or slow the disease progression of Friedreich's Ataxia by 10%, 20%, 30%, 40%, 50%, 60%, 70%, 80%, 90%, 95% or more than 95% using a known analysis method and comparator group for Friedreich's Ataxia. As a non-limiting example, the delivery of the AAV particles may halt or slow progression of Friedreich's Ataxia progression as measured by mFARS/SARA by 50% relative to a comparator group. In one embodiment the polynucleotide used to treat Friedreich's Ataxia comprises any one of SEQ ID NOs 1-93 wherein the payload is replaced by Frataxin or any other payload known in the art for treating Friedreich's Ataxia. As a non-limiting example, the payload may be a sequence such as NM_000144.4 (GI: 239787167), NM_181425.2 (GI: 239787185), NM_001161706.1 (GI: 239787197) and fragment or variants thereof.

In another embodiment, the neurological disease, disorder and/or condition is Amyotrophic lateral sclerosis (ALS). In one embodiment, the delivery of the AAV particles may halt or slow the disease progression of ALS by 10%, 20%, 30%, 40%, 50%, 60%, 70%, 80%, 90%, 95% or more than 95% using a known analysis method and comparator group for ALS. In one embodiment the polynucleotide used to treat ALS comprises any one of SEQ ID NOs 1-93 wherein the payload is replaced by an shRNA, miRNA, siRNA, RNAi for SOD1 or any other payload known in the art for treating ALS.

In another embodiment, the neurological disease, disorder and/or condition is Huntington's disease. In one embodiment, the delivery of the AAV particles may halt or slow the disease progression of Huntington's disease by 10%, 20%, 30%, 40%, 50%, 60%, 70%, 80%, 90%, 95% or more than 95% using a known analysis method and comparator group for Huntington's disease. In one embodiment the polynucleotide used to treat Huntington's disease comprises any one of SEQ ID NOs 1-93 wherein the payload is replaced by an shRNA, miRNA, siRNA, RNAi for Htt or any other payload known in the art for treating Huntington's disease.

In another embodiment, the neurological disease, disorder or condition is spinal muscular atrophy (SMA). In one embodiment, the delivery of the AAV particles may halt or slow the disease progression of SMA by 10%, 20%, 30%, 40%, 50%, 60%, 70%, 80%, 90%, 95% or more than 95% using a known analysis method and comparator group for SMA. In one embodiment the polynucleotide used to treat SMA comprises any one of SEQ ID NOs 1-93 wherein the payload is replaced by SMN or any other payload known in the art for treating SMA. As anon-limiting example, the payload may be a sequence such as NM_001297715.1 (GI: 663070993), NM_000344.3 (GI: 196115055), NM_022874.2 (GI: 196115040) and fragment or variants thereof.

In one embodiment, the AAV particle encoding a payload may increase the amount of protein encoded by the transgene by 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, 97%, 99% or more than 100%.

In one embodiment, the AAV particle encoding a payload may increase the amount of protein encoded by the transgene by 1-5%, 1-10%, 1-15%, 1-20%, 5-10%, 5-15%, 5-20%, 5-25%, 10-20%, 10-30%, 15-35%, 20-40%, 20-50%, 30-50%, 30-60%, 40-60%, 40-70%, 50-60%, 50-70%, 60-80%, 60-90%, 70-80%, 70-90%, 80-90%, 80-99% or 90-100%.

### DEFINITIONS

At various places in the present specification, substituents of compounds of the present disclosure are disclosed in groups or in ranges. It is specifically intended that the present disclosure include each and every individual sub-combination of the members of such groups and ranges. The following is a non-limiting list of term definitions.

*Adeno-associated virus:* The term "adeno-associated virus" or "AAV" as used herein refers to members of the dependovirus genus comprising any particle, sequence, gene, protein, or component derived therefrom. The term "AAV particle" as used herein comprises a capsid and a polynucleotide referred to as the AAV genome or viral genome (VG). The AAV particle may be derived from any serotype, described herein or known in the art, including combinations of serotypes (i.e., "pseudotyped" AAV) or from various genomes (e.g., single stranded or self-complementary). In addition, the AAV particle may be replication defective and/or targeted.

*Activity:* As used herein, the term "activity" refers to the condition in which things are happening or being done. Compositions of the invention may have activity and this activity may involve one or more biological events.

*Administered in combination:* As used herein, the term "administered in combination" or "combined administration" refers to simultaneous exposure to two or more agents (e.g., AAV) administered at the same time or within an interval such that the subject is at some point in time simultaneously exposed to both and/or such that there may be an overlap in the effect of each agent on the patient. In some embodiments, at least one dose of one or more agents is administered within about 24 hours, 12 hours, 6 hours, 3 hours, 1 hour, 30 minutes, 15 minutes, 10 minutes, 5 minutes, or 1 minute of at least one dose of one or more other agents. In some embodiments, administration occurs in overlapping dosage regimens. As used herein, the term "dosage regimen" refers to a plurality of doses spaced apart in time. Such doses may occur at regular intervals or may include one or more hiatus in administration. In some embodiments, the administration of individual doses of one or more compounds and/or compositions of the present invention, as described herein, are spaced sufficiently closely together such that a combinatorial (*e.g*., a synergistic) effect is achieved.

*Amelioration*: As used herein, the term "amelioration" or "ameliorating" refers to a lessening of severity of at least one indicator of a condition or disease. For example, in the context of neurodegeneration disorder, amelioration includes the reduction of neuron loss.

*Animal*: As used herein, the term "animal" refers to any member of the animal kingdom. In some embodiments, "animal" refers to humans at any stage of development. In some embodiments, "animal" refers to non-human animals at any stage of development. In certain embodiments, the non-human animal is a mammal (*e.g*., a rodent, a mouse, a rat, a rabbit, a monkey, a dog, a cat, a sheep, cattle, a primate, or a pig). In some embodiments, animals include, but are not limited to, mammals, birds, reptiles, amphibians, fish, and worms. In some embodiments, the animal is a transgenic animal, genetically-engineered animal, or a clone.

*Antisense strand*: As used herein, the term "the antisense strand" or "the first strand" or "the guide strand" of a siRNA molecule refers to a strand that is substantially complementary to a section of about 10-50 nucleotides, e.g., about 15-30, 16-25, 18-23 or 19-22 nucleotides of the mRNA of the gene targeted for silencing. The antisense strand or first strand has sequence sufficiently complementary to the desired target mRNA sequence to direct target-specific silencing, e.g., complementarity sufficient to trigger the destruction of the desired target mRNA by the RNAi machinery or process.

*Approximately:* As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Associated with:* As used herein, the terms "associated with," "conjugated," "linked," "attached," and "tethered," when used with respect to two or more moieties, mean that the moieties are physically associated or connected with one another, either directly or via one or more additional moieties that serve as linking agents, to form a structure that is sufficiently stable so that the moieties remain physically associated under the conditions in which the structure is used, *e.g*., physiological conditions. An "association" need not be strictly through direct covalent chemical bonding. It may also suggest ionic or hydrogen bonding or a hybridization based connectivity sufficiently stable such that the "associated" entities remain physically associated.

*Biomolecule:* As used herein, the term "biomolecule" is any natural molecule which is amino acid-based, nucleic acid-based, carbohydrate-based or lipid-based, and the like.

*Biologically active:* As used herein, the phrase "biologically active" refers to a characteristic of any substance (e.g., an AAV) that has activity in or on a biological system and/or organism. For instance, a substance that, when administered to an organism, has a biological effect on that organism, is considered to be biologically active. In particular embodiments, a compounds and/or compositions of the present invention may be considered biologically active if even a portion of is biologically active or mimics an activity considered to biologically relevant.

*Biological system:* As used herein, the term "biological system" refers to a group of organs, tissues, cells, intracellular components, proteins, nucleic acids, molecules (including, but not limited to biomolecules) that function together to perform a certain biological task within cellular membranes, cellular compartments, cells, tissues, organs, organ systems, multicellular organisms, or any biological entity. In some embodiments, biological systems are cell signaling pathways comprising intracellular and/or extracellular cell signaling biomolecules. In some embodiments, biological systems comprise growth factor signaling events within the extracellular/cellular matrix and/or cellular niches.

*Central Nervous System or CNS:* As used herein, "Central Nervous System" or "CNS" refers to one of the two major subdivisions of the nervous system, which in vertebrates includes of the brain and spinal cord. The central nervous system coordinates the activity of the entire nervous system.

*Cervical Region*: As used herein, "Cervical Region" refers to the region of the spinal cord comprising the cervical vertebrae C1, C2, C3, C4, C5, C6, C7, and C9.

*CNS tissue:* As used herein, "CNS tissue" or "CNS tissues" refers to the tissues of the central nervous system, which in vertebrates, include the brain and spinal cord and sub-structures thereof.

*CNS structures:* As used herein, "CNS structures" refers to structures of the central nervous system and sub-structures thereof. Non-limiting examples of structures in the spinal cord may include, ventral horn, dorsal horn, white matter, and nervous system pathways or nuclei within. Non limiting examples of structures in the brain include, forebrain, midbrain, hindbrain, diencephalon, telencephalon, myelencepphalon, metencephalon, mesencephalon, prosencephalon, rhombencephalon, cortices, frontal lobe, parietal lobe, temporal lobe, occipital lobe, cerebrum, thalamus, hypothalamus, tectum, tegmentum, cerebellum, pons, medulla, amygdala, hippocampus, basal ganglia, corpus callosum, pituitary gland, ventricles and sub-structures thereof.

*CNS Cells:* As used herein, "CNS Cells" refers to cells of the central nervous system and sub-structures thereof. Non-limiting examples of CNS cells include, neurons and sub-types thereof, glia, microglia, oligodendrocytes, ependymal cells and astrocytes. Non-limiting examples of neurons include sensory neurons, motor neurons, interneurons, unipolar cells, bipolar cells, multipolar cells, psuedounipolar cells, pyramidal cells, basket cells, stellate cells, purkinje cells, betz cells, amacrine cells, granule cell, ovoid cell, medium aspiny neurons and large aspiny neurons.

*Complementary and substantially complementary:* As used herein, the term "complementary" refers to the ability of polynucleotides to form base pairs with one another. Base pairs are typically formed by hydrogen bonds between nucleotide units in antiparallel polynucleotide strands. Complementary polynucleotide strands can form base pair in the Watson-Crick manner (e.g., A to T, A to U, C to G), or in any other manner that allows for the formation of duplexes. As persons skilled in the art are aware, when using RNA as opposed to DNA, uracil rather than thymine is the base that is considered to be complementary to adenosine. However, when a U is denoted in the context of the present invention, the ability to substitute a T is implied, unless otherwise stated. Perfect complementarity or 100% complementarity refers to the situation in which each nucleotide unit of one polynucleotide strand can form hydrogen bond with a nucleotide unit of a second polynucleotide strand. Less than perfect complementarity refers to the situation in which some, but not all, nucleotide units of two strands can form hydrogen bond with each other. For example, for two 20-mers, if only two base pairs on each strand can form hydrogen bond with each other, the polynucleotide strands exhibit 10% complementarity. In the same example, if 18 base pairs on each strand can form hydrogen bonds with each other, the polynucleotide strands exhibit 90% complementarity. As used herein, the term "substantially complementary" means that the siRNA has a sequence (e.g., in the antisense strand) which is sufficient to bind the desired target mRNA, and to trigger the RNA silencing of the target mRNA.

*Composition:* As used herein, the term "composition" comprises an AAV polynucleotide, AAV genome or AAV particle and at least one excipient.

*Compound:* As used herein, the term "compound," refers to a distinct chemical entity. In some embodiments, a particular compound may exist in one or more isomeric or isotopic forms (including, but not limited to stereoisomers, geometric isomers and isotopes). In some embodiments, a compound is provided or utilized in only a single such form. In some embodiments, a compound is provided or utilized as a mixture of two or more such forms (including, but not limited to a racemic mixture of stereoisomers). Those of skill in the art appreciate that some compounds exist in different such forms, show different properties and/or activities (including, but not limited to biological activities). In such cases it is within the ordinary skill of those in the art to select or avoid particular forms of the compound for use in accordance with the present invention. For example, compounds that contain asymmetrically substituted carbon atoms can be isolated in optically active or racemic forms. Methods on how to prepare optically active forms from optically active starting materials are known in the art, such as by resolution of racemic mixtures or by stereoselective synthesis.

*Conserved:* As used herein, the term "conserved" refers to nucleotides or amino acid residues of polynucleotide or polypeptide sequences, respectively, that are those that occur unaltered in the same position of two or more sequences being compared. Nucleotides or amino acids that are relatively conserved are those that are conserved among more related sequences than nucleotides or amino acids appearing elsewhere in the sequences.

In some embodiments, two or more sequences are said to be "completely conserved" if they are 100% identical to one another. In some embodiments, two or more sequences are said to be "highly conserved" if they are at least 70% identical, at least 80% identical, at least 90% identical, or at least 95% identical to one another. In some embodiments, two or more sequences are said to be "highly conserved" if they are about 70% identical, about 80% identical, about 90% identical, about 95%, about 98%, or about 99% identical to one another. In some embodiments, two or more sequences are said to be "conserved" if they are at least 30% identical, at least 40% identical, at least 50% identical, at least 60% identical, at least 70% identical, at least 80% identical, at least 90% identical, or at least 95% identical to one another. In some embodiments, two or more sequences are said to be "conserved" if they are about 30% identical, about 40% identical, about 50% identical, about 60% identical, about 70% identical, about 80% identical, about 90% identical, about 95% identical, about 98% identical, or about 99% identical to one another. Conservation of sequence may apply to the entire length of an oligonucleotide or polypeptide or may apply to a portion, region or feature thereof.

In one embodiment, conserved sequences are not contiguous. Those skilled in the art are able to appreciate how to achieve alignment when gaps in contiguous alignment are present between sequences, and to align corresponding residues not withstanding insertions or deletions present.

*Delivery:* As used herein, "delivery" refers to the act or manner of delivering a parvovirus e.g., AAV compound, substance, entity, moiety, cargo or payload to a target. Such target may be a cell, tissue, organ, organism, or system (whether biological or production).

*Delivery Agent:* As used herein, "delivery agent" refers to any agent which facilitates, at least in part, the delivery of one or more substances (including, but not limited to a compounds and/or compositions of the present invention, e.g., viral particles or AAV vectors) to targeted cells.

*Destabilized:* As used herein, the term "destable," "destabilize," or "destabilizing region" means a region or molecule that is less stable than a starting, reference, wild-type or native form of the same region or molecule.

*Detectable label:* As used herein, "detectable label" refers to one or more markers, signals, or moieties which are attached, incorporated or associated with another entity, which markers, signals or moieties are readily detected by methods known in the art including radiography, fluorescence, chemiluminescence, enzymatic activity, absorbance, immunological detection and the like. Detectable labels may include radioisotopes, fluorophores, chromophores, enzymes, dyes, metal ions, ligands, biotin, avidin, streptavidin and haptens, quantum dots, polyhistidine tags, myc tags, flag tags, human influenza hemagglutinin (HA) tags and the like. Detectable labels may be located at any position in the entity with which they are attached, incorporated or associated. For example, when attached, incorporated in or associated with a peptide or protein, they may be within the amino acids, the peptides, or proteins, or located at the N- or C- termini.

*Effective amount:* As used herein, the term "effective amount" of an agent is that amount sufficient to effect beneficial or desired results, for example, upon single or multiple dose administration to a subject or a cell, in curing, alleviating, relieving or improving one or more symptoms of a disorder and, as such, an "effective amount" depends upon the context in which it is being applied. For example, in the context of administering an agent that treats ALS, an effective amount of an agent is, for example, an amount sufficient to achieve treatment, as defined herein, of ALS, as compared to the response obtained without administration of the agent.

*Engineered:* As used herein, embodiments of the invention are "engineered" when they are designed to have a feature or property, whether structural or chemical, that varies from a starting point, wild-type or native molecule. Thus, engineered agents or entities are those whose design and/or production include an act of the hand of man.

*Epitope:* As used herein, an "epitope" refers to a surface or region on a molecule that is capable of interacting with a biomolecule. For example a protein may contain one or more amino acids, e.g., an epitope, which interacts with an antibody, e.g., a biomolecule. In some embodiments, when referring to a protein or protein module, an epitope may comprise a linear stretch of amino acids or a three dimensional structure formed by folded amino acid chains.

*Expression:* As used herein, "expression" of a nucleic acid sequence refers to one or more of the following events: (1) production of an RNA template from a DNA sequence (*e.g*., by transcription); (2) processing of an RNA transcript (*e.g*., by splicing, editing, 5' cap formation, and/or 3' end processing); (3) translation of an RNA into a polypeptide or protein; (4) folding of a polypeptide or protein; and (5) post-translational modification of a polypeptide or protein.

*Feature:* As used herein, a "feature" refers to a characteristic, a property, or a distinctive element.

*Formulation:* As used herein, a "formulation" includes at least a compound and/or composition of the present invention (e.g., a vector, AAV particle, etc.) and a delivery agent.

*Fragment:* A "fragment," as used herein, refers to a contiguous portion of a whole. For example, fragments of proteins may comprise polypeptides obtained by digesting full-length protein isolated from cultured cells. In some embodiments, a fragment of a protein includes at least 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 150, 200, 250 or more amino acids.

*Functional:* As used herein, a "functional" biological molecule is a biological entity with a structure and in a form in which it exhibits a property and/or activity by which it is characterized.

*Gene expression:* The term "gene expression" refers to the process by which a nucleic acid sequence undergoes successful transcription and in most instances translation to produce a protein or peptide. For clarity, when reference is made to measurement of "gene expression", this should be understood to mean that measurements may be of the nucleic acid product of transcription, e.g., RNA or mRNA or of the amino acid product of translation, e.g., polypeptides or peptides. Methods of measuring the amount or levels of RNA, mRNA, polypeptides and peptides are well known in the art.

*High Cervical Region:* As used herein, the term "high cervical region" refers to the region of the spinal cord comprising the cervical vertebrae C1, C2, C3 and C4 or any subset thereof.

*Homology:* As used herein, the term "homology" refers to the overall relatedness between polymeric molecules, *e.g*. between nucleic acid molecules (*e.g.* DNA molecules and/or RNA molecules) and/or between polypeptide molecules. In some embodiments, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% identical or similar. The term "homologous" necessarily refers to a comparison between at least two sequences (polynucleotide or polypeptide sequences). In accordance with the invention, two polynucleotide sequences are considered to be homologous if the polypeptides they encode are at least about 50%, 60%, 70%, 80%, 90%, 95%, or even 99% for at least one stretch of at least about 20 amino acids. In some embodiments, homologous polynucleotide sequences are characterized by the ability to encode a stretch of at least 4-5 uniquely specified amino acids. For polynucleotide sequences less than 60 nucleotides in length, homology is typically determined by the ability to encode a stretch of at least 4-5 uniquely specified amino acids. In accordance with the invention, two protein sequences are considered to be homologous if the proteins are at least about 50%, 60%, 70%, 80%, or 90% identical for at least one stretch of at least about 20 amino acids. In many embodiments, homologous protein may show a large overall degree of homology and a high degree of homology over at least one short stretch of at least 3, 4, 5, 6, 7, 8, 9, 10, 11, 12, 13, 14, 15, 16, 17, 18, 19, 20, 25, 30, 35, 40, 45, 50 or more amino acids. In many embodiments, homologous proteins share one or more characteristic sequence elements. As used herein, the term "characteristic sequence element" refers to a motif present in related proteins. In some embodiments, the presence of such motifs correlates with a particular activity (such as biological activity).

*Identity:* As used herein, the term "identity" refers to the overall relatedness between polymeric molecules, *e.g*., between oligonucleotide molecules (*e.g.* DNA molecules and/or RNA molecules) and/or between polypeptide molecules. Calculation of the percent identity of two polynucleotide sequences, for example, may be performed by aligning the two sequences for optimal comparison purposes (*e.g*., gaps can be introduced in one or both of a first and a second nucleic acid sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using methods such as those described in Computational Molecular Biology, Lesk, A. M., ed., Oxford University Press, New York, 1988; Biocomputing: Informatics and Genome Projects, Smith, D. W., ed., Academic Press, New York, 1993; Sequence Analysis in Molecular Biology, von Heinje, G., Academic Press, 1987; Computer Analysis of Sequence Data, Part I, Griffin, A. M., and Griffin, H. G., eds., Humana Press, New Jersey, 1994; and Sequence Analysis Primer, Gribskov, M. and Devereux, J., eds., M Stockton Press, New York, 1991; each of which is incorporated herein by reference in its entirety. For example, the percent identity between two nucleotide sequences can be determined, for example using the algorithm of Meyers and Miller (CABIOS, 1989, 4:11-17), which has been incorporated into the ALIGN program (version 2.0) using a PAM120 weight residue table, a gap length penalty of 12 and a gap penalty of 4. The percent identity between two nucleotide sequences can, alternatively, be determined using the GAP program in the GCG software package using an NWSgapdna.CMP matrix. Methods commonly employed to determine percent identity between sequences include, but are not limited to those disclosed in Carillo, H., and Lipman, D., SIAM J Applied Math., 48:1073 (1988); incorporated herein by reference in its entirety. Techniques for determining identity are codified in publicly available computer programs. Computer software to determine homology between two sequences include, but are not limited to, GCG program package, Devereux, J., et al., Nucleic Acids Research, 12(1), 387 (1984)), BLASTP, BLASTN, and FASTA Altschul, S. F. et al., J. Molec. Biol., 215, 403 (1990)).

*Inhibit expression of a gene:* As used herein, the phrase "inhibit expression of a gene" means to cause a reduction in the amount of an expression product of the gene. The expression product may be RNA transcribed from the gene (*e.g*. mRNA) or a polypeptide translated from mRNA transcribed from the gene. Typically a reduction in the level of mRNA results in a reduction in the level of a polypeptide translated therefrom. The level of expression may be determined using standard techniques for measuring mRNA or protein.

*In vitro:* As used herein, the term *"in vitro"* refers to events that occur in an artificial environment, *e.g*., in a test tube or reaction vessel, in cell culture, in a Petri dish, *etc.,* rather than within an organism (*e.g*., animal, plant, or microbe).

*In vivo:* As used herein, the term *"in vivo"* refers to events that occur within an organism (*e.g*., animal, plant, or microbe or cell or tissue thereof).

*Isolated*: As used herein, the term "isolated" is synonymous with "separated", but carries with it the inference separation was carried out by the hand of man. In one embodiment, an isolated substance or entity is one that has been separated from at least some of the components with which it was previously associated (whether in nature or in an experimental setting). Isolated substances may have varying levels of purity in reference to the substances from which they have been associated. Isolated substances and/or entities may be separated from at least about 10%, about 20%, about 30%, about 40%, about 50%, about 60%, about 70%, about 80%, about 90%, or more of the other components with which they were initially associated. In some embodiments, isolated agents are more than about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components.

*Substantially isolated*: By "substantially isolated" is meant that the compound is substantially separated from the environment in which it was formed or detected. Partial separation can include, for example, a composition enriched in the compound of the present disclosure. Substantial separation can include compositions containing at least about 50%, at least about 60%, at least about 70%, at least about 80%, at least about 90%, at least about 95%, at least about 97%, or at least about 99% by weight of the compound of the present disclosure, or salt thereof. Methods for isolating compounds and their salts are routine in the art. In some embodiments, isolation of a substance or entity includes disruption of chemical associations and/or bonds. In some embodiments, isolation includes only the separation from components with which the isolated substance or entity was previously combined and does not include such disruption.

*Lumbar Region*: As used herein, the term "lumbar region" refers to the region of the spinal cord comprising the lumbar vertebrae L1, L2, L3, L4, and L5.

*Modified:* As used herein, the term "modified" refers to a changed state or structure of a molecule or entity as compared with a parent or reference molecule or entity. Molecules may be modified in many ways including chemically, structurally, and functionally. In some embodiments, compounds and/or compositions of the present invention are modified by the introduction of non-natural amino acids, or non-natural nucleotides.

*Mutation*: As used herein, the term "mutation" refers to a change and/or alteration. In some embodiments, mutations may be changes and/or alterations to proteins (including peptides and polypeptides) and/or nucleic acids (including polynucleic acids). In some embodiments, mutations comprise changes and/or alterations to a protein and/or nucleic acid sequence. Such changes and/or alterations may comprise the addition, substitution and or deletion of one or more amino acids (in the case of proteins and/or peptides) and/or nucleotides (in the case of nucleic acids and or polynucleic acids). In embodiments wherein mutations comprise the addition and/or substitution of amino acids and/or nucleotides, such additions and/or substitutions may comprise 1 or more amino acid and/or nucleotide residues and may include modified amino acids and/or nucleotides.

*Naturally occurring:* As used herein, "naturally occurring" or "wild-type" means existing in nature without artificial aid, or involvement of the hand of man.

*Non-human vertebrate:* As used herein, a "non-human vertebrate" includes all vertebrates except *Homo sapiens,* including wild and domesticated species. Examples of non-human vertebrates include, but are not limited to, mammals, such as alpaca, banteng, bison, camel, cat, cattle, deer, dog, donkey, gayal, goat, guinea pig, horse, llama, mule, pig, rabbit, reindeer, sheep water buffalo, and yak.

*Nucleic acid:* As used herein, the term "nucleic acid", "polynucleotide" and 'oligonucleotide" refer to any nucleic acid polymers composed of either polydeoxyribonucleotides (containing 2-deoxy-D-ribose), or polyribonucleotides (containing D-ribose), or any other type of polynucleotide which is an N glycoside of a purine or pyrimidine base, or modified purine or pyrimidine bases. There is no intended distinction in length between the term "nucleic acid", "polynucleotide" and "oligonucleotide", and these terms will be used interchangeably. These terms refer only to the primary structure of the molecule. Thus, these terms include double- and single-stranded DNA, as well as double- and single stranded RNA.

*Off-target:* As used herein, "off target" refers to any unintended effect on any one or more target, gene and/or cellular transcript.

*Operably linked:* As used herein, the phrase "operably linked" refers to a functional connection between two or more molecules, constructs, transcripts, entities, moieties or the like.

*Particle:* As used herein, a "particle" is a virus comprised of at least two components, a protein capsid and a polynucleotide sequence enclosed within the capsid.

*Patient:* As used herein, "patient" refers to a subject who may seek or be in need of treatment, requires treatment, is receiving treatment, will receive treatment, or a subject who is under care by a trained (e.g., licensed) professional for a particular disease or condition.

*Payload:* As used herein, "payload" refers to one or more polynucleotides or polynucleotide regions encoded by or within a viral genome or an expression product of such polynucleotide or polynucleotide region, e.g., a transgene, a polynucleotide encoding a polypeptide or multi-polypeptide or a modulatory nucleic acid or regulatory nucleic acid.

*Payload construct:* As used herein, "payload construct" is one or more polynucleotide regions encoding or comprising a payload that is flanked on one or both sides by an inverted terminal repeat (ITR) sequence. The payload construct is a template that is replicated in a viral production cell to produce a viral genome.

*Payload construct vector:* As used herein, "payload construct vector" is a vector encoding or comprising a payload construct, and regulatory regions for replication and expression in bacterial cells. The payload construct vector may also comprise component for viral expression in a viral replication cell

*Peptide:* As used herein, the term "peptide" refers to a chain of amino acids that is less than or equal to about 50 amino acids long, e.g., about 5, 10, 15, 20, 25, 30, 35, 40, 45, or 50 amino acids long.

*Pharmaceutically acceptable:* The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

*Pharmaceutically acceptable excipients:* As used herein, the term "pharmaceutically acceptable excipient," as used herein, refers to any ingredient other than active agents (e.g., as described herein) present in pharmaceutical compositions and having the properties of being substantially nontoxic and non-inflammatory in subjects. In some embodiments, pharmaceutically acceptable excipients are vehicles capable of suspending and/or dissolving active agents. Excipients may include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspending or dispersing agents, sweeteners, and waters of hydration. Excipients include, but are not limited to: butylated hydroxytoluene (BHT), calcium carbonate, calcium phosphate (dibasic), calcium stearate, croscarmellose, cross-linked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E, vitamin C, and xylitol.

*Pharmaceutically acceptable salts:* Pharmaceutically acceptable salts of the compounds described herein are forms of the disclosed compounds wherein the acid or base moiety is in its salt form (e.g., as generated by reacting a free base group with a suitable organic acid). Examples of pharmaceutically acceptable salts include, but are not limited to, mineral or organic acid salts of basic residues such as amines; alkali or organic salts of acidic residues such as carboxylic acids; and the like. Representative acid addition salts include acetate, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Pharmaceutically acceptable salts include the conventional non-toxic salts, for example, from non-toxic inorganic or organic acids. In some embodiments a pharmaceutically acceptable salt is prepared from a parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418, Pharmaceutical Salts: Properties, Selection, and Use, P.H. Stahl and C.G. Wermuth (eds.), Wiley-VCH, 2008, and Berge et al., Journal of Pharmaceutical Science, 66, 1-19 (1977), each of which is incorporated herein by reference in its entirety. *Pharmaceutically acceptable solvate:* The term "pharmaceutically acceptable solvate," as used herein, refers to a crystalline form of a compound wherein molecules of a suitable solvent are incorporated in the crystal lattice. For example, solvates may be prepared by crystallization, recrystallization, or precipitation from a solution that includes organic solvents, water, or a mixture thereof. Examples of suitable solvents are ethanol, water (for example, mono-, di-, and tri-hydrates), *N*-methylpyrrolidinone (NMP), dimethyl sulfoxide (DMSO), *N*,*N'*-dimethylformamide (DMF), *N*,*N'*-dimethylacetamide (DMAC), 1,3-dimethyl-2-imidazolidinone (DMEU), 1,3-dimethyl-3,4,5,6-tetrahydro-2-(1H)-pyrimidinone (DMPU), acetonitrile (ACN), propylene glycol, ethyl acetate, benzyl alcohol, 2-pyrrolidone, benzyl benzoate, and the like. When water is the solvent, the solvate is referred to as a "hydrate." In some embodiments, the solvent incorporated into a solvate is of a type or at a level that is physiologically tolerable to an organism to which the solvate is administered (e.g., in a unit dosage form of a pharmaceutical composition).

*Pharmaceutical Composition:* As used herein, the term "pharmaceutical composition" or pharmaceutically acceptable composition" comprises an AAV polynucleotides, AAV genomes or AAV particle and one or more pharmaceutically acceptable excipients.

*Pharmacokinetic:* As used herein, "pharmacokinetic" refers to any one or more properties of a molecule or compound as it relates to the determination of the fate of substances administered to living organisms. Pharmacokinetics are divided into several areas including the extent and rate of absorption, distribution, metabolism and excretion. This is commonly referred to as ADME where: (A) Absorption is the process of a substance entering the blood circulation; (D) Distribution is the dispersion or dissemination of substances throughout the fluids and tissues of the body; (M) Metabolism (or Biotransformation) is the irreversible transformation of parent compounds into daughter metabolites; and (E) Excretion (or Elimination) refers to the elimination of the substances from the body. In rare cases, some drugs irreversibly accumulate in body tissue.

*Physicochemical:* As used herein, "physicochemical" means of or relating to a physical and/or chemical property.

*Preventing:* As used herein, the term "preventing" refers to partially or completely delaying onset of an infection, disease, disorder and/or condition; partially or completely delaying onset of one or more symptoms, features, or clinical manifestations of a particular infection, disease, disorder, and/or condition; partially or completely delaying onset of one or more symptoms, features, or manifestations of a particular infection, disease, disorder, and/or condition; partially or completely delaying progression from an infection, a particular disease, disorder and/or condition; and/or decreasing the risk of developing pathology associated with the infection, the disease, disorder, and/or condition.

*Proliferate:* As used herein, the term "proliferate" means to grow, expand, replicate or increase or cause to grow, expand, replicate or increase. "Proliferative" means having the ability to proliferate. "Anti-proliferative" means having properties counter to or in opposition to proliferative properties.

*Protein of interest:* As used herein, the terms "proteins of interest" or "desired proteins" include those provided herein and fragments, mutants, variants, and alterations thereof.

*Purified:* As used herein, the term "purify" means to make substantially pure or clear from unwanted components, material defilement, admixture or imperfection. "Purified" refers to the state of being pure. "Purification" refers to the process of making pure.

*Region:* As used herein, the term "region" refers to a zone or general area. In some embodiments, when referring to a protein or protein module, a region may comprise a linear sequence of amino acids along the protein or protein module or may comprise a three dimensional area, an epitope and/or a cluster of epitopes. In some embodiments, regions comprise terminal regions. As used herein, the term "terminal region" refers to regions located at the ends or termini of a given agent. When referring to proteins, terminal regions may comprise N- and/or C-termini. N-termini refer to the end of a protein comprising an amino acid with a free amino group. C-termini refer to the end of a protein comprising an amino acid with a free carboxyl group. N- and/or C-terminal regions may there for comprise the N- and/or C-termini as well as surrounding amino acids. In some embodiments, N- and/or C-terminal regions comprise from about 3 amino acid to about 30 amino acids, from about 5 amino acids to about 40 amino acids, from about 10 amino acids to about 50 amino acids, from about 20 amino acids to about 100 amino acids and/or at least 100 amino acids. In some embodiments, N-terminal regions may comprise any length of amino acids that includes the N-terminus, but does not include the C-terminus. In some embodiments, C-terminal regions may comprise any length of amino acids, which include the C-terminus, but do not comprise the N-terminus.

In some embodiments, when referring to a polynucleotide, a region may comprise a linear sequence of nucleic acids along the polynucleotide or may comprise a three dimensional area, secondary structure, or tertiary structure. In some embodiments, regions comprise terminal regions. As used herein, the term "terminal region" refers to regions located at the ends or termini of a given agent. When referring to polynucleotides, terminal regions may comprise 5' and 3' termini. 5' termini refer to the end of a polynucleotide comprising a nucleic acid with a free phosphate group. 3' termini refer to the end of a polynucleotide comprising a nucleic acid with a free hydroxyl group. 5' and 3' regions may there for comprise the 5' and 3' termini as well as surrounding nucleic acids. In some embodiments, 5' and 3' terminal regions comprise from about 9 nucleic acids to about 90 nucleic acids, from about 15 nucleic acids to about 120 nucleic acids, from about 30 nucleic acids to about 150 nucleic acids, from about 60 nucleic acids to about 300 nucleic acids and/or at least 300 nucleic acids. In some embodiments, 5' regions may comprise any length of nucleic acids that includes the 5' terminus, but does not include the 3' terminus. In some embodiments, 3' regions may comprise any length of nucleic acids, which include the 3' terminus, but does not comprise the 5' terminus.

*RNA or RNA molecule:* As used herein, the term "RNA" or "RNA molecule" or "ribonucleic acid molecule" refers to a polymer of ribonucleotides; the term "DNA" or "DNA molecule" or "deoxyribonucleic acid molecule" refers to a polymer of deoxyribonucleotides. DNA and RNA can be synthesized naturally, e.g., by DNA replication and transcription of DNA, respectively; or be chemically synthesized. DNA and RNA can be single-stranded (i.e., ssRNA or ssDNA, respectively) or multi-stranded (e.g., double stranded, i.e., dsRNA and dsDNA, respectively). The term "mRNA" or "messenger RNA", as used herein, refers to a single stranded RNA that encodes the amino acid sequence of one or more polypeptide chains.

*RNA interference:* As used herein, the term "RNA interference" or "RNAi" refers to a sequence specific regulatory mechanism mediated by RNA molecules which results in the inhibition or interference or "silencing" of the expression of a corresponding protein-coding gene.

*Sacral Region:* As used herein, the term "sacral region" refers to the region of the spinal cord comprising the sacral vertebrae S1, S2, S3, S4, and S5.

*Sample:* As used herein, the term "sample" refers to an aliquot or portion taken from a source and/or provided for analysis or processing. In some embodiments, a sample is from a biological source such as a tissue, cell or component part (e.g. a body fluid, including but not limited to blood, mucus, lymphatic fluid, synovial fluid, cerebrospinal fluid, saliva, amniotic fluid, amniotic cord blood, urine, vaginal fluid and semen). In some embodiments, a sample may be or comprise a homogenate, lysate or extract prepared from a whole organism or a subset of its tissues, cells or component parts, or a fraction or portion thereof, including but not limited to, for example, plasma, serum, spinal fluid, lymph fluid, the external sections of the skin, respiratory, intestinal, and genitourinary tracts, tears, saliva, milk, blood cells, tumors, organs. In some embodiments, a sample is or comprises a medium, such as a nutrient broth or gel, which may contain cellular components, such as proteins or nucleic acid molecule. In some embodiments, a "primary" sample is an aliquot of the source. In some embodiments, a primary sample is subjected to one or more processing (e.g., separation, purification, etc.) steps to prepare a sample for analysis or other use.

*Self-complementary viral particle:* As used herein, a "self-complementary viral particle" is a particle comprised of at least two components, a protein capsid and a polynucleotide sequence encoding a self-complementary genome enclosed within the capsid.

*Sense strand:* As used herein, the term "the sense strand" or "the second strand" or "the passenger strand" of a siRNA molecule refers to a strand that is complementary to the antisense strand or first strand. The antisense and sense strands of a siRNA molecule are hybridized to form a duplex structure. As used herein, a "siRNA duplex" includes a siRNA strand having sufficient complementarity to a section of about 10-50 nucleotides of the mRNA of the gene targeted for silencing and a siRNA strand having sufficient complementarity to form a duplex with the siRNA strand.

*Signal Sequences:* As used herein, the phrase "signal sequences" refers to a sequence which can direct the transport or localization.

*Single unit dose:* As used herein, a "single unit dose" is a dose of any therapeutic administered in one dose/at one time/single route/single point of contact, i.e., single administration event. In some embodiments, a single unit dose is provided as a discrete dosage form (e.g., a tablet, capsule, patch, loaded syringe, vial, etc.).

*Similarity:* As used herein, the term "similarity" refers to the overall relatedness between polymeric molecules, *e.g.* between polynucleotide molecules (*e.g.* DNA molecules and/or RNA molecules) and/or between polypeptide molecules. Calculation of percent similarity of polymeric molecules to one another can be performed in the same manner as a calculation of percent identity, except that calculation of percent similarity takes into account conservative substitutions as is understood in the art.

*Small*/*short interfering RNA:* As used herein, the term "small/short interfering RNA" or "siRNA" refers to an RNA molecule (or RNA analog) comprising between about 5-60 nucleotides (or nucleotide analogs) which is capable of directing or mediating RNAi. Preferably, a siRNA molecule comprises between about 15-30 nucleotides or nucleotide analogs, more preferably between about 16-25 nucleotides (or nucleotide analogs), even more preferably between about 18-23 nucleotides (or nucleotide analogs), and even more preferably between about 19-22 nucleotides (or nucleotide analogs) (e.g., 19, 20, 21 or 22 nucleotides or nucleotide analogs). The term "short" siRNA refers to a siRNA comprising 5-23 nucleotides, preferably 21 nucleotides (or nucleotide analogs), for example, 19, 20, 21 or 22 nucleotides. The term "long" siRNA refers to a siRNA comprising 24-60 nucleotides, preferably about 24-25 nucleotides, for example, 23, 24, 25 or 26 nucleotides. Short siRNAs may, in some instances, include fewer than 19 nucleotides, e.g., 16, 17 or 18 nucleotides, or as few as 5 nucleotides, provided that the shorter siRNA retains the ability to mediate RNAi. Likewise, long siRNAs may, in some instances, include more than 26 nucleotides, e.g., 27, 28, 29, 30, 35, 40, 45, 50, 55, or even 60 nucleotides, provided that the longer siRNA retains the ability to mediate RNAi or translational repression absent further processing, e.g., enzymatic processing, to a short siRNA. siRNAs can be single stranded RNA molecules (ss-siRNAs) or double stranded RNA molecules (ds-siRNAs) comprising a sense strand and an antisense strand which hybridized to form a duplex structure called siRNA duplex.

*Split dose:* As used herein, a "split dose" is the division of single unit dose or total daily dose into two or more doses.

*Stable:* As used herein "stable" refers to a compound or entity that is sufficiently robust to survive isolation to a useful degree of purity from a reaction mixture, and preferably capable of formulation into an efficacious therapeutic agent.

*Stabilized:* As used herein, the term "stabilize", "stabilized," "stabilized region" means to make or become stable. In some embodiments, stability is measured relative to an absolute value. In some embodiments, stability is measured relative to a reference compound or entity.

*Subject:* As used herein, the term "subject" or "patient" refers to any organism to which a composition in accordance with the invention may be administered, *e.g*., for experimental, diagnostic, prophylactic, and/or therapeutic purposes. Typical subjects include animals (*e.g*., mammals such as mice, rats, rabbits, non-human primates, and humans).

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Substantially equal:* As used herein as it relates to time differences between doses, the term means plus/minus 2%.

*Substantially simultaneously*: As used herein and as it relates to plurality of doses, the term typically means within about 2 seconds.

*Suffering from:* An individual who is "suffering from" a disease, disorder, and/or condition has been diagnosed with or displays one or more symptoms of a disease, disorder, and/or condition.

*Susceptible to:* An individual who is "susceptible to" a disease, disorder, and/or condition has not been diagnosed with and/or may not exhibit symptoms of the disease, disorder, and/or condition but harbors a propensity to develop a disease or its symptoms. In some embodiments, an individual who is susceptible to a disease, disorder, and/or condition (for example, cancer) may be characterized by one or more of the following: (1) a genetic mutation associated with development of the disease, disorder, and/or condition; (2) a genetic polymorphism associated with development of the disease, disorder, and/or condition; (3) increased and/or decreased expression and/or activity of a protein and/or nucleic acid associated with the disease, disorder, and/or condition; (4) habits and/or lifestyles associated with development of the disease, disorder, and/or condition; (5) a family history of the disease, disorder, and/or condition; and (6) exposure to and/or infection with a microbe associated with development of the disease, disorder, and/or condition. In some embodiments, an individual who is susceptible to a disease, disorder, and/or condition will develop the disease, disorder, and/or condition. In some embodiments, an individual who is susceptible to a disease, disorder, and/or condition will not develop the disease, disorder, and/or condition.

*Synthetic:* The term "synthetic" means produced, prepared, and/or manufactured by the hand of man. Synthesis of polynucleotides or polypeptides or other molecules of the present invention may be chemical or enzymatic.

*Targeting:* As used herein, "targeting" means the process of design and selection of nucleic acid sequence that will hybridize to a target nucleic acid and induce a desired effect.

*Targeted Cells:* As used herein, "targeted cells" refers to any one or more cells of interest. The cells may be found *in vitro, in vivo, in situ* or in the tissue or organ of an organism. The organism may be an animal, preferably a mammal, more preferably a human and most preferably a patient.

*Therapeutic Agent:* The term "therapeutic agent" refers to any agent that, when administered to a subject has a therapeutic, diagnostic, and/or prophylactic effect and/or elicits a desired biological and/or pharmacological effect.

*Therapeutically effective amount:* As used herein, the term "therapeutically effective amount" means an amount of an agent to be delivered (*e.g*., nucleic acid, drug, therapeutic agent, diagnostic agent, prophylactic agent, *etc.)* that is sufficient, when administered to a subject suffering from or susceptible to an infection, disease, disorder, and/or condition, to treat, improve symptoms of, diagnose, prevent, and/or delay the onset of the infection, disease, disorder, and/or condition. In some embodiments, a therapeutically effective amount is provided in a single dose. In some embodiments, a therapeutically effective amount is administered in a dosage regimen comprising a plurality of doses. Those skilled in the art will appreciate that in some embodiments, a unit dosage form may be considered to comprise a therapeutically effective amount of a particular agent or entity if it comprises an amount that is effective when administered as part of such a dosage regimen.

*Therapeutically effective outcome*: As used herein, the term "therapeutically effective outcome" means an outcome that is sufficient in a subject suffering from or susceptible to an infection, disease, disorder, and/or condition, to treat, improve symptoms of, diagnose, prevent, and/or delay the onset of the infection, disease, disorder, and/or condition.

*Thoracic Region*: As used herein, a "thoracic region" refers to a region of the spinal cord comprising the thoracic vertebrae T1, T2, T3, T4, T5, T6, T7, T8, T9, T10, T11, and T12.

*Total daily dose:* As used herein, a "total daily dose" is an amount given or prescribed in a 24 hour period. It may be administered as a single unit dose.

*Treating*: As used herein, the term "treating" refers to partially or completely alleviating, ameliorating, improving, relieving, delaying onset of, inhibiting progression of, reducing severity of, and/or reducing incidence of one or more symptoms or features of a particular infection, disease, disorder, and/or condition. For example, "treating" cancer may refer to inhibiting survival, growth, and/or spread of a tumor. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition.

*Unmodified*: As used herein, "unmodified" refers to any substance, compound or molecule prior to being changed in any way. Unmodified may, but does not always, refer to the wild-type or native form of a biomolecule or entity. Molecules or entities may undergo a series of modifications whereby each modified product may serve as the "unmodified" starting molecule or entity for a subsequent modification.

*Vector*: As used herein, a "vector" is any molecule or moiety which transports, transduces or otherwise acts as a carrier of a heterologous molecule. Vectors of the present invention may be produced recombinantly and may be based on and/or may comprise adeno-associated virus (AAV) parent or reference sequence. Such parent or reference AAV sequences may serve as an original, second, third or subsequent sequence for engineering vectors. In non-limiting examples, such parent or reference AAV sequences may comprise any one or more of the following sequences: a polynucleotide sequence encoding a polypeptide or multi-polypeptide, which sequence may be wild-type or modified from wild-type and which sequence may encode full-length or partial sequence of a protein, protein domain, or one or more subunits of a protein; a polynucleotide comprising a modulatory or regulatory nucleic acid which sequence may be wild-type or modified from wild-type; and a transgene that may or may not be modified from wild-type sequence. These AAV sequences may serve as either the "donor" sequence of one or more codons (at the nucleic acid level) or amino acids (at the polypeptide level) or "acceptor" sequences of one or more codons (at the nucleic acid level) or amino acids (at the polypeptide level).

*Viral construct vector:* As used herein, a "viral construct vector" is a vector which comprises one or more polynucleotide regions encoding or comprising Rep and or Cap protein. A viral construct vector may also comprise one or more polynucleotide region encoding or comprising components for viral expression in a viral replication cell.

*Viral genome*: As used herein, a "viral genome" is a polynucleotide encoding at least one inverted terminal repeat (ITR), at least one regulatory sequence, and at least one payload. The viral genome is derived by replication of a payload construct from the payload construct vector. A viral genome encodes at least one copy of the payload construct.

### EQUIVALENTS AND SCOPE

Those skilled in the art will recognize, or be able to ascertain using no more than routine experimentation, many equivalents to the specific embodiments in accordance with the invention described herein. The scope of the present invention is not intended to be limited to the above Description, but rather is as set forth in the appended claims.

In the claims, articles such as "a," "an," and "the" may mean one or more than one unless indicated to the contrary or otherwise evident from the context. claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or the entire group members are present in, employed in, or otherwise relevant to a given product or process.

It is also noted that the term "comprising" is intended to be open and permits but does not require the inclusion of additional elements or steps. When the term "comprising" is used herein, the term "consisting of" is thus also encompassed and disclosed.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

In addition, it is to be understood that any particular embodiment of the present invention that falls within the prior art may be explicitly excluded from any one or more of the claims. Since such embodiments are deemed to be known to one of ordinary skill in the art, they may be excluded even if the exclusion is not set forth explicitly herein. Any particular embodiment of the compositions of the invention (e.g., any antibiotic, therapeutic or active ingredient; any method of production; any method of use; etc.) can be excluded from any one or more claims, for any reason, whether or not related to the existence of prior art.

It is to be understood that the words which have been used are words of description rather than limitation, and that changes may be made within the purview of the appended claims without departing from the true scope and spirit of the invention in its broader aspects.

While the present invention has been described at some length and with some particularity with respect to the several described embodiments, it is not intended that it should be limited to any such particulars or embodiments or any particular embodiment, but it is to be construed with references to the appended claims so as to provide the broadest possible interpretation of such claims in view of the prior art and, therefore, to effectively encompass the intended scope of the invention.

### EXAMPLES

### Example 1. Design of Payload Constructs

Payload constructs were designed to comprise at a minimum a nucleic acid sequence encoding a frataxin protein.

Once designed, the sequence was engineered or synthesized or inserted in a plasmid or vector and administered to a cell or organism. Suitable plasmids or vectors were any which transduce or transfect the target cell.

Adeno-associated viral vectors (AAV), viral particles or entire viruses may be used.

Administration resulted in the processing of the payload construct to generate the frataxin protein which alters the etiology of the disease, in this case Friedriech's Ataxia.

AAV constructs were designed and built using standard molecular cloning techniques. FXN-tag transgenes were cloned into either pAAVss, pAAVsc, or pcDNA3.1 plasmid and the resulting clones were further sequenced to confirm the correctness of all elements such as ITRs, promoters, and tags.

In one non-limiting example, plasmids containing a payload construct are described herein and some are described in Table 3. These payload constructs in Table 3 may comprise a pCDNA3.1, pAAVss, or pAAVsc vectors and may comprise the following components: a CMV, CB6, CB7, PGK, GFAP, hSYN, mCMVe-hEF1p, SV40, CBA or FXN promoter; an SV40 or MVM/CBA intron; a full or partial Kozak sequence; a FXN (Frataxin), CS (citrate synthase), RPL (ribosomal protein), SOD2 (superoxide dismutase), or AH (aconitate hydratase) signal peptide, also known as a mitochondrial targeting sequence (MTS); a cmyc, flag, cmycflag3, 3flag, 3flagcmyc, HA long or HA short tag; a SV40, rabbit beta-globin, or bGH poly (A) signal, 3' and/or 5' ITR sequences derived from any AAV genome comprising a partial and/or wild type sequence; and either wild type Frataxin or codon optimized Frataxin.

**Table 3. AAV constructs**

| **Vector** | **Promoter** | **Intron** | **Kozak** | **0.5 Kozak** | **Signal Peptide** | **Payload** | **Tag** | **5'ITR** | **Poly(A)** | **3'ITR** | **SEQ ID NO** |
|---|---|---|---|---|---|---|---|---|---|---|---|
| pCD NA3. 1(+) | CMV | N/A | - | - | FXN | FXN | cmyc 3flag | - | bGH | - | 1 |
| pCD NA3. 1(+) | CMV | N/A | - | - | FXN | FXN | - | - | bGH | - | 2 |
| pCD NA3. 1(+) | CMV | N/A | - | - | FXN | FXN | 3flag | - | bGH | - | 3 |
| pCD NA3. 1(+) | CMV | N/A | - | - | FXN | FXN | 3flagc myc | - | bGH | - | 4 |
| pCD NA3. 1(+) | CMV | N/A | - | - | FXN | FXN | cmyc | - | bGH | - | 5 |
| pCD NA3. 1(+) | CMV | N/A | - | - | FXN | FXN | HA(L) | - | bGH | - | 6 |
| pCD NA3. 1(+) | CMV | N/A | - | - | FXN | FXN | HA(S) | - | bGH | - | 7 |
| pCD NA3. 1(+) | CMV | N/A | + | - | CS | FXN | - | - | bGH | - | 8 |
| pCD NA3. 1(+) | CMV | N/A | - | + | FXN | FXN | - | - | bGH | - | 9 |
| pAA Vss | CB6 | N/A | - | + | FXN | FXN | - | + | SV40 | + | 10 |
| pAA Vss | CB6 | MV M/CBA | - | + | FXN | FXN | - | + | SV40 | + | 11 |
| pAA Vss | CB6 | SV40 | - | - | FXN | FXN | - | + | SV40 | + | 12 |
| pAA Vss | CB6 | SV40 | - | - | FXN | CodOp FXN | - | + | SV40 | + | 13 |
| pAA Vss | CB6 | SV40 | - | - | FXN | CodOp FXN | - | + | SV40 | + | 14 |
| pAA Vss | CB6 | SV40 | - | - | FXN | CodOp FXN | - | + | SV40 | + | 15 |
| pAA Vss | CB6 | SV40 | - | - | FXN | CodOp FXN | - | + | SV40 | + | 16 |
| pAA Vss | CB6 | SV40 | - | - | FXN | CodOp FXN | - | + | SV40 | + | 17 |
| pAA Vss | CB6 | SV40 | - | - | FXN | CodOp FXN | - | + | SV40 | + | 18 |
| pAA Vss | CB6 | SV40 | - | - | FXN | CodOp FXN | - | + | SV40 | + | 19 |
| pAA Vss | CB6 | SV40 | - | - | FXN | CodOp FXN | - | + | SV40 | + | 20 |
| pAA Vss | CB6 | SV40 | - | - | FXN | CodOp FXN | - | + | SV40 | + | 21 |
| pAA Vss | CB6 | SV40 | - | - | FXN | CodOp FXN | - | + | SV40 | + | 22 |
| pAA Vss | CB6 | SV40 | - | - | FXN | CodOp FXN | - | + | SV40 | + | 23 |
| pAA Vss | CB6 | SV40 | - | - | FXN | CodOp FXN | - | + | SV40 | + | 24 |
| pAA Vss | CB6 | SV40 | + | - | AH | FXN | - | + | SV40 | + | 25 |
| pAA Vss | CB6 | SV40 | + | - | CS | FXN | - | + | SV40 | + | 26 |
| pAA Vss | CB6 | SV40 | + | - | CS | FXN | HA(L) | + | SV40 | + | 27 |
| pAA Vss | CB6 | SV40 | + | - | CS | FXN | HA(S) | + | SV40 | + | 28 |
| pAA Vss | CB6 | SV40 | - | + | FXN | FXN | - | + | SV40 | + | 29 |
| pAA Vss | CB6 | SV40 | - | + | FXN | CodOp FXN | - | + | SV40 | + | 30 |
| pAA Vss | CB6 | SV40 | - | + | FXN | CodOp FXN | - | + | SV40 | + | 31 |
| pAA Vss | CB6 | SV40 | - | + | FXN | CodOp FXN | - | + | SV40 | + | 32 |
| pAA Vss | CB6 | SV40 | - | + | FXN | CodOp FXN | - | + | SV40 | + | 33 |
| pAA Vss | CB6 | SV40 | - | + | FXN | CodOp FXN | - | + | SV40 | + | 34 |
| pAA Vss | CB6 | SV40 | - | + | FXN | CodOp FXN | - | + | SV40 | + | 35 |
| pAA Vss | CB6 | SV40 | - | + | FXN | CodOp FXN | - | + | SV40 | + | 36 |
| pAA Vss | CB6 | SV40 | + | - | RPL | FXN | - | + | SV40 | + | 37 |
| pAA Vss | CB6 | SV40 | + | - | RPL | CodOp FXN | - | + | SV40 | + | 38 |
| pAA Vss | CB6 | SV40 | - | + | SOD2 | CodOp FXN | - | + | SV40 | + | 39 |
| pAA Vss | CB7 | SV40 | - | - | FXN | FXN | - | + | SV40 | + | 40 |
| pAA Vss | CMV | N/A | - | + | FXN | FXN | - | + | SV40 | + | 41 |
| pAA Vss | CMV | SV40 | - | - | FXN | FXN | - | + | SV40 | + | 42 |
| pAA Vss | CMV | SV40 | - | - | FXN | CodOp FXN | - | + | SV40 | + | 43 |
| pAA Vss | CMV | SV40 | - | - | FXN | CodOp FXN | - | + | SV40 | + | 44 |
| pAA Vss | CMV | SV40 | - | - | FXN | CodOp FXN | - | + | SV40 | + | 45 |
| pAA Vss | CMV | SV40 | - | - | FXN | CodOp FXN | - | + | SV40 | + | 46 |
| pAA Vss | CMV | SV40 | - | + | FXN | FXN | - | + | SV40 | + | 47 |
| pAA Vss | FXNp | SV40 | - | - | FXN | FXN | - | + | SV40 | + | 48 |
| pAA Vss | FXNp | SV40 | - | - | FXN | CodOp FXN | - | + | SV40 | + | 49 |
| pAA Vss | FXNp | SV40 | - | - | FXN | CodOp FXN | - | + | SV40 | + | 50 |
| pAA Vss | FXNp | SV40 | - | - | FXN | CodOp FXN | - | + | SV40 | + | 51 |
| pAA Vss | FXNp | SV40 | - | - | FXN | CodOp FXN | - | + | SV40 | + | 52 |
| pAA Vss | GFAP | SV40 | - | - | FXN | FXN | - | + | SV40 | + | 53 |
| pAA Vss | hSYN | SV40 | - | - | FXN | FXN | - | + | SV40 | + | 54 |
| pAA Vss | mCMVe-hEF1p | SV40 | - | - | FXN | FXN | - | + | SV40 | + | 55 |
| pAA Vss | mCMVe-hEF1p | SV40 | - | - | FXN | FXN | - | + | SV40 | + | 56 |
| pAA Vss | PGK | SV40 | - | - | FXN | FXN | - | + | SV40 | + | 57 |
| pAA Vss | PGK | SV40 | - | - | FXN | CodOp FXN | - | + | SV40 | + | 58 |
| pAA Vss | PGK | SV40 | - | - | FXN | CodOp FXN | - | + | SV40 | + | 59 |
| pAA Vss | PGK | SV40 | - | - | FXN | CodOp FXN | - | + | SV40 | + | 60 |
| pAA Vss | PGK | SV40 | - | - | FXN | CodOp FXN | - | + | SV40 | + | 61 |
| pAA Vss | SV40 | SV40 | - | - | FXN | FXN | - | + | SV40 | + | 62 |
| pAA Vss | CBA | SV40 | - | - | FXN | FXN | - | + | SV40 | + | 63 |
| pAA Vsc | CBA | SV40 | - | - | FXN | FXN | HA(S) | + | SV40 | + | 64 |
| pAA Vsc | CBA | SV40 | - | - | FXN | FXN | 3flag | + | SV40 | + | 65 |
| pAA Vsc | CBA | SV40 | - | - | FXN | FXN | 3flagc myc | + | SV40 | + | 66 |
| pAA Vsc | CBA | SV40 | - | - | FXN | FXN | cmyc | + | SV40 | + | 67 |
| pAA Vsc | CBA | SV40 | - | - | FXN | FXN | cmyc 3flag | + | SV40 | + | 68 |
| pAA Vsc | CBA | SV40 | - | - | FXN | CodOp FXN | - | + | SV40 | + | 69 |
| pAA Vsc | CBA | SV40 | - | - | FXN | CodOp FXN | - | + | SV40 | + | 70 |
| pAA Vsc | CBA | SV40 | - | - | FXN | CodOp FXN | - | + | SV40 | + | 71 |
| pAA Vsc | CBA | SV40 | - | - | FXN | CodOp FXN | - | + | SV40 | + | 72 |
| pAA | CMV | SV40 | - | - | FXN | FXN | - | + | SV40 | + | 73 |
| pAA Vsc | CMV | SV40 | - | - | FXN | CodOp FXN | - | + | SV40 | + | 74 |
| pAA Vsc | CMV | SV40 | - | - | FXN | CodOp FXN | - | + | SV40 | + | 75 |
| pAA Vsc | CMV | SV40 | - | - | FXN | CodOp FXN | - | + | SV40 | + | 76 |
| pAA Vsc | CMV | SV40 | - | - | FXN | CodOp FXN | - | + | SV40 | + | 77 |
| pAA Vsc | CMV | SV40 | - | + | FXN | FXN | - | + | SV40 | + | 78 |
| pAA Vsc | FXNp | SV40 | - | - | FXN | FXN | - | + | SV40 | + | 79 |
| pAA Vsc | FXNp | SV40 | - | - | FXN | CodOp FXN | - | + | SV40 | + | 80 |
| pAA Vsc | FXNp | SV40 | - | - | FXN | CodOp FXN | - | + | SV40 | + | 81 |
| pAA Vsc | FXNp | SV40 | - | - | FXN | CodOp FXN | - | + | SV40 | + | 82 |
| pAA Vsc | FXNp | SV40 | - | - | FXN | CodO p FXN | - | + | SV40 | + | 83 |
| pAA Vsc | GFAP | SV40 | - | - | FXN | FXN | - | + | SV40 | + | 84 |
| pAA Vsc | PGK | SV40 | - | - | FXN | FXN | - | + | SV40 | + | 85 |
| pAA Vsc | PGK | SV40 | - | - | FXN | CodOp FXN | - | + | SV40 | + | 86 |
| pAA Vsc | PGK | SV40 | - | - | FXN | CodOp FXN | - | + | SV40 | + | 87 |
| pAA Vsc | PGK | SV40 | - | - | FXN | CodOp FXN | - | + | SV40 | + | 88 |
| pAA Vsc | PGK | SV40 | - | - | FXN | CodOp FXN | - | + | SV40 | + | 89 |
| pAA Vss | CB6 | SV40 | - | + | FXN | FXN | - | + | SV40 | + | 90 |
| pAA Vsc | CBA | SV40 | - | + | FXN | FXN | - | + | SV40 | + | 91 |
| pAA Vss | N/A | SV40 | - | - | FXN | FXN | - | + | SV41 | + | 92 |
| pAA Vss | CBA | SV40 | - | - | FXN | FXN | - | + | SV42 | + | 93 |

Plasmid constructs suitable for use in AAV particles include those listed in the sequence listing.

### Example 2. Plasmid Construct Component Modules

Plasmid constructs were designed according to Table 4. The start and stop positions of various components given are relative to the AAV particles described in the sequence listing.

**Table 4. Component modules or sequence regions of plasmid constructs**

| **SEQ ID NO** | | **73** | **63** | **79** | **85** | **75** | **70** | **81** | **87** |
|---|---|---|---|---|---|---|---|---|---|
| **5'ITR** | **Start** | 65 | 1 | 65 | 65 | 65 | 1 | 65 | 65 |
| | **Stop** | 169 | 105 | 169 | 169 | 169 | 105 | 169 | 169 |
| **Promoter** | **Start** | 205 | 138 | 217 | 214 | 205 | 138 | 217 | 214 |
| | **Stop** | 883 | 789 | 1569 | 735 | 883 | 789 | 1569 | 735 |
| **SV40** | **Start** | 897 | 803 | 1576 | 742 | 897 | 803 | 1576 | 742 |
| | **Stop** | 1068 | 974 | 1747 | 913 | 1068 | 974 | 1747 | 913 |
| **FXN** | **Start** | 1086 | 992 | 1765 | 931 | 1086 | 992 | 1765 | 931 |
| | **Stop** | 1718 | 1624 | 2397 | 1563 | 1718 | 1624 | 2397 | 1563 |
| **Poly(A)** | **Start** | 1798 | 1704 | 2477 | 1643 | 1752 | 1658 | 2431 | 1597 |
| | **Stop** | 1924 | 1830 | 2603 | 1769 | 1878 | 1784 | 2557 | 1723 |
| **3'ITR** | **Start** | 1968 | 1874 | 2647 | 1813 | 1922 | 1828 | 2601 | 1767 |
| | **Stop** | 2097 | 2003 | 2776 | 1942 | 2051 | 1957 | 2730 | 1896 |

### Example 3. ELISA Assay for Detecting Differential Payload Expression from Regulatory Elements

The HEK293 cell line was transfected with AAV constructs, SEQ ID Nos. 10-12, 14, 16, 19, 20, 22-26, 29-39, 41-52, 57-61, 63, 70, 73, 75, 79, 81, 85, or 87 to assay the level of expression of a Frataxin payload sequence under control of various regulatory elements. The gWiz-GFP plasmid was co-transfected with each construct as an internal transfection efficiency control. The transfected 293FT cells were harvested 48 hours post-transfection, lysed using the THERMO SCIENTIFIC™ PIERCE™ M-PER™ Mammalian Protein Extraction Reagent, and resuspended in 200ul of lysis buffer. Protein concentration in each of the samples was measured using the THERMO SCIENTIFIC™ PIERCE™ BCA™ Protein Assay.

ELISA assays for detecting expressed FXN and GFP in cell lysates were performed using the Abcam SimpleStep ELISA kit for FXN and GFP. The results in Tables 5-11 represent Frataxin expression values normalized to GFP. Comparison of expression levels between FXN constructs is represented by further normalization of Frataxin levels relative to reference Frataxin levels.

The results in Tables 5 and 6 represent Frataxin expression values from constructs containing a Kozak Sequence (GCCACCATG) compared to constructs lacking a Kozak sequence.

**Table 5. Effect of Kozak Sequence on Frataxin Expression In Vitro**

| **Kozak** | **Relative Expression** | **SEQ ID NO.** |
|---|---|---|
| No | 1.91 | 42 |
| Yes | 1.73 | 47 |
| No | 1.49 | 20 |
| Yes | 0.76 | 30 |
| Yes | 1.24 | 29 |
| No | 0.98 | 12 |

**Table 6. Effect of Kozak Sequence on Frataxin Expression In Vitro**

| **Kozak** | **Relative Expression** | **SEQ ID NO.** |
|---|---|---|
| No | 1.45 | 23 |
| Yes | 1.56 | 31 |
| No | 0.76 | 24 |
| Yes | 0.77 | 32 |
| N/A | 1.00 | 12 |

The construct encoding a codon-optimized frataxin driven by the CB6 promoter and lacking a Kozak sequence (SEQ ID NO: 20) gave the highest FXN expression compared to a similiar construct with a Kozak sequence (SEQ ID NO: 30).

The results in Table 7 represent Frataxin expression values from constructs comprising a mitochondrial targeting sequence (MTS), also referred to herein as a signal peptide, compared to constructs lacking an MTS. A wild-type FXN MTS (SEQ ID NO: 29) results in the highest level of FXN protein expression in this experiment.

**Table 7. Effect of Signal Peptide - Mitochondrial Targeting Sequence on Frataxin Expression In Vitro**

| **Signal Peptide** | **Relative Expression** | **Standard Deviation** | **SEQ ID NO.** |
|---|---|---|---|
| RPL | 0.64 | 0.00 | 37 |
| CS | 0.53 | 0.01 | 26 |
| SOD2 | 0.80 | 0.01 | 39 |
| AH | 0.49 | 0.01 | 25 |
| N/A | 0.98 | 0.02 | 29 |
| N/A | 1.02 | 0.06 | 12 |

The results in Table 8 represent Frataxin expression values from constructs comprising an SV40 intron compared to constructs lacking an SV40 intron. The presence of an SV40 intron had no significant effect on CMV and CB6-driven FXN expression in HEK293 cells.

**Table 8. Effect of SV40 Intron on Frataxin Expression In Vitro**

| **SV40 Intron** | **Relative Expression** | **Standard Deviation** | **SEQ ID NO.** |
|---|---|---|---|
| Yes | 1.24 | 0.00 | 29 |
| No | 1.03 | 0.02 | 10 |
| Yes | 2.26 | 0.11 | 47 |
| No | 2.43 | 0.23 | 41 |
| No | 1.26 | 0.03 | 11 |
| No | 1.00 | 0.09 | 12 |

The results in Table 9 represent Frataxin expression values from constructs comprising codon optimized frataxin sequences compared to a construct comprising a wild-type frataxin sequence. The effect of Frataxin transgene codon optimization on expression level was variable, with the highest expression levels observed in SEQ ID NOs. 20 and 22.

**Table 9. Effect of Codon Optimization on Frataxin Expression In Vitro**

| **Codon Optimized** | **Relative Expression** | **Standard Deviation** | **SEQ ID NO.** |
|---|---|---|---|
| Yes | 0.96 | 0.02 | 33 |
| Yes | 0.81 | 0.01 | 34 |
| Yes | 1.32 | 0.10 | 35 |
| Yes | 1.06 | 0.03 | 36 |
| Yes | 1.56 | N/A | 31 |
| Yes | 0.77 | N/A | 32 |
| Yes | 1.72 | 0.04 | 14 |
| Yes | 1.88 | 0.36 | 16 |
| Yes | 1.07 | 0.22 | 19 |
| Yes | 2.21 | 0.20 | 20 |
| Yes | 2.20 | 0.16 | 22 |
| No | 1.00 | N/A | 12 |

The results in Table 10 represent Frataxin expression values from constructs comprising CB6, CMV, or PGK promoter sequences compared to constructs comprising a wild-type frataxin promoter sequence. The values for SEQ ID NO: 12 represent expression levels in two separate experiments. The presence of a CMV or CB6 promoter had the greatest effect of enhancing the expression level of Frataxin approximately 2 fold.

**Table 10. Effect of CB6, CMV or PGK Promoters on Frataxin Expression In Vitro**

| **Promoter** | **Relative Expression** | **Standard Deviation** | **SEQ ID NO.** |
|---|---|---|---|
| CB6 | 1.03 | 0.05 | 12 |
| CB6 | 1.00 | 0.09 | 12 |
| CB6 | 1.72 | 0.04 | 14 |
| CB6 | 1.88 | 0.36 | 16 |
| CB6 | 1.07 | 0.22 | 19 |
| CB6 | 2.21 | 0.20 | 20 |
| CB6 | 2.20 | 0.16 | 22 |
| CMV | 1.78 | 0.36 | 43 |
| CMV | 2.16 | 0.17 | 44 |
| CMV | 2.20 | 0.29 | 45 |
| CMV | 2.54 | 0.03 | 46 |
| CMV | 1.63 | 0.12 | 42 |
| PGK | 0.20 | N/A | 57 |
| PGK | 0.23 | N/A | 58 |
| PGK | 0.26 | N/A | 59 |
| PGK | 0.36 | N/A | 60 |
| PGK | 0.72 | N/A | 61 |
| FXN | 0.37 | N/A | 48 |
| FXN | 0.45 | N/A | 49 |
| FXN | 1.07 | N/A | 50 |
| FXN | 1.65 | N/A | 51 |
| FXN | 1.21 | N/A | 52 |

The results in Table 11 represent Frataxin expression values from pAAVsc-Promoter-SV40. The results in Table 11 represent Frataxin expression values from self-complementary AAV constructs comprising CB6, CMV, or PGK promoter sequences compared to constructs comprising a wild-type frataxin promoter sequence. The presence of a CMV promoter had the greatest effect of enhancing the expression level of Frataxin approximately 2-3 fold.

**Table 11. Effect of CB6, CMV or PGK Promoter on Relative Expression From pAAVsc-Promoter-SV40 Constructs in 293FT Cells**

| **Relative Expression** | **Standard Deviation** | **SEQ ID NO.** |
|---|---|---|
| 3.10 | 0.24 | 73 |
| 3.02 | 0.11 | 63 |
| 2.18 | 0.06 | 75 |
| 1.00 | 0.20 | 70 |
| 0.14 | 0.01 | 85 |
| 0.13 | 0.00 | 87 |
| 0.13 | 0.00 | 79 |
| 0.10 | 0.00 | 81 |
| 0.03 | N/A | 73 |

### Example 4. AAV-FXN constructs and ELISA assays for detecting Frataxin expression

AAV constructs used for this study were designed and built using standard molecular cloning techniques. FXN-tag transgenes were cloned into pAAVsc plasmid and pcDNA3.1 plasmid and the resulting clones were further sequenced to confirm the correctness of all elements such as ITRs, CBA, SV40 and FXN-tags. The pAAVsc-FXN-tag and pcDNA3.1-FXN-tag constructs were transfected to 293FTcells to confirm the expression of FXN-tag proteins. Western blotting using either anti-tag (e.g., anti-HA) or anti-frataxin antibodies confirmed that cloned frataxin-tag is expressed in 293FTcells (data not known).

ELISA assays for detecting expressed FXN-tags were also developed. A three-step sandwich ELISA assay using either anti-tag (e.g. HA, c-myc, flag) or anti-frataxin antibodies as capture antibodies was tested and the ELISA assay may be used for determining frataxin expression levels in CNS delivery studies *in vivo.* Each serotype was also confirmed by silver-stain-SDS page analysis. The AAV viral genome titers (either self-complementary (sc) or single stranded (ss) AAVs) are described in Table 12.

**Table 12. AAV Viral Genome titers**

| **Particles** | **Genome** | **LOT NO.** | **Vg plasmid** | **Rep-Cap** | **Ad helper** | **Titer** |
|---|---|---|---|---|---|---|
| AA V2-SEQ ID NO: 90 | SS | VCAV-01870 | A1d-46819 | VCPV-0136 | A1d-39062A | 0.9x10¹³ |
| AAV5-SEQ ID NO: 90 | SS | VCAV-01871 | A1d-46819 | VCPV0128-1 | A1d-39062A | 1.4x10¹³ |
| AAV6-SEQ ID NO: 90 | SS | VCAV-01851 | A1d-46819 | VCPV-0132 | A1d-39062A | 0.7x10¹³ |
| AAV9-SEQ ID NO: 90 | SS | VCA V-02037 | A1d-48657 | A1d-44533 | A1d-29504 | 1.6x10¹³ |
| AAVrh10-SEQ ID NO: 90 | SS | VCAV-01842 | A1d-46819 | VCPV-0135 | A1d-39062A | 1.1x10¹³ |
| AAVDJ-SEQ ID NO: 90 | SS | VCAV-01858 | A1d-46819 | VCPI-412 | A1d-39062A | 1.0x10¹³ |
| AAVDJ/8-SEQ ID NO: 90 | SS | VCAV-01888 | - | - | - | 1.3x10¹³ |
| AAVrh10-SEQ ID NO: 91 | SC | VCA V-01991 | A1d-47191 | VCPV-0135 | A1d-39062A | 0.7x10¹³ |

### Example 5. In vivo frataxin delivery study

### Study design

This study is designed to compare several AAV capsids for payload delivery to the central nervous system (CNS) after direct CNS administration. A total of 160 wild type mice (male, C57BL/6), 10-12 weeks old, were divided into 11 study groups. Mice in each study group were administered an AAV (either self-complementary (sc) or single stranded (ss) AAV) as described in the study design in Table 13. The AAV genomes (either self-complementary (sc) or single stranded (ss) AAVs) with a CB promoter, described in Table 14, were formulated in Phosphate Buffered Saline (PBS), 5% Sorbitol and 0.001% F-68 (Formulation 1) or PBS and 0.001% F-68 (Formulation 2) and administered via intracerebroventricular (ICV) or intrastriatal (IS) injection.

**Table 13. Mouse Capsid Comparison Study - Design**

| Test Articles (Lot No.) | AAV construct | AAV Genome | Total Dose (VG) | Route | Formulation | n | End of Study |
|---|---|---|---|---|---|---|---|
| Vehicle | - | - | 0 | ICV | Formulation 1 | 8 | Day 56 |
| | | | | IS | Formulation 1 | 8 | Day 28 |
| VCAV-01801-B | AAV9-SEQ ID NO: 91 | SC | 5 x 10¹⁰ | ICV | Formulation 1 | 8 | Day 56 |
| | | | | IS | Formulation 1 | 8 | Day 28 |
| VCAV-01791 | AAV9-SEQ ID NO: 90 | SS | 5x10¹⁰ | ICV | Formulation 1 | 8 | Day 56 |
| | | | | IS | Formulation 1 | 8 | Day 28 |
| VCAV-01870 | AAV2-SEQ ID NO: 90 | SS | 5x10¹⁰ | ICV | Formulation 1 | 8 | Day 56 |
| | | | | IS | Formulation 1 | 8 | Day 28 |
| VCAV-01871 | AAV5-SEQ ID NO: 90 | SS | 5 x 10¹⁰ | ICV | Formulation 1 | 8 | Day 56 |
| | | | | IS | Formulation 1 | 8 | Day 28 |
| VCAV-01851 | AAV6-SEQ ID NO: 90 | SS | 5 x 10¹⁰ | ICV | Formulation 1 | 8 | Day 56 |
| | | | | IS | Formulation 1 | 8 | Day 28 |
| VCAV-01842 | AAVrh10-SEQ ID NO: 90 | SS | 5 x 10¹⁰ | ICV | Formulation 1 | 8 | Day 56 |
| | | | | IS | Formulation 1 | 8 | Day 28 |
| VCAV-01858 | AAVDJ-SEQ ID NO: 90 | SS | 5 x 10¹⁰ | ICV | Formulation 1 | 8 | Day 56 |
| | | | | IS | Formulation 1 | 8 | Day 28 |
| VCAV-01888 | AAVDJ/8-SEQ ID NO: 90 | SS | 5 x 10¹⁰ | ICV | Formulation 1 | 8 | Day 56 |
| | | | | IS | Formulation 1 | 8 | Day 28 |
| VCAV-01962-B | AAVrh10-SEQ ID NO: 91 | SC | 2 x 10⁹ | IS | Formulation 2 | 8 | Day 28 |
| VCAV-01962-T | AAVrh10-SEQ ID NO: 91 | SC | 5 x 10¹⁰ | IS | Formulation 2 | 8 | Day 28 |

**Table 14. Mouse Capsid Comparison Study - AAV Genome Sequences**

| **Test article** | **Capsid** | **AAV Genome** | **Genome SEQ ID NO** |
|---|---|---|---|
| VCAV-01801-B | AAV9 | SC | 91 |
| VCAV-01791 | AAV9 | SS | 90 |
| VCAV-01870 | AAV2 | SS | 90 |
| VCAV-01871 | AAV5 | SS | 90 |
| VCAV-01851 | AAV6 | SS | 90 |
| VCAV-01842 | AAVrh10 | SS | 90 |
| VCAV-01858 | AAVDJ | SS | 90 |
| VCAV-01888 | AAVDJ/8 | SS | 90 |
| VCAV-01962-B | AAVrh10 | SC | 91 |
| VCAV-01962-T | AAVrh10 | SC | 91 |

### In vivo study

The mice were given a bolus (5 x 10¹⁰ VG/5 ul total dose at a 1 x 10¹³ VG/mL composition concentration) single unilateral injection (0.5 ul/min) via either intracerebroventricular (ICV) or intrastriatal (IS) administration with a Hamilton 1701 gauge 33 bevel 60 degree needle. The injection site for ICV administration was AP -0.35, ML 1.0, DV -2.5 mm from bregma and the injection site for IS administration was AP 0.5, ML -2.0, DV -4 mm from bregma. Two mm coronal slabs were prepared from the brain. Tissue at the injection site and remaining tissue from the same coronal slab, as well as adjacent slabs immediately anterior (containing frontal cortex) and posterior to the injection site, were taken for human frataxin protein quantitation using the SimpleStep human frataxin ELISA (Abcam) which is specific for human frataxin (no detection of mouse frataxin). Mouse endogenous frataxin protein levels were quantified by a different frataxin ELISA (Abcam).

### Intrastriatal Injection

Human frataxin levels in left striatum (CPuL) on day 28 following IS injection were determined using ELISA assays and the average frataxin expression (ng/mg protein) are shown in Table 15, wherein the AAV genomes are self-complementary (sc) or single stranded (ss). High human frataxin levels were observed following administration of ssAAV9-FXN, ssAAV6-FXN, and ssAAVDJ8-FXN (Lot Nos. VCAV-01791, VCAV-01851, and VCAV-01888). The self-complementary particles, scAAV9-FXN (Lot No. VCAV-01801-B) and scAAVrh10-FXN (Lot No. VCAV-01962-T) resulted in levels 3 and 14 fold higher than single-stranded ssAAV9-FXN (Lot No. VCAV-01791) and ssAAVrh10-FXN (Lot No. VCAV-01842). At the injection site, all the vectors resulted in levels greater than mouse endogenous frataxin, which was measured to be 10 ng/mg protein.

Human frataxin levels in the 2 mm brain slice immediately posterior to the injection site, on day 28 following IS injection were determined using ELISA assays and the average frataxin expression levels (ng/mg protein) are shown in Table 15 (Slab3). ssAAV9, ssAAVDJ8, and ssAAVrh10 (Lot Nos. VCAV-01791, VCAV-01888 and VCAV-01842) IS injected mice had robust expression of human frataxin in the brain, greater than 7-16 fold mouse endogenous frataxin levels, compared to the other viral particles. ssAAV5 (Lot No.VCAV-01871) and ssAAV6 (Lot No.VCAV-01851) IS injected mice resulted in moderate expression, about 2-3 fold mouse frataxin levels, compared to the other viral particles. The results also indicated that ssAAV constructs (e.g., ssAAV 9 (Lot. No.VCAV-01791) and ssAAVrh10 (Lot. No.VCAV-01842)) resulted in higher expression levels than scAAV constructs (e.g., scAAV9 (Lot No.VCAV-01801-B) and scAAVrh10 (Lot No. VCAV-01962-T)).

**Table 15. Mouse Capsid Comparison Study - Brain Levels of Human FXN Following IS Injection**

| **Capsid** | **AAV Genome** | **Genome SEQ ID NO** | **CPuL AVG ± SEM** | **Slab3 AVG ± SEM** |
|---|---|---|---|---|
| AAV2 | SS | 90 | 52.10 ± 10.82 | 3.11 ± 0.82 |
| AAV5 | SS | 90 | 75.10 ± 19.56 | 30.80 ± 5.12 |
| AAV6 | SS | 90 | 707.71 ± 203.62 | 16.64 ± 7.06 |
| AAVDJ | SS | 90 | 95.77 ± 29.76 | 6.89 ± 1.04 |
| AAVDJ8 | SS | 90 | 395.14 ± 49.50 | 144.94 ± 18.99 |
| AAV9 | SS | 90 | 809.48 ± 80.21 | 160.89 ± 6.78 |
| AAVrh10 | SS | 90 | 293.58 ± 40.60 | 70.57 ± 16.03 |
| scAAV9 | SC | 91 | 2463.73 ± 357.92 | 4.98 ± 0.47 |
| scAAVrh10-T | SC | 91 | 4233.40 ± 535.40 | 7.71 ± 0.94 |
| scAAVrh10-B | SC | 91 | 176.11 ± 29.10 | 25.89 ± 5.61 |
| Sorbitol formulation (background) | | | 0.02 ± 0.01 | 0.02 ± 0.01 |

### ICV Injection

On day 56 following ICV injection, human frataxin protein levels were measured by ELISA at the injection site (Ing. Site, Table 16) and surrounding tissue from tehe same 2 mm slice (Slab 2, Table 16). In addition, human frataxin protein levels were measured by ELISA in a 2 mm slice immediately anterior (containing mostly frontal cortex, Slab 1, Table 16), and in a 2 mm slice immediately posterior (Slab 3, Table 16). The AAV genomes were self-complementary (sc) or single stranded (ss). The injection of ssAAV6-FXN VCAV-01851 resulted in moderate expression at the injection site (5 fold mouse frataxin). The self-complementary vector scAAV9-FXN VCAV-01801-B resulted in high expression levels, 100 fold higher than single-stranded ssAAV9-FXN VCAV-01791. The spread of human frataxin expression following ssAAV2-FXN VCAV-01870 and ssAAV5-FXN VCAV-01871 is limited, with highest levels found at the injection site and much lower levels anterior (Slab 2) and posterior (Slab 3) to the injection site. In contrast, ssAAV9-FXN VCAV01791 and ssAAVrh10-FXN VCAV-01842 show a more consistent expression levels in the different antero-posterior samples analyzed. ssAAVDJ-FXN VCAV-1858 and ssAAVDJ8-FXN VCAV-01888 resulted in similar levels of human frataxin. Of the single-stranded vectors, ssAAV6-FXN VCAV-01851 resulted in the highest maximum and overall levels of FXN expression.

**Table 16. Mouse Capsid Comparison Study - Brain Levels of human FXN Following ICV Injection**

| **Capsid** | **AAV Genome** | **Genome SEQ ID NO** | **Slab1 AVG ± SEM** | **Slab2 AVG ± SEM** | **Inj. Site AVG ± SEM** | **Slab3 AVG ± SEM** |
|---|---|---|---|---|---|---|
| AAV2 | SS | 90 | 0.07 ± 0.01 | 0.28 ± 0.04 | 1.98 ± 0.59 | 0.18 ± 0.06 |
| AAV5 | SS | 90 | 0.13 ± 0.06 | 1.68 ± 0.89 | 47.53 ± 15.53 | 5.41 ± 1.33 |
| AAV6 | SS | 90 | 1.06 ± 0.14 | 12.52 ± 2.40 | 53.23 ± 12.28 | 23.11 ± 4.93 |
| AAVDJ | SS | 90 | 1.82 ± 0.25 | 2.80 ± 0.60 | 9.27 ± 3.32 | 0.97 ± 0.22 |
| AAVDJ8 | SS | 90 | 0.92 ± 0.34 | 0.99 ± 0.48 | 3.94 ± 2.56 | 0.31 ± 0.07 |
| AAV9 | SS | 90 | 0.60 ± 0.08 | 1.28 ± 0.61 | 1.99 ± 0.90 | 1.36 ± 1.07 |
| AAVrh10 | SS | 90 | 0.35 ± 0.05 | 0.40 ± 0.04 | 0.35 ± 0.03 | 0.17 ± 0.02 |
| AAV9 | SC | 91 | 5.52 ± 1.30 | 39.48 ± 12.40 | 197.15 ± 66.93 | 4.64 ± 1.17 |
| Sorbitol formulation (background) | | | 0.04 ± 0.01 | 0.05 ± 0.03 | 0.06 ± 0.02 | 0.04 ± 0.02 |

### Example 6. In Vivo ICV (lateral ventricle) and CM (Cisterna Magna) Injection Study

To compare expression following intracerebroventrical (ICV, lateral ventricle) or intracisternal (Cisterna Magna, CM) injections, more than 18 wild type mice (C57BL/6), 7 weeks old, were administered an AAV as described in the study design in Table 17. The AAV, described in Table 18, was formulated in PBS and 0.001% F-68 and administered via intracerebroventricular (ICV, lateral ventricle) or intracisternal (CM, cisterna magna) injection.

**Table 17. Mouse ICV vs CM Study - Design**

| **AAV Genome** | **Dose (VG)** | **Route** | **n** | **End of Study** |
|---|---|---|---|---|
| SS | 5 x 10¹⁰ | ICV | 6 | Day 28 |
| | | CM | 6 | Day 28 |
| N/A | 0 | ICV | 3 | Day 28 |
| | | CM | 3 | Day 28 |

**Table 18. Mouse ICV vs CM - AAV Construct Sequences**

| **AAV Capsid** | **AAV Genome** | **Genome SEQ ID NO** |
|---|---|---|
| rh10 | SS | 90 |

The mice were given a bolus (5 x 10¹⁰ VG/5 ul total dose at a 1 x 10¹³ VG/mL composition concentration) single unilateral injection (0.5 ul/min) via either intracerebroventricular (ICV) or intracisternal (CM) administration. 22 samples were taken for each administration group after 28 days.

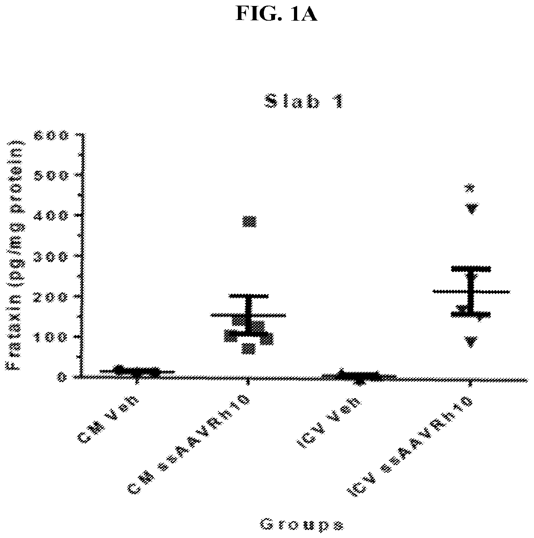

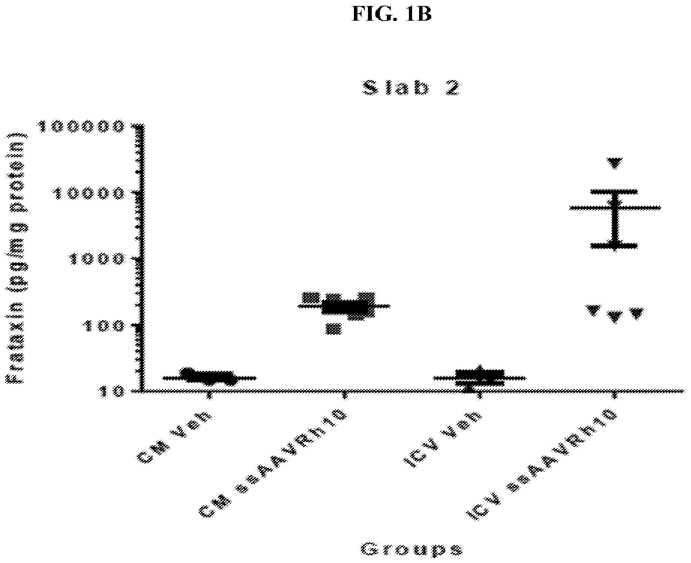

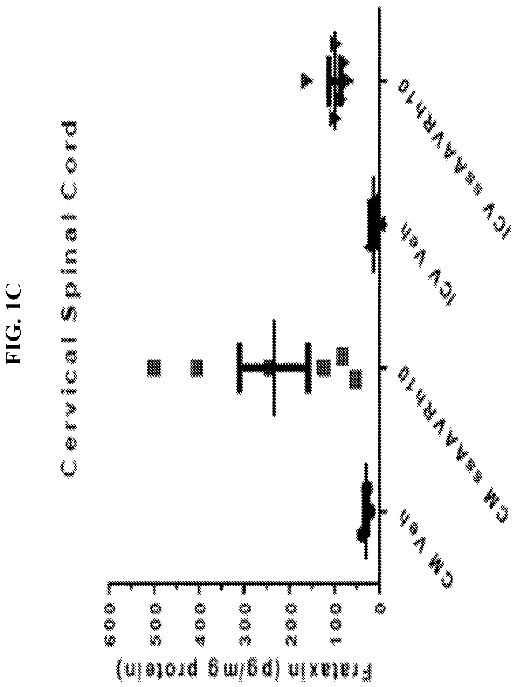

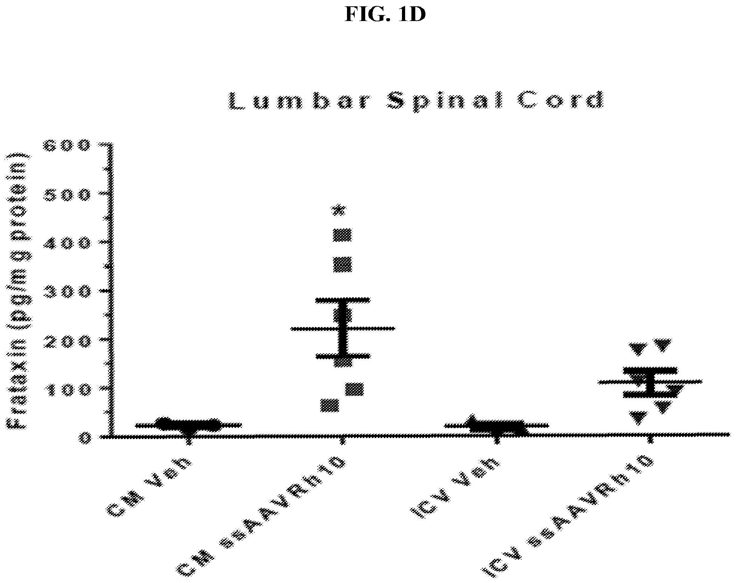

On day 28, human frataxin levels in a 2 mm anterior slice containing mostly frontal cortex (Slab 1), an adjacent 2 mm slice containing striatum (Slab 2), cervical spinal cord and lumbar spinal cord were determined using the SimpleStep human frataxin ELISA assay (Abcam) specific for human frataxin protein, and the average frataxin expression levels (ng/mg protein) are shown in FIG. 1A-1D. Frataxin protein expression was higher in brain slices after ICV administration than after CM administration (FIG. 1A-1B) whereas frataxin protein expression was higher in cervical and lumbar spinal cord after CM administration than after ICV administration (FIG. 1C-1D).

### Example 7. Non-Human Primate Capsid Comparison Study of AAV Delivery via Intrathecal Lumber (IT-L) Administration

### Animal test system

Forty male cynomolgus monkeys (Covance, Inc., Alice, TX) approximately 3 to 5 years old (approximately 3 to 5kg) were used for this study. All the animals were bred for this study only and received no other experimental treatments. The test animals were screened for neutralizing antibody titers to the viral particles that they would later be administered, to confirm seronegativity before inclusion into this study. All the screening results were recorded for each animal and each animal had an AAV antibody titer less than 1:10.

All the test animals were first quarantined for at least four weeks at the study center with daily observations.

All the animals were housed in standard animal cages in compliance with the Guide for the Care and Use of Laboratory Animals, DHHS, (NIH) No. 86-23, and the Animal Welfare Act (9 CFR 3). The animals were also provided with environmental enrichment as per AAALAC (Association for Assessment and Accreditation of Laboratory Animal Care) accredited program.

### Test articles/particles

Eight different AAV test articles (Test particles) encoding human frataxin (FXN) were delivered via intrathecal lumber (IT-L) implanted catheter. AAV2, AAV5, AAV6, AAV9, AAVrh10, AAVDJ and AAVDJ8 serotypes which were packaged with a transgene (FXN) under the CB6 promoter were tested and compared for distribution and level of transgene (FXN) expression in NHP spinal cord and dorsal root ganglia (DRGs). The test particles used in this study are listed in Table 19, wherein the AAV genomes are self-complementary (SC) or single stranded (SS).

**Table 19. NHP Capsid Comparison Study - Test Articles**

| **Test articles** | **Lot No.** | **AAV Capsid** | **Genome** | **Genome SEQ ID NO.** |
|---|---|---|---|---|
| 1 | VCAV-01870 | AAV2 | SS | 90 |
| 2 | VCAV-01871 | AAV5 | SS | 90 |
| 3 | VCAV-01851 | AAV6 | SS | 90 |
| 4 | VCAV-02037 | AAV9 | SS | 90 |
| 5 | VCAV-01842 | AAVrh10 | SS | 90 |
| 6 | VCAV-01858 | AAVDJ | SS | 90 |
| 7 | VCAV-01888 | AAVDJ/8 | SS | 90 |
| 8 | VCAV-01991 | AAVrh10 | SC | 91 |

The test articles comprised preparations of vector with and without sorbitol. The test articles and AAV vehicle (PBS with 0.001% F-68) were stored at the dosing concentration of 1 x 10¹³ vg/mL at -75°C or below; and were thawed at room temperature on the day of administration.

### Surgery and surgical recovery

38 cynomolgus monkeys (including 4 alternates) were surgically implanted with IT-L catheters following the procedures as described below.

For IT-L catheter implantation, an incision was made over the dorsal process of the lumbar spine at L4, L5, or L6. The muscle was dissected and a hemilaminectomy was made for the insertion of a polyethylene lined polyurethane open ended catheter. The catheter was then advanced to the area of the thoraco-lumbar junction. The proximal end of the catheter terminates in a low volume titanium subcutaneous access port (MIN LOVOL, Access Technologies). Proper IT-L sampling catheter placement was verified by the ability to collect CSF at surgery. The skin was closed with sutures and tissue adhesive.

Catheter patency was verified by myelogram with Isovue 300. Dosing was performed 7-14 days after surgery.

### Experimental design

Cynomolgus monkeys (Male 3-5 years of age with an AAV antibody titer of <1:10; including 4 alternates) surgically implanted with IT catheters in the lumbar region (L1) were divided into 9 study groups and each animal was administered three 1.0 ml slow bolus injections of test article dosing solution delivered via IT catheter at 60 minute intervals (i.e. 0 minute, 60 minutes and 120 minutes on day 1 of the study) followed by 0.2 ml of AAV vehicle immediately after the third injection to flush the dose from the catheter system. Study groups are shown in the following Table 20, wherein the AAV genomes are self-complementary (SC) or single stranded (SS).

**Table 20. NHP Capsid Comparison Study - Study Design**

| **Group** | **No. of animals** | **Test articles** | **Genome** | **Dose (vg)** | **Conc.(vg/ml** ) | **Day of Necropsy** |
|---|---|---|---|---|---|---|
| 1 | 2 | AAV2-SEQ ID NO: 90 | SS | 3 x 10¹³ | 1 x 10¹³ | 43 |
| 2 | 4 | AAV5-SEQ ID NO: 90 | SS | 3 x 10¹³ | 1 x 10¹³ | 43 |
| 3 | 4 | AAV6-SEQ ID NO: 90 | SS | 3 x 10¹³ | 1 x 10¹³ | 43 |
| 4 | 4 | AAV9-SEQ ID NO: 90 | SS | 3 x 10¹³ | 1 x 10¹³ | 43 |
| 5 | 4 | AAVrh10-SEQ ID NO: 90 | SS | 3 x 10¹³ | 1 x 10¹³ | 43 |
| 6 | 4 | AAVDJ-SEQ ID NO: 90 | SS | 3 x 10¹³ | 1 x 10¹³ | 43 |
| 7 | 4 | AAVDJ/8-SEQ ID NO: 90 | SS | 3 x 10¹³ | 1 x 10¹³ | 43 |
| 8 | 4 | AAVrh10-SEQ ID NO: 91 | SC | 3 x 10¹³ | 1 x 10¹³ | 43 |
| 9 | 4 | Vehicle | | N/A | N/A | 43 |

During the period of the study, in-life observations and measurements such as body weights, food consumption, clinical observations, and clinical pathology evaluations, and other observations were recorded.

No significant adverse effects were observed during the study as reported in these in-life parameters of each animal.

On Day 43, the animals were sacrificed and selected tissues harvested for bioanalytical evaluation.

### Example 8. ALT and AST analysis of tested animals

Serum samples from animals treated with AAV particles that were collected on Day 1 (immediately prior to dosing), and Day 43 (just prior to necropsy) were analyzed for Alanine Aminotransferase (ALT) and Aspartate Aminotransferase (AST) levels.

In cynomolgus monkeys, the median values and reference intervals for ALT were 33U/L and 15-64U/L; respectively; and the median values and reference intervals for AST were 31U/L and 20-63U/L.

For most test articles, ALT and AST serum levels were not elevated. The AAVrh10-treated animals showed an increase in ALT and AST levels. Additional results from another NHP pilot study also indicated the scAAVrh10-FXN can induce elevation of ALT and AST in the blood. As a group, scAAVrh10 ALT values were 3-folder higher than the upper reference interval.

### Example 9. Transgene (Frataxin) mRNA expression analysis

Frataxin mRNA expression in all the tissues collected at necropsy as described in Example 8 was measured using q-PCR. All samples were run in duplex qRT-PCR reactions for frataxin and control mRNAs (beta-actin and GAPDH). A statistical test (Grubbs test) was used to determine single outliers values in each data set. The fold-change ratios were used to determine single outliers among replicates. For all the samples tested, rates of exclusion for replications were 5.9% of 816 spinal cord values, 4.6% of 544 DRG values, and 1.6% of 272 dentate nucleus and liver values. The absolute and relative quantification for frataxin mRNA expression by qRT-PCR were also determined.

Overall, the 8 AAV serotypes injected intrathecally led to human frataxin expression levels higher than 0.5 fold over the human brain reference control. After 6 weeks, all tested animals showed a rise in neutralizing AAV antibodies of the injected serotype in serum and in CSF.

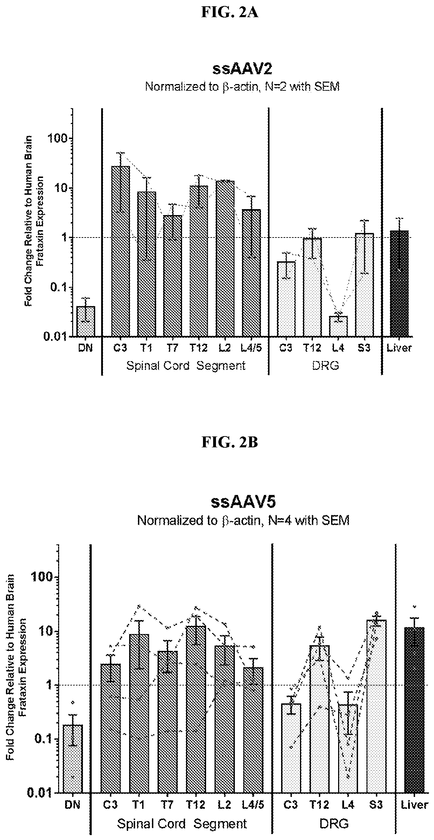

Frataxin mRNA expression levels in the CNS and selected peripheral organs in tested monkeys after IT-L1 bolus dosing of each capsid are shown in FIG. 2A to 2H.

*ssAAV2 serotype:* Frataxin mRNA expression is low in dentate nucleus at 4% human brain level. The expression in spinal cord is 2.8-27.2 fold over human brain level, but no apparent rostro-caudal gradient was observed in the study. AAV2-FXN expression is variable in DRGs with about 0.025 to 1.2 fold over human brain level. In general, the AAV2-FXN expression in DRGs is lower than that in spinal cord. Similarly, the average expression level of AAV2-FXN in liver is the lowest of all AAV capsids tested, normalized to human brain levels (FIG. 2A). As compared to the other serotypes, AAV2 generates the highest average expression level of frataxin in spinal cord (n=2).

*ssAAV5 serotype:* Frataxin mRNA expression is third highest in dentate nucleus at 18% human brain level. The expression in spinal cord is 2.1-12.3 fold over human brain level, but no apparent rostro-caudal gradient was observed in the study. AAV5-FXN expression is variable in DRGs with about 0.43 to 15.9 fold over human brain level. The AAV5-FXN expression in liver roughly matches peak levels in the spinal cord and DRGs (FIG. 2B).

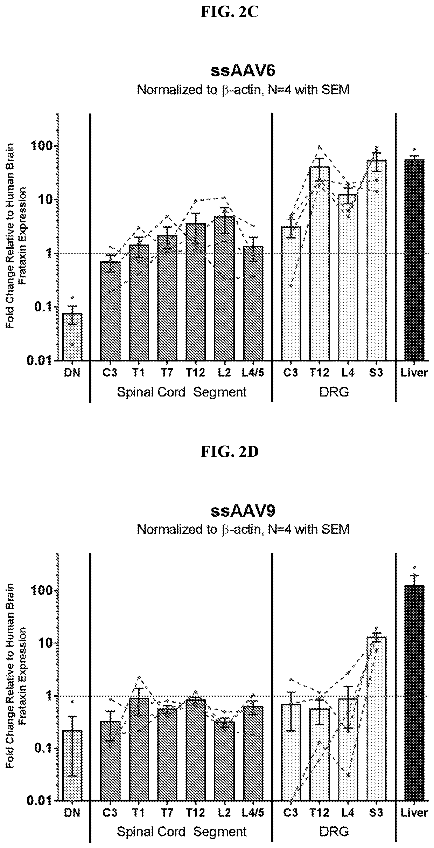

*ssAAV6 serotype*: Frataxin mRNA expression is low in dentate nucleus at 7% human brain level. The expression in spinal cord is 0.69-4.8 fold over human brain level, and a rostro-caudal gradient from C3 to L2 in expression was observed in the study, with levels highest at L2. The highest frataxin mRNA expression level in DRGs was detected for AAV6-FXN compared with all ssAAV treated groups. The AAV6-FXN expression in DRGs ranges from 3.1 to 54.1 fold over human brain level. DRGs show greater AAV6-FXN expression levels than in matching spinal cord segments C3, T12 and L4. Furthermore, the observed expression pattern in DRGs is similar with the matching spinal cord segments. The average expression level of AAV6-FXN in liver is high, 55.9 fold of human brain level, similar to the high levels of frataxin expression in T12 and S3 DRGs (FIG. 2C).

*ssAAV9 serotype:* The highest frataxin mRNA expression level in dentate nucleus was detected for ssAAV9 serotype compared with other ssAAV treated groups at 22% human brain level. The frataxin expression in spinal cord ranges from 0.32 to 0.9 fold over human brain level, and is generally consistent across spinal cord segments. The average ssAAV9-FXN expression in DRGS is similar to that in the spinal cord, but an elevated expression in S3 DRGs (n=4 animals) was detected at 13.1 fold over human brain levels. The expression pattern in DRGs is more variable than that in the spinal cord. ssAAV9 leads to the highest expression in liver of all ssAAV treated groups at 123 fold over human brain level, which exceeds all other samples in the study (FIG. 2D).

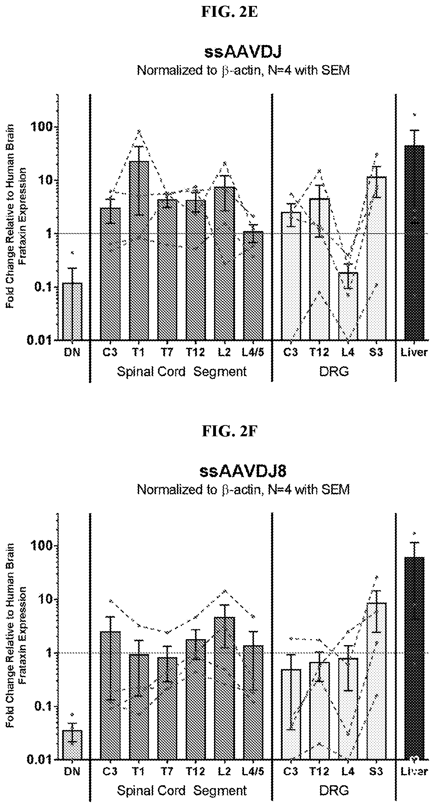

*ssAAVDJ serotype*: Frataxin mRNA expression in dentate nucleus is at 12% human brain level. After ssAAVDJ-FXN administration, frataxin expression peaked at T1 segments in the spinal cord with a range from 1.1 to 22.6 fold over human brain level. The expression in C3 and T12 DRGs is similar to that in the spinal cord, but the expression in L4 DRGs is about 5 fold lower than that in L4/L5 segments in the spinal cord. ssAAVDJ-FXN is expressed in liver higher than all the CNS tissues tested. The expression in liver is about 44.1 fold over human brain level (FIG. 2E).

*ssAAVDJ8 serotype:* Frataxin mRNA expression in dentate nucleus is low at 3% human brain level. The ssAAVDJ8-FXN expression is consistent across the spinal cord ranging from 0.93-4.6 fold over human brain level. The average expression in DRGs is consistent with the matching spinal cord segments, with a range from 0.49-0.78 in segments from C3 to L4 and 8.4 fold in S3. Similar to the ssAAVDJ serotype, ssAAVDJ8 has relatively high expression level in liver compared with other ssAAV treated groups. The expression in liver is about 60.2 fold over human brain level (FIG. 2F).

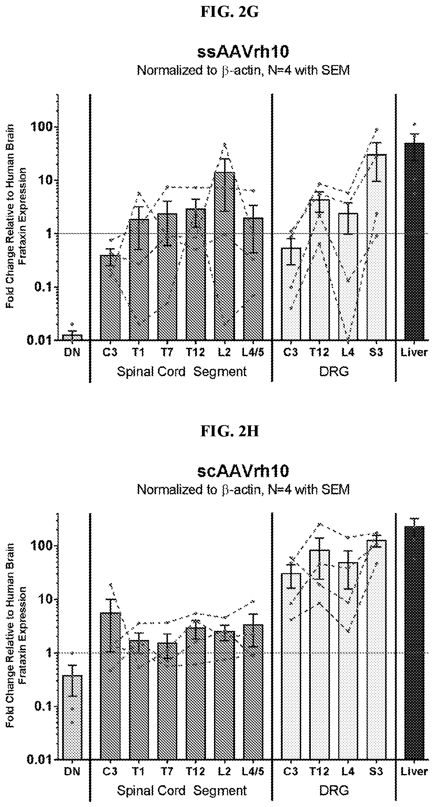

*ssAAVrh10 serotype:* Frataxin mRNA expression in dentate nucleus is very low. The ssAAVrh10-FXN expression in the spinal cord ranges from 0.39-14 fold over human brain level, with rostro-caudal griadent of expression across C3 to L2, with levels highest at L2. The average expression in DRGs is similar to the matching spinal cord segments and elevated in S3 segment to 30.2 fold over human brain level. This result is similar to that in ssAAVDJ8 and ssAAV9 driven transgene expression, indicating these three serotypes ssAAVrh10, ssAAVDJ8 and ssAAV9 have high transduction efficiency to the S3 segment of the spinal cord and matching DRGs. The expression in liver is about 49 fold over human brain level, slightly greater than the highest DRG level (FIG. 2G).

*scAAVrh10 serotype:* Frataxin mRNA expression in dentate nucleus is the highest among the tested capsid at 37% human brain level. The scAAVrh10-FXN expression is consistent across the spinal cord from C3 to L4/5, ranging from 1.5-5.6 fold over human brain level. The expression pattern across the spinal cord is more homogeneous than that of ssAAVrh10 serotype. scAAVrh10 can drive a similar level of expression as compared to the ssAAV treated groups but the level is lower than that of ssAAV2. The frataxin mRNA expression in DRGs is consistently high at all segments ranging from 30.4 -127 fold over human brain level. Similarly, the expression in liver is the highest among all the tested groups at about 229 fold over human brain level, with a highest individual liver expression of 500 fold over human brain level (FIG. 2H).

### Example 10. Comparison of transgene (Frataxin) mRNA expression in NHPs treated with different serotypes

Frataxin mRNA expression levels obtained from different tissue samples from all treated groups were further compared.

The expression levels from ssAAVrh10 and scAAVrh10 were compared. The results indicate that Frataxin expression is greatly improved in scAAVrh10 as compared to ssAAVrh10, while both viral particles show a similar average range of expression throughout the spinal cord. DRG expression in the scAAVrh10 treated animals is driven above the matching spinal cord segment expression and is more uniform across the spinal cord. There is also about 4.5 fold difference in average liver expression between scAAVrh10 and ssAAVrh10 (FIG. 2G and 2H).

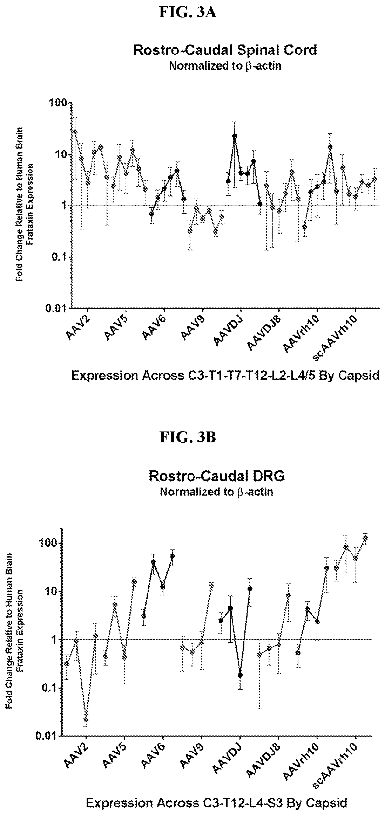

Frataxin expression profiles for each capsid serotypes in spinal cord segments and matching DRGs also show that certain serotypes result in much higher transgene expression than others. As shown in FIG. 3A, capsids, in order of highest average frataxin mRNA expression across the tested spinal cord segments, were ranked: ssAAV2 (11x) > ssAAVDJ (7x) > ssAAV5 (6x) > ssAAVrh10 (4x) > scAAVrh10 (3x) > ssAAV6 (2x) > ssAAVDJ8 (2x) > ssAAV9 (0.6x). As shown in FIG. 3B, capsids, in order of highest average frataxin mRNA expression across the tested dorsal root ganglia (DRG), were ranked: scAAVrh10 (72x) > ssAAV6 (28x) > ssAAVrh10 (9x) > ssAAV5 (6x) > ssAAVDJ (5x) > ssAAV9 (4x) > ssAAVDJ8 (3x) > ssAAV2 (0.6x).

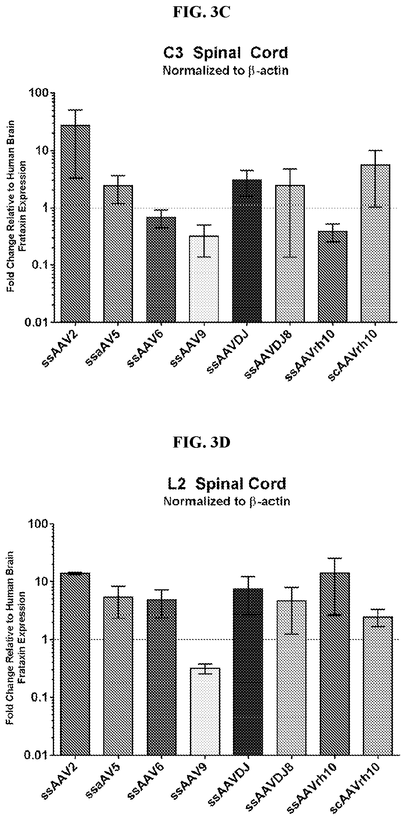

A comparison of frataxin expression by each capsid along the rostro-caudal axis of the spinal cord reveals that most of capsids tested have similar ranges of expression in the L2 segment, one segment caudal to the L1 site for administration, with the exception of ssAAV9 which results in less than 1/10th of the expression with other capsids (FIG. 3D). The C3 segment, the most rostral segment tested, demonstrates pronounced differences in expression across capsids tested and increased animal to animal viability for several AAV serotypes, notably ssAAVDJ8 and ssAAV2 (FIG. 3C). Furthermore, the expression in C3 segment is reduced in ssAAV6 and ssAAVrh10 treated animals, and slightly reduced (or consistent) in ssAAV5, ssAAVDJ and scAAVrh10 treated animals, as compared to the expression in L2 segment. Among the tested capsids, ssAV2 has the highest overall frataxin mRNA level across all tested spinal cord segments (FIG. 3C and FIG. 3D).

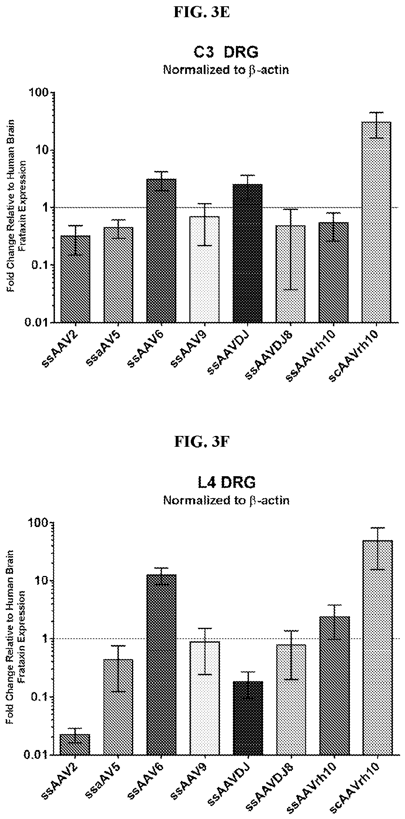

A similar comparison of frataxin expression by capsids in C3 and L4 DRGs reveals that among tested DRGs, C3 and L4 show lower levels of expression in all tested capsids, as compared to the expression levels in T12 and S3 DRGs. The results also indicate that only ssAAV6 and scAAVrh10 treatments result in frataxin mRNA expression above human brain level in both tissues. Consistent levels of expression were detected in ssAAV5 and ssAAV9 DRGs, but a more variable pattern was detected in ssAAVDJ8 DRGs. In ssAAV2 and ssAAVDJ treatments, frataxin mRNA expression is reduced significantly in L4 DRGs as compared to C3 DRGs. In addition, both ssAAVrh10 and scAAVrh10 show a slight increase in frataxin expression in L4 DRGs as compared to C3 DRGs. ssAAV2 is the strongest overall performer in overall spinal cord expression, but the poorest in overall DRG expression (FIG. 3E and FIG. 3F).

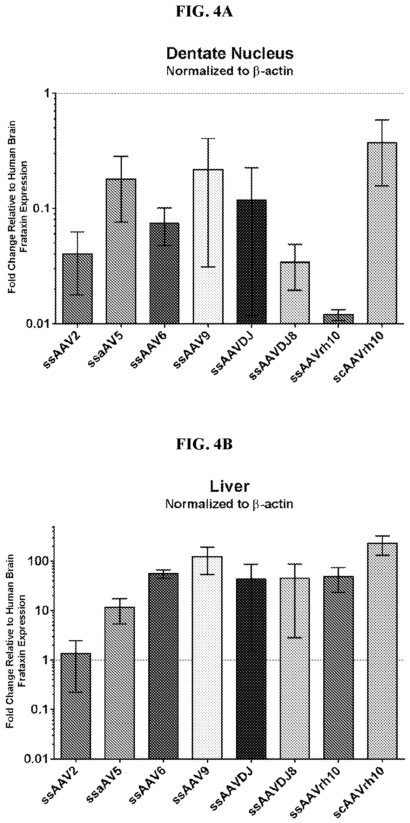

Frataxin mRNA expression in dentate nucleus reflects the ability of a capsid to penetrate into the deeper brain region after intrathecal injection. The results suggest that the expression in dentate nucleus was achieved in the scAAVrh10, ssAAV9 and ssAAV5 treatment groups. ssAAVrh10 consistently resulted in the lowest levels of frataxin expression in the dentate nucleus (FIG. 4A).

Frataxin mRNA expression in liver for all tested capsids was evaluated to determine the extent of extra-CNS transduction. In most animals, frataxin expression in liver is greater than that in human brain (FIG. 4B).

### Example 11. ELISA Assay for Detecting Differential Payload Expression from Regulatory Elements in Various Cell Types

The HEK293 cell line was transfected with AAV constructs, SEQ ID Nos. 13-22, 40, 48, 54-56, 62, 63, 70, 73, 75, 79, 81, 85, 87, 92, and 93 to assay the level of expression of a Frataxin payload sequence under control of various regulatory elements in human embryonic kidney 293 (HEK 293), primary human fibroblast (FA), rat primary dorsal root ganglia (DRG) neurons (rDRG), or human induced pluripotent stem cell (iPSC) derived neural stem cells (hNSC) cell types.

HEK 293 cells were co-transfected in triplicate using FUGENE® HD reagent with each construct (0.5 µg) and the gWiz-GFP plasmid (100ng) as an internal transfection efficiency control. The transfected 293FT cells were harvested 30-36 hours post-transfection, lysed using the THERMO SCIENTIFIC™ PIERCE™ M-PER™ Mammalian Protein Extraction Reagent, and resuspended in 200ul of lysis buffer. Protein concentration in each of the samples was measured using the Thermo Scientific™ Pierce™ BCA™ Protein Assay.

Primary human fibroblast, HIP™ Neural Stem Cells (Human iPSC-Derived), and rat primary dorsal root ganglia (DRG) neurons (rDRG) cells were co-transfected in triplicate using Lipofectamine LTX-Plus reagent with each construct (0.5 µg) and the gWiz-GFP plasmid (100ng) as an internal transfection efficiency control. The transfected 293FT cells were harvested 30-36 hours post-transfection, lysed using the THERMO SCIENTIFIC™ PIERCE™ M-PER™ Mammalian Protein Extraction Reagent, and resuspended in 200ul of lysis buffer. Protein concentration in each of the samples was measured using the THERMO SCIENTIFIC™ PIERCE™ BCA™ Protein Assay.

ELISA assays for detecting expressed FXN and GFP in cell lysates were performed using the Abcam SimpleStep ELISA kit for FXN and GFP. The results in Tables 21-23 represent exogenous Frataxin expression, subtracting endogenous Frataxin, values relative to GFP. Comparison of expression levels between FXN constructs is represented by normalized Frataxin compared to reference Frataxin levels.

The results in Table 21 represent Frataxin expression values from constructs comprising hEF1α, hEF1α (cpg free), CB6, frataxin (FXN), human synapsin (hSYN), SV40, ITR or CBA promoter sequences. Expression in the ITR construct is driven only by the AAV ITR sequence without any additional promoter sequence. The constructs were expressed in human embryonic kidney 293 (HEK 293), primary human fibroblast (FA), rat primary dorsal root ganglia (DRG) neurons (rDRG), or human induced pluripotent stem cell (iPSC) derived neural stem cells (hNSC). Constructs expressing Frataxin driven by a hEFla promoter results in highest FXN expression in primary fibroblast cells. The presence of a CpG island in the hEF1α promoter promotes FXN expression in all cell types tested compared to CpG-free hEF1a promoter.

**Table 21. Effect of Promoter on Driving Frataxin Expression in Various Cell Types In Vitro**

| **Promoter** | **HEK293** | **FA** | **rDRG** | **hNSC** | **SEQ ID NO.** |
|---|---|---|---|---|---|
| hEF1α (CpG free) | 0.12 | 6.36 | 0.89 | 0.37 | 55 |
| hEF1α | 1.33 | 8.67 | 1.26 | 1.12 | 56 |
| CB6 | 3.09 | 1.46 | 1.19 | 1.52 | 40 |
| FXN | 0.06 | 0.00 | 0.02 | 0.14 | 48 |
| hSYN | 0.12 | 0.03 | 0.15 | 0.16 | 54 |
| ITR | 0.03 | 0.04 | 0.02 | | 92 |
| SV40 | 0.16 | 0.44 | 0.30 | 0.33 | 62 |
| CBA | 1.00 | 1.00 | 1.00 | 1.00 | 93 |

The results in Table 22 represent Frataxin expression values from self-complementary AAV (scAAV) constructs comprising frataxin (FXN), PGK, CBA, or CMV promoter sequences expressed in primary human fibroblast (FA) cells.

The presence of a CMV promoter had the greatest effect of enhancing the expression level of Frataxin in primary fibroblast cells.

**Table 22. Effect of Promoter on Driving Frataxin Expression in Primary Human Fibroblast Cells In Vitro**

| **Frataxin Sequence** | **Promoter** | **Relative Expression** | **SEQ ID NO.** |
|---|---|---|---|
| FXN | FXN | 0.05 | 79 |
| FXN | PGK | 0.05 | 85 |
| FXN | CBA | 0.14 | 63 |
| FXN | CMV | 0.49 | 73 |
| Codon Optimized FXN | FXN | 0.06 | 81 |
| Codon Optimized FXN | PGK | 0.03 | 87 |
| Codon Optimized FXN | CBA | 0.44 | 70 |
| Codon Optimized FXN | CMV | 1.06 | 75 |

The results in Table 23 represent Frataxin expression values from constructs comprising codon optimized frataxin sequences compared to a construct comprising a wild-type frataxin sequence. The constructs were expressed in human embryonic kidney 293 (HEK 293), primary human fibroblast (FA), rat primary dorsal root ganglia (DRG) neurons (rDRG), or human induced pluripotent stem cell (iPSC) derived neural stem cell (hNSC) cell types. The constructs encoding a codon-optimized FXN were observed to have higher levels of expression than native FXN in all cell types tested.

**Table 23. Effect of Codon Optimization on Relative Frataxin Expression from pAAVsc-Promoter-SV40 Constructs in Various Cell Types**

| **Relative Expression** | **HEK293** | **hFibroblast** | **rDRG** | **hNSC** | **SEQ ID NO.** |
|---|---|---|---|---|---|
| Opti-1 | 2.51 | 2.37 | 1.72 | 1.67 | 13 |
| Opti-2 | 3.30 | 2.58 | 2.65 | 2.71 | 14 |
| Opti-3 | 3.24 | 1.34 | 1.55 | N/A | 15 |
| Opti-4 | 2.67 | 1.64 | 2.02 | 1.20 | 16 |
| Opti-5 | 3.04 | 1.69 | 1.96 | N/A | 17 |
| Opti-6 | 3.63 | 1.65 | 1.70 | N/A | 18 |
| Opti-7 | 3.25 | 2.15 | 2.12 | 2.43 | 19 |
| Opti-8 | 2.60 | 1.93 | 2.42 | 3.99 | 20 |
| Opti-9 | 2.05 | 1.76 | 1.56 | N/A | 21 |
| Opti-10 | 2.69 | 3.04 | 2.39 | 2.60 | 22 |
| FXN | 1.00 | 1.00 | 1.00 | 1.00 | 93 |

### Example 12. Non-Human Primate Delivery Study

Non-human primates (NHPs) (n=4 Cynomolgus and n=4 Rhesus) were administered an AAV particle, serotype rh.10, containing one of the DNA constructs described in Table 24, formulated in phosphate buffered saline (PBS) with 0.001% F-68 (pluronic acid), via bolus or continuous intrathecal (IT) delivery using implanted catheters at cervical, thoracic and/or lumbar levels.

**Table 24. Delivery Study in Non-Human Primates - AAV Genome Sequences**

| **Promoter** | **Promoter Position** | **AAV Genome** | **SEQ ID NO** |
|---|---|---|---|
| CBA (AKA CB6) | 535-794 | Self-Complementary | 91 |
| CB6 | 602-861 | Single-Stranded | 93 |
| CB6 | 472-736 | Self-Complementary | 94 |

The study design is shown in Table 25. Groups A and D were administered sequential 1 hour infusions at C1, T1 and L1 via a syringe pump (NE-300) at 1.5 ml/hour. Group B was administered a 10 hour infusion at L1 via an external CADD-Micro pump at 1.0 ml/hour and the NHPs were ambulatory wearing a jacket containing the pump. Group C was administered 3 bolus injections at L1 with 1 hour inter-dose intervals.

For Groups A, C and D, the NHPs were in a restrained prone position during administration. For Group D, the NHPs were returned to their cage between injections.

After the administration of the AAV construct, 200- 500 ul of excipient (PBS with 0.001% F-68) was used to flush the catheter.

**Table 25. Delivery Study in Non-Human Primates - Study Design**

| **Group** | **Capsid** | **AAV Genome** | **Payload** | **SEQ ID NO** | **Site(s)** | **Rate** | **Vol.** | **Conc. (vg/ml)** | **Dose (vg)** | **N Species** |
|---|---|---|---|---|---|---|---|---|---|---|
| A | AAV rh10 | SC | FXN | 91 | C1, T1, L1 | 1.5 ml/h | 3 ml | 1 x 10¹³ | 3 x 10¹³ | 2 Rhesus |
| B | AAV rh10 | SC | FXN | 91 | L1 | 1.0 ml/h | 10 ml | 0.3 x 10¹³ | 3 x 10¹³ | 2 Cynomolgus |
| C | AAV rh10 | SS | FXN | 93 | L1 | 3x Bolus | 3 ml | 1 x 10¹³ | 3 x 10¹³ | 2 Cynomolgus |
| D | AAV rh10 | SC | GFP | 94 | C1, T1, L1 | 1.5 ml/h | 3 ml | 1 x 10¹³ | 3 x 10¹³ | 2 Rhesus |

The NHPs were observed twice daily and the weights were taken weekly. Clinical pathology was done on Days 1, 3 and 29, serum antibodies were collected at day 1 and at day 29, CSF was collected prior to the study and at Day 0, 3 and 29. Three (3) weeks (for Group D) or 4 weeks (for Groups A, B and C) after AAV administration a saline perfusion was performed and the brain, spinal cord, dorsal root ganglia (DRGs), liver, spleen, heart, kidney, pancreas, ovaries and the sural nerve were collected as well as a skin biopsy.

Spinal cord cross sections were taken at cervical, thoracic and lumbar levels, and the segments were sliced into 2-3 mm thick cross sections. DRGs were collected at cervical, thoracic, lumbar and sacral levels, and the nerve roots were trimmed away prior to processing. Two mm diameter tissue punches from 3 mm thick coronal brain slices were taken from the pre-frontal cortex, motor cortex, putamen, dentate nucleus and cerebellar cortex. The level of frataxin mRNA was measured using a TaqMan two-steps real time quantitative PCR (RT-qPCR) assay. RNA extraction was performed on the tissue samples using Rneasy Plus Universal kit (Qiagen 73404). 1 (One) µg of total RNA was then reverse transcribed into single-stranded cDNA using High-Capacity cDNA Reverse Transcription Kit (Life Technology 4368813). The TaqMan gene expression assay specific for human frataxin (Hs00175940_ml) was carried in combination with either GAPDH (Rh02621745_g1), or Actin (Hs01060665_g1) in a 20 ul reaction volume.

Assays were performed in duplicate by two experimenters using a Lightcycler 480 II instrument (Roche). The amount of human frataxin mRNA for each sample was normalized to GAPDH or beta-Actin and further normalized relative to a control human brain sample. Each plate contained a No Template Control, a No Reverse Transcriptase control, a serially diluted human RNA and a serially diluted plasmid construct containing human frataxin. Amplification efficiency was determined for target and reference genes.

The relative quantity of human frataxin mRNA was averaged from four replicates and calculated according to the delta-delta Ct method (Livak and Schmittgen, 2001). The same procedure was performed to quantify eGFP mRNA using a TaqMan gene expression assay specific for eGFP (Mr04329676_mr). For eGFP, normalized Ct ratios were compared. Results for the 2 NHPs in Group A are shown in Table 26.

In Table 26, "BD" indicates that the detection of frataxin is below the lower limit of quantification of the assay. In Table 26, samples are identified using vertebral segmentation annotation.

**Table 26. Delivery Study in Non-Human Primates, Group A - Frataxin expression after sequential 1 hour IT infusions at C1, T1 and L1**

| **Sample** | | **Fold Change Relative to Human Brain Frataxin Expression** | |
|---|---|---|---|
| | | **NHP A1** | **NHP A2** |
| **Brain** | Prefrontal Cortex | 4.79 | 0.05 |
| | Motor Cortex | 3.71 | BD |
| | Putamen | BD | BD |
| | Dentate Nucleus | BD | BD |
| | Cerebellar Cortex | 0.85 | 1.34 |
| **Spinal Cord** | C1 | 3.39 | 5.71 |
| | C3 | 3.89 | 1.91 |
| | C5 | 1.91 | 0.95 |
| | C7 | 5.44 | 0.42 |
| | T2 | 5.75 | 0.77 |
| | T4 | 7.23 | 0.92 |
| | T7 | 7.81 | 0.80 |
| | T8 | 25.39 | 2.49 |
| | T10 | 18.19 | 0.54 |
| | T12 | 21.75 | 1.91 |
| | L2 | 60.07 | 17.78 |
| **DRG** | C3 | 109 | 1.89 |
| | C5 | 96.67 | 1.50 |
| | T4 | 87.16 | 0.74 |
| | T7 | 15.22 | 1.23 |
| | L2 | 3.86 | 17.67 |
| | L6 | 121.10 | 13.04 |
| | S3 | 23.45 | 23.64 |
| **Liver** | | 156.90 | 256.20 |
| **Spleen** | | 24.72 | 4.80 |

Frataxin expression was achieved in the pre-fontal, motor and cerebellar cortices but not the dentate nucleus. The 3-site IT infusion at C1, T1 and L1 resulted in a 31- to 42-fold spread of expression across the spinal cord. The DRGs generally showed expression greater than or equivalent to the spinal cord (with a 31- to 32-fold spread) and there was significant expression detected in the liver and spleen.

NHP A1 had a gradient of expression with the highest RNA levels in the lumbar region that were ∼30X level in human brain. Expression in the cervical region was 2-3-fold normal.

NHP A2, while showing lower expression than NHP A1, again had the highest expression (∼10-fold) in the lumbar region. The thoracic cord had the lowest level of expression, which was up to 2-fold normal.

DRG expression was similar to the spinal cord with much higher levels in NHP A1 than NHP A2. FXN expression was 10-100 fold higher than human brain levels in this f animal. NHP A2 had lower FXN expression with cervical and thoracic DRGs at levels comparable to the human brain, however the lumbar and sacral DRGs had higher levels, suggesting a preferential transduction of these lower DRGs despite infusions at multiple levels along the cord.

Results for the 2 NHPs in Group B are shown in Table 27.

In Table 27, "BD" indicates that the detection of frataxin is below the lower limit of quantification of the assay. In Table 27, samples are identified using vertebral segmentation annotation.

**Table 27. Delivery Study in Non-Human Primates, Group B - Frataxin expression after 10 hour IT infusion at L1**

| **Sample** | | **Fold Change Relative to Human Brain Frataxin Expression** | |
|---|---|---|---|
| | | **NHP B1** | **NHP B2** |
| **Brain** | Prefrontal Cortex | 0.33 | BD |
| | Motor Cortex | 0.92 | BD |
| | Putamen | BD | BD |
| | Dentate Nucleus | 0.10 | 0.08 |
| | Cerebellar Cortex | 0.95 | 1.56 |
| **Spinal Cord** | C1 | 2.26 | 0.27 |
| | C3 | 2.43 | 1.06 |
| | C5 | 3.00 | 0.95 |
| | C7 | 5.24 | 2.20 |
| | T2 | 6.46 | 1.21 |
| | T4 | 10.31 | 7.47 |
| | T7 | 11.99 | 0.90 |
| | T8 | 15.85 | 3.61 |
| | T10 | 20.31 | 1.42 |
| | T12 | 12.23 | 2.82 |
| | L2 | 10.04 | 2.82 |
| **DRG** | C3 | 0.62 | 2.03 |
| | C5 | 1.00 | 2.82 |
| | T4 | 67.28 | 24.71 |
| | T7 | 0.73 | 1.13 |
| | L2 | 4.38 | 5.19 |
| | L6 | 0.46 | 19.51 |
| | S3 | 42.80 | 28.77 |
| **Liver** | | 416.70 | 729.00 |
| **Spleen** | | 5.51 | 5.00 |

Frataxin expression was achieved in the pre-fontal, motor and cerebellar cortices with significant expression in the dentate nucleus. The 10 hour IT infusion at L1 resulted in a 9 to 28-fold spread of expression across the spinal cord with higher levels at thoracic and lumbar levels.

NHP B1 showed gradient of expression with highest levels (10-20 fold human brain) in the lower thoracic region rostral to the site of infusion and lower levels (∼2 fold normal) in the cervical spinal cord. NHP B2 also had a gradient of FXN expression with higher levels in the lower thoracic region and less FXN in the cervical spinal cord.

The DRGs generally showed expression greater than or equivalent to the spinal cord (with a 25- to 146-fold spread) and significant expression was detected in the liver and spleen.

Results for the 2 NHPs in Group C are shown in Table 28.

In Table 28, "BD" indicates that the detection of frataxin is below the lower limit of quantification of the assay. In Table 28, samples are identified using vertebral segmentation annotation.

**Table 28. Delivery Study in Non-Human Primates, Group C - Frataxin expression after IT triple bolus injection at L1**

| **Sample** | | **Fold Change Relative to Human Brain Frataxin Expression** | |
|---|---|---|---|
| | | **NHP C1** | **NHP C2** |
| **Brain** | Prefrontal Cortex | BD | BD |
| | Motor Cortex | BD | BD |
| | Putamen | BD | BD |
| | Dentate Nucleus | 0.04 | BD |
| | Cerebellar Cortex | 0.13 | 0.11 |
| **Spinal Cord** | C1 | 0.78 | 0.12 |
| | C3 | 0.57 | 0.30 |
| | C5 | 0.47 | 5.51 |
| | C7 | 13.32 | 0.62 |
| | T2 | 0.71 | 0.91 |
| | T4 | 1.79 | 2.22 |
| | T7 | 2.12 | 1.11 |
| | T8 | 3.32 | 1.28 |
| | T10 | 2.38 | 1.46 |
| | T12 | 2.87 | 2.51 |
| | L2 | 4.05 | 2.66 |
| **DRG** | C3 | 0.15 | 0.05 |
| | C5 | 2.87 | BD |
| | T4 | 0.16 | 0.10 |
| | T7 | BD | 0.07 |
| | L2 | 0.25 | 0.21 |
| | L6 | 40.71 | 17.20 |
| | S3 | 0.31 | 4.09 |
| **Liver** | | 1.19 | 4.53 |
| **Spleen** | | 0.5 | 0.35 |

Comparison of prolonged and bolus delivery approaches demonstrated that prolonged infusion yielded more homogenous levels of protein expression across the spinal cord vs. bolus dosing at one or multiple levels. There was less variability with prolonged infusion across the spinal cord. Such prolonged infusion showed no more than 2-fold variability whereas bolus infusion exhibited over 4-fold differences across the spinal cord. For the prolonged infusion, the highest spinal cord expression was observed just above (rostral) to the tip of the infusion catheter. The DRGs generally had lower expression than with multi-site infusion. In the multi-site infusion, a caudal-rostral gradient in the spinal cord was observed, despite cervical, thoracic and lumbar infusion sites. DRG expression was very high across all levels. With the lumbar bolus, a caudal-to-rostral gradient of expression in the spinal cord with variable expression in DRGs was observed.

Low levels of frataxin expression were seen in the cerebellar cortex with very low or no expression seen in the pre-frontal and motor cortices, putamen and dentate nucleus. The triple IT bolus at L1 yielded a rostral-caudal expression pattern with the highest levels seen at the lower thoracic and lumbar levels and spikes in the lower cervical region with a 28- to 46-fold spread.

The DRG expression showed greater than 100-fold spread with no detectable expression in isolated cervical/thoracic DRGs. Moderate levels of expression were seen in the liver and spleen.

As shown in Table 29, the high expresser in Group D, the GFP control, showed a transgene (GFP) mRNA expression pattern across the spinal cord that was similar to the transgene (human frataxin) mRNA expression pattern in the high expresser in Group A (NHP A1). In Table 29, samples are identified using vertebral segmentation annotation.

**Table 29. Delivery Study in Non-Human Primates - High Expressers in Group A and Group D after Sequential 1 hour IT infusions at C1, T1 and L1**

| | **Fold Transgene Expression Relative to beta-actin** | |
|---|---|---|
| **Spinal Cord Segment** | **NHP A1 (Group A)** | **NHP D1 (Group D)** |
| C1 | 0.03 | 0.05 |
| C5 | 0.02 | 0.08 |
| C7 | 0.05 | 0.13 |
| T2 | 0.05 | 0.14 |
| T7 | 0.07 | 0.10 |
| T10 | 0.16 | 0.15 |
| T12 | 0.19 | 0.15 |

### Example 13. Comparison of human FXN expression following intrastriatal delivery in mice of AAV constructs containing three different promoters.

To compare human FXN expression driven by PGK, CMV and CBA and FXN promoters, more than 93 wild type mice (C57BL/6), 6-8 weeks old, were administered an AAV with dose levels shown in Tables 30-32. The AAVs were formulated in PBS and 0.001% F-68, and 5 uL administered via intrastriatal (IS) injection. Human FXN (hFXN) protein levels in striatum were quantified after 7 days (Tables 30 and 31) or 28 days (Table 32) by ELISA with an assay (Abcam) specific for human FXN (no detection of mouse FXN).

Seven days after AAV intrastriatal administration (5E9 VG), all AAV constructs resulted in human frataxin expression. The CBA promoter drove the highest expression, followed by PGK and CMV promoters. The same rank order of promoter-driven expression (CBA > PGK > CMV) was observed with constructs expressing wild-type human frataxin (Table 30) and codon optimized human frataxin (Table 31) at 7 days post-administration. With the CBA promoter, wild-type frataxin (scAAVrh10-CBA-FXN) and codon-optimized frataxin (scAAVrh10-CBA-Opti10FXN) resulted in similar levels of human frataxin protein in the striatum at this time point (Table 31).

**Table 30. Striatum Levels of human FXN at 7 days Following Intrastriatal Injection of Wild-Type Frataxin Constructs**

| **Test Article** | **AAV Genome** | **Dose (VG)** | **Inj. Site AVG ± SEM** |
|---|---|---|---|
| scAA Vrh10-CBA-FXN | SC | 5 x 10⁹ | 120.1 ± 20.18 |
| scAA Vrh10-CMV-FXN | SC | 5 x 10⁹ | 31.20 ± 12.18 |
| scAA Vrh10-PGK-FXN | SC | 5 x 10⁹ | 55.49 ± 4.69 |
| Vehicle | - | - | 2.35 ± 1.33 |

**Table 31. Striatum Levels of human FXN at 7 days Following Intrastriatal Injection of Codon-Optimized Frataxin (Opti10FXN) Constructs**

| **Test Article** | **AAV Genome** | **Dose (VG)** | **Inj. Site AVG ± SEM** |
|---|---|---|---|
| scAA Vrh10-CBA-Opti10FXN | SC | 5 x 10⁹ | 64.12 ± 15.45 |
| scAA Vrh10-CMV-Opti10FXN | SC | 5 x 10⁹ | 18.85 ± 3.93 |
| scAA Vrh10-PGK-Opti10FXN | SC | 5 x 10⁹ | 29.96 ± 6.16 |
| scAA Vrh10-CBA-FXN | SC | 5 x 10⁹ | 61.89 ± 3.77 |
| Vehicle | - | - | 2.00 ± 0.09 |

Twenty-eight days after AAV intrastriatal administration (5E8, 5E9 or 5E10 VG), all AAV constructs resulted in human frataxin expression in the striatum (Table 32). For all 3 promoters (CBA, CMV, PGK), each log increase in dose resulted in an approximately 6-8 fold increase in human frataxin protein levels in the striatum. The CBA promoter drives the highest level of expression in the striatum, followed by the CMV and PGK promoters. The rank order of promoter-driven expression (CBA > CMV > PGK) was observed at 28 days post-administration, with the CBA promoter resulting in approximately 3-fold higher levels of human FXN protein expression than the CMV promoter, and the CMV promoter resulting in approximately 3-fold higher levels of human FXN protein expression than the PGK promoter, across the dose levels used.

**Table 32. Striatum Levels of human FXN at 28 days Following Intrastriatal Injection of Wild-Type and Codon-Optimized Frataxin Constructs**

| **Test Article** | **AAV Genome** | **Dose (VG)** | **Inj. Site AVG ± SEM** |
|---|---|---|---|
| scAA Vrh10-CBA-FXN | SC | 5 x 10⁸ | 113.51 ± 10.32 |
| scAA Vrh10-CBA-FXN | SC | 5 x 10⁹ | 748.53 ± 120.54 |
| scAA Vrh10-CBA-FXN | SC | 5 x 10¹⁰ | 4915.25 ± 896.59 |
| scAA Vrh10-CMV-FXN | SC | 5 x 10⁸ | 33.37 ± 4.91 |
| scAA Vrh10-CMV-FXN | SC | 5 x 10⁹ | 260.67 ± 12.61 |
| scAA Vrh10-CMV-FXN | SC | 5 x 10¹⁰ | 1687.10 ± 278.23 |
| scAA Vrh10-PGK-FXN | SC | 5 x 10⁸ | 12.49 ± 1.02 |
| scAA Vrh10-PGK-FXN | SC | 5 x 10⁹ | 79.93 ± 1.60 |
| scAA Vrh10-PGK-FXN | SC | 5 x 10¹⁰ | 515.81 ± 29.32 |
| scAAVrh10-CBA-Opti10FXN | SC | 5 x 10⁹ | 777.94 ± 176.08 |
| Vehicle | | | 5.47 ± 2.76 |

While the present invention has been described at some length and with some particularity with respect to the several described embodiments, it is not intended that it should be limited to any such particulars or embodiments or any particular embodiment, but it is to be construed with references to the appended claims so as to provide the broadest possible interpretation of such claims in view of the prior art and, therefore, to effectively encompass the intended scope of the invention.

All publications, patent applications, patents, and other references mentioned herein are incorporated by reference in their entirety. In case of conflict, the present specification, including definitions, will control. In addition, section headings, the materials, methods, and examples are illustrative only and not intended to be limiting.

The present disclosure also includes the following items:
1. A polynucleotide encoding human frataxin comprising a sequence selected from the group consisting of SEQ ID NOs 1-94.
2. The polynucleotide of item 1, wherein the polynucleotide comprises a 5' untranslated region (UTR), poly A signal and 3' UTR.
3. The polynucleotide of item 2, wherein the polynucleotide comprises at least one 5' inverted terminal repeat (ITR) and one 3' inverted terminal repeat ITR.
4. The polynucleotide of any one of items 1-3, wherein the open reading frame encoding human frataxin is codon optimized.
5. The polynucleotide of any one of items 1-3, wherein the polynucleotide comprises a promoter region.
6. The polynucleotide of item 5, wherein the polynucleotide comprises an enhancer, wherein the enhancer is a CMV enhancer.
7. A polynucleotide encoding human frataxin comprising a sequence having at least 95% identity to a sequence selected from the group consisting of SEQ ID NOs 1-94 or variants thereof.
8. A polynucleotide encoding human frataxin comprising a sequence having at least 99% identity to a sequence selected from the group consisting of SEQ ID NOs 1-94 or variants thereof.
9. A polynucleotide comprising a sequence selected from the group consisting of SEQ ID NOs 1-94, wherein the frataxin payload is replaced by gene encoding a CNS protein of interest or a modulatory nucleic acid.
10. A polynucleotide having at least 95% identity to a polynucleotide sequence of item 9.
11. A polynucleotide having at least 99% identity to a polynucleotide sequence of item 9.
12. An AAV particle comprising the polynucleotide of any of items 1-11 packaged in a capsid, said capsid having a serotype selected from the group consisting of AAV1, AAV2, AAV3, AAV4, AAV5, AAV6, AAV7, AAV8, AAV9, AAV9.47, AAV9(hu14), AAV10, AAV11, AAV12, AAVrh8, AAVrh10, AAV-DJ and AAVDJ8.
13. The AAV particle of item 12, wherein the capsid serotype is AAVrh10.
14. The AAV particle of item 12, wherein the capsid serotype is AAV9 (hu14).
15. The AAV particle of item 12, wherein the capsid serotype is AAV DJ.
16. The AAV particle of item 12, wherein the capsid serotype is AAV9.47.
17. The AAV particle of item 12, wherein the capsid serotype is AAVDJ8.
18. A method of treating a disease or disorder in a subject in need thereof comprising administering to said subject a therapeutically effective amount of a polynucleotide comprising a sequence selected from the group consisting of SEQ ID NOs 1-94.
19. The method of item 18, wherein the polynucleotide in packaged in an AAV particle, said AAV particle comprising a capsid having a capsid serotype selected from the group consisting of AAV1, AAV2, AAV3, AAV4, AAV5, AAV6, AAV7, AAV8, AAV9, AAV9.47, AAV9(hu14), AAV10, AAV11, AAV12, AAVrh8, AAVrh10, AAV-DJ and AAVDJ8.
20. The method of item 19, wherein the capsid serotype is AAVrh10.
21. The method of item 19, wherein the capsid serotype is AAV9 (hu14).
22. The method of item 19, wherein the capsid serotype is AAV-DJ.
23. The method of item 19, wherein the capsid serotype is AAV9.47.
24. The method of item 19, wherein the capsid serotype is AAVDJ8.
25. The method of any one of items 18-24, wherein the disease or disorder is Friedreich's Ataxia.
26. A method of increasing the level of a protein in the CNS of a subject in need thereof comprising administering to said subject via prolonged infusion an effective amount of an AAV particle comprising a viral genome encoding the polynucleotide of items 1-6 or a therapeutically effective amount of a polynucleotide comprising a sequence selected from the group consisting of SEQ ID NOs 1-94 wherein the frataxin payload has been replaced by gene encoding a CNS protein of interest or a modulatory nucleic acid.
27. The method of item 26, wherein the prolonged infusion is by intracerebroventricular (ICV) administration.
28. The method of item 26, wherein the prolonged infusion is by injection into the ventricular system of the brain.
29. The method of item 28, wherein the injection to the ventricular system occurs in at least one location selected from the group consisting of the right lateral ventricle, left lateral ventricle, third ventricle, and fourth ventricle.
30. The method of item 28, wherein the injection into the ventricular system occurs in at least one location selected from the group consisting of interventricular foramina (also called the foramina of Monro), cerebral aqueduct, and central canal.
31. The method of item 28, wherein the injection into the ventricular system occurs in at least one aperture of the ventricular system selected from the group consisting of the median aperture, right lateral aperture, and left lateral aperture.
32. The method of item 28, wherein the injection into the ventricular system occurs in the perivascular space in the brain.
33. The method of item 26, wherein the prolonged infusion is by intrathecal (IT) administration.
34. The method of item 33, wherein IT administration occurs by injection into the subarachnoid space.
35. The method of item 34, wherein injection into the subarachnoid space occurs in at least one location selected from the group consisting of the cervical, thoracic, lumbar and sacral regions of the spine.
36. The method of item 35, wherein the injection occurs in the cervical region, and
   wherein the injection to the cervical region occurs in at least one location selected from the group consisting of C1, C2, C3, C4, C5, C6, and C7.
37. The method of item 35, wherein the injection occurs in the thoracic region, and
   wherein the injection to the thoracic region occurs in at least one location selected from the group consisting of T1, T2, T3, T3, T4, T5, T6, T7, T8, T9, T10, T11, and T12.
38. The method of item 35, wherein the injection occurs in the lumbar region, and
   wherein the injection to the lumbar region occurs in at least one location selected from the group consisting of L1, L2, L3, L4, and L5.
39. The method of item 35, wherein the injection occurs in the lumbar region, and
   wherein the injection to the lumbar region occurs in at least one location selected from the group consisting of S1, S2, S3, S4 and S5.
40. The method of item 39 comprising at least three locations of prolonged infusion.
41. The method of item 40, wherein the prolonged infusion occurs at three locations and wherein the three locations of prolonged infusion are at C1, T1, and L1.
42. The method of item 27 or 33 wherein the prolonged infusion occurs at a volume of more than 1 mL.
43. The method of item 27 or 33 comprising administration of less than 1 mL.
44. The method of item 27 or 33 comprising administration of between about 0.1 mL to about 120 mL.
45. The method of item 42 wherein the prolonged infusion occurs at a volume of at least 3 mL.
46. The method of item 42 wherein the prolonged infusion occurs at a volume of 3 mL.
47. The method of item 42 wherein the prolonged infusion occurs at a volume of at least 10 mL.
48. The method of item 42 wherein the prolonged infusion occurs at a volume of 10 mL.
49. The method of item 27 or 33 wherein the prolonged infusion occurs for at least a duration selection from the group consisting of 0.17, 0.33, 0.5, 0.67, 0.83, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, and 36 hour(s).
50. The method of item 49 wherein the prolonged infusion occurs for a duration of at least 30 minutes.
51. The method of item 49 wherein the prolonged infusion occurs for a duration of at least one hour.
52. The method of item 49 wherein the prolonged infusion occurs for a duration of at least 10 hours.
53. The method of item 27 or 33 wherein the prolonged infusion comprises at least one dose of AAV comprising at least one polynucleotide encoding a payload.
54. The method of item 27 or 33, wherein the prolonged infusion occurs at a constant rate.
55. The method of item 27 or 33, wherein the prolonged infusion occurs at a ramped rate.
56. The method of item 55, wherein the ramped rate increases over the duration of the prolonged infusion.
57. The method of item 27 or 33, wherein the prolonged infusion occurs at a complex rate alternating between high and low rates over the duration of the prolonged infusion.
58. The method of any one of items 54-57, wherein the rate of prolonged infusion is between about 0.1 mL/hour and about 25.0 mL/hour.
59. The method of item 58, wherein the comprising a rate of prolonged infusion is selected from the group consisting of 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, 12.0, 12.1, 12.2, 12.3, 12.4, 12.5, 12.6, 12.7, 12.8, 12.9, 13.0, 13.1, 13.2, 13.3, 13.4, 13.5, 13.6, 13.7, 13.8, 13.9, 14.0, 14.1, 14.2, 14.3, 14.4, 14.5, 14.6, 14.7, 14.8, 14.9, 15.0, 15.1, 15.2, 15.3, 15.4, 15.5, 15.6, 15.7, 15.8, 15.9, 16.0, 16.1, 16.2, 16.3, 16.4, 16.5, 16.6, 16.7, 16.8, 16.9, 17.0, 17.1, 17.2, 17.3, 17.4, 17.5, 17.6, 17.7, 17.8, 17.9, 18.0, 18.1, 18.2, 18.3, 18.4, 18.5, 18.6, 18.7, 18.8, 18.9, 19.0, 19.1, 19.2, 19.3, 19.4, 19.5, 19.6, 19.7, 19.8, 19.9, 20.0, 20.1, 20.2, 20.3, 20.4, 20.5, 20.6, 20.7, 20.8, 20.9, 21.0, 21.1, 21.2, 21.3, 21.4, 21.5, 21.6, 21.7, 21.8, 21.9, 22.0, 22.1, 22.2, 22.3, 22.4, 22.5, 22.6, 22.7, 22.8, 22.9, 23.0, 23.1, 23.2, 23.3, 23.4, 23.5, 23.6, 23.7, 23.8, 23.9, 24.0, 24.1, 24.2, 24.3, 24.4, 24.5, 24.6, 24.7, 24.8, 24.9, and 25.0 mL/hour.
60. The method of item 59, wherein the rate of prolonged infusion is 1.0 mL/hour.
61. The method of item 59, wherein the rate of prolonged infusion is 1.5 mL/hour.
62. The method of any one of items 54-57, wherein the administration comprises a total dose between about 1x10⁶ VG and about 1x10¹⁶ VG of AAV comprising at least one polynucleotide encoding a payload.
63. The method of item 62, wherein the total dose is selected from the group consisting of about 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, ⁹x10¹⁰, 1x10¹¹, 2x10¹¹, 3x10¹¹, 4x10¹¹, 5x10¹¹, 6x10¹¹, 7x10¹¹, 8x10¹¹, 9x10¹¹, 1x10¹², 2x10¹², 3x10¹², 4x10¹², 5x10¹², 6x10¹², 7x10¹², 8x10¹², 9x10¹², 1x10¹³, 2x10¹³, 3x10¹³, 4x10¹³, 5x10¹³, 6x10¹³, 7x10¹³, 8x10¹³, 9x10¹³, 1x10¹⁴, 2x10¹⁴, 3x10¹⁴, 4x10¹⁴, 5x10¹⁴, 6x10¹⁴, 7x10¹⁴, 8x10¹⁴, 9x10¹⁴, 1x10¹⁵, 2x10¹⁵, 3x10¹⁵, 4x10¹⁵, 5x10¹⁵, 6x10¹⁵, 7x10¹⁵, 8x10¹⁵, 9x10¹⁵, and 1x10¹⁶ VG.
64. The method of item 63, wherein the total dose is about 5x10¹⁰ VG.
65. The method of item 63, wherein the total dose is about 3x10¹³ VG.
66. The method of items 54-57, wherein the rate of prolonged infusion exceeds the rate of cerebrospinal fluid (CSF) absorption.
67. The method of any one of items 54-57, wherein the prolonged infusion comprises a composition concentration between about 1x106 VG/mL and about 1x10¹⁶ VG/mL of AAV comprising at least one polynucleotide encoding a payload.
68. The method of item 67, wherein the concentration is selected from the group consisting of 1x10⁶, 2x10⁶, 3x10⁶, 4x10⁶, 5x10⁶, 6x10⁶, 7x10⁶, 8x10⁶, 9x10⁶, 1x10⁷, 2x10⁷, 3x10⁷, 4x10⁷, 5x10⁷, 6x10⁷, 7x10⁷, 8x10⁷, 9x10⁷, 1x10⁸, 2x10⁸, 3x10⁸, 4x10⁸, 5x10⁸, 6x10⁸, 7x10⁸, 8x10⁸, 9x10⁸, 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6x10¹⁰, 7x10¹⁰, 8x10¹⁰, 9x10¹⁰, 1x10¹¹, 2x10¹¹, 3x10¹¹, 4x10¹¹, 5x10¹¹, 6x10¹¹, 7x10¹¹, 8x10¹¹, 9x10¹¹, 1x10¹², 2x10¹², 3x10¹², 4x10¹², 5x10¹², 6x10¹², 7x10¹², 8x10¹², 9x10¹², 1x10¹³, 2x10¹³, 3x10¹³, 4x10¹³, 5x10¹³, 6x10¹³, 7x10¹³, 8x10¹³, 9x10¹³, 1x10¹⁴, 2x10¹⁴, 3x10¹⁴, 4x10¹⁴, 5x10¹⁴, 6x10¹⁴, 7x10¹⁴, 8x10¹⁴, 9x10¹⁴, 1x10¹⁵, 2x10¹⁵, 3x10¹⁵, 4x10¹⁵, 5x10¹⁵, 6x10¹⁵, 7x10¹⁵, 8x10¹⁵, 9x10¹⁵, and 1x10¹⁶ VG/mL.
69. The method of item 68, wherein the concentration is 1x10¹³ VG/ml.
70. The method of item 68, wherein the concentration is 3x10¹² VG/ml.
71. The method of any one of items 54-57 wherein the AAV comprising the polynucleotide is in a buffered composition of between pH 3.0 and 8.0.
72. The method of item 71 wherein the pH is selected from the group consisting of about 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, and 8.0.
73. The method of item 71, wherein the pH is between 7.2-7.4.
74. The method of any one of items 54-57, wherein the AAV comprising the polynucleotide is in an isobaric composition, and wherein the baricity of the composition at 37°C is approximately 1g/mL.
75. The method of any one of items 54-57, wherein the AAV comprising the polynucleotide is in a hypobaric composition, and wherein the baricity of the composition at 37°C is less than 1g/mL.
76. The method of any one of items 54-57, wherein the AAV comprising the polynucleotide is in a hyperbaric composition, and wherein the baricity of the composition at 37°C is greater than 1g/mL.
77. The method of item 76, wherein the composition comprises approximately 5% to 8% dextrose.
78. The method of item 77, wherein the percentage of dextrose in the composition is selected from the group consisting of 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, and 8.0%.
79. The method of any of items 54-57, further comprising co-administration of intravenous mannitol.
80. The method of item 79, wherein a dose of approximately 0.25 to 1.0 g/kg intravenous mannitol is co-administered.
81. The method of item 80, wherein a dose of intravenous mannitol is selected from the group consisting of 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.50, 0.51, 0.52, 0.53, 0.54, 0.55, 0.56, 0.57, 0.58, 0.59, 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99, and 1.00 g/ kg.
82. The method of any one of items 54-57, wherein the AAV comprising the polynucleotide is in a composition and wherein the composition is at a temperature of 37°C.
83. The method of any one of items 54-57, wherein the AAV comprising the polynucleotide is in a composition and wherein the temperature of the composition is between approximately 20°C and 26°C.
84. The method of item 83, wherein the temperature of the composition is selected from the group consisting of 20.0°C, 20.1°C, 20.2°C, 20.3°C, 20.4°C, 20.5°C, 20.6°C, 20.7°C, 20.8°C, 20.9°C, 21.0°C, 21.1°C, 21.2°C, 21.3°C, 21.4°C, 21.5°C, 21.6°C, 21.7°C, 21.8°C, 21.9°C, 22.0°C, 22.1°C, 22.2°C, 22.3°C, 22.4°C, 22.5°C, 22.6°C, 22.7°C, 22.8°C, 22.9°C, 23.0°C, 23.1°C, 23.2°C, 23.3°C, 23.4°C, 23.5°C, 23.6°C, 23.7°C, 23.8°C, 23.9°C, 24.0°C, 24.1°C, 24.2°C, 24.3°C, 24.4°C, 24.5°C, 24.6°C, 24.7°C, 24.8°C, 24.9°C, 25.0°C, 25.1°C, 25.2°C, 25.3°C, 25.4°C, 25.5°C, 25.6°C, 25.7°C, 25.8°C, 25.9°C, and 26.0°C.
85. The method of any one of items 54-57, wherein the AAV comprises a hydrophilic capsid.
86. The method of any one of items 54-57, wherein the AAV comprises a lipophilic capsid.
87. The method of any one of items 54-57, wherein the AAV comprises a capsid which targets a specific receptor.
88. The method of any one of items 54-57 wherein the AAV comprises a capsid which comprises a specific ligand.
89. The method of items 54-57 wherein the AAV comprises a capsid selected from the group consisting of AAV1, AAV2, AAV3, AAV4, AAV5, AAV6, AAV7, AAV8, AAV9, AAV10, AAV11, AAV12, AAVrh8, AAVrh10, AAV-DJ, and AAV-DJ8.
90. The method of item 89, wherein the AAV comprises a self-complementary (SC) genome.
91. The method of item 89, wherein the AAV comprises a single stranded (SS) genome.
92. The method of item 90 or 91 wherein the AAV comprises a cell specific promoter region.
93. The method of item 90 or 91 wherein the AAV comprises a ubiquitous promoter region.
94. The method of any one of items 54-57, wherein the subject is horizontal for the duration of the prolonged infusion.
95. The method of any one of items 54-57, wherein the subject is vertical for the duration of the prolonged infusion.
96. The method of any one of items 54-57, wherein the subject is at an angle between approximately horizontal 0° to about vertical 90° for the duration of the prolonged infusion.
97. The method of item 96, wherein the subject is at an angle selected from the group consisting of 0°, 1°, 2°, 3°, 4°, 5°, 6°, 7°, 8°, 9°, 10°, 11°, 12°, 13°, 14°, 15°, 16°, 17°, 18°, 19°, 20°, 21°, 22°, 23°, 24°, 25°, 26°, 27°, 28°, 29°, 30°, 31°, 32°, 33°, 34°, 35°, 36°, 37°, 38°, 39°, 40°, 41°, 42°, 43°, 44°, 45°, 46°, 47°, 48°, 49°, 50°, 51°, 52°, 53°, 54°, 55°, 56°, 57°, 58°, 59°, 60°, 61°, 62°, 63°, 64°, 65°, 66°, 67°, 68°, 69°, 70°, 71°, 72°, 73°, 74°, 75°, 76°, 77°, 78°, 79°, 80°, 81°, 82°, 83°, 84°, 85°, 86°, 87°, 88°, 89°, and 90°.
98. The method of item 97, wherein the position of the subject changes during the duration of the prolonged infusion from horizontal to vertical.
99. The method of item 97, wherein the position of the subject changes during the duration of the prolonged infusion from vertical to horizontal.
100. The method of any one of items 54-57, wherein the administration to a subject is by a prolonged infusion pump or device.
101. The method of item 100, wherein the infusion pump or device further comprises a catheter.

## Claims

1. A recombinant adeno-associated virus particle (rAAV) for use in a method of treating CNS disorders by delivering a payload to the CNS by direct injection into the brain by intrastriatal administration, wherein the rAAV comprises a nucleic acid encoding the payload and an AAV capsid, wherein the AAV capsid is AAV9, AAVDJ8, AAVrh10 or AAV6.

2. The rAAV for use of claim 1, wherein the AAV capsid is AAV9.

3. The rAAV for use of claim 2, wherein the rAAV comprises a self-complimentary vector genome.

4. The rAAV for use of claim 1, wherein the AAV capsid is AAVDJ8.

5. The rAAV for use of claim 1, wherein the AAV capsid is AAVrh10.

6. The rAAV for use of claim 5, wherein the rAAV comprises a self-complimentary vector genome.

7. A recombinant adeno-associated virus particle (rAAV) for use in a method of treating CNS disorders by delivering a payload to the CNS by intracerebroventricular administration, wherein the rAAV comprises a nucleic acid encoding the payload and an AAV capsid, wherein the AAV capsid is AAV6, AAV5 or AAV9.

8. The rAAV for use of claim 7, wherein administration is by delivery to either of the two lateral ventricles left and right, third ventricle, and/or fourth ventricle.

9. The rAAV for use of claim 7, or 8 wherein the AAV capsid is AAV6.

10. The rAAV for use of claim 7 or 8, wherein the AAV capsid is AAV5.

11. The rAAV for use of claim 7 or 8, wherein the AAV capsid is AAV9.

12. The rAAV for use of claim 11, wherein the rAAV comprises a self-complimentary vector genome.

13. A recombinant adeno-associated virus particle (rAAV) for use in a method of treating CNS disorders by delivering a payload to the CNS by direct injection into the Cisterna Magna of the brain by intracisternal administration, wherein the rAAV comprises a nucleic acid encoding the payload and an AAVrh10 capsid.

14. A recombinant adeno-associated virus particle (rAAV) for use in a method of treating CNS disorders by delivering a payload to the CNS by direct injection into the lumbar spinal cord by intrathecal administration, wherein the rAAV comprises a nucleic acid encoding the payload and an AAVrh10 capsid.

15. The rAAV for use of claim 14, wherein the rAAV comprises a self-complimentary vector genome.
